(19) Europäisches Patentamt
European Patent Office
Office européen des brevets

(11) **EP 4 047 083 A1**

(12) **EUROPEAN PATENT APPLICATION**
published in accordance with Art. 153(4) EPC

(43) Date of publication:
**24.08.2022 Bulletin 2022/34**

(21) Application number: **20864985.5**

(22) Date of filing: **21.09.2020**

(51) International Patent Classification (IPC):
**C12N 5/0775** (2010.01)          **A61K 35/545** (2015.01)

(52) Cooperative Patent Classification (CPC):
**A61K 35/28; A61K 35/545; A61P 1/00; A61P 1/16; A61P 3/00; A61P 9/00; A61P 11/00; A61P 13/12; A61P 15/00; A61P 17/00; A61P 19/02; A61P 19/08; A61P 25/00; A61P 27/02; A61P 37/00;**          (Cont.)

(86) International application number:
**PCT/CN2020/116626**

(87) International publication number:
**WO 2021/052503 (25.03.2021 Gazette 2021/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.09.2019   CN 201910893475**
**24.06.2020   CN 202010587096**

(71) Applicants:
• **Beijing Institute for Stem Cell and Regenerative Medicine**
**Beijing 100101 (CN)**
• **Institute Of Zoology, Chinese Academy Of Sciences**
**Beijing 100101 (CN)**

(72) Inventors:
• **ZHOU, Qi**
**Beijing 100101 (CN)**
• **HU, Baoyang**
**Beijing 100101 (CN)**

• **HAO, Jie**
**Beijing 100101 (CN)**
• **LI, Wei**
**Beijing 100101 (CN)**
• **WU, Jun**
**Beijing 100101 (CN)**
• **WANG, Liu**
**Beijing 100101 (CN)**
• **GUO, Baojie**
**Beijing 100101 (CN)**
• **LI, Zhongwen**
**Beijing 100101 (CN)**
• **GAO, Tingting**
**Beijing 100101 (CN)**
• **CHEN, Yanxia**
**Beijing 100101 (CN)**
• **WANG, Hongmei**
**Beijing 100101 (CN)**

(74) Representative: **Casci, Tamara et al**
**Barzanò & Zanardo Milano S.p.A.**
**Via Borgonuovo, 10**
**20121 Milano (IT)**

(54) **PLURIPOTENT STEM CELL, PHARMACEUTICAL COMPOSITION, AND PREPARATION METHOD THEREFOR AND APPLICATION THEREOF**

(57) The present invention relates to the field of cell therapy, and specifically relates to a method for producing a mesenchymal stem cell population, the mesenchymal stem cell population and a culture supernatant thereof produced by the method, and a pharmaceutical composition containing such cells or the culture supernatant thereof. The present invention further relates to use of the mesenchymal stem cell population and the culture supernatant thereof for preventing and treating diseases.

**MMP1**

FIG. 2A

(52) Cooperative Patent Classification (CPC): (Cont.)
     **A61P 37/06**

## Description

### Technical Field

The present invention relates to the field of cell therapy. Specifically, the present invention relates to a method for producing a mesenchymal stem cell population, a mesenchymal stem cell population produced by the method and a culture supernatant thereof, and a pharmaceutical composition comprising such cells or culture supernatant thereof. The present invention also relates to a use of the mesenchymal stem cell population and the culture supernatant thereof, as well as the pharmaceutical composition comprising such cells or culture supernatant thereof for the prevention and/or treatment of a disease.

### Background Art

Mesenchymal stem cells (MSCs) are adult stem cells with self-replication ability and multi-directional differentiation potential, which come from a wide range of sources and can be isolated from almost any connective tissue, such as brain, spleen, liver, kidney, lung, bone marrow, etc. Over the past few decades, researchers have made MSCs the focus of stem cell treatment research. MSCs are easily obtained from different connective tissues and can be expanded in large quantities to obtain a large number of cells. Embryonic stem cells (ESCs)/induced pluripotent stem cells (iPSCs) are limited in clinical application due to their potential tumorigenicity and ethical issues. Mesenchymal stem cells have multi-directional differentiation ability, immunomodulatory function and low immunogenicity, and they have not yet been found to be tumorigenic, so they have become seed cells with clinical application value for stem cell treatment.

So far, most therapeutic applications take those derived from adult bone marrow or umbilical cord as research objects. Although they can be easily isolated from bone marrow and umbilical cord, there still exist problems such as limited sources and the decrease in their proliferation capacity and differentiation potential with the increase of in vitro culture time. Therefore, the problem of unstable cell quantity and quality affects the clinical application of those derived from bone marrow sources.

To solve the problem of MSC sources, it is possible to seek new sources of MSCs. Embryonic stem cells have the ability to proliferate indefinitely, and have the potential to differentiate into various cells and tissues of mesoderm, endoderm and ectoderm, so they can be used as a new source of MSCs. In recent years, many studies have reported methods of inducing mesenchymal-like cells from human embryonic stem cells. However, there are still shortcomings such as low induction efficiency, complicated induction process, and long induction time, and most methods require the use of heterologous substances such as serum, so that they cannot be used clinically.

In conclusion, in view of the fact that the current methods for obtaining mesenchymal stem cells are limited by some defects, it is very necessary to find a method for generating mesenchymal stem cells with high purity, high yield and short time-consuming, so as to be used for clinical treatment and prevention of various diseases.

### Contents of the present invention

After a lot of experiments and repeated explorations, the inventors of the present application have obtained a method for producing mesenchymal stem cells in vitro from stem cells (e.g., totipotent stem cells or pluripotent stem cells). The mesenchymal stem cells obtained by this method have significantly improved cytokine secretion amount, and thus completed the present invention. Herein, the cells of the present invention or cells obtained by the methods of the present invention may be referred to as M cells.

### Mesenchymal stem cell population

Accordingly, in a first aspect, the present invention provides a mesenchymal stem cell population, wherein the mesenchymal stem cell population has an average MMP1 expression level (e.g., in the absence of genetic modification) of at least about 10 times (e.g., at least about 20 times, at least about 30 times, at least about 40 times, at least about 50 times, at least about 60 times, at least about 70 times, at least about 80 times, at least about 90 times, at least about 100 times, at least about 150 times, at least about 200 times, at least about 300 times, at least about 400 times, at least about 500 times, at least about 1,000 times, at least about 2,000 times, at least about 3,000 times, at least about 5,000 times, at least about 8,000 times, at least about 10,000 times, or at least about 12,000 times) higher than that of a primary mesenchymal stem cell; and/or, the mesenchymal stem cell population has an average PGE2 expression level (e.g., in the absence of genetic modification) of at least about 10 times (e.g., at least about 20 times, at least about 30 times, at least about 50 times, at least about 60 times, or at least about 80 times) higher than that of a primary mesenchymal stem cell.

As used herein, the term "primary mesenchymal stem cell" refers to a mesenchymal stem cell isolated from a tissue (e.g., adipose tissue, umbilical cord, bone marrow or umbilical cord blood) directly removed from the body.

In certain embodiments, the MMP1 expression level of the mesenchymal stem cell population is at least about 10 times (e.g., at least about 50 times, at least about 100 times, at least about 200 times, at least about 300 times, at least about 400 times, at least about 500 times, at least about 1,000 times, at least about 2,000 times, at least about 3,000 times, at least about 5,000 times, at least about 8,000 times, at least about 10,000 times, or at least about 12,000 times) higher than that of primary mesenchymal stem cells at the same amount.

In certain embodiments, the PGE2 expression level of the mesenchymal stem cell population is at least about 10 times (e.g., at least about 20 times, at least about 30 times, at least about 50 times, at least about 60 times, or at least about 80 times) higher than that of primary mesenchymal stem cells at the same amount. In certain embodiments, the PGE2 expression level of the mesenchymal stem cell population is about 80 times higher than that of primary mesenchymal stem cells at the same amount.

In certain embodiments, after stimulation with IFN-$\gamma$ (e.g., 25 to 100 ng/ml), the mesenchymal stem cell population has an average PD-L1 expression level (e.g., in the absence of genetic modification) higher than that of a primary mesenchymal stem cell. In certain embodiments, after stimulation with IFN-$\gamma$ (e.g., 25 to 100 ng/ml), the average PD-L1 expression level of the mesenchymal stem cell population is at least about 2 times (e.g., at least about 3 times) higher than that of a primary mesenchymal stem cell. In certain embodiments, after stimulation with 50 to 100 ng/ml of IFN-$\gamma$, the average PD-L1 expression level of the mesenchymal stem cell population is about 3 times higher than that of a primary mesenchymal stem cell. In certain embodiments, after stimulation with IFN-$\gamma$ (e.g., 25 to 100 ng/ml), the average PD-L1 expression level of the mesenchymal stem cell population is at least 2 times (e.g., at least about 3 times) higher than that of primary mesenchymal stem cells at the same amount. In certain embodiments, after stimulation with 50 to 100 ng/ml of IFN-$\gamma$, the average PD-L1 expression level of the mesenchymal stem cell population is about 3 times higher than that of primary mesenchymal stem cells at the same amount.

In certain embodiments, the average IDO expression level of the mesenchymal stem cell population (e.g., in the absence of genetic modification) is higher than that of a primary mesenchymal stem cell. In certain embodiments, the average IDO expression level of the mesenchymal stem cell population is at least about 10 times (e.g., at least about 20 times, at least about 30 times, at least about 50 times, at least about 60 times, at least about 80 times, at least about 100 times, or at least about 110 times) higher than that of a primary mesenchymal stem cell. In certain embodiments, the average IDO expression level of the mesenchymal stem cell population is about 110 times higher than that of a primary mesenchymal stem cell. In certain embodiments, the mesenchymal stem cell population has an IDO expression level of at least about 10 times (e.g., at least about 20 times, at least about 30 times, at least about 50 times, at least about 60 times, at least about 80 times, at least about 100 times, or at least about 110 times) higher than that of primary mesenchymal stem cells at the same amount. In certain embodiments, the mesenchymal stem cell population has an IDO expression level of at least about 110 times higher than that of primary mesenchymal stem cells at the same amount.

Herein, the expression can be monitored by measuring the level of the gene's full-length mRNA, mRNA fragment, full-length protein or protein fragment. Thus, in certain embodiments, the expression level is the mRNA level or the protein level.

In some embodiments, the expression is assessed by analyzing the expression of mRNA transcript of the gene. For example, the expression of the aforementioned genes in the cell population is determined by determining the presence or content of mRNA of IDO, MMP1, PDL1 or PGE2 in the cell population by RT-PCR.

In other embodiments, the expression is assessed by analyzing the expression of protein product of the gene. For example, the expression of the aforementioned genes in the cell population is determined by determining the presence or content of IDO, MMP1, PDL1 or PGE2 protein in the culture supernatant of the cell population by immunological detection. Thus, in certain embodiments, the expression level of the gene (e.g., IDO, MMP1, PDL1 or PGE2) is assessed by the level of the corresponding protein secreted in the culture supernatant.

In certain embodiments, the mesenchymal stem cell population has one or more of the aforementioned gene expression characteristics in the absence of genetic modification, in which the term "in the absence of genetic modification" refers to without undergoing a process where an exogenous genetic material in the form of DNA or RNA is introduced into the total genetic material of a cell, wherein the term "exogenous genetic material" may refer to an artificially introduced nucleotide sequence which is exogenous relative to a cell that has not been genetically modified. It is easy to understand that the above-mentioned "in the absence of genetic modification" is only used to describe the condition that the mesenchymal stem cell population of the present invention has one or more of the aforementioned gene expression characteristics, and is not used as a limitation that the mesenchymal stem cell population of the present invention shall not contain a genetic modification. Thus, in certain embodiments, the mesenchymal stem cell population of the present invention may comprise one or more genetic modifications.

In certain embodiments, the mesenchymal stem cell population is generated from a stem cell. In certain embodiments, the stem cell is a totipotent stem cell or pluripotent stem cell. In certain embodiments, the pluripotent stem cell is selected from embryonic stem cell, haploid stem cell, induced pluripotent stem cell, or adult stem cell. In certain embodiments, the mesenchymal stem cell population is generated from an embryonic stem cell or induced pluripotent stem cell.

In certain embodiments, the mesenchymal stem cell population is generated in vitro.

In certain embodiments, the mesenchymal stem cell population further has the following characteristics:

(1) comprising ≥80% (e.g., ≥85%, ≥90%, ≥95%, ≥96%, ≥97%, ≥98%, ≥99%, or 100%) of cells expressing one or more selected from the group consisting of CD105, CD73, CD90, CD13, CD29, CD44, CD166 and HLA-ABC;

(2) comprising ≤2% (e.g., ≤1%, ≤0.5%, ≤0.2%, ≤0.1%, or ≤0.01%) of cells expressing one or more selected from the group consisting of CXCL1, CD34, CD45, CD133, FGFR2, CD271, Stro-1, and CXCR4.

In certain embodiments, the mesenchymal stem cell population further has one or more of the following characteristics:

(3) having a cell expressing CD274;

(4) having a cell expressing CD24;

(5) having a cell expressing CD31.

In certain embodiments, the CD274+ cell has a proportion of no less than 80%, such as 80% to 95%, such as about 80%, about 85%, about 86%, about 87%, about 88%, about 89%, about 90%, about 91%, about 92%, about 93%, about 94%, or about 95%.

In certain embodiments, the CD24+ cell has a proportion of no less than 50%, such as 50% to 70%, such as about 50%, about 55%, about 56%, about 57%, about 58%, about 59%, about 60%, about 61%, about 62%, about 63%, about 64%, about 65%, or about 70%.

In certain embodiments, the CD31+ cell has a proportion of no less than 5%, such as 5% to 20%, such as about 5%, about 10%, about 12%, about 15%, about 18%, or about 20%.

The mesenchymal stem cell population of the present invention can be formulated and administered as a pharmaceutical composition. Such pharmaceutical composition can be in any form known in the medical field, which is preferably an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

**Preparation method of mesenchymal stem cell population**

In a second aspect, the present invention provides a method for producing a mesenchymal stem cell population, or a method for producing the mesenchymal stem cell population described in the first aspect, comprising the following steps:

(1) culturing a stem cell to form an embryoid body using a first culture medium; wherein the first culture medium is a basal medium supplemented with the following substances: one or more serum replacements, one or more non-essential amino acids, glutamine or a stabilized dipeptide of L-alanyl-L-glutamine, and bFGF;

(2) culturing the embryoid body using a second culture medium to induce its differentiation into mesenchymal stem cells; wherein the second culture medium is a basal medium supplemented with the following substances: one or more serum replacements, one or more non-essential amino acids, glutamine or a stabilized dipeptide of L-alanyl-L-glutamine, and one or more growth factors.

In certain embodiments, the step (2) comprises: attaching the embryoid body to a culture container and culturing it with the second culture medium.

As used herein, the term "basal medium" refers to any culture medium capable of supporting cell growth, typically comprising inorganic salt, vitamin, glucose, buffer system and essential amino acids, and typically having an osmolarity of about 280 to 330 mOsmol.

In certain embodiments, the stem cell described in the step (1) is a totipotent stem cell or pluripotent stem cell. In certain embodiments, the pluripotent stem cell is selected from embryonic stem cell, haploid stem cell, induced pluripotent stem cell, or adult stem cell.

In certain embodiments, the first culture medium has one or more of the following characteristics:

(i) the one or more serum replacements has a total content of 3 to 30% (v/v), for example about 3% (v/v), about 5% (v/v), about 8% (v/v), about 10% (v/v), about 12% (v/v), about 15% (v/v), about 18% (v/v), about 20% (v/v), about 22% (v/v), about 25% (v/v), about 28% (v/v) or about 30% (v/v);

(ii) the one or more non-essential amino acids each has a content of 0.1 to 0.5 mM, such as about 0.1 mM, about 0.2 mM, about 0.3 mM, about 0.4 mM, or about 0.5 mM;

(iii) the glutamine or stabilized dipeptide of L-alanyl-L-glutamine has a content of 1 to 5 mM, such as about 1 mM, about 2 mM, about 3 mM, about 4 mM, or about 5 mM;

(iv) the bFGF has a content of 1 to 100 ng/ml, such as 2 to 100 ng/ml, 2 to 50 ng/ml, 5 to 100 ng/ml, 5 to 50 ng/ml, or 5 to 20 ng/ml; e.g., about 1 ng/ml, about 2 ng/ml, about 3 ng/ml, about 5 ng/ml, about 8 ng/ml, about 10 ng/ml, about 15 ng/ml, about 20 ng/ml, about 25 ng/ml, about 30 ng/ml, about 35 ng/ml, about 40 ng/ml, about 45 ng/ml, about 50 ng/ml, about 55 ng/ml, about 60 ng/ml, about 65 ng/ml, about 70 ng/ml, about 75 ng/ml, about 80 ng/ml, about 85 ng/ml, about 90 ng/ml, about 95 ng/ml, or about 100 ng/ml.

In certain embodiments, the first culture medium has one or more of the following characteristics:

(a) the serum replacement is selected from the group consisting of KOSR, MSC serum-free Supplement, Ultraser™ G and any combination thereof; preferably, the serum replacement is KnockOut™ SR (e.g., Thermo: Cat. No. 10828028) (hereinafter referred to as KOSR);

(b) the non-essential amino acid is selected from the group consisting of glycine, L-alanine, L-asparagine, L-aspartic acid, L-glutamic acid, L-proline, L-serine and any combination thereof;

(c) the basal medium is selected from the group consisting of KnockOut™ DMEM (e.g., Gibco: Cat. No. 10829018) (hereinafter referred to as KO-DMEM), KnockOut™ DMEM/F-12 (e.g., Gibco: Cat. No. 12660-012) (hereinafter referred to as KO-DMEM) KO-DMEM/F12), DMEM, α-MEM, F-12, MEM, BME, RPMI 1640, G-MEM and any combination thereof; preferably, the basal medium is selected from the group consisting of KO-DMEM, KO-DMEM/F 12, DMEM, DMEM/F12; preferably, the basal medium is KO-DMEM.

In certain embodiments, the first culture medium comprises: KO-DMEM, KOSR, glycine, L-alanine, L-asparagine, L-aspartic acid, L-glutamic acid, L-proline, L-serine, stabilized dipeptide of L-alanyl-L-glutamine, and bFGF. In certain embodiments, the first culture medium comprises: 3 to 30% (v/v) KOSR, 1 to 5 mM stabilized dipeptide of L-alanyl-L-glutamine, 1 to 100 ng/ml bFGF, and the following amino acids each at a concentration of about 0.1 mM glycine, L-alanine, L-asparagine, L-aspartic acid, L-glutamic acid, L-proline, L-serine.

In certain embodiments, the first culture medium further comprises β-mercaptoethanol. In certain embodiments, the β-mercaptoethanol has a content of 0.1 to 0.5% (v/v), e.g., about 0.1% (v/v), about 0.2% (v/v), about 0.3% (v/v), about 0.4% (v/v), or about 0.5% (v/v).

In certain embodiments, the first culture medium consists essentially of the following components: KO-DMEM, KOSR, glycine, L-alanine, L-asparagine, L-aspartic acid, L-glutamic acid, L-proline, L-serine, stabilized dipeptide of L-alanyl-L-glutamine, bFGF and β-mercaptoethanol.

In certain embodiments, the first culture medium comprises: 3 to 30% (v/v) KOSR, 1 to 5 mM stabilized dipeptide of L-alanyl-L-glutamine, 1 to 100 ng/ml bFGF, 0.1 to 0.5% (v/v) β-mercaptoethanol, and the following amino acids each at a concentration of about 0.1 mM: glycine, L-alanine, L-asparagine, L-aspartic acid, L-glutamic acid, L-proline, L-serine.

In certain embodiments, the first culture medium comprises: about 15% (v/v) KOSR, about 1 mM stabilized dipeptide of L-alanyl-L-glutamine, about 8 ng/ml bFGF, about 0.1% (v/v) β-mercaptoethanol, and the following amino acids each at a concentration of about 0.1 mM: glycine, L-alanine, L-asparagine, L-aspartic acid, L-glutamic acid, L-proline, L-serine.

In certain embodiments, the first culture medium comprises: about 18% (v/v) KOSR, about 1 mM stabilized dipeptide of L-alanyl-L-glutamine, about 12 ng/ml bFGF, about 0.1% (v/v) β-mercaptoethanol, and the following amino acids each at a concentration of about 0.1 mM: glycine, L-alanine, L-asparagine, L-aspartic acid, L-glutamic acid, L-proline, L-serine.

In certain embodiments, the first culture medium comprises: about 20% (v/v) KOSR, about 2 mM stabilized dipeptide of L-alanyl-L-glutamine, about 10 ng/ml bFGF, about 0.1% (v/v) β-mercaptoethanol, and the following amino acids each at a concentration of about 0.1 mM: glycine, L-alanine, L-asparagine, L-aspartic acid, L-glutamic acid, L-proline, L-serine.

In certain embodiments, the first culture medium comprises: about 22% (v/v) KOSR, about 2 mM stabilized dipeptide of L-alanyl-L-glutamine, about 12 ng/ml bFGF, about 0.2% (v/v) β-mercaptoethanol, and the following amino acids each at a concentration of about 0.1 mM: glycine, L-alanine, L-asparagine, L-aspartic acid, L-glutamic acid, L-proline, L-serine.

In certain embodiments, the first culture medium comprises: about 22% (v/v) KOSR, about 2 mM stabilized dipeptide of L-alanyl-L-glutamine, about 12 ng/ml bFGF, about 0.1% (v/v) β-mercaptoethanol, and the following amino acids each at a concentration of about 0.2 mM: glycine, L-alanine, L-asparagine, L-aspartic acid, L-glutamic acid, L-proline, L-serine.

In certain exemplary embodiments, the first culture medium consists essentially of: KO-DMEM (Gibco: Cat. No. 10829018), KOSR (Thermo: Cat. No. 10828028), NEAA (Gibco: Cat. No. 11140050), GlutaMAX (Gibco: Cat. No. A1286001), bFGF and β-mercaptoethanol.

In certain exemplary embodiments, the first culture medium comprises: 18 to 22% (v/v) KOSR, 0.5 to 1.5% (v/v) GlutaMAX, 1 to 100 ng/ml bFGF, 0.1 to 0.5% (v/v) β-mercaptoethanol, and 1 to 2% (v/v) NEAA.

In certain exemplary embodiments, the first culture medium comprises: about 15% (v/v) KOSR, about 0.5% (v/v) GlutaMAX, about 8 ng/ml bFGF, about 0.1% (v/v) β-mercaptoethanol, and about 1% (v/v) NEAA.

In certain exemplary embodiments, the first culture medium comprises: about 18% (v/v) KOSR, about 0.5% (v/v) GlutaMAX, about 12 ng/ml bFGF, about 0.1% (v/v) β-mercaptoethanol, and about 1% (v/v) NEAA.

In certain exemplary embodiments, the first culture medium comprises: about 20% (v/v) KOSR, about 1% (v/v) GlutaMAX, about 10 ng/ml bFGF, about 0.1% (v/v) β-mercaptoethanol, and about 1% (v/v) NEAA.

In certain exemplary embodiments, the first culture medium comprises: about 22% (v/v) KOSR, about 1% (v/v) GlutaMAX, about 12 ng/ml bFGF, about 0.2% (v/v) β-mercaptoethanol, and about 1% (v/v) NEAA.

In certain exemplary embodiments, the first culture medium comprises: about 22% (v/v) KOSR, about 1% (v/v) GlutaMAX, about 12 ng/ml bFGF, about 0.1% (v/v) β-mercaptoethanol, and about 1% (v/v) NEAA.

In certain embodiments, the second culture medium has one or more of the following characteristics:

(i) the one or more serum replacements has a total content of 1 to 40% (v/v), e.g., 1 to 35% (v/v), 1 to 30% (v/v), 2 to 30%(v/v), 5 to 30%(v/v), 1 to 20%(v/v), 2 to 20%(v/v), 5 to 20%(v/v), 1 to 10% (v/v), 2 to 10% (v/v), or 5 to 10% (v/v); such as about 1% (v/v), about 2% (v/v), about 3% (v/v), about 5% (v/v), about 8% (v/v), about 10% (v/v), about 12% (v/v), about 15% (v/v), about 18% (v/v), about 20% (v/v), about 22% (v/v), about 25% (v/v), about 28% (v/v), or about 30%(v/v);

(ii) the one or more non-essential amino acids each has a content of 0.1 to 0.5 mM, such as 0.1 to 0.2 mM, such as about 0.1 mM, about 0.2 mM, about 0.3 mM, about 0.4 mM, or about 0.5 mM;

(iii) the glutamine or stabilized dipeptide of L-alanyl-L-glutamine has a content of 1 to 5 mM, such as 1 to 3 mM, such as about 1 mM, about 2 mM, about 3 mM, about 4 mM or about 5 mM;

(iv) the one or more growth factors each has a content of 1 to 100 ng/ml, such as about 1 ng/ml, about 2 ng/ml, about 3 ng/ml, about 5 ng/ml, about 8 ng/ml, about 10 ng/ml, about 15 ng/ml, about 20 ng/ml, about 25 ng/ml, about 30 ng/ml, about 35 ng/ml, about 40 ng/ml, about 45 ng/ml, about 50 ng/ml, about 55 ng/ml, about 60 ng/ml, about 65 ng/ml, about 70 ng/ml, about 75 ng/ml, about 80 ng/ml, about 85 ng/ml, about 90 ng/ml, about 95 ng/ml, or about 100 ng/ml.

In certain embodiments, the second culture medium has one or more of the following characteristics:

(a) the serum replacement is selected from the group consisting of KOSR, MSC serum-free Supplement, Ultroser™ G and any combination thereof;

(b) the non-essential amino acid is selected from the group consisting of glycine, L-alanine, L-asparagine, L-aspartic acid, L-glutamic acid, L-proline, L-serine and any combination thereof;

(c) the basal medium is selected from the group consisting of KO-DMEM, KO-DMEM/F12, α-MEM, DMEM, F12, MEM, BME, RPMI 1640, G-MEM and any combination thereof; preferably, the basal medium is selected from the group consisting of KO-DMEM, KO-DMEM/F12, α-MEM, DMEM, DMEM/F12.

In certain embodiments, the serum replacement is KOSR, and one selected from the group consisting of MSC serum-free Supplement (e.g., TBD: Cat. No. SC2013-G-B) and Ultraser™ G (e.g., PALL: Cat. No. 15950-017) (hereinafter cited as Ultroser G). In certain embodiments, the volume ratio of KOSR to MSC serum-free Supplement or Ultraser G ranges from 2:1 to 150:1, such as about 2:1, about 3:1, about 4:1, about 5:1, about 6:1, about 7:1, about 8:1, about 9:1, about 10:1, about 20:1, about 50:1, about 80:1, about 100:1, about 120:1, or about 150:1. In certain embodiments, the volume ratio of KOSR to MSC serum-free Supplement or Ultraser G is 10:1 to 1:2, such as about 10:1, about 9:1, about 8:1, about 7:1, about 6:1, about 5:1, about 4:1, about 3:1, about 2:1, about 1:1, or about 1:2.

In certain embodiments, the KOSR has a content of about 1 to 30% (v/v), about 1 to 20% (v/v), about 1 to 10% (v/v), about 2 to 10% (v/v), or about 5 to 10% (v/v). In certain embodiments, the MSC serum-free Supplement or Ultraser G has a content of at about 1 to 10% (v/v), or about 1 to 5% (v/v).

In certain embodiments, the second culture medium comprises: KO-DMEM/F12, $\alpha$-MEM, MSC serum-free Supplement or Ultraser G, KOSR, glycine, L-alanine, L-asparagine, L-aspartic acid, L-glutamic acid, L-proline, L-serine, stabilized dipeptide of L-alanyl-L-glutamine, one or more growth factors (e.g., one or more selected from the group consisting of VEGF, bFGF, EGF, TGF$\beta$, PDGF). In certain embodiments, the second culture medium comprises: 1 to 10% (v/v) Ultraser G, 1 to 20% (v/v) KOSR, 1 to 5 mM stabilized dipeptide of L-alanyl-L-glutamine, one or more growth factors (e.g., one or more selected from the group consisting of VEGF, bFGF, EGF, TGF$\beta$, PDGF) each at a concentration of 1 to 100 ng/ml), and the following amino acids each at concentration of 0.1 to 0.5 mM: glycine, L-alanine, L-asparagine, L-aspartic acid, L-glutamic acid, L-proline, L-serine.

In certain embodiments, the second culture medium further comprises ascorbic acid. In certain embodiments, the ascorbic acid has a content of 1 to 100 $\mu$g/ml, such as 1 to 100 $\mu$g/ml, 1 to 50 $\mu$g/ml, 1 to 20 $\mu$g/ml or 5 to 20 $\mu$g/ml; such as about 1 $\mu$g/ml, about 10 $\mu$g/ml, about 100 $\mu$g/ml, about 500 $\mu$g/ml, or about 1,000 $\mu$g/ml.

In certain embodiments, the second culture medium consists essentially of: KO-DMEM/F12, $\alpha$-MEM, MSC serum-free Supplement or Ultraser G, KOSR, glycine, L-alanine, L-asparagine, L-aspartic acid, L-glutamic acid, L-proline, L-serine, stabilized dipeptide of L-alanyl-L-glutamine, ascorbic acid, and one or more growth factors (e.g., one or more selected from the group consisting of VEGF, bFGF, EGF, TGF$\beta$, PDGF).

In certain embodiments, the second culture medium comprises: 1 to 5% (v/v) (e.g., about 1% (v/v), about 2% (v/v), about 3% (v/v), about 4% (v/v) or about 5% (v/v)) of Ultraser G, 2 to 20% (v/v) (e.g., about 2% (v/v), about 4% (v/v), about 6% (v/v), about 8% (v/v), about 10% (v/v), about 12% (v/v), about 14% (v/v), about 16% (v/v), about 18% (v/v) or about 20% (v/v)) of KOSR, 1 to 5 mM stabilized dipeptide of L-alanyl-L-glutamine, 1 to 1,000 $\mu$g/ml ascorbic acid, one or more growth factors (e.g., one or more selected from VEGF, bFGF, EGF, TGF$\beta$, PDGF) each at a concentration of 1 to 100 ng/ml, and the following amino acids each at a concentration of 0.1 to 0.5 mM: glycine, L-alanine, L-asparagine, L-aspartic acid, L-glutamic acid, L-proline, L-serine.

In certain embodiments, the second culture medium comprises: about 1% (v/v) Ultroser G, about 4% (v/v) KOSR, about 2 mM stabilized dipeptide of L-alanyl-L-glutamine, about 100 $\mu$g/ml ascorbic acid, one or more growth factors (e.g., one or more selected from the group consisting of VEGF, bFGF, EGF, TGF$\beta$, PDGF) each at a concentration of about 1 to 100 ng/ml, and the following amino acids each at a concentration of about 0.1 mM: glycine, L-alanine, L-asparagine, L-aspartic acid, L-glutamic acid, L-proline, L-serine.

In certain embodiments, the second culture medium comprises: about 1% (v/v) Ultroser G, about 6% (v/v) KOSR, about 2 mM stabilized dipeptide of L-alanyl-L-glutamine, about 100 $\mu$g/ml ascorbic acid, one or more growth factors (e.g., one or more selected from the group consisting of VEGF, bFGF, EGF, TGF$\beta$, PDGF) each at a concentration of about 1 to 100 ng/ml, and the following amino acids each at a concentration of about 0.1 mM: glycine, L-alanine, L-asparagine, L-aspartic acid, L-glutamic acid, L-proline, L-serine.

In certain embodiments, the second culture medium comprises: about 1% (v/v) Ultroser G, about 8% (v/v) KOSR, about 2 mM stabilized dipeptide of L-alanyl-L-glutamine, about 100 $\mu$g/ml ascorbic acid, one or more growth factors (e.g., one or more selected from the group consisting of VEGF, bFGF, EGF, TGF$\beta$, PDGF) each at a concentration of about 1 to 100 ng/ml, and the following amino acids each at a concentration of about 0.1 mM: glycine, L-alanine, L-asparagine, L-aspartic acid, L-glutamic acid, L-proline, L-serine.

In certain embodiments, the second culture medium comprises: about 2% (v/v) Ultroser G, about 4% (v/v) KOSR, about 1 mM stabilized dipeptide of L-alanyl-L-glutamine, about 100 $\mu$g/ml ascorbic acid, one or more growth factors (e.g., one or more selected from the group consisting of VEGF, bFGF, EGF, TGF$\beta$, PDGF) each at a concentration of about 1 to 100 ng/ml, and the following amino acids each at a concentration of about 0.1 mM: glycine, L-alanine, L-asparagine, L-aspartic acid, L-glutamic acid, L-proline, L-serine.

In certain embodiments, the second culture medium comprises: about 2% (v/v) Ultroser G, about 6% (v/v) KOSR, about 1 mM stabilized dipeptide of L-alanyl-L-glutamine, about 100 $\mu$g/ml ascorbic acid, one or more growth factors (e.g., one or more selected from the group consisting of VEGF, bFGF, EGF, TGF$\beta$, PDGF) each at a concentration of about 1 to 100 ng/ml, and the following amino acids each at a concentration of about 0.1 mM: glycine, L-alanine, L-asparagine, L-aspartic acid, L-glutamic acid, L-proline, L-serine.

In certain embodiments, the second culture medium comprises: about 2% (v/v) Ultroser G, about 8% (v/v) KOSR, about 1 mM stabilized dipeptide of L-alanyl-L-glutamine, about 100 $\mu$g/ml ascorbic acid, one or more growth factors (e.g., one or more selected from the group consisting of VEGF, bFGF, EGF, TGFβ, PDGF) each at a concentration of about 1 to 100 ng/ml, and the following amino acids each at a concentration of about 0.1 mM: glycine, L-alanine, L-asparagine, L-aspartic acid, L-glutamic acid, L-proline, L-serine.

In certain exemplary embodiments, the second culture medium consists essentially of the following components: KO-DMEM/F12 (Gibco: Cat. No. 12660-012), α-MEM (HyClone: Cat. No. SH30265.01B), Ultraser G (PALL: Cat. No. 15950-017), KOSR (Thermo: Cat. No. 10828028), NEAA (Gibco: Cat. No. 11140050), GlutaMAX (Gibco: Cat. No. A1286001), ascorbic acid, one or more growth factors (e.g., one or more selected from the group consisting of VEGF, bFGF, EGF, TGFβ, PDGF).

In certain exemplary embodiments, the second culture medium comprises: 1 to 2% (v/v) Ultraser G, 4 to 6% (v/v) KOSR, 0.5 to 1.5% (v/v) GlutaMAX, 1 to 1,000 μg/ml ascorbic acid, one or more growth factors (e.g., one or more selected from the group consisting of VEGF, bFGF, EGF, TGFβ, PDGF) each at a concentration of about 1 to 100 ng/ml, and 1 to 2% (v/v) NEAA.

In certain exemplary embodiments, the second culture medium comprises: about 1% (v/v) Ultroser G, about 4% (v/v) KOSR, about 1% (v/v) GlutaMAX, about 100 μg/ml ascorbic acid, one or more growth factors (e.g., one or more selected from the group consisting of VEGF, bFGF, EGF, TGFβ, PDGF) each at a concentration of about 1 to 10 ng/ml, and about 1% (v/v) NEAA.

In certain exemplary embodiments, the second culture medium comprises: about 1% (v/v) Ultroser G, about 6% (v/v) KOSR, about 1% (v/v) GlutaMAX, about 100 μg/ml ascorbic acid, one or more growth factors (e.g., one or more selected from the group consisting of VEGF, bFGF, EGF, TGFβ, PDGF) each at a concentration of about 1 to 10 ng/ml, and about 1% (v/v) NEAA.

In certain exemplary embodiments, the second culture medium comprises: about 1% (v/v) Ultroser G, about 8% (v/v) KOSR, about 1% (v/v) GlutaMAX, about 100 μg/ml ascorbic acid, one or more growth factors (e.g., one or more selected from the group consisting of VEGF, bFGF, EGF, TGFβ, PDGF) each at a concentration of about 1 to 10 ng/ml, and about 1% (v/v) NEAA.

In certain exemplary embodiments, the second culture medium comprises: about 2% (v/v) Ultroser G, about 4% (v/v) KOSR, about 0.5% (v/v) GlutaMAX, about 100 μg/ml ascorbic acid, one or more growth factors (e.g., one or more selected from the group consisting of VEGF, bFGF, EGF, TGFβ, PDGF) each at a concentration of about 1 to 10 ng/ml, and about 1% (v/v) NEAA.

In certain exemplary embodiments, the second culture medium comprises: about 2% (v/v) Ultroser G, about 6% (v/v) KOSR, about 0.5% (v/v) GlutaMAX, about 100 μg/ml ascorbic acid, one or more growth factors (e.g., one or more selected from the group consisting of VEGF, bFGF, EGF, TGFβ, PDGF) each at a concentration of about 1 to 10 ng/ml, and about 1% (v/v) NEAA.

In certain exemplary embodiments, the second culture medium comprises: about 2% (v/v) Ultroser G, about 8% (v/v) KOSR, about 0.5% (v/v) GlutaMAX, about 100 μg/ml ascorbic acid, one or more growth factors (e.g., one or more selected from the group consisting of VEGF, bFGF, EGF, TGFβ, PDGF) each at a concentration of about 1 to 10 ng/ml, and about 1% (v/v) NEAA.

In certain embodiments, the components contained in the first culture medium and the second culture medium are all of cell therapy grade (CTS grade). In certain embodiments, the basal medium (e.g., KO-DMEM), serum replacement (e.g., KOSR), stabilized dipeptide of L-alanyl-L-glutamine contained in the first culture medium and the second culture medium are all of cell therapy grade (CTS grade).

In certain embodiments, the step (1) comprises culturing the pluripotent stem cells in a low-attachment cell culture vessel.

As used herein, the term "low-attachment cell culture vessel" refers to a culture vessel with a coating on its surface that prevents the adsorption of proteins on the surface of the culture vessel, thereby minimizing the adhesion of monolayer cells to the culture container. Such cell culture vessel is well known to those of skill in the art and includes, but is not limited to, Coming's low-attachment dish (Cat. No. 3262).

In certain embodiments, the culturing in step (1) has a duration time of 3 to 14 days, such as about 3 days, about 4 days, about 5 days, about 7 days, about 10 days, or about 14 days.

In certain embodiments, the culturing in step (1) has a duration time of 4 to 7 days, such as about 5 days.

In certain embodiments, the step (2) comprises inoculating the embryoid body of step (1) at a density of about 1 embryoid body/cm$^2$ in the culture container.

In certain embodiments, the step (2) comprises culturing the embryoid body in a culture vessel coated with gelatin, collagen type I, collagen type IV, vitronectin, fibronectin or polylysine.

In certain embodiments, the step (2) comprises culturing the embryoid body in a culture vessel coated with vitronectin.

In certain embodiments, the culturing in step (2) has a duration time of 10 to 21 days, such as about 10 days, about 14 days, or about 21 days.

In certain embodiments, the culturing in step (2) has a duration time of 10 to 14 days, such as about 14 days.

In certain embodiments, the step (2) comprises replacing with fresh second culture medium every day or every 1 to 7 days (e.g., every 1, 2, 3, 4, 5, 6, or 7 days). In certain embodiments, the step (2) comprises discarding the spent media and replacing with fresh second culture medium every day or every 1 to 7 days (e.g., every 1, 2, 3, 4, 5, 6, or 7 days).

In certain embodiments, in steps (1) to (2), the culturing is performed under a condition of 37°C, 5% $CO_2$. In certain embodiments, in steps (1) to (2), the culturing is performed in an incubator at 37°C, 5% $CO_2$.

In some embodiments, the cells attached to the culture container in the step (2) are mesenchymal stem cells of P0 (Passage 0). In certain embodiments, when the cells attached to the culture container as described in step (2) have a confluency degree of no less than about 80% (e.g., no less than about 85%, no less than about 90%, or no less than about 95%), the cells can be separated from the culture container so as to obtain mesenchymal stem cells of P0 (Passage 0).

Therefore, in certain embodiments, the method further comprises: (3) separating the cells attached to the culture container in step (2), thereby obtaining mesenchymal stem cells.

In certain embodiments, the method further comprises passaging the mesenchymal stem cells of step (3).

In certain embodiments, the cells are passaged when the cells have a confluence of greater than or equal to about 80% (e.g., greater than or equal to about 85%, greater than or equal to about 90%, or greater than or equal to about 95%).

In certain embodiments, the mesenchymal stem cells are passaged for 1, 2, 3, 4, or 5 passages.

The method for passaging cells is well known to those skilled in the art. For example, the method may comprise: separating cells from a culture container and uniformly dispersing the cells in a culture medium, then inoculating them in a culture container. After adding an appropriate amount of medium, the replacement with an appropriate amount of fresh culture medium is performed at a regular interval (for example, every 1 to 5 days) according to the cell growth state, and the passaging operation is repeated when the cells grow to reach a confluence of 70 to 100%. Each time the cells are passaged, the passage is increased by 1.

In certain embodiments, the passaging comprises: performing the passaging at a cell density of about $5 \times 10^3$ to $5 \times 10^4$ cells/cm$^2$ (e.g., about $5 \times 10^3$ cells/cm$^2$, about $1 \times 10^4$ cells/cm$^2$, about $2 \times 10^4$ cells/cm$^2$, about $3 \times 10^4$ cells/cm$^2$, about $4 \times 10^4$ cells/cm$^2$, or about $5 \times 10^4$ cells/cm$^2$).

In certain embodiments, the passaging comprises: inoculating the cells in the second culture medium for culturing.

In certain embodiments, the separating comprises disrupting the adhesion of the mesenchymal stem cells to the culture container by the following method: (i) contacting the culture with one or more enzymes selected from the group consisting of trypsin or analog thereof, collagenase, dispase, papain, mixture of collagenase and dispase, and mixture of collagenase and trypsin or analog thereof; (ii) performing mechanical separation by using a cell scraper, etc.; alternatively, (iii) contacting the culture with EDTA or EGTA.

In certain embodiments, the separating comprises disrupting the adhesion of the mesenchymal stem cells to the culture container by enzymatic digestion.

In certain embodiments, the enzyme is trypsin (e.g., Gibco: Cat. No. 25200072).

Optionally, after the umbilical cord mesenchymal stem cells are obtained by culture, the cell growth curve can be determined by MTT method, WST method, DNA content detection method, ATP detection method, etc., so as to evaluate the growth activity of the umbilical cord mesenchymal stem cells. In addition, the separated and cultured umbilical cord mesenchymal stem cells can be identified by flow cytometry detection of cell surface markers, three-directional differentiation assay, and detection of cell expression genes by PCR.

Prior to step (1), stem cells (e.g., totipotent stem cells or pluripotent stem cells, such as embryonic stem cells, haploid stem cells, induced pluripotent stem cells, or adult stem cells) can be propagated and maintained using any culture method known in the art. For example, embryonic stem cells can be cultured in the presence of feeder cells such as murine cells (e.g., murine embryonic fibroblasts (MEF)), human feeder cells (e.g., adult skin cells, neonatal dermal fibroblasts (HNDF), etc.). For example, embryonic stem cells can be cultured in a xenobiotic-free culture, and/or under condition without feeder cells. See Klimanskaya et al, Lancet. 2005 May 7 to 13; 365(9471): 1636-41; Richards et al, stem cell Cells. 2003; 21(5): 546-56; U.S. Patent No. 7,410,798; Ilic et al., stem cells Dev., 2009 Nov; 18(9): 1343-5; Xu et al., Nat Biotechnol., 2001 Oct; 19(10): 971-4, each of these references is hereby incorporated by reference in its entirety. For example, embryonic stem cells can be cultured on a matrix, and the matrix can be selected from the group consisting of: laminin, fibronectin, vitronectin, proteoglycan, nestin, collagen, collagen I, collagen IV, collagen VIII, heparan sulfate, Matrigel (TM) (a soluble preparation derived from Engelbreth-Holm-Swarm (EHS) mouse sarcoma cells), Cell Start, human basement membrane extract, and any combination thereof.

In a third aspect, the present invention also relates to a mesenchymal stem cell population produced by the method of the second aspect.

In certain embodiments, the mesenchymal stem cell population is as defined in the first aspect.

## Kit

In a fourth aspect, the present invention provides a kit, which comprises a first culture medium and a second culture medium provided separately, wherein,

the first culture medium is a basal medium supplemented with the following substances: one or more serum replacements, one or more non-essential amino acids, glutamine or stabilized dipeptide of L-alanyl-L-glutamine, and bFGF;

the second culture medium is a basal medium supplemented with the following substances: one or more serum replacements, one or more non-essential amino acids, glutamine or stabilized dipeptide of L-alanyl-L-glutamine, and one or more growth factors.

In certain embodiments, the first culture medium and the second culture medium are as defined in any one of the embodiments of the second aspect.

## Culture and culture supernatant

In a fifth aspect, the present invention provides a culture, which comprises the mesenchymal stem cell population of the first or third aspect, and a culture medium.

The culture medium is any culture medium that can be used for culturing stem cells, and its example includes KO-DMEM, KO-DMEM/F12 (mixture of KO-DMEM and F-12 in equivalent amount), $\alpha$-MEM, DMEM/F-12 (mixture of DMEM and F-12 in equivalent amount), DMEM, IMDM, F-12, RPMI1640, and a mixed culture medium formed by any combination of the above. The above culture medium optionally further comprises a supplemented substance such as serum (e.g., fetal bovine serum, human serum, goat serum, etc.), serum replacement (e.g., KOSR, etc.), bovine serum albumin (BSA), antibiotic, vitamin, mineral.

In certain embodiments, the culture medium is the second culture medium as defined in any one of embodiments of the second aspect.

The culture of the present invention can be formulated and administered as a pharmaceutical composition. Such pharmaceutical composition can be in any form known in the medical field, preferably injection (including injection solution, lyophilized powder). In certain preferred embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In the sixth aspect, the present invention provides a culture supernatant, which is a supernatant of the culture described in the fifth aspect; alternatively, is a culture supernatant produced by culturing the mesenchymal stem cell population described the first or third aspect in a culture medium.

In certain embodiments, the culture medium is the second culture medium as defined in any one of embodiments of the second aspect.

In certain embodiments, the culture supernatant is free of the mesenchymal stem cell population.

The culture supernatant of the present invention can be formulated and administered as a pharmaceutical composition. Such pharmaceutical composition may be in any form known in the medical field, such as tablet, pill, suspension, emulsion, solution, gel, capsule, powder, granule, elixir, lozenge, suppository, injection (including injection solution, lyophilized powder) and other forms.

In a seventh aspect, the present invention also relates to a method for preparing the culture supernatant as described herein, comprising the following steps:

(1) culturing the mesenchymal stem cell population of the first aspect or the third aspect; and

(2) recovering a supernatant of a culture obtained in the step (1) (i.e., the culture supernatant).

In certain embodiments, the method further comprises: (3) subjecting the supernatant obtained in step (2) to a treatment, wherein the treatment is selected from centrifugation, concentration, solvent replacement, dialysis, freezing, drying, freeze drying, dilution, desalination, preservation, and any combination thereof.

In the present invention, the mesenchymal stem cell population of the present invention can be cultured using any culture medium and culture conditions known in the art that can be used for culturing stem cells. In certain embodiments, the mesenchymal stem cell population is cultured in step (1) using the second culture medium as defined in any one of embodiments of the second aspect.

In certain embodiments, the culture supernatant of the present invention is free of serum to improve safety. Thus, in certain exemplary embodiments, in step (1), the mesenchymal stem cell population may be cultured using a serum-free culture medium (e.g., basal medium or serum-free medium), thereby obtaining a serum-free culture supernatant. In such embodiments, the serum-free culture medium can be used for the entire culture process, or for the culture in the last or last few passages. In certain exemplary embodiments, the culture supernatant obtained in step (2) may be subjected to dialysis or solvent replacement to remove serum, thereby also obtaining a serum-free culture supernatant.

## Microcarrier and cryopreservation method

In an eighth aspect, the present invention also relates to a method of culturing the mesenchymal stem cell population of the first aspect or the third aspect, comprising using a microcarrier. In certain embodiments, the microcarrier includes carrier table (e.g., TableTrix), carrier sphere (e.g., CultiSpher, Coring, Cytodex 1, 2, 3, Solohill, Cytopore, Cytoline), etc., or liquid microcarrier, macroporous gelatin microcarrier, polystyrene microcarrier, PHEMA microcarrier, chitin microcarrier, polyurethane foam microcarrier, alginate gel microcarrier and magnetic microcarrier, etc.

In certain embodiments, the microcarrier of the present invention can improve the survival rate of the mesenchymal stem cell population after cryopreservation. In certain embodiments, the mesenchymal stem cell population and/or the mesenchymal stem cell population cultured on microcarrier can recover a survival rate of at least 50%, 60%, 70%, 80%, 90% in the range of 37°C±3°C, 37°C±2°C, or 37°C±1°C after being cryopreserved for at least 1 day, 2 days, 3 days, 4 days, 5 days, 6 days, 7 days, 8 days, 9 days, 10 days, 1 month, 2 months, 3 months, half year, and one year.

In certain embodiments, the microcarrier comprises, consists of, or consists essentially of a substance selected from the group consisting of protein, cellulose, polyethylene, polystyrene, glass, dextran, diethylaminoethanol(DEAE)-dextran, collagen, collagen-gylcose-aminoglycan, gelatin, acrylamide (e.g., polyacrylamide), and any combination thereof.

In certain embodiments, the microcarrier does not have a matrix coating. In certain embodiments, the surface of the microcarrier is coated with a matrix.

In certain embodiments, the matrix includes an extracellular matrix. In certain embodiments, the matrix comprises one or more selected from the group consisting of Matrigel™ (BD Biosciences), hyaluronic acid, laminin, fibronectin, vitronectin, collagen, elastin, heparan sulfate, dextran, dextran sulfate, chondroitin sulfate.

In certain embodiments, the microcarrier is a non-porous microcarrier. In certain embodiments, the microcarrier is a porous microcarrier.

In certain embodiments, the microcarrier culturing is performed under a static culture condition.

In certain embodiments, the microcarrier culturing is performed under a dynamic culture condition.

In certain embodiments, the culturing is performed by a method comprising: performing the microcarrier culturing of the mesenchymal stem cell population in a culture medium, wherein the growth culture medium is a basal medium comprising the following substances: one or more serum replacements, one or more non-essential amino acids, glutamine or stabilized dipeptide of L-alanyl-L-glutamine, and one or more growth factors. In certain embodiments, the growth culture medium is the second culture medium as described in any one of embodiments of the second aspect.

## Pharmaceutical composition

In a ninth aspect, the present invention provides a pharmaceutical composition, which comprises at least one or more selected from the following: the mesenchymal stem cell population described in the first aspect or the third aspect, the culture described in the fifth aspect, the culture supernatant described in the sixth aspect. In some embodiments, the pharmaceutical composition further comprises a pharmaceutically acceptable carrier or excipient, or other adjuvants that may be required.

Herein, the term "adjuvant" refers to a substance other than the main drug that is necessary in the preparation or formulation of a pharmaceutical preparation. It is generally required that such substance has no physiological activity and does not affect the efficacy, content determination and stability of the drug in the pharmaceutical preparation. The main purpose of adding adjuvant is to facilitate the preparation and clinical application of the preparation. Preferably, the adjuvant used in the pharmaceutical composition of the present invention is pharmaceutically acceptable and also compatible with the active component.

In some embodiments, the pharmaceutical composition comprises a therapeutically effective amount of the mesenchymal stem cell population and/or the culture and/or a therapeutically effective amount of the culture supernatant as described herein.

In some embodiments, the pharmaceutical composition comprises a pharmaceutically acceptable biomaterial, which includes but is not limited to, collagen (e.g., collagen gel, collagen scaffold, gelatin microcarrier), gelatin (e.g.,

gelatin gel, gelatin electrospinning silk, gelatin scaffold, gelatin microcarrier, etc.), aminated gelatin (e.g., aminated gelatin gel, aminated gelatin electrospinning silk, aminated gelatin scaffold, aminated gelatin microcarrier, etc.), chitosan (e.g., chitosan gel, chitosan scaffold, etc.), decellularized scaffold (e.g., uterine decellularized scaffold, heart decellularized scaffold, etc.), skin repair membrane, bone repair membrane, oral repair membrane, cellulose, fibrin, polylactic acid, cellulose polylactic acid, polyurethane, tropoelastin, hyaluronic acid, sodium alginate, polyethylene oxide, polyethylene glycol, polylactic glycolic acid, poly(ε-caprolactone), silicate, silicone rubber, extracellular matrix or any combination thereof, etc. In some embodiments, the pharmaceutical composition comprises a sterile pharmaceutically acceptable isotonic aqueous or non-aqueous solution (e.g., balanced salt solution or normal saline), dispersion, suspension or emulsion.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some preferred embodiments, the pharmaceutical composition is an injection (including injection solution, lyophilized powder) or aerosol.

In some embodiments, the pharmaceutical composition is a spray that can be used for skin resurfacing or transplantation, and the like.

In some embodiments, the pharmaceutical composition can be implanted in the form of a suspension, gel, colloid, serous fluid or mixture.

General principles regarding the formulation of pharmaceutical composition comprising the mesenchymal stem cell population of the present invention may refer to Cell Therapy: stem cell Transplantation, Gene Therapy and Cellular Immunotherapy, edited by G. Morstyn and W. Sheridan, Cambridge University Press, 1996; and Hematopoietic stem cell treatment, E.D. Ball, J. Lister & P.Law, Churchill Livingstone, 2000. In some embodiments, the pharmaceutical composition comprises injection solution (including normal saline, lactated Ringer's solution, compound electrolyte injection solution, 5% glucose injection solution, 20% HSA injection solution, succinyl gelatin injection solution, succinyl gelatin MIX injection solution, MZJ injection solution 1, MZJ injection solution 2, MZJ injection solution 3, human serum protein injection solution, Plasmalyte-A, potassium chloride injection solution, magnesium sulfate injection solution, sodium bicarbonate injection solution, glucose sodium chloride injection solution, compound sodium chloride injection solution (Ringer's solution), dextran-20 glucose injection solution (small molecule), amino acid injection solution, hydroxyethyl starch-40 sodium chloride injection solution, hydroxy ethyl starch-40 sodium chloride injection solution, hydroxyethyl starch-40 sodium chloride injection solution, low-molecular weight heparin calcium for injection, heparin sodium injection solution, coenzyme A for injection, disodium cytidine triphosphate, lysine hydrochloride for injection, vitamin C injection solution, citicoline sodium chloride, fat-soluble vitamin II for injection, reduced glutathione for injection, cerebroprotein hydrolysate for injection, sodium deoxynucleotide injection solution, multi-trace elements injection solution II, mannitol injection solution, arginine hydrochloride injection solution, potassium chloride injection solution, disodium cytidine triphosphate for injection, ornithine aspartate for injection, etc.), and a mixture comprising one or more of the following materials: propylene glycol, sodium bicarbonate, cholesterol, heparin, FBS, culture medium, dimethyl sulfoxide, sodium glycerophosphate solution, hydroxyethyl starch, mannitol solution, ethylene glycol, polyvinyl alcohol, trehalose, polyvinylpyrrolidone, human umbilical cord mesenchymal stem cell-derived exosome solution, human umbilical cord mesenchymal stem cell-derived short peptide or polypeptide compound solution, sodium lactate, mannitol, dextran, potassium chloride, calcium chloride, azone, low-molecular dextran. In some embodiments, the cells can maintain a survival rate of at least 50%, 60%, 70%, 80%, or 90% at about 4°C within 14 days, 13 days, 12 days, 11 days, 10 days, 9 days, 8 days, 7 days, 6 days, 5 days, 4 days, 3 days, 2 days, or 1 day after being non-cryopreserved or cryopreserved in the pharmaceutical composition.

In some embodiments, the pharmaceutical composition comprises a pharmaceutically acceptable biomaterial (for example, but not limited to, collagen scaffold, skin repair membrane, aminated gelatin, chitosan, silk fibroin, cellulose polylactic acid, elastic proteinogen, hyaluronic acid.)

In some embodiments, the pharmaceutical composition comprises a sterile pharmaceutically acceptable isotonic aqueous or non-aqueous solution (e.g., balanced salt solution or normal saline), dispersion, suspension or emulsion.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some preferred embodiments, the pharmaceutical composition is an injection (including injection solution, lyophilized powder) or aerosol.

In some embodiments, the pharmaceutical composition is an injection, such as a solution-type injection.

In some embodiments, the injection comprises one or more additives for injection, which for example, is selected from solubilizer, wetting agent, emulsifier, buffer, suspending agent, chelating agent, antioxidant, bacteriostatic agent, local anesthetic, isotonicity modifier, filler, protective agent and any combination thereof.

In some embodiments, the injection comprises an isotonic or hypertonic solution; preferably, the solution is selected from NaCl injection solution (e.g., 0.9% to 2.7% NaCl injection solution), glucose injection solution (e.g., 4% to 5% glucose injection solution), sodium lactate Ringer's injection solution, compound electrolyte injection solution, HSA injection solution (e.g., 10% to 20% HSA injection solution), succinyl gelatin injection solution (e.g., 4% to 5% succinyl gelatin injection solution) and any combination thereof.

In some embodiments, the mesenchymal stem cells are administered at a dosage of no less than $1 \times 10^4$ cells/ml (e.g., no less than $1 \times 10^4$ cells/ml, no less than $3 \times 10^4$ cells/ml, no less than $5 \times 10^4$ cells/ml, no less than $7 \times 10^4$ cells/ml, no less than $1 \times 10^5$ cells/ml, no less than $3 \times 10^5$ cells/ml, no less than $5 \times 10^5$ cells/ml, no less than $7 \times 10^5$ cells/ml, no less than $1 \times 10^6$ cells/ml, no less than $3 \times 10^6$ cells/ml, no less than $5 \times 10^6$ cells/ml, no less than $7 \times 10^6$ cells/ml, no less than $1 \times 10^7$ cells/ml, no less than $3 \times 10^7$ cells/ml, no less than $5 \times 10^7$ cells/ml, no less than $7 \times 10^7$ cells/ml, no less than $1 \times 10^8$ cells/ml, no less than $3 \times 10^8$ cells/ml, no less than $5 \times 10^8$ cells/ml, no less than $7 \times 10^8$ cells/ml, no less than $1 \times 10^9$ cells/ml, no less than $3 \times 10^9$ cells/ml, no less than $5 \times 10^9$ cells/ml, no less than $7 \times 10^9$ cells/ml, no less than $1 \times 10^{10}$ cells/ml, no less than $3 \times 10^{10}$ cells/ml, no less than $5 \times 10^{10}$ cells/ml or not less $7 \times 10^{10}$ cells/ml, for another example, $1 \times 10^5$ to $1 \times 10^8$, $7 \times 10^5$ to $7 \times 10^6$, $1 \times 10^6$ to $5 \times 10^6$, preferably $1 \times 10^6$ , $3 \times 10^6$ , $5 \times 10^6$ cells/ml, more preferably $3 \times 10^6$ cells/ml).

In some embodiments, the mesenchymal stem cells are administered at a dosage of no less than $1 \times 10^3$ cells/kg (e.g., no less than $1 \times 10^3$ cells/kg, no less than $3 \times 10^3$ cells/kg, less than $5 \times 10^3$ cells/kg, no less than $7 \times 10^3$ cells/kg, no less than $1 \times 10^6$ cells/kg, no less than $3 \times 10^4$ cells/kg, no less than $5 \times 10^4$ cells/kg, less than $7 \times 10^4$ cells/kg, no less than $1 \times 10^5$ cells/kg, no less than $3 \times 10^5$ cells/kg, no less than $5 \times 10^5$ cells/kg, no less than $7 \times 10^5$ cells/kg, no less than $1 \times 10^6$ cells/kg, no less than $3 \times 10^6$ cells/kg, no less than $5 \times 10^6$ cells/kg, no less than $7 \times 10^6$ cells/kg, no less than $1 \times 10^7$ cells/kg, no less than $3 \times 10^7$ cells/kg, no less than $5 \times 10^7$ cells/kg, no less than $7 \times 10^7$ cells/kg, no less than $1 \times 10^8$ cells/kg, no less than $3 \times 10^8$ cells/kg, no less than $5 \times 10^8$ cells/kg, no less than $7 \times 10^8$ cells/kg, no less than $1 \times 10^9$ cells/kg, no less than $3 \times 10^9$ cells/kg, no less than $5 \times 10^9$ cells/kg, no less than $7 \times 10^9$ cells/kg, no less than $1 \times 10^{10}$ cells/kg, no less than $3 \times 10^{10}$ cells/kg, no less than $5 \times 10^{10}$ cells/kg or no less than $7 \times 10^{10}$ cells/kg, for another example $1 \times 10^5$ to $1 \times 10^8$, $7 \times 10^5$ to $7 \times 10^6$, $1 \times 10^6$ to $5 \times 10^6$ cells, preferably $1 \times 10^6$ , $3 \times 10^6$ , $5 \times 10^6$ cells/kg, more preferably $3 \times 10^6$ cells/kg).

In some embodiments, the administration dosage is not less than $1 \times 10^4$ cells/time (e.g., not less than $1 \times 10^6$ cells/time, not less than $3 \times 10^4$ cells/time, not less than $5 \times 10^4$ cells/time, not less than $7 \times 10^4$ cells/time, not less than $1 \times 10^5$ cells/time, not less than $3 \times 10^5$ cells/time, not less than $5 \times 10^5$ cells/time, not less than $7 \times 10^5$ cells/time, not less than $1 \times 10^6$ cells/time, not less than $3 \times 10^6$ cells/time, not less than $5 \times 10^6$ cells/time, not less than $7 \times 10^6$ cells/time, not less than $1 \times 10^7$ cells/time, not less than $3 \times 10^7$ cells/time, not less than $5 \times 10^7$ cells/time, not less than $7 \times 10^7$ cells/time, not less than $1 \times 10^8$ cells/time, not less than $3 \times 10^8$ cells/time, not less than $5 \times 10^8$ cells/time, not less than $7 \times 10^8$ cells/time, not less than $1 \times 10^9$ cells/time, not less than $3 \times 10^9$ cells/time, not less than $5 \times 10^9$ cells/time, not less than $7 \times 10^9$ cells/time, not less than $1 \times 10^{10}$ cells/time, not less than $3 \times 10^{10}$ cells/time, not less than $5 \times 10^{10}$ cells/time or not less than $7 \times 10^{10}$ cells/time), preferably 3 to $6 \times 10^6$ cells/time.

In some embodiments, the injection further comprises the following functional component:

(1) a component that maintains the activity of the mesenchymal stem cells;

(2) a component that promotes the proliferation of the mesenchymal stem cells; and/or,

(3) a component that promotes the differentiation of the mesenchymal stem cells.

In some embodiments, the functional component is selected from the group consisting of serum replacement, non-essential amino acid, glutamine, stabilized dipeptide of L-alanyl-L-glutamine, growth factor, and any combination thereof.

In some embodiments, the functional component is selected from the group consisting of KOSR, MSC serum-free Supplement, Ultraser™ G, glycine, L-alanine, L-asparagine, L-aspartic acid, L-glutamic acid, L-proline, L-serine, VEGF, bFGF, EGF, TGFβ, PDGF, and any combination thereof.

In some embodiments, the pharmaceutical composition is a spray that can be used for skin resurfacing or transplantation, and the like.

In some embodiments, the pharmaceutical composition can be implanted in the form of a suspension, gel, colloid, serous fluid or mixture.

General principles regarding the formulation of pharmaceutical composition comprising the mesenchymal stem cell population of the present invention may refer to Cell Therapy: stem cell Transplantation, Gene Therapy and Cellular Immunotherapy, edited by G. Morstyn and W. Sheridan, Cambridge University Press, 1996; and Hematopoietic stem cell treatment, E.D. Ball, J. Lister & P.Law, Churchill Livingstone, 2000. In some embodiments, the pharmaceutical composition comprises injection solution (including normal saline, lactated Ringer's solution, compound electrolyte injection solution, 5% glucose injection solution, 20% HSA injection solution, succinyl gelatin injection solution, succinyl gelatin MIX injection solution, MZJ injection solution 1, MZJ injection solution 2, MZJ injection solution 3, human serum protein injection solution, Plasmalyte-A, potassium chloride injection solution, magnesium sulfate injection solution, sodium bicarbonate injection solution, glucose sodium chloride injection solution, compound sodium chloride injection solution (Ringer's solution), dextran-20 glucose injection solution (small molecule), amino acid injection solution, hydroxyethyl starch-40 sodium chloride injection solution, hydroxyethyl starch-40 sodium chloride injection solution, hydroxyethyl starch-40 sodium chloride injection solution, low-molecular weight heparin calcium for injection, heparin sodium injection solution, coenzyme A for injection, disodium cytidine triphosphate, lysine hydrochloride for injection, vitamin C injection solution, citicoline sodium chloride, fat-soluble vitamin II for injection, reduced glutathione for injection, cerebroprotein hydrolysate for injection, sodium deoxynucleotide injection solution, multi-trace elements injection solution II, mannitol injection solution, arginine hydrochloride injection solution, potassium chloride injection solution, disodium cytidine triphosphate for injection, ornithine aspartate for injection, etc.), and a mixture comprising one or more of the following materials: propylene glycol, sodium bicarbonate, cholesterol, heparin, FBS, culture medium, dimethyl sulfoxide, sodium glycerophosphate solution, hydroxyethyl starch, mannitol solution, ethylene glycol, polyvinyl alcohol, trehalose, polyvinylpyrrolidone, human umbilical cord mesenchymal stem cell-derived exosome solution, human umbilical cord mesenchymal stem cell-derived short peptide or polypeptide compound solution, sodium lactate, mannitol, dextran, potassium chloride, calcium chloride, azone, low-molecular dextran. In some embodiments, the cells can maintain a survival rate of at least 50%, 60%, 70%, 80%, or 90% at about 4°C within 14 days, 13 days, 12 days, 11 days, 10 days, 9 days, 8 days, 7 days, 6 days, 5 days, 4 days, 3 days, 2 days, or 1 day after being non-cryopreserved or cryopreserved in the pharmaceutical composition.

In some embodiments, the pharmaceutical composition comprises a pharmaceutically acceptable biomaterial (for example, but not limited to, collagen scaffold, skin repair membrane, aminated gelatin, chitosan, silk fibroin, cellulose polylactic acid, elastic proteinogen, hyaluronic acid.)


**Bioscaffold**

In one aspect, the present invention provides an article, which comprises a mesenchymal stem cell population and a bioscaffold, wherein the mesenchymal stem cell population has an average MMP1 expression level of at least about 10 times that of primary mesenchymal stem cells; and/or, the mesenchymal stem cell population has an average PGE2 expression level of at least about 10 times that of primary mesenchymal stem cells.

In another aspect, the present invention provides an article, which comprises a mesenchymal stem cell population and a bioscaffold, wherein the mesenchymal stem cell population has an average MMP1 expression level of at least about 10 times that of primary mesenchymal stem cells; optionally, the mesenchymal stem cell population has an average PGE2 expression level of at least about 10 times that of primary mesenchymal stem cells.

In certain embodiments, the mesenchymal stem cell population is generated in vitro.

In certain embodiments, the mesenchymal stem cell population is as above defined or described.

In certain embodiments, the mesenchymal stem cell population is partially or fully loaded on the bioscaffold.

In certain embodiments, the bioscaffold is prepared with a degradable or non-degradable material.

In certain embodiments, the bioscaffold is prepared with a material that is naturally occurring, artificially synthesized, recombinantly produced, modified, or any combination thereof.

In certain embodiments, the naturally occurring material is selected from the group consisting of collagen (e.g., type I, type II, type III collagen), fibrin, silk protein, cellulose, chitosan, alginate (e.g., sodium alginate), starch, hyaluronic acid, laminin, elastin, agarose, gelatin, dextran, extracellular matrix, silicate, or any combination thereof.

In certain embodiments, the artificially synthesized material is selected from the group consisting of polyphosphazene, polyacrylic acid and derivative thereof (e.g., polymethacrylic acid, copolymer of acrylic acid and methacrylic acid), polylactic acid (PLA), polyglycolic acid (PGA), copolymer of polylactic acid-glycolic acid (PLGA), polyorthoester (POE), polycaprolactone (PCL), polyhydroxybutyrate (PHB), polyamino acid (e.g., polylysine), polyurethane, polyethylene oxide, polyethylene glycol, polylactic-glycolic acid, silicone rubber, decellularized scaffold or any combination thereof.

In certain embodiments, the modified material is selected from the group consisting of modified alginate, modified gelatin, or a combination thereof, preferably, the modified alginate is oxidized alginate (e.g., oxidized sodium alginate), preferably, the modified gelatin is aminated gelatin.

In certain embodiments, the material used to prepare the bioscaffold is selected from the group consisting of collagen, aminated gelatin, chitosan, or any combination thereof.

In certain embodiments, the bioscaffold is a solid or semi-solid (e.g., a gel).

In certain embodiments, the bioscaffold has a layered structure (e.g., monolayer, bilayer, or multilayer, e.g., biofilm, skin repair membrane), or a sheet-like structure (e.g., rectangle, square, circle, oval, hexagonal or irregularly shaped sheet-like structure), or hollow tubular structure, or hollow annular (e.g., circular ring-like) structure, or hollow three-dimensional structure (e.g., hollow cube, hollow sphere, hollow rectangular prism, hollow cylinder, or hollow irregularly shaped three-dimensional structure), or solid three-dimensional structure (e.g., solid cube, solid sphere, solid rectangular prism, solid cylinder, or solid irregularly shaped three-dimensional structure), or any combination thereof.

In certain embodiments, the bioscaffold mimics a shape of a native tissue or organ.

In certain embodiments, the bioscaffold has a sheet-like structure, layered structure, or hollow annular (e.g., circular ring-like) structure.

In certain embodiments, the bioscaffold is selected from the group consisting of collagen scaffold, skin repair membrane, gelatin scaffold, aminated gelatin scaffold, chitosan scaffold.

In certain embodiments, the bioscaffold is loaded with the mesenchymal stem cells in an amount of no less than $1\times10^4$/ml (e.g., no less than $1\times10^4$ /ml, no less than $3\times10^4$/ml, no less than $5\times10^4$/ml, no less than $7\times10^4$/ml, no less than $1\times10^5$/ml, no less than $3\times10^5$/ml, no less than $5\times10^5$/ml, no less than $7\times10^5$/ml, no less than $1\times10^6$/ml, no less than $3\times10^6$/ml, no less than $5\times10^6$/ml, no less than $7\times10^6$/ml, no less than $1\times10^7$/ml, no less than $3\times10^7$/ml, no less than $5\times10^7$/ml, no less than $7\times10^7$/ml, no less than $1\times10^8$/ml, no less than $3\times10^8$/ml, no less than $5\times10^8$/ml, no less than $7\times10^8$/ml, no less than$1\times10^9$/ml, no less than $3\times10^9$/ml, no less than $5\times10^9$/ml, no less than $7\times10^9$/ml, no less than $1\times10^{10}$/ml, no less than $3\times10^{10}$/ml, no less than $5\times10^{10}$/ml or no less than $7\times10^{10}$/ml).

In certain embodiments, the article further comprises an additional active component.

In certain embodiments, the additional active component is partially or fully loaded on the bioscaffold.

In certain embodiments, the additional active component is selected from the group consisting of drug for treating osteoarthropathy (e.g., meniscus injury, bone injury), drug for treating cardiac disease (e.g., myocardial infarction), drug for treating nervous system disease (e.g., spinal cord injury), drug for treating skin disease (e.g., skin injury, burn, scald), drug for treating eye disease (e.g., corneal alkali burn), or any combination thereof.

In certain embodiments, the drug for treating osteoarthropathy is selected from glucosamine sulfate capsule, aminated chondroitin sulfate, sodium hyaluronate injection, anti-hyperostosis tablet, ossotide tablet, Henggu bone wound healing agent, Gentongping granules, non-steroidal anti-inflammatory drug (e.g., loxoprofen sodium tablet, diclofenac sodium sustained-release tablets, celecoxib, meloxicam, indomethacin tablet, Voltaren ointment), or any combination thereof.

In certain embodiments, the drug for treating cardiac disease is selected from the group consisting of aspirin, clopidogrel, ticagrelor, ACE1, ARBs, β-blocker, calcium antagonist, nitrate vasodilator, trimetazidine hydrochloride, nicorandil, lidocaine, amiodarone, quinidine, or any combination thereof.

In certain embodiments, the drug for treating nervous system disease is selected from the group consisting of carbamazepine, phenobarbital, phenytoin, sodium valproate, clonazepam, lamotrigine, oxcarbazepine, donepezil, memantine, vitamin B1, Vaccinia vaccination of rabbits inflammation of the skin extract, alteplase, aspirin, clopidogrel, low-molecular weight heparin, edaravone, urinary kallidinogenase, butylphthalide, gamma-globulin for injection, pyridostigmine, glucocorticoids, or any combination thereof.

In certain embodiments, the drug for treating skin disease is selected from the group consisting of ebastine tablet, loratadine tablet, cetirizine tablet, mometasone furoate ointment, halometasone ointment, mupirocin ointment, fusidic acid ointment, cefixime tablet, roxithromycin tablet, naftifine ketoconazole ointment, sertaconazole ointment, itraconazole tablet, terbinafine tablet, acyclovir tablet, valaciclovir tablet, penciclovir ointment, interferon gel, or any combination thereof.

In certain embodiments, the drug for treating eye disease is an antibacterial and anti-inflammatory drug.

In certain embodiments, the article further comprises a component for culturing/differentiating cells.

In certain embodiments, the component for culturing/differentiating cells is selected from the group consisting of serum replacement, non-essential amino acid, stabilized dipeptide of glutamine or L-alanyl-L-glutamine, growth factor, or any combination thereof.

In certain embodiments, the serum replacement is selected from the group consisting of KOSR, MSC serum-free Supplement, Ultroser™ G, or any combination thereof.

In certain embodiments, the non-essential amino acid is selected from the group consisting of glycine, L-alanine, L-asparagine, L-aspartic acid, L-glutamic acid, L-proline, L-serine, or any combination thereof.

In certain embodiments, the growth factor is selected from the group consisting of VEGF, bFGF, EGF, TGFβ, PDGF, or any combination thereof.

In another aspect, the present invention provides use of the aforementioned article in the manufacture of a medicament for the treatment and/or prevention of osteoarthropathy (e.g., meniscus injury, bone injury), cardiac disease (e.g., myocardial infarction), nervous system disease (e.g., spinal cord injury), skin disease (e.g., skin injury, burn, scald), eye disease (e.g., corneal alkali burn), or any combination thereof, in a subject.

In certain embodiments, the medicament is used for the treatment and/or prevention of spinal cord injury, skin injury, corneal alkali burn, or any combination thereof, in a subject.

In another aspect, the present invention provides a method for treating and/or preventing a disease in a subject, which comprises administering (e.g., implanting or adhering, preferably implanting) the aforementioned article to a subject in need thereof, and the disease is selected from the group consisting of osteoarthropathy (e.g., meniscus injury, bone injury), cardiac disease (e.g., myocardial infarction), nervous system disease (e.g., spinal cord injury), skin disease (e.g.,

skin injury, burn, scald), eye disease (e.g., corneal alkali burn), or any combination thereof.

In certain embodiments, the disease is selected from the group consisting of spinal cord injury, skin injury, corneal alkali burn, or any combination thereof.

## Use for the prevention and/or treatment of disease

In a tenth aspect, the present invention relates to a use of the mesenchymal stem cell population, the culture, the culture supernatant or the pharmaceutical composition as described herein for the prevention and/or treatment of a disease in a subject, or a use for the manufacture of a medicament for the prevention and/or treatment of a disease in a subject, and a method for preventing and/or treating a disease in a subject, which comprises administering to a subject in need thereof the mesenchymal stem cell population, culture, culture supernatant of the present invention, or the pharmaceutical composition of the present invention.

In certain embodiments, the subject is a mammal, such as a human.

In certain embodiments, the disease is selected from the group consisting of osteoarthropathy (e.g., meniscus injury, osteoarthritis, or bone injury, etc.), reproductive system disease (e.g., ovarian aging, ovarian insufficiency, endometrial damage, uterine trauma, intrauterine adhesion, or thin uterus, etc.), heart disease (e.g., myocardial infarction, etc.), lung disease (e.g., idiopathic pulmonary fibrosis, acute respiratory distress disorder, pneumoconiosis, or pneumonia, etc.), skin disease (e.g., psoriasis, skin lesion, bedsore, pressure ulcer, or burn, etc.), eye disease (e.g., corneal damage, etc.), nervous system disease (e.g., spinal cord injury, cerebral palsy, stroke, Alzheimer's disease, senile dementia, or neuropathic pain, etc.), digestive system disease (e.g., inflammatory bowel disease, colitis, Crohn's disease, or irritable bowel syndrome, etc.), kidney disease (e.g., anti-glomerular basement membrane disease, diabetic nephropathy, lupus nephritis, or acute nephritis, etc.), liver disease (liver injury, liver fibrosis, hepatitis, liver cirrhosis, or liver failure, etc.), autoimmune disease (e.g., scleroderma, lupus erythematosus, or multiple sclerosis, etc.), transplant rejection (e.g., graft-versus-host disease, etc.), metabolic disease (e.g., diabetes, etc.).

In the present invention, the mesenchymal stem cell population or the culture as described herein, or the pharmaceutical composition comprising the mesenchymal stem cell population or the culture, can be administered to a subject by various suitable means. In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain embodiments, the pharmaceutical composition comprises a therapeutically effective amount of the culture supernatant as described herein. The culture supernatant of the present invention can be formulated and administered as a drug. Such pharmaceutical composition may comprise a therapeutically effective amount of the culture supernatant.

In the present invention, the culture supernatant of the present invention, or the pharmaceutical composition comprising the culture supernatant, can be administered to a subject in various suitable ways. In certain preferred embodiments, the culture supernatant of the present invention, or the pharmaceutical composition comprising the culture supernatant, can be administered by intradermal injection, subcutaneous injection, intramuscular injection, intravenous injection, oral administration, and the like.

## Female reproductive system disease

In one aspect, the present invention provides a use of the mesenchymal stem cell population or its culture supernatant in the manufacture of a medicament for the prevention, treatment, delay and/or alleviation of a female reproductive system disease; alternatively, the present invention provides a method for preventing, treating, delaying and/or alleviating a disease of a female reproductive system disease, which comprises administering a prophylactically and/or therapeutically effective amount of the mesenchymal stem cell population or its culture supernatant to a subject in need thereof.

In certain embodiments, the female reproductive system disease includes: (1) gynecological inflammation, such as vulvitis, vaginitis, cervicitis, endometritis, uterine inflammation, as well as pelvic inflammation, adnexitis; (2) gynecological tumor, such as benign tumor and malignant tumor; (3) menstrual disorder, such as dysmenorrhea, increased menstrual flow, decreased menstrual flow; (4) infertility, such as infertility caused by ovulatory dysfunction, infertility caused by blocked fallopian tubes, immune infertility. In certain embodiments, the female reproductive system disease is selected from the group consisting of ovarian aging, ovarian insufficiency, endometrial damage, uterine trauma, intrauterine adhesion, thin uterus.

In certain embodiments, the endometrial damage is primarily a damage of endometrial basal layer, manifested by irregular menstrual cycles, or low overall menstrual bleeding and shortened menstrual bleeding duration. Generally speaking, endometrial damage mostly occurs after abortion, including induced abortion or medical abortion. Moreover, the related intrauterine operations may also cause endometrial damage.

In certain embodiments, endometritis is an inflammation of endometrium. According to the duration of disease, it can be divided into acute endometritis and chronic endometritis.

In certain embodiments, "intrauterine adhesion" is a kind of uterine disease that belongs to the female reproductive system diseases, and refer to an endometrial damage caused by various causes, including gestational and non-gestational uterine trauma, which result in endometrial basal layer damage, so that the uterine cavity and (or) cervical canal are partially or completely occluded, and the uterine walls are adhered to each other, resulting in abnormal menstruation, infertility or repeated miscarriage, etc., and it usually has no typical symptoms. Its nature is endometrial fibrosis. Examples include: endometrial lesion, intrauterine foreign body, benign endometrial lesion, uterine malformation, malignant endometrial lesion, etc.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the additional active component is selected from the group consisting of estrogen, antiestrogen or selective estrogen receptor modulator, androgen, antiandrogen, or progestin.

In certain embodiments, the medicament is in an unit dosage form, and the unit dose of the medicament contains the mesenchymal stem cells in an amount of no less than $1\times10^6$ cells/ml (e.g., no less than $1\times10^6$ cells/ml, no less than $3\times10^4$ cells/ml, no less than $5\times10^4$ cells/ml, no less than $7\times10^4$ cells/ml, no less than $1\times10^5$ cells/ml, no less than $3\times10^5$ cells/ml, no less than $5\times10^5$ cells/ml, no less than $7\times10^5$ cells/ml, no less than $1\times10^6$ cells/ml, no less than $3\times10^6$ cells/ml, no less than $5\times10^6$ cells/ml, no less than $7\times10^6$ cells/ml, no less than $1\times10^7$ cells/ml, no less than $3\times10^7$ cells/ml, no less than $5\times10^7$ cells/ml, no less than $7\times10^7$ cells/ml, no less than $1\times10^8$ cells/ml, no less than $3\times10^8$ cells/ml, no less than $5\times10^4$ cells/ml, no less than $7\times10^8$ cells/ml, no less than $1\times10^6$ cells/ml, no less than $3\times10^9$ cells/ml, no less than $5\times10^4$ cells/ml, no less than $7\times10^9$ cells/ml, no less than $1\times10^{10}$ cells/ml, no less than $3\times10^{10}$ cells/ml, no less than $5\times10^{10}$ cells/ml or no less than $7\times10^{10}$ cells/ml). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

In another aspect, the present invention also provides a product for preventing, treating, delaying and/or alleviating a female reproductive system disease, which comprises the mesenchymal stem cell population of the present invention. In certain embodiments, the product further comprises an additional active component as defined above. In certain embodiments, the mesenchymal stem cell population and the additional active component are present as separate components or as a single formulation. In certain embodiments, the product is an injection, microinjection, mucosal patch, enema, suppository, gel, oral, aerosol, drop, ointment, implant or capsule, preferably is injection. In certain embodiments, the product is an implant.

Intrauterine adhesion

Intrauterine adhesion (including endometrial fibrosis, partial or total occlusion of uterine cavity caused by endometrial damage, which subsequently induce oligomenorrhea, amenorrhea, infertility or recurrent miscarriage, etc.). In recent years, due to the frequent operation of uterine cavity and the popularization of hysteroscopic surgery, the incidence and detection rate of intrauterine adhesion have gradually increased, and the age of onset has become younger, and it has become the second leading cause of female secondary infertility. Although clinicians continue to seek new treatment options, its cure rate and pregnancy rate have not improved significantly, and its recurrence rate is high (the recurrence rate of mild patients after treatment is high, and the recurrence rate of severe patients after treatment is even higher), which result in obstetric complications such as infertility, recurrent miscarriage, premature birth, placenta previa, placenta adhesion or implantation that are serious threats to women's reproductive health. Its high incidence and the resulting damage to women's reproductive function have become an urgent clinical problem to be solved. The current clinical treatment aims to restore the shape of uterine cavity, prevent the recurrence of adhesion, promote the repair and regeneration of the damaged endometrium, and restore the normal reproductive function. The treatment steps comprise hysteroscopic separation of intrauterine adhesion, intraoperative placement of intrauterine device and postoperative administration of estrogen and progesterone, but there are problems such as long treatment cycle, low cure rate, easy recurrence of adhesion, low pregnancy rate, high-dose estrogen application that increases the risk of breast and endometrial tumors in patients, and in severe muscular or connective tissue adhesions, the endometrial basal layer has been destroyed and may not respond well to estrogen and progesterone.

So far, scholars at home and abroad have carried out a lot of research on the pathogenesis of the disease, and it is agreed that the disorder of endometrial repair may be the main mechanism of formation. For example, after abortion or other uterine cavity operations, due to some pathological factors, endometrial repair is hindered, resulting in scar formation and adhesion.

In one aspect, the present invention provides a use of the mesenchymal stem cell population or its culture supernatant in the manufacture of a medicament for preventing, treating, delaying and/or reducing intrauterine adhesion; alternatively, the present invention provides a method for preventing, treating, delaying and/or reducing intrauterine adhesion, which comprises administering to a subject in need thereof a prophylactically and/or therapeutically effective amount of the mesenchymal stem cell population or its culture supernatant.

In certain embodiments, the mesenchymal stem cell population of the present invention are capable of thickening endometrium, increasing blood vessel, gland and cell, improving intrauterine adhesion, restoring uterine cavity shape, and/or reducing uterine effusion.

In certain embodiments, the mesenchymal stem cell population of the present invention are capable of preventing or treating a symptom of uterine adhesion, such as reducing the accumulation of uterine effusion.

In certain embodiments, the intrauterine adhesion includes an uterine adhesion of a sterility patient or an uterine adhesion caused by abortion, etc.

In certain embodiments, the mesenchymal stem cell population of the present invention are capable of improving uterine morphology and inhibiting intrauterine adhesion.

In certain embodiments, the mesenchymal stem cell population of the present invention are capable of promoting endometrial repair and regeneration, such as repair and regeneration of damaged endometrium, so as to enhance the ability to reproduce offspring.

In certain embodiments, the mesenchymal stem cell population of the present invention are capable of restoring an uterine scar, so as to prevent or treat intrauterine adhesion.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the mesenchymal stem cell population of the present invention can be administrated by intradermal injection, subcutaneous injection, intramuscular injection, intravenous injection, oral administration, etc.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the medicament comprises a sterile pharmaceutically acceptable isotonic aqueous or non-aqueous solution (e.g., balanced salt solution or normal saline), dispersion, suspension or emulsion.

In certain embodiments, the pharmaceutical composition comprises a pharmaceutically acceptable biomaterial, including but not limited to, collagen scaffold, skin repair membrane, aminated gelatin, chitosan, silk fibroin, cellulose polylactic acid, elastic proteinogen, hyaluronic acid, etc.

In certain embodiments, the mesenchymal stem cell population can be transplanted in the form of a suspension, gel, colloid, slurry or mixture.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the additional active component is selected from the group consisting of estrogen, antiestrogen or selective estrogen receptor modulator, androgen, antiandrogen, or progestogen.

In certain embodiments, the medicament is in an unit dosage form, and the unit dose of the medicament contains the mesenchymal stem cells in an amount of no less than $1 \times 10^6$ cells/ml (e.g., no less than $1 \times 10^6$ cells/ml, no less than $3 \times 10^4$ cells/ml, no less than $5 \times 10^4$ cells/ml, no less than $7 \times 10^4$ cells/ml, no less than $1 \times 10^5$ cells/ml, no less than

$3\times10^5$ cells/ml, no less than $5\times10^5$ cells/ml, no less than $7\times10^5$ cells/ml, no less than $1\times10^6$ cells/ml, no less than $3\times10^6$ cells/ml, no less than $5\times10^6$ cells/ml, no less than $7\times10^6$ cells/ml, no less than $1\times10^7$ cells/ml, no less than $3\times10^7$ cells/ml, no less than $5\times10^7$ cells/ml, no less than $7\times10^7$ cells/ml, no less than $1\times10^8$ cells/ml, no less than $3\times10^8$ cells/ml, no less than $5\times10^4$ cells/ml, no less than $7\times10^8$ cells/ml, no less than $1\times10^6$ cells/ml, no less than $3\times10^9$ cells/ml, no less than $5\times10^4$ cells/ml, no less than $7\times10^9$ cells/ml, no less than $1\times10^{10}$ cells/ml, no less than $3\times10^{10}$ cells/ml, no less than $5\times10^{10}$ cells/ml or no less than $7\times10^{10}$ cells/ml). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

Primary ovarian insufficiency

Primary ovarian insufficiency (POI) refers to the loss of ovarian function in women before the age of 40. In the 2015 ESHER guidelines, it is defined as: (1) amenorrhea/oligomenorrhea for at least 4 months; (2) 2 times blood FSH>25U/L (monitoring time interval is at least 4 weeks). It is characterized by menstrual disorders (amenorrhea or oligomenorrhea), elevated gonadotropin, and low estrogen levels (hot flashes, sweating, facial flushing, low libido, etc.). The incidence of POI is about 1%, and the incidence varies slightly among different ethnic groups. The incidence of POI in patients with primary amenorrhea is 10% to 28%, and the incidence of POI in patients with secondary amenorrhea is 4% to 18%.

The causes of POI include genetic, immune, iatrogenic (radiotherapy, chemotherapy, immunosuppressive therapy, and surgical treatment, etc.) and other causes, but most POI causes are unknown. POI may be associated with a variety of endocrine disorders, including hypoparathyroidism and hypoadrenalism. Pelvic surgery may also lead to impaired ovarian function. Adrenal or ovarian antibodies are present in approximately 4% of patients with POI, suggesting that the disease is autoimmune. In many cases, the mechanism is unclear. POI may cause loss of female fertility and increase the risk of osteoporosis, lipid metabolism disorder, and cardiovascular disease. Early amenorrhea and loss of fertility during the reproductive period will increase the psychological burden of women and reduce the quality of married life, resulting in a series of serious psychological and social problems.

In one aspect, the present invention provides a use of the mesenchymal stem cell population or its culture supernatant in the manufacture of a medicament for preventing, treating, delaying and/or alleviating ovarian insufficiency (e.g., primary ovarian insufficiency); alternatively, the present invention provides a method for preventing, treating, delaying and/or alleviating ovarian insufficiency (e.g., primary ovarian insufficiency), which comprises administering to a subject in need thereof a prophylactically and/or therapeutically effective amount of the mesenchymal stem cell population or its culture supernatant.

In certain embodiments, the mesenchymal stem cell population of the present invention can improve a blood sex hormone (e.g., FSH and E2) level, increase follicle number, improve body weight and ovarian weight, restore ovulation level, and/or improve fertility level.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the mesenchymal stem cell population of the present invention can be administrated by intradermal injection, subcutaneous injection, intramuscular injection, intravenous injection, oral administration, etc. In certain embodiments, the administration is performed by abdominal or intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, skin repair membrane, aminated gelatin, chitosan, silk fibroin, cellulose polylactic acid, elastic proteinogen, hyaluronic acid, etc.

In certain embodiments, the mesenchymal stem cell population can be transplanted in the form of a suspension, gel, colloid, slurry or mixture.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the additional active component is selected from the group consisting of estrogen, antiestrogen or selective estrogen receptor modulator, androgen, antiandrogen, or progestogen.

In certain embodiments, the medicament is in an unit dosage form, and the unit dose of the medicament contains the mesenchymal stem cells in an amount of no less than $1 \times 10^6$ cells/ml (e.g., no less than $1 \times 10^6$ cells/ml, no less than $3 \times 10^4$ cells/ml, no less than $5 \times 10^4$ cells/ml, no less than $7 \times 10^4$ cells/ml, no less than $1 \times 10^5$ cells/ml, no less than $3 \times 10^5$ cells/ml, no less than $5 \times 10^5$ cells/ml, no less than $7 \times 10^5$ cells/ml, no less than $1 \times 10^6$ cells/ml, no less than $3 \times 10^6$ cells/ml, no less than $5 \times 10^6$ cells/ml, no less than $7 \times 10^6$ cells/ml, no less than $1 \times 10^7$ cells/ml, no less than $3 \times 10^7$ cells/ml, no less than $5 \times 10^7$ cells/ml, no less than $7 \times 10^7$ cells/ml, no less than $1 \times 10^8$ cells/ml, no less than $3 \times 10^8$ cells/ml, no less than $5 \times 10^4$ cells/ml, no less than $7 \times 10^8$ cells/ml, no less than $1 \times 10^6$ cells/ml, no less than $3 \times 10^9$ cells/ml, no less than $5 \times 10^4$ cells/ml, no less than $7 \times 10^9$ cells/ml, no less than $1 \times 10^{10}$ cells/ml, no less than $3 \times 10^{10}$ cells/ml, no less than $5 \times 10^{10}$ cells/ml or no less than $7 \times 10^{10}$ cells/ml). In certain embodiments, the unit dose of the medicament contains the mesenchymal stem cells in an amount of $1 \times 10^4$ to $1 \times 10^{10}$ (e.g., $1 \times 10^6$ to $1 \times 10^8$, $1 \times 10^6$ to $1 \times 10^7$, or $1 \times 10^6$ to $5 \times 10^6$).

## Male reproductive system disease

Male reproductive system disease comprises abnormal urination, pyuria, abnormal urethral discharge, pain, mass, sexual dysfunction and male infertility related to urological diseases, and mainly includes urinary system inflammation, such as cystitis, urethritis, urinary incontinence, urinary retention, etc.; reproductive system inflammation, such as orchiepididymitis, seminal vesiculitis, prostatitis, etc.; reproductive tract tuberculosis, such as testicular epididymal tuberculosis, seminal vesicle tuberculosis, etc.; reproductive system tract injury, such as testicular contusion, penis fracture, urethral rupture, etc.; male infertility disease, such as varicocele, asthenozoospermia, congenital vas deferens obstruction, absence of vas deferens, etc.; male sexual dysfunction disease, such as male erectile dysfunction, premature ejaculation, hypaphrodisia, non-ejaculation, delayed ejaculation, etc. In this article, the term "male infertility" refers to infertility caused by male factors, generally, the woman is not pregnant after marriage in which no contraceptive measures are taken for more than 2 years of cohabitation. Male infertility includes testicular atrophy, testicular hypoplasia, oligospermia, spermatogenic failure, azoospermia, obstructive azoospermia, asthenozoospermia, Klinefelter's syndrome, XYY syndrome, Kallmann's syndrome, selective LH deficiency and FSH deficiency, adrenal cortical hyperplasia, hyperprolactinemia, varicocele, sperm deformity, etc. As used herein, the term "oligospermia" refers to a lower number of sperm in the semen than that of normal healthy fertile men, including oligospermia caused by endocrine dysfunction, reproductive system infection, varicocele, antisperm antibody, cryptorchidism, hydrocele, malnutrition, chemotherapy, radiotherapy, obesity, etc.

In one aspect, the present invention provides a use of the mesenchymal stem cell population or its culture supernatant in the manufacture of a medicament for preventing, treating, delaying and/or alleviating a male reproductive system disease; alternatively, the present invention provides a method for preventing, treating, delaying and/or alleviating a male reproductive system disease, which comprises administering to a subject in need thereof a prophylactically and/or therapeutically effective amount of the mesenchymal stem cell population or its culture supernatant.

In certain embodiments, the male reproductive system disease is azoospermia or oligospermia. In certain embodiments, the mesenchymal stem cell population of the present invention can be used in azoospermia to increase sperm concentration, increase sperm motility, restore testis and/or restore seminal vesicle function.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the mesenchymal stem cell population of the present invention can be administrated by intradermal injection, subcutaneous injection, intramuscular injection, intravenous injection, oral administration, etc. In certain embodiments, the administration is performed by intratesticular or seminiferous tubule or intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, skin repair membrane, aminated gelatin, chitosan, silk fibroin, cellulose polylactic acid, elastic proteinogen, hyaluronic acid, etc.

In certain embodiments, the mesenchymal stem cell population can be transplanted in the form of a suspension, gel, colloid, slurry or mixture.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the additional active component is selected from the group consisting of estrogen, antiestrogen or selective estrogen receptor modulator, androgen, antiandrogen.

In certain embodiments, the medicament is in an unit dosage form, and the unit dose of the medicament contains the mesenchymal stem cells in an amount of no less than $1\times10^6$ cells/ml (e.g., no less than $1\times10^6$ cells/ml, no less than $3\times10^4$ cells/ml, no less than $5\times10^4$ cells/ml, no less than $7\times10^4$ cells/ml, no less than $1\times10^5$ cells/ml, no less than $3\times10^5$ cells/ml, no less than $5\times10^5$ cells/ml, no less than $7\times10^5$ cells/ml, no less than $1\times10^6$ cells/ml, no less than $3\times10^6$ cells/ml, no less than $5\times10^6$ cells/ml, no less than $7\times10^6$ cells/ml, no less than $1\times10^7$ cells/ml, no less than $3\times10^7$ cells/ml, no less than $5\times10^7$ cells/ml, no less than $7\times10^7$ cells/ml, no less than $1\times10^s$ cells/ml, no less than $3\times10^8$ cells/ml, no less than $5\times10^4$ cells/ml, no less than $7\times10^8$ cells/ml, no less than $1\times10^6$ cells/ml, no less than $3\times10^9$ cells/ml, no less than $5\times10^4$ cells/ml, no less than $7\times10^9$ cells/ml, no less than $1\times10^{10}$ cells/ml, no less than $3\times10^{10}$ cells/ml, no less than $5\times10^{10}$ cells/ml or no less than $7\times10^{10}$ cells/ml). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

In another aspect, the present invention also provides a product for preventing, treating, delaying and/or alleviating a male reproductive system disease, which comprises the mesenchymal stem cell population of the present invention. In certain embodiments, the product further comprises an additional active component, and the additional active component is as defined above. In certain embodiments, the mesenchymal stem cell population and the additional active component are present as separate components or as a single formulation. In certain embodiments, the product is an injection, microinjection, mucosal patch, enema, suppository, gel, oral preparation, aerosol, drop, ointment, implant, or capsule. In certain embodiments, the product is an implant.

## Digestive system disease

In one aspect, the present invention provides use of the mesenchymal stem cell population or its culture supernatant in manufacture of a medicament, wherein the medicament is used for preventing and/or treating digestive system disease; alternatively, the present invention provides a method for preventing and/or treating digestive system disease, comprising administering to a subject in need thereof a prophylactically and/or therapeutically effective amount of the mesenchymal stem cell population or its culture supernatant.

In certain embodiments, the digestive system disease is selected from the group consisting of esophageal disease, gastric disease, intestinal disease, liver disease, gallbladder disease, pancreatic disease, or any combination thereof.

The intestinal disease is selected from the group consisting of duodenal ulcer, functional colon disease, intestinal polyps, colon cancer, rectal cancer, inflammatory bowel disease (IBD), colitis, proctitis, irritable bowel syndrome, dyspepsia, functional constipation, gastroesophageal reflux disease, esophagitis, peritonitis, autoimmune liver disease, gastritis, tuberculous bowel disease, or any combination thereof.

In certain embodiments, the intestinal disease is an intestinal inflammatory disease.

In certain embodiments, the intestinal inflammatory disease is selected from the group consisting of inflammatory bowel disease (IBD), colitis, proctitis, or any combination thereof.

In certain embodiments, the intestinal inflammatory disease is inflammatory bowel disease (IBD).

The gastrointestinal disease (stomach disease, intestinal disease) mainly refers to general inflammatory gastrointestinal disease (acute or chronic gastritis, acute or chronic appendicitis, etc.), peptic ulcer, gastric cancer, esophageal cancer, colorectal cancer and irritable bowel syndrome, etc. The inflammatory bowel disease is a disease in the gastrointestinal tract.

The liver disease is a general term for all diseases that occur in the liver. According to the different causes of liver injury, it is divided into viral liver disease, including hepatitis A, B, C, E, etc., as well as abnormal metabolism liver disease, alcoholic liver disease, drug-induced or toxic liver disease, and non-alcoholic fatty liver disease, autoimmune liver disease, etc. According to the speed of onset, it is divided into chronic liver disease and acute liver disease.

The liver disease is selected from the group consisting of liver injury, liver fibrosis, hepatitis (e.g., viral hepatitis A, viral hepatitis B, viral hepatitis C), cirrhosis, liver failure, liver abscess, liver cyst, intrahepatic hemangioma, liver cancer, intrahepatic bile duct stone, liver fluke disease, hepatic hydatid disease, alcoholic liver disease, non-alcoholic fatty liver disease, or any combination thereof.

The liver disease includes fatty liver. Herein, the term "fatty liver" refers to a pathological change caused by excessive accumulation of fat in liver cells due to various causes, and it is a common liver pathological change rather than an independent disease. The fatty liver is generally divided into two categories: alcoholic fatty liver and non-alcoholic fatty liver.

The liver disease includes steatohepatitis, and herein, the term "steatohepatitis" is a type of fatty liver. Mild fatty liver generally has no obvious liver injury and no obvious symptoms. Moderate to severe fatty liver is generally accompanied by liver cell damage, called steatohepatitis, with corresponding clinical symptoms.

The liver disease includes non-alcoholic fatty liver disease. In this context, the term "non-alcoholic fatty liver disease" refers to a clinicopathological syndrome mainly characterized by excessive deposition of fat in liver cells and caused by factors excluding alcohol and other definite liver-damaging factors, which is an acquired metabolic stress-induced liver injury closely associated with insulin resistance and genetic susceptibility. It includes simple fatty liver, non-alcoholic steatohepatitis and related cirrhosis.

The liver disease includes non-alcoholic steatohepatitis, and the term "non-alcoholic steatohepatitis" used herein refers to an inflammatory subtype of non-alcoholic fatty liver disease, which is accompanied with hepatic steatosis and evidence of liver cell damage (ballooning change) and inflammation, and with or without liver fibrosis. Over time, the non-alcoholic steatohepatitis may progress to liver fibrosis, cirrhosis, end-stage liver disease, or need for liver transplantation.

The liver disease includes hepatic steatosis, and the term "hepatic steatosis" used herein refers to the presence of fat droplets within the cytoplasm of hepatocytes. When hepatic steatosis occurs, in mild cases, there is not obvious abnormality by naked eye observation; in severe cases, the liver is enlarged, soft in texture, light yellow to earthy yellow in color, fuzzy in structure on the cut surface, and greasy. Microscopically, vacuoles of different sizes appear in the denatured liver cytoplasm, and in severe cases, they may merge into a large vacuole, resembling fat cells.

The liver disease includes liver injury. The liver injury is selected from the group consisting of acute liver injury, chronic liver injury, chemical liver injury, physical liver injury, or any combination thereof. As used herein, the term "liver injury" is a pathological consequence of various liver diseases. The liver injury caused by various harmful factors mainly includes viral liver injury, alcohol-induced liver injury, drug-induced liver injury, etc. In certain embodiments, the liver injury is acute liver injury.

In certain embodiments, the medicament further comprises a carrier or excipient.

In certain embodiments, the carrier is selected from the group consisting of gelatin, chitosan, sodium alginate, collagen, silk protein, cellulose, fibrin, polylactic acid, polyurethane, polyethylene oxide, polyethylene glycol, polylactic glycolic acid, poly(ε-caprolactone), silicate, silicone rubber, extracellular matrix, decellularized scaffold, or any combination thereof.

In certain embodiments, the carrier is selected from the group consisting of gelatin, collagen, or any combination thereof.

In certain embodiments, the medicament further comprises a second active component.

In certain embodiments, the second active component is selected from the group consisting of diisopropylamine ascorbate, choline chloride, inositol, dehydrocholic acid, magnesium sulfate, polyene phosphatidyl choline (Essentiale), glucuronolactone (Glucurone), glutathione (Gluthion, Atomolan), tiopronin (Capen), glycyrrhizin preparation, adenosylmethionine (Transmetil), hepatocyte growth-promoting factor, Schisandra chinensis (biphenyl diester), silymarin (Silibinin, Legalon, Baoganning), oleanolic acid (Ganshu tablet), Yinzhihuang preparation, Herba Artemisiae Capillariae or any combination thereof.

In certain embodiments, the second active component is selected from the group consisting of aminosalicylic drug, corticosteroid drug, immunosuppressive agent, biological agent, or any combination thereof. In certain embodiments, the aminosalicylic drug is 5-aminosalicylic acid. In certain embodiments, the corticosteroid drug is glucocorticoid. In certain embodiments, the immunosuppressive agent is selected from azathioprine, 6-mercaptopurine, methotrexate, cyclosporine A, tacrolimus, or any combination thereof. In certain embodiments, the biological agent is TNF antagonist.

In certain embodiments, the medicament is in an unit dosage form, and the unit dose of the medicament contains the mesenchymal stem cells in an amount of no less than $1\times10^4$/ml (e.g., no less than $1\times10^4$/ml, no less than $3\times10^4$/ml, no less than $5\times10^4$/ml, no less than $7\times10^4$/ml, no less than $1\times10^5$/ml, no less than $3\times10^5$/ml, no less than $5\times10^5$/ml, no less than $7\times10^5$/ml, no less than $1\times10^6$/ml, no less than $3\times10^6$/ml, no less than $5\times10^6$/ml, no less than $7\times10^6$/ml, no less than $1\times10^7$/ml, no less than $3\times10^7$/ml, no less than $5\times10^7$/ml, no less than $7\times10^7$/ml, no less than $1\times10^8$/ml, no less than $3\times10^8$/ml, no less than $5\times10^8$/ml, no less than $7\times10^8$/ml, no less than $1 \times 10^9$/ml, no less than $3\times10^9$/ml, no less than $5\times10^9$/ml, no less than $7\times10^9$/ml, no less than $1\times10^{10}$/ml, no less than $3\times10^{10}$/ml, no less than $5\times10^{10}$/ml or no less than $7\times10^{10}$/ml). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

In certain embodiments, the route of administration of the mesenchymal stem cells is selected from the group consisting of injection administration, smear administration, adhesive administration, enema administration, perfusion administration, rectal administration, and oral administration.

In certain embodiments, the method further comprises administering to a subject in need thereof a second active component as previously described or defined.

In certain embodiments, the subject is a mammal, such as a human.

In another aspect, the present invention provides a product for treating digestive system disease, which comprises a mesenchymal stem cell population as first active component.

In certain embodiments, the mesenchymal stem cell population is as previously described or defined.

In certain embodiments, the digestive disease is as described or defined above.

In certain embodiments, the product further comprises a second active component.

In certain embodiments, the second active component is as previously described or defined.

In certain embodiments, the first active component and the second active component are present alone or in combination.

In certain embodiments, the first active component is administered in combination with a second active component selected from those previously described.

In certain embodiments, the product is an implant, preferably, the implant is used to improve microenvironment and inhibit immune rejection.

In certain embodiments, the subject is a mammal, such as a human.

Acute liver injury

In this article, the term "acute liver injury" refers to that acute injury or necrosis of liver cells occurs in a short period of time, accompanied with abnormal liver function, and even liver failure in some patients. The causes of the acute liver injury mainly include viral infection, improper drug use, food additives, excessive intake of ethanol, mistakenly ingested poisonous food, radiation damage, and the like. The acute liver injury includes viral, chemical, and drug-induced liver injuries.

As used herein, the term "chemical acute liver injury" refers to liver injury caused by chemical hepatotoxic substances. These chemicals include alcohol, chemical toxicants in the environment (e.g., carbon tetrachloride), and certain drugs.

The liver has strong defense and repair capabilities. When liver injury is caused by various reasons, it can rely on hepatocyte regeneration to rebuild the liver structure and restore liver function. The acute liver injury is a type of disease characterized by short-term liver insufficiency caused by virus, drug, alcohol, autoimmune abnormality and other factors. The main pathological changes are extensive necrosis and apoptosis of liver cells, making the liver unable to perform normal synthesis and metabolic function. If the disease progression is not intervened in a short period of time, it may deteriorate rapidly, resulting in coagulation dysfunction, jaundice, ascites, and hepatic encephalopathy, which may further lead to multiple organ failure. When it progresses to liver failure, the overall prognosis is extremely poor due to rapid progression and difficult treatment. Autologous liver transplantation is the most effective method for the treatment of severe liver injury, but there are various risks such as lack of liver donors, high surgical costs, postoperative complications and immune rejection, and thus the application of liver transplantation is limited. Given the high mortality of acute liver injury and the limitations of liver transplantation, stem cell treatment has shown great potential and advantages in the treatment of acute and chronic liver diseases.

In one aspect, the present invention relates to use of the mesenchymal stem cell population, the culture, the culture supernatant or the pharmaceutical composition as described herein for the prevention and/or treatment of acute liver injury in a subject, or in the manufacture of a medicament for the prevention and/or treatment of acute liver injury in a subject. The above use may protect the liver, maintain and/or prolong and/or improve liver function.

More specifically, in a certain embodiment, the pharmaceutical composition can inhibit or reduce the weight loss rate of patients with acute liver injury, and reduce the mortality rate of patients with acute liver injury.

In a certain embodiment, the pharmaceutical composition is capable of reducing the content of transaminase and/or alkaline phosphatase in serum.

In a certain embodiment, the pharmaceutical composition is capable of preventing inflammatory cell infiltration.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the culture supernatant of the present invention, or a pharmaceutical composition comprising the culture supernatant can be administrated by intradermal injection, subcutaneous injection, intramuscular injection, intravenous injection, oral administration, etc.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

Non-alcoholic steatohepatitis

Non-alcoholic steatohepatitis (NASH), also known as metabolic steatohepatitis, is a progressive form of non-alcoholic fatty liver disease, defined as the presence of 5% or more hepatic steatosis accompanied with inflammation and hepatocyte damage (e.g., ballooning change), with or without fibrosis. NASH easily develops into liver cirrhosis, liver cancer and other diseases. There are 3% to 5% of NASH patients worldwide. There are about 1.09 million patients with liver cirrhosis in China, and it will increase to 2.32 million in 2030. The development of NASH is closely related to heredity (polymorphism of PNPLA3), living habits (host's eating habits, number of meals, sleep-wake cycle, etc.), obesity, metabolic syndrome, etc. Common symptoms of NASH include anorexia, fatigue, abdominal distension, nausea and vomiting, dull pain in liver area, and hepatomegaly. Environmental, metabolic, and genetic factors lead to the accumulation of free fatty acids in the liver, which in turn causes a series of cell damage. Currently, there are non-clinical and clinical treatments for NASH treatment. The former includes lifestyle changes to improve disease course, while the latter includes liver transplantation, surgery and drugs under investigation to treat the disease. A therapeutic strategy for developing a healthy lifestyle is more suitable for adjuvant therapy. Liver transplantation is expensive and donors are scarce; surgical treatment requires patients to meet eligibility criteria and has limitations; there are no FDA-approved drugs for treatment of NASH currently. Therefore, the treatment of NASH is still in a state of urgent shortage.

In one aspect, the present invention relates to use of the mesenchymal stem cell population, the culture, the culture supernatant or the pharmaceutical composition as described herein for the prevention and/or treatment of non-alcoholic steatohepatitis in a subject, or in the manufacture of a medicament for preventing non-alcoholic steatohepatitis, or delaying, or reducing non-alcoholic steatohepatitis, or preventing/alleviating non-alcoholic steatohepatitis in a subject.

In certain embodiments, the pharmaceutical composition is capable of reducing liver weight, inhibiting fat accumulation in liver, and/or reducing hepatic steatosis.

In certain embodiments, the pharmaceutical composition is capable of reducing the content of transaminase and improving liver function.

In certain embodiments, the pharmaceutical composition is capable of inhibiting fibrosis, and/or inhibiting inflammation.

In certain embodiments, the subject is a mammal, such as a human.

In certain embodiments, the medicament comprises a therapeutically effective amount of the mesenchymal stem cell population and/or the culture, and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain embodiments, the administration is performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

Inflammatory bowel disease

Inflammatory bowel disease (IBD) is a typical chronic relapsing disease associated with dysregulation of the mucosal immune system and commensal ecosystem, embodying the interaction between host genetics, host immunology, microbiome, and environmental exposures.

IBD is manifested by two major clinical entities: Crohn's disease (CD) and Ulcerative colitis (UC). UC affects the colon, CD may affect any area of the gastrointestinal tract, but occurs mainly in terminal ileum of the small intestine.

Intestinal complications of IBD include abscess, intestinal obstruction, intestinal perforation, colon cancer, anal fissure, fistula, worsening of menstrual symptom, and toxic megacolon. Certain complications of IBD (Crohn's disease and Ulcerative colitis) may be life-threatening and require prompt treatment to prevent more serious disease.

Abscess: A more common abscess in Crohn's disease than in Ulcerative colitis is the accumulation of pus at the site of infection. It may invisibly occur in the body, such as inside intestinal wall, outside intestinal wall, in skin, etc. Internal abscess may be treated with antibiotics, but if it cannot be solved, it needs to be drained. This can be done by inserting a catheter through the skin into the abscess site. The catheter can be inserted in other ways, such as through the stomach wall. In some cases, surgery is required to drain the pus.

Intestinal obstruction: Intestinal obstruction refers to that a part of small or large intestine is partially or completely obstructed, which prevents the body from excreting waste. The obstruction is usually accompanied by severe pain, vomiting and constipation. In some cases, a nasogastric tube can help relieve symptoms, but surgery may be needed to remove the blockage.

Intestinal perforation: The risk of intestinal perforation (hole) is rare, but it is a potentially fatal complication of IBD. The perforation is most common during the first episode of ulcerative colitis and in people whose intestinal wall has become very thin due to severe disease. The perforation is most often treated surgically to repair the hole or even remove a part of the intestine.

Colon cancer: Patients with IBD are at increased risk of developing colon cancer, especially those who have had ulcerative colitis of the whole colon for 8 to 10 years. The patients with Crohn's disease are also at risk, although there is little information about the degree of risk. Colon cancer must be carefully monitored by colonoscopy for anyone with IBD, especially those at highest risk.

Anal fissure: Anal fissure is a painful tear in the anal canal that may cause bleeding. Most fissures heal without surgery, and treatments such as topical creams may be used to ensure smooth bowel movements without tension. Fissures that do not heal and become chronic may require surgery.

Fistula: Fistula is an abnormal connection between two body cavities or between a body cavity and skin. Fistulas are more common in Crohn's disease than in ulcerative colitis, and in fact, about 25 percent of patients with Crohn's disease may develop a fistula at some point during their disease process. Some fistulas may be treated with medication, but the more severe or extensive they are, the more likely they are to require surgery.

Premenstrual syndrome: Some women with IBD notice that their symptoms worsen during menstruation. Diarrhea and pain may increase before and during menstruation. The cause of these symptoms may be an increase in hormones during the menstrual cycle.

Toxic megacolon: Toxic megacolon is rare, but it is a life-threatening disease. If left untreated, the toxic megacolon may cause shock, perforation, or infection of abdomen or blood. In some cases, it may be medically treated, but severe cases may require surgery.

In one aspect, the present invention relates to use of the mesenchymal stem cell population, the culture, the culture supernatant and/or the pharmaceutical composition as described herein for the prevention and/or treatment of inflammatory bowel disease in a subject, or in the manufacture of a medicament for the prevention and/or treatment of inflammatory bowel disease in a subject.

The aforementioned use can prevent and treat infiltration of inflammatory cells, protect colon, reduce inflammatory factor (e.g., IFN-γ, IL-6, TFN-α, iNOS, etc.), increase or upregulate anti-inflammatory factor (e.g., IL10, etc.), inhibit inflammation onset, secrete nutritional factor (e.g., VEGF, HGF, SDF-1a, etc.), and promote colon tissue repair and so on, so as to achieve the functions such as inhibiting inflammation and promoting tissue repair in the prevention and/or treatment of inflammatory bowel disease, thereby protecting colon tissue, protecting intestine, and improving tissue repair ability.

In certain embodiments, the pharmaceutical composition is capable of treating Crohn's disease (CD) and/or Ulcerative colitis (UC).

In certain embodiments, the subject is a mammal, such as a human.

In certain embodiments, the medicament comprises a therapeutically effective amount of the mesenchymal stem cell population and/or the culture, and/or a therapeutically effective amount of the culture supernatant as described herein.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the culture supernatant of the present invention or a pharmaceutical composition comprising the culture supernatant can be administered by intradermal injection, subcutaneous injection, intramuscular injection, intravenous injection, oral administration, etc. In certain embodiments, the pharmaceutical composition is administered by intravenous or intraperitoneal injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the bioscaffold or a pharmaceutically acceptable biomaterial, including but not limited to, collagen scaffold, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

In certain embodiments, the pharmaceutical composition also comprises another pharmaceutical combination, including but not limited to 5-aminosalicylic acid, NF-κB and activator protein-1 (Activator protein-1, API), immunosuppressive drug (Azathioprine), 6-mercaptopurine, methotrexate, Cyclosporin A, tacrolimus, tumor necrosis factor (TNF), antagonist, etc.

**Nervous system disease**

In one aspect, the present invention provides use of the mesenchymal stem cell population or its culture supernatant in the manufacture of a medicament, wherein the medicament is used for preventing and/or treating a nervous system disease; alternatively, the present invention provides a method for preventing and/or treating a nervous system disease, comprising administering to a subject in need thereof a prophylactically and/or therapeutically effective amount of the mesenchymal stem cell population or its culture supernatant.

Nervous system disease is a disease mainly manifested by dysfunction of the nervous system, and its symptoms mainly include abnormal mental behavior, forgetfulness, insomnia, emotional change, and intellectual change. It includes cerebrovascular disease, degenerative disease of nervous system, infectious disease of central nervous system, demyelinating disease of central nervous system, movement disorder, epilepsy, spinal cord disease, genetic disease of nervous system, dysplasia of nervous system, systemic toxic disease of central nervous system, tumor disease of central nervous system, immune disease of central nervous system, etc. Examples include cerebrovascular disease, periodic paralysis, progressive muscular dystrophy, myotonic dystrophy, ataxia, insomnia, neurasthenia, epilepsy, trigeminal neuralgia, neurodegenerative disease, neuropathic headache (e.g., migraine headache, etc.) and neuropathy, etc.

Neuropathy

Neuropathy is a disease with clinical manifestations of sensory, motor, consciousness, and autonomic dysfunction of the central nervous system, peripheral nervous system, and autonomic nervous system.

Neurodegenerative disease

Neurodegenerative disease is a disease of dysfunction caused by neurons gradually lose structure or function, or even die, and includes amyotrophic lateral sclerosis, Parkinson's disease, Alzheimer's disease, epilepsy, Huntington's disease and spinal muscular atrophy, brain injury, different types of spinocerebellar ataxia, dentatorubral-pallidoluysian atrophy, transmissible spongiform encephalopathy, primary lateral sclerosis, multiple sclerosis, cardiovascular and cerebrovascular dementia, neuropathic pain, glaucoma, traumatic spinal cord injury, multiple system atrophy, etc.

(1) Amyotrophic lateral sclerosis (ALS):

Amyotrophic lateral sclerosis (ALS), commonly known as ALS, is a spontaneous and fatal neurodegenerative disease that affects the upper motor neurons of motor cortex and the lower motor neurons of brainstem and spinal cord. The loss of large numbers of motor neurons results in muscle wasting and spontaneous contraction and spasm. ALS is divided into two categories: familial ALS (FALS) and sporadic ALS (SALS), the former accounts for 10% and the latter accounts for 90%. The onset age of ALS patients is usually after the age of 40, and the high incidence of FALS and SALS occurs at the age of 47-52 and 58-63, respectively, while the incidence decreases after the age of 80, and men are more prone to the disease than women. Patients generally survive 3 to 5 years from the onset of the disease. Various factors are closely related to the incidence of ALS, such as: genetics, occupation, lifestyle, age, etc. On the one hand, the pathogenesis of ALS is that astrocytes fail to restore in time the glutamate accumulated in the synapse, resulting in glutamate excitotoxicity; on the other hand, the mutated genes including SOD1, UBQLN2, OPTN, VCP, TDP43, FUS, C9ORF72 lead to the production of wrong protein conformation polymers that brings toxicity, and the production of toxic RNA species, which aggravate motor neuron damage, cause synapse retraction and fails to bind to postsynaptic membrane receptor and to complete electrical signal transmission, and finally the clinical manifestations appear. Drug treatments are available for the treatment of ALS. Currently, only two neuroprotective drugs approved by the U.S. Food and Drug Administration (FDA) and the European Medicines Agency (EMA) can extend life for some patients by several months: riluzole, which is able to block excess glutamine neurotransmission; edaravone, which is able to prevent oxidative stress damage; surgical treatment: nasogastric feeding or gastrostomy may be performed if the patient has difficulty in swallowing or masticating. If the respiratory muscles are paralyzed, tracheotomy should be performed as soon as possible, and ventilation should be used to maintain breathing; there is also adjuvant therapy: rehabilitation training. Clinically, there are specific treatment methods corresponding to specific symptoms. The aforementioned treatment methods can only extend the survival time of patients by several months, but do not significantly improve the patient's quality of life. Therefore, there is still an urgent need for more effective treatments. In addition to the above treatment methods, gene editing is currently a hot topic in preclinical research. For example, the direct editing of SOD1 through the CRISPR/Cas9 gene editing system is used to treat amyotrophic lateral sclerosis in vitro and in transgenic mice. However, there are still many uncertainties in gene editing.

In some embodiments, the medicament of the present invention can be administered by intravenous injection or brain tissue injection in addition to the aforementioned administration methods.

In a certain embodiment, the medicament delays the onset of disease.

In a certain embodiment, the medicament is capable of enhancing limb coordination, motor ability, reaction capacity such as grasping power, and the like.

In a certain embodiment, the medicament is capable of improving muscle strength, reducing motor neuron damage in amyotrophic lateral sclerosis.

In a certain embodiment, the medicament is capable of improving motor neurons, reducing microglia and astrocytes, and alleviating disease progression in amyotrophic lateral sclerosis.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the culture supernatant of the present invention or a pharmaceutical composition comprising the culture supernatant can be administrated by intradermal injection, subcutaneous injection, intramuscular injection, intravenous injection, oral administration, etc. In some embodiments, the pharmaceutical composition is administrated by intravenous injection or brain tissue injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a pharmaceutically acceptable sterile isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

(2) Epilepsy

Epilepsy is a chronic brain disease caused by a variety of etiologies and characterized by sudden, recurrent and transient central nervous system dysfunction caused by excessive firing of brain neurons. It includes idiopathic epilepsy syndrome, symptomatic epilepsy syndrome, possible symptomatic epilepsy syndrome or cryptogenic epilepsy, reflex epilepsy syndrome, benign epilepsy syndrome, epileptic encephalopathy.

Epilepsy is a chronic brain disorder characterized by recurring seizures that occur suddenly and for no apparent reason, and is the second most common neurological disorder after stroke. The "abnormal firing" of neurons in the brain causes epileptic seizures, which are repetitive and short-lived. Epilepsy affects more than 70 million people worldwide, and the incidence rate in the Chinese population is between 5 to 7‰, with 6.5 to 9.1 million patients nationwide. Some cerebrovascular complications, head trauma, central nervous system infection, etc. can lead to secondary epilepsy; and sleep, age and genetics are closely related to idiopathic epilepsy. In the treatment of epilepsy, antiepileptic drugs are the most widely used. However, despite the existence of 30 antiepileptic drugs (AEDs) with different molecular targets, there are still many challenges in the drug treatment of epilepsy, such as drug resistance, side effects, toxicity associated with frequent dependence toxicity and memory deficits, etc. In addition, brain surgery is the most important alternative treatment; however, eligibility for enrolment as well as risks and costs must be considered. At present, clinical trials are mainly drug treatment, and more than 200 are in phase III.

In one aspect, the present invention relates to use of the mesenchymal stem cell population, the culture, the culture supernatant or the pharmaceutical composition as described herein in the manufacture of a medicament for preventing and/or treating epilepsy, delaying or alleviating epileptic seizure, or preventing epileptic seizure; alternatively, the present invention relates to a method for preventing and/or treating epilepsy, delaying or alleviating epileptic seizure, or preventing epileptic seizure, which comprises administering to a subject in need thereof an effective amount of the mesenchymal stem cell population, the culture, the culture supernatant or the pharmaceutical composition as described herein.

The mesenchymal stem cell population, the culture, the culture supernatant or the pharmaceutical composition of the present invention can prevent epilepsy, or delay, or alleviate epileptic seizure, or prevent epileptic seizure.

In some preferred embodiments, the administration is performed by cerebral or intravenous injection.

In some embodiments, the pharmaceutical composition is capable of increasing the number of GABAergic neurons in brain, or activating GABAergic neurons, or reducing the number of microglia, or remodeling and repairing neural circuit of deficient in GABAergic neurons in a model, or promoting the differentiation ability of endogenous stem cells to GABAergic lineage, or inhibiting inflammatory response.

In some embodiments, the pharmaceutical composition is capable of improving memory and learning ability in a model animal.

In some embodiments, the pharmaceutical composition is capable of providing intracerebral secretion level of nutritional molecule (e.g., GDNF) so as to protect neurological function.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the culture supernatant of the present invention or a pharmaceutical composition comprising the culture supernatant can be administrated by intradermal injection, subcutaneous injection, intramuscular injection, intravenous injection, oral administration, etc. In some embodiments, the pharmaceutical composition is administrated by cerebral or intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

(3) Alzheimer's disease (AD)

Alzheimer's disease (AD) is the most common form of senile dementia and one of the most common chronic diseases in old age, accounting for about 50 to 70% of senile dementia. It affects more than 35 million people worldwide. The clinical manifestations of AD are progressive memory loss and cognitive dysfunction. Alzheimer's disease is associated with two pathogenic features, namely, extracellular amyloid beta (Aβ) deposition and intracellular neurofibrillary tangles (Neurofibrillary tangles, NTFs), accompanied with neuroinflammation and extensive neuronal and synaptic loss, leading to progressive memory loss and cognitive dysfunction. At present, there is not a specific drug that can cure Alzheimer's disease or effectively reverse the disease process. The combination of drug therapy, non-drug therapy and careful nursing can alleviate and delay the onset of the disease. Therefore, it is important to develop effective therapeutic strategies that can cure AD or reverse AD. At present, clinical trials have been carried out mainly on drug research, and there are more than 200 clinical trials. There are 10 clinical trials of mesenchymal stem cells (MSCs), which are in clinical phases I and II.

In one aspect, the present invention relates to use of the mesenchymal stem cell population, the culture, the culture supernatant or the pharmaceutical composition as described herein in the manufacture of a medicament for preventing and/or treating Alzheimer's disease or cerebrovascular disease, delaying or alleviating Alzheimer's disease or cerebrovascular disease, or preventing and alleviating Alzheimer's disease or cerebrovascular disease; alternatively, relates to a method for preventing and/or treating Alzheimer's disease or cerebrovascular disease, delaying or alleviating Alzheimer's disease, or preventing or alleviating Alzheimer's disease or cerebrovascular disease, which comprises administering to a subject in need thereof an effective amount of the mesenchymal stem cell population, the culture, the culture supernatant or the pharmaceutical composition as described herein.

In some embodiments, the medicament is capable of improving learning and memory ability, and improving memory and cognition deficits.

In some embodiments, the medicament is capable of reducing the accumulation of amyloid deposit in brain and reducing the adverse effect of amyloid deposit on nerve.

In some embodiments, the medicament is capable of inhibiting the conversion of microglia into a proinflammatory form, preventing excessive activation and dysfunction of microglia.

In some embodiments, the medicament is capable of increasing the phagocytic ability of microglia, scavenging amyloid deposit and apoptotic cell debris, inhibiting the production of A1 astrocyte in the inflammatory environment of AD brain, and inhibiting over-activated immunity.

In some embodiments, the medicament is capable of increasing neural survival, improving cognition and memory.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the medicament is in an unit dose form, and the unit dosage of the medicament contains the mesenchymal stem cells in an amount of no less than $1 \times 10^4$ cells/ml (e.g., no less than $1 \times 10^4$ cells/ml, no less than $3 \times 10^4$ cells/ml, no less than $5 \times 10^4$ cells/ml, no less than $7 \times 10^4$ cells/ml, no less than $1 \times 10^5$ cells/ml, no less than $3 \times 10^5$ cells/ml, no less than $5 \times 10^5$ cells/ml, no less than $7 \times 10^5$ cells/ml, no less than $1 \times 10^4$ cells/ml, no less than $3 \times 10^6$ cells/ml, no less than $5 \times 10^6$ cells/ml, no less than $7 \times 10^6$ cells/ml, no less than $1 \times 10^7$ cells/ml, no less than $3 \times 10^7$ cells/ml, no less than $5 \times 10^7$ cells/ml, no less than $7 \times 10^7$ cells/ml, no less than $1 \times 10^8$ cells/ml, no less than $3 \times 10^8$ cells/ml, no less than $5 \times 10^8$ cells/ml, no less than $7 \times 10^8$ cells/ml, no less than $1 \times 10^4$ cells/ml, no less than $3 \times 10^8$ cells/ml, no less than $5 \times 10^8$ cells/ml, no less than $7 \times 10^9$ cells/ml, no less than $1 \times 10^{10}$ cells/ml, no less than $3 \times 10^{10}$ cells/ml, no less than $5 \times 10^{10}$ cells/ml or no less than $7 \times 10^{10}$ cells/ml, preferably 3 to $6 \times 10^6$).

In some embodiments, the medicament further comprises a pharmaceutically acceptable carrier or excipient; preferably, the carrier is selected from the group consisting of gelatin, chitosan, sodium alginate, collagen, silk protein, cellulose, fibrous protein, polylactic acid, polyurethane, polyethylene oxide, polyethylene glycol, polylactic glycolic acid, poly($\varepsilon$-caprolactone), silicate, silicone rubber, extracellular matrix, decellularized scaffold and any combination thereof; preferably, the carrier is selected from the group consisting of gelatin, collagen and any combination thereof; preferably, the medicament is an injection, microinjection, mucosal patch, enema, suppository, gel, oral preparation, aerosol, drop, ointment, implant or capsule, preferably injection; preferably, the medicament further comprises a pharmaceutically acceptable sterile isotonic aqueous or non-aqueous solution, dispersion, suspension or emulsion.

In some embodiments, the medicament further comprises an additional active component, the additional active component is selected from for example the group consisting of: $\beta$-secretase inhibitor (e.g., OM99-2), $\gamma$-secretase inhibitor (e.g., R-flurbiprofen), cholinesterase inhibitor (e.g., donepezil, donepezil hydrochloride, rivastigmine, huperzine A, tacrine, galantamine or galantamine hydrobromide), M choline receptor agonist or antagonist (e.g., M1 choline receptor agonist including zanomeline, saccomeline, nefiracetam, AF-102B, and SR-46559A; M2 choline receptor antagonist including BIBN-99 and AF-DX 11; or N-choline receptor agonist including nicotine and ABT-418), glutamate receptor antagonist (e.g., memantine, memantine hydrochloride, or riluzole), calcium ion antagonist (e.g., nimodipine or flunarizine), antioxidant (e.g., vitamin E, L-deprenyl, melatonin, melatonin, deferoxamine, idebenone, or tiritazad mesylate), A$\beta$-inhibiting drug (e.g., estrogen, chloroquine, congo red, or phenyl aminophenylacetate), dopamine substitute (e.g., levodopa), peripheral decarboxylase inhibitor (e.g., carbidopa or benserazide), dopamine D-receptor agonist (e.g., bromocriptine, pergolide, apomorphine, pramipexole, or ropinirole), neurotrophic agent (e.g., piperacetam, aniracetam, oxiracetam, pramiracetam, or nefiracetam), anticholinergic (e.g., trihexyphenidyl hydrochloride, procyclidine, biperiden, or benztropine), antidepressant (e.g., amitriptyline, phenelzine, tranylcypromine, isocarboxazid, or istradefylline), 5-hydroxytryptamine agonist (e.g., sarizotan or budipine), MAO-B inhibitor (e.g., selegiline or rasagiline), dopamine $\beta$-hydroxylase inhibitor (e.g., fusarinic acid), COMT inhibitor (e.g., entacapone or tolcapone), immunosuppressive agent (e.g., azathioprine, 6-mercaptopurine, methotrexate, cyclosporine A, or tacrolimus) and any combination thereof; preferably, the mesenchymal stem cell population and the additional active component are present alone or in combination.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some preferred embodiments, the administration is performed by cerebral or intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

In some embodiments, the administration dosage is not less than $1 \times 10^4$ cells/ml (e.g., not less than $1 \times 10^4$ cells/ml, not less than $3 \times 10^4$ cells/ml, not less than $5 \times 10^4$ cells/ml, not less than $7 \times 10^4$ cells/ml, not less than $1 \times 10^5$ cells/ml, not less than $3 \times 10^5$ cells/ml, not less than $5 \times 10^5$ cells/ml, not less than $7 \times 10^5$ cells/ml, not less than $1 \times 10^6$ cells/ml, not less than $3 \times 10^6$ cells/ml, not less than $5 \times 10^6$ cells/ml, not less than $7 \times 10^6$ cells/ml, not less than $1 \times 10^7$ cells/ml, not less than $3 \times 10^7$ cells/ml, not less than $5 \times 10^7$ cells/ml, not less than $7 \times 10^7$ cells/ml, not less than $1 \times 10^8$ cells/ml, not less than $3 \times 10^8$ cells/ml, not less than $5 \times 10^8$ cells/ml, not less than $7 \times 10^8$ cells/ml, not less than $1 \times 10^4$ cells/ml, not less than $3 \times 10^8$ cells/ml, not less than $5 \times 10^8$ cells/ml, not less than $7 \times 10^9$ cells/ml, not less than $1 \times 10^{10}$ cells/ml, not less than $3 \times 10^{10}$ cells/ml, not less than $5 \times 10^{10}$ cells/ml or not less than $7 \times 10^{10}$ cells/ml).

In some embodiments, a prophylactically and/or therapeutically effective amount of the mesenchymal stem cell population is administered to a subject by injection administration, mucosal administration, cavity administration, oral administration, respiratory tract administration or skin administration.

In some embodiments, the method further comprises administering to the subject simultaneously, sequentially or alternately a prophylactically and/or therapeutically effective amount of an additional active component, in which the additional active component is selected from for example the group consisting of: β-secretase inhibitor (e.g., OM99-2), γ-secretase inhibitor (e.g., R-flurbiprofen), cholinesterase inhibitor (e.g., donepezil, donepezil hydrochloride, rivastigmine, huperzine A, tacrine, galantamine or galantamine hydrobromide), M choline receptor agonist or antagonist (e.g., M1 choline receptor agonist including zanomeline, saccomeline, nefiracetam, AF-102B, and SR-46559A; M2 choline receptor antagonist including BIBN-99 and AF-DX 11; or N-choline receptor agonist including nicotine and ABT-418), glutamate receptor antagonist (e.g., memantine, memantine hydrochloride, or riluzole), calcium ion antagonist (e.g., nimodipine or flunarizine), antioxidant (e.g., vitamin E, L-deprenyl, melatonine, melatonin, deferoxamine, idebenone, or tiritazad mesylate), Aβ-inhibiting drug (e.g., estrogen, chloroquine, congo red, or phenyl aminophenylacetate), dopamine substitute (e.g., levodopa), peripheral decarboxylase inhibitor (e.g., carbidopa or benserazide), dopamine D-receptor agonist (e.g., bromocriptine, pergolide, apomorphine, pramipexole, or ropinirole), neurotrophic agent (e.g., piperacetam, aniracetam, oxiracetam, pramiracetam, or nefiracetam), anticholinergic (e.g., trihexyphenidyl hydrochloride, procyclidine, biperiden, or benztropine), antidepressant (e.g., amitriptyline, phenelzine, tranylcypromine, isocarboxazid, or istradefylline), 5-hydroxytryptamine agonist (e.g., sarizotan or budipine), MAO-B inhibitor (e.g., selegiline or rasagiline), dopamine β-hydroxylase inhibitor (e.g., fusarinic acid), COMT inhibitor (e.g., entacapone or tolcapone), immunosuppressive agent (e.g., azathioprine, 6-mercaptopurine, methotrexate, cyclosporine A, or tacrolimus) and any combination thereof; preferably, the mesenchymal stem cell population and the additional active component are present alone or in combination.

(4) Extrapyramidal and movement disorder

Extrapyramidal and movement disorder, including Parkinson's disease, secondary Parkinson's disease, Parkinson's disease caused by diseases classified elsewhere, other degenerative disease of the basal ganglia, dystonia, other extrapyramidal and movement disorder, extrapyramidal and movement disorder caused by diseases classified elsewhere.

Movement disorder, also known as extrapyramidal disease, is mainly characterized by dysfunction of voluntary movement regulation without affecting muscle strength, sensory and cerebellar functions. This group of diseases originates from the dysfunction of basal ganglia and is usually divided into two categories: hypertonia-decreased exercise and hypotonia-excessive exercise. The former is characterized by lack of exercise, while the latter is mainly characterized by abnormal involuntary movements.

Parkinson's disease (PD) is a movement disorder, and a chronic neurodegenerative disease affecting the central nervous system, mainly the motor nervous system. Its symptoms usually appear slowly over time, the most obvious early symptoms are tremor, limb stiffness, decreased motor function and abnormal gait, and cognitive and behavioral problems may also be present; dementia is quite common in patients with severe disease, while major depressive disorder and anxiety disorder also occur in more than a third of cases. Other possible symptoms include perception, sleep and emotional problems. The main motor symptoms associated with Parkinson's disease are collectively known as Parkinson's syndrome.

In one aspect, the present invention relates to use of the mesenchymal stem cell population, the culture, the culture supernatant or the pharmaceutical composition as described herein in the manufacture of a medicament for preventing and/or treating Parkinson's disease, delaying or alleviating Parkinson's disease, or preventing or alleviating Parkinson's disease; alternatively, relates to a method for preventing and/or treating Parkinson's disease, delaying or alleviating Parkinson's disease, or preventing or alleviating Parkinson's disease, which comprises administering to a subject in need thereof an effective amount of the mesenchymal stem cell population, the culture, the culture supernatant or the pharmaceutical composition as described herein.

In some embodiments, the medicament is capable of reducing dopaminergic denervation, or/and ameliorating the progression of Parkinson's disease.

In some embodiments, the medicament is capable of improving the stiffness of limb, or/and enhancing the ability to exercise.

In some embodiments, the medicament is capable of protecting neuron, reducing neuronal damage and death, having trophic and synaptic regeneration effects on neuron, reducing inflammation in brain, or/and improving the microenvironment in brain.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some embodiments, the administration is performed by intravenous or cerebral injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Spinal cord injury (SCI)

"Spinal cord injury" refers to a transverse injury of spinal cord structure and function caused by various pathogenic factors (trauma, inflammation, tumor, etc.), resulting impairment of spinal nerve function (motor, sensory, sphincter and autonomic nerve function) below the level of the injured segment, which is divided into primary spinal cord injury and secondary spinal cord injury, the former including traumatic spinal cord injury, the latter including spinal tuberculosis, spinal suppurative infection, transverse myelitis, spinal degenerative diseases, congenital scoliosis, tethered cord syndrome.

Spinal cord injury (SCI) is a traumatic spinal surgical disease caused by trauma, which manifests as sensory, motor and autonomic dysfunction below the injured segment. Foreign epidemiological surveys show that there are 130,000 new spinal cord injury patients worldwide each year, and more than 2.5 million patients are suffering from different degrees of spinal cord injury sequelae, and the annual medical expenditure of these SCI patients will exceed 6 billion US dollars, resulting in a heavy burden to the family and society.

There are two main common outcomes of primary spinal cord injury: spinal cord contusion and spinal cord compression (exogenous force or endogenous force). Secondary injury refers to a secondary injury of spinal cord due to the spinal compression caused by external force-induced spinal cord edema, hematoma formed by hemorrhage of small blood vessels in the spinal canal, compression fracture, and broken intervertebral disc tissues.

In one aspect, the present invention relates to use of the mesenchymal stem cell population as described herein or its culture supernatant or pharmaceutical composition in the manufacture of a medicament for preventing and/or treating a spinal cord injury, delaying or alleviating a spinal cord injury, or preventing and alleviating a spinal cord injury; alternatively, the present invention relates to a method for preventing and/or treating a spinal cord injury, delaying or alleviating a spinal cord injury, or preventing and alleviating a spinal cord injury, which comprises administering to a subject in need thereof an effective amount of the mesenchymal stem cell population, the culture, the culture supernatant or the pharmaceutical composition as described herein.

In some embodiments, the medicament is capable of improving exercise ability.

In some embodiments, the medicament is capable of reducing bladder outlet resistance and detrusor hyperactivity, and improving urination function.

In some embodiments, the medicament is capable of promoting cell survival and enhancing axonal regeneration, inhibiting glial cell activation, anti-fibrosis, and reducing inflammatory response at the site of injury.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the medicament can be transplanted in the form of a suspension, gel, colloid, serous fluid or mixture.

In some embodiments, the medicament further comprises prostacyclin, endothelin-1 receptor antagonist or/and phosphodiesterase type 5 inhibitor in addition to the aforementioned drug.

Cerebrovascular disease

"Cerebrovascular disease" refers to a group of diseases that occur in the cerebral blood vessels, and relate to brain tissue damage and cerebral dysfunction caused by intracranial blood circulation disorder, including stroke, cerebral palsy, cerebral atherosclerosis, cerebral arteritis, cerebral artery injury, cerebral aneurysm, intracranial vascular malformation, cerebral arteriovenous fistula, cerebrovascular accident, cerebral vasospasm, etc.

(1) Stroke

Stroke refers to a group of diseases that relate to brain tissue injury due to the sudden rupture of blood vessels in brain or blockage of blood vessels that prevent blood from flowing into the brain, including ischemic and hemorrhagic two categories. The ischemic stroke comprises cerebral thrombosis, cerebral embolism, cerebral infarction; while the hemorrhagic stroke comprises subarachnoid hemorrhage, hypertensive cerebral hemorrhage, etc.

Stroke is a type of cerebrovascular disease, wherein cerebral blood vessels are narrowed, blocked or ruptured, leading to ischemia or hemorrhage of cerebral tissue, resulting in necrosis of brain cells and tissues. It is divided into ischemic stroke (also known as cerebral infarction) and hemorrhagic stroke (including brain parenchymal hemorrhage, cerebroventricular hemorrhage, and subarachnoid hemorrhage). The incidence rates of ischemic stroke in men and women are 212/100,000 and 170/100,000, respectively; hemorrhagic stroke: 12-15/100,000. However, people with lifestyles such as smoking, poor diet, inactivity, etc. and those with complications including hypertension, diabetes, hyperlipidemia, obesity, etc. are often prone to stroke. At present, for the treatment of stroke, the most widely used is thrombolytic drug tissue plasminogen activator (t-PA). The application of t-PA treatment requires patients to meet the eligibility criteria, so that t-PA is for specific stroke patients, and the treatment time window is short, limited to 4.5 hours. In addition, endovascular therapy is also a major treatment strategy. However, there are also drawbacks, and endovascular stents are only suitable for solving the problem of blocked blood flow in large blood vessels. Although the use of preventive measures, including medication and a healthy lifestyle and aerobic exercise, has led to a decrease in the incidence of stroke, the high recurrence rate is a real headache. Therefore, the treatment of stroke is still a big problem. Existing clinical trials mainly focus on some electronic technology products or software systems to help stroke patients recover, behavioral and lifestyle improvements, drug therapy and cell therapy for stroke patients' recovery. In clinical trials, mesenchymal stem cells (MSCs) are basically in phases I and II.

In one aspect, the present invention relates to use of the mesenchymal stem cell population, the culture, the culture supernatant or the pharmaceutical composition as described herein in the manufacture of a medicament for preventing and/or treating a stroke, delaying or alleviating a stroke, or preventing a stroke; alternatively, the present invention relates to a method for preventing and/or treating a stroke, delaying or alleviating a stroke, or preventing a stroke, which comprises administering to a subject in need thereof an effective amount of the mesenchymal stem cell population, the culture, the culture supernatant or the pharmaceutical composition as described herein.

In some embodiments, the medicament is capable of reducing the degree of cerebral infarction, reducing the infarct size of cerebral tissue, reducing the water content of cerebral tissue, increasing the utilization rate of lateral forelimb, increasing the exercise time, and attenuating the degree of nerve cell damage caused by stroke.

In some embodiments, the medicament is capable of promoting neuronal regeneration, reducing neuronal damage and death, providing nutrition to neuron, and promoting synaptic regeneration.

In some embodiments, the medicament is capable of attenuating the inflammatory response in brain and improving the microenvironment in brain.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some embodiments, the administration is performed by intravenous injection or brain tissue injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Neuropathic pain

Neuropathic pain refers to a pain induced or caused by a disease such as primary lesion or dysfunction affecting the peripheral nerve, nervous system, sensory nerve, etc. of the body, and is characterized by spontaneous pain, allodynia, and hyperalgesia. The disease can be caused by trauma and (or) disease-induced damage to peripheral nerve, spinal cord dorsal root, spinal cord and some parts of central nervous system. Examples include hemifacial spasm, diabetic neuropathy-induced pain, fascial disease, central neuralgia, peripheral neuropathic pain, radicular pain, post-surgical back syndrome, chronic regional pain syndrome and peripheral nerve injury), ischemic pain (e.g., peripheral vascular disease and angina), epileptic seizure, movement disorder associated with Parkinson's syndrome (e.g., tremor, paralysis, rigidity, and dyskinesia), neuropathic pain associated with spinal cord injury, discogenic pain, great occipital neuralgia, sciatica, intercostal neuralgia, cerebrovascular disease, epilepsy, cerebral edema, hydrocephalus, cancerous neuralgia, encephalitis, meningitis, neurodermatitis, neuropathic headache and other neuropathic pains.

Neuropathic pain is a difficult-to-treat pain condition caused by damage or abnormality of nervous system, and is pain that occurs in nerve tissue, such as neuritis. This kind of pain is mainly characterized by nerve lesions and nerve pain, and is paroxysmal in some extents. Sometimes there is a feeling of local pain, but there is no pain when pressing. This is what neuropathic pain is all about. The general treatment of nerve pain is mainly the use of drugs for nourishing nerves and drugs for pain relief, preferably under the guidance of a doctor.

In one aspect, the present invention relates to use of the mesenchymal stem cell population, the culture, the culture supernatant or the pharmaceutical composition as described herein in the manufacture of a medicament for preventing and/or treating a neuropathic pain-induced injury or a relevant neuropathic pain, or for preventing a neuropathic pain, or delaying, or reducing a neuropathic pain, or preventing or alleviating a neuropathic pain; alternatively, the present invention relates to a method for preventing and/or treating a neuropathic pain-induced injury or a relevant neuropathic pain, or preventing a neuropathic pain, or delaying, or reducing a neuropathic pain, or preventing and alleviating a neuropathic pain, which comprises administering to a subject in need thereof an effective amount of the mesenchymal stem cell population, the culture, the culture supernatant or the pharmaceutical composition as described herein.

In some embodiments, the pharmaceutical composition is capable of improving the subject's tolerance to pain, the tolerance to allodynia, and promoting motor coordination.

In some embodiments, the pharmaceutical composition is capable of reducing a proinflammatory factor (IL-1β, IL-6 and IL-17) and inhibiting an inflammatory response.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Demyelinating disease

Demyelinating disease is a group of acquired diseases with different etiologies and different clinical manifestations, but with similar characteristics, and its characteristic pathological changes are the demyelination of nerve fibers with relatively intact nerve cells. The function of myelin sheath is to protect neurons and make nerve impulses transmit quickly on neurons, so the loss of myelin sheath will affect the transmission of nerve impulses.

Demyelinating disease in central nervous system includes multiple sclerosis, other acute disseminated demyelination, and other demyelinating diseases of central nervous system.

Multiple sclerosis

Multiple sclerosis: Multiple sclerosis (MS) is a demyelinating neuropathy, wherein the insulating material (i.e., myelin) on the surface of nerve cells in the patient's brain or spinal cord is damaged, and the transmission of information in the nervous system is impaired, resulting in a range of possible symptoms that affect the patient's activity, mind, and even mental state. These symptoms may include diplopia, unilateral vision impairment, muscle weakness, dysesthesia, or coordination disturbance. Multiple sclerosis is a variable condition, and patients may experience repeated episodes or worsening symptoms. Between episodes, symptoms may disappear completely, but permanent nerve damage remains, especially in severely ill patients.

In one aspect, the present invention relates to use of the mesenchymal stem cell population, the culture, the culture supernatant or the pharmaceutical composition as described herein in the manufacture of a medicament for preventing and/or treating a multiple sclerosis, or for preventing a multiple sclerosis, or delaying, or reducing a multiple sclerosis, or preventing or alleviating a multiple sclerosis; alternatively, the present invention relates to a method for preventing and/or treating a multiple sclerosis, or preventing a multiple sclerosis, or delaying, or reducing a multiple sclerosis, or preventing or alleviating a multiple sclerosis, which comprises administering to a subject in need thereof an effective amount of the mesenchymal stem cell population, the culture, the culture supernatant or the pharmaceutical composition as described herein.

In some embodiments, the medicament is capable of reducing spinal cord demyelination.

In some embodiments, the medicament is capable of inhibiting inflammation at spinal cord site, reducing the number of astrocytes, protecting oligodendrocytes, or/and reducing inflammation in spinal cord segment.

In some embodiments, the medicament is capable of reducing the content of a proinflammatory factor (e.g., IFN-$\gamma$, IL-17, TFN-$\alpha$, IL-2), increasing an anti-inflammatory factor (e.g., IL-10), and inhibiting inflammation.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament the present invention can be administrated by intradermal injection, subcutaneous injection, intramuscular injection, intravenous injection, oral administration, etc. In some embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Neuroinflammation

Neuroinflammation is an inflammatory and degenerative disease of peripheral nerves caused by traumatic brain injury, stroke, cerebral hemorrhage and various neurodegenerative diseases. Under normal state, neuroinflammation maintains homeostasis and promotes tissue repair. However, uncontrolled neuroinflammation can be harmful to the brain. Therefore, controlling deleterious inflammatory responses is a promising therapeutic approach for neurological disorders.

"Neuroinflammation" refers to inflammation caused by degeneration or deterioration of nerve or nerve group due to various reasons, including central neuroinflammation and peripheral neuroinflammation caused by poisoning, infection, nutritional and metabolic disorders, immune abnormalities, aging, genetic variation, etc.

"Central nervous system infection" refers to an acute or chronic inflammatory (or non-inflammatory) disease caused by various biological pathogens (including virus, bacteria, rickettsia, spirochete, parasite, prion protein, etc.) invading the parenchyma, capsule and blood vessels of the central nervous system, including cerebritis, cerebellitis, diencephalitis, brainstemitis, encephalomyelitis, meningoencephalitis, etc. caused by viral, bacterial, fungal, and parasitic infections.

As used herein, the term "bacterial meningoencephalitis" refers to inflammation of pia mater and brain parenchyma caused by bacterial infection, including meningitis, encephalitis or meningoencephalitis caused by streptococcus, staphylococcus, pneumococcus, diplococcus, pasteurella multocida, bacillus pyogenes, necrobacillus, proteusbacillus, corynebacterium pyogenes, Listeria monocytogenes, etc., as well as bacterial meningitis, encephalitis or meningoencephalitis caused by traumatic brain injury, tympanitis, nose-throat inflammation, other local inflammation of head, and emboli transfer through lymph or blood after the rupture of infection focus.

In one aspect, the present invention relates to use of the mesenchymal stem cell population, the culture, the culture supernatant or the pharmaceutical composition as described herein in the manufacture of a medicament for preventing and/or treating a neuroinflammation, delaying or reducing a neuroinflammation, or preventing or alleviating a neuroinflammation; alternatively, relates to a method for preventing and/or treating a neuroinflammation, delaying or reducing a neuroinflammation, or preventing or alleviating a neuroinflammation, which comprises administering to a subject in need thereof an effective amount of the mesenchymal stem cell population, the culture, the culture supernatant or the pharmaceutical composition as described herein.

In some embodiments, the medicament is capable of reducing a proinflammatory factor (e.g., IL-1β, IL-6), increasing an anti-inflammatory factor (e.g., IL-10), and reducing inflammation.

In some embodiments, the medicament is capable of promoting neuronal regeneration, reducing neuronal damage and death, reducing neuroinflammation response, providing nutrition to neurons and promoting synaptic regeneration.

In some embodiments, the medicament is capable of attenuating inflammatory response and improving the microenvironment of a nervous system.

In some embodiments, the medicament is capable of improving contextual memory.

In some embodiments, the subject is a mammal, such as a human.

In some embodiments, the medicament comprises a therapeutically effective amount of the mesenchymal stem cell population and/or the culture, and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament the present invention can be administrated by intradermal injection, subcutaneous injection, intramuscular injection, intravenous injection, oral administration, etc. In some embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Mental disorder

Mental disorder refers to the general term for the disorders in brain function activities, resulting in different degrees of impairments in mental activities such as cognition, emotion, behavior and volition. The common mental disorder includes emotional disorders, cerebral organic mental disorders and so on. The pathogenic factors are multi-faceted: congenital heredity, personality characteristics and physical factors, organic factors, social environmental factors, etc. Mental disorder includes schizophrenia, manic-depressive mental disorder, paranoid disorder (delusion, hallucination), phobic disorder (phobia, anxiety), behavioral volitional disorder (obsessive-compulsive disorder), postpartum mental disorder (postpartum psychosis, postpartum depression, maternal depression), menopausal disorder, paranoid mental disorder and various mental disorders due to organic lesions (delirium, amnestic syndrome, dementia, bulimia/anorexia nervosa, post-traumatic stress disorder).

(1) Mood disorder

Mood disorder, also known as affective mental disorder, refer to a group of disorders that are mainly characterized by significant and long-lasting emotional or mood changes caused by various reasons. Clinically, it is mainly manifested as hyperthymia or hypothymia, accompanied by corresponding cognitive and behavioral changes and psychotic symptoms such as hallucinations and delusions. Mood disorder includes depression, mania, bipolar disorder, persistent mood disorder, and dysthymia.

(2) Depression

Depression, also known as depressive disorder, is a major type of mood disorder characterized by significant and persistent low mood.
The main clinical manifestations are depression, slow thinking, decreased volitional activity, cognitive impairment and sleep disturbance and other somatic symptoms.
In one aspect, the present invention relates to use of the mesenchymal stem cell population, the culture, the culture supernatant or the pharmaceutical composition as described herein, in the manufacture of a medicament for preventing and/or treating a depression, or delaying, or reducing a depression, or preventing or alleviating a depression; alternatively, the present invention relates to a method for preventing and/or treating a depression, or delaying, or reducing a depression, or preventing or alleviating a depression, which comprises administering to a subject in need thereof an effective amount of the mesenchymal stem cell population, the culture, the culture supernatant or the pharmaceutical composition as described herein.
In some embodiments, the medicament is capable of promoting nerve growth and development.
In some embodiments, the subject is a mammal, such as a human.
In some embodiments, the medicament comprises a therapeutically effective amount of the mesenchymal stem cell population and/or the culture, and/or a therapeutically effective amount of the culture supernatant as described herein.
In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.
In some preferred embodiments, the medicament of the present invention can be administrated by intradermal injection, subcutaneous injection, intramuscular injection, intravenous injection, oral administration, etc. In some embodiments, the administration is preformed by intravenous or cerebral injection.
In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.
In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.
In some embodiments, the mesenchymal stem cell population can be transplanted in the form of a suspension, gel, colloid, serous fluid or mixture.
In another aspect, the present invention also provides a product for preventing, treating, delaying and/or alleviating a nervous system disease, comprising the mesenchymal stem cell population of the present invention. In some embodiments, the product further comprises an additional active component, wherein the additional active component is as defined above. In some embodiments, the mesenchymal stem cell population and the additional active component are present as separate components or as a single formulation. In some embodiments, the product is an injection, microinjection, mucosal patch, enema, suppository, gel, oral preparation, aerosol, drop, ointment, implant, or capsule. In some embodiments, the product is an implant.

## Skin disease

In one aspect, the present invention provides use of the mesenchymal stem cell population or its culture supernatant in the manufacture of a medicament for preventing and/or treating skin disease; alternatively, the present invention provides a method for preventing and/or treating skin disease, comprising administering to a subject in need thereof a prophylactically and/or therapeutically effective amount of the mesenchymal stem cell population or its culture supernatant.

Skin is the organ with the largest surface area in the human body. It is a key structure in protecting internal tissues from the effects of mechanical damage, microbial infection, UV radiation and extreme temperatures.

Skin system disease includes viral skin disease, bacterial skin disease, fungal skin disease, animal skin disease, physical skin disease, dermatitis, eczema, drug eruption, urticarial skin disease, pruritic skin disease, erythematous scaly skin disease, connective tissue disease, bullous skin disease, vasculitic skin disease, skin appendage disease, pigmentary disorder skin disease, hereditary skin disease, skin tumor, sexually transmitted disease.

Scaly skin disease is selected from the group consisting of psoriasis, parapsoriasis, erythema multiforme, erythema annulare, pityriasis simplex, pityriasis rosea, pityriasis circinata, pityriasis asbestos, lichen planus, lichen glossy, lichen ruber moniliformis, lichen sclerosis et atrophicus, lichen striatus, exfoliative dermatitis, or any combination thereof.

Psoriasis is a type of erythematous scaly skin disease.

Dermatitis is a skin inflammatory disease.

Dermatitis has obvious skin lesions, which mostly occurs on neck back or both sides thereof, cubital fossa, popliteal fossa, forearm, thigh, calf and lumbosacral region, etc., often appearing in sheets, triangular or polygonal flat-topped papules, thickened skin, raised skin ridges, deepened skin grooves, moss-like shape, and often in reddish or light brown. It is characterized by phenomena that skin becomes peeling, flaking, thickening, discoloration, and itching when touched. It comprises:
Neurodermatitis: It is more common in young and middle-aged people, with severe itching first, followed by skin lesions; its rash is flat papules, lichenoid, without exudation; its rash is more common on neck, extensor sides of limbs, lumbosacral region, popliteal fossa, and vulva; its course is chronic, often recurring.

Atopic dermatitis: The initial lesions are mostly on cheeks. The initial lesions are scattered or clustered small red papules or erythema, which gradually increase, and small blisters, yellow to white scaly crusts can be seen, and there may be exudation, erosion and secondary infection. There is severe itching. Chronic manifestations are dry, larger, raised brownish red papules and coarse and scaly tan lichen-like changes, which may merge into pieces. After scratching, there is often a little exudation, exfoliation and scratches.

Summer dermatitis: At the beginning, the skin lesions are pinpoint-sized erythema and papules, while scratches, blood scabs, skin hypertrophy and hyperpigmentation may appear after scratching due to itching, there is no erosion and exudation, and it tends to occur at extensor limbs of adults. When the temperature drops, its condition is obviously improved and may be cured by itself, and there is an obviously relation between its condition and the climate.

Seborrheic dermatitis: Its rash begins as small red papules around hair follicle orifice, which gradually develop and merge into yellow to red patches covered with greasy scales or crusts. Due to the different locations of lesions, the clinical manifestations are slightly different.

Infant seborrheic dermatitis usually occurs 1 to 3 months after birth. The front head top or entire scalp may be covered with greasy grayish to yellow or yellowish to brown crusts of varying thickness, which may involve the eyebrow area, nasolabial fold, ear hind, etc., with slight itching. It usually heals within 3 to 4 weeks; if continued unhealed, it often is complicated by infection or atopic dermatitis.

Solar dermatitis: It is a tardive photoallergic skin disease induced by sunlight. The clinical manifestations are pleomorphic rashes with erythema, papules, blisters, erosions, scales, and lichenification, often dominated by a certain rash.

Candida dermatitis occurs mostly in the skin folds such as groin, perianal buttock fissure, armpits, skin under female breast, and may also occur in the glans foreskin, labia majora and labia minora, nail grooves and mouth corners. Its rash is mostly local skin flushing, mild swelling, with surface erosion and smelly secretions. Sometimes it may also be dry and desquamated. Pediatric candida dermatitis also often involves the trunk and neck skin, showing extensive and dense red maculopapular rashes that look like reddish sudamen. It may also invade the oral or vulvar mucosa, often with cheese-like secretions that are pseudomembranous.

Mosquito-bite dermatitis: It is a dermatitis caused by being bitten by an insect, contacting the venom or powdery hair of the insect. The common pests are fleas, lice, midges, thorn caterpillars, moths, mosquitoes, bed bugs, bees and so on. Symptoms such as erythema, papules, and wheals may appear. In severe cases, blisters or bullae may appear, and petechiae or blisters may be seen at the sting site.

Hormone-dependent dermatitis is a dermatitis caused by repeatedly and inappropriately using a topical hormone over a long period of time. After topical use of high-efficiency corticosteroids at the same site for more than 3 weeks, erythema, papules, dry desquamation, atrophy, striae atrophicae, telangiectasia, purpura, acne, abnormal pigmentation, rosacea-like dermatitis, perioral dermatitis, photosensitivity, hairy, unrecognizable ringworm, ichthyosis-like changes and other secondary symptoms may appear on the skin, and local obvious conscious itching or burning sensation may occur.

In certain embodiments, the medicament further comprises a carrier or excipient.

In certain embodiments, the carrier is selected from the group consisting of gelatin, chitosan, sodium alginate, collagen, silk protein, cellulose, fibrin, polylactic acid, polyurethane, polyethylene oxide, polyethylene glycol, polylactic glycolic acid, poly($\varepsilon$-caprolactone), silicate, silicone rubber, extracellular matrix, decellularized scaffold, or any combination thereof.

In certain embodiments, the carrier is selected from the group consisting of gelatin, collagen, or any combination thereof.

In certain embodiments, the medicament further comprises a second active component.

In certain embodiments, the second active component is selected from the group consisting of ebastine tablet, loratadine tablet, cetirizine tablet, mometasone furoate ointment, halometasone ointment, mupirocin ointment, fusidic acid ointment, cefixime tablet, roxithromycin tablet, naftifine-ketoconazole ointment, sertaconazole ointment, itraconazole tablet, terbinafine tablet, acyclovir tablet, valaciclovir tablet, penciclovir ointment, interferon gel, or any combination thereof.

In certain embodiments, the medicament is in an unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$ no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$, preferably $1\times10^6$, $3\times10^6$, $5\times10^6$, more preferably $3\times10^6$).

In certain embodiments, the administration dosage of the mesenchymal stem cells is not less than $1\times10^4$/ml (e.g., not less than $1\times10^4$, not less than $3\times10^4$, not less than $5\times10^4$, not less than $7\times10^4$, not less than $1\times10^5$, not less than $3\times10^5$, not less than $5\times10^5$, not less than $7\times10^5$, not less than $1\times10^6$, not less than $3\times10^6$ , not less than $5\times10^6$, not less than $7\times10^6$, not less than $1\times10^7$, not less than $3\times10^7$, not less than $5\times10^7$, not less than $7\times10^7$, not less than $1\times10^8$, not less than $3\times10^8$, not less than $5\times10^8$, not less than $7\times10^8$, not less than $1\times10^9$, not less than $3\times10^9$, not less than $5\times10^9$, not less than $7\times10^9$, not less than $1 \times 10^{10}$, not less than $3 \times 10^{10}$, not less than $5\times10^{10}$ or not less than $7\times10^{10}$, preferably $1\times10^6$, $3\times10^6$, $5\times10^6$/ml, more preferably $3\times10^6$/ml).

In certain embodiments, the administration route of the mesenchymal stem cells is selected from the group consisting of injection administration, smear administration, adhesive administration, enema administration, perfusion administration, rectal administration, and oral administration.

In certain embodiments, the method further comprises administering to a subject in need thereof a second active component as previously described or defined.

In certain embodiments, the subject is a mammal, such as a human.

In another aspect, the present invention provides a product for treating skin disease, which comprises a mesenchymal stem cell population as first active component.

In certain embodiments, the mesenchymal stem cell population is as previously described or defined.

In certain embodiments, the skin disease is as previously described or defined.

In certain embodiments, the product further comprises a second active component.

In certain embodiments, the second active component is as previously described or defined.

In certain embodiments, the first active component and the second active component are present alone or in combination.

In certain embodiments, the first active component is administered in combination with a second active component selected from those previously described.

In certain embodiments, the product is an implant, preferably, the implant is used to improve microenvironment and inhibit immune rejection.

In certain embodiments, the subject is a mammal, such as a human.

Atopic dermatitis (AD)

Atopic dermatitis (AD) is a chronic, recurrent, pruritic and inflammatory skin disease. AD has become an important public health problem with a prevalence of up to 20% in children and 3 to 10% in adults. The pathogenesis of AD is complex, involving many factors such as genetics, immunity and environment, among which the abnormal immune function, especially the immune response effect of immune cells, plays an important role in the onset of AD.

At present, the treatment of AD usually involves the topical and/or systemic use of glucocorticoids and immunosuppressive agent, but the topical use of glucocorticoids shows limited effect in moderate to severe AD patients, while the systemic use of immune preparations has risks such as myelosuppression and increased infection opportunity; the new biological agents such as anti-interleukin (IL)-4R monoclonal antibody Dupilum-ab and anti-immunoglobulin IgE monoclonal antibody Omalizumab show limited research results and significant differences, so that it is necessary to develop new and safe and effective methods for the treatment of AD.

In one aspect, the present invention relates to use of the mesenchymal stem cell population, the culture, the culture supernatant or the pharmaceutical composition as described herein for the prevention and/or treatment of atopic dermatitis in a subject, or in the manufacture of a medicament for preventing atopic dermatitis, or delaying, or reducing atopic dermatitis, or preventing and alleviating atopic dermatitis in a subject.

The present invention provides treatment of atopic dermatitis with M cells. The M cell treatment can improve the microenvironment of mouse skin and inhibit inflammation. The skin appendages are more than those in the OVA group, indicating that M cells can protect the skin appendages, and a very good therapeutic effect is achieved on atopic dermatitis.

In certain embodiments, the pharmaceutical composition is capable of alleviating erythema rash, reducing atopic dermatitis phenotype, reducing AD-like lesions, reducing fat layer thickness, or/and reducing stratum corneum thickness.

In certain embodiments, the pharmaceutical composition is capable of reducing the degree of pruritus, protecting the appendages of skin, reducing the proliferation of mast cells, mediating the imbalance of Th1/Th2 cells, reducing the intensity of IgE expression of CD19 positive cells, improving allergic disease, and/or inhibiting inflammatory response.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain preferred embodiments, the administration is performed by subcutaneous injection or subcutaneous spot injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

In certain embodiments, the pharmaceutical composition also comprises a biological antibody (for example, but not limited to, dupilum-ab, omalizumab).

Burn or scald

Burn: It generally refers to a tissue damage caused by heat, including hot liquid (water, soup, oil, etc.), steam, high-temperature gas, flame, hot metal liquid or solid (e.g., molten steel, steel ingot), etc., mainly involving skin and/or mucous membranes, and in severe cases, subcutaneous or/and submucosal tissues, such as muscles, bones, joints and even internal organs, may also be injured. Scald is a tissue damage caused by hot liquid, steam, etc., and is a type of thermal burn.

Scald: It is a tissue damage caused by flameless high-temperature liquid (boiling water, hot oil, molten steel), high-temperature solid (heated metal, etc.) or high-temperature steam. Low-heat scalds are common, and low-heat scalds are also known as low-temperature scalds, which are scalds caused by prolonged exposure of skin to a low-heat object that is higher than the body temperature.

Burns often cause large-scale skin damage, resulting in loss of skin barrier function and disturbance of internal environment balance, and wound healing takes a long time. Clinical treatment often requires large-area skin transplantation, but burn patients have limited skin, and there is secondary damage during skin extraction; wound infection may lead to various complications such as difficult wound healing and septic shock, progressive deepening of infected and necrotic wounds; and scar healing after wound healing may lead to contracture deformities, resulting in unsightly appearance and functional obstacles. The prognosis of patients is poor, the function recovery is poor, and the later rehabilitation treatment increases the psychological and economic burden of patients. Therefore, finding a method that can promote wound healing faster and restore the appearance and function of skin better has become the problem to be solved in the field of burn.

So far, although skin injury has been treated by autograft of skin or grafting of artificial skin, it is still insufficient in large-area burns and scalds. The emergence of mesenchymal stem cells and the combined treatment with materials have brought some hope for skin injury.

In one aspect, the present invention relates to use of the mesenchymal stem cell population, the culture, the culture supernatant or the pharmaceutical composition as described herein for preventing and/or treating burn or scald in a subject, or in the manufacture of a medicament for preventing and/or treating burn or scald in a subject.

In certain embodiments, the pharmaceutical composition is capable of being used to solve the problems of no functional recovery after skin injury, limited source of skin graft, limited autologous skin, and the like.

In a certain embodiment, the pharmaceutical composition is capable of regenerating appendages, accelerating wound healing, reducing fibrosis, etc. after skin injury, thereby restoring skin function, reducing wound area, treating skin injury, and protecting skin.

In a certain embodiment, the pharmaceutical composition is capable of reducing inflammation at the wound site after scalding, thereby inhibiting inflammation.

In certain embodiments, the pharmaceutical composition is capable of promoting vascular regeneration in skin wound.

In certain embodiments, the pharmaceutical composition is capable of promoting hair follicle regeneration and upregulating factors such as β-Catenin, CD133, Ki67 and the like.

In certain embodiments, the pharmaceutical composition is capable of reducing collagen deposition, thereby treating skin damage.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the culture supernatant of the present invention, or a pharmaceutical composition comprising the culture supernatant can be administrated by intradermal injection, subcutaneous injection, intramuscular injection, intravenous injection, oral administration, etc. In some preferred embodiments, the pharmaceutical composition is administrated by topical application, surface implantation or injection, or surface spray.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, medicament can be transplanted in the form of a suspension, gel, colloid, slurry or mixture.

Refractory skin injury

Refractory skin injury is a phenomenon of skin damage caused by various diseases or injuries, which is manifested by repeated skin ulceration, loss of partial skin function, and easy generation of scars and other skin hyperplasia tissues. Common causes of refractory skin injury include burns, diabetes (leading to diabetic foot), lupus erythematosus, and psoriasis.

Diabetic foot: The main symptoms of diabetic foot disease are lower extremity pain and skin ulcers. Diabetic foot ulcers and gangrene are the main causes of clinical non-traumatic amputation, and also seriously endanger the working ability and life quality of diabetic patients. Diabetic foot is usually the result of a combination of lower extremity neuropathy, vascular disease, and infection.

Diabetic complications

1. Diabetic nephropathy

It is one of the most important comorbidities in diabetic patients. Its incidence rate in China is also on the rise, and it has become the second cause of end-stage renal disease, second only to various glomerulonephritis. Because of its complex metabolic disorders, once it progresses to end-stage renal disease, it is often more difficult to treat than other kidney diseases. However, active and appropriate intervention measures can significantly reduce and delay the occurrence of diabetic nephropathy, especially in the early stage of the disease course.

2. Diabetic eye complications

(1) Diabetic retinopathy is the most important manifestation of diabetic microangiopathy, an ocular fundus disease with specific changes, and one of the serious complications of diabetes. Clinically, depending on the presence or absence of retinal neovascularization, the diabetic retinopathy without retinal neovascularization is called nonproliferative diabetic retinopathy (or simple type or background type), and the diabetic retinopathy with retinal neovascularization is called proliferative diabetic retinopathy.

(2) Diabetes-related uveitis generally roughly comprises the following four conditions: (i) uveitis related to diabetes itself; (ii) infectious uveitis, in which the chance of endogenous infectious endophthalmitis in diabetic patients is significantly higher than that in normal people; (iii) uveitis accompanied by some specific types, in which the two are accidental coincidences, or there is an inherent connection; (iv) infectious endophthalmitis or aseptic endophthalmitis after intraocular surgery. It mostly occurs in middle-aged and elderly patients with diabetes.

(3) Diabetic cataract occurs in juvenile diabetic patients whose blood sugar is not well controlled. It mostly occurs in both eyes, develops rapidly, and may even develop into complete opacity within days, weeks, or months.

3. Diabetic foot

Foot is a complex target organ for diabetes, a multisystem disease. Due to the combination of peripheral neuropathy and peripheral vascular disease in diabetic patients, excessive mechanical pressure may cause the destruction and deformity of the soft tissue and bone and joint systems of the foot, and then lead to a series of foot problems, ranging from mild neurological symptoms to severe ulcers., infection, vascular disease, Charcot arthropathy, and neuropathic fractures. In fact, similar pathological changes may also occur in the upper limbs, face and trunk, but the incidence of diabetic foot is significantly higher than those of other parts.

4. Diabetic cardiovascular complication

It includes microvascular disease on heart and large vessels, cardiomyopathy, cardiac autonomic neuropathy, and is the leading cause of death in diabetic patients. Coronary heart disease is the main macrovascular complication of diabetes. Studies have shown that the risk of death from coronary heart disease in diabetic patients is 3 to 5 times higher than that in non-diabetic patients. The pathological mechanism is atherosclerosis, and high blood sugar, high systolic pressure, high cholesterol, increased low-density lipoprotein, decreased high-density lipoprotein, age, sex, smoking, and family history are all risk factors for its onset.

5. Diabetic cerebrovascular disease

It refers to intracranial macrovascular and microvascular lesions caused by diabetes. According to statistics, 20% to 40% of patients with type 2 diabetes will develop cerebrovascular disease, mainly manifested as cerebral arteriosclerosis, ischemic cerebrovascular disease, cerebral hemorrhage, brain atrophy, etc., and thus it is one of the main causes of death in patients with diabetes.

6. Diabetic neuropathy

The most common type of diabetic neuropathy is chronic distal symmetrical sensorimotor polyneuropathy, that is diabetic peripheral neuropathy, and it has a high incidence. Some patients already have peripheral neuropathy when they are newly diagnosed with diabetes. Unfortunately, in terms of treatment, especially in the radical cure of diabetic neuropathy, it is quite difficult, so the focus is on preventing its occurrence and controlling its development.

Refractory skin injury is not a disease, but a phenomenon of skin damage caused by a variety of diseases or injuries, which is manifested in easy and repeated ulceration of skin, loss of partial skin function, easy generation of scars and other skin hyperplasia tissues. Common factors that lead to refractory skin injury include burns and scalds, diabetes, lupus erythematosus, and psoriasis. At present, there is no one-size-fits-all solution to these problems, because such damage is often accompanied by complex immune disorders and tissue regeneration disorders, and a single treatment plan cannot solve all problems.

In one aspect, the present invention relates to use of the mesenchymal stem cell population, the culture, the culture supernatant or the pharmaceutical composition as described herein for the prevention and/or treatment of refractory skin injury in a subject, or in the manufacture of a medicament for preventing refractory skin injury, or delaying, or alleviating or preventing scleroderma, or alleviating symptoms of refractory skin injury in a subject. In some cases, the refractory skin injury results from these factors (for example, but not limited to burns and scalds, diabetes, lupus erythematosus, psoriasis, etc.)

In certain preferred embodiments, the administration may be performed by subcutaneous injection.

In certain embodiments, the pharmaceutical composition is capable of accelerating healing speed of wounds, reducing the wound area, promoting the regeneration of blood vessels in skin wounds, and making the skin regenerate after injury, during the treatment of refractory skin injury.

In certain embodiments, the pharmaceutical composition is capable of reducing the expression of CD3, F4/80, MPO gene or protein, and inhibiting inflammation during the treatment of refractory skin injury.

In certain embodiments, the pharmaceutical composition is capable of increasing the expression of $\beta$-Catenin, CD133, Ki67, CD31 gene or protein during the treatment of refractory skin injury.

In certain embodiments, the pharmaceutical composition is capable of promoting hair follicle regeneration.

In certain embodiments, the pharmaceutical composition is capable of reducing the expression of proinflammatory factors IL-1$\beta$, IL-6 and TNF$\alpha$ in diabetic nephropathy, reducing mesangial thickening and macrophage infiltration, reducing diabetes-induced glomerulopathy, increasing kidney weight, kidney and body mass index in rats, so that it may have a good therapeutic effect on diabetic nephropathy.

In certain embodiments, the pharmaceutical composition is capable of accelerating the healing of diabetic foot, reducing the inflammation of skin wounds, promoting the regeneration of blood vessels and hair follicles, reducing the deposition of collagen, and inhibiting the occurrence of fibrosis in diabetic foot, so that it may effectively treat skin injury.

In certain embodiments, the pharmaceutical composition is capable of reducing blood sugar and regulating inflammatory response in diabetic eye complication, significantly reducing fasting blood glucose and HbAlc levels, and improving visual function and macular edema to a certain extent, so that it may treat diabetic eye complication well.

In certain embodiments, the pharmaceutical composition is capable of inhibiting vascular calcification in vascular calcification complicated by diabetes, so that it has a good therapeutic effect on the vascular calcification disease in the complications.

In certain embodiments, the pharmaceutical composition is capable of enhancing the ability of astrocytes to resist oxidative stress in diabetic neuropathy, enhancing their ability to clear intracerebral glutamate and maintain intracerebral K+ balance, thereby promoting neuronal function, brain homeostasis and synapse formation, and improving cognitive impairment caused by diabetes.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain preferred embodiments, the administration is performed by subcutaneous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

Psoriasis

Psoriasis is a common chronic inflammatory skin disease with intractable and recurrent characteristics. Its etiology is unknown, but it is currently believed to be a disease caused by the interaction of genetic factors, environmental factors and other factors.

Clinically, it is divided into four common types: vulgaris, pustular, joint, and erythrodermic. Its skin lesions are characterized by erythematous papules at first, covered with layers of silver-white scales on the surface, dry skin, desquamation and scabs, and some skin symptoms are linked together, like a map, and some cases have itching, pus and water, and blood stains that are unbearable to see.

Psoriasis vulgaris is very obvious because of red papules caused by dermatitis, which have size of mung bean, then slowly grow to form silvery-white dry scales. In severe cases, large white scales will cover the body and look particularly scary. It may even be accompanied by bleeding, which is unacceptable.

Pustular type: There are very dense water pustules that vary in size. With the aggravation of the disease, the pustules will continue to grow, and finally form erythema. This symptom is an emergency and comes on suddenly. With this type of psoriasis, people have a fever and experience joint pain and swelling.

Erythrodermic psoriasis: It is manifested in diffuse flushing, infiltration and swelling of the whole body, accompanied by a large number of bran-like scales, during which there may be flaky normal skin, and may be accompanied by systemic symptoms such as fever, superficial lymphadenopathy, etc. The course of the disease is long and it is easy to relapse.

Arthritis psoriasis: In addition to skin lesions, joint lesions may occur, and any joint may be affected, including the elbows, greater knee joints, small finger and toe joints, spine and sacroiliac joints. It can be manifested as joint swelling and pain, limited movement, joint deformity in severe cases, and progressive development, but rheumatoid factor tests are often negative.

Psoriasis (commonly known as psora) is a well-known skin disease. Once it occurs, red papules or plaques may appear on the skin, and are covered with multiple layers of silvery white scales. It tends to occur on the limbs, the head and back, and even the whole body, and some cases last almost a lifetime. There is currently no effective treatment. The disease mainly affects young and middle-aged people, has a great impact on the physical health and mental status of patients, and has caused a huge burden on the society and economy. Epidemiological surveys show that there are currently about 6.5 million psoriasis patients in China, with an incidence rate of 0.47%.

At present, psoriasis is considered to be an autoimmune skin disease caused by the domination of dendritic cells (DC) and T lymphocytes, the participation of innate and adaptive immunity, and the interaction of genetic background and environmental factors. The characteristic lesions of psoriasis include excessive proliferation of keratinocytes caused by inflammatory conditions, and so on. Antagonistic biological agents targeting key cytokines (TFN-$\alpha$, IL-12, IL-23, IL-17) in the pathogenesis of psoriasis are extremely effective in clinical treatment, but the high costs for maintaining long-term treatment and the potential serious adverse reactions limit the wide application of such biological agents.

In one aspect, the present invention relates to use of the mesenchymal stem cell population, the culture, the culture supernatant or the pharmaceutical composition as described herein for the prevention and/or treatment of psoriasis in a subject, or in the manufacture of a medicament for preventing psoriasis, or delaying, or alleviating psoriasis, or preventing and alleviating psoriasis in a subject.

In certain embodiments, the pharmaceutical composition is capable of alleviating erythema rash, alleviating scale, alleviating infiltration, reducing psoriatic dermatitis phenotype, reducing psoriatic lesion, reducing spinous layer of epidermis, or reducing stratum corneum thickness.

In certain embodiments, the pharmaceutical composition is capable of reducing ROS level, reducing the recruitment of splenic neutrophil cells and dendritic cells, reducing inflammatory infiltrating cells, or/and modulating immune function.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain preferred embodiments, the administration is performed by back or intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

## Autoimmune disease

Immune system disease refers to a pathological reaction caused by an immune system damage. It mainly includes: infectious disease, hypersensitivity disease, autoimmune disease, immunoproliferative disease, immunodeficiency disease and immune-related disease. As used herein, the term "autoimmune disease" refers to a disease in which an immune system malfunction causes the body to attack its own tissues. Common autoimmune diseases often involve multiple systems and organs (e.g., skin, bones, muscles, internal organs, etc.), thus forming systemic autoimmune diseases, including systemic lupus erythematosus, ankylosing spondylitis, rheumatoid arthritis, psoriasis, erythroderma, glomerulonephritis, Anca-associated vasculitis, scleroderma, primary systemic amyloidosis, autoimmune hepatitis, autoimmune pancreatitis, autoimmune gastritis, Crohn's disease, ulcerative colitis, erythema nodosum, Hashimoto's thyroiditis, alopecia areata, eczema, type 1 diabetes.

In one aspect, the present invention provides a use of the mesenchymal stem cell population or its culture supernatant in the manufacture of a medicament for preventing, treating, delaying and/or alleviating an autoimmune disease; alternatively, the present invention provides a method for preventing, treating, delaying and/or alleviating an autoimmune disease, which comprises administering to a subject in need thereof a prophylactically and/or therapeutically effective amount of the mesenchymal stem cell population or its culture supernatant.

In certain embodiments, the autoimmune disease is selected from the group consisting of scleroderma, lupus erythematosus (e.g., systemic lupus erythematosus), psoriasis, rheumatoid arthritis, dermatomyositis, multiple sclerosis, myasthenia gravis, polymyositis, inflammatory bowel disease (e.g., ulcerative colitis (UC), Crohn's disease (CD)), Sjogren's syndrome, vasculitis (e.g., systemic vasculitis), adult Still's disease, or any combination thereof.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with an additional therapeutic agent. In certain embodiments, the mesenchymal stem cell population and the additional active component are present as separate components or as a single formulation.

In certain embodiments, the additional active component is selected from the group consisting of anti-inflammatory drug or immunosuppressive agent. In certain embodiments, the additional active component is selected from the group consisting of non-steroidal anti-inflammatory drug (e.g., ibuprofen, diclofenac, naproxen, indomethacin, piroxicam, meloxicam, nabumetone or nimesulide), steroidal anti-inflammatory drug (e.g., prednisone, dexamethasone, or hydrocortisone), antibody or antagonist of proinflammatory cytokine (e.g., antibody or receptor antagonist of TNFα, IL-1, IL-6, IL-8, GM-CSF, or PAF), anti-inflammatory cytokine (e.g., IL-10, IL-4, IL-11, IL-13 or TGFβ), antiproliferative/antimetabolite drug (e.g., cyclophosphamide, methotrexate, azathioprine, leflunomide), calcineurin inhibitor (e.g., cyclosporine, tacrolimus), or any combination thereof.

In certain embodiments, the medicament is in an unit dosage form, and the unit dose of the medicament contains the mesenchymal stem cells in an amount of no less than $1 \times 10^4$ cells (e.g., no less than $1 \times 10^4$ cells, no less than $3 \times 10^4$ cells, no less than $5 \times 10^4$ cells, no less than $7 \times 10^4$ cells, no less than $1 \times 10^5$ cells, no less than $3 \times 10^5$ cells, no less than $5 \times 10^5$ cells, no less than $7 \times 10^5$ cells, no less than $1 \times 10^6$ cells, no less than $3 \times 10^6$ cells, no less than $5 \times 10^6$ cells, no less than $7 \times 10^6$ cells, no less than $1 \times 10^7$ cells, no less than $3 \times 10^7$ cells, no less than $5 \times 10^7$ cells, no less than $7 \times 10^7$ cells, no less than $1 \times 10^8$ cells, no less than $3 \times 10^8$ cells, no less than $5 \times 10^8$ cells, no less than $7 \times 10^8$ cells, no less than $1 \times 10^9$ cells, no less than $3 \times 10^9$ cells, no less than $5 \times 10^9$ cells, no less than $7 \times 10^9$ cells, no less than $1 \times 10^{10}$ cells, no less than $3 \times 10^{10}$ cells, no less than $5 \times 10^{10}$ cells or no less than $7 \times 10^{10}$ cells). In certain embodiments, the unit dose of the medicament contains the mesenchymal stem cell in an amount of $1 \times 10^5$ to $1 \times 10^8$ cells, (e.g., $1 \times 10^6$ to $1 \times 10^8$ cells, $1 \times 10^6$ to $1 \times 10^7$ cells, or $1 \times 10^6$ to $5 \times 10^6$ cells).

In another aspect, the present invention also provides a product for preventing, treating, delaying and/or alleviating an autoimmune disease, which comprises the mesenchymal stem cell population of the present invention. In certain embodiments, the product further comprises an additional active component as defined above. In certain embodiments, the mesenchymal stem cell population and the additional active component are present as separate components or as a single formulation. In certain embodiments, the product is an injection, microinjection, mucosal patch, enema, suppository, gel, oral preparation, aerosol, drop, ointment, implant, or capsule. In certain embodiments, the product is an implant.

## Lupus erythematosus

Lupus erythematosus is a typical autoimmune connective tissue disease, more common in women aged 15 to 40. Lupus erythematosus is a spectrum disease that can be divided into several subtypes such as discoid lupus erythematosus (DLE), subacute cutaneous lupus erythematosus (SCLE), systemic lupus erythematosus (SLE), lupus erythematosus profundus (LEP), neonatal lupus erythematosus (NLE) and drug-induced lupus erythematosus (DIL). The term "systemic lupus erythematosus" refers to an autoimmune disease with slow onset, insidious onset, and diverse and varied clinical manifestations, involving many systems and organs, in which multiple autoantibodies are generated due to cellular and humoral immune dysfunctions. It may affect the skin, serosa, joint, kidney and central nervous system, and is characterized by autoimmunity. There are a variety of autoantibodies in patients, which not only affect humoral immunity, but also affect cellular immunity, and the complement system also changes.

In one aspect, the present invention provides a use of the mesenchymal stem cell population or its culture supernatant in the manufacture of a medicament for preventing, treating, delaying and/or alleviating a lupus erythematosus (e.g., systemic lupus erythematosus); alternatively, the present invention provides a method for preventing, treating, delaying and/or alleviating a lupus erythematosus (e.g., systemic lupus erythematosus), which comprises administering to a subject in need thereof a prophylactically and/or therapeutically effective amount of the mesenchymal stem cell population or its culture supernatant.

In certain embodiments, the mesenchymal stem cell population of the present invention are capable of slowing the systemic pathogenetic process by reducing an anti-double-stranded DNA antibody.

In certain embodiments, the mesenchymal stem cell population of the present invention is capable of slowing the pathogenetic process of lupus erythematosus by avoiding or preventing or inhibiting the enlargement of spleen and nuchal lymph node.

In certain embodiments, the mesenchymal stem cell population of the present invention is capable of promoting the formation of glomeruli.

In certain embodiments, the mesenchymal stem cell population of the present invention is capable of inhibiting a proinflammatory factor.

In certain embodiments, the mesenchymal stem cell population of the present invention is capable of reducing the number of T cell populations (e.g., CD3$^+$ T cells, CD4$^+$ T cells, and CD4$^+$ T cells) in spleen.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the mesenchymal stem cell population of the present invention can be administrated by intradermal injection, subcutaneous injection, intramuscular injection, intravenous injection, oral administration, etc. In certain preferred embodiments, the administration is performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the additional active component is selected from the group consisting of anti-inflammatory drugs or immunosuppressive agent. In certain embodiments, the additional active component is selected from the group consisting of non-steroidal anti-inflammatory drug (e.g., ibuprofen, diclofenac, naproxen, indomethacin, piroxicam, meloxicam, nabumetone or nimesulide), steroidal anti-inflammatory drug (e.g., prednisone, dexamethasone, or hydrocortisone), antibody or antagonist of proinflammatory cytokine (e.g., antibody or receptor antagonist of TNF$\alpha$, IL-1, IL-6, IL-8, GM-CSF, or PAF), anti-inflammatory cytokine (e.g., IL-10, IL-4, IL-11, IL-13 or TGF$\beta$), antiproliferative/antimetabolite rug (e.g., cyclophosphamide, methotrexate, azathioprine, leflunomide), calcineurin inhibitor (e.g., cyclosporine, tacrolimus), or any combination thereof.

In certain embodiments, the medicament is in an unit dosage form, and the unit dose of the medicament contains the mesenchymal stem cells in an amount of no less than $1\times10^4$ cells (e.g., no less than $1\times10^4$ cells, no less than $3\times10^4$ cells, no less than $5\times10^4$ cells, no less than $7\times10^4$ cells, no less than $1\times10^5$ cells, no less than $3\times10^5$ cells, no less than $5\times10^5$ cells, no less than $7\times10^5$ cells, no less than $1\times10^6$ cells, no less than $3\times10^6$ cells, no less than $5\times10^6$ cells, no less than $7\times10^6$ cells, no less than $1\times10^7$ cells, no less than $3\times10^7$ cells, no less than $5\times10^7$ cells, no less than $7\times10^7$ cells, no less than $1\times10^8$cells, no less than $3\times10^8$ cells, no less than $5\times10^8$ cells, no less than $7\times10^8$ cells, no less than $1\times10^9$ cells, no less than $3\times10^9$ cells, no less than $5\times10^9$ cells, no less than $7\times10^9$ cells, no less than $1\times10^{10}$ cells, no less than $3\times10^{10}$ cells, no less than $5\times10^{10}$ cells or no less than $7\times10^{10}$ cells). In certain embodiments, the unit dose of the medicament contains the mesenchymal stem cell in an amount of $1\times10^4$ to $1\times10^{10}$ cells, (e.g., $1\times10^6$ to $1\times10^8$ cells, $1\times10^6$ to $1\times10^7$ cells, or $1\times10^6$ to $5\times10^6$ cells).

Scleroderma

The skin is the organ with the largest surface area in the human body. It is a key structure in protecting internal tissues from mechanical damage, microbial infection, UV radiation and extreme temperatures. Skin system diseases include viral skin disease, bacterial skin disease, fungal skin disease, animal skin disease, physical skin disease, dermatitis, eczema, drug eruption, urticarial skin disease, pruritic skin disease, erythematous scaly skin disease, connective tissue disease, bullous skin disease, vasculitic skin disease, skin appendage disease, skin pigment disorder, hereditary skin disease, skin tumor, sexually transmitted disease.

Scleroderma is a type of skin connective tissue disease. Scleroderma or systemic sclerosis (SSC), is a progressive, debilitating autoimmune disease characterized by the deposition of excess protein in the extracellular matrix by skin fibroblasts, also known as skin fibrosis. Typical skin lesions go through three stages: swelling, infiltration and atrophy in sequence. The lesions are symmetrical, and the lesions mostly extend from the fingers to the proximal end, and involve the connective tissues of internal organs such as the heart, lungs, kidneys, and digestive tract.

Scleroderma is an autoimmune disease characterized by skin thickening and localized or diffuse fibrosis, which can affect the lungs, kidneys, liver, heart and other organs. Its pathogenesis is unknown. Current studies have found that the disease mainly involves three aspects: small vessel disease, fibrosis caused by excessive accumulation of extracellular matrix, and immune abnormality. Inflammatory cell infiltration is the main feature in the early stage of scleroderma, mainly T lymphocyte infiltration. Studies have shown that T lymphocytes can release a variety of cytokines, causing inflammation and vascular lesions, activating fibroblasts and promoting the synthesis of collagen fibers. At present, immunosuppressive agents and symptomatic treatment are mainly used for scleroderma, but the treatment effect is not ideal, and there are many adverse reactions, so it is necessary to find more effective treatment methods.

In one aspect, the present invention provides a use of the mesenchymal stem cell population or its culture supernatant in the manufacture of a medicament for preventing, treating, delaying and/or alleviating a scleroderma; alternatively, the present invention provides a method for preventing, treating, delaying and/or alleviating a scleroderma, which comprises administering to a subject in need thereof a prophylactically and/or therapeutically effective amount of the mesenchymal stem cell population or its culture supernatant.

In certain embodiments, the mesenchymal stem cell population is capable of significantly thinning dermis and reducing collagen fiber accumulation in scleroderma.

In certain embodiments, the mesenchymal stem cell population is capable of reducing the occurrence of skin sclerosis and thickening in scleroderma, and has an effective therapeutic effect on scleroderma.

In certain embodiments, the mesenchymal stem cell population is capable of increasing the number of hair follicles and reducing dermis thickness in scleroderma.

In certain embodiments, the mesenchymal stem cell population is capable of preventing the fat layer from significantly thinning in scleroderma, and preventing the reduction of skin appendages.

In certain embodiments, the mesenchymal stem cell population is capable of inhibiting inflammatory factor (e.g., IL-17, IL-6, TNF), inhibiting expression level of inflammatory factor, or/and increasing anti-inflammatory factor expression level (e.g., IL10), increasing MMP1 protein expression level, and reducing or inhibiting smooth muscle actin (a-SMA) expression in scleroderma.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the mesenchymal stem cell population of the present invention can be administrated by intradermal injection, subcutaneous injection, intramuscular injection, intravenous injection, oral administration, etc. In certain preferred embodiments, the administration is performed by subcutaneous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the additional active component is selected from the group consisting of anti-inflammatory drugs or immunosuppressive agent. In certain embodiments, the additional active component is selected from the group consisting of non-steroidal anti-inflammatory drug (e.g., ibuprofen, diclofenac, naproxen, indomethacin, piroxicam, meloxicam, nabumetone or nimesulide), steroidal anti-inflammatory drug (e.g., prednisone, dexamethasone, or hydrocortisone), antibody or antagonist of proinflammatory cytokine (e.g., antibody or receptor antagonist of TNF$\alpha$, IL-1, IL-6, IL-8, GM-CSF, or PAF), anti-inflammatory cytokine (e.g., IL-10, IL-4, IL-11, IL-13 or TGF$\beta$), antiproliferative/antimetabolite rug (e.g., cyclophosphamide, methotrexate, azathioprine, leflunomide), calcineurin inhibitor (e.g., cyclosporine, tacrolimus), or any combination thereof.

In certain embodiments, the medicament is in an unit dosage form, and the unit dose of the medicament contains the mesenchymal stem cells in an amount of no less than $1\times10^4$ cells (e.g., no less than $1\times10^5$ cells, no less than $5\times10^5$ cells, no less than $1\times10^6$ cells, no less than $2\times10^6$ cells, no less than $3\times10^6$ cells, no less than $4\times10^6$ cells, no less than $5\times10^6$ cells, no less than $6\times10^6$ cells, no less than $7\times10^6$ cells, no less than $8\times10^6$ cells, no less than $9\times10^6$ cells, no less than $1\times10^7$ cells, no less than $3\times10^7$ cells, no less than $5\times10^7$ cells, no less than $7\times10^7$ cells, no less than $1\times10^8$ cells, no less than $3\times10^8$ cells, no less than $5\times10^8$ cells, no less than $7\times10^8$ cells). In certain embodiments, the unit dose of the medicament contains the mesenchymal stem cell in an amount of $1\times10^5$ to $1\times10^8$ cells, (e.g., $1\times10^6$ to $1\times10^8$ cells, $1\times10^6$ to $1\times10^7$ cells, or $1\times10^6$ to $5\times10^6$ cells).

**Respiratory disease**

In one aspect, the present invention provides use of the mesenchymal stem cell population or its culture supernatant in the manufacture of a medicament for preventing and/or treating a respiratory disease; alternatively, the present invention provides a method for preventing and/or treating a respiratory disease, which comprises administering to a subject in need thereof a prophylactically and/or therapeutically effective amount of the mesenchymal stem cell population or its culture supernatant.

Respiratory system is a general term for a series of organs that relate to gas exchange between the human body and the outside air, including tissues such as nose, pharynx, larynx, trachea, bronchi, and lungs composed of a large number of alveoli, blood vessels, lymphatic vessels, nerves, and pleura. Clinically, the nose, pharynx and larynx are often referred to as the upper respiratory tract, and the part of gas passages below the trachea (including the bronchi at all levels in the lungs) is referred to as the lower respiratory tract.

Pulmonary disease refers to a disease of the lung itself or a pulmonary manifestation of a systemic disease. It mainly includes infectious lung disease, air pollution and smoking-related lung disease, occupation-related lung disease, immune-related lung disease, genetic-related lung disease, and unexplained lung disease. In some embodiments, the pulmonary disease is selected from the group consisting of pulmonary vascular disease, idiopathic pulmonary fibrosis, acute respiratory distress, pneumoconiosis and pneumonia. In some embodiments, the pulmonary vascular disease is selected from the group consisting of pulmonary hypertension, cor pulmonale, pulmonary embolism, pulmonary vasculitis, chronic obstructive pulmonary disease, and interstitial pulmonary disease. In some embodiments, the pulmonary disease is pulmonary arterial hypertension (PAH).

Occupation-related lung disease:

It refers to a lung disease, such as pneumoconiosis, caused by lung damage induced by inhaling harmful dust, fume or poison in a certain occupation. Hazardous dust includes silica (i.e., quartz), silicate, coal, iron, and tin. Fume includes sulfur dioxide, nitrogen dioxide, ammonium, hydrochloric acid, chlorine, phosgene and other harmful gas and strong acid fume. Toxic substance includes uranium, nickel, chromate, asbestos, dichloromethyl ether, etc.

Pneumoconiosis:

Pneumoconiosis is a systemic disease mainly manifested by diffuse fibrosis (scar) of lung tissue induced by long-term inhalation of productive dust (ash) during occupational activities and retention thereof in the lungs. Pneumoconiosis can be divided into inorganic pneumoconiosis and organic pneumoconiosis according to the type of inhaled dust. Pneumoconiosis caused by inhalation of inorganic dust in production labor is called inorganic pneumoconiosis. Most of the pneumoconiosis is inorganic pneumoconiosis. Pneumoconiosis caused by inhalation of organic dust is called organic pneumoconiosis, such as cotton pneumoconiosis and farmers' lung.

Pneumoconiosis is a progressive chronic disease. Unlike acute infectious diseases or other chronic diseases (e.g., tuberculosis, hypertension, diabetes, etc.), in which obvious therapeutic effects can be observed in a short period of time, it generally requires long-term treatment for several years to obtain more obvious curative effect.

In one aspect, the present invention relates to use of the mesenchymal stem cell population, the culture, the culture supernatant or the pharmaceutical composition as described herein in the manufacture of a medicament for preventing and/or treating a pneumoconiosis, or delaying, or reducing, or preventing or alleviating a pneumoconiosis; alternatively, the present invention relates to a method for preventing and/or treating a pneumoconiosis, or delaying, or reducing, or preventing or alleviating a pneumoconiosis, which comprises administering to a subject in need thereof an effective amount of the mesenchymal stem cell population, the culture, the culture supernatant or the pharmaceutical composition as described therein.

In some embodiments, the medicament of the present invention is capable of reducing an inflammatory factor level in serum, improving lung function, reducing lung compact area, and/or reducing fibrosis formation.

In some embodiments, the subject is a mammal, such as a human.

In some embodiments, the medicament comprises a therapeutically effective amount of the mesenchymal stem cell population and/or the culture and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some preferred embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises the aforementioned biological scaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the medicament can be transplanted in the form of a suspension, gel, colloid, serous fluid or mixture.

Pneumonia:

Pneumonia refers to an infectious inflammation of alveoli, distal airways, and pulmonary interstitium, which can be caused by bacterial, viral, and other pathogens, among which bacterial and viral pneumonia are the most common. In a broad sense, pneumonia can be caused by pathogenic microorganisms, physicochemical factors, immune damage, allergies, and drugs. Patients often have typical symptoms such as fever, cough, and difficulty breathing.

Emphysema:

Emphysema is a pathological state in which the airway elasticity of distal terminal bronchioles are reduced, hyperinflated, inflated, and increased in lung volume or accompanied by airway wall destruction. According to its etiology, emphysema has the following types: senile emphysema, compensatory emphysema, interstitial emphysema, focal emphysema, paraseptal emphysema, obstructive emphysema.

Bronchitis:

Bronchitis is a chronic non-specific inflammation of trachea, bronchial mucosa and surrounding tissues thereof. The main cause of bronchitis is chronic non-specific inflammation of the bronchi due to repeated infections by viruses and bacteria. It mainly includes acute bronchitis and chronic bronchitis.

Chronic bronchitis:

Chronic bronchitis is a chronic non-specific inflammation of trachea, bronchial mucosa and surrounding tissues thereof. The main symptoms are cough, expectoration, or wheezing.

Chronic obstructive pulmonary disease:

Chronic obstructive pulmonary disease is a chronic bronchitis and/or emphysema characterized by airflow obstruction, which can further develop into common chronic diseases such as cor pulmonale and respiratory failure. It is related to the abnormal inflammatory response to harmful gases and harmful particles, and has a high disability rate and fatality rate.

In one aspect, the present invention relates to use of the mesenchymal stem cell population, the culture, the culture supernatant or the pharmaceutical composition as described herein, in the manufacture of a medicament for preventing and/or treating a chronic pulmonary obstruction, or delaying, or reducing a chronic pulmonary obstruction, or preventing or alleviating a chronic pulmonary obstruction; alternatively, the present invention relates to a method for preventing and/or treating a chronic pulmonary obstruction, or delaying, or reducing, or preventing or alleviating a chronic pulmonary obstruction, which comprises administering to a subject in need thereof an effective amount of the mesenchymal stem cell population, the culture, the culture supernatant or the pharmaceutical composition as described therein.

In some embodiments, the medicament of the present invention is capable of reducing lung compact area, improving a lung function including vital capacity, improving maximal ventilation, reducing airway resistance, reducing mean alveolar intercept, maintaining lung structural integrity, or/and increasing arterial blood oxygen partial pressure.

In some embodiments, the medicament is capable of reducing a proinflammatory factor level, increasing an anti-inflammatory factor level, and inhibiting inflammation.

In some embodiments, the medicament is capable of reducing expression levels of Collagen I and $\alpha$-SMA proteins in lung, and is capable of inhibiting fibrosis occurrence.

In some embodiments, the subject is a mammal, such as a human.

In some embodiments, the medicament comprises a therapeutically effective amount of the mesenchymal stem cell population and/or the culture and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. In some preferred embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises the aforementioned biological scaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the medicament can be transplanted in the form of a suspension, gel, colloid, serous fluid or mixture.

Immune-related lung disease:

Immune-related lung disease refers to a protective immune and allergic response in lungs when the lungs are attacked by external allergens. It is mainly manifested in acute, subacute or chronic interstitial pneumonia.

Infectious lung disease:

Infectious lung disease refers to a disease caused by pathogenic microorganism infection in lungs. It is mainly divided into bacterial pneumonia, viral pneumonia, mycoplasma pneumonia, fungal pneumonia and tuberculosis. Bacterial pneumonia is a pneumonia caused by bacterial infection, including pneumonia caused by *Streptococcus pneumoniae, Staphylococcus aureus, Gram-negative bacilli* and other infections. Viral pneumonia is a pneumonia caused by viral infection in upper respiratory tract, mainly including influenza, pharyngitis, atypical pneumonia (SARS), Middle East respiratory syndrome (MERS) and novel coronavirus pneumonia (COVID-19), which are caused by influenza virus, parainfluenza virus, cytomegalovirus, adenovirus, rhinovirus, coronavirus (SARS virus, MERS virus, and new coronavirus) and some enteroviruses, etc. Mycoplasma pneumonia is a pneumonia caused by mycoplasma pneumoniae. Fungal pneumonia includes pneumonia caused by fungi such as *Aspergillus.* Tuberculosis is a lung disease caused by infection with *Mycobacterium tuberculosis.*

Acute respiratory failure:

Acute respiratory failure is an acute respiratory failure caused by hypoventilation that is induced by a respiratory disease, such as severe respiratory infection, acute respiratory obstructive disease, severe or critical asthma, acute pulmonary edema of various causes, pulmonary vascular disease, thoracic trauma or surgical injury, pulmonary ventilation and/or ventilation dysfunction caused by spontaneous pneumothorax and acute increase of pleural effusion, acute intracranial infection, craniocerebral trauma, cerebrovascular disease and so on that directly or indirectly inhibit respiratory center, poliomyelitis, myasthenia gravis, organophosphate poisoning, cervical spine trauma and so on that damages the neuromuscular conduction system.

Respiratory distress syndrome (ARDS):

It is a type of acute respiratory failure, in which for various reasons, the fluid exchange dysfunction of pulmonary vascular tissue causes the increase of pulmonary water content, the decrease of lung compliance, the collapse of pulmonary alveoli, and the imbalance of ventilation to blood flow ratio. The typical symptoms are severe hypoxemia and extreme respiratory distress. It is mainly caused by internal and external factors such as severe infection, trauma and shock. Respiratory distress syndrome includes acute respiratory distress syndrome and neonatal respiratory distress syndrome.

The pneumonia "COVID-19" caused by the infection of novel coronavirus "SARS-CoV-2" has a long latent period, is highly contagious and highly harmful. Up to now, there is no effective treatment for COVID-19, but severe and critically ill patients with COVID-19 have poor prognosis and high mortality, and their clinical treatment needs are particularly urgent.

According to the latest epidemiological data, some patients of COVID-19 develop acute respiratory distress syndrome (ARDS), which leads to respiratory failure, and in turn affects the functions of other organs and even leads to death. ARDS manifests as a clinical syndrome of rapidly progressive dyspnea, hypoxemia, diffuse pulmonary infiltration, and respiratory failure. The current treatment options for ARDS are limited to symptomatic treatments such as basic medical care and supportive ventilation strategies, and are still unable to reverse the disease process, improve the life quality of patients, and reduce the mortality rate. Mechanical ventilation is the mainstay of treatment for patients with acute respiratory distress syndrome. In the process of mechanical ventilation, complications such as ventilator-related pneumonia, ventilator-related lung injury, deep vein thrombosis, difficulty in weaning from mechanical ventilation, and pulmonary fibrosis often occur. Drug treatment methods include: corticosteroids, statins, aspirin, β-2 receptor agonists, surfactants, and inhaled NO, all of which have not shown significant efficacy. The above two treatment methods, together with auxiliary methods such as blood purification treatment, nutritional intervention, and fluid control, cannot meet the treatment of ARDS caused by COVID-19.

In one aspect, the present invention relates to use of the mesenchymal stem cell population, the culture, the culture supernatant or the pharmaceutical composition as described herein, in the manufacture of a medicament for preventing and/or treating a respiratory distress syndrome, or delaying, or reducing a respiratory distress syndrome; alternatively, the present invention relates to a method for preventing and/or treating a respiratory distress syndrome, or delaying, or reducing, or preventing or alleviating a respiratory distress syndrome, which comprises administering to a subject in need thereof an effective amount of the mesenchymal stem cell population, the culture, the culture supernatant or the pharmaceutical composition as described herein.

In some embodiments, the medicament is capable of alleviating asthma, promoting absorption and improvement at lesion site, inhibiting inflammation, and/or restoring lung function.

In some embodiments, the medicament is capable of reducing a proinflammatory cytokine (e.g., IL-1$\alpha$, IL-1$\beta$, IL-5, IL-8, IL-25 and CXCL10/IP-10), increasing an anti-inflammatory cytokine (e.g., IL-1RA, RANTES) level.

In some embodiments, the subject is a mammal, such as a human.

In some embodiments, the medicament comprises a therapeutically effective amount of the mesenchymal stem cell population and/or the culture and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. Preferably, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises the aforementioned biological scaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Idiopathic Interstitial Pneumonia:

Idiopathic interstitial pneumonia (IIP), also known as idiopathic interstitial pulmonary fibrosis, in which idiopathic means unexplained cause, is a group of progressive lower respiratory tract diseases of unknown cause, and its pathological process is generally slow-progressive diffuse alveolitis and/or alveolar structural disorder, eventually leading to the destruction of alveolar structure, resulting in complete fibrosis in alveolar space and vesicular honeycomb lung. IIPs are divided into major IIPs, rare IIPs and unclassifiable IIPs. There are 6 major types of IIPs, including idiopathic pulmonary fibrosis (IPF), idiopathic non-specific interstitial pneumonia (iNSIP), respiratory bronchiolitis accompanied with interstitial lung disease (RB-ILD), desquamative interstitial pneumonia (DIP), cryptogenic organizing pneumonia (COP), acute interstitial pneumonia (AIP). There are two types of rare IIPs, including idiopathic lymphocytic interstitial pneumonia (iLIP) and idiopathic pleuropulmonary parenchymal fibroelastosis (iPPFE).

Idiopathic pulmonary fibrosis (IPF):

Idiopathic pulmonary fibrosis (IPF) is a chronic progressive lung disease mainly characterized by pulmonary interstitial fibrosis, and its cause is still unknown. It is the most common type of major idiopathic interstitial pneumonia. The disease is more common in the elderly, and its incidence has been on the rise in recent years. However, the diagnosis of IPF is still a clinical problem. The onset of IPF is insidious, there are often no obvious clinical manifestations in the early stage, and the imaging and pulmonary function manifestations are not typical. Therefore, patients with IPF are often diagnosed after the disease has progressed to multiple complications. However, there is currently no effective treatment for IPF, and the lung function of patients continues to deteriorate with the progression of the disease, and the median survival time is only 2 to 3 years.

In one aspect, the present invention relates to use of the mesenchymal stem cell population, the culture, the culture supernatant or the pharmaceutical compositions as described herein, in the manufacture of a medicament for preventing and/or treating an idiopathic pulmonary fibrosis, or delaying, or reducing an idiopathic pulmonary fibrosis, or preventing or alleviating an idiopathic pulmonary fibrosis; alternatively, the present invention relates to a method for preventing and/or treating an idiopathic pulmonary fibrosis, or delaying, or reducing an idiopathic pulmonary fibrosis, or preventing or alleviating an idiopathic pulmonary fibrosis, which comprises administering to a subject in need thereof an effective amount of the mesenchymal stem cell population, the culture, the culture supernatant or the pharmaceutical composition as described herein.

In some embodiments, the pharmaceutical composition is capable of promoting the absorption and improvement at pulmonary lesion site and reducing pulmonary fibrosis.

In some embodiments, the subject is a mammal, such as a human.

In some embodiments, the medicament comprises a therapeutically effective amount of the mesenchymal stem cell population and/or the culture and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some preferred embodiments, the medicament of the present invention can be administrated by intradermal injection, subcutaneous injection, intramuscular injection, intravenous injection, oral administration, etc. Preferably, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises the aforementioned biological scaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

Pulmonary vascular disease:

Pulmonary vascular disease is a congenital, inherited or acquired changes in the structure and/or function of the pulmonary circulation, including lesions in pulmonary artery, pulmonary vein, and pulmonary microvascular. Its main symptoms are primary or secondary lesions of pulmonary hypertension, and pulmonary venous obstructive disease. The main reason is related to the interaction of genetic susceptibility and environmental factors. Secondary pulmonary hypertension is associated with pulmonary venous hypertension, chronic hypoxia, thrombotic or embolic disease, and may also directly involve pulmonary vasculopathy.

Pulmonary hypertension (PH):

Pulmonary hypertension refers to a hemodynamic and pathophysiological state in which the pulmonary arterial pressure rises above a certain threshold, which can lead to right heart failure and can be an independent disease, a complication, or a syndrome. Patients are accompanied by major symptoms such as weakness and dyspnea. Without treatment, the course of the disease progresses rapidly, and often develops to right heart failure, which leads to death. It is characterized by pulmonary vascular remodeling, vascular occlusion causing pulmonary vascular resistance (Pulmonary vascular resistance, PVR), increased pulmonary artery pressure and right ventricular hypertrophy.

According to the pathological manifestations, hemodynamic characteristics and clinical diagnosis and treatment strategies, pulmonary hypertension can be divided into five categories: (i) arterial pulmonary hypertension; (ii) pulmonary hypertension caused by left heart disease; (iii) pulmonary hypertension caused by hypoxia and/or lung disease; (iv) chronic thromboembolic pulmonary hypertension; (v) pulmonary hypertension caused by multiple mechanisms and/or unknown mechanisms.

At present, the most effective treatment method is drug therapy, including three categories of drugs: prostacyclin, endothelin-1 receptor antagonist, and phosphodiesterase type 5 inhibitor. Although these drugs can improve the condition, they do not fundamentally improve the pulmonary vascular remodeling, and the overall price is high, which cannot meet the needs of long-term treatment.

In one aspect, the present invention relates to use of the mesenchymal stem cell population, the culture, the culture supernatant or the pharmaceutical composition as described herein in the manufacture of a medicament for preventing and/or treating a pulmonary hypertension, delaying or alleviating a pulmonary hypertension; alternatively, the present invention relates to a method for preventing and/or treating a pulmonary hypertension, delaying or alleviating a pulmonary hypertension, which comprises administering to a subject in need thereof an effective amount of the mesenchymal stem cell population, the culture, the culture supernatant or the pharmaceutical composition as described herein.

In some embodiments, the medicament is capable of reducing right ventricular systolic blood pressure in the treatment of pulmonary hypertension.

In some embodiments, the medicament is capable of inhibiting the formation of pulmonary arterial hypertension in the treatment of pulmonary arterial hypertension, increasing the acceleration time of pulmonary arterial blood flow, decreasing the diameter ratio of right ventricle to left ventricle, decreasing the mean pulmonary arterial pressure, and decreasing the pulmonary arteriole media thickness and pulmonary arteriole wall area in pulmonary arterial hypertension rats.

In some embodiments, the medicament is capable of inhibiting inflammation, increasing an anti-inflammatory factor level, and reducing a proinflammatory factor level.

In some embodiments, the medicament is in unit dose form, and the unit dosage of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$, preferably 3 to 6 $\times10^6$).

In some embodiments, the medicament further contains an additional active component, for example, the additional active component is selected from the group consisting of prostacyclin analog, endothelin receptor antagonist and phosphodiesterase inhibitor; preferably, the prostacyclin analog is selected from the group consisting of beraprost sodium, iloprost, epoprostenol, treprostinil and any combination thereof; preferably, the endothelin receptor antagonist is selected from the group consisting of bosentan, ambrisentan, macitentan and any combination thereof; preferably, the phosphodiesterase inhibitor is selected from the group consisting of sildenafil, tadalafil, vardenafil, riociguat and any combination thereof.

In some embodiments, the subject is a mammal, such as a human.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

The medicament of the present invention can be administrated by intradermal injection, subcutaneous injection, intramuscular injection, intravenous injection, oral administration, etc. In some preferred embodiments, the administration is performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the medicament further comprises a pharmaceutically acceptable carrier or excipient; preferably, the carrier is selected from the group consisting of gelatin, chitosan, sodium alginate, collagen, silk protein, cellulose, fibrous protein, polylactic acid, polyurethane, polyethylene oxide, polyethylene glycol, polylactic glycolic acid, poly(ε-caprolactone), silicate, silicone rubber, extracellular matrix, decellularized scaffold and any combination thereof; preferably, the carrier is selected from the group consisting of gelatin, collagen and any combination thereof; preferably, the medicament is an injection, microinjection, mucosal patch, enema, suppository, gel, oral preparation, aerosol, drop, ointment, implant or capsule, preferably injection; preferably, the medicament further comprises a pharmaceutically acceptable sterile isotonic aqueous or non-aqueous solution, dispersion, suspension or emulsion.

In some embodiments, the pharmaceutical composition comprises the aforementioned biological scaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

In some embodiments, the administration dosage is not less than $1 \times 10^4$ cells/time (e.g., not less than $1 \times 10^4$ cells/time, not less than $3 \times 10^4$ cells/time, not less than $5 \times 10^4$ cells/time, not less than $7 \times 10^4$ cells/time, not less than $1 \times 10^5$ cells/time, not less than $3 \times 10^5$ cells/time, not less than $5 \times 10^5$ cells/time, not less than $7 \times 10^5$ cells/time, not less than $1 \times 10^6$ cells/time, not less than $3 \times 10^6$ cells/time, not less than $5 \times 10^6$ cells/time, not less than $7 \times 10^6$ cells/time, not less than $1 \times 10^7$ cells/time, not less than $3 \times 10^7$ cells/time, not less than $5 \times 10^7$ cells/time, not less than $7 \times 10^7$ cells/time, not less than $1 \times 10^8$ cells/time, not less than $3 \times 10^8$ cells/time, not less than $5 \times 10^8$ cells/time, not less than $7 \times 10^8$ cells/time, not less than $1 \times 10^9$ cells/time, not less than $3 \times 10^9$ cells/time, not less than $5 \times 10^9$ cells/time, not less than $7 \times 10^9$ cells/time, not less than $1 \times 10^{10}$ cells/time, not less than $3 \times 10^{10}$ cells/time, not less than $5 \times 10^{10}$ cells/time or not less than $7 \times 10^{10}$ cells/time), preferably 3 to 6 $\times 10^6$ cells/day.

In some embodiments, a prophylactically and/or therapeutically effective amount of the mesenchymal stem cell population is administered to a subject by injection administration, mucosal administration, lumen administration, oral administration, respiratory tract administration or skin administration.

In some embodiments, the method further comprises administering to the subject simultaneously, sequentially or alternately a prophylactically and/or therapeutically effective amount of an additional active component, the additional active component for example is selected from the group consisting of prostacyclin analog, endothelin receptor antagonist and phosphodiesterase inhibitor; preferably, the prostacyclin analog is selected from the group consisting of beraprost sodium, iloprost, epoprostenol, treprostinil and any combination thereof; preferably, the endothelin receptor antagonist is selected from the group consisting of bosentan, ambrisentan, macitentan and any combination thereof; preferably, the phosphodiesterase inhibitor is selected from the group consisting of sildenafil, tadalafil, vardenafil, riociguat, and any combination thereof.

In another aspect, the present invention also provides a product for preventing, treating, delaying and/or alleviating a respiratory disease, which comprises the mesenchymal stem cell population of the present invention. In some embodiments, the product further comprises an additional active component as defined above. In some embodiments, the mesenchymal stem cell population and the additional active component are present as separate components or as a single formulation. In some embodiments, the product is an injection, microinjection, mucosal patch, enema, suppository, gel, oral preparation, aerosol, drop, ointment, implant, or capsule. In some embodiments, the product is an implant.

## Eye disease

In one aspect, the present invention provides use of the mesenchymal stem cell population or its culture supernatant in the manufacture of a medicament for preventing and/or treating eye disease; alternatively, the present invention provides a method for preventing and/or treating eye disease, comprising administering to a subject in need thereof a prophylactically and/or therapeutically effective amount of the mesenchymal stem cell population or its culture supernatant.

The eye disease is selected from the group consisting of ocular surface injury (e.g., corneal damage), dry eye, meibomian gland dysfunction (MGD), glaucoma, cataract, conjunctivitis, keratitis, blepharitis, chalazion, hordeolum, retinopathy, retinal prolapse, fundus venous vasculopathy, or any combination thereof.

In certain embodiments, the medicament further comprises a carrier or excipient.

In certain embodiments, the carrier is selected from the group consisting of gelatin, chitosan, sodium alginate, collagen, silk protein, cellulose, fibrin, polylactic acid, polyurethane, polyethylene oxide, polyethylene glycol, polylactic glycolic acid, poly(ε-caprolactone), silicate, silicone rubber, extracellular matrix, decellularized scaffold, or any combination thereof.

In certain embodiments, the carrier is selected from the group consisting of gelatin, collagen, or any combination thereof.

In certain embodiments, the medicament further comprises a second active component.

In certain embodiments, the second active component is, for example, an antibacterial or anti-inflammatory drug.

In certain embodiments, the second active component is selected from the group consisting of tetracycline hydrochloride eye drop, prednisone acetate eye ointment, hydrocortisone acetate eye drop, hydrocortisone acetate eye ointment, dexamethasone eye drop liquid, polymyxin B eye drop, glutathione eye drop, erythromycin eye ointment, yellow mercuric oxide eye ointment, chlortetracycline eye ointment, atropine sulfate eye ointment, boric acid eye ointment, or any combination thereof.

In some embodiments, the medicament is in unit dosage form, and the unit dose of the medicament contains the mesenchymal stem cells in an amount of no less than $1 \times 10^4$ (e.g., no less than $1 \times 10^4$, no less than $3 \times 10^4$, no less

than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$, preferably $3\times10^6$, $5\times10^6$, more preferably $3\times10^6$).

In certain embodiments, the dosage of the mesenchymal stem cells is not less than $1\times10^4$/ml (e.g., not less than $1\times10^4$, not less than $3\times10^4$, not less than $5\times10^4$, not less $7\times10^4$, not less than $1\times10^5$, not less than $3\times10^5$, not less than $5\times10^5$, not less than $7\times10^5$, not less than $1\times10^6$, not less than $3\times10^6$, not less than $5\times10^6$, not less than $7\times10^6$, not less than $1\times10^7$, not less than $3\times10^7$, not less than $5\times10^7$, not less than $7\times10^7$, not less than $1\times10^8$, not less than $3\times10^8$, not less than $5\times10^8$, not less than $7\times10^8$, not less than $1\times10^9$, not less than $3\times10^9$, not less than $5\times10^9$, not less than $7\times10^9$, not less than $1\times10^{10}$, not less than $3\times10^{10}$, not less than $5\times10^{10}$ or not less than $7\times10^{10}$, preferably $1\times10^6$, $3\times10^6$, $5\times10^6$/ml, more preferably $3\times10^6$/ml).

In certain embodiments, the route of administration of the mesenchymal stem cells is selected from the group consisting of injection administration, smear administration, adhesive administration, enema administration, perfusion administration, rectal administration, and oral administration.

In certain embodiments, the method further comprises administering to a subject in need thereof a second active component as previously described or defined.

In certain embodiments, the subject is a mammal, such as a human.

In another aspect, the present invention provides a product for treating eye disease, comprising a mesenchymal stem cell population as first active component.

In certain embodiments, the mesenchymal stem cell population is as previously described or defined.

In certain embodiments, the eye disease is as previously described or defined.

In certain embodiments, the product further comprises a second active component.

In certain embodiments, the second active component is as previously described or defined.

In certain embodiments, the first active component and the second active component are present alone or in combination.

In certain embodiments, the first active component is administered in combination with a second active component selected from those previously described.

In certain embodiments, the product is an implant, preferably, the implant is used for improving the microenvironment and inhibiting immune rejection.

In certain embodiments, the subject is a mammal, such as a human.

Ocular surface injury

Ocular surface injury is selected from the group consisting of chemical burn (e.g., alkali, acid burns) of eye (e.g., cornea), thermal burn of eye (e.g., cornea), corneal injury, or any combination thereof.

Ocular surface injury is one of the main causes of blindness in the world, among which the most common causes are ocular chemical burns (e.g., alkali and acid burns) and thermal burns, which seriously damage the ocular surface and are difficult to treat, the prognosis is poor, often leading to blindness and even loss of the eyeball. Corneal alkali burns are the most serious chemical burns. Alkaline substances can cause corneal tissue liquefaction and necrosis, resulting in serious damage to limbal stem cells. Severe depletion of limbal stem cells results in persistent inflammation, corneal and conjunctival epithelial metaplasia, ingrowth of new blood vessels, and scarring of corneal stroma. The subsequently induced immune inflammatory response is more likely to develop deep, and cause corneal ulcer and perforation, secondary glaucoma and concurrent cataract, and severely damage the anatomical structure and visual function of the eye.

In one aspect, the present invention relates to use of the mesenchymal stem cell population, the culture, the culture supernatant or the pharmaceutical composition as described herein for preventing and/or treating ocular surface injury in a subject, or in the manufacture of a medicament for preventing ocular surface injury, or delaying, or reducing, or preventing or alleviating ocular surface injury in a subject.

Corneal alkali burn is a type of ocular surface injury.

In certain embodiments, the pharmaceutical composition is capable of treating a corneal alkali burn, alleviating a corneal alkali burn, promoting recovery from a corneal alkali injury, reducing inflammation, reducing corneal myeloperoxidase (MPO) concentration, reducing the number of new blood vessels, decreasing MMP-9 level, or/and decreasing levels of proinflammatory cytokine (IL-6 and IL-1 inhibitors) and chemokine (CXCL1/cincl and CCL2/MCP-1).

Cornea: It is the transparent fibrous membrane without blood vessels at the front end of the eyeball wall, which is round and occupies one-sixth of the outer layer area, and mainly composed of avascular connective tissue; histologically, there are five layers from front to back: epithelial layer, pre-elastic layer, stroma layer, post-elastic layer and endothelial layer. The cornea is highly transparent with smooth surface, bulges in front and depresses in back, has a shape like a convex-concave lens, is curved like a spherical surface, and has the effect of refraction.

Corneal alkali burn: After the solution, dust or gas of alkaline substance contacts the cornea, the fat and protein are dissolved, the tissue is destroyed, which further promotes that the alkaline substance continues to diffuse and penetrate into the deep tissue, resulting in the decomposition and necrosis of corneal tissue cells, which mostly occurs in chemical plants, laboratories or construction site.

Keratitis: Keratitis is inflammation caused by the invasion of corneal tissue by exogenous or endogenous pathogenic factors when the defense capacity of the cornea is weakened. The main symptoms of the patients were eye irritation, such as eye pain, photophobia, lacrimation, and blepharospasm. In the early stage, keratitis is generally limited to part of the cornea, and it may progress gradually when effective treatment is unavailable. In the advanced stage, it may cause irreversible visual impairment. Infectious keratitis mostly occurs in the central area of the cornea, while immune keratopathy tends to occur in the peripheral area of the cornea.

In certain embodiments, the subject is a mammal, such as a human.

In certain embodiments, the medicament comprises a therapeutically effective amount of the mesenchymal stem cell population and/or the culture, and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain preferred embodiments, the administration is performed by corneal injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the mesenchymal stem cell population can be transplanted in the form of a suspension, gel, colloid, slurry or mixture.

## Movement system disease and bone-related disease

Movement system disease refers to a disease that occurs in bone, joint, muscle, ligament, etc., and are common in clinical practice. It may manifest as local disease or systemic disease. The local disease includes for example trauma, fracture, dislocation, deformity, etc. The systemic disease such as rheumatoid arthritis may occur in hand, wrist, knee and hip. Osteoarticular tuberculosis often occurs in spine, hip joint and other parts. Many local lesions of the movement system are diagnosed and treated in orthopaedic surgery. Some systemic diseases (e.g., rheumatoid arthritis) of the movement system are diagnosed and treated in internal medicine, while some (e.g., osteoarticular tuberculosis) are still diagnosed and treated in orthopaedic surgery. It can be classified by etiology or site of disease. In general textbooks, the movement system diseases are sometimes divided into two categories: trauma and bone disease. The trauma is further divided into fracture, dislocation and soft tissue injury, etc. The bone disease is classified by etiology or anatomical part.

Bone-related diseases include all diseases related to bone, joint, ligament, cartilage and structure that supports limb, neck and back. The related disease is selected from the group consisting of sprain, strain and laceration of cartilage, arthritis, bursitis, acute and chronic back pain, osteoporosis, trigger finger, osteogenesis imperfecta, and comorbidities thereof, and the like.

In one aspect, the present invention provides a use of the mesenchymal stem cell population or its culture supernatant in the manufacture of a medicament for preventing, treating, delaying and/or alleviating a movement system disease or bone-related disease; alternatively, the present invention provides a method for preventing, treating, delaying and/or alleviating a movement system disease or bone-related disease, which comprises administering to a subject in need thereof a prophylactically and/or therapeutically effective amount of the mesenchymal stem cell population or its culture supernatant.

In certain embodiments, the movement system disease includes, for example, trauma (e.g., fracture, dislocation, soft tissue injury, etc.), or bone disease. In certain embodiments, the bone-related disease is selected from the group consisting of sprain, strain and laceration of cartilage, arthritis, bursitis, acute and chronic back pain, osteoporosis, trigger finger, osteogenesis imperfecta and comorbidities thereof.

In certain embodiments, the mesenchymal stem cell population of the present invention is administered in combination with an additional active component, and thus the medicament may also comprise an additional active component. In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the medicament is in unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

In another aspect, the present invention also provides a product for preventing, treating, delaying and/or alleviating a movement system disease or bone-related disease, which comprises the mesenchymal stem cell population of the present invention. In certain embodiments, the product further comprises an additional active component as defined above. In certain embodiments, the mesenchymal stem cell population and the additional active component are present as separate components or as a single formulation. In certain embodiments, the product is an injection, microinjection, mucosal patch, enema, suppository, gel, oral preparation, aerosol, drop, ointment, implant, or capsule. In certain embodiments, the product is an implant.

Arthritis or joint injury

Arthritis is any disease that affects the joints, its symptoms often include joint pain and stiffness, and other possible symptoms include redness, warmth, swelling, and reduced range of motion in the affected joint. Certain arthritis may also affect other organs besides the joints. Its onset may be gradual or sudden and acute. There are more than 100 types of arthritis, and the most common ones are osteoarthritis (degenerative joint disease) and rheumatoid arthritis.

As used herein, the term "osteoarthritis" refers to a chronic joint disease characterized by degeneration, destruction of articular cartilage, and bone hyperplasia. The cause of most cases of osteoarthritis is unknown and is referred to as "primary osteoarthritis." When the cause of osteoarthritis is known, it is called "secondary osteoarthritis". Secondary osteoarthritis results from other diseases or conditions. Conditions that can lead to secondary osteoarthritis include repeated damage or surgery to joint structures, joint abnormalities at birth (congenital abnormalities), gout, diabetes, and other hormonal disorders. Other forms of arthritis include systemic diseases such as rheumatoid arthritis and systemic lupus erythematosus.

In one aspect, the present invention provides a use of the mesenchymal stem cell population or its culture supernatant in the manufacture of a medicament for preventing, treating, delaying and/or alleviating an arthritis or joint injury; alternatively, the present invention provides a method for preventing, treating, delaying and/or alleviating an arthritis or joint injury, which comprises administering to a subject in need thereof a prophylactically and/or therapeutically effective amount of the mesenchymal stem cell population or its culture supernatant.

In certain embodiments, the arthritis is selected from the group consisting of osteoarthritis, traumatic arthritis, and autoimmune arthritis. In certain embodiments, the joint injury is a meniscus injury.

In certain embodiments, the mesenchymal stem cell population is used for preventing, treating, delaying and/or alleviating an osteoarthritis (OA) in a subject.

In certain embodiments, the mesenchymal stem cell population is capable of improving motor activity, inhibiting pain generated by nerve, treating tissue injury, or/and relieving a symptom of arthritis.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

The mesenchymal stem cell population of the present invention can be administrated by intradermal injection, subcutaneous injection, intramuscular injection, intravenous injection, oral administration, etc. In certain preferred embodiments, the administration is performed by intra-articular injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the pharmaceutical composition comprises a cell sphere formed from the mesenchymal stem cell population. In certain embodiments, the pharmaceutical composition comprises a mixture of the cell sphere and a biological material.

In certain embodiments, the mesenchymal stem cell population can be transplanted in the form of a suspension, gel, colloid, slurry or mixture.

In certain embodiments, the mesenchymal stem cell population of the present invention are administered in combination with an additional active component, and thus the medicament may further comprise an additional active component (e.g., an additional therapeutic agent for treating osteoarthritis). In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the medicament is in unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

Meniscus injury

The meniscus is one of the important structures that constitute the knee joint, composed of two meniscus fibrocartilages, and located between the femoral condyle and the tibial plateau. Its lateral edge is thicker and its medial edge is thinner. The medial meniscus is "c" shaped and the lateral meniscus is approximately "o" shaped. The function of meniscus is to stabilize the knee joint, transmit the load force of the knee joint, and promote intra-articular nutrition. Meniscus injury refers to a rupture of meniscus caused by factors such as rotational force, compression, and disease of the meniscus itself, which manifests as severe knee pain, inability to straighten, and swelling, and is one of the most common injuries to the knee. Knee meniscus injury is manifested as localized pain in the knee joint, and some patients have the phenomenon of limping or knee joint locking.

In one aspect, the present invention provides a use of the mesenchymal stem cell population or its culture supernatant in the manufacture of a medicament for preventing, treating, delaying and/or alleviating a meniscus injury; alternatively, the present invention provides a method for preventing, treating, delaying and/or alleviating a meniscus injury, which comprises administering to a subject in need thereof a prophylactically and/or therapeutically effective amount of the mesenchymal stem cell population or its culture supernatant.

In certain embodiments, the mesenchymal stem cell population is capable of reducing knee pain, reducing local edema, alleviating lameness, alleviating joint locking, or/and alleviating pain.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

The mesenchymal stem cell population of the present invention can be administrated by intradermal injection, subcutaneous injection, intramuscular injection, intravenous injection, oral administration, etc. In certain preferred embodiments, the administration is performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the mesenchymal stem cell population can be transplanted in the form of a suspension, gel, colloid, slurry or mixture.

In certain embodiments, the mesenchymal stem cell population of the present invention is administered in combination with an additional active component, and thus the medicament may further comprise an additional active component (e.g., additional therapeutic agent for treating meniscus injury). In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the medicament is in unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).


Bone injury


Bone injury is a disease of the movement system, which refers to the bone defect or defect repair caused by congenital or acquired factors, as well as the complete or partial rupture of the continuity of the bone structure. For example, fractures often include multiple fracture, pelvic fracture, femur fracture, clavicle fracture, femoral neck fracture, hip fracture, thoracolumbar fracture, compression fracture due to osteoporosis, ulna fracture, calcaneus fracture, distal radius fracture, tibial shaft fracture, tibial plateau fracture, intertrochanteric fracture, ankle fracture, lower extremity fracture, long bone diaphyseal fracture, spinal fracture and severe open fracture. Bone defect is a shortage of bone due to trauma or surgery or injury, such as comminuted fracture, open fracture, large bone tissue defect caused by trauma, inflammation, bone disease, etc.; bone necrosis and detachment caused by inflammation, bone defect caused by necrosis of large bone pieces due to bone infarction or bone ischemic necrosis, etc., all belonging to the bone defects caused by diseases. The bone defects also include the bone defects caused by surgery, the fractured bone pierced through the limbs during trauma, or the removal of deactivated bone during the debridement of open fracture. Bone injuries have different symptoms depending on the location of the injury. The main symptoms are pain, swelling and limited mobility. For example, when the meniscus is injured, the patient feels a sense of tearing and crunchy feeling, local pain and tenderness of the knee joint, unable to fully extend the knee, and sound of the knee joint when moving. In the chronic stage, the symptoms of joint pain are relieved, but the knee joint may be limp, and the pain is aggravated when going up and down the stairs, and it can be relieved after rest. Over time, it can be associated with traumatic arthritis and quadriceps muscle atrophy. Some patients may also have knee locking symptoms.

In one aspect, the present invention provides a use of the mesenchymal stem cell population or its culture supernatant in the manufacture of a medicament for preventing, treating, delaying and/or alleviating a bone injury;

alternatively, the present invention provides a method for preventing, treating, delaying and/or alleviating a bone injury, which comprises administering to a subject in need thereof a prophylactically and/or therapeutically effective amount of the mesenchymal stem cell population or its culture supernatant thereof.

In certain embodiments, the mesenchymal stem cell population is capable of accelerating the healing of bone lesion, inhibiting pain generated by nerve, treating tissue damage, or/and relieving symptoms of arthritis.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

The mesenchymal stem cell population of the present invention can be administrated by intradermal injection, subcutaneous injection, intramuscular injection, intravenous injection, oral administration, etc. In certain preferred embodiments, the administration is performed by intramuscular injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the pharmaceutical composition comprises a cell sphere formed from the mesenchymal stem cell population. In certain embodiments, the pharmaceutical composition comprises a mixture of the cell sphere and a biological material.

In certain embodiments, the mesenchymal stem cell population can be transplanted in the form of a suspension, gel, colloid, slurry or mixture.

In certain embodiments, the mesenchymal stem cell population of the present invention are administered in combination with an additional active component, and thus the medicament may further comprise an additional active component (e.g., an additional therapeutic agent for treating bone injury). In certain embodiments, the mesenchymal stem cells are administered simultaneously, separately, or sequentially with the additional therapeutic agent. In certain embodiments, the mesenchymal stem cell population and the additional active component are present as separate components or as a single formulation.

In certain embodiments, the medicament is in unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$). In certain embodiments, the unit dose of the medicament contains the mesenchymal stem cells in an amount of $1\times10^4$ to $1\times10^{10}$ (e.g., $1\times10^6$ to $1\times10^8$, $1\times10^6$ to $1\times10^7$, or $1\times10^6$ to $5\times10^6$).

## Mucosal immune system

The mucosal immune system (MIS) refers to the lymphoid tissue widely distributed in the mucosal membranes of respiratory tract, gastrointestinal tract, genitourinary tract, and some exocrine glands, and is the main site for performing local specific immune functions. The mucosal immune system consists of four parts: intestinal mucosa-associated lymphoid tissue (GALT), bronchial mucosa-associated lymphoid tissue (BALT), ocular conjunctiva-associated lymphoid tissue (CALT), and urogenital mucosa-associated lymphoid tissue (UALT), which play a very important role in the antiviral immune response. The so-called mucosa-associated lymphoid tissues, that are lymphoid tissues distributed along the mucosal epithelium of the respiratory tract, digestive tract, urogenital tract and some exocrine glands (Harder's gland, pancreas, breast, lacrimal duct, salivary gland secretory duct, etc.) and widely exist under the epithelium, are the sites where mucosae contact and uptake antigens and initial immune responses occur. The related diseases include gastrointestinal mucosal injury (gastrointestinal infection, gastritis, enteritis, etc.), genitourinary tract related diseases (urethral infection, urethral mucosal inflammation, reproductive tract infection and related inflammation, etc.), oral mucosal disease (oral mucosal infectious disease, oral mucosal allergic disease, oral mucosal ulcer disease, oral mucosal bullous disease, oral mucosal streak disease, oral mucosal granulomatous disease, lip and tongue disease, sexually transmitted disease, oral mucosal pigment abnormality, etc.), middle ear mucosal inflammation (otitis media, etc.), nasal mucosal lesion (sinusitis, olfactory disturbance, allergic rhinitis, etc.), respiratory mucosal disease (chronic obstructive pulmonary disease, asthma, respiratory disease caused by bacterial or viral infection), etc.

In one aspect, the present invention provides a use of the mesenchymal stem cell population or its culture supernatant in the manufacture of a medicament for preventing and/or treating a mucosal immune system disease; alternatively, the present invention provides a method for preventing and/or treating a mucosal immune system disease, comprising administering to a subject in need thereof a prophylactically and/or therapeutically effective amount of the mesenchymal stem cell population or its culture supernatant.

**Nasal disease**

In one aspect, the present invention provides use of the mesenchymal stem cell population or its culture supernatant in manufacture of a medicament for preventing and/or treating nasal disease; alternatively, the present invention provides a method for preventing and/or treating nasal disease, comprising administering to a subject in need thereof a prophylactically and/or therapeutically effective amount of the mesenchymal stem cell population or its culture supernatant thereof.

"Diseases of nose" include diseases of the external nose, nasal vestibule, nasal cavity and sinuses, which may be divided into infection, hemorrhage, allergy, tumor, trauma, foreign body, congenital malformation and structural abnormality. The nose is often affected by external adverse factors and is prone to various diseases. Microbial infection can cause nasal boils, nasal vestibulitis, inflammation of nasal mucosa and sinuses; the nasal cavity is the portal for allergens to enter the body, and is also the site of allergic diseases, so hay fever and allergic rhinitis are common diseases; Certain oral diseases such as root infection can cause odontogenic maxillary sinusitis. The external nose is located in the middle of the face and is vulnerable to trauma. The external nose is an important symbol for maintaining a correct appearance. Therefore, the surgical requirements for congenital and acquired nasal deformities are higher. The nasal cavity and paranasal sinuses are the most common sites for malignant tumors, and maxillary sinus cancer is the most common, followed by nasal cavity cancer and ethmoid sinus cancer. The nose is anatomically adjacent to the cranial cavity, orbit and oral cavity. Diseases of the nasal vestibule, nasal cavity and sinuses may lead to serious complications, such as meningitis, orbital cellulitis, etc.; through blood flow, they may cause infection of the cavernous sinus, severe cases may lead to blindness and even death.

The term "nasal mucosa" refers to the mucosa covering the surface of the nasal cavity, beneath which is cartilage, bone or skeletal muscle. Common nasal mucosal lesions include sinusitis (the mucosa of the sinuses is continuous with the respiratory mucosa, so rhinitis and colds may easily lead to sinusitis), olfactory disorder (in the case of inflammatory infection or local space-occupying lesion, the mucosa is swollen and hyperemic, and the secretion is excessive; on the one hand, it causes nasal obstruction, so that the airflow carrying olfactory elements is blocked and cannot reach the olfactory area), allergic rhinitis (called allergic rhinitis, which is a nasal mucosal non-infectious inflammatory disease mainly mediated by IgE after the body is exposed to allergens), and the like.

The nasal disease is selected from the group consisting of rhinitis, sinusitis, nasal vestibulitis, nasal mucosa disease, or any combination thereof.

Rhinitis

The term "rhinitis" refers to an inflammatory disease of the nasal cavity, which is an inflammation of the nasal mucosa caused by viruses, bacteria, allergens, various physicochemical factors and certain systemic diseases. Rhinitis is mainly divided into the following four types: chronic rhinitis, acute rhinitis, drug-induced rhinitis, and atrophic rhinitis, which include: chronic simple rhinitis, chronic hypertrophic rhinitis, chronic dry rhinitis, allergic rhinitis (seasonal rhinitis, perennial rhinitis), dry rhinitis, vasomotor rhinitis, eosinophilic non-allergic rhinitis, hyperreflectory rhinitis, idiopathic rhinitis, structural rhinitis, local allergic rhinitis. In addition to local and environmental factors, long-term chronic diseases, such as endocrine disorder, cardiovascular disease and so on, vitamin deficiency, excessive tobacco and alcohol use, and long-term use of blood drugs may cause nasal vasodilation and result in rhinitis and other symptoms. Chronic diseases, including blood diseases, tuberculosis, diabetes, rheumatism, acute infectious disease and chronic heart, liver and kidney diseases, may cause long-term congestion or reflex congestion in the nasal mucosa; chronic inflammation of nasal cavity and sinuses, or influence of adjacent infection sites, promotes the occurrence of chronic rhinitis.

Allergic rhinitis (AR), also known as rhinallergosis, is a common otolaryngology disease and a common respiratory allergic disease. The disease is an allergic disease that occurs in the nasal mucosa and is characterized by itching, sneezing, rhinorrhea, swollen nasal mucosa, . The prevalence of allergic rhinitis is 10% to 40%, among which pollen allergy is more common in Europe and North America, and perennial allergic rhinitis is more common in Asia. Although allergic rhinitis is not fatal, the patient's nose and general discomfort is obvious, which affects the patient's study and work. If not properly treated, about 30% of patients will develop bronchial asthma, and even pulmonary heart diseases and other diseases that seriously affect the health and quality of patients' life. Corticosteroids and antihistamines are currently the first-line drugs for allergic rhinitis. Allergic rhinitis is an allergic inflammatory reaction mediated by IgE under the action of environmental factors in vitro, and is dominated by the immune response of nasal mucosa.

In one aspect, the present invention relates to use of the mesenchymal stem cell population, the culture, the culture supernatant or the pharmaceutical composition as described herein for preventing and/or treating rhinitis (e.g., allergic rhinitis) in a subject, or in the manufacture of a medicament for preventing rhinitis, or delaying, or reducing rhinitis, or preventing or alleviating rhinitis in a subject.

In certain embodiments, the pharmaceutical composition is capable of reducing sneezing caused by rhinitis, and reducing the number of nose scratching by the subject.

In certain embodiments, the pharmaceutical combination is capable of reducing the level of serum antigen-specific antibody response and reducing the expression of inflammatory mediator.

In certain embodiments, the pharmaceutical combination is capable of promoting angiogenesis at inflammation site, promoting epithelial cell generation, and reducing inflammatory cell infiltration.

In certain embodiments, the pharmaceutical combination is capable of restoring nasal mucosa epithelial surface, normalizing cilia, normalizing fibroblast, and normalizing cytoplasmic cytoplasm.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain preferred embodiments, the administration is performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

In certain embodiments, the medicament further comprises a carrier or excipient.

In certain embodiments, the carrier is selected from the group consisting of gelatin, chitosan, sodium alginate, collagen, silk protein, cellulose, fibrin, polylactic acid, polyurethane, polyethylene oxide, polyethylene glycol, polylactic glycolic acid, poly(ε-caprolactone), silicate, silicone rubber, extracellular matrix, decellularized scaffold, or any combination thereof.

In certain embodiments, the carrier is selected from the group consisting of gelatin, collagen, or any combination thereof.

In certain embodiments, the medicament further comprises a second active component.

In certain embodiments, the second active component is selected from the group consisting of glucocorticoid, nasal decongestant, antihistamine (e.g., azelastine), leukotriene receptor antagonist (e.g., montelukast sodium), anticholinergic (e.g., ipratropium bromide), antiallergic drug (e.g., sodium cromoglycate), mucolytic drug (e.g., myrtle oil), antibacterial, or any combination thereof.

In certain embodiments, the glucocorticoid is selected from the group consisting of beclomethasone propionate, budesonide, fluticasone propionate, mometasone furoate, or any combination thereof.

In certain embodiments, the antibacterial agent is selected from the group consisting of amoxicillin, cefpodoxime proxetil, cefuroxime axetil, cefdinir, trimethoprim, sulfamethoxazole, doxycycline, azithromycin, clarithromycin, erythromycin, gatifloxacin, levofloxacin, moxifloxacin, ceftriaxone, or any combination thereof.

In certain embodiments, the medicament is in an unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$, no less than $7\times10^5$, no less than $1\times10^6$, no less than $3\times10^6$, no less than $5\times10^6$, no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$, for example $1\times10^5$ to $1\times10^8$, $7\times10^5$ to $7\times10^6$, $1\times10^6$ to $5\times10^6$, preferably $1\times10^6$, $3\times10^6$, $5\times10^6$, more preferably $3\times10^6$).

In certain embodiments, the administration dosage of the mesenchymal stem cells is not less than $1\times10^4$/ml (e.g., not less than $1\times10^4$, not less than $3\times10^4$, not less than $5\times10^4$, not less than $7\times10^4$, not less than $1\times10^5$, not less than $3\times10^5$, not less than $5\times10^5$, not less than $7\times10^5$, not less than $1\times10^6$, not less than $3\times10^6$, not less than $5\times10^6$, not less than $7\times10^6$, not less than $1\times10^7$, not less than $3\times10^7$, not less than $5\times10^7$, not less than $7\times10^7$, not less than $1\times10^8$, not less than $3\times10^8$, not less than $5\times10^8$, not less than $7\times10^8$, not less than $1\times10^9$, not less than $3\times10^9$, not less than $5\times10^9$, not less than $7\times10^9$, not less than $1\times10^{10}$, not less than $3\times10^{10}$, not less than $5\times10^{10}$ or not less than $7\times10^{10}$, for example $1\times10^5$ to $1\times10^8$, $7\times10^5$ to $7\times10^6$, $1\times10^6$ to $5\times10^6$/ml, preferably $1\times10^6$, $3\times10^6$, $5\times10^6$/ml, more preferably $3\times10^6$/ml).

In certain embodiments, the route of administration of the mesenchymal stem cells is selected from the group consisting of injection administration, smear administration, adhesive administration, enema administration, perfusion administration, rectal administration, and oral administration.

In certain embodiments, the method further comprises administering to the subject in need thereof a second active component as previously described or defined.

In certain embodiments, the subject is a mammal, such as a human.

In another aspect, the present invention provides a product for treating nasal disease, comprising a mesenchymal stem cell population as first active component.

In certain embodiments, the mesenchymal stem cell population is as previously described or defined.

In certain embodiments, the nasal disease is as previously described or defined.

In certain embodiments, the product further comprises a second active component.

In certain embodiments, the second active component is as previously described or defined.

In certain embodiments, the first active component and the second active component are present alone or in combination.

In certain embodiments, the first active component is administered in combination with a second active component selected from those previously described.

In certain embodiments, the product is an implant, preferably, the implant is used for improving microenvironment and inhibiting immune rejection.

In certain embodiments, the subject is a mammal, such as a human.

## Kidney disease

In one aspect, the present invention provides use of the mesenchymal stem cell population or its culture supernatant in the manufacture of a medicament for preventing and/or treating kidney disease; alternatively, the present invention provides a method for preventing and/or treating kidney disease, comprising administering to a subject in need thereof a prophylactically and/or therapeutically effective amount of the mesenchymal stem cell population or its culture supernatant thereof.

Kidney disease is a disease that damages kidney or reduces kidney function due to various factors. Kidney disease is a general term for common disease that seriously endangers human health. Kidney diseases mainly include different types of nephritis, acute renal failure, kidney stone, kidney cyst, chronic kidney disease and so on. The main clinical manifestations of kidney disease are proteinuria, hematuria, edema, hypertension, and renal insufficiency.

"Kidney disease" refers to a kidney-related disease, mainly including: primary glomerular disease, secondary glomerulonephritis, hereditary kidney disease, urinary tract infectious kidney disease, renal tubular disease, interstitial nephritis, renal stone and obstructive nephropathy, cystic kidney disease and kidney tumor, renal vascular disease, kidney and hypertension, pregnancy and kidney disease, elderly kidney disease, drug (food)-induced kidney damage, renal failure.

Kidney disease includes renal injury disease. The main manifestations of renal injury are edema, hypertension, vomiting and renal insufficiency.

Kidney disease includes renal insufficiency. Renal insufficiency is a group of syndromes caused by a variety of reasons, in which severely damaged glomeruli cause disorders in terms of excreting metabolic wastes and regulating water, electrolyte, and acid-base balance. It can be divided into acute renal insufficiency and chronic renal insufficiency. It is poor in prognosis and is one of the major life-threatening conditions. The causes of renal insufficiency can be summarized as follows: a. renal diseases: such as acute and chronic glomerulonephritis, pyelonephritis, renal tuberculosis, acute renal tubular degeneration and necrosis caused by chemical and biological toxicants, renal tumors and congenital kidney disease, etc.; b. extrarenal diseases: such as systemic blood circulation disorders (shock, heart failure, hypertension), systemic metabolic disorders (e.g., diabetes), and urinary tract diseases (urolithiasis, oncothlipsis), etc.

Kidney disease includes nephrectomy. Nephrectomy is a surgical operation to treat kidney disease. Its indications include renal malignancy, renal tuberculosis, severe hydronephrosis or kidney stone, severe renal injury and unilateral pyonephrosis.

Kidney disease includes kidney fibrosis.

Kidney disease includes nephritis.

"Nephritis" refers to the non-purulent inflammatory lesions of both kidneys, with nephritis phenomena such as edema, hypertension, proteinuria due to damaged renal corpuscles, and is the most common type of kidney disease. According to etiology, nephritis can be divided into secondary and primary glomerulonephritis. Secondary glomerulonephritis is caused by other diseases (e.g., diabetes, hypertension, systemic lupus erythematosus, allergic purpura, vasculitis, etc.), and is a systemic disease involving the kidneys. According to clinical classification, nephritis can be divided into acute, chronic and rapidly progressive nephritic syndromes, latent nephritis (asymptomatic hematuria and/or proteinuria). Chronic nephritis includes mesangial proliferative glomerulonephritis, focal segmental glomerulosclerosis, membranous nephropathy, mesangial capillary glomerulonephritis, and sclerosing nephritis. The pathological changes of rapidly progressive nephritis are characterized by the formation of crescents in the glomeruli, also known as crescentic nephritis.

Kidney disease includes drug-induced kidney disease.

"Drug-induced kidney disease" refers to the drug-induced damage to kidney caused by the use of anti-infective drug, non-steroidal anti-inflammatory drug, urate-lowering drug, anti-tumor chemotherapeutic drug, immunosuppressive agent, and Chinese herbal medicine during the treatment of disease.

In certain embodiments, the medicament further comprises a carrier or excipient.

In certain embodiments, the carrier is selected from the group consisting of gelatin, chitosan, sodium alginate, collagen, silk protein, cellulose, fibrin, polylactic acid, polyurethane, polyethylene oxide, polyethylene glycol, polylactic glycolic acid, poly($\varepsilon$-caprolactone), silicate, silicone rubber, extracellular matrix, decellularized scaffold, or any combination thereof.

In certain embodiments, the carrier is selected from the group consisting of gelatin, collagen, or any combination thereof.

In certain embodiments, the medicament further comprises a second active component.

In certain embodiments, the second active component is selected from the group consisting of glucocorticoid (prednisone, prednisolone), other immunosuppressive agent (cyclophosphamide, nitrogen mustard, leukeran), angiotensin converting enzyme inhibitor, calcium channel blocker, diuretic, gastrointestinal adsorbent, acid-base balance regulator, or any combination thereof.

In some embodiments, the medicament is in unit dosage form, and the unit dose of the medicament contains the mesenchymal stem cells in an amount of no less than $1\times10^4$ (e.g., no less than $1\times10^4$, no less than $3\times10^4$, no less than $5\times10^4$, no less than $7\times10^4$, no less than $1\times10^5$, no less than $3\times10^5$, no less than $5\times10^5$ , no less than $7\times10^5$, no less than $1\times10^6$, no less than $3\times10^6$ , no less than $5\times10^6$ , no less than $7\times10^6$, no less than $1\times10^7$, no less than $3\times10^7$, no less than $5\times10^7$, no less than $7\times10^7$, no less than $1\times10^8$, no less than $3\times10^8$, no less than $5\times10^8$, no less than $7\times10^8$, no less than $1\times10^9$, no less than $3\times10^9$, no less than $5\times10^9$, no less than $7\times10^9$, no less than $1\times10^{10}$, no less than $3\times10^{10}$, no less than $5\times10^{10}$ or no less than $7\times10^{10}$, for example, $1\times10^5$ to $1\times10^8$, $7\times10^5$ to $7\times10^6$, $1\times10^6$ to $5\times10^6$, preferably $1\times10^6$, $3\times10^6$, $5\times10^6$, more preferably $3\times10^6$).

In certain embodiments, the administration dosage of the mesenchymal stem cells is not less than $1\times10^4$/ml (e.g., not less than $1\times10^4$, not less than $3\times10^4$, not less than $5\times10^4$, not less than $7\times10^4$, not less than $1\times10^5$, not less than $3\times10^5$, not less than $5\times10^5$, not less than $7\times10^5$, not less than $1\times10^6$, not less than $3\times10^6$, not less than $5\times10^6$, not less than $7\times10^6$, not less than $1\times10^7$, not less than $3\times10^7$, not less than $5\times10^7$, not less than $7\times10^7$, not

less than $1\times10^8$, not less than $3\times10^8$, not less than $5\times10^8$, not less than $7\times10^8$, not less than $1\times10^9$, not less than $3\times10^9$, not less than $5\times10^9$, not less than $7\times10^9$, not less than $1\times10^{10}$, not less than $3\times10^{10}$, not less than $5\times10^{10}$ or not less than $7\times10^{10}$, preferably $1\times10^6$, $3\times10^6$, $5\times10^6$/ml, more preferably $3\times10^6$/ml).

In certain embodiments, the route of administration of the mesenchymal stem cells is selected from the group consisting of injection administration, smear administration, adhesive administration, enema administration, perfusion administration, rectal administration, and oral administration.

In certain embodiments, the method further comprises administering to a subject in need thereof a second active component as previously described or defined.

In certain embodiments, the subject is a mammal, such as a human.

In another aspect, the present invention provides a product for treating kidney disease, comprising a mesenchymal stem cell population as first active component.

In certain embodiments, the mesenchymal stem cell population is as previously described or defined.

In certain embodiments, the kidney disease is as previously described or defined.

In certain embodiments, the product further comprises a second active component.

In certain embodiments, the second active component is as previously described or defined.

In certain embodiments, the first active component and the second active component are present alone or in combination.

In certain embodiments, the first active component is administered in combination with a second active component selected from those previously described.

In certain embodiments, the product is an implant, preferably, the implant is used for improving microenvironment and inhibiting immune rejection.

In certain embodiments, the subject is a mammal, such as a human.

In certain embodiments, the pharmaceutical composition is capable of restoring body weight.

In certain embodiments, the pharmaceutical composition is capable of reducing uric acid, urea, and uric anhydride.

In certain embodiments, the pharmaceutical composition is capable of generating protection effect against renal injury and improving renal function.

Crescentic nephritis

"Crescentic nephritis" is also known as rapidly progressive nephritis. Crescentic nephritis is a general term for a group of glomerulonephritis that develops rapidly, with hematuria, proteinuria, edema, and hypertension as the main clinical manifestations, and rapidly develops into oliguria, anuria and renal failure with poor prognosis. According to the etiology, it can be divided into two categories: primary and secondary. Among them, primary crescentic nephritis can be divided into anti-glomerular basement membrane antibody type, immune complex type, and pathogenesis-unknown type. Secondary crescentic nephritis may be caused by primary glomerular diseases, such as membranous proliferative nephritis, membranous nephropathy, IgA nephropathy (less common), etc.; secondary to infectious diseases: such as infective endocarditis, nephritis after streptococcal infection, occult visceral bacterial lesion, hepatitis B and influenza, etc.; secondary to other systemic diseases: such as systemic lupus erythematosus, systemic vasculitis, pulmonary hemorrhage-nephritis syndrome, allergic purpura, spontaneous cryoglobulinemia, malignant tumor and relapsing polychondritis.

In one aspect, the present invention relates to use of the mesenchymal stem cell population, the culture, the culture supernatant or the pharmaceutical composition as described herein for preventing and/or treating crescentic nephritis in a subject, or in the manufacture of a medicament for preventing crescentic nephritis, or delaying, or reducing, or preventing or alleviating crescentic nephritis in a subject.

In certain embodiments, the pharmaceutical composition is capable of reducing urinary protein and serum creatinine, restoring kidney function, or/and inhibiting crescent formation.

In certain embodiments, the pharmaceutical composition is capable of down-regulating Th1, Th2 and Th17 factors in spleen and kidney, reducing cells expressing IL-1$\beta$, CD8 and ED1 genes, increasing Treg cells, and inhibiting kidney inflammation.

In certain embodiments, the pharmaceutical composition is capable of reducing the expression of proinflammatory factor (e.g., IFN-$\gamma$, IL-6, TFN-$\alpha$, iNOS, etc.), increasing the expression of anti-inflammatory factor IL-10, and inhibiting inflammation in the subject.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain embodiments, the medicament of the present invention can be administered by intradermal injection, subcutaneous injection, intramuscular injection, intravenous injection, oral administration, and the like. In certain preferred embodiments, the administration may be performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

Renal fibrosis

"Kidney fibrosis" refers to a pathophysiological change of kidney, is a progressive chronic kidney disease, and is a gradual process in which the function of kidneys changes from healthy to injured, then damaged, and finally to loss of function. Due to the stimulation of various pathogenic factors such as trauma, infection, inflammation, blood circulation disorder, and immune response, the intrinsic cells of kidney are damaged, and a large amount of collagen deposition and accumulation occurs in the later stage of development, causing the renal parenchyma to gradually harden and form scars until the kidney completely loses organ function. The process of fibrosis and hardening of intrinsic cells in the kidney is also the process of renal fibrosis. Renal fibrosis is characterized by abnormal deposition of extracellular matrix (ECM). For example: acute kidney injury, acute kidney injury, glomerular disease, glomerular disease, diabetic nephropathy, reversible posterior encephalopathy syndrome, chronic renal failure, acute renal failure, etc.

In one aspect, the present invention relates to use of the mesenchymal stem cell population, the culture, the culture supernatant or the pharmaceutical composition as described herein for preventing and/or treating renal fibrosis in a subject, or in the manufacture of a medicament for preventing renal fibrosis, or delaying, or reducing renal fibrosis, or preventing or alleviating renal fibrosis in a subject.

In certain embodiments, the pharmaceutical composition is capable of restoring body weight and reducing urinary microalbumin (e.g., uric anhydride, urea, uric acid) in the subject.

In certain embodiments, the pharmaceutical composition is capable of improving kidney structure, reducing collagen deposition, and delaying progression of renal fibrosis.

In certain embodiments, the pharmaceutical composition is capable of inhibiting the expression of profibrogenic factor such as TGF-$\beta$1 in renal interstitium, and is capable of reversing interstitial transition, thereby protecting the kidney.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the medicament of the present invention can be administrated by intradermal injection, subcutaneous injection, intramuscular injection, intravenous injection, oral administration, etc. In certain preferred embodiments, the administration is performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

Nephrectomy-induced or related renal disease

Nephrectomy is a surgical operation to treat kidney disease. Its indications include renal malignancy, renal tuberculosis, severe hydronephrosis or kidney stones, severe renal injury and unilateral pyonephrosis.

In one aspect, the present invention relates to use of the mesenchymal stem cell population, the culture, the culture supernatant or the pharmaceutical composition as described herein for preventing and/or treating nephrectomy-induced injury or related renal disease in a subject, or in the manufacture of a medicament for preventing nephrectomy-induced injury or related renal disease, or delaying, or reducing nephrectomy-induced injury or related renal disease, or preventing or alleviating nephrectomy-induced injury or related renal disease in a subject.

In certain embodiments, the pharmaceutical composition is capable of restoring body weight.

In certain embodiments, the pharmaceutical composition is capable of reducing uric acid, urea, and uric anhydride.

In certain embodiments, the pharmaceutical composition is capable of generating protection effect against renal injury and improving renal function.

In certain embodiments, the pharmaceutical composition is capable of repairing kidney and inhibiting kidney injury-related disease, such as renal fibrosis and renal failure.

In certain embodiments, the subject is a mammal, such as a human.

In certain embodiments, the medicament comprises a therapeutically effective amount of the mesenchymal stem cell population and/or the culture and/or a therapeutically effective amount of the culture supernatant as described herein.

In certain embodiments, the mesenchymal stem cell population or the pharmaceutical composition as described herein is administered to a subject by local injection transplantation (e.g., stereotaxic intracerebral injection transplantation, or spinal cord local injection transplantation), blood circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain embodiments, the medicament of the present invention can be administrated by intradermal injection, subcutaneous injection, intramuscular injection, intravenous injection, oral administration, etc. In certain preferred embodiments, the administration is performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises pharmaceutically acceptable sterile isotonic solution or non-aqueous solution (e.g., balanced salt solution or normal saline), dispersion, suspension or emulsion. General principles regarding the formulation of this pharmaceutical composition may be found in Cell Therapy: Stem Cell Transplantation, Gene Therapy, and Cellular Immunotherapy, edited by G. Morstyn and W. Sheridan, Cambridge University Press, 1996; and Hematopoietic Stem Cell Therapy, E.D. Ball, J. Lister & P. Law, Churchill Livingstone, 2000.

In certain embodiments, the pharmaceutical composition comprises the aforementioned bioscaffold, or a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the medicament can be transplanted in the form of a suspension, gel, colloid, slurry or mixture.

## Graft versus host disease (GVHD)

Graft-versus-host disease (GVHD) refers to a pathological reaction that occurs between the transplanted tissue and the host recipient due to differences in minor histocompatibility antigens between donors and recipients. Graft-versus-host disease (GVHD) tends to attack epithelial tissues, especially skin, liver, and gastrointestinal tract mucosa. It is mainly divided into acute GVHD (which may cause adverse reactions such as skin, gastrointestinal tract and liver injury) and chronic GVHD (which may cause infections in more parts including lung and eye in addition to skin, gastrointestinal tract and liver). At present, GVHD is commonly seen after hematopoietic stem cell and solid organ transplantation. In addition, GVHD can also be seen as a potentially lethal clinical complication that may occur when allogeneic T lymphocytes are transplanted into immunocompromised patients. The examples showed that M cells had an obvious therapeutic effect on GVHD caused by hPBMC transplantation, improved the survival rate of chimeric mice, reduced the cell chimerism rate, and increased the expression of GVHD-induced anti-inflammatory factors in the intestine, kidney, liver and lung.

Graft versus host disease (GVHD) is mainly due to the fact that the T lymphocytes in allogeneic donor transplant after transplantation, through the influence of related cytokines in the recipient, enhance the immune response to the recipient antigens, and launch cytotoxic attack against target cells of recipient, of which the skin, liver and intestinal tract are the main targets. The occurrence of GVHD mainly depends on the following three points: (1) the graft contains immunocompetent cells; (2) the histocompatibility antigens of the donor and the recipient are different; (3) the immunocompetent cells of the donor survive because they are not rejected, and divide and proliferate when recognizing different histocompatibility antigens. It is generally believed that the immunocompetent cells involved in GVHD are the contaminated mature T cells, and the higher the contamination rate, the greater the probability of GVHD

In one aspect, the present invention provides a use of the mesenchymal stem cell population or its culture supernatant in the manufacture of a medicament for preventing, treating, delaying and/or alleviating a graft-versus-host disease; alternatively, the present invention provides a method for preventing, treating, delaying and/or alleviating a graft-versus-host disease, which comprises administering to a subject in need thereof a prophylactically and/or therapeutically effective amount of the mesenchymal stem cell population or its culture supernatant.

In certain embodiments, the mesenchymal stem cell population is capable of increasing the body weight of the subject.

In certain embodiments, the mesenchymal stem cell population is capable of improving the survival rate of the subject.

In certain embodiments, the mesenchymal stem cell population is capable of reducing the infiltration of exogenous cells into bone marrow, and/or reducing inflammatory response and tissue damage.

In certain embodiments, the mesenchymal stem cell population is capable of inhibiting inflammation, reducing a proinflammatory factor level, and increasing an anti-inflammatory factor level.

In certain embodiments, the subject is a mammal, such as a human.

In certain embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In certain preferred embodiments, the mesenchymal stem cell population of the present invention can be administered by intradermal injection, subcutaneous injection, intramuscular injection, intravenous injection, oral administration, and the like. In certain preferred embodiments, the administration may be performed by intravenous injection.

In certain embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In certain embodiments, the pharmaceutical composition is an injection (including injection solution, lyophilized powder). In certain embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may be found in Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In certain embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In certain embodiments, the mesenchymal stem cell population can be transplanted in the form of a suspension, gel, colloid, slurry or mixture.

In another aspect, the present invention also provides a product for preventing, treating, delaying and/or alleviating a graft-versus-host disease, comprising the mesenchymal stem cell population of the present invention. In certain embodiments, the product further comprises an additional active component as defined above. In certain embodiments, the mesenchymal stem cell population and the additional active component are present as separate components or as a single formulation. In certain embodiments, the product is an injection, microinjection, mucosal patch, enema, suppository, gel, oral preparation, aerosol, drop, ointment, implant, or capsule. In certain embodiments, the product is an implant.

## Heart disease

In one aspect, the present invention provides use of the mesenchymal stem cell population or its culture supernatant in the manufacture of a medicament for preventing and/or treating a cardiac system disease; alternatively, the present invention provides a method for preventing and/or treating a cardiac system disease, comprising administering to a subject in need thereof a prophylactically and/or therapeutically effective amount of the mesenchymal stem cell population or its culture supernatant.

Heart disease is a relatively common circulatory system disease. The circulatory system consists of the heart, blood vessels, and neurohumoral tissues that regulate blood circulation. Circulatory system diseases are also called cardiovascular diseases, including diseases of all the aforementioned tissues and organs. They are common diseases in internal medicine, among which heart disease is the most common and may significantly impact labor ability of patients.

1. Cardiomyopathy

Cardiomyopathy is a disease of heart muscle that affects the systolic or diastolic function of the heart and may even lead to heart failure or death. Cardiomyopathy may result from a heart injury (e.g., heart attack) or genetic disorder, and manifest as abnormal hypertrophy or dilation of the ventricles or other pathophysiological changes. Cardiomyopathy is divided into four categories: dilated cardiomyopathy, hypertrophic cardiomyopathy, restrictive cardiomyopathy, and arrhythmogenic cardiomyopathy.

2. Myocardial ischemia

Myocardial ischemia refers to a pathological state in which the blood perfusion of heart is reduced, resulting in a decrease in oxygen supply to the heart, abnormal myocardial energy metabolism, and inability to support the normal work of the heart.

3. Coronary heart disease

Coronary atherosclerotic heart disease is a common cardiovascular system disease caused by atherosclerosis of coronary arteries, resulting in stenosis or occlusion of lumen, leading to myocardial ischemia, hypoxia or necrosis, which is briefly referred to as coronary heart disease, also known as ischemic heart disease.

4. Myocardial infarction

Myocardial infarction, also known as miocrdil infrction, is a common acute cardiovascular disease. It is mainly due to the occlusion of main blood vessels supplying the heart and the interruption of blood flow, resulting in the ischemic necrosis of myocardium. It belongs to the category of acute coronary syndrome. Clinically, left ventricular myocardial infarction is more common, and patients are mainly manifested as severe and long-lasting chest pain, fever and frequent nausea, vomiting, etc., and even severe arrhythmia, shock, heart failure, etc., which seriously endanger their life.

5. Ischemia reperfusion

In recent years, with the progress of treatment for shock and the establishment and promotion of arterial bypass surgery, thrombolysis therapy, percutaneous transluminal coronary angioplasty, cardiopulmonary bypass, cardiopulmonary cerebral resuscitation, amputated limb replantation and organ transplantation, and other methods, many tissues and organs regain blood perfusion after ischemia, which is called ischemia reperfusion.

6. Ischemia reperfusion injury

After tissue ischemia caused by various factors, the tissue restores blood reperfusion after effective intervention, so that the function of tissues and organs is restored, the damaged structure is repaired, and the patient's condition is improved and recovered; but sometimes the reperfusion after ischemia not only does not restore the function of tissues and organs, but aggravates the dysfunction and structural damage of tissues and organs. This kind of phenomenon in which tissue damage is aggravated after blood flow is restored on the basis of ischemia, and even irreversible damage occurs, is called ischemia-reperfusion injury.

Prevention and/or treatment of myocardial ischemia/reperfusion injury (MI/RI)

Ischemic heart disease is the leading cause of death in humans, and early and successful recovery of myocardial reperfusion is the most effective way to improve clinical outcomes. However, the process of restoring blood flow to the ischemic myocardium may cause damage, and this phenomenon is called myocardial ischemia/reperfusion injury (MI/RI). I/R will bring some adverse effects, such as oxidative stress, intracellular calcium overload, etc., and these adverse effects may lead to cardiomyocyte apoptosis. Apoptosis is an important reason for the loss of tissue function in ischemia-reperfusion injury. It is a very complex process, and its detailed triggering mechanism is not completely clear.

In one aspect, the present invention relates to use of the mesenchymal stem cell population, the culture, the culture supernatant or pharmaceutical composition as described herein in the manufacture of a medicament for preventing and/or treating a myocardial ischemia/reperfusion injury, delaying or reducing a myocardial ischemia/reperfusion injury, or preventing or alleviating a myocardial ischemia/reperfusion injury; alternatively, the present invention relates to a method for preventing and/or treating a myocardial ischemia/reperfusion injury, delaying or reducing a myocardial ischemia/reperfusion injury, or preventing or alleviating a myocardial ischemia/reperfusion injury, which comprises administering to a subject in need thereof an effective amount of the mesenchymal stem cell population, the culture, the culture supernatant or the pharmaceutical composition as described herein.

In some embodiments, the medicament is capable of improving cardiac function, reducing cardiac infarct size, ameliorating myocardial ischemia, or/and reducing myocardial presystolic resistance or load.

In some embodiments, the subject is a mammal, such as a human.

In some embodiments, the medicament comprises a therapeutically effective amount of the mesenchymal stem cell population and/or the culture and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some embodiments, the medicament can be administered by intradermal injection, subcutaneous injection, intramuscular injection, intravenous injection, oral administration, and the like. In some preferred embodiments, the administration may be performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

In another aspect, the present invention also provides a product for preventing, treating, delaying and/or alleviating a cardiac system disease, comprising the mesenchymal stem cell population of the present invention. In some embodiments, the product further comprises an additional active component as defined above. In some embodiments, the mesenchymal stem cell population and the additional active component are present as separate components or as a single formulation. In some embodiments, the product is an injection, microinjection, mucosal patch, enema, suppository, gel, oral preparation, aerosol, drop, ointment, implant, or capsule. In some embodiments, the product is an implant.

Anemia

In one aspect, the present invention provides use of the mesenchymal stem cell population or its culture supernatant in the manufacture of a medicament for preventing and/or treating anemia; alternatively, the present invention provides a method for preventing and/or treating anemia, comprising administering to a subject in need thereof a prophylactically and/or therapeutically effective amount of the mesenchymal stem cell population or its culture supernatant.

Anemia refers to a syndrome in which the volume of red blood cells in the human peripheral blood decreases, which is lower than the lower limit of normal range, and cannot transport enough oxygen to the tissues. It is mainly divided into:

1. Anemia with reduced erythropoiesis

If any one and/or several of the three major elements, i.e., hematopoietic cells, hematopoietic regulation and hematopoietic raw materials, are abnormal, resulting in decreased erythropoiesis, anemia occurs.

2. Anemia caused by excessive destruction of red blood cells

Red blood cells are destroyed due to their own factors and/or external factors, and their lifespan is shortened. Anemia occurs when the rate of destruction of red blood cells exceeds the bone marrow's compensatory capacity.

3. Hemorrhagic anemia

It is a syndrome in which the body's blood volume decreases due to trauma and/or disease, leading to a decrease in the oxygen-carrying capacity of the blood.

4. Megaloblastic anemia

The anemia caused by the disorder of nuclear deoxyribonucleic acid synthesis due to the lack of folic acid or vitamin B12 or some drugs that affect nucleotide metabolism is called megaloblastic anemia. The disease is characterized by macrocytic anemia, with megaloblastic, granulocyte and megakaryocyte series in the bone marrow.

5. Aplastic anemia

It is a kind of bone marrow hematopoietic failure that may be caused by different etiologies and mechanisms, and mainly manifested as a syndrome with low marrow hematopoietic function, pancytopenia, and the resulting hypohemia, haemorrhage and infection.

Anemia is a common complication of cancer patients, and 50% of cancer patients have anemia, which not only brings a variety of clinical symptoms, but also reduces the life quality of patients, and is also one of the factors that affect the prognosis. Additionally, it may also cause many bad consequences due to the increase of blood transfusions. Tumor-associated anemia is a result of multiple factors, most of which are caused by the tumor itself, and it belongs to chronic anemia; in addition, the use of cytotoxic drugs or nephrotoxic drugs for chemotherapy may also cause anemia. Cisplatin is a widely used chemotherapeutic drug in patients, it has renal toxicity, and with the increase of the cumulative dose of cisplatin, anemia is exacerbated. Although erythropoietin can relieve chronic cancer anemia, it has been reported in the literature that its effective rate for correcting anemia is about 60%. How to further improve the anemia of tumor patients and improve the prognosis and life quality of the patients is still an important topic.

In one aspect, the present invention relates to use of the mesenchymal stem cell population, the culture, the culture supernatant or the pharmaceutical composition as described herein in the manufacture of a medicament for preventing and/or treating an anemia (for example, but not limited to tumor-associated anemia, chronic anemia, anemia caused by chemotherapy with cytotoxic or nephrotoxic drugs), delaying or alleviating a scleroderma, or preventing an anemia (for example, but not limited to tumor-associated anemia, chronic anemia, anemia caused by chemotherapy with cytotoxic or nephrotoxic drugs); alternatively, the present invention relates to a method for preventing and/or treating an anemia (for example, but not limited to tumor-associated anemia, chronic anemia, anemia caused by chemotherapy with cytotoxic or nephrotoxic drugs), delaying or alleviating a scleroderma, or preventing an anemia (for example, but not limited to tumor-associated anemia, chronic anemia, anemia caused by chemotherapy with cytotoxic or nephrotoxic drugs), which comprises administering to a subject in need thereof an effective amount of the mesenchymal stem cell population, the culture, the culture supernatant or the pharmaceutical composition as described herein.

In some embodiments, the medicament is capable of increasing body weight in the treatment of anemia.

In some embodiments, the medicament is capable of increasing the number of peripheral white blood cells, increasing the number of peripheral red blood cells, reducing the volume of peripheral red blood cells, restoring a blood routine index of peripheral blood, restoring a hemoglobin index of peripheral blood, or restoring bone marrow in the treatment of anemia.

In some embodiments, the subject is a mammal, such as a human.

In some embodiments, the medicament comprises a therapeutically effective amount of the mesenchymal stem cell population and/or the culture and/or a therapeutically effective amount of the culture supernatant as described herein.

In some embodiments, the mesenchymal stem cell population or the pharmaceutical composition as described herein is administered to a subjected by local injection transplantation (e.g., stereotaxic intracerebral injection transplantation, or spinal cord local injection transplantation), circulatory route transplantation (e.g., intravenous injection transplantation, or intra-arterial injection transplantation), or cerebrospinal fluid route transplantation (e.g., lumbar puncture subarachnoid injection transplantation). Those skilled in the art know how to select an appropriate cell transplantation route according to the location and nature of the lesion.

In some embodiments, the medicament of the present invention can be administered by intradermal injection, subcutaneous injection, intramuscular injection, intravenous injection, oral administration, and the like. In some preferred embodiments, the administration may be performed by intravenous injection.

In some embodiments, the pharmaceutical composition may be in any form known in the medical field. For example, the pharmaceutical composition can be in the form of tablet, pill, suspension, emulsion, solution, gel, capsule, powder, granule, elixir, lozenge, suppository, injection (including injection solution, lyophilized powder) etc. In some embodiments, the pharmaceutical composition is an injection (including injection solution, lyophilized powder). In some embodiments, the pharmaceutical composition comprises a sterile pharmaceutically acceptable isotonic aqueous or non-aqueous solution (e.g., balanced salt solution or normal saline), dispersion, suspension or emulsion. General principles regarding the formulation of this pharmaceutical composition may refer to Cell Therapy: stem cell Transplantation, Gene Therapy, and Cellular Immunotherapy, edited by G. Morstyn and W. Sheridan, Cambridge University Press, 1996; and Hematopoietic stem cell treatment, E.D. Ball, J. Lister & P. Law, Churchill Livingstone, 2000.

In some embodiments, the pharmaceutical composition comprises a pharmaceutically acceptable biomaterial, including but not limited to, collagen scaffold, Matrigel, skin repair membrane, aminated gelatin, chitosan, silk fibroin, cellulose polylactic acid, elastic proteinogen, hyaluronic acid, etc.

In some embodiments, the mesenchymal stem cell population can be transplanted in the form of a suspension, gel, colloid, serous fluid or mixture.

In another aspect, the present invention also provides a product for preventing, treating, delaying and/or alleviating an anemia, comprising the mesenchymal stem cell population of the present invention. In some embodiments, the product further comprises an additional active component as defined above. In some embodiments, the mesenchymal stem cell population and the additional active component are present as separate components or as a single formulation. In some embodiments, the product is an injection, microinjection, mucosal patch, enema, suppository, gel, oral preparation, aerosol, drop, ointment, implant, or capsule. In some embodiments, the product is an implant.

### Definition of terms

In the present invention, unless otherwise specified, the scientific and technical terms used herein have the meanings commonly understood by those skilled in the art. In addition, the operation steps of molecular genetics, nucleic acid chemistry, cell culture, biochemistry, cell biology and so on as used herein are all routine steps widely used in the corresponding fields. Meanwhile, for a better understanding of the present invention, definitions and explanations of related terms are provided below.

As used herein, the term "embryoid body (EB)" has the meaning commonly understood by those skilled in the art, which refers to a cell aggregate formed by pluripotent stem cells (e.g., embryonic stem cells), and may also be known as "EB sphere". Methods for inducing pluripotent stem cells (e.g., embryonic stem cells) to form embryoid body generally comprises: allowing the suspended pluripotent stem cells to aggregate and form an embryoid body by preventing the pluripotent stem cells from attaching to the surface of culture container.

As used herein, the term "in vitro" refers to an artificial environment, and the processes and reactions therein. The in vitro environment is exemplified by, but not limited to, test tubes and cell cultures.

As used herein, the term "in vivo" refers to a natural environment (i.e., animal or cell) and the processes and reactions therein.

As used herein, the term "culture" refers to a product obtained after culturing cells (e.g., the mesenchymal stem cell population of the present invention) in a culture medium.

As used herein, the term "culture supernatant" refers to a culture solution that is obtained by culturing cells (e.g., the mesenchymal stem cell population of the present invention) but does not contain the cells themselves. Therefore, a culture supernatant usable in the present invention can be obtained, for example, by separating and removing cell components after culturing. The culture supernatant may also be subjected to other treatments, such as centrifugation, concentration, solvent replacement, dialysis, freezing, drying, freeze drying, dilution, desalting, preservation, and the like.

As used herein, the term "pharmaceutically acceptable carrier or excipient" refers to a carrier and/or excipient that is pharmacologically and/or physiologically compatible with the subject and active component, which are well known in the art (see, for example, Remington's Pharmaceutical Sciences. Edited by Gennaro AR, 19th ed. Pennsylvania:

Mack Publishing Company, 1995) and include, but are not limited to: pH adjusting agent, surfactant, ionic strength enhancer, agent for maintaining osmotic pressure, agent for delaying absorption, diluent, adjuvant, preservative, etc. For example, pH adjusting agent includes, but is not limited to, phosphate buffer. Surfactant includes, but is not limited to, cationic, anionic or nonionic surfactant, such as Tween-80. Ionic strength enhancer includes, but is not limited to, sodium chloride. Agent for maintaining osmotic pressure includes, but is not limited to, sugar, NaCl, and the like. Agent for delaying absorption includes, but is not limited to, monostearate salt and gelatin. Diluent includes, but is not limited to, water, aqueous buffer (e.g., buffered saline), alcohol and polyol (e.g., glycerol), and the like. Adjuvant includes, but is not limited to, aluminum adjuvant (e.g., aluminum hydroxide), Freund's adjuvant (e.g., complete Freund's adjuvant), and the like. Preservative includes, but is not limited to, various antibacterial and antifungal agents, such as thimerosal, 2-phenoxyethanol, paraben, chlorobutanol, phenol, sorbic acid, and the like. In certain embodiments, the pharmaceutically acceptable carrier or excipient is a sterile isotonic aqueous or non-aqueous solution (e.g., balanced salt solution or normal saline), dispersion, suspension or emulsion.

As used herein, the term "prevention" refers to a method performed in order to prevent or delay the occurrence of a disease or disorder or symptom in a subject or to minimize its effects if it occurs. The term "treatment" refers to a method performed to obtain a beneficial or desired clinical outcome. Beneficial or desired clinical outcome includes, but is not limited to, reduced rate of disease progression, improved or alleviated disease state, and regression or improved prognosis, whether detectable or undetectable. The amount of therapeutic agent effective to relieve symptoms of any particular disease may vary depending on factors such as the patient's disease state, age and weight, and the ability of drug to elicit a desired response in a subject. Remission of a disease symptom can be assessed by any clinical measures, and these measures are commonly used by physicians or other skilled healthcare providers to assess the severity or progressive state of the symptom.

As used herein, the term "effective amount" refers to an amount sufficient to obtain, or at least partially obtain, a desired effect. For example, a prophylactically effective amount refers to an amount sufficient to prevent, inhibit, or delay the onset of a disease; a therapeutically effective amount refers to an amount sufficient to cure or at least partially prevent a disease and its complication in a patient already suffering from the disease. Determining such effective amount is well within the ability of those skilled in the art. For example, a therapeutically effective amount depends on the severity of disease to be treated, the general state of patient's own immune system, the patient's general condition such as age, weight and sex, the mode of administration, and other concurrently applied therapies, etc.

As used herein, the term "subject" includes, but is not limited to, various animals, such as mammals, such as bovine, equine, ovine, porcine, canine, feline, leporidae, rodent (e.g., mouse or rat), non-human primate (e.g., rhesus or cynomolgus), or human.

Beneficial effects of the present invention

The mesenchymal stem cells prepared by the method of the present invention have a significantly increased MMP1 expression level, are suitable for the prevention and treatment of various diseases (e.g., inflammatory diseases, etc.), and have great clinical values.

**Brief Description of the Drawings**

FIG. 1 shows the flow cytometry detection of hESC-M cell surface protein expression. *, $P<0.05$; **, $P<0.01$.

FIGS. 2A to 2D show the qPCR detection of cytokine mRNA expression levels in hESC-M cells. *, $P<0.05$; **, $P<0.01$.

FIG. 3 shows the effect of hESC-M cell culture supernatant on the expression levels of $\alpha$-SMA and Collagen I in lung fibroblasts.

FIGS. 4A to 4C show the effects of IFN-$\gamma$ stimulation on the expression levels of IDO (FIG. 4A), PD-L1 (FIG. 4B), and PGE2 (FIG. 4C) in hESC-M cells.

FIG. 5 shows morphological photos of embryoid bodies and M cells at P0 and P5 generations formed by different formulations in Example 2 (II).

FIG. 6 shows the flow cytometry results of Formula 1-M cells in Example 2 (I).

FIG. 7 shows the flow cytometry results of Formula 2-M cells in Example 2 (I).

FIG. 8 shows the flow cytometry results of Formula 3-M cells in Example 2 (I).

FIG. 9 shows the flow cytometry results of Formula 4-M cells in Example 2 (I).

FIG. 10 shows the flow cytometry results of Formula 5-M cells in Example 2 (I).

FIG. 11 shows the expression of MMP1 detected by qPCR for the P5 generation of M cells produced with different formulations of the first culture medium in Example 2 (I).

FIG. 12 shows the expression of PGE2 detected by qPCR for the P5 generation of M cells produced with different formulations of the first culture medium in Example 2 (I).

FIG. 13 shows the cell morphology of the embryoid body and the P5 generation of M cells formed by different formulations in Example 2 (II).

FIG. 14 shows the flow cytometry results of Formula 1-M cells in Example 2 (II).

FIG. 15 shows the flow cytometry results of Formula 2-M cells in Example 2 (II).

FIG. 16 shows the flow cytometry results of Formula 3-M cells in Example 2 (II).

FIG. 17 shows the flow cytometry results of Formula 4-M cells in Example 2 (II).

FIG. 18 shows the flow cytometry results of Formula 5-M cells in Example 2 (II).

FIG. 19 shows the expression of MMP1 detected by qPCR for the P5 generation of M cells produced with different formulations of the first culture medium in Example 2 (II).

FIG. 20 shows the expression of PGE2 detected by qPCR for the P5 generation of M cells produced with different formulations of the first culture medium in Example 2 (II).

FIG. 21 shows the cell morphology of the P5 generation of M cells formed with different formulations in Example 2 (III).

FIG. 22 to FIG. 25 show the flow cytometry results of M cells prepared with different formulations in Example 2 (III).

FIG. 26 shows the expression of MMP1 detected by qPCR for the P5 generation of M cells produced with different formulations of the second culture medium in Example 2 (III).

FIG. 27 shows the expression of PGE2 detected by qPCR for the P5 generation of M cells produced with different formulations of the second culture medium in Example 2 (III).

FIG. 28 shows the cell morphology of the P5 generation of M cells formed by different formulations in Example 2 (IV).

FIG. 29 shows the flow cytometry results of M cells prepared with Formula 3-1 in Example 2 (IV).

FIG. 30 shows the flow cytometry results of M cells prepared with Formula 3-3 in Example 2 (IV).

FIG. 31 shows the flow cytometry results of M cells prepared with Formula 3-4 in Example 2 (IV).

FIG. 32 shows the expression of MMP1 detected by qPCR for the P5 generation of M cells produced with different formulations in Example 2 (IV).

FIG. 33 shows the expression of PGE2 detected by qPCR for the P5 generation of M cells produced with different formulations in Example 2 (IV).

FIG. 34 shows the cell morphology of the P5 generation of M cells formed by different formulations in Example 2 (V).

FIG. 35: Flow cytometry results of M cells prepared by Formula 3-2-3 in Example 2 (V).

FIG. 36 shows the flow cytometry results of M cells prepared with Formula 3-5-4 in Example 2 (V).

FIG. 37 shows the expression of MMP1 detected by qPCR for the P5 generation of M cells produced with different formulas in Example 2 (V).

FIG. 38 shows the expression of PGE2 detected by qPCR for the P5 generation of M cells produced with different formulations in Example 2 (V).

FIG. 39 shows the schematic diagram of the apparatus for spraying M cells.

FIG. 40 shows the detection of proliferative capacity of M cells before and after spray. Before and after spray of M cells, CCK8 was used to detect the change of proliferative capacity. After spray of M cells, the morphology was normal, and the proliferative capacity was not much different from that of non-sprayed cells, indicating that the spray system could well support the application of spray of M cells.

FIG. 41 shows the fluorescent photo of GFP-labeled M cells inoculated on collagen scaffold. The results showed that the GFP-labeled M cells could well adhere and grow on the collagen material.

FIG. 42 shows the fluorescent photo of GFP-labeled M cells inoculated on electrospun gelatin fibers. The results showed that the GFP-labeled M cells could well adhere and grow on the electrospun gelatin fibers.

FIG. 43 shows the fluorescent photo of GFP-labeled M cells inoculated on collagen scaffold. The results showed that GFP-labeled M cells could well adhere and grow on the collagen scaffold.

FIG. 44 shows the fluorescent photo of GFP-labeled M cells inoculated on skin repair membrane. The results showed that the GFP-labeled M cells could well adhere and grow on the skin repair membrane.

FIG. 45 shows the co-culture of M cells with collagen, hyaluronic acid, sodium alginate hydrogels. The results showed that the GFP-labeled M cells could well adhere and grow on the skin repair membrane.

FIG. 46 shows the cell viability assay results of M cells resuspended in normal saline.

FIG. 47 shows the cell viability assay results of M cells resuspended in lactated Ringer's injection solution.

FIG. 48 shows the cell viability assay results of M cells resuspended in compound electrolyte injection.

FIG. 49 shows the cell viability assay results of M cells resuspended in 5% glucose inj ection.

FIG. 50 shows the cell viability assay results of M cells resuspended in 20% HSA injection.

FIG. 51 shows the cell viability assay results of M cells resuspended in succinylated gelatin injection solution.

FIG. 52 shows the cell viability assay results of M cells resuspended in MZJ injection solution 1.

FIG. 53 shows the cell viability assay results of M cells resuspended in succinylated gelatin MIX injection solution.

FIG. 54 shows the cell viability assay results of M cells frozen with MZJ injection solution 1 at 80°C .

FIG. 55 shows M cells cultured with Cytodex3.

FIG. 56 shows the digestion of M cells cultured with Cytodex3.

FIG. 57 shows the live-dead assay results of M cells cultured with Cytodex3.

FIG. 58 shows the in situ cryopreservation assay results of M cells cultured with Cytodex3.

FIG. 59 shows the M cells cultured with Cultispher.

FIG. 60 shows the digestion of M cells cultured with Cultispher.

FIG. 61 shows the in situ cryopreservation assay results of M cells cultured with Cultispher.

FIG. 62 shows the M cells cultured with TableTrix.

FIG. 63 shows the digestion of M cells cultured with TableTrix.

FIG. 64 shows the live-dead assay results of M cells cultured with TableTrix.

FIG. 65 shows the in situ cryopreservation assay results of M cells cultured with TableTrix.

FIG. 66 shows the M cells cultured with Solohill.

FIG. 67 shows the digestion of M cells cultured with Solohill.

FIG. 68 shows the live-dead assay results of M cells cultured with Solohill.

FIG. 69 shows the in situ cryopreservation assay results of M cells cultured with Solohill.

FIG. 70 shows the M cells cultured on Coring polystyrene microcarrier.

FIG. 71 shows the digestion of M cells cultured on Coring polystyrene microcarrier.

FIG. 72 shows the live-dead assay results of M cells cultured on Coring polystyrene microcarrier.

FIG. 73 shows the in situ cryopreservation assay results of M cells cultured on Coring polystyrene microcarrier.

FIG. 74 shows the M cells cultured on the microcarrier prepared by 8GeL-toluene method.

FIG. 75 shows the live-dead assay results of M cells cultured on the microcarrier prepared by 8GeL-toluene method.

FIG. 76 shows the passage of M cells cultured on the microcarrier prepared by 8GeL-toluene method. M cells cultured on the microcarrier prepared by 8GeL-toluene method, could be passaged from sphere to sphere and proliferate.

FIG. 77 shows the live-dead assay of M cells cultured on the microcarrier prepared by 8GeL-toluene method.

FIG. 78 shows the M cells cultured with 25GF-Gel microcarrier.

FIG. 79 shows the live-dead assay results of the M cells cultured with 25GF-Gel microcarrier.

FIG. 80 shows the passage of the M cells cultured with 25GF-Gel microcarrier.

FIG. 81 shows the live-dead assay of the M cells cultured with 25GF-Gel microcarrier.

FIG. 82 shows the M cells cultured with Gel microcarrier.

FIG. 83 shows the live-dead assay results of the M cells cultured with Gel microcarrier.

FIG. 84 shows the passage of the M cells cultured with Gel microcarrier.

FIG. 85 shows the live-dead assay of the passage of M cells cultured with Gel microcarrier.

FIG. 86 shows the M cells cultured with 25GF-2HA microcarrier.

FIG. 87 shows the live-dead assay results of the M cells cultured with 25GF-2HA microcarrier.

FIG. 88 shows the Masson staining results of lung tissue of pneumoconiosis model mice in Example 43.

FIG. 89 shows the M cells cultured with 25GF-2HA microcarrier.

FIG. 90 shows the live-dead assay results of the M cells cultured with 25GF-2HA microcarrier.

FIG. 91 shows the M cells cultured with Alg microcarrier.

FIG. 92 shows the live-dead assay results of the M cells cultured with Alg microcarrier.

FIG. 93 shows the M cells cultured with Alg-lysine microcarrier.

FIG. 94 shows the live-dead assay results of the M cells cultured with Alg-lysine microcarrier.

FIG. 95 shows the M cells cultured with Gel-lysine microcarrier.

FIG. 96 shows the live-dead assay results of the M cells cultured with Gel-lysine microcarrier.

FIG. 97 shows the digestion of the M cells cultured with Gel-lysine microcarrier.

FIG. 98 shows the live-dead assay results of the passage of M cells cultured with Gel microcarrier.

FIG. 99 shows the live-dead assay results of M cells dynamically cultured on TableTrix microcarrier.

FIG. 100 shows the flow cytometry results of M cells dynamically cultured on TableTrix microcarrier.

FIG. 101 shows the light microscope photos of the uteruses in Example 9 of the present invention. The uterine adhesions in the model group were more obvious and uterine dropsies were more severe; while in the M cell group, the uterine adhesions and uterine dropsies showed a significant improvement. This showed that the M cells could be well used in the treatment of intrauterine adhesions.

FIG. 102 shows the HE-staining pictures of uteruses in Example 9 of the present invention. In the model group, the endometrium became thinner and the glands disappeared; while in the M cell group, it was observed that both of endometrium and myometrium were thicker, with a large number of blood vessels, glands and cells, the intrauterine adhesions were significantly improved, the uterine cavity morphology was restored, and the fluid accumulated in uterine was reduced, indicating that the M cells could promote the repair and regeneration of damaged endometrium.

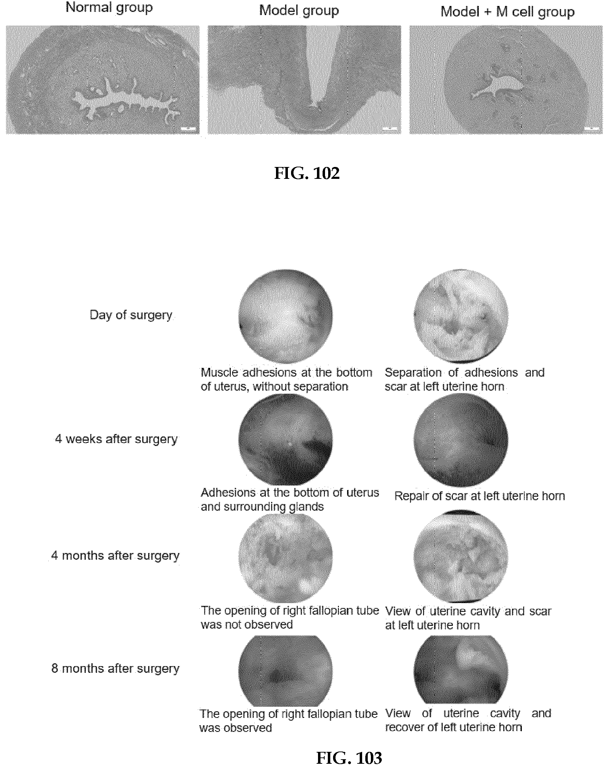

FIG. 103 shows the clinical treatment of intrauterine adhesions by the M cells in Example 9 of the present invention. The patient had moderate-severe intrauterine adhesions, with muscle adhesion at the bottom of the uterus and formation of scars. The fallopian tube was obstructed and the opening was not obvious. After treatment with M cells by uterine wall injection, the patient's uterine shape returned to normal, no adhesions was were found, the scar was repaired, and the bilateral fallopian tube openings were visible. The results suggested that the M cells could be used for clinical treatment of intrauterine adhesions. The figure showed that (1) the patient had intrauterine adhesions and bilateral ureteral obstruction and the adhesions were moderate-severe (mixed type) at the time of surgery; the M cells ($3 \times 10^6$ cells) were injected after separation of the adhesion; (2) during the 4-weeks follow-up, it was found that the scar was recovered; (3) during the 4-months follow-up, the condition of the uterine cavity was poor; and (4) during the 8-months follow-up, the uterine cavity basically returned to normal.

FIG. 104 shows the statistical graph of the survival rate of mice in each of the groups in Example 10 of the present invention.

FIG. 105 shows the statistical diagram of the serum liver function detection of mice in each of the groups in Example 10 of the present invention.

FIG. 106 shows the statistics of the incidence of ALS mice in Example 11 of the present invention. The results showed that the incidence of the M cell group was significantly lower than that of the solvent group (50% vs. 80%) after 27 weeks, and the M cells significantly delayed the onset in the mice.

FIG. 107 shows the rotarod behavior statistics of the mice in Example 11 of the present invention. The residence time of ALS mice on the rotarod in the M cell group was significantly higher than that in the solvent group, especially after 29 weeks (144 vs. 244), with a significant difference, **$p<0.01$; indicating that the M cells could significantly improve the exercise performance of mice.

FIG. 108 shows the grasping force behavioral statistics of the mice in Example 11 of the present invention. The grasping force of mice in the M cell group was higher than that in the solvent group, with a significant difference after 29 weeks, **$p<0.01$; it showed that the M cells significantly improved muscle strength in mice, which indirectly indicated that the M cells could reduce motor neuron damage.

FIG. 109 shows the flow chart of the inflammatory bowel disease induced by 2.5% DSS in Example 12 of the present invention. There were 6 mice in each group, and the mice were induced by drinking water for 7 days. On the $0^{th}$, $3^{rd}$, and $6^{th}$ days, 300 $\mu$l of cell suspension was intraperitoneally injected with an amount of $3\times10^6$ cells, and the cells were M cells of the P5 generation. Perfusion sampling was performed on the 11th day.

FIG. 110 shows the light microscope photos of the mouse colon samples of Example 12 of the present invention. On the $11^{th}$ day, the mice were anesthetized and perfused for sampling, and the colons of the mice were photoed and subjected to statistics.

FIG. 111 shows the mouse colon length of Example 12 of the present invention. The statistics of length of the sampled mouse colons was performed, and it was found that the length of colon in the M cell group was longer than that in the 2.5% DSS group, and the length of colon in the M cell treatment group was closer to that of the normal group, indicating that the intraperitoneal injection of M cells could play a role in the protection of mouse colon.

FIG. 112 shows the pictures of the HE-stained mouse colons in Example 12 of the present invention. The structure of colon tissue of the mice in the M cell treatment group was intact with less inflammatory cell infiltration, while the mice in the 2.5% DSS induction group had more colonic inflammatory cell infiltration with destroyed intestinal crypt structure, indicating that the intraperitoneal injection of M cells could plays a role in the protection of mouse colon, and had an effective therapeutic effect on inflammatory bowel disease.

FIG. 113 shows the statistics of pathological score of the HE-stained mouse colon in Example 12 of the present invention. The pathological score of mouse colon HE section of the M cell group was significantly lower than that of the 2.5% DSS group, and there was a statistical difference, indicating that the M cells could have good protective and therapeutic effects on the intestinal tract of mice.

FIG. 114 shows the flow chart of 5% DSS-induced inflammatory bowel disease in Example 12 of the present invention. The induction of mice were performed by drinking water for 7 days, and on the $0^{th}$, $3^{rd}$, and $6^{th}$ day, 300 $\mu$l of cell suspension was intraperitoneally injected, with a cell amount of $3\times10^6$, and the cells were P5 generation M cells.

FIG. 115 shows the pictures of the HE-stained mouse colons in Example 12 of the present invention. The structure of colon tissue of the mice in the M cell treatment group was intact with less inflammatory cell infiltration, while the mice in the 5% DSS induction group had more colonic inflammatory cell infiltration with completely destroyed intestinal crypt structure, indicating that the M cells could well protect the colon tissue of mice and play an effective therapeutic role in inflammatory bowel disease.

FIG. 116 shows the pathological score statistics of the HE-stained mouse colons in Example 12 of the present invention. The score of the M cell group was significantly lower than that of the 5% DSS group, and there was a statistical difference, indicating that the M cells could have good protective and therapeutic effects on the intestinal tract of mice.

FIG. 117 shows the statistics of survival rate of the mice in Example 12 of the present invention. On the $11^{th}$ day, all the mice in the model group died, while 7 mice of the M cell group still survived (modeling, n=12) with stable vital signs at the end of the experiment (the $27^{th}$ day), indicating that the M cell treatment could greatly improve the survival rate of mice.

FIG. 118 shows the photos of wounds on different days in Example 13 of the present invention. The light microscope photos of the control group and the M cell group on different days showed that the wounds of the M cell group were significantly smaller than those of the control group on the $14^{th}$ and $21^{st}$ days. It shows that the M cells could accelerate the healing of skin wounds.

FIG. 119 shows the wound area size statistics of Example 13 of the present invention. For the statistics of wound area in FIG. 118, ImageJ was used for statistics. In the M cell treatment group, the wound area was significantly smaller than that in the control group. For the treatment of skin damage, the M cells showed a better treatment trend, indicating that the M cells could effectively treat skin damage.

FIG. 120 shows the wound non-healing rate statistics of Example 13 of the present invention. Non-healing rate = (wound area/initial wound area) $\times$ 100%. According to the non-healing rate statistics, that of the M cell group was significantly lower than that of the control group, and showed a better treatment trend, indicating that the M cells could well treat skin damage and accelerate the healing of skin wounds.

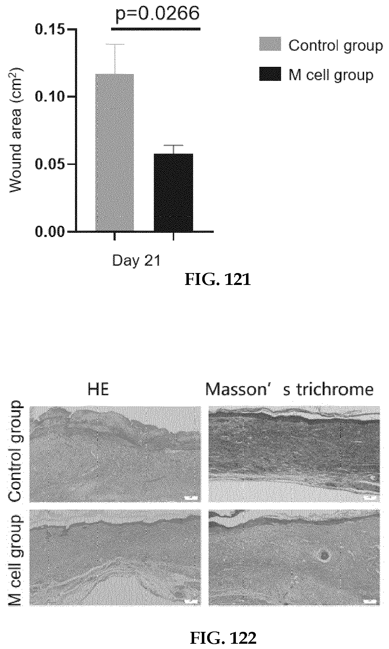

FIG. 121 shows the wound area size statistics on the 21st day of Example 13 of the present invention. The unhealed area of the control group was 11.7% of the initial wound area, while the unhealed wound area of the M cell treatment group was 5.7% of the initial wound area, and there was a significant difference, that was, that of the M cells was significantly lower than that of the control group, indicating the M cells showed a better treatment trend and could well treat skin damage and accelerate the healing of skin wounds. One-way ANOVA in Prism 7.0 statistical analysis software was used for analysis of variance and significance test, and the experimental data were expressed as mean $\pm$ standard error (Mean $\pm$ SE). *, $p<0.05$; **, $p<0.01$; ***, $p<0.001$.

FIG. 122 shows the HE and Masson staining in Example 13 of the present invention. The results of HE staining showed that the wound healing in the M cell group was better than that in the control group. The M cell group showed complete healing, that was, the epidermis had healed completely, while the middle part of the epidermis of the wound in the control group had not yet healed completely. The results of Masson staining showed that in the control group, the coloration was darker with more deposited collagen, indicating severe fibrosis; while in the M cell treatment group, the coloration was shallow with less deposited collagen, and the degree of fibrosis was lower than that of the control group. After the transplantation treatments with M cells and Matrigel, the wound recovery speed of the rat skin injury was accelerated; the tissue section showed less fibrosis.

FIG. 123 shows the light microscope photos of the testis in Example 14 of the present invention. The results of the samples on the 20th day showed that after transplantation of M cells, the testis of the rats recovered significantly, while the testis of the model group showed obvious atrophy, and the seminal vesicles became smaller.

FIG. 124 shows the weight ratio of left testis to right testis in Example 14 of the present invention. The M cell group had a ratio of left testis to right testis which was significantly higher than that in the model group, and produced more sperm with less unhealthy sperm. One-way ANOVA in Prism 7.0 statistical analysis software was used for analysis of variance and significance test, and the experimental data were expressed as mean $\pm$ standard error (Mean $\pm$ SE). *, $p<0.05$; **, $p<0.01$; ***, $p<0.001$.

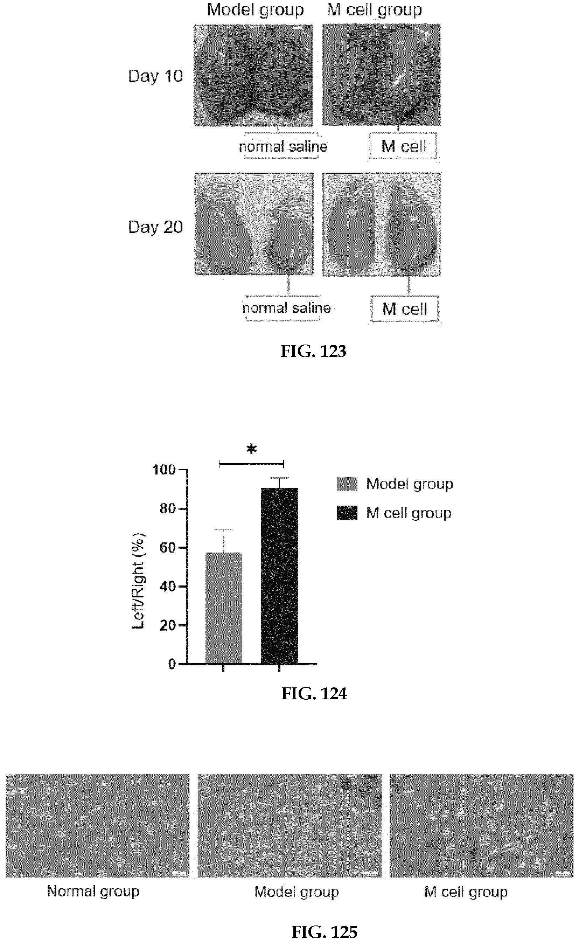

FIG. 125 shows the HE-staining pictures of testis in Example 14 of the present invention. HE staining results showed that there were Sertoli cells on the wall of testicular seminiferous tubules, cells formed in the center of the tubular cavity, and a large number of germ cells seen in the tubular cavity, in the rats of the M cell transplantation group; while in the model group, there were only Sertoli cells in the seminiferous tubules, and no new cells were formed. This showed that the M cells had a good protective effect on the testis of azoospermic rats, which helped the recovery of rat testis and had protective effect on germ cells.

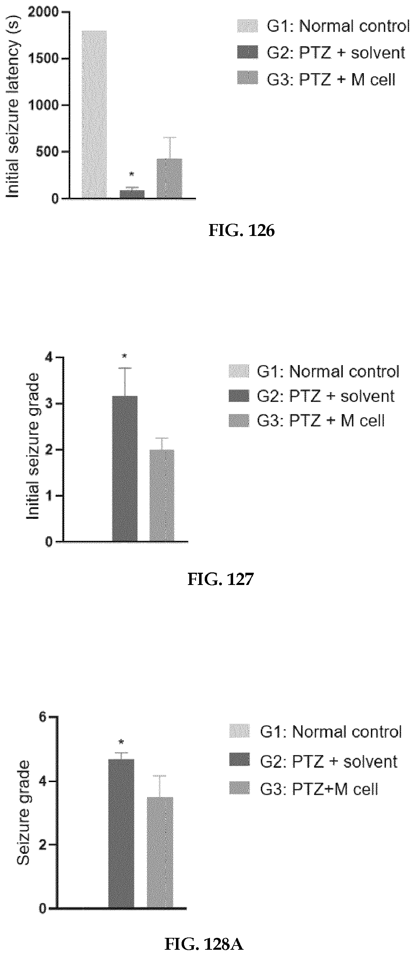

FIG. 126 shows the statistics of the initial epileptic seizure latency of rats in Example 15 of the present invention. The total observation time for the statistics of the initial epileptic seizure latency in rats was 30 minutes. Compared with the normal control group, the initial epileptic seizure latency of the solvent group was significantly shortened, with a significant difference, * $p<0.05$; the initial epileptic seizure latency of the solvent group was 92.2 seconds, and the initial epileptic seizure latency of the M cell group was 429.5 seconds, the time was significantly prolonged, indicating that the M cells could delay epileptic seizures.

FIG. 127 shows the statistics of the initial epileptic seizure grades of rats in Example 15 of the present invention. The M cell group down-regulated the seizure grade, from grade 3 to grade 2, indicating that the M cells could alleviate epileptic seizures.

FIG. 128A shows the statistics of the initial epileptic seizure grades of rats in Example 15 of the present invention.

The tail vein injection of M cells significantly reduced the epileptic seizure grade, indicating that the M cells could alleviate epileptic seizures.

FIG. 128B shows the statistics of the grand mal seizure latency of rats in Example 15 of the present invention.

FIG. 129 shows the proportion statistics of the grand mal seizures of rats in Example 15 of the present invention. The tail vein injection of M cells significantly reduced the proportion of grand mal seizures, and as compared with the solvent group, the proportion decreased by 43.3%, indicating that the M cells could significantly reduce the frequency of grand mal seizures.

FIG. 130 shows the back photos of the mice on the 28th day in Example 16 of the present invention. When the samples were taken on the 28th day, in the BLM group, the skin of the BLM injection site was thickened, hardened, and its elasticity became poor; while in the M cell injection group, the mouse skin was closer to that of the normal group, and no sclerosis occurred, indicating that the M cells could reduce the occurrence of sclerosis and thickening of skin, and had effective therapeutic effect on scleroderma.

FIG. 131 shows the HE-staining pictures of the mouse skins in Example 16 of the present invention. It could be seen that in the BLM group, the dermis was significantly thickened, and the number of hair follicles was significantly reduced; while in the M cell treatment group, there were more hair follicles and thinner dermis as compared with the BLM group, showing a significant improvement, which indicated that the M cell treatment could well alleviate the symptoms of scleroderma and had an effective therapeutic effect on scleroderma.

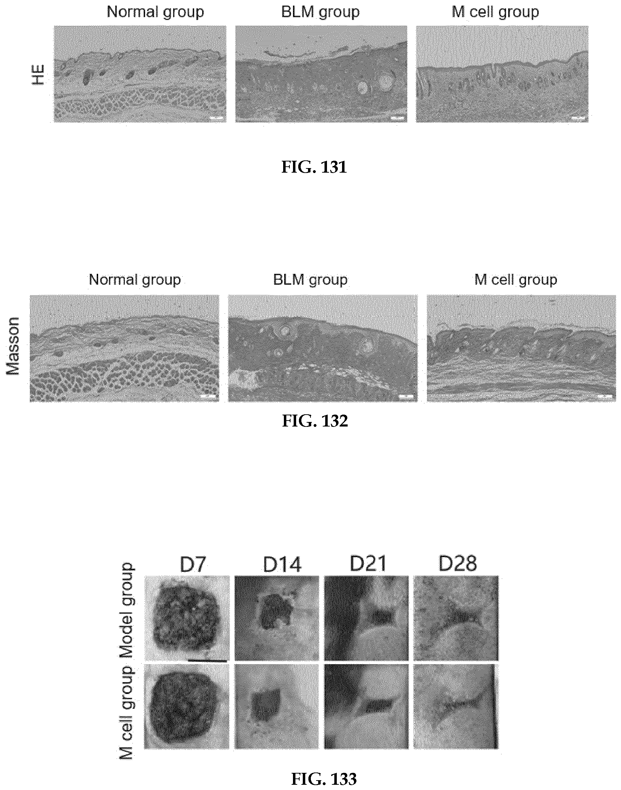

FIG. 132 shows the Masson-staining pictures of the mouse skins in Example 16 of the present invention. Compared with the normal group, in the BLM group, the collagen fibers were significantly thickened and enlarged, the obvious fibrosis occurred, the dermis was significantly thickened, the fat layer was significantly thinned, and skin appendages were reduced; while after the treatment with M cells, the accumulation of collagen fibers decreased, the skin dermis was obviously thinned, the fat layer was not thinned, and normal skin appendages were observed. This indicated that the M cells could well treat scleroderma in mice.

FIG. 133 shows the light microscope photos of skin wounds at different time points after modeling in Example 17 of the present invention, in which the wounds in the M cell group were smaller than those in the model group.

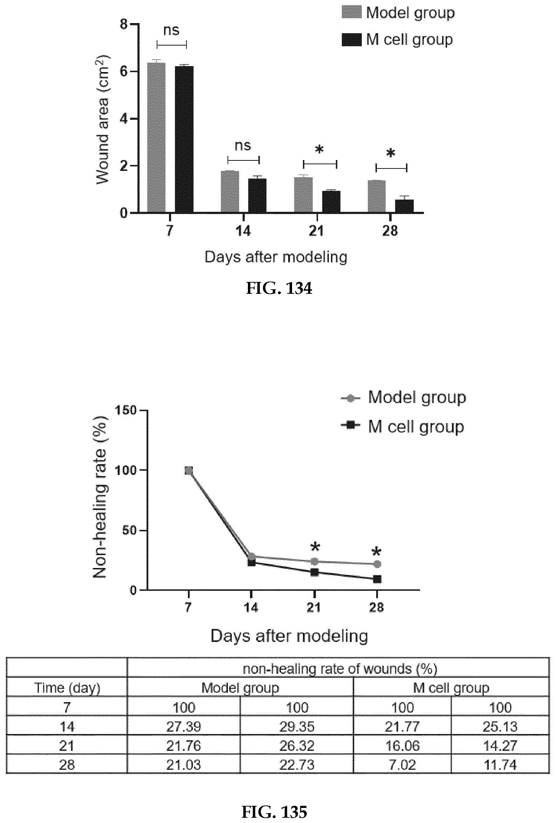

FIG. 134 shows the area size statistics of the wounds of FIG. 133 in Example 17 of the present invention, and Image J was used for the statistics. On the 21st and 28th days, the wound area of the M cell group was significantly smaller than that of the model group, and there was a statistical difference, indicating that the M cell treatment could accelerate wound healing. One-way ANOVA in Prism 7.0 statistical analysis software was used for analysis of variance and significance test, and the experimental data were expressed as mean $\pm$ standard error (Mean $\pm$ SE). *, $p<0.05$; **, $p<0.01$; ***, $p<0.001$.

FIG. 135 shows the non-healing rate of wounds in Example 17 of the present invention. The proportion of unhealed wounds in the M cell treatment group was significantly lower than that in the model group, and there was a statistical difference, indicating that the M cells could accelerate wound healing and had a good therapeutic effect on skin damage.

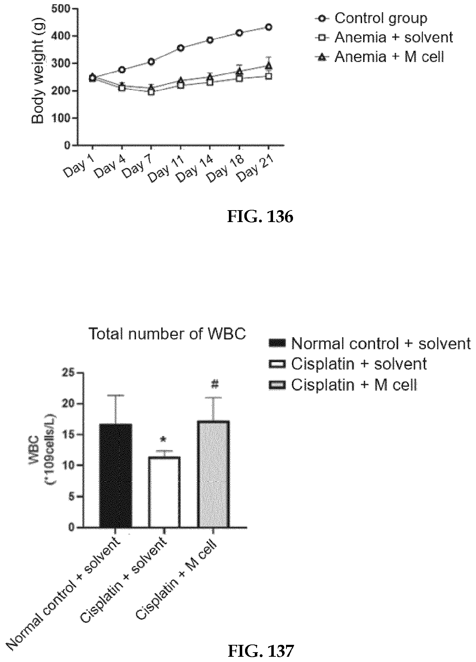

FIG. 136 shows the body weight change trend of the anemia model rats in Example 18 of the present invention. On the 3rd day after cisplatin injection, the body weight of rats stopped increasing and began to decrease, while the body weight decreased to a minimum after 6 days of cisplatin injection, and then slowly increased. At the end of the experiment, the body weight of rats in the control group was significantly higher than those in the solvent group and the M cell treatment group ($P<0.001$). From the day 7 to day 21, the body weight of rats in the M cell treatment group was significantly higher than that in the solvent group ($P<0.05$). The above results showed that after cisplatin-induced anemia in rats, the M cells could promote the body weight in the anemia model rats.

FIG. 137 shows the analysis of the total number of leukocytes in peripheral blood of the cisplatin-induced anemia model rats in Example 18 of the present invention. On the 21st day after cisplatin injection, the total number of leukocytes in blood of rats of the cisplatin+solvent group was significantly lower than that of the control group ($P<0.05$). The results showed that the model rats had symptoms of anemia, indicating that the model was successfully constructed. The total number of leukocytes of rats in the cisplatin+M cell treatment group was significantly higher than that in the cisplatin+solvent group ($P<0.05$), and there was no significant difference as compared with the control group+solvent. The above results showed that the M cells had obvious therapeutic effect in the anemia model rats.

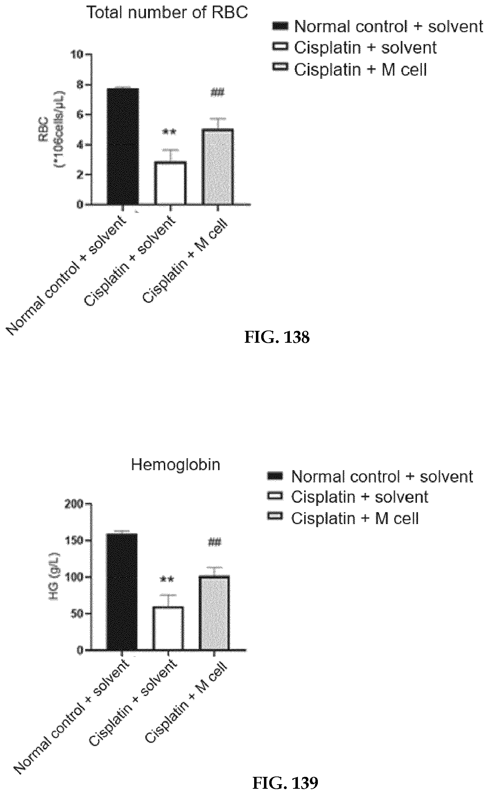

FIG. 138 shows the analysis of the total number of red blood cells in peripheral blood of the cisplatin-induced anemia model rats in Example 18 of the present invention. On the 21st day after cisplatin injection, the total number of red blood cells in blood of rats of the cisplatin+solvent group was significantly lower than that of the control group (P<0.05). The results showed that the model rats had symptoms of anemia, indicating that the model was successfully constructed. The total number of red blood cells of the rats in the cisplatin+M cell treatment group was significantly higher than that in the cisplatin+solvent group (P<0.05). The above results showed that the M cells had obvious therapeutic effect in the anemia model rats.

FIG. 139 shows the analysis of hemoglobin content in peripheral blood of the cisplatin-induced anemia model rats in Example 18 of the present invention. On the 21st day after cisplatin injection, the hemoglobin content in blood of rats of the cisplatin+solvent group was significantly lower than that of the control group (P<0.05). The results showed that the model rats had symptoms of anemia, indicating that the model was successfully constructed. The hemoglobin content in the cisplatin+M cell treatment group was significantly higher than that in the cisplatin+solvent group (P<0.05). The above results showed that the M cells had obvious therapeutic effect in the anemia model rats.

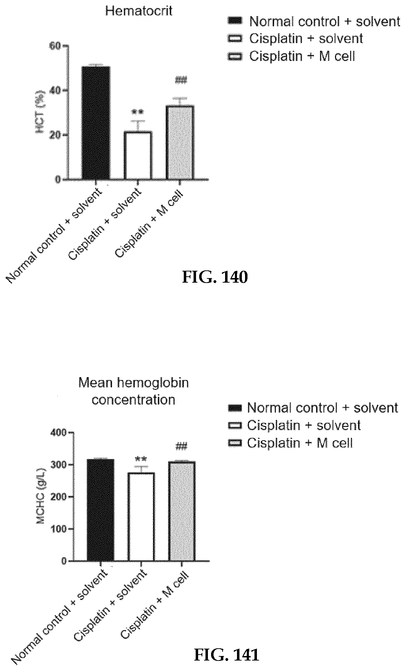

FIG. 140 shows the analysis of hematocrit of peripheral blood of the cisplatin-induced anemia model rats in Example 18 of the present invention. On the 21st day after cisplatin injection, the hematocrit of blood of rats in the cisplatin+solvent group was significantly lower than that in the control group (P<0.05). The results showed that the model rats had symptoms of anemia, indicating that the model was successfully constructed. The hematocrit of the cisplatin+M cell treatment group was significantly higher than that of the cisplatin+solvent group (P<0.05). The above results showed that the M cells had obvious therapeutic effect in the anemia model rats.

FIG. 141 shows the analysis of mean hemoglobin concentration of peripheral blood of the cisplatin-induced anemia model rats in Example 18 of the present invention. On the 21st day after cisplatin injection, the mean hemoglobin concentration of blood of rats in the cisplatin+solvent group was significantly lower than that in the control group (P<0.05). The results showed that the model rats had symptoms of anemia, indicating that the model was successfully constructed. The mean hemoglobin concentration in the cisplatin+M cell treatment group was significantly higher than that in the cisplatin+solvent group (P<0.05). The above results showed that the M cells had obvious therapeutic effect in the anemia model rats.

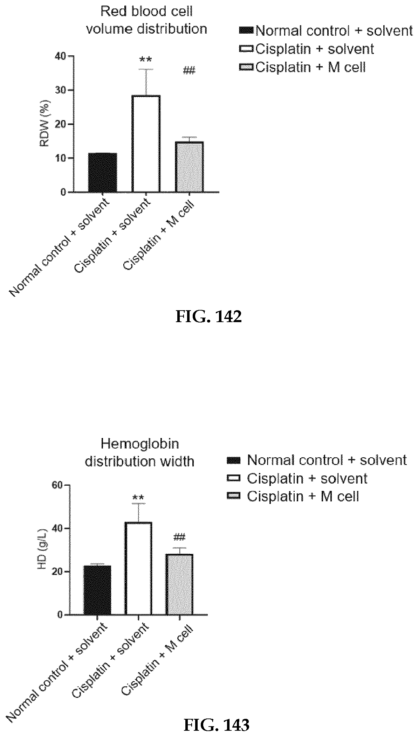

FIG. 142 shows the analysis of red blood cell volume distribution width of peripheral blood of the cisplatin-induced anemia model rats in Example 18 of the present invention. On the 21st day after cisplatin injection, the red blood cell distribution width of blood of rats in the cisplatin+solvent group was significantly lower than that in the control group (P<0.05). The results showed that the model rats had symptoms of anemia, indicating that the model was successfully constructed. The red blood cell volume distribution width in the cisplatin+M cell treatment group was significantly higher than that in the cisplatin+solvent group (P<0.05). The above results showed that the M cells had obvious therapeutic effect in the anemia model rats.

FIG. 143 shows the analysis of hemoglobin content in peripheral blood of rats of the cisplatin-induced anemia model rats in Example 18 of the present invention. On the 21st day after cisplatin injection, the hemoglobin content in blood of rats in the cisplatin+solvent group was significantly lower than that in the control group (P<0.05). The results showed that the model rats had symptoms of anemia, indicating that the model was successfully constructed. The hemoglobin content in the cisplatin+M cell treatment group was significantly higher than that in the cisplatin+solvent group (P<0.05). The above results showed that the M cells had obvious therapeutic effect in the anemia model rats.

FIG. 144 shows the pulmonary artery blood flow acceleration time of each group measured by the ultrasonography method in Example 19 of the present invention.

FIG. 145 shows the inner diameter of pulmonary artery in each group measured by the ultrasonography method in Example 19 of the present invention.

FIG. 146 shows the inner diameter ratio of right ventricle to left ventricle in each group measured by the ultrasonography method in Example 19 of the present invention.

FIG. 147 shows the average pulmonary arterial pressure in each group measured by the ultrasonography method in Example 19 of the present invention.

FIG. 148 shows the HE staining results of paraffin sections in each group of Example 19 of the present invention.

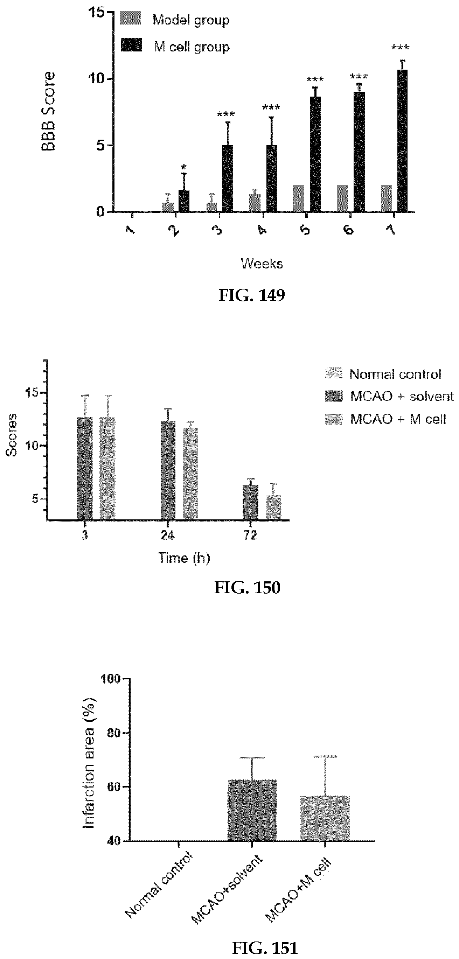

FIG. 149 shows the rat BBB score of Example 20 of the present invention. After intravenous injection of M cells, the BBB score of rats was significantly increased, and the BBB score of the M cell treatment group was always higher than that of the model group, indicating that M cells could improve the exercise capacity of rats after spinal cord injury, and had therapeutic effect on spinal cord injury. The BBB score of the M cell treatment group has significant difference to from that of the model group. One-way ANOVA in Prism 7.0 statistical analysis software was used for analysis of variance and significance test, and the experimental data were expressed as mean $\pm$ standard error (Mean $\pm$ SE). *, $p<0.05$; **, $p<0.01$; ***, $p<0.001$.

FIG. 150 shows the mNSS behavioral scores after 3, 24 and 72 hours in Example 21 of the present invention. The intravenous injection of M cells reduced mNSS scores within 3 hours after surgery, within 24 hours (12.67 vs 10.33) and 72 hours (6.33 vs 5.33) after surgery, indicating that the M cells could improve the behavior of stroke rats.

FIG. 151 shows the statistics of the rat cerebral infarction area after 72 hours in Example 21 of the present invention. The intravenous injection of M cells reduced the infarct size of brain tissue by 6.05% within 3 hours after operation as compared with the solvent group, indicating that the M cells reduced the degree of brain tissue necrosis.

FIG. 152 shows the statistics of water content of brain tissue of rats after 72 hours in Example 21 of the present invention. The intravenous injection of M cells reduced the water content of brain tissue by 2% within 3 hours after surgery as compared with the solvent group (82.94 vs 80.49), indicating that the M cells reduced the degree of brain tissue edema.

FIG. 153 shows the optical photos of rat eyes of Example 22 of the present invention. Compared with the NaOH group and the collagen group, the corneal opacity of the rats in the M cell group was lower, the pupils were visible, and the new blood vessels were less, which confirmed that the M cells had a good therapeutic effect on the corneal alkaloid burn in the rats.

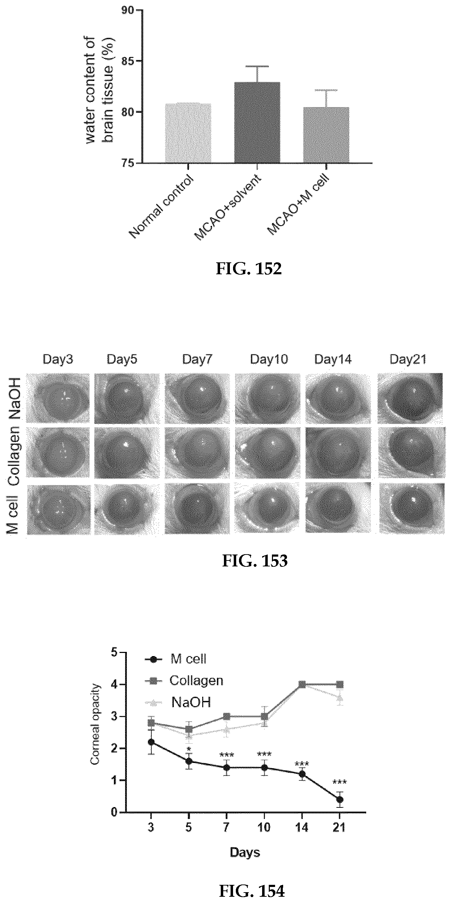

FIG. 154 shows the rat corneal opacity scores of Example 22 of the present invention. According to the statistics of corneal opacity scores in FIG. 153, the corneal opacity of the rats in the M cell treatment group was lower, and on the 21st day, the cornea was transparent, the pupil was visible, and the score was lower, and there was a statistical difference, indicating that the M cell treatment showed obvious effects. One-way ANOVA in Prism 7.0 statistical analysis software was used for analysis of variance and significance test, and the experimental data were expressed as mean $\pm$ standard error (Mean $\pm$ SE). *, $p<0.05$; **, $p<0.01$; ***, $p<0.001$.

FIG. 155 shows the photos of rat eyeballs in Example 22 of the present invention. It was found from rat samples that the eyeballs of the M cell treatment group were closer to that of the normal group, and there was no accumulation of fluid and extravasated blood. This showed that the M cells could reduce the inflammation of corneal alkali burn animal model and promote the recovery of corneal alkali injury.

FIG. 156 shows the back pictures of mice on different days in Example 23 of the present invention. In the M cell treatment group, the mice had less scale and less rash, indicating that the M cells could well alleviate the symptoms of psoriasis mice and had effective therapeutic effect on psoriasis.

FIG. 157 shows the HE staining pictures of Example 23 of the present invention. Compared with the imiquimod group and the IMQ group, the stratum corneum of the mice after the M cell treatment was thinner, indicating that the M cell treatment could improve mouse skin microenvironment and inhibit inflammation, and there were more skin appendages in comparison with the IMQ group, indicating that the M cells could protect the skin appendages and had good therapeutic effect on psoriasis.

FIG. 158 shows the statistics of the total number of times the mice traversed across the platform in Example 24 of the present invention. The total traversing time of the normal control group was 1, while compared with the normal control group, the total traversing time of the mice in the solvent group was 0, with a significant difference, * $p<0.05$; the total traversing time of the mice in the M cell group was 0.66, which was significantly more than that of the solvent group, indicating that the M cells could effectively improve the spatial learning and memory ability in mice.

FIG. 159 shows the statistics of the time the mice arrived at the platform for the first time in Example 24 of the present invention. Compared with the solvent group, the time spent by the rats of the M cell group was significantly reduced (38.0 vs 59.8 seconds), indicating that the M cells could effectively improve the spatial learning and memory ability of the mice.

FIG. 160 shows the light microscopy pictures of rat joints. The joint surface of the model group was rough and ulcerated, and osteophytes were formed around the joint, while the joint of the M cell treatment group recovered to a certain extent. This showed that the M cell treatment could relieve the symptoms of arthritis and could treat arthritis very well.

FIG. 161 shows the left hindlimb CT images of the fracture model rats on the 10th day after administration. The results showed that the healing of bone injury in the M cell treatment group had a better trend than that in the model group.

FIG. 162 shows the left hindlimb CT images of the fracture model rats on the 22nd day after administration. The results showed that the healing of bone injury in the M cell treatment group had obvious advantages compared with the model group, and the bone injury area was smaller, indicating that the M cell transplantation could accelerate the healing of bone injury, and the M cells had good therapeutic effect on bone injury.

FIG. 163 shows the left hindlimb CT images of the fracture model rats on the 50th day after administration. The results showed that the bone injury site in the M cell transplantation treatment group had healed completely, while the model group had not yet healed completely. This showed that the M cells could treat bone injury very well.

FIG. 164 shows the statistics of sneezing in rhinitis model mice. After the M cell treatment, the number of sneezing in the mice was significantly reduced, indicating that the M cells had a good therapeutic effect on alleviating the symptoms of rhinitis.

FIG. 165 shows the statistics of nose-scratching in rhinitis model mice. After the M cell treatment, the number of nose-scratching in the mice was significantly reduced, indicating that the M cells had a good therapeutic effect on alleviating the rhinitis symptoms.

FIG. 166 shows the embryoid body formed with iPS as starting material and the cell morphology of M cells at P0 and P5 generations.

FIG. 167 shows the flow cytometry results of iPS-MSC cells.

FIG. 168 shows the qPCR detection of MMP1 expression in iPS-MSC cells.

FIG. 169 shows the qPCR detection of PGE2 expression in iPS-MSC cells.

FIG. 170 shows the statistical graph of the weight change rate of mice in each group of the GVHD animal model. There was a significant difference in the survival rate between the control group and the GVHD group (*** $p < 0.001$); there was a significant difference in the survival rate between the GVHD group and the GVHD+high-dose M cell treatment group (** $p < 0.01$).

FIG. 171 shows the statistical graph of the survival rate of mice in each group of the GVHD animal model. The bone marrow chimeric rate in the GVHD+high-dose M cell group was significantly lower than that in the GVHD group (* $p<0.05$), indicating that the M cells alleviated GVHD by reducing the infiltration of hPBMC in the bone marrow.

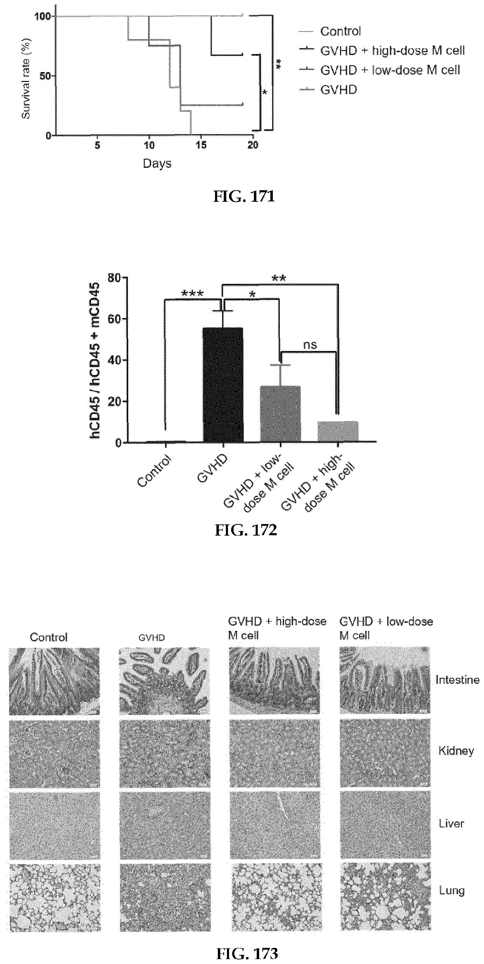

FIG. 172 shows the statistical graph of the bone marrow chimeric rate of mice in each group of the GVHD animal model. On the 14th day, the intestine, kidney, liver and lung were taken for paraffin section and HE staining. The results showed that the intestinal crypt structure of the GVHD+low/high dose M cell groups was significantly better than that of the GVHD group, and had more complete intestinal crypt structure. The infiltration of inflammatory cells in various organs in the GVHD+low/high dose M cell groups was significantly lower than that in the GVHD group, indicating that the M cells had the function of inhibiting inflammation and maintaining the integrity of tissue structure.

FIG. 173 shows the HE staining diagram of mouse organs in each group of the GVHD animal model. The results showed that the intestinal crypt structure of the GVHD+low/high dose M cell groups was significantly better than that of the GVHD group, and had more complete intestinal crypt structure. The infiltration of inflammatory cells in various organs in the GVHD+ low/high dose M cell groups was significantly lower than that in the GVHD group, indicating that the M cells had the function of inhibiting inflammation and maintaining the integrity of tissue structure. The results showed that the M cells could reduce inflammation and tissue damage in the GVHD mice.

FIG. 174 showed that after the M cell injection in mice with premature ovarian failure, their sex hormone levels, body weight and ovarian weight were significantly restored.

FIG. 175 showed that after the M cell injection in mice with premature ovarian failure, ovulation was significantly restored.

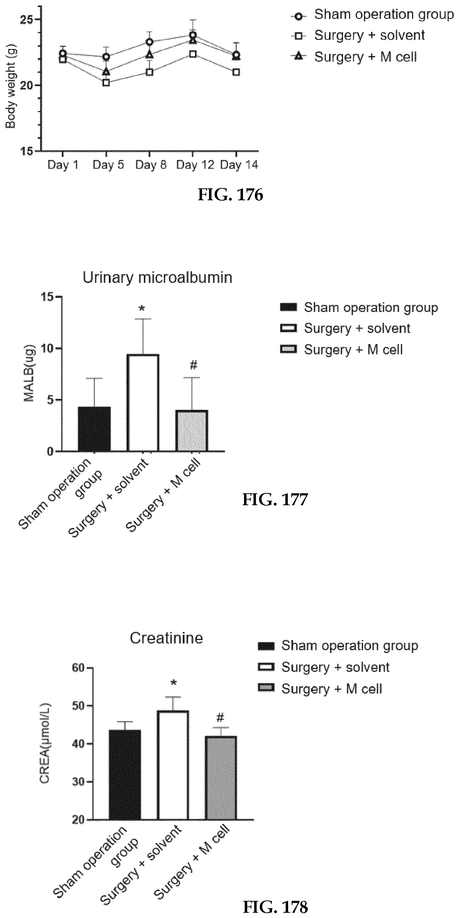

FIG. 176 shows the statistics of body weight of mice in each group of the renal fibrosis model. The body weight of the sham operation group at each time point was significantly higher than that of the surgery+solvent group (P < 0.05); the body weights of the surgery+M cell treatment group on the 12$^{th}$ and 14$^{th}$ days were significantly higher than those of the surgery+solvent group (P < 0.05), respectively. On the 12$^{th}$ and 14$^{th}$ day, there was no significant difference in body weight between the sham operation group and the surgery+M cell treatment group. The above results showed that the M cell treatment group had a significant promoting effect on the body weight of the renal fibrosis mice.

FIG. 177 shows the statistics of urinary microalbumin of mice in each group of the renal fibrosis model. The statistics of urinary microalbumin values of different groups of mice on the 14$^{th}$ day were performed. The urinary microalbumin value of the surgery+solvent group was significantly higher than that of the sham operation group (*, P<0.05), indicating that the model was successfully constructed. The urinary microalbumin content of the M cell treatment group was not significantly different from that of the sham operation group, but was significantly lower than that of the solvent group (#, P<0.05), indicating that the M cells had a certain therapeutic effect on the renal fibrosis (P<0.05).

FIG. 178 shows the statistics of urinary creatinine content of mice in each group of the renal fibrosis model. The statistics of urine creatinine content values of different groups of mice on the 14$^{th}$ day was performed. The urine creatinine value of the surgery+solvent group was significantly higher than that of the sham operation group (*, P<0.05), indicating that the model was successfully constructed. The urinary creatinine content of the M cell treatment group was not significantly different from that of the sham operation group, but was significantly lower than that of the solvent group (#, P<0.05), indicating that the M cells had a certain therapeutic effect on the renal fibrosis.

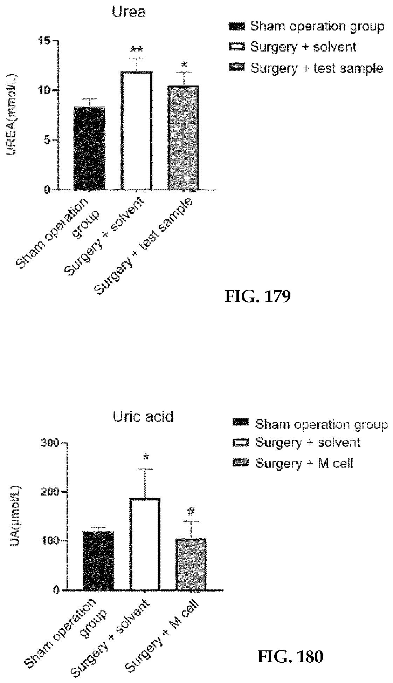

FIG. 179 shows the statistical graph of urea content in each group of mice in the renal fibrosis model. The statistics of urea content values of different groups of mice on the 14$^{th}$ day was performed. The urea value of the surgery+solvent group was significantly higher than that of the sham operation group (**, P<0.01), indicating that the model was successfully constructed. The urea content of the M cell treatment group was significantly higher than that of the sham operation group (*, P<0.05), and showed a decreasing trend in comparison with the solvent group, but there was no significant difference, indicating that the M cells had a certain treatment trend for the renal fibrosis.

FIG. 180 shows the statistics of uric acid content in each group of mice in the renal fibrosis model. The statistics of uric acid content values of different groups of mice on the 14$^{th}$ day was performed. The uric acid value of the surgery+solvent group was significantly higher than that of the sham operation group (*, P<0.05), indicating that the model was successfully constructed. The uric acid content of the M cell treatment group was significantly lower than that of the solvent group (#, P<0.05), indicating that the M cells had a certain degree of therapeutic effect on the renal fibrosis.

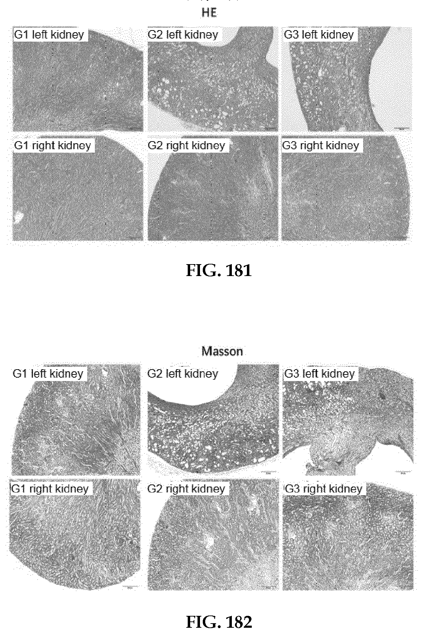

FIG. 181 shows the HE staining of kidneys of mice in each group of the renal fibrosis model. The mice of different groups were sampled on the 14$^{th}$ day, and the obtained kidneys were embedded and sectioned, followed by HE staining. Among them, G1 was the sham operation group, G2 was the surgery+solvent group, and G3 was the operation+M cell group. The left kidney was the operated kidney, and the right kidney was not operated and used as the control. It could be seen from the figure that the basic kidney structure of the mice in the G2 group disappeared, and a large number of fibroblasts proliferated; the kidney structure of the mice in the G3 group was improved in a certain extent, the kidney tubular atrophy was alleviated, the infiltration of inflammatory factors and the proliferation of fibroblasts were reduced, and the necrosis region was reduced in a certain extent.

FIG. 182 shows the Masson staining of kidneys of mice in each group of the renal fibrosis model. The mice of different groups were sampled on the 14$^{th}$ day, and the obtained kidneys were embedded and sectioned, followed by Masson staining. Among them, G1 was the sham operation group, G2 was the surgery+solvent group, and G3 was the operation+M cell group. The left kidney was the operated kidney, and the right kidney was not operated and used as the control. It could be seen from the figure that there was no obvious collagen deposition in the kidney tissue of the G1 group; there were sheet-like positive areas stained in blue in the G2 group, which were mostly distributed around the renal tubules, indicating that a large number of collagen fibers were deposited in the renal interstitium; the blue areas of the kidney tissue of the rats in the G3 group were significantly reduced, and the color was lightened. The above results demonstrated that the M cells showed an inhibitory effect in the mouse model of renal fibrosis, improved renal structure, reduced collagen deposition, and delayed the progression of renal fibrosis.

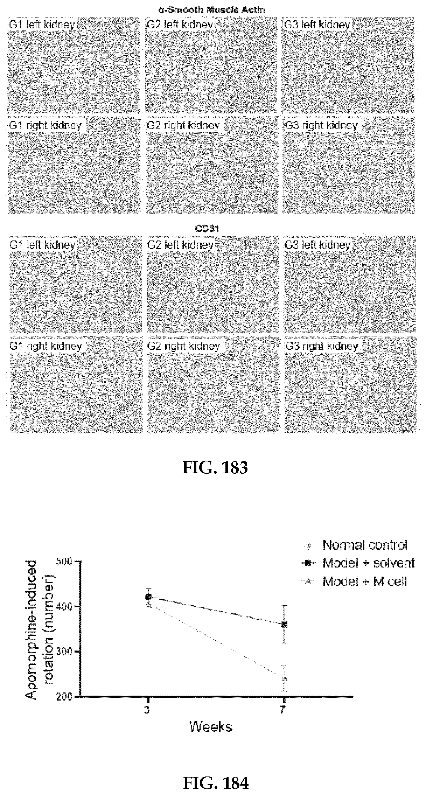

FIG. 183 shows the immunohistochemical staining of kidneys of mice in each group of the renal fibrosis model. The mice of different groups were sampled on the 14th day, and the obtained kidneys were subjected to immunohisto-chemical staining for $\alpha$-SMA and CD31. Among them, G1 was the sham operation group, G2 was the surgery+solvent group, and G3 was the operation+M cell group. The left kidney was the operated kidney, and the right kidney was not operated and used as the control. EndotheliaL-mesenchymal transition (EndoMT) is an important mechanism for the generation of myofibroblasts in the injured kidneys. EndoMT refers to a process in which endothelial cells lose their anchoring connections and polar functions, and then transform into highly invasive and migratory slender spindle-shaped mesenchymal cells; the endothelial cells change in morphology and polarity, as well as in biochemical properties, and lose their characteristic marker CD31, etc., but regain the mesenchymal cell marker $\alpha$-smooth muscle actin (a-SMA), thereby converting into viable mesenchymal cells. It could be seen from the figure that compared with the left kidney of the G1 group, the expression of $\alpha$-SMA in the kidney tissue of the G2 group increased on the 14th day, and the expression of CD31 did not change significantly, indicating the successful construction in which the mice were developed into a renal fibrosis model. On the 14th day, the expression of $\alpha$-SMA in the left kidney of the G3 group was lower than that of the G2 group, and the expression of CD31 was higher.

FIG. 184 shows the statistics of rotation number in apomorphine-induced Parkinson's model rats. The statistics of rotation number of apomorphine-induced rats was performed at the 3rd week and the 7th week, respectively. At the 7th week, the rotation number of the M cell group was significantly lower than that of the solvent group (240.5 vs 360.5), indicating that the M cells could alleviate dopaminergic denervation and improve the severity of Parkinson's disease.

FIG. 185 shows the body weight of mice in each group of the forced swimming-induced mouse depression model.

FIG. 186 shows the immobility time during forced swimming of mice in each group of the forced swimming-induced mouse depression model.

FIG. 187 shows the back pictures of the dermatitis model mice on different days. In the M cell treatment group, the rash of mice was less severe and the skin lesions were relieved. It showed that the M cells could well relieve the symptoms of dermatitis mice and had effective therapeutic effect on dermatitis.

FIG. 188 shows the HE staining pictures of dermatitis model mice. Compared with the OVA group, the stratum corneum of the mice after the M cell treatment was thinner and the fat layer was thicker, indicating that the M cell treatment could improve mouse skin microenvironment, inhibit inflammation, and there were more skin appendages than the OVA group, indicating that the M cells could protect the skin appendages and had a good therapeutic effect on the atopic dermatitis.

FIG. 189 shows the statistical results of IL-1$\beta$ content (pg/ml) in brain tissue of mice with LPS-induced neuroinflam-mation. IL-1$\beta$ promotes inflammation and is a proinflammatory factor. The content of IL-1$\beta$ in brain tissue of the solvent group was significantly higher than that of the normal group. Compared with the solvent group, the content of IL-1$\beta$ in the M cell group had a tendency to decrease.

FIG. 190 shows the statistical results of IL-6 (pg/mg) in brain tissue of mice with LPS-induced neuroinflammation. Compared with the normal group, the content of IL-6 in brain tissue of the solvent group was significantly increased. IL-6 promotes inflammation and is a proinflammatory factor. Compared with the solvent group, the content of IL-6 in the M cell group had a tendency to decrease.

FIG. 191 shows the statistics of IL-10 (pg/mg) in brain tissue of mice with LPS-induced neuroinflammation. IL-10 inhibits the occurrence of inflammation and is an anti-inflammatory factor. The concentration of IL-10 in the brain tissue of the M cell group was significantly higher than that of the solvent group (2.0 vs 2.7), indicating that the M cells had an anti-inflammatory effect.

FIG. 192 shows the trend chart of mean VAS scores in subjects with meniscus injury. Although the VAS scores of the two dose groups fluctuated up and down, they showed a downward trend as a whole, that was, the subjects' knee pain was relieved to a certain extent after receiving the injection of the study drug.

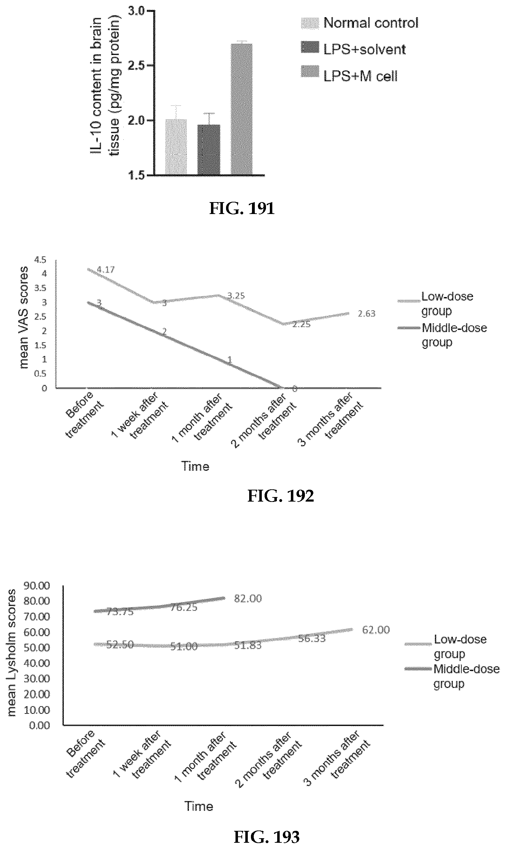

FIG. 193 shows the trend chart of mean Lysholm scores in subjects with meniscus injury. After the subjects in the two dose groups received the injection of study drug, the total Lysholm score showed an upward trend. According to the single evaluation results, the increase in the score value after the injection of study drug was mainly reflected in claudication, locking and pain. In other single evaluations, there was no significant change in the score. Therefore, after the subjects received the injection of study drug, there was some improvement in the Lysholm score, mainly in the alleviation of claudication, locking and pain.

FIG. 194 shows the MRI image of a subject with meniscus injury. Before the M cell treatment, there was meniscus injury and severe knee joint pain. After 3 months of intra-articular transplantation of the M cell preparation, the meniscus injury was completely recovered, the knee joint pain score changed from 8 points to 2 points, and the local edema was alleviated, indicating that the intra-articular transplantation of the M cells could effectively treat the meniscus injury.

FIG. 195 shows the statistics of ALT content in blood. The results of blood ALT content showed that the ALT of the solvent group was significantly increased compared with that of the normal control group (58 vs 987), while the M cell group had a significantly decreased ALT content as compared with the solvent group.

FIG. 196 shows the statistics of AST content in blood. The AST of the solvent group was significantly increased as compared with that of the normal control group (81 vs 812), while the M cell group had a significantly decreased AST content as compared with the solvent group (473 vs 812).

FIG. 197 shows the statistics of TG content in liver. The statistics of TG content in liver showed that the concentration of TG in the liver of the mice in the solvent group was significantly higher than that in the normal group (82.6 vs 4.8), *** $p < 0.001$; the M cell group had a TG concentration decreased to 49 umol/g as compared with the solvent group, and there was a significant difference, **$p < 0.01$.

FIG. 198 shows the blood oxygen saturation in ARDS patients.

FIG. 199 shows the chest CT of ARDS patients before (left) and after (right) the infusion of M cells. Before the first cell infusion, the patient's chest CT showed multiple patches, sheet-like ground-glass opacity and high-density shadows (yellow arrows) in both lungs. On the second day after the patient received the second infusion of the M cells (interval was 6 days), CT showed that the absorption of lesions was improved; and 1 month after the first infusion, CT showed that it returned to normal and there was not fibrosis.

FIG. 200 shows the change in IL-1RA level in ARDS patients. On the 8th day after the first infusion, the level of anti-inflammatory cytokine IL-1RA was elevated.

FIG. 201 shows the change in RANTES level in ARDS patients. On the 8th day after the first infusion, the level of anti-inflammatory cytokine RANTES was elevated.

FIG. 202 shows the changes in IL-1$\alpha$ level in ARDS patients. On the 8th day after the first infusion, the IL-1$\alpha$ proinflammatory cells were significantly reduced.

FIG. 203 shows the change in IL-1$\beta$ level in ARDS patients. The IL-1$\beta$ proinflammatory cells were significantly reduced.

FIG. 204 shows the change in IL-5 level in ARDS patients. The IL-5 proinflammatory cells were significantly reduced.

FIG. 205 shows the changes in IL-8 level in ARDS patients. The IL-8 proinflammatory cells were significantly reduced.

FIG. 206 shows the changes in IL-25 level in ARDS patients. The IL-25 proinflammatory cells were significantly reduced.

FIG. 207 shows the changes in CXCL10/IP-10 level in ARDS patients. The CXCL10/IP-10 proinflammatory cells were significantly reduced.

FIG. 208 shows the chest CT image of IPF patients after the infusion of the M cells. Before the infusion of M cells, the patient's chest CT showed multiple patches, sheet-like ground-glass opacity, and high-density shadows (indicated by arrows). After the patients received the infusion of M cells, CT showed that the absorption of lesions was improved in all the patients; and 1 month after the infusion, CT showed that it basically returned to normal. After 50 days, the pulmonary fibrosis was significantly absorbed and improved in all patients.

FIG. 209 shows the statistic results of cardiac LVEDP (mmHg) in the myocardial ischemia-reperfusion model.

FIG. 210 shows the statistical results of blood LDH (U/L) content in the myocardial ischemia-reperfusion model.

FIG. 211 shows the statistical results of blood CK (U/L) content in the myocardial ischemia-reperfusion model.

FIG. 212 shows the statistical results of body weight of rats in different groups in the nephrectomy model.

FIG. 213 shows the graph of the detection results of serum anti-double-stranded DNA antibody level in each group of Example 46.

FIG. 214 shows the HE staining results of lung tissue of pneumoconiosis model mice in Example 43.

FIG. 215 shows the cell viability assay results of the M cells after cryopreservation with MZJ injection solution 2.

FIG. 216 shows the cell viability assay results of the M cells after cryopreservation with MZJ injection solution 3.

## Sequence Information

The information of partial sequences involved in the present invention is provided in Table 1 below.

Table 1: Description of sequences

| SEQ ID NO | Description | Sequence |
| --- | --- | --- |
| 1 | IDO-F | GCCAGCTTCGAGAAAGAGTTG |
| 2 | IDO-R | ATCCCAGAACTAGACGTGCAA |
| 3 | MMP1-F | AAAATTACACGCCAGATTTGCC |
| 4 | MMP1-R | GGTGTGACATTACTCCAGAGTTG |
| 5 | PDL1-R | GGACAAGCAGTGACCATCAAG |
| 6 | PDL1-F | CCCAGAATTACCAAGTGAGTCCT |
| 7 | PGE2-R | GGCGGGCGTTTCGAACTT |
| 8 | PGE2-F | CGGGTCCATGTTCGCTCC |
| 9 | GAPDH-F | CTCTGCTCCTCCTGTTCGAC |
| 10 | GAPDH-R | CGACCAAATCCGTTGACTCC |

Examples The present invention is further described with reference to the following examples, which are intended to illustrate, but not limit, the present invention.

Unless otherwise indicated, the experiments and methods described in the examples were performed essentially according to conventional methods well known in the art and described in various references. If the specific conditions are not indicated in the examples, it is carried out according to the conventional conditions or the conditions suggested by the manufacturer. The reagents or instruments used without the manufacturer's indication are conventional products that can be obtained from the market. Those skilled in the art appreciate that the examples describe the present invention by way of example and are not intended to limit the scope of the present invention as claimed. All publications and other references mentioned herein are incorporated by reference in their entirety.

Preparation Example 1: Preparation of human embryonic stem cell-derived M cells

1.1 Generation of embryoid body (EB)

a. the original culture medium was removed, followed by washing with PBS;

b. Dispase was used to digest human embryonic stem cells (Q-CTS-hESC-2, National stem cell Resource Bank);

c. the enzyme solution was discarded, 1ml of KO-DMEM/F12 was added, and lines were drawn arranged in parallel crosses by holding the pipette perpendicular to the plate;

d. the pipette tip was moistened, then the liquid in 6-well plate was pipetted and transferred to 15ml centrifuge tube for centrifugation;

e. the supernatant after centrifugation was removed, the cells were resuspended with a small amount of EB culture medium in culture dish, added to a low-attachment culture dish (Corning: Cat. No. 3262), and cultured in a 37°C incubator.

Preparation of EB culture medium (first culture medium): 10% (v/v) KOSR, 1% (v/v) NEAA (i.e., 0.1 mM), 1% (v/v) GlutaMAX (i.e., 2 mM), 8 ng/ml bFGF, and 0.1% (v/v) $\beta$-mercaptoethanol were added to KO-DMEM.

1.2 Adherent culture of EB spheres

a. vitronectin was coated in a 6-well plate;

b. MSC culture medium was preheated to 37°C;

c. all the EB spheres obtained in 1.1 were transferred to a 50ml centrifuge tube and allowed to stand for 5 to 10 minutes;

d. the coated matrix was sucked out, and 2ml of MSC culture medium was added;

e. the supernatant was removed, 1 mL of MSC culture medium was taken to resuspend the EB spheres, and the EB spheres were added to the culture plate wells;

f. after well shaking, the culture was performed in an incubator.

The culture medium was changed every day until about 14 days, and the MSCs were passaged.
Preparation of M cell culture medium (second culture medium): 1% (v/v) Ultraser G, 5% (v/v) KOSR, 1% (v/v) NEAA, 1% (v/v) GlutaMAX, 5 $\mu$g/ml ascorbic acid, 5 ng/ml bFGF, and 5 ng/ml TGF$\beta$ were added into $\alpha$-MEM.

1.3 Passaging of MSCs

a. the above MSC culture medium was preheated to 37°C;

b. the original culture medium was removed, the PBS that had been placed at room temperature was added to wash once;

c. after Trypsin was added, it was placed into an incubator to perform incubation and digestion for 2 to 3 minutes;

d. when the cells fall off the wall of the dish, the PBS of same amount as the digestion solution was added to terminate the digestion;

e. the cells were gently pipetted until the cells were dispersed;

f. the cell suspension was collected and centrifuged in a centrifuge tube, and the supernatant was discarded.

g. the MSC culture medium was added to a new culture dish, and the cells were inoculated into the new culture dish; the 5th generation was used for subsequent operations (e.g., cell therapy), or for cryopreservation with Cryostor CS10.

The M cells prepared above could be referred to as hESC-M cells.

The reagents involved in the above steps were as follows:

| Name | Manufacturer | Cat. No. |
|------|-------------|----------|
| Vitronectin | Sigma | V8379 |
| α-MEM | HyClone | SH30265.01B |
| β-Mercaptoethanol | Invitrogen | 21985-023 |
| KnockOut SR | Thermo | 10828028 |
| NEAA | Gibco | 11140050 |
| GlutaMAX | Gibco | A1286001 |
| Ultroser G | PALL | 15950-017 |
| Ascorbic acid | Selleck | S3114 |
| TGFβ | Peprotech | 96-100-21-10 |
| bFGF | Thermo | 13256029 |
| Trypsin | Gibco | 25200072 |
| PBS | Gibco | C10010500BT |
| Cryostor CS10 | stem cell | 07930 |
| Dispase | Gibco | 17105041 |

Comparative Example 1: Preparation of human embryonic stem cell-derived mesenchymal stem cells

Mesenchymal stem cells were prepared in this example according to Hwang NS et al. Proc Natl Acad Sci US A. 2008 Dec 30;105(52):20641 to 6.

1. Culture of EB

1.1 Preparation of EB culture medium
KnockOut DMEM/F12 + 15% FBS + 5% KOSR + 2 mM NEAA + 2 mM Glutamine + 0.1 mM β-mercaptoethanol

1.2 Formation of EB

1.2.1 The original culture medium was removed and 1 mL of PBS was added to wash.

1.2.2 1 mL of Dispase was added to digest for 3 to 10 min.

1.2.3 The enzyme solution was discarded, 1 mL of KODMEM was added, and lines were drawn arranged in parallel crosses by holding the pipette perpendicular to the plate.

1.2.4 The pipette tip was moistened, the liquid in 6-well plate was pipetted and transferred to a centrifuge tube for centrifugation.

1.2.5 The supernatant was discarded, the cells were resuspended with EB medium, added to a low-attachment 10 cm culture dish (Corning: Cat. No. 3262), and cultured in a 37°C incubator.

1.2.6 The culture medium was changed every two days until the 10th day.

2. Culture of EB-M cells

2.1 Culture of EB-M cells

2.1.1 A 6-well plate was pre-coated with 1 mg/ml gelatin.

2.1.2 All the EB spheres were transferred to a 50 mL centrifuge tube and allowed to stand for 5 to 10 minutes.

2.1.3 After the EB spheres were settled, the supernatant was pumped away, and 1 mL of EB culture medium was taken for resuspending, and inoculation was performed in a 6-well plate covered with gelatin.

2.1.4 It was placed in an incubator.

2.1.5 The culture medium was changed every two days until the 10th day.

3. Culture of M cells

3.1 Preparation of M cell culture medium
DMEM + 10% FBS + 2mM glutamine

3.2 Passage of M cells

3.2.1 The cells were washed with 1 mL of DPBS, 1 mL of Trypsin was added, digestion was carried out at 37°C for about 3 minutes, the wall of dish was tapped lightly to detach the cells, and 1 mL of DPBS was added to stop the digestion.

3.2.2 The cells were collected, and centrifuged at 1200 rpm for 3 min.

3.2.3 The supernatant was discarded, the M cells were resuspended with 5 mL of culture medium, and the cells were filtrated with a cell sieve, and counted.

3.2.4 Inoculation was carried out in a 10 cm culture dish at a density of $2 \times 10^5$ cells/cm$^2$, denoted as M P1.

3.2.5 the MSC was passaged to the 5th generation by the same method, and the subsequent operations were carried out.

The reagents involved in the above steps were as follows:

KnockOut DMEM/F12 (Gibco, 10829018)

FBS (Gibco, 16000044)

KOSR (Thermo, 10828028)

NEAA (Gibco, 11140050)

Glutamine (Gibco, A1286001)

β-Mercaptoethanol (Invitrogen, 21985 to 023)

PBS (Gibco, C10010500BT)

Dispase (Gibco, 17105041)

Trypsin (Gibco, 25200072)

Comparative Example 2: Preparation of primary mesenchymal stem cells

1. The umbilical cord was immersed in PBS and transported on ice to the laboratory.

2. The umbilical cord was cut into small pieces of about 3 cm, and washed with PBS until the surface is free of blood.

3. Three blood vessels in the umbilical cord were removed.

4. After the removal of blood vessels, the umbilical cord was cut into small tissue pieces with ophthalmic scissors.

5. The tissue pieces were attached on a 10cm dish, and each of the tissue pieces was evenly separated to each part of the dish.

6. The dish was placed upside down in a $CO_2$ incubator overnight.

7. On the second day, each dish was placed upright and added with 5 mL of culture medium, and placed in a $CO_2$ incubator for cultivation.

8. The fluid was changed every other day, and the cells would be seen crawling out within about 10 days.

9. The passaging was carried out when about 70% of the cells crawled out.

Example 1-1: Detection of surface markers of M cells

The expression of surface protein of the M cells obtained in Preparation Example 1 was detected by flow cytometry:

1. The culture supernatant was discarded, washing was performed once by adding PBS, and digestion was carried out for 3 to 5 minutes by adding Trypsin, and terminated by adding PBS.

2. The cell suspension was collected and centrifuged at 1200 rpm for 3 min.

3. The supernatant was discarded, and the cells were resuspended in PBS, filtered through a cell sieve to remove cell clusters, counted, and subpackaged, $2\times10^6$ cells per tube.

4. Centrifugation was carried out at 1200 rpm for 3 min.

5. After blocking with 2% BSA blocking solution for 20 min, centrifugation was carried out at 1200 rpm for 3 min.

6. The supernatant was discarded, the cells were resuspended with 100 μL of 1% BSA antibody diluent, the directly labeled antibody was added, and incubated at room temperature for 30 to 45 minutes.

7. Washing was performed three times with 1 mL of PBS, centrifugation was carried out at 1200 rpm for 3 min, and the supernatant was discarded.

8. After resuspending in 300 μL of DPBS, the cells were filtered with a 40 μm cell sieve, then loaded and detected.

The antibody information involved in the above steps was as follows:

| Name | Company | Cat. No. |
|------|---------|----------|
| CD10 | BD | 561002 |
| CD24 | BD | 555428 |
| IL-11 | abcam | ab 187167 |
| AIRE-1 | abcam | ab65040 |
| ANG-1 | abcam | ab102015 |

(continued)

| Name | Company | Cat. No. |
|---|---|---|
| CXCL1 | RD | IC275P |
| CD105 | BioLegend | 323206 |
| CD73 | eBioscience | 11-0739-42 |
| CD90 | eBioscience | 12-0909-42 |
| CD13 | BD | 560998 |
| CD29 | BioLegend | 303004 |
| CD44 | BD | 561858 |
| CD166 | BD | 560903 |
| CD274 | BD | 561787 |
| HLA-ABC | BD | 560965 |
| CD31 | BD | 560983 |
| CD34 | BD | 555822 |
| CD45 | eBioscience | 11-9459-42 |
| CD133 | BD | 566593 |
| FGFR2 | RD | FAB684G |
| CD271 | BD | 560927 |
| Stro-1 | abcam | ab190282 |
| CXCR4(CD184) | BD | 561733 |
| TLR3(CD283) | BD | 565984 |

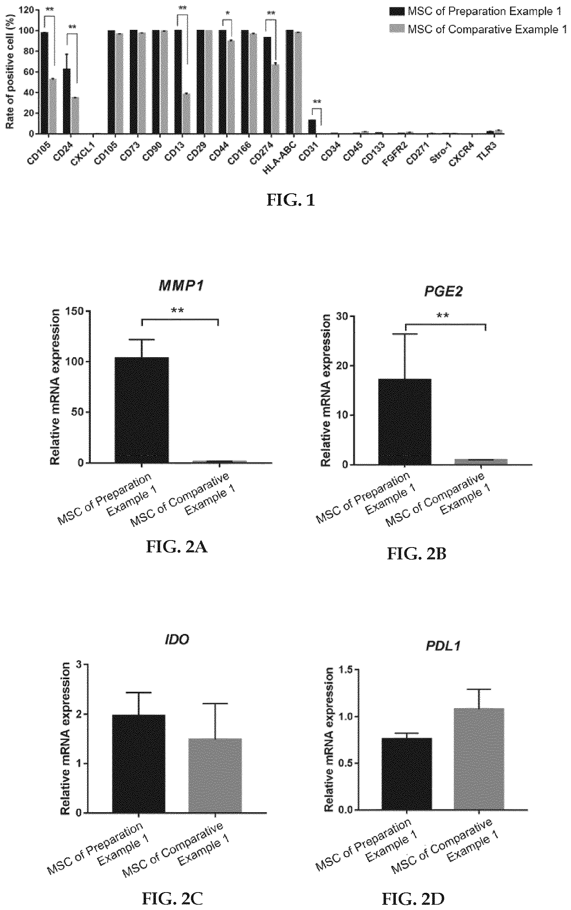

The detection results were shown in FIG. 1. Compared with the primary MSC and the MSC obtained by the prior art method, the M cells obtained in the Preparation Example 1 had significant differences in terms of surface markers: CD105, CD24, CD13, CD44, CD274 and CD31.

Example 1-2: Detection of cytokine expression levels in M cells

The cytokine expression levels of the M cells of Preparation Example 1 were determined by Real-time PCR.

1. Extraction of cellular RNA

RNA extraction kit was used for extraction, and the specific steps were as follows:

(1) 10 $\mu$L of $\beta$-mercaptoethanol was added into RL lysis solution per ml, and the cells were lysed on ice according to the amount of cells;

(2) The lysed liquid was transferred to CS column, and centrifuged at 12,000 rpm for 2 min;

(3) 1 volume of 70% ethanol was added to the filtrate, mixed well and transferred to CR3, and centrifuged at 12,000 rpm for 1 min;

(4) The filtrate was discarded, 350 $\mu$L of RW1 was added to CR3, and centrifugation was carried out at 12,000 rpm for 1 min;

(5) The filtrate was discarded, 80 $\mu$L of DNase I working solution was added to CR3, and allowed to stand for 15 min at room temperature;

(6) To the CR3, 350 μL of RW1 was added, and centrifugation was carried out at 12,000 rpm for 1 min;

(7) The filtrate was discarded, 500 μL of RW was added to CR3, allowed to stand at room temperature for 2 min, and centrifugation was carried out at 12,000 rpm for 1 min;

(8) The step (7) was repeated;

(9) The filtrate was discarded, without adding any liquid, centrifugation was carried out at 12,000 rpm for 1 min to remove the residual rinse solution;

(10) The CR3 was transferred to a new centrifuge tube, dried in the air to remove the residual alcohol, 30 μL of RNase-free water was added, allowed to stand at room temperature for 2 min, and centrifuged at 12,000 rpm for 2 min to obtain RNA;

(11) The RNA concentration was measured with Nanodrop UV spectrophotometer;

(12) It was directly subjected to cDNA synthesis or short-term storage in -80°C refrigerator.

2. Reverse transcription of cDNA

A reverse transcription kit was used to synthesize single-stranded cDNA, and the specific steps were as follows:
(1) After adding oligo dt Primer, dNTP mixture, RNA template and water, the reaction was carried out at 65°C for 5 min. The 10 μL reaction system was as follows:

| Reagent | Volume |
| --- | --- |
| oligo dt Primer | 1 μL |
| dNTP mixture | 1 μL |
| RNA | 2 μL |
| RNase-free water | added to 1 μL |

(2) After the reaction in step (1) was completed, it was placed in ice immediately. The amplification reaction of reverse transcription was performed at 42°C for 60 min; 70°C for 15 min.

| Reagent | Volume |
| --- | --- |
| 5×Primscript Buffer | 4 μL |
| RNase inhibitor | 0.5 μL |
| Primscript Rtase | 0.5 μL |
| Solution from step (1) | 10 μL |
| RNase-free water | 5 μL |

(3) After the reaction was completed, it was placed in ice immediately, and stored in a 4°C refrigerator for a short period of time.

3. Real-time quantitative PCR (qRCR)

TOYOBO Realtime PCR Kit was used for qPCR, and a 10 μL reaction system was prepared as follows:

| Reagent | Volume |
| --- | --- |
| 2×SYBR Green Mix | 5 μL |
| F primer | 0.3 μL |
| R primer | 0.3 μL |
| cDNA | 1 μL |
| Water | 3.4 μL |

Reaction program: pre-denaturation at 95°C for 1 min; denaturation at 95°C for 15 s, annealing and extension at 60°C for 45 s, 40 cycles; dissolution curve. The results as shown were average values of three replicate experiments.

4. Information of the required reagents:

RNA extraction kit (Tiangen, DP430), cDNA reverse transcription kit (TAKARA, 6110A), SYBR Green Realtime PCR kit (TOYOBO, QPS to 201). The primer information involved was as follows:

| Gene Name | Forward (5' to 3') | Reverse (5' to 3') |
| --- | --- | --- |
| IDO | GCCAGCTTCGAGAAAGAGTTG | ATCCCAGAACTAGACGTGCAA |
| MMP1 | AAAATTACACGCCAGATTTGCC | GGTGTGACATTACTCCAGAGTTG |
| PDL1 | GGACAAGCAGTGACCATCAAG | CCCAGAATTACCAAGTGAGTCCT |
| PGE2 | GGCGGGCGTTTCGAACTT | CGGGTCCATGTTCGCTCC |
| GAPDH | CTCTGCTCCTCCTGTTCGAC | CGACCAAATCCGTTGACTCC |

The detection results were shown in FIGS. 2A to 2D. The results showed that the relative expression level of MMP1 in the M cells of Preparation Example 1 was 103.3014, and the relative expression level of MMP1 in the MSC of Comparative Example 1 was 1.151253. It can be seen that the MMP1 expression level of the M cells obtained by the method of the present invention was significantly increased, which was nearly 90 times that of the MSC obtained by the prior art technical method. MMP1 is a kind of proteolytic enzyme that can degrade a variety of connective tissue components, including gelatin, fibronectin, collagen, mucin, and laminin. Studies have confirmed that MMP1 plays an important role in fibrosis. Therefore, it can be expected that the mesenchymal stem cells of the present invention have better activity in the treatment of liver fibrosis, pulmonary fibrosis and the like.

Examples 1-3: Preparation of iPSC-derived M cells and characterization identification thereof

(A) Generation of embryoid body (EB)

Embryoid bodies (EBs) were prepared from human induced pluripotent stem cells (iPS) using the same method as in Preparation Example 1.1, wherein the EB culture medium (first culture medium) was: KO-DMEM + 20% KOSR + 1% NEAA + 1 % Glutamine + 5 ng/ml bFGF

(2) Adherent culture of EB spheres

EB spheres were cultured in a VN-coated 6-well plate, the M cell culture medium (second culture medium):$\alpha$-MEM with addition of 1% (v/v) Ultroser G, 5% (v/v) KOSR, 1% (v/v) NEAA, 1% (v/v) GlutaMAX, 5 ng/ml bFGF, 5 ng/ml TGF$\beta$. The culture medium was changed every two days until the 14th day.

(3) Passage of M cells

Digestion was performed with Tryple, MSC culture medium was added to a new dish, the cells were inoculation into the new dish; the 5th generation was used for subsequent operations (e.g., cell therapy), or cryopreservation using Cryostor CS10.

The information of the above-required reagents was as follows

VN (Gibco, A14700), $\alpha$-MEM (Gibco, 12561 to 049), KO-DMEM (Gibco, A12861 to 01), KOSR (Gibco, A3020902), NEAA (Gibco, 11140050), Glutamine (Gibco, A1286001), Ultroser G (Pall, 15950-017), DPBS (Gibco, A1285801), Dispase (Gibco, 17105041), Tryple (Gibco, A1285901), bFGF (RD, 233 to FB), TGF$\beta$ (RD, 240 to B)

The cells obtained by the above method could be referred to as ips-M cells.

(4) Observation of cell morphology

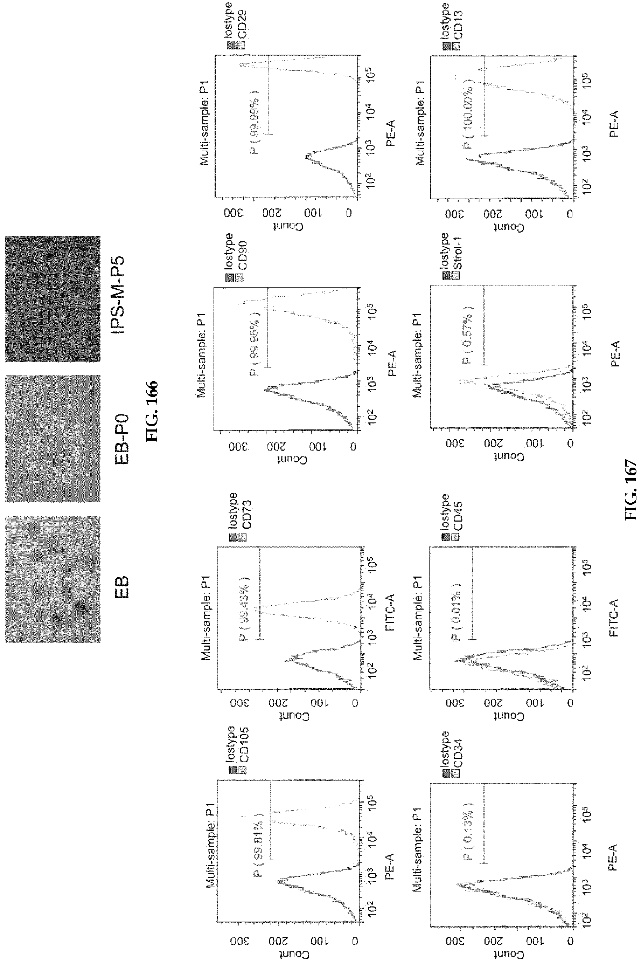

FIG. 166 showed the cell morphology of embryoid body and ips-M cells at P0 and P5 generations, and the results showed that the M cells with normal morphology were obtained.

(5) Positive and negative surface markers of M cells detected by flow cytometry

The expression of surface markers of ips-M cells was determined by flow cytometry, and the specific method was shown in Example 1-1. The results of the positive and negative surface markers for the ips-M cells were mostly similar to the M cells from Preparation Example 1 (FIG. 167).

(6) Expression of PGE2 and MMP1 detected by qPCR

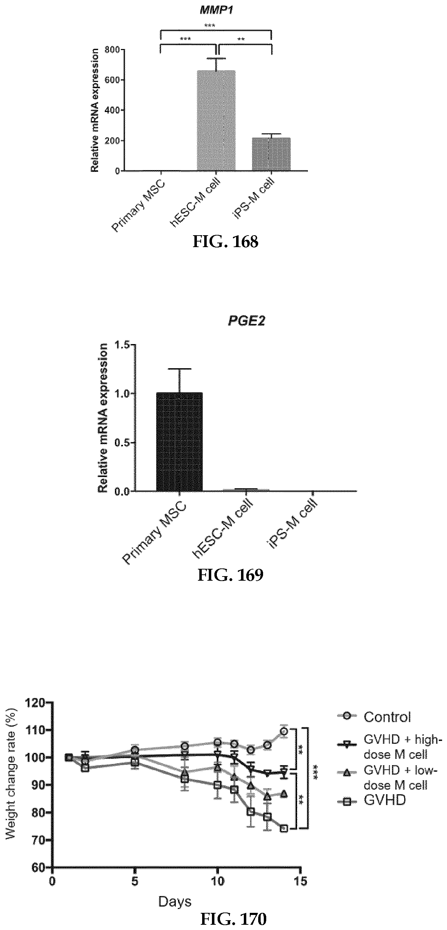

The expression of PGE2 and MMP1 was determined by qPCR, and the specific method was shown in Example 1-2. The expression level of MMP1 in the ips-M cells was more than 10 times higher than that in the primary mesenchymal stem cells (FIG. 168). The qPCR results of PGE2 were shown in FIG. 169.

Example 2: M cells derived from human embryonic stem cells (hESCs) prepared in different culture medium ranges and determination of their properties

1. Cell preparation

1. EB culture

1.1 Preparation of EB culture medium

(1) KO-DMEM + 20% KOSR + 1% NEAA + 1% Glutamine + 5 ng/ml bFGF; or

(2) Formulated according to the specific groupings described below.

1.2 Formation of EB

1.2.1 The original culture solution was pumped off, and 1 mL of DPBS was added for washing.

1.2.2 The digestion was performed for 3 min by adding 1 mL of Dispase (the edges of the clone clump were curled up), indicating that the digestion was complete.

1.2.3 The enzyme solution was discarded, 1 mL of KO-DMEM was added, and lines were drawn arranged in parallel crosses by holding a 5 mL centrifuge tube perpendicular to the plate.

1.2.4 The pipette tip was moistened, the liquid in the 6-well plate was pipetted, transferred to a 15 mL centrifuge tube, and centrifuged at 800 rpm for 3 min.

1.2.5 The supernatant was discarded, the cells were resuspended with EB culture medium, then transferred to a 10 cm low-attachment culture dish, and cultured in a 37°C incubator.

1.2.6 The culture medium was changed every day until the 5th day.

2. Culture of EB-MSC

2.1 Culture of EB-MSC

2.1.1 A 6-well plate was precoated with 1 mg/ml VN

2.1.2 All the EB spheres were transferred to a 15 mL centrifuge tube and allowed to stand for 5 to 10 minutes.

2.1.3 After the EB spheres settled, the supernatant was removed with a pump, and 1 mL of EB culture medium was taken to resuspended them, and about 10 EB spheres were added to each well and inoculated in the VN-plated 6-well plate.

2.1.4 After shaken well by "figure-8 method", it was placed in an incubator.

2.1.5 The culture medium was changed every two days until the 14th day.

3. Culture of M cells

3.1 Preparation of M cell culture medium

○,[1]Preparation of M cell culture medium: adding 1% (v/v) Ultraser G, 5% (v/v) KOSR, 1% (v/v) NEAA, 1% (v/v) GlutaMAX, 5 ng/ml bFGF, 5 ng/ml TGFβ to α-MEM; or,

○,[2]Formulated according to the specific groupings described below.

3.2 Passage of M cells

3.2.1 The cells were washed with 1 mL of DPBS, the digestion was performed at 37°C for about 3 minutes by adding 1 mL of Tryple, the wall of the dish was tapped slightly to detach the cells, and 1 mL of DPBS was added to stop the digestion.

3.2.2 The cells were collected, and centrifuged at 1200 rpm for 3 min.

3.2.3 The supernatant was discarded, the cells were resuspended in 5 mL of M cell culture medium, the cells were filtered with a 70 μm cell sieve, and counted.

3.2.4 The cells were inoculated in a 10 cm culture vessel at a density of $2 \times 10^5$ cells/cm$^2$, and recorded as M cell P1.

3.2.5 According to the same method, the M cells were passaged to the 5th generation for detection.

4. Information of the above required reagents

VN (Gibco, A14700)

α-MEM (Gibco, 12561 to 049)

KO-DMEM (Gibco, A12861 to 01)

KOSR (Gibco, A3020902)

NEAA (Gibco, 11140050)

Glutamine (Gibco, A1286001)

Ultroser G (Pall, 15950-017)

DPBS (Gibco, A1285801)

Dispase (Gibco, 17105041)

Tryple (Gibco, A1285901)

bFGF (RD, 233 to FB)

TGFβ (RD, 240 to B)

EGF (Solarbio, P00033)

PDGF (Solarbio, P00031)

VEGF (Solarbio, P00063)

Ascorbic acid (Selleck, Selleck)

5. Information of the above-required instruments

| Reagent/Equi pment | Manufacturer | Cat. No. |
|---|---|---|
| $CO_2$ incubator | Thermo | 3131 |
| Biological safety cabinet | Haier | HR40-IIA2 |
| Centrifuge | Xiangyi | TD25-WS |
| Cell counter | Life technologies | Countess II FL |
| Microscope | Leica | DMi1 |
| Vacuum pump | KNF | N86KN.18 |
| 100 to 1000 $\mu$L pipette | eppendorf | J46045F |
| 20 to 200$\mu$L pipette | eppendorf | L22687F |
| 10 to 100$\mu$L pipette | eppendorf | M46287F |
| 0.5 to 10$\mu$L pipette | eppendorf | K19138F |
| 0.1 to 2.5$\mu$L pipette | eppendorf | L22220F |
| Refrigerator | Haier | BCD-256KDC |
| Low-attachment 10cm dish | Corning | 3262 |
| 10cm dish | Corning | 430167 |
| 6-well plate | Corning | 3335 |

2. Flow cytometry detection of M cell surface proteins

1. The culture supernatant was discarded, washing was carried out with PBS, Tryple was added to perform digesting for 3 min, and DPBS was added for termination.
2. The cell suspension was collected and centrifuged at 1200 rpm for 3 min.
3. The supernatant was discarded, the cells were resuspended in DPBS, filtered through a 40 $\mu$m cell sieve to remove cell clusters, counted, and subpackaged, $2 \times 10^6$ cells per tube.
4. Centrifugation was carried out at 1200 rpm for 3 min.
5. After blocking with 2% BSA blocking solution for 20 min, centrifugation was carried out at 1200 rpm for 3 min.
6. The supernatant was discarded, the cells were resuspended with 100 $\mu$L of 1% BSA antibody diluent, the directly labeled antibody was added, and incubated at room temperature for 30 to 45 minutes.
7. After washing three times with 1 mL of PBS, centrifugation was carried out at 1200 rpm for 3 min, and the supernatant was discarded.
8. After resuspending in 300 $\mu$L of DPBS, the cells were filtered with a 40 $\mu$m cell sieve, and loaded for detection.
9. Information of the required antibodies was as follows:

| Name | Company | Cat. No. |
|---|---|---|
| CXCL1 | RD | IC275P |
| CD105 | BioLegend | 323206 |

(continued)

| Name | Company | Cat. No. |
|---|---|---|
| CD73 | eBioscience | 11-0739-42 |
| CD90 | eBioscience | 12-0909-42 |
| CD13 | BD | 560998 |
| CD29 | BioLegend | 303004 |
| CD44 | BD | 561858 |
| CD166 | BD | 560903 |
| HLA-ABC | BD | 560965 |
| CD34 | BD | 555822 |
| CD45 | eBioscience | 11-9459-42 |
| CD133 | BD | 566593 |
| FGFR2 | RD | FAB684G |
| CD271 | BD | 560927 |
| Stro-1 | abcam | ab190282 |
| CXCR4(CD184) | BD | 561733 |
| PE-IgG 1 Isotype Control | BD | 555749 |
| FITC-IgG 1 Isotype Control | BD | 555748 |

10. Information of the required instruments

| Reagent/Equi pment | Manufacturer | Cat. No. |
|---|---|---|
| $CO_2$ incubator | Thermo | 3131 |
| Biological safety cabinet | Haier | HR40-IIA2 |
| Centrifuge | Xiangyi | TD25-WS |
| Cell counter | Life technologies | Countess II FL |
| Vacuum pump | KNF | N86KN.18 |
| 100 to 1000 μL pipette | eppendorf | J46045F |
| 20 to 200μL pipette | eppendorf | L22687F |
| 10 to 100μL pipette | eppendorf | M46287F |
| 0.5 to 10μL pipette | eppendorf | K19138F |
| 0.1 to 2.5μL pipette | eppendorf | L22220F |
| Flow cytometer | Beckman | Cyto FLEX |

3. Real-time PCR

3.1 Extraction of cellular RNA

RNA extraction kit was used for extraction, and the specific steps were as follows:

1. 10 μL of β-mercaptoethanol was added to per ml of RL lysis solution, and the cells were lysed on ice according to the amount of cells;

2. The liquid after the lysis was transferred to a CS column, and centrifuged at 12,000 rpm for 2 min;

3. 1 volume of 70% ethanol was added to the filtrate, mixed well, transferred to CR3, and centrifuged at 12,000 rpm for 1 min;

4. The filtrate was discarded, 350 μL of RW1 was added to CR3, and centrifuged at 12,000 rpm for 1 min;

5. The filtrate was discarded, 80 μL of DNase I working solution was added to CR3, allowed to stand at room temperature for 15 min;

6. 350 μL of RW1 was added to CR3, and centrifuged at 12,000 rpm for 1 min;

7. The filtrate was discarded, 500 μL of RW was added to CR3, allowed to stand at room temperature for 2 min, and centrifuged at 12,000 rpm for 1 min;

8. The step 7 was repeated;

9. The filtrate was discarded, without adding any liquid, centrifugation was carried out at 12,000 rpm for 1 min to remove the residual rinse solution;

10. The CR3 was transferred to a new centrifuge tube, dried in the air to remove the residual alcohol, 30 μL of RNase-free water was added, allowed to stand at room temperature for 2 minutes, and centrifuged at 12,000 rpm for 2 minutes to obtain RNA;

11. RNA concentration was measured with Nanodrop UV spectrophotometer;

12. It was subjected to direct cDNA synthesis or short-time storage in -80°C refrigerator.

3.2 Reverse transcription of cDNA

A reverse transcription kit was used to synthesize single-stranded cDNA, and the specific steps were as follows:
1. After adding oligo dt Primer, dNTP mixture, RNA template and water, the reaction was carried out at 65°C for 5 min, and the 10 μL reaction system was as follows:

| Reagent | Volume |
| --- | --- |
| oligo dt Primer | 1 μL |
| dNTP mixture | 1 μL |
| RNA | 2 μL |
| RNase-free water | added to 1 μL |

2. After the reaction in step 1 was completed, it was placed in ice immediately. The amplification reaction for reverse transcription was performed at 42°C for 60 min; 70°C for 15 min.

| Reagent | Volume |
| --- | --- |
| 5 ×Primscript Buffer | 4 μL |
| RNase inhibitor | 0.5 μL |
| Primscript Rtase | 0.5 μL |
| Solution from step (1) | 10 μL |
| RNase-free water | 5 μL |

3. After the reaction was completed, it was placed in ice immediately and stored in a 4°C refrigerator for a short period of time.

3.3 Real-time quantitative PCR (qRCR)

TOYOBO Realtime PCR Kit was used for qPCR, and a 10 μL reaction system as follows was prepared:

| Reagent | Volume |
|---|---|
| 2×SYBR Green Mix | 5 μL |
| F primer | 0.3 μL |
| R primer | 0.3 μL |
| cDNA | 1 μL |
| Water | 3.4 μL |

Reaction program: pre-denaturation at 95°C for 1 min; denaturation at 95°C for 15 s, annealing and extension at 60°C for 45 s, 40 cycles; dissolution curve.

3.4 Information of the required reagents:

RNA extraction kit (Tiangen, DP430), cDNA reverse transcription kit (TAKARA, 6110A), SYBR Green Realtime PCR kit (TOYOBO, QPS-201)

Required primer sequence

| Gene Name | Forward (5' to 3') | Reserve (5' to 3') |
|---|---|---|
| *MMP1* | AAAATTACACGCCAGATTTGCC | GGTGTGACATTACTCCAGAGTTG |
| *PGE2* | GGCGGGCGTTTCGAACTT | CGGGTCCATGTTCGCTCC |
| *GAPDH* | CTCTGCTCCTCCTGTTCGAC | CGACCAAATCCGTTGACTCC |

3.5 Information of the required instruments:

| Reagent/Equi pment | Manufacturer | Cat. No. |
|---|---|---|
| 100 to 1000 μL pipette | eppendorf | J46045F |
| 20 to 200μL pipette | eppendorf | L22687F |
| 10 to 100μL pipette | eppendorf | M46287F |
| 0.5 to 10μL pipette | eppendorf | K19138F |
| 0.1 to 2.5μL pipette | eppendorf | L22220F |
| Refrigerate centrifuge | Beckman | Allegra X-15R |
| Fluorescence quantitative PCR instrument | Agilent | M3005P |

4. Statistical analysis

One-way ANOVA and T-TEST in Prism 7.0 statistical analysis software were used for variance analysis and significance test, and the experimental data were expressed as mean ± standard error (Mean ± SE). *, $p < 0.05$; **, $p < 0.01$; ***, $p < 0.001$.

5. Experimental results

(1) Concentration ranges of the first culture medium

1. The first culture medium: the concentrations were set as follows

Table 2-1: Contents of each component of the first culture medium.

| Component | ko-DMEM (mL) | KOSR (%) | NEAA (mM) | GlutaMax (mM) | bFGF (ng/mL) |
|---|---|---|---|---|---|
| Formulation 1 | 39 | 20 | 0.1 | 2 | 10 |
| Formulation 2 | 41.75 | 3 | 0.1 | 1 | 1 |
| Formulation 3 | 33 | 30 | 0.5 | 5 | 100 |
| Formulation 4 | 39 | 20 | 0.1 | 2 | 1 |
| Formulation 5 | 39 | 20 | 0.1 | 2 | 100 |

2. Steps: The embryoid bodies were cultured in suspension using the aforementioned five culture media, and then M cells were cultured using the M cell culture medium, and the detection was carried out after passage to the 5th generation.

3. Observation of cell morphology

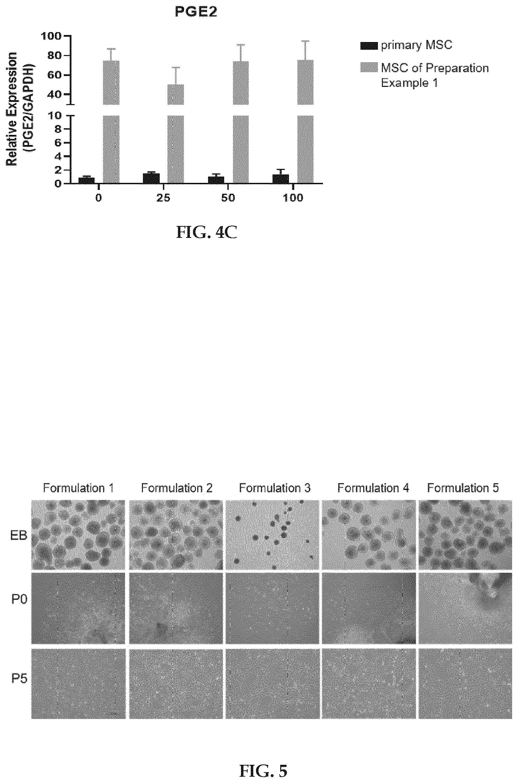

EB spheres were obtained from all formulations, and the cells crawl out after adherence. When passaged to the 5th generation, the M cells with adherent growth and spindle shape were formed. Formulation 3 differed from the other formulations in forming EB spheres, but the M cells with normal morphology were obtained (FIG. 5).

4. Flow cytometry for positive and negative surface markers of M cells

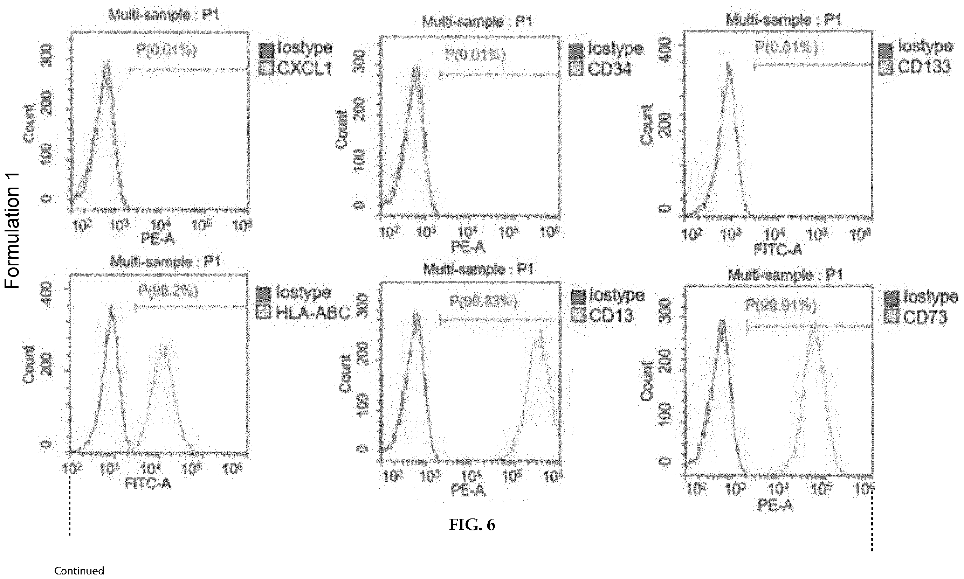

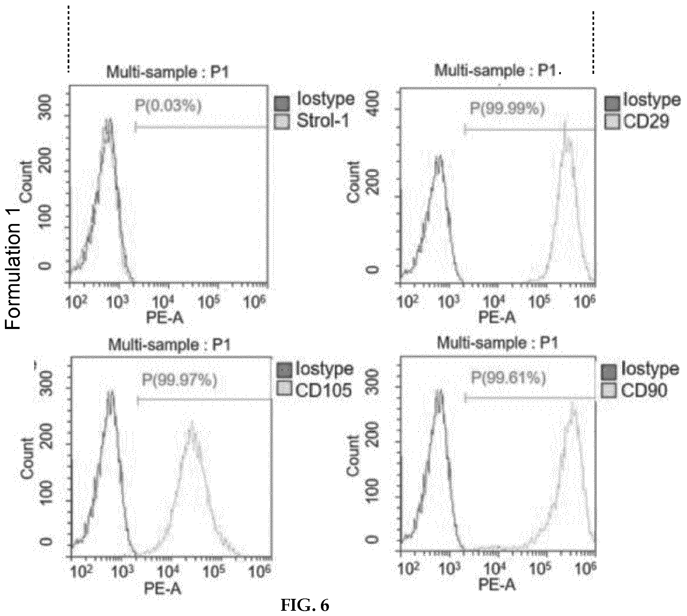

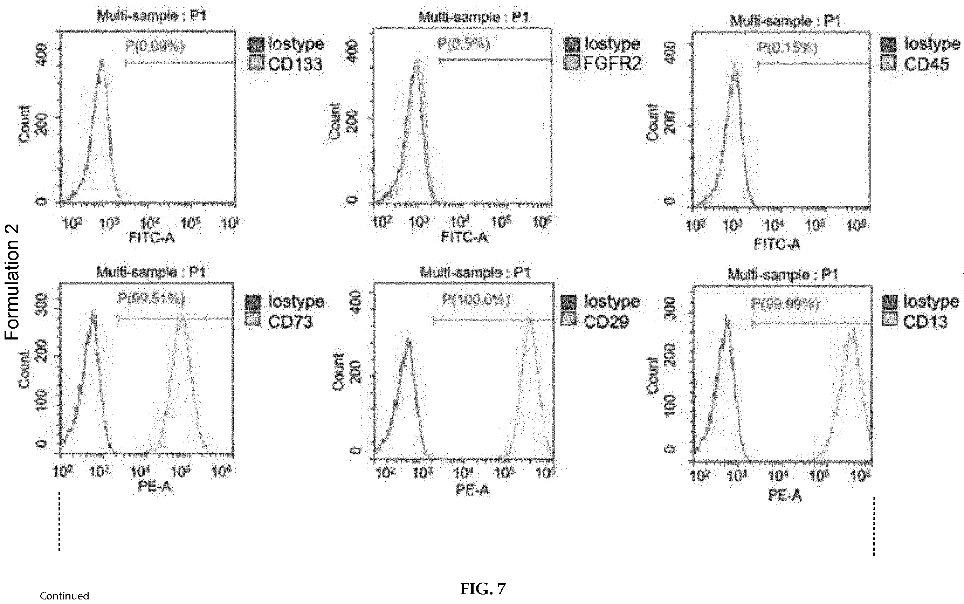

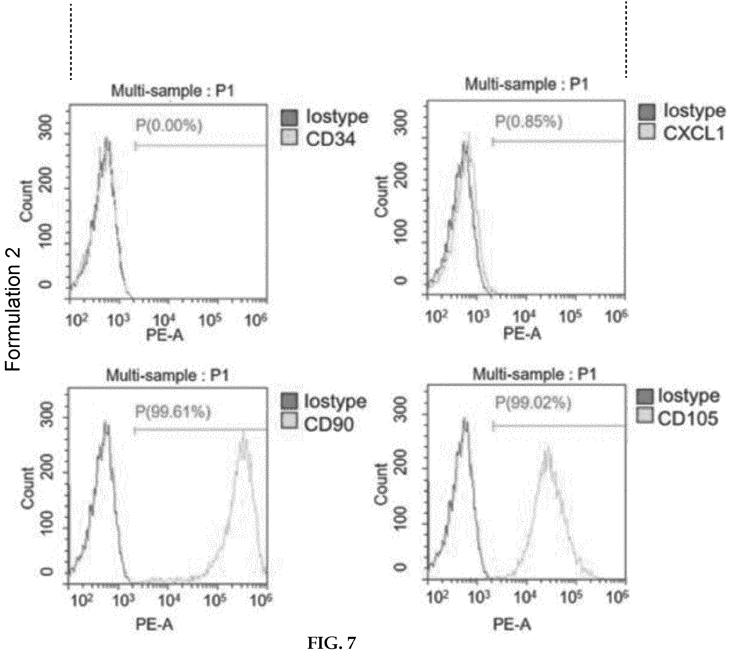

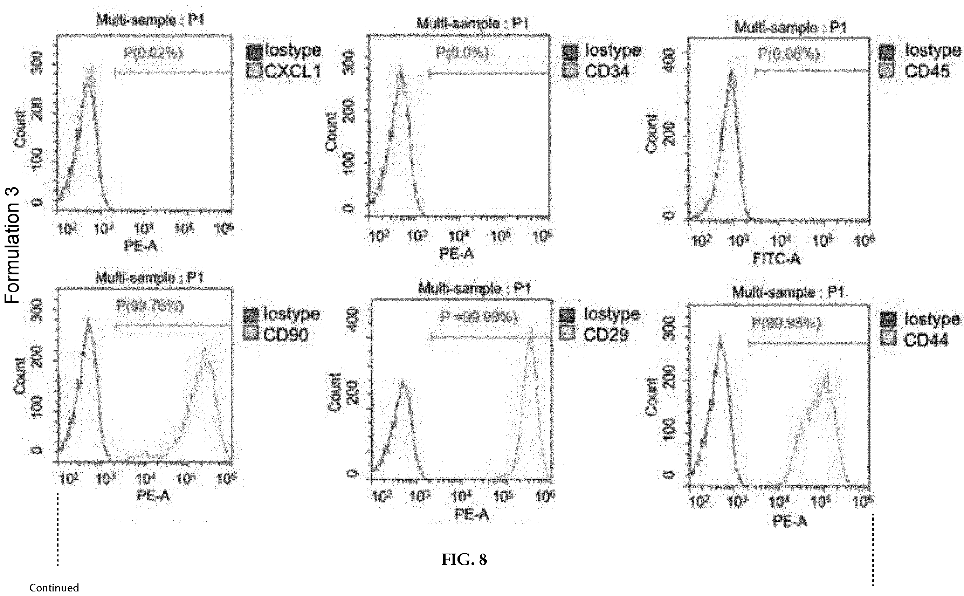

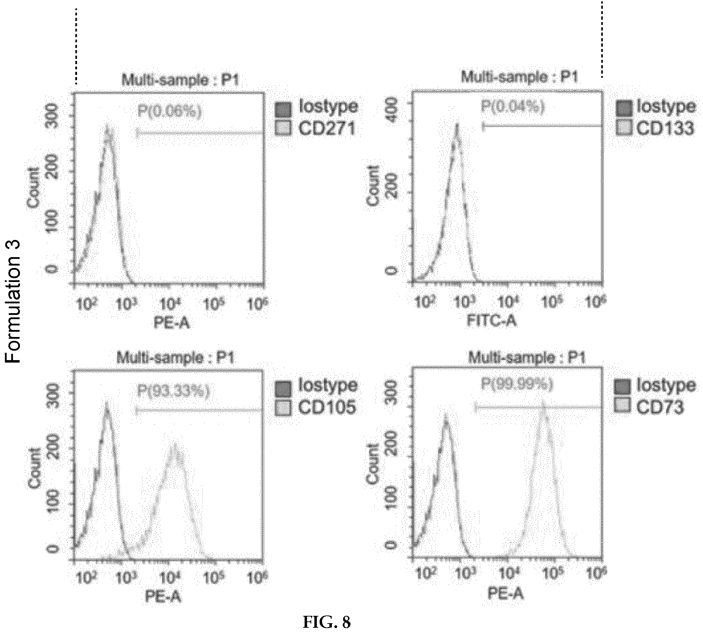

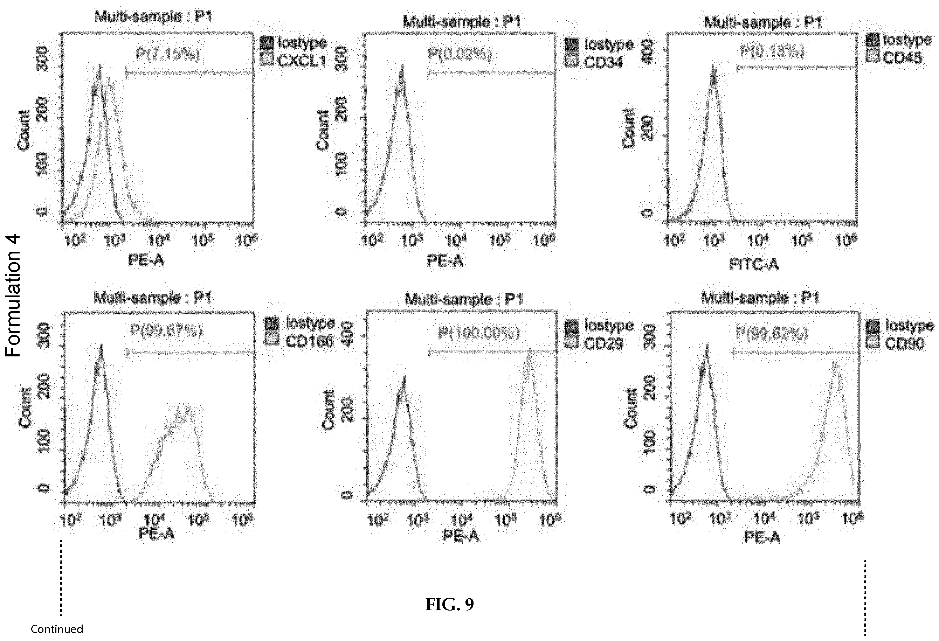

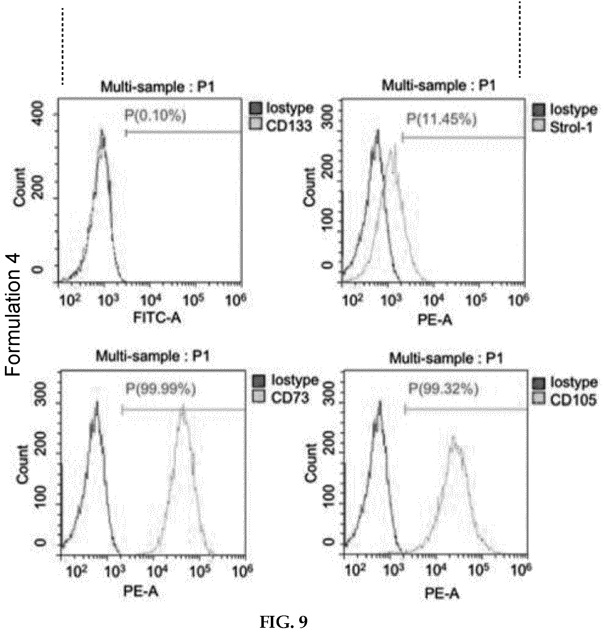

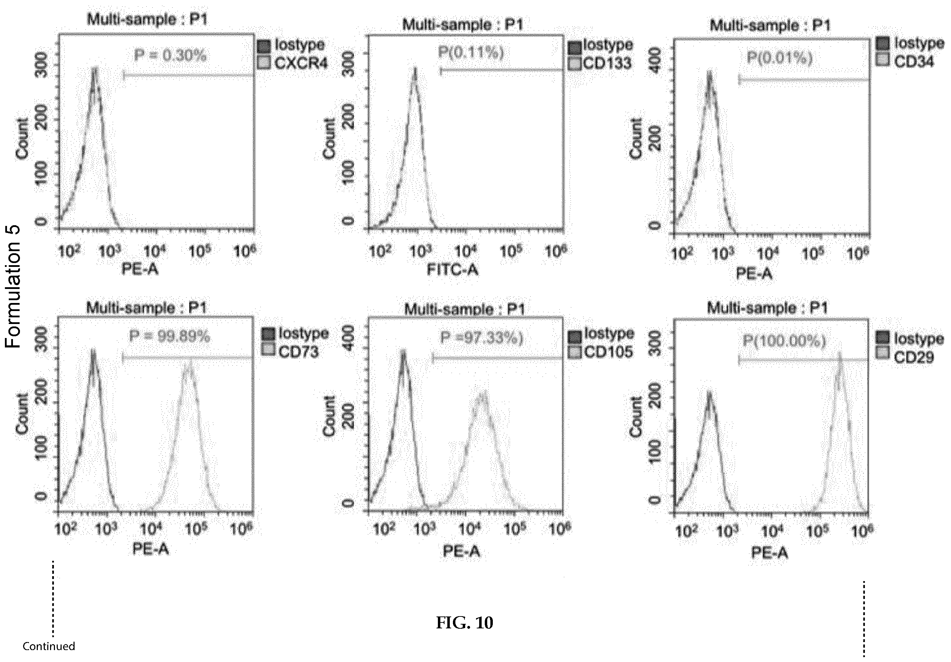

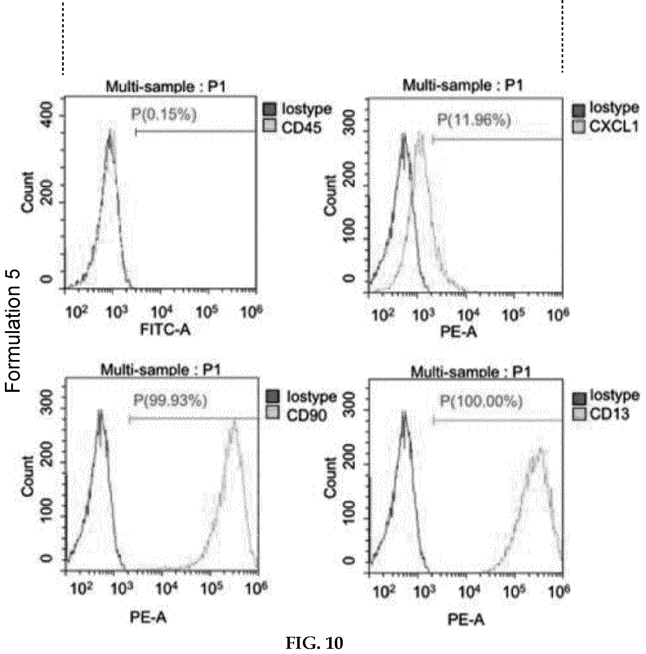

The results of positive and negative surface markers of the M cells prepared with different formulations were mostly similar to those of the M cells of Preparation Example 1 (FIGS. 6 to 10, Table 2-2).

Table 2-2: Statistics of flow cytometry results for M cells

| | CD13 3 | FGFR 2 | CD4 5 | CXCL 1 | CD34 | HLA-ABC | CD13 | CD7 3 | CD10 5 | CD29 | CD9 0 | CD4 4 | CD27 1 | CD16 6 | Strol-1 | CXCR 4 |
|---|---|---|---|---|---|---|---|---|---|---|---|---|---|---|---|---|
| Formulation 1 | 0.01 | | | 0.01 | 0.01 | 98.20 | 99.83 | 99.9 1 | 99.97 | 99.99 | 99.6 1 | | | | 0.03 | |
| Formulation 2 | 0.09 | 0.50 | 0.15 | 0.85 | 0.00 | | 99.99 | 99.5 1 | 99.02 | 100.0 0 | 99.6 1 | | | | | |
| Formulation 3 | 0.04 | | 0.06 | 0.02 | 0.00 | | | 99.9 9 | 93.33 | 99.99 | 99.7 6 | 99.9 5 | 0.06 | | | |
| Formulation 4 | 0.10 | | 0.13 | 7.15 | 0.02 | | | 99.9 8 | 98.32 | 100.0 0 | 99.6 2 | | | 99.67 | 11.45 | |
| Formulation 5 | 0.11 | | 0.15 | 11.96 | 0.01 | | 100.0 0 | 99.8 9 | 97.33 | 100.0 0 | 99.9 3 | | | | | 0.30 |

5. Expression of PGE2 and MMP1 detected by qPCR

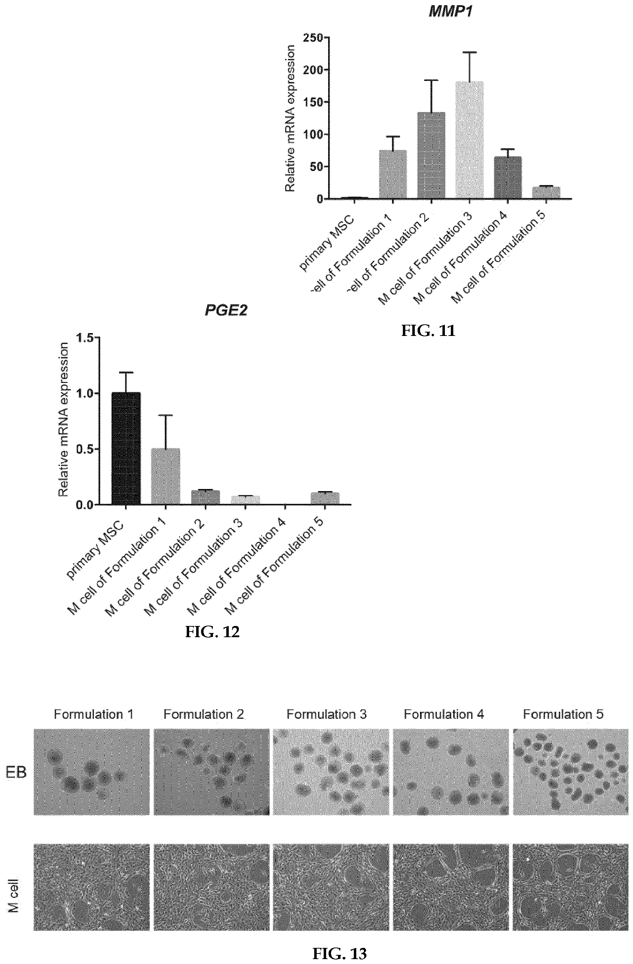

The expression levels of MMP1 for Formulations 1 to 5 of the first culture medium were more than 10 times that of the primary mesenchymal stem cells (FIG. 11, Table 2-3). The qPCR results of PGE2 were shown in FIG. 12 and Table 2-4.

Table 2-3: Statistics of qPCR results of MMP1 of M cells for different formulations of the first culture medium

| | Primary MSC | Formulation 1 | Formulation 2 | Formulation 3 | Formulation 4 | Formulation 5 |
|---|---|---|---|---|---|---|
| Relative mRNA expression level | 1.15 | 74.16 | 132.91 | 180.80 | 64.13 | 17.41 |
| | 1.42 | 90.35 | 95.37 | 200.04 | 70.43 | 18.91 |
| | 0.81 | 55.31 | 153.64 | 164.31 | 58.32 | 16.07 |

Table 2-4: Statistics of qPCR results of PGE2 of M cells for different formulations of the first culture medium

| | Primary MSC | Formulation 1 | Formulation 2 | Formulation 3 | Formulation 4 | Formulation 5 |
|---|---|---|---|---|---|---|
| Relative mRNA expression level | 1.01 | 0.40 | 0.12 | 0.07 | 0 | 0.10 |
| | 1.25 | 0.32 | 0.10 | 0.04 | 0 | 0.19 |
| | 0.80 | 0.46 | 0.16 | 0.33 | 0 | 0.07 |

(2) Concentration ranges of NEAA in the first culture medium

1. NEAA concentration setting in the first culture medium

The concentration gradients for the NEAA concentration were set as follows:

Table 2-5: Table of NEAA content in the first culture medium.

| Component | ko-DMEM (mL) | KOSR (%) | NEAA (mM) | GlutaMax (mM) | bFGF (ng/mL) |
|---|---|---|---|---|---|
| Formulation 1 | 39 | 20 | 0.1 | 2 | 10 |
| Formulation 2 | 38.5 | 20 | 0.2 | 2 | 10 |
| Formulation 3 | 38 | 20 | 0.3 | 2 | 10 |
| Formulation 4 | 37.5 | 20 | 0.4 | 2 | 10 |
| Formulation 5 | 37 | 20 | 0.5 | 2 | 10 |

2. Steps: The aforementioned five formulations were used for the suspension culture of embryoid bodies, and then M cells were cultured with the M cell culture medium, and detected after passaged to the 5th generation.

3. Morphological observation

EB spheres were obtained from all formulations, and cells crawl out after adherence. When passaged to the 5th generation, the M cells with adherent growth and spindle shape were formed. When EB spheres were formed, although there were some dead cells in Formulation 5, the M cells with normal morphology were obtained (FIG. 13).

4. Flow cytometry for positive and negative surface markers of M cells

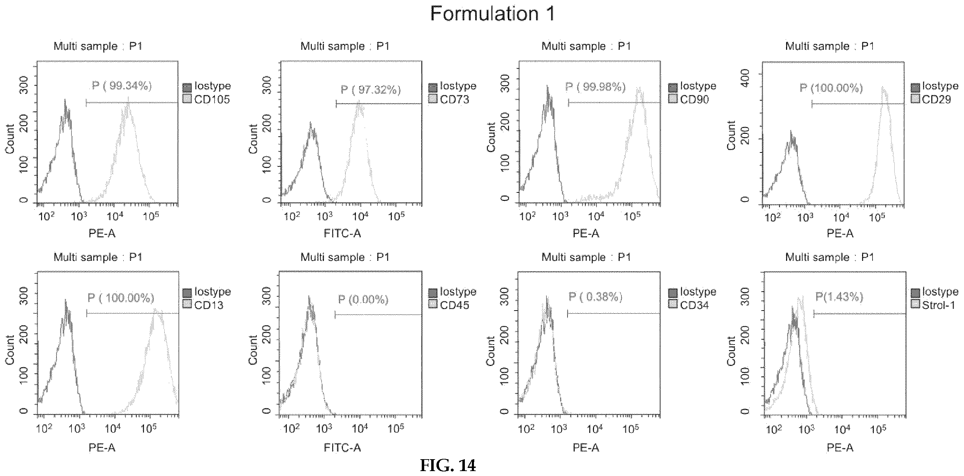

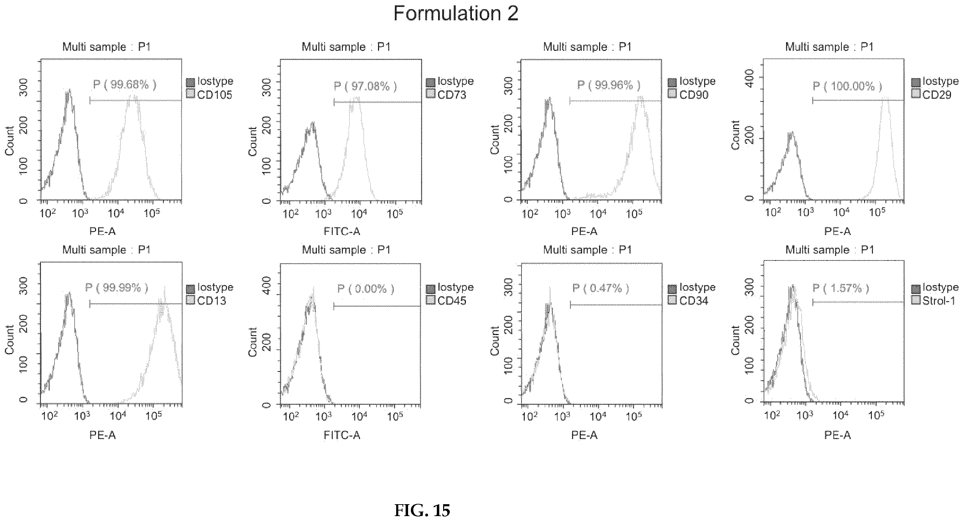

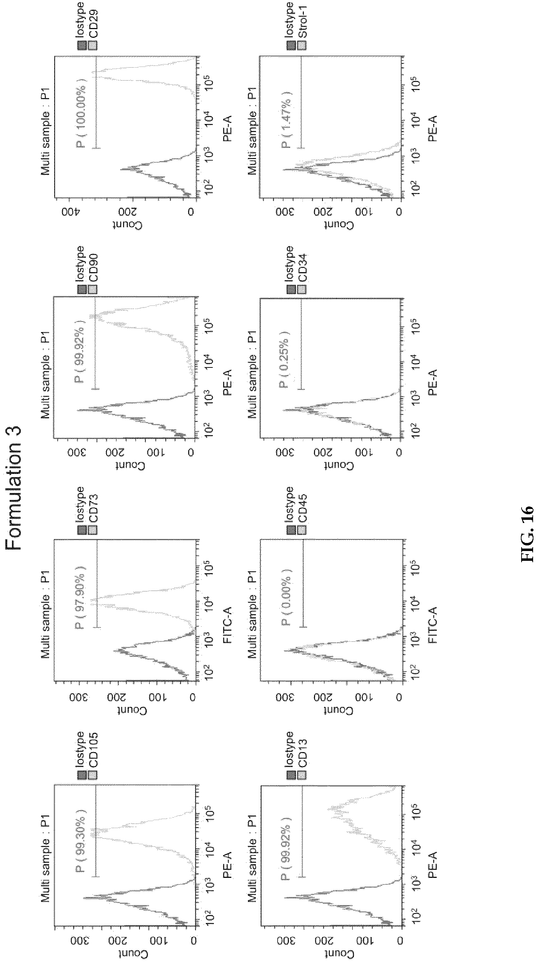

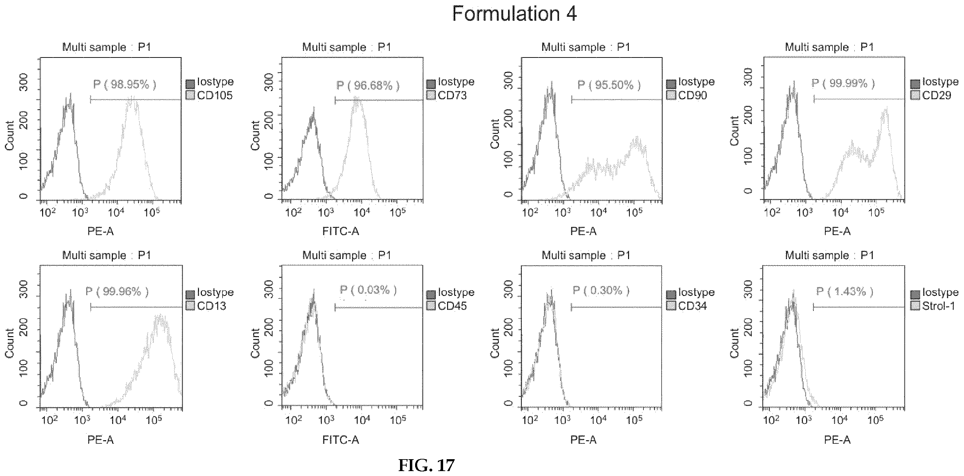

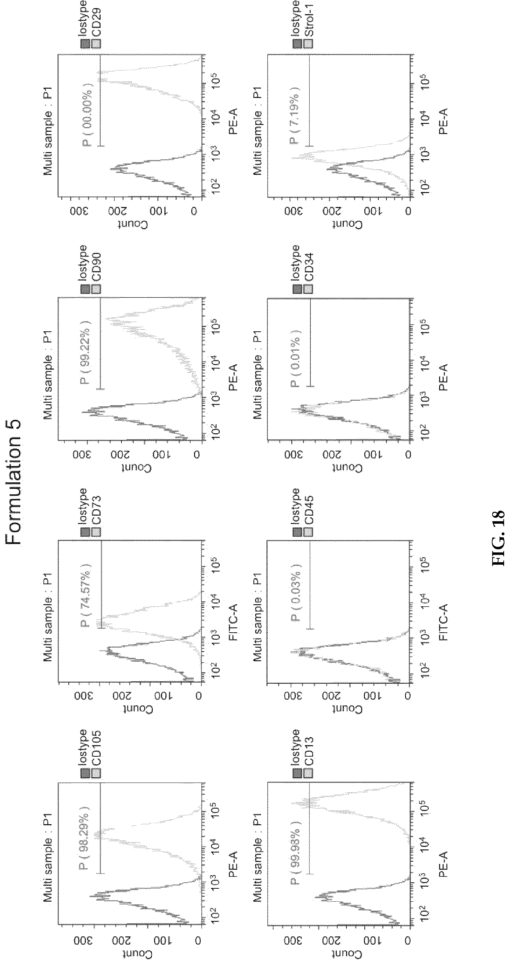

The flow cytometry results showed that most of the detected indicators were similar to those of the M cells of Preparation Example 1 (Table 2-6, FIGS. 14 to 18).

Table 2-6: Statistics of flow cytometry results for M cells

| | CD45 | CD34 | CD13 | CD73 | CD105 | CD29 | CD90 | Strol-1 |
|---|---|---|---|---|---|---|---|---|
| Formulation 1 | 0.00 | 0.38 | 100.00 | 97.32 | 99.34 | 100.00 | 99.98 | 1.43 |
| Formulation 2 | 0.00 | 0.47 | 99.99 | 97.08 | 99.68 | 100.00 | 99.96 | 1.57 |
| Formulation 3 | 0.00 | 0.25 | 99.92 | 97.90 | 99.30 | 100.00 | 99.92 | 1.47 |

(continued)

|  | CD45 | CD34 | CD13 | CD73 | CD105 | CD29 | CD90 | Strol-1 |
|---|---|---|---|---|---|---|---|---|
| Formulation 4 | 0.03 | 0.30 | 99.96 | 96.68 | 98.95 | 99.99 | 95.50 | 1.43 |
| Formulation 5 | 0.03 | 0.01 | 99.98 | 74.57 | 98.29 | 100.00 | 99.22 | 7.19 |

5. Expression of PGE2 and MMP1 detected by qPCR

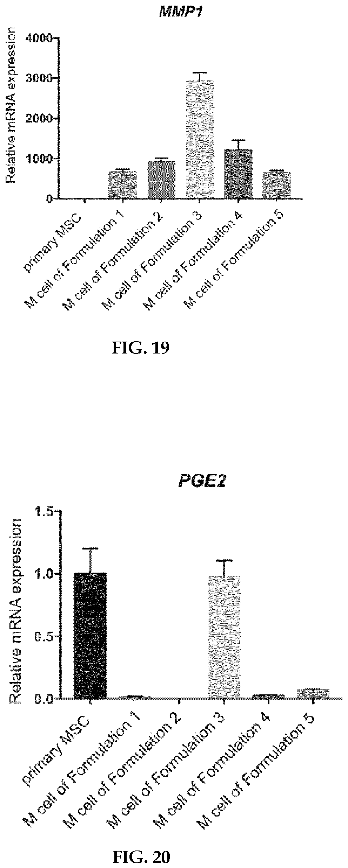

The MMP1 expression levels of M cells produced with Formulations 1 to 5 of the first culture medium were all more than 10 times that of the primary mesenchymal stem cells (Table 2-7, FIG. 19). The PGE2 detection results were shown in Table 2-8 and FIG. 20.

Table 2-7: Statistics of qPCR results of MMP1 of M cells for different formulations of the first culture medium

|  | Primary MSC | Formulation 1 | Formulation 2 | Formulation 3 | Formulation 4 | Formulation 5 |
|---|---|---|---|---|---|---|
| Relative mRNA expression level | 1.31 | 657.53 | 911.53 | 2920.70 | 1215.30 | 634.95 |
|  | 1.01 | 627.45 | 821.62 | 3100.04 | 1432.57 | 618.93 |
|  | 0.78 | 683.72 | 957.15 | 2604.14 | 1058.24 | 661.74 |

Table 2-8: Statistics of qPCR results of PGE2 of M cells for different formulations of the first culture medium

|  | Primary MSC | Formulation 1 | Formulation 2 | Formulation 3 | Formulation 4 | Formulation 5 |
|---|---|---|---|---|---|---|
| Relative mRNA | 1.01 | 0.02 | 0 | 0.98 | 0.03 | 0.07 |
|  | 1.25 | 0.01 | 0 | 1.04 | 0.01 | 0.14 |
| expression level | 0.80 | 0.04 | 0 | 0.73 | 0.06 | 0.05 |

(3) Concentration ranges of the second culture medium

1. Concentration ranges of each component in the second culture medium

Table 2-9: Contents of components in the second culture medium

| Component | a-MEM (mL) | KOSR (%) | Ultroser G (%) | NEAA (mM) | GlutaMax (mM) | Ascorbic acid (ug/mL) | bFGF (ng/mL) | TGFβ (ng/mL) | VEGF (ng/mL) | EGF (ng/mL) | PDGF (ng/mL) |
|---|---|---|---|---|---|---|---|---|---|---|---|
| Formulation 1 | 46 | 5 | 1 | 0.1 | 2 | 10 | 10 | 4 | | | |
| Formulation 2 | 48.5 | 1 | 1 | 0.1 | 1 | 1 | 1 | 1 | | | |
| Formulation 3 | 12 | 30 | 30 | 0.5 | 5 | 1000 | 100 | 100 | | | |
| Formulation 4 | 46 | 5 | 1 | 0.1 | 2 | 10 | 1 | 1 | | | |
| Formulation 5 | 46 | 5 | 1 | 0.1 | 2 | 10 | 100 | 100 | | | |
| Formulation 6 | 46 | 5 | 1 | 0.1 | 2 | 10 | 10 | 4 | 1 | 1 | 1 |
| Formulation 7 | 46 | 5 | 1 | 0.1 | 2 | 10 | 10 | 4 | 100 | 100 | 100 |
| Formulation 8 | 46 | 5 | 1 | 0.1 | 2 | 10 | 10 | 4 | 10 | | |
| Formulation 9 | 46 | 5 | 1 | 0.1 | 2 | 10 | 10 | 4 | | 10 | |
| Formulation 10 | 46 | 5 | 1 | 0.1 | 2 | 10 | 10 | 4 | | | 10 |
| Formulation 11 | 46 | 5 | 1 | 0.1 | 2 | 10 | | | 100 | 100 | 100 |

2. Steps: The embryoid bodies were cultured in suspension with EB medium, and then the M cells were cultured using the above 11 formulations, and tested after passage to the 5$^{th}$ generation.

3. Morphological observation

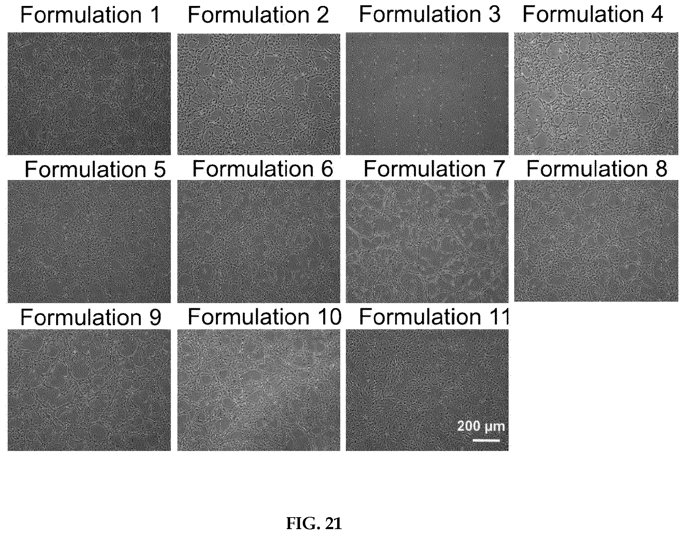

The cells in Formulation 3 (the highest concentration for all components) died and could not be cultured, and M cells of adherent growth and spindle shape could be obtained by the other formulations (FIG. 21).

4. Flow cytometry for positive and negative surface markers of M cells

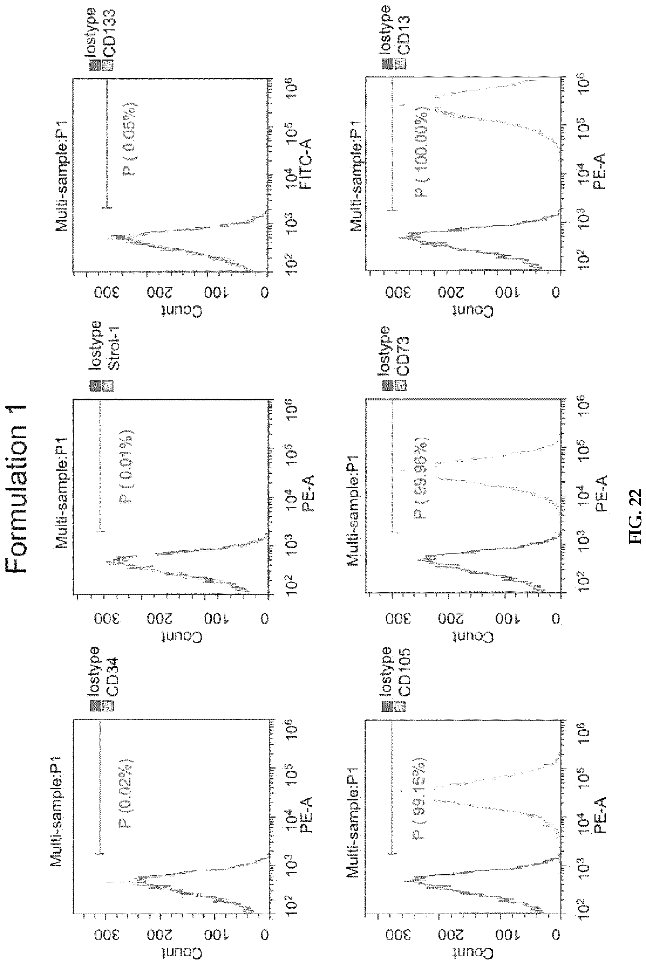

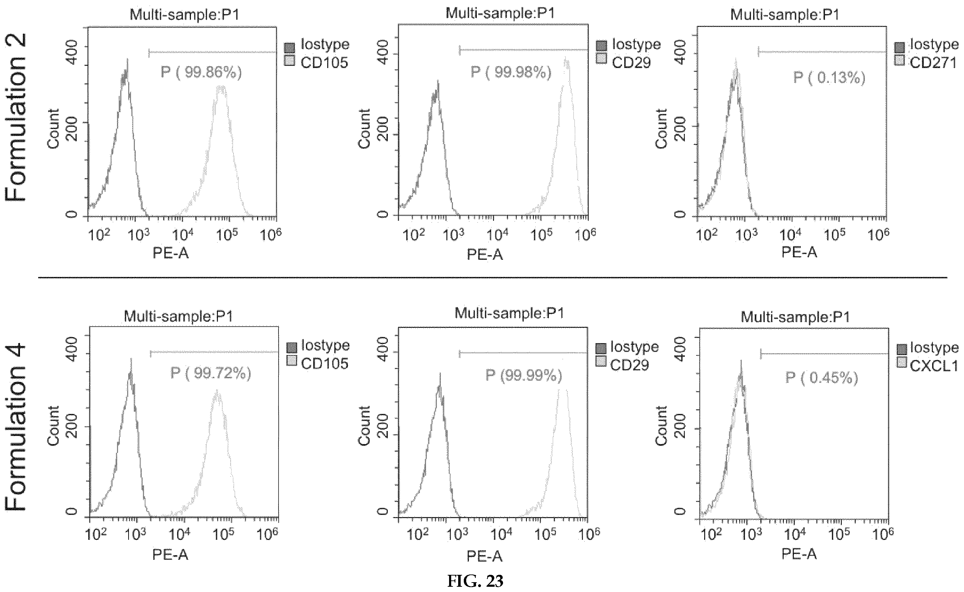

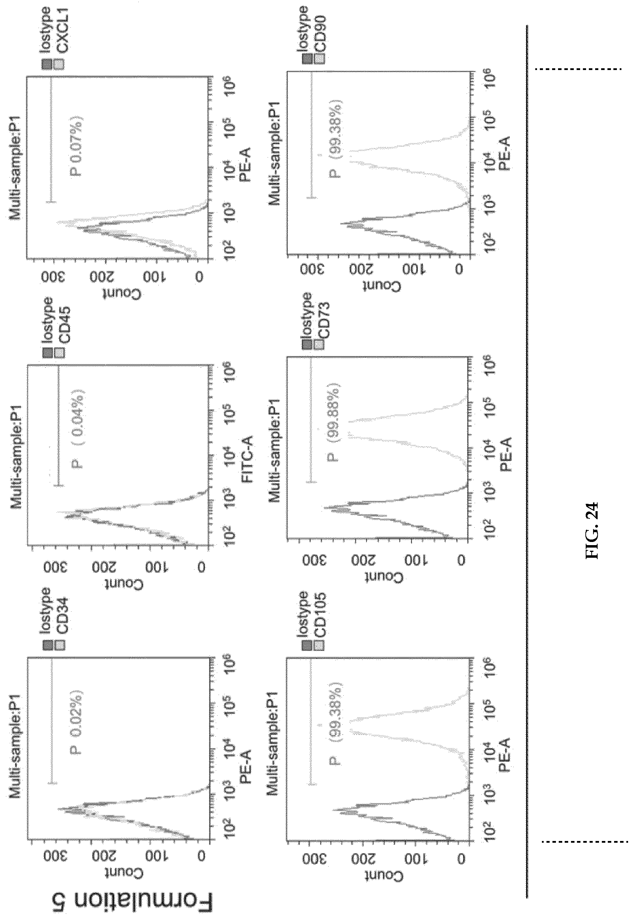

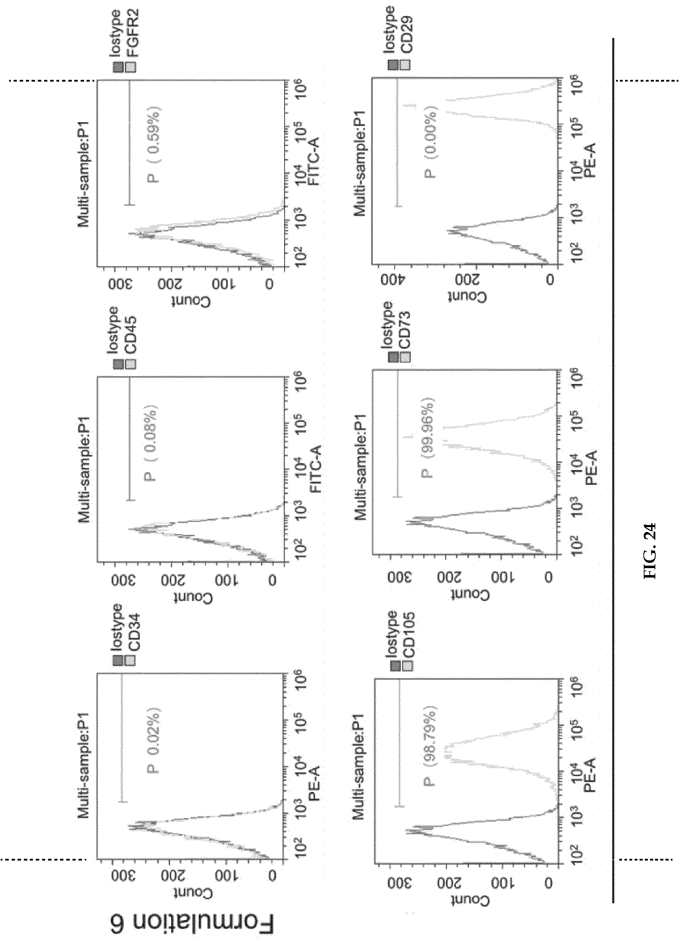

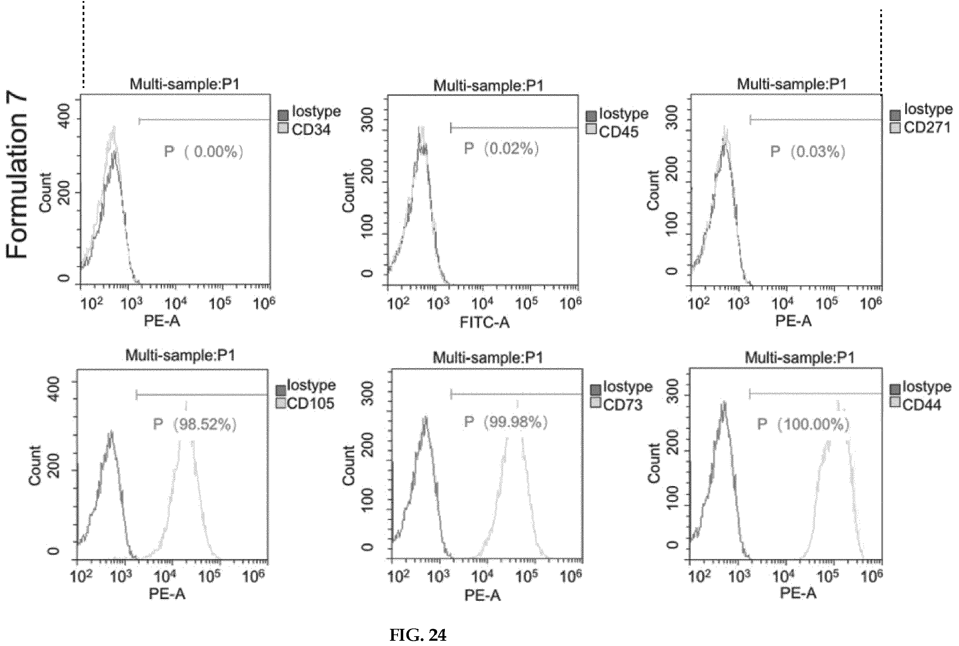

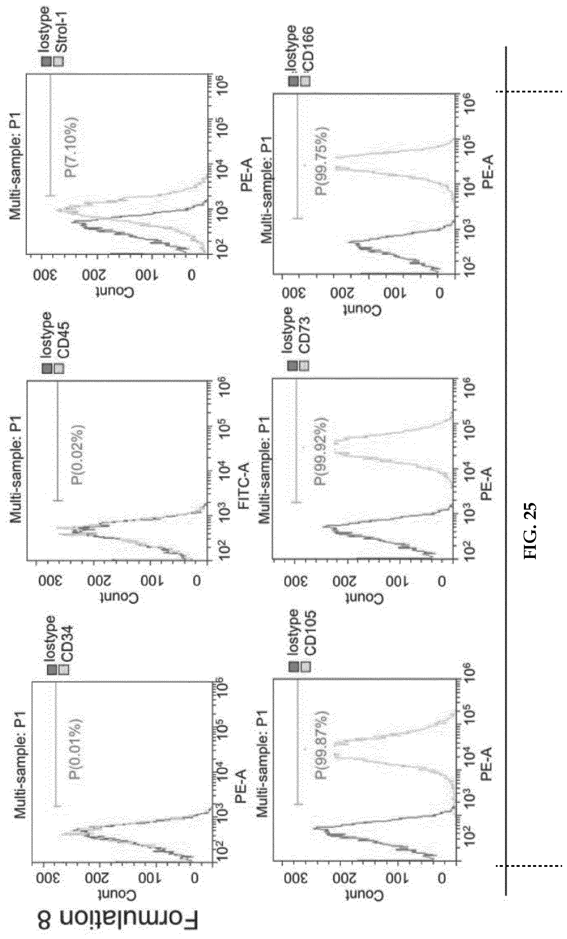

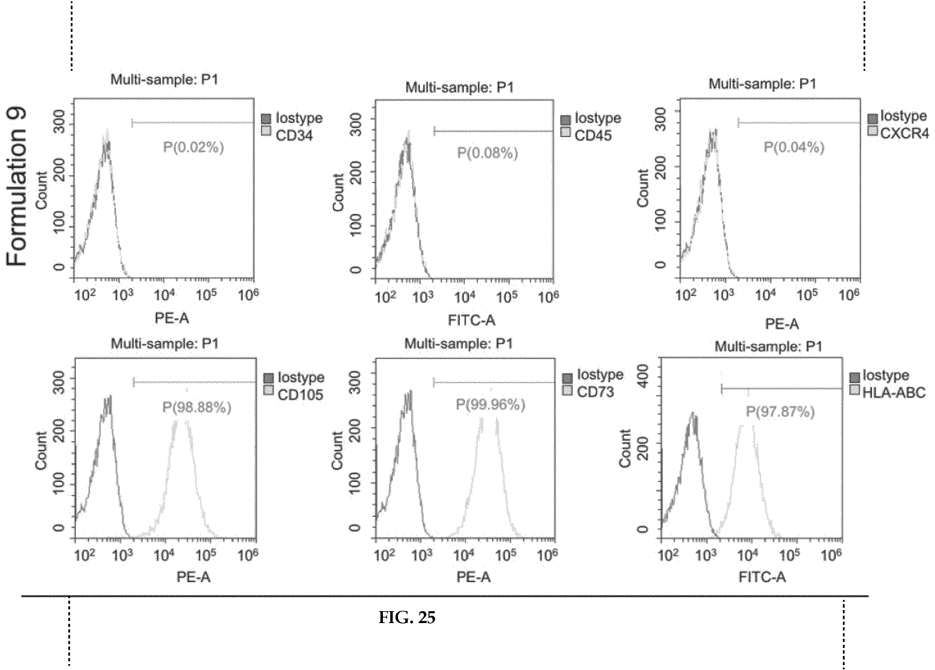

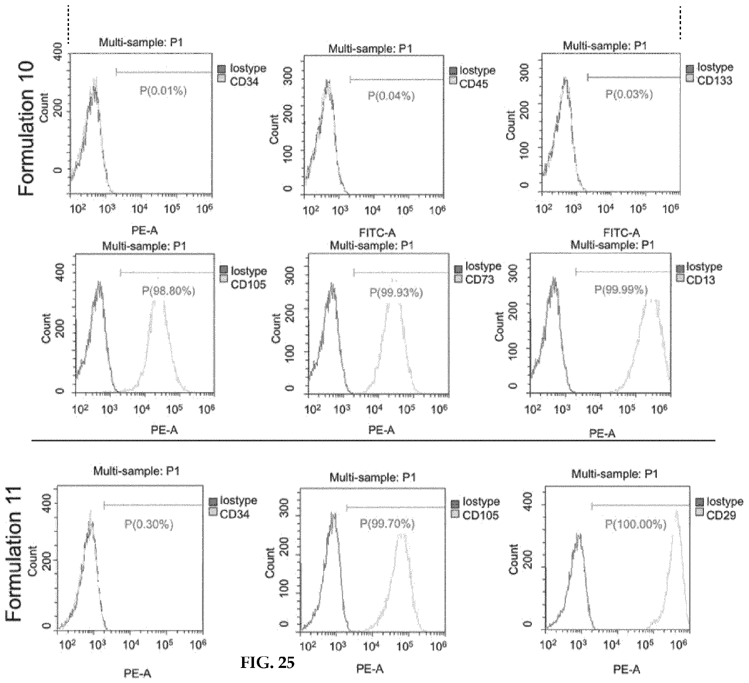

The flow cytometry results showed that the results of positive and negative surface markers of the detected M cells were basically similar to those of the M cells of Preparation Example 1 (Table 2-10, FIGS. 22 to 25).

Table 2-10: Summary of flow cytometry results for M cells

| | CD13 3 | FGF R2 | CD4 5 | CXCL 1 | CD3 4 | HLA-A BC | CD13 | CD7 3 | CD10 5 | CD29 | CD9 0 | CD44 | CD27 1 | CD16 6 | Strol-1 | CXCR 4 |
|---|---|---|---|---|---|---|---|---|---|---|---|---|---|---|---|---|
| Formulation 1 | 0.05 | | | | | | 100.00 | 99.96 | 99.15 | | | | | | 0.01 | |
| Formulation 2 | | | | | | | | | 99.86 | 99.98 | | | 0.13 | | | |
| Formulation 4 | | | | 0.45 | | | | | 99.72 | 99.99 | | | | | | |
| Formulation 5 | | | 0.05 | | 0.02 | | | 99.88 | 99.38 | | 99.38 | | | | | |
| Formulation 6 | | 0.59 | 0.08 | | 0.02 | | | 99.96 | 98.79 | 100.0 0 | | | | | | |
| Formulation 7 | | | 0.02 | | 0.00 | | | 99.98 | 98.52 | | | 100.0 0 | 0.03 | | | |
| Formulation 8 | | | 0.02 | | 0.01 | | | 99.92 | 98.87 | | | | | 99.75 | 7.10 | |
| Formulation 9 | | | 0.08 | | | 97.87 | | 99.96 | 98.88 | | | | | | | 0.04 |
| Formulation 10 | 0.03 | | 0.04 | | 0.01 | | 99.99 | 99.93 | 99.80 | | | | | | | |
| Formulation 11 | | | | | 0.30 | | | | 99.70 | 100.0 0 | | | | | | |

5. Expression of PGE2 and MMP1 detected by qPCR

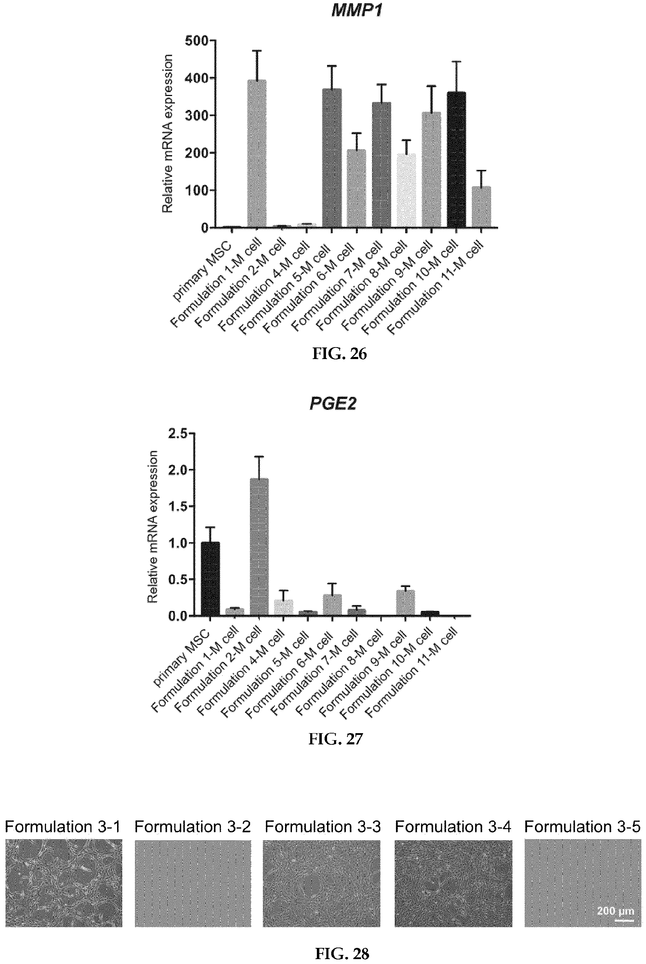

For MMP1, the expression levels of MMP1 in most formulations were more than 10 times that of the primary mesenchymal stem cells (Table 2-11, FIG. 26). The detection results of PGE2 were shown in Table 2-12 and FIG. 27.

Table 2-11: Summary of the qPCR results of MMP1 in M cells for different formulations of the second culture medium

| | Primary MSC | Formulation 1 | Formulation 2 | Formulation 3 | Formulation 4 | Formulation 5 | Formulation 7 | Formulation 8 | Formulation 9 | Formulation 10 | Formulation 11 |
|---|---|---|---|---|---|---|---|---|---|---|---|
| Relative mRNA expression level | 1.00 | 393.09 | 3.14 | 8.86 | 367.99 | 206.64 | 332.55 | 195.95 | 306.73 | 360.18 | 106.79 |
| | 1.03 | 371.35 | 3.21 | 8.04 | 324.74 | 118.39 | 321.42 | 180.40 | 432.75 | 318.99 | 108.93 |
| | 0.81 | 368.27 | 3.57 | 8.14 | 358.42 | 261.47 | 357.43 | 160.41 | 258.32 | 361.47 | 91.42 |

EP 4 047 083 A1

Table 2-12: Summary of qPCR results of PGE2 of M cells for different formulations of the second culture medium

| | Primary MSC | Formulation 1 | Formulation 2 | Formulation 3 | Formulation 4 | Formulation 5 | Formulation 7 | Formulation 8 | Formulation 9 | Formulation 10 | Formulation 11 |
|---|---|---|---|---|---|---|---|---|---|---|---|
| Relative mRNA expression level | 1.00 | 0.09 | 1.87 | 0.21 | 0.05 | 0.28 | 0.08 | 0 | 0.34 | 0.05 | 0 |
| | 1.03 | 0.03 | 0.21 | 0.08 | 0.26 | 0.18 | 0.12 | 0 | 0.33 | 0.09 | 0 |
| | 0.81 | 0.07 | 0.52 | 0.13 | 0.84 | 0.61 | 0.57 | 0 | 0.25 | 0.07 | 0 |

(4) Concentration ranges of the second culture medium

1. Setting the concentration of each component of the second culture medium according to the following table

Table 2-13: Contents of components in the second culture medium.

| Component | a-MEM (mL) | KOSR (%) | Ultroser G (%) | NEAA (mM) | GlutaMax (mM) | Ascorbic acid (ug/mL) | bFGF (ng/mL) | TGF β (ng/mL) |
|---|---|---|---|---|---|---|---|---|
| Formulation 3-1 | 33.5 | 30 | 1 | 0.1 | 2 | 10 | 10 | 4 |
| Formulation 3-2 | 31.5 | 5 | 30 | 0.1 | 2 | 10 | 10 | 4 |
| Formulation 3-3 | 44 | 5 | 1 | 0.5 | 2 | 10 | 10 | 4 |
| Formulation 3-4 | 45.25 | 5 | 1 | 0.1 | 5 | 10 | 10 | 4 |
| Formulation 3-5 | 41 | 5 | 1 | 0.1 | 2 | 1000 | 10 | 4 |

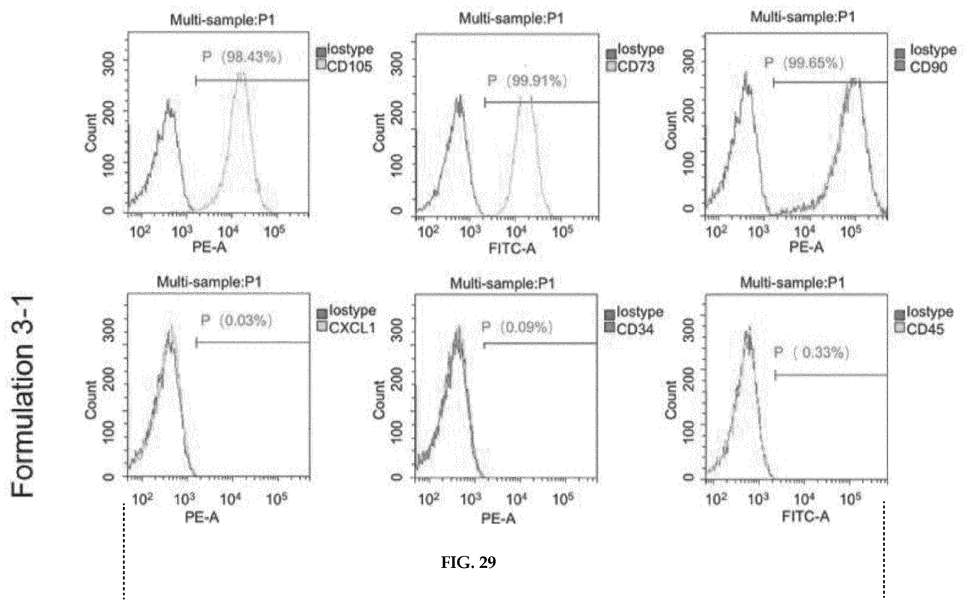

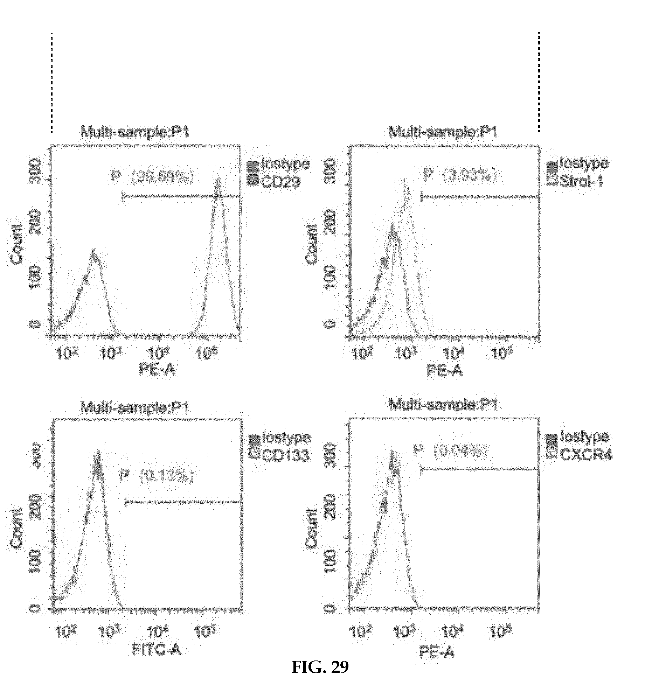

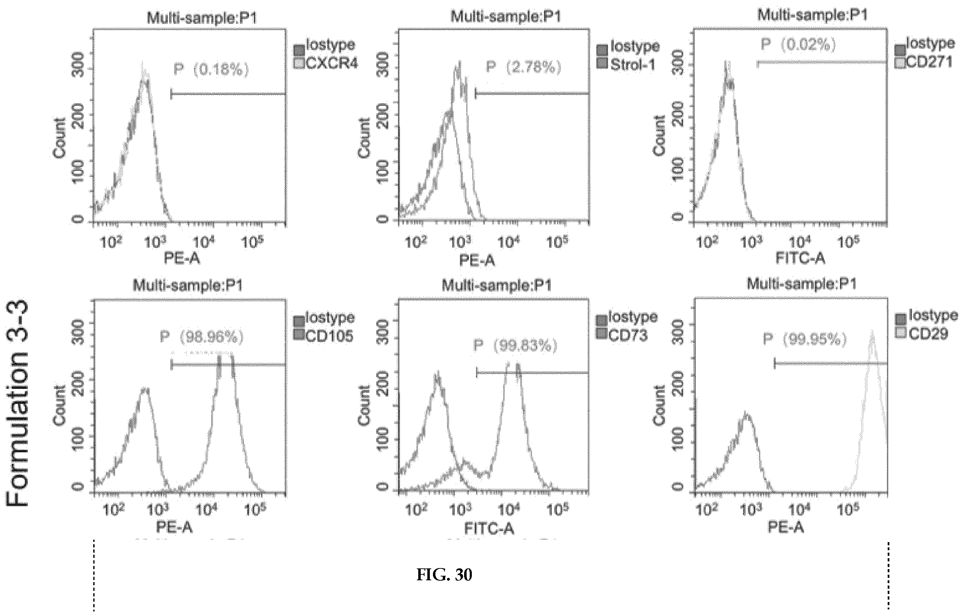

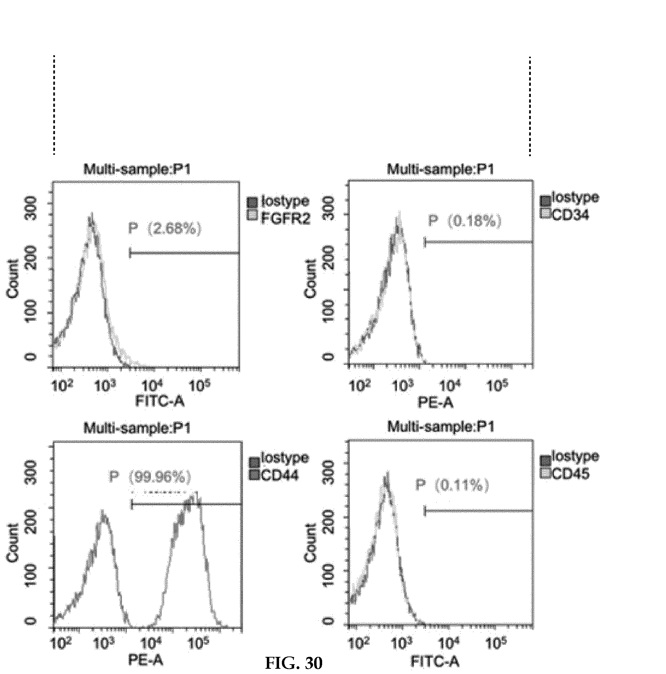

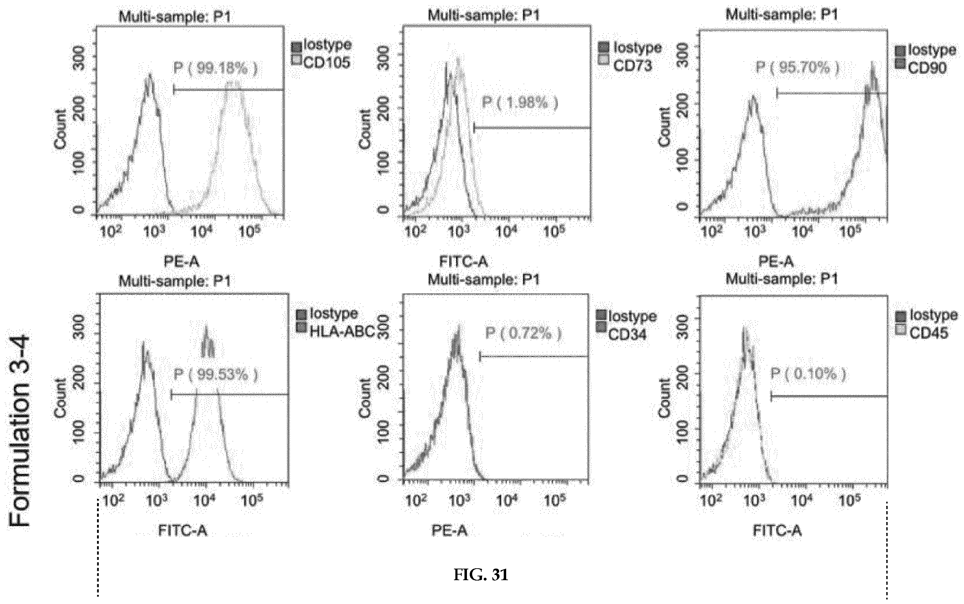

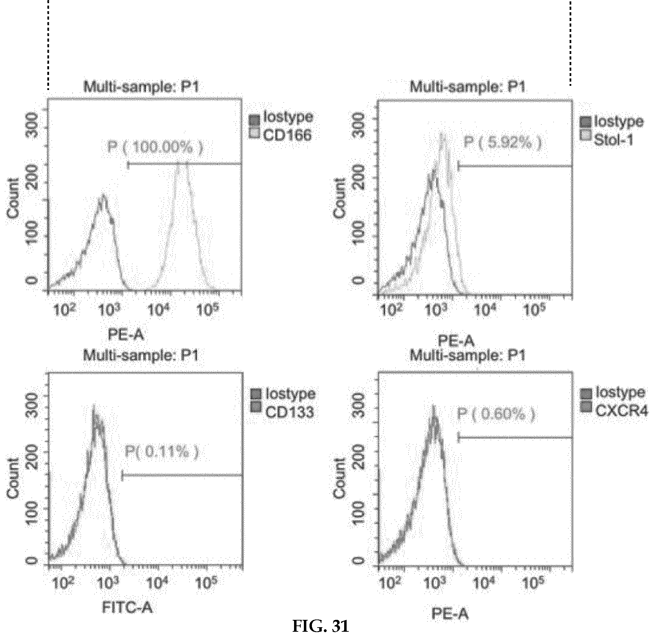

2. Steps: The embryoid bodies were cultured in suspension with EB medium, and then the M cells were cultured using the aforementioned five formulations, and detected after passage to the 5th generation.
3. Observation of cell morphology
For Formulations 3-2 (the highest concentration of Ultroser G) and 3-5 (the highest concentration of ascorbic acid), all cells were apoptotic, while for the other formulations, the M cells of adherent growth and spindle shape were obtained (FIG. 28).
4. Flow cytometry for positive and negative surface markers of M cells
The results of positive and negative surface markers of the detected M cells were mostly similar to those of the M cells of Preparation Example 1 (Table 2-14, FIGS. 29 to 31).

Table 2-14: Summary of flow cytometry results for M cells.

| | CD13 3 | FGFR 2 | CD4 5 | CXCL 1 | CD3 4 | HLA-AB C | CD1 3 | CD7 3 | CD10 5 | CD2 9 | CD9 0 | CD4 4 | CD27 1 | CD16 6 | Strol-1 | CXCR 4 |
|---|---|---|---|---|---|---|---|---|---|---|---|---|---|---|---|---|
| Formulation 3-1 | 0.13 | | 0.33 | 0.03 | 0.09 | | | 99.91 | 98.43 | 99.69 | 99.65 | | | | 3.93 | 0.04 |
| Formulation 3-3 | | 2.68 | 0.11 | | 0.18 | | | 99.83 | 98.96 | 99.95 | | 99.96 | 0.02 | | 2.78 | 0.18 |
| Formulation 3-4 | 0.11 | | 0.10 | | 0.72 | 99.53 | | 99.98 | 99.18 | | 95.70 | | | 100.00 | 5.92 | 0.60 |

5. Expression of PGE2 and MMP1 detected by qPCR

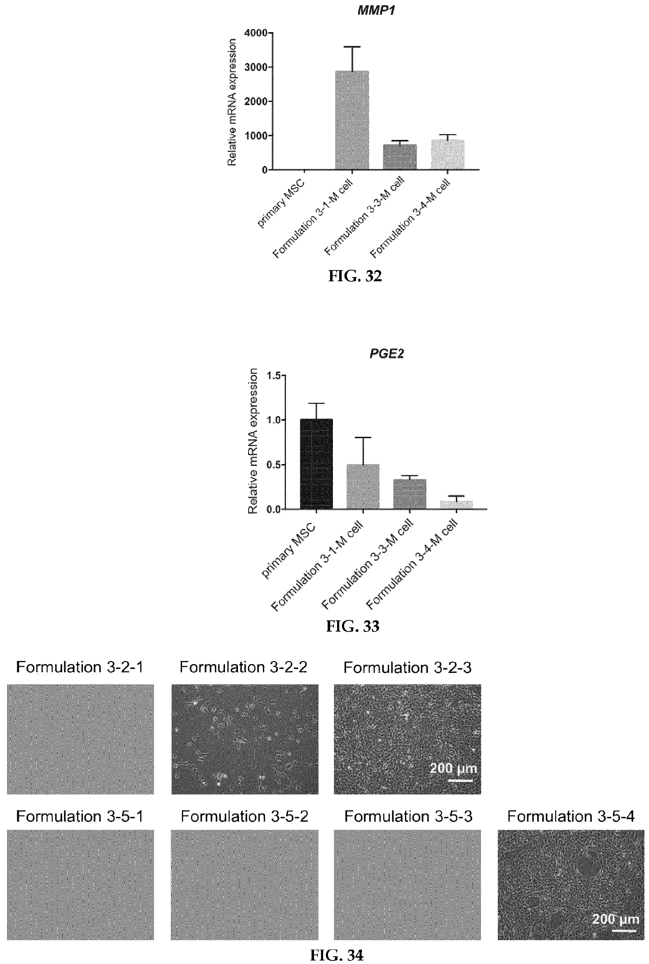

For MMP1, its levels for all formulations were more than 10 times higher than that of the primary mesenchymal stem cells (Table 2-15, FIG. 32). The results of PGE2 were shown in Table 2-16 and FIG. 33.

Table 2-15: Summary of qPCR results of MMP1 of M cells for different formulations of each culture medium

|  | Primary MSC | Formulation 3-1 | Formulation 3-3 | Formulation 3-4 |
|---|---|---|---|---|
| Relative mRNA expression | 1.00 | 2875.66 | 715.27 | 853.72 |
|  | 1.33 | 2371.52 | 721.25 | 804.25 |
|  | 0.61 | 3268.14 | 657.32 | 874.21 |

Table 2-16: Summary of qPCR results of PGE2 of M cells for different formulations of each culture medium

|  | Primary MSC | Formulation 3-1 | Formulation 3-3 | Formulation 3-4 |
|---|---|---|---|---|
|  | 1.00 | 0.50 | 0.33 | 0.09 |
| Relative mRNA expression | 1.01 | 0.35 | 0.22 | 0.04 |
|  | 0.71 | 0.72 | 0.45 | 0.15 |

(5) Concentration ranges of the second culture medium

1. Based on Formulations 3-2 and 3-5 in (4), the concentration gradients of Ultroser G and ascorbic acid were further set.

Table 2-17: Contents of components in the second culture medium

| Component | a-MEM (mL) | KOSR (%) | Ultroser G (%) | NEAA (mM) | GlutaMax (mM) | Ascorbic acid (ug/mL) | bFGF (ng/mL) | TGF $\beta$ (ng/mL) |
|---|---|---|---|---|---|---|---|---|
| Formulation 3-2-1 | 36.5 | 5 | 20 | 0.1 | 2 | 10 | 10 | 4 |
| Formulation 3-2-2 | 41.5 | 5 | 10 | 0.1 | 2 | 10 | 10 | 4 |
| Formulation 3-2-3 | 44 | 5 | 5 | 0.1 | 2 | 10 | 10 | 4 |
| Formulation 3-5-1 | 43.5 | 5 | 1 | 0.1 | 2 | 500 | 10 | 4 |
| Formulation 3-5-2 | 44.75 | 5 | 1 | 0.1 | 2 | 250 | 10 | 4 |
| Formulation 3-5-3 | 45.5 | 5 | 1 | 0.1 | 2 | 100 | 10 | 4 |
| Formulation 3-5-4 | 46 | 5 | 1 | 0.1 | 2 | 50 | 10 | 4 |

2. Steps: The embryoid bodies were cultured in suspension with EB medium, and then the MSC cells were cultured using the above 7 Formulations, and detected after passage to the 5th generation.

3. Observation of cell morphology

The cells of Formulations 3-2-1, 3-5-1, 3-5-2 and 3-5-3 were all apoptotic. A small number of cells of Formulation 3-2-2 survived, and the cells grew slowly. For all other formulations, the M cells of adherent growth and spindle shape were obtained (FIG. 34).

4. Flow cytometry for positive and negative surface markers of M cells

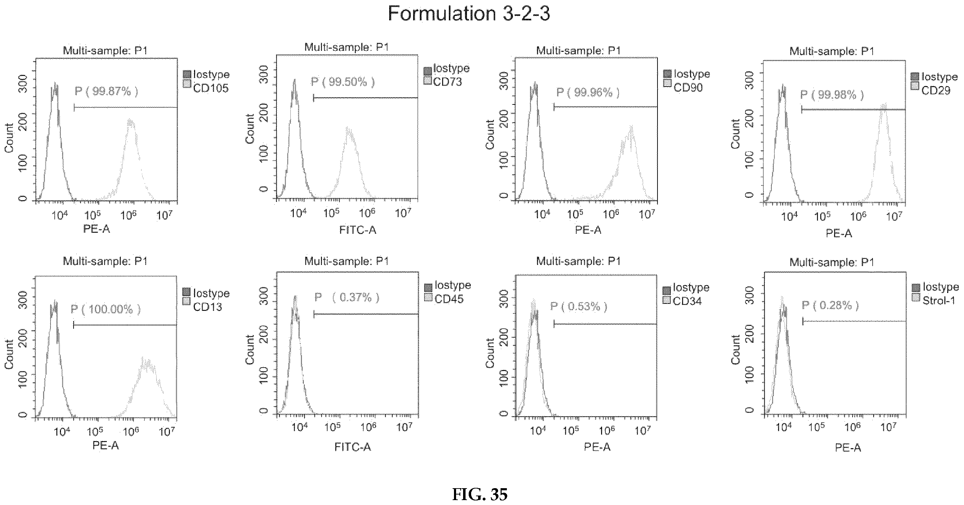

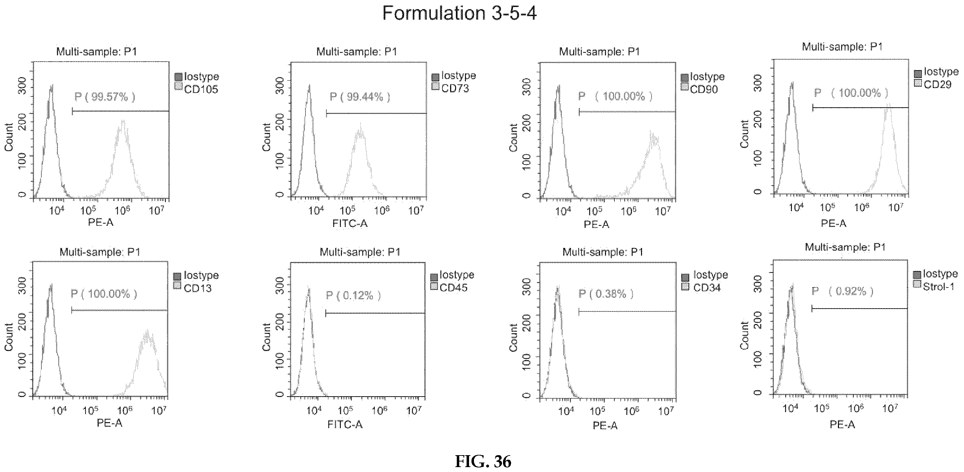

The flow cytometry results showed that the results of positive and negative surface markers of the detected M cells were similar to those of the M cells of Preparation Example 1 (Table 2-18, FIG. 35, FIG. 36).

Table 2-18: Summary of flow cytometry results for M cells.

|  | CD45 | CD34 | CD13 | CD73 | CD105 | CD29 | CD90 | Strol-1 |
|---|---|---|---|---|---|---|---|---|
| Formulation 3-2-3 | 0.37 | 0.53 | 100.00 | 99.50 | 99.87 | 99.98 | 99.96 | 0.28 |
| Formulation 3-5-4 | 0.12 | 0.38 | 100.00 | 99.44 | 99.57 | 100.00 | 100.00 | 0.92 |

5. Expression of PGE2 and MMP1 detected by qPCR

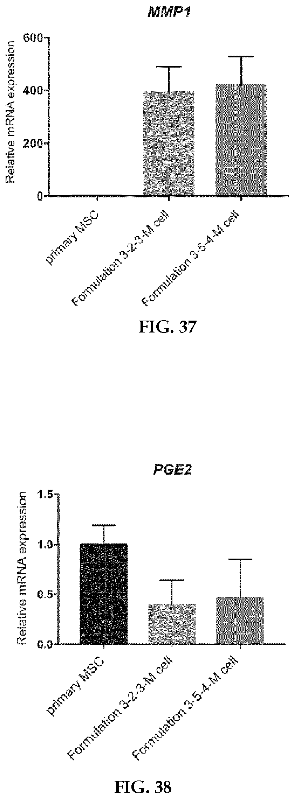

For MMP1, its levels for all formulations were more than 10 times higher than that of the primary mesenchymal stem cells (Table 2-19, FIG. 37). The results of PGE2 were shown in Table 2-20 and FIG. 38.

Table 2-19: Summary of qPCR results of MMP1 of M cells for different formulations of each medium

|  | Primary MSC | Formulation 3-2-3-M cells | Formulation 3-5-4-M cells |
|---|---|---|---|
| Relative mRNA expression | 1.00 | 393.05 | 420.83 |
|  | 1.53 | 315.55 | 452.13 |
|  | 0.53 | 448.24 | 403.72 |

Table 2-20: Summary of qPCR results of PGE2 of M cells for different formulations of each medium

|  | Primary MSC | Formulation 3-2-3-M cells | Formulation 3-5-4-M cells |
|---|---|---|---|
| Relative mRNA expression | 1.00 | 0.40 | 0.46 |
|  | 0.63 | 0.75 | 0.32 |
|  | 1.41 | 0.24 | 0.57 |

Unless otherwise specified, the M cells used in the following examples were prepared by the method in Preparation Example 1.

Example 3: Spray system for spraying M cells

Preparation and culture of M cells: The embryonic stem cells suspended as EB spheres were subjected to adherent differentiation so as to obtain P0 generation M cells, which were passaged and screened, and cryopreserved at P3 generation for subsequent experiments.

The M cells at P3 generation were resuscitated, digested and passaged, and P5 was used for subsequent experiments.

| Reagent/Equi pment | Manufacturer | Cat. No. |
|---|---|---|
| Biological safety cabinet | Thermo | 1389 A2 |
| 0.3mm spray pen, paint pen | Domestic | LEwpCB6tm1021 |
| Cell Counting kit-8 | Dogesce | CK04 |
| 10-100$\mu$L pipette | Eppendorf | M46287F |
| 96-well cell culture plate | Corning | CLS3599-100EA |

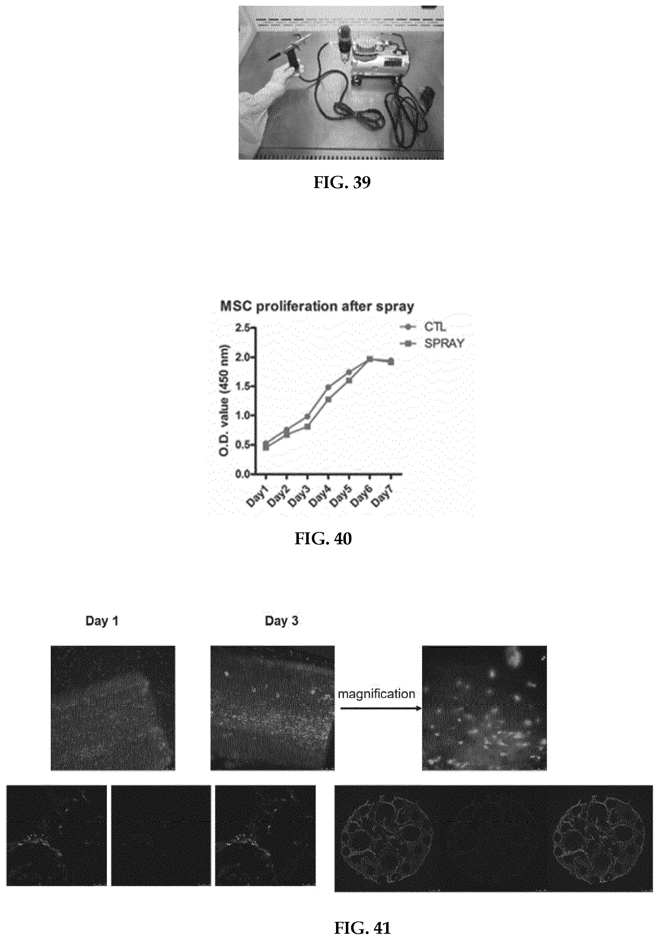

1. Spray method: The M cells of the P5 generation were resuspended in the M cell culture medium (second culture medium), adjusted to have a density to $8 \times 10^4$ cells/ml, sprayed with a spray pen, in which the pressure of pump was adjusted to <10 kPa, and the pore size was 0.8 mm, and the cells were sprayed onto a 10 cm dish. The apparatus for spraying M cells was shown in FIG. 39.

After spraying, the cells were inoculated in a 96-well culture plate, 8,000 cells per well. The M cells without spraying were inoculated in a 96-well plate at the same density as a control. The proliferation ability of M cells before and after spraying was detected by CCK8 kit every other day.

2. CCK8 detection method:

(1) The cell suspension was prepared and counted.

(2) The cell suspension was inoculated in a 96-well plate, about 100 $\mu$l per well, with three repeats for one sample.

(3) The culture plate was placed in an incubator to perform preculture for a period of time (37°C, 5% $CO_2$), and the cells adhered wall within 4 hours.

(4) To each well, 10 $\mu$l of CCK-8 solution was added, and it was tried to avoid the generation of air bubbles during the process of adding sample.

(5) The culture plate was placed in an incubator and incubated for 2 hours.

(6) The absorbance value (OD) at 450nm was measured with a microplate reader.

3. Detection of cell damage: LDH kit method (Biyuntian, C0017)

4. Flow cytometry detection of M cell surface proteins

(1) The culture supernatant was discarded, washing was performed once with PBS, Tryple was added to perform digestion for 3 min, and DPBS was added to stop the digestion.

(2) The cell suspension was collected and centrifuged at 1200 rpm for 3 min.

(3) The supernatant was discarded, the cells were resuspended in DPBS, filtered with a 40 $\mu$m cell sieve to remove the cell clusters, counted, and subpackaged, $2\times10^6$ cells per tube.

(4) Centrifugation was carried out at 1200 rpm for 3 min.

(5) After blocking with 2% BSA blocking solution for 20 min, centrifugation was carried out at 1200 rpm for 3 min.

(6) The supernatant was discarded, the cells were resuspended with 100 $\mu$L of 1% BSA antibody diluent, then added with directly labeled antibody, and incubated at room temperature for 30 to 45 minutes.

(7) Washing was performed three times with 1 mL of PBS, centrifugation was carried out at 1200 rpm for 3 min, and then the supernatant was discarded.

(8) After resuspending in 300 $\mu$L DPBS, the cells were filtered with a 40 $\mu$m cell sieve, then loaded for detection.

(9) The information of required antibodies was as follows:

| Name | Company | Cat. No. |
|---|---|---|
| CD105 | BioLegend | 323206 |
| CD73 | eBioscience | 11-0739-42 |
| CD90 | eBioscience | 12-0909-42 |
| CD13 | BD | 560998 |
| CD29 | BioLegend | 303004 |
| CD34 | BD | 555822 |
| CD45 | eBioscience | 11-9459-42 |
| Stro-1 | abcam | ab190282 |
| PE-IgG 1 Isotype Control | BD | 555749 |
| FITC-IgG 1 Isotype Control | BD | 555748 |

Information of the required instruments

| Reagent/Equi pment | Manufacturer | Cat. No. |
|---|---|---|
| $CO_2$ incubator | Thermo | 3131 |

(continued)

| Reagent/Equipment | Manufacturer | Cat. No. |
|---|---|---|
| Biological safety cabinet | Haier | HR40-IIA2 |
| Centrifuge | Xiangyi | TD25-WS |
| Cell counter | Life technologies | Countess II FL |
| Vacuum pump | KNF | N86KN.18 |
| 100 to 1000 μL pipette | eppendorf | J46045F |
| 20 to 200μL pipette | eppendorf | L22687F |
| 10 to 100μL pipette | eppendorf | M46287F |
| 0.5 to 10μL pipette | eppendorf | K19138F |
| 0.1 to 2.5μL pipette | eppendorf | L22220F |
| Flow cytometer | Beckman | Cyto FLEX |

5. Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 48-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex ManagerTM.

(4) 250 μL of standard dilution HB was added to the standard bottle, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1× with Bio-Plex detection buffer, and stored in the dark.

(7) The diluted magnetic beads were vortexed, and 50 μL of the magnetic beads was added to each well.

(8) The plate was washed twice with 100 μL of washing solution.

(9) The sample, standard, blank, and control of known concentration were vortexed, and added in an amount of 50 μL to each well.

(10) The plate was sealed with a sealing film, and shaken on a high-frequency shaker at 850±50 rpm at room temperature for 30 min.

(11) In step (10), when the remaining 10 min of shaking time was left, the detection antibody was vortexed for 5 s and diluted to 1×.

(12) The plate was washed twice with 100 μL of washing solution.

(13) The diluted antibody was vortexed, and added in an amount of 250 μL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1×.

(17) The plate was washed twice with 100 μL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 μL of detection solution, the plate was sealed with a sealing film, and shaken on a high-frequency shaker at room temperature at 850±50 rpm for 30 s.

(22) After the sealing film was discarded, loading to machine was started.
The multi-factor suspension chip system used above was Bio-Plex® 200 (Bio to Rad).

6. Detection of cell viability: (hemocytometer method) from T/CSCB 0002-2020 "Human Embryonic stem cells"

(1) Preparation of cell suspension
The cells to be tested were collected, formulated with phosphate buffer to obtain a cell suspension, and diluted to an appropriate concentration. The number of cells in each 1 mm$^2$ square should be 20 to 50 cells. If more than 200 cells, a dilution was required.

(2) Cell staining
A trypan blue staining solution was well mixed with the cell suspension at a volume ratio of 1:1.

(3) Cell counting

A coverslip was placed on the counting chamber of the hemocytometer, 10 μL of the mixture was taken and dropped on the edge of the coverslip on one side of the counting chamber, and another 10 μL of the mixture was taken and dropped on the edge of the coverslip on the other side of the counting chamber, so that the mixture was filled between the coverslip and the counting plate. After being allowed to stand for 30 s, the counting plate was placed under a microscope to count the stained cells and the total number of cells, respectively.
For the counting chamber of 16×25 size, 4 middle grids (i.e., 100 small grids) of 1mm$^2$ at the upper left, upper right, lower left and lower right according to the diagonal position were taken for counting. For the counting chamber of 25×16 size, 5 middle grids (i.e., 80 small grids) at the upper left, upper right, lower left, lower right and center according to the diagonal position were taken for counting. When encountering the cells on large grid line, generally only the cells on the upper and left lines of the large grid were counted (or only the cells on the lower and right lines were counted).

(4) Calculation and analysis
Cell viability was calculated according to Formula (I):

$$S = (M\text{-}D)/M \times 100\% \qquad (I)$$

In Formula (I):

S: cell viability

M: total number of cells

D: number of stained cells

The cell viability was an average of 2 samples. The average of the viable cell ratios of two counts was calculated and recorded as the average cell viability. The microscope used was DMi1 (Leica).

By detecting the content of intracellular LDH, the degree of cell damage was judged. It was found that there was no difference in the degree of cell damage between the sprayed cells and the non-sprayed cells. The expression of marker proteins of the sprayed cells and the non-sprayed cells was detected by flow cytometry, and it was found that there was no difference in M cell-specific markers, such as CD73, CD105, and CD29. The secretion levels of immunoregulatory factors of the sprayed cells and the non-sprayed cells were detected, and the results showed that there was no difference in IDO and IL1RA, etc. There was no difference in the viability between the sprayed cells and non-sprayed cells. The cells had normal morphology after spraying, and their proliferation ability was not significantly different from that of the non-sprayed cells. FIG. 40 shows the results of proliferation ability of M cells detected by CCK8 method before and after spraying.

Example 4: M cell friendly culture material

By the method of Preparation Example 1, the EB spheres were obtained by using GFP-labeled embryonic stem cells as the starting material, and the GFP-labeled M cells of P0 generation were further obtained by adherent differentiation, which were passaged and screened, and cryopreserved at P3 generation for subsequent experiments.

The GFP-labeled M cells of P3 generation were resuscitated, digested and passaged, and those of P5 were used for subsequent experiments.

| Reagent/Equi pment | Manufacturer | Cat. No. |
|---|---|---|
| Biological safety cabinet | Thermo | 1389 A2 |
| Inverted fluorescence microscope | Leica | DMI3000B |
| High-strength cross-linked collagen scaffold | Xiamen Ningfu Biotechnology Co., Ltd. | None |
| Haifu® Skin repair membrane | handong Zhenghai | None |
| Electrospun gelatin fibers | Institute of Physics and Chemistry, Chinese Academy of Sciences | None |
| Aminated gelatin | Institute of Zoology, Chinese Academy of Sciences | None |
| Collagen scaffold | Institute of Zoology, Chinese Academy of Sciences | None |
| 24-well cell culture plate | Corning | 3524 |
| 10$\mu$l pipette tip | Axygen | YC-HC01019 |
| 1ml pipette tip | Axygen | TF-1000-R-S |
| 0.5-10$\mu$L | Eppendorf | 1449888 |
| 1000$\mu$L pipette | Eppendorf | J46096F |
| Gelatin | Sigma | G1890 |

The density of GFP-labeled M cells was adjusted to form a high-density cell suspension, which was added dropwise to the material, and the culture medium was changed after 24 hours, and then the cell growth was observed under a fluoroscope.

1. Live/dead kit was used for detection.

2. CCK8 detection method was adopted, and the method was as described in Example 3.

3. Flow cytometry was used for the detection of M cell surface proteins, and the method was as described in Example 3.

4. The suspension chip system was used to detect inflammatory factors, and the method was as described in Example 3.

5. The cell viability was detected, and the method was as described in Example 3.

6. Scanning electron microscope: 1) the cells was fixed with glutaraldehyde, the supernatant was discarded, washing was performed 3 times by adding PBS, for 6 min, 7 min, and 8 min each time; 2) 50% ethanol was added for soaking for 14 min; 3) 85% ethanol was added for soaking for 14 min; 4) 95% ethanol was added for soaking for 15min; 5) 100% ethanol was added for soaking for 15min; 6) critical point drying was performed; 7) the sample was glued to a metal platform and subjected to gold spraying treatment; 8) observation was carried out by a scanning electron microscope.

7. Experimental results:

The ring-shaped collagen scaffolds were cut into 5mm long pieces of material, placed in a 24-well plate, and a high-density cell suspension (50 $\mu$l containing $2\times10^6$ GFP-labeled M cells) was added dropwise on the material; after culturing at 37°C in an incubator for 1 hour, 1 ml of GFP-labeled M cell culture medium was added to each well. After that, the culture medium was changed every day, and the observation under fluoroscopy was carried out on the 1st and 3rd days. The fluorescent photos were shown in FIG. 41. It can be seen from the fluorescent photos that the GFP-labeled M cells were well attached to and grown on the collagen material. The ring-shaped collagen scaffolds could be used in the subsequent spinal cord injury transplantation experiments.

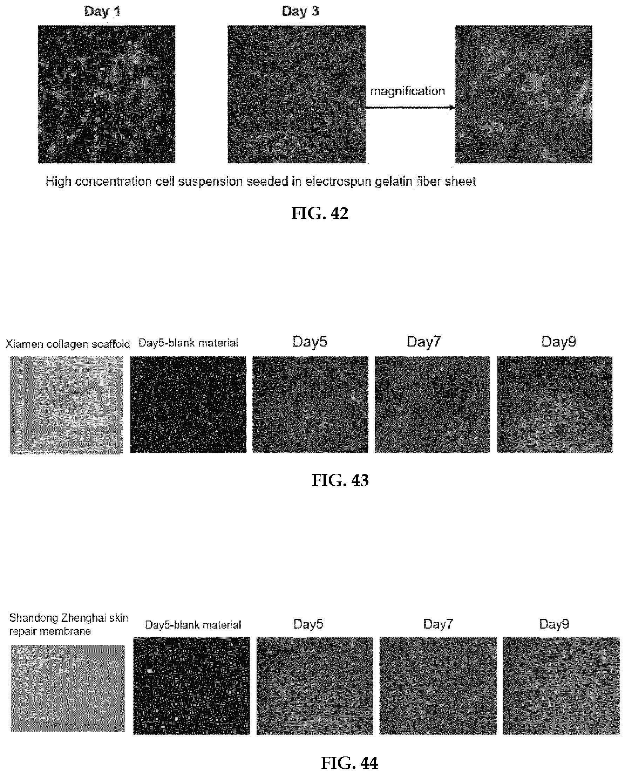

The electrospun gelatin fibers were cut into 5mm×5mm material, placed in a 24-well plate, a high-density cell suspension (50 $\mu$l containing $2\times10^6$ GFP-labeled M cells) was added dropwise on the material; after culturing at 37°C in an incubator for 1 hour, 1 ml of GFP-labeled M cell culture medium was added to each well. After that, the culture medium was changed every day, and the observation under fluoroscopy was carried out on the 1st and 3rd days. The fluorescent photos were shown in FIG. 42. It can be seen from the fluorescent photos that the GFP-labeled M cells were well attached to and grown on the electrospun gelatin fibers. The cell-loaded electrospun gelatin fibers could be used in the subsequent transplantation treatment experiments on skin, cornea, mucosa, etc.

The collagen scaffolds were cut into 5mm×5mm materials, placed in a 24-well plate, and a high-density cell suspension (50 $\mu$l containing $2\times10^6$ GFP-labeled M cells) was added dropwise on the material; after culturing at 37°C in an incubator for 1 hour, 1 ml of GFP-labeled M cell culture medium was added to each well. After that, the culture medium was changed every day, and the observation under fluoroscopy was carried out on the 5th, 7th, and 9th days. The fluorescent photos were shown in FIG. 43. It can be seen from the fluorescent photos that the GFP-labeled M cells were well attached to and grown on the collagen scaffolds. The cell-loaded collagen scaffolds could be used in the subsequent transplantation treatment experiments of skin, cornea, mucosa, etc.

The skin repair membrane was cut into 5mm×5mm material, and placed in a 24-well plate, a high-density cell suspension (50 $\mu$l containing $2\times10^6$ GFP-labeled M cells) was added dropwise on the material; after culturing at 37°C in an incubator for 1 hour, 1 ml of GFP-labeled M cell culture medium was added to each well. After that, the culture medium was changed every day, and the observation under fluoroscopy was carried out on the 5th, 7th, and 9th days. The fluorescent photos were shown in FIG. 44. It can be seen from the fluorescent photos that the GFP-labeled M cells were well attached to and grown on the skin repair membrane. The cell-loaded skin repair membrane could be used in the subsequent transplantation treatment experiments of skin, cornea, mucosa, etc.

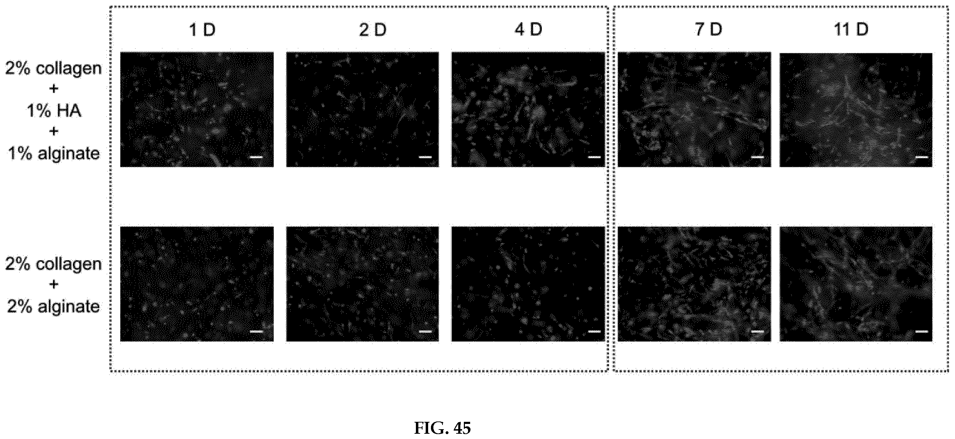

Two mixed gels were prepared according to 2% collagen+1% hyaluronic acid+1% sodium alginate, and 2% collagen+2% sodium alginate, respectively, placed in a 24-well plate, and a high-density cell suspension (50 $\mu$l containing $2\times10^6$ GFP-labeled M cells) as added dropwise on these materials; after culturing at 37°C in an incubator for 1 hour, 1 ml of GFP-labeled M cell culture medium was added to each well. After that, the culture medium was changed every day, and the observation under fluoroscopy was carried out on the 5th, 7th, and 9th days. The fluorescent photos were shown in FIG. 45. It can be seen from the fluorescent photos that the GFP-labeled M cells were well attached to and grown on the skin repair membrane. The skin repair membrane loaded with cells could be used in the subsequent transplantation treatment experiments of skin, cornea, mucosa, etc. When the M cells were inoculated on gelatin, they could grow and proliferate normally, have normal morphology, express normal levels of specific marker proteins, and secrete normal levels of immunoregulatory factors.

Chitosan: The proliferation of M cells on chitosan scaffolds was detected by the CCK8 kit method, and the results showed that the M cells could grow and proliferate normally on chitosan.

The M cells were inoculated in the mixed collagen-chitosan scaffold, the proliferation of the cells on the scaffold was detected by the CCK8 kit method, and the growth and attachment of the cells on the scaffold were observed by scanning electron microscope. The results showed that the M cells could attach, grow and proliferate normally.

The M cells were cultured in the polymer ion complex formed by chitosan and collagen or alginic acid, and it was found that the M cells could grow in the complex, and the complex did not degrade and shrink during the cell culturing process. This result showed that it could be an ideal scaffold for tissue engineering.

Sodium alginate: The growth state of M cells in alginic acid-hydrogel microspheres was investigated in detail through the activity detection by the CCK8 kit and the detection of cell viability. The results showed that the M cells could grow on sodium alginate, the adherent cells were of fusiform and fibrous morphology, and had the ability to differentiate into bone, fat and cartilage.

Sodium alginate-chitosan: The M cells could grow on this composite material. After the assay of cell viability, it was found that the cells had a high viability and normal shape.

Silk fibroin: The effect of silk fibroin on the growth of M cells was observed by viable cell counting method. The results showed that the M cells could grow normally on silk fibroin, showing a normal growth and proliferation curve.

Cellulose polylactic acid: The M cells were compounded on a cellulose polylactic acid scaffold, and the cell growth was observed by scanning electron microscopy and fluorescence electron microscopy. The results showed that the M cells could normally grow on the material, and the combination of M cells with this material was expected to be used as a scaffold material for biological tissue engineering.

Tropoelastin: The M cells were inoculated on tropoelastin, and observed by scanning electron microscope and fluorescence electron microscope, it was found that the M cells could grow on tropoelastin.

Hyaluronic acid: The M cells were inoculated on a hyaluronic acid material, and observed by scanning electron microscope and fluorescence electron microscope, it was found that the M cells could grow normally on the hyaluronic acid material.

The above results showed that the collagen scaffold, skin repair membrane, aminated gelatin, chitosan and other materials could carry the M cells to grow, and could make the M cells differentiate well.

Example 5: Preparation of ready-to-use injection of human embryonic stem cell-derived M cells

This example provided several preparation methods for effectively preserving human embryonic stem cell-derived M cell injections, which were simple to operate and could effectively preserve the M cells.

Experimental process and methods:

The M cells were cultured to the P5 generation and harvested, and after the cells were washed with DPBS, they were resuspended with normal saline, 5% glucose injection, sodium lactated Ringer's injection, compound electrolyte injection, 20% HSA injection, and succinyl gelatin injection, respectively, and the cells had a cell density of 3 to 6 $\times 10^6$ cells/ml; after sampling and detection of cell viability, each of the resuspended cell suspensions was divided into 2 tubes, that were stored at room temperature and 4°C, respectively, and subjected to viability detection regularly, so as to determine the cell preservation effect.

Reagents, consumables and instruments used were as follows:

| Reagents | | | |
|---|---|---|---|
| Name | Manufacturer | Cat. No. | |
| 20% Human Serum Albumin | Shandong Taibang Biological Products Co., Ltd. | SFDA Approval No. S10970005 | |
| Sodium lactate Ringer's injection | Shijiazhuang No. 4 Pharmaceutical Co., Ltd. | SFDA Approval No. H20044961 | |
| 5% Glucose injection | China Resources Shuanghe Pharmaceutical Co., Ltd. | SFDA Approval No. H11020627 | |
| Compound electrolyte solution | Shanghai Baite | A6E2543 | |
| NaCl injection | Shijiazhuang No. 4 Pharmaceutical Co., Ltd. | SFDA Approval No. H13023200 | |
| Trypan blue | Gibco | 15250-061 | |
| Consumables | | | |
| Name | Manufacturer | Cat. No. | |
| Disposable dispensing syringe | Jiangsu Zhiyu Medical Equipment Co., Ltd. | CFDSM Approval No. 20173150338 | |
| 15ml Centrifuge tube | CORING | 430791 | |
| Blue tip | Axygen | T-1000-B-R-S | |

(continued)

| Reagents | | | |
|---|---|---|---|
| Name | Manufacturer | Cat. No. | |
| White tip | Axygen | T-300-R-S | |
| Yellow tip | Axygen | T-200-Y-R-S | |
| 2ml Cryovial | CORING | 430659 | |
| Instrument | | | |
| Name | Manufacturer | No. | Model |
| Biological safety cabinet | Thermo | 09RS1200 | 1300 series A2 |
| Cell counter | Countess | 15RS0304 | Countess II FL |
| 4°C Refrigerator | Haier | 08AS1021 | RCD-256 KDC |
| 100-1000$\mu$L pipette | eppendorf | 18RS1217 | J26825H |
| 20-200$\mu$L pipette | eppendorf | 18RS1218 | I16892H |
| 10-100$\mu$L pipette | eppendorf | 18RS1219 | R21337G |
| 2-20$\mu$L pipette | eppendorf | 18RS1220 | J51636H |
| 0.5-10$\mu$L pipette | eppendorf | 18RS1221 | H30366 H |

(I) Cells resuspended in normal saline

1. Experimental steps:

(1) The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of normal saline was taken to perform resuspension;

(4) The cell suspension solution was divided equally into two tubes, about 1.5ml/tube, one of them was kept at room temperature (RT), and the other was stored at 4°C. Samples of 50ul to 100ul of cell suspension were taken before the storage, stained with trypan blue, and subjected to detection of cell density and viability;

(5) The sampling and detection were performed at different time points in sequence: 3h, 5h, 24h, 48h.

2. Experimental results:

Table 5-1: Viability detection results of cells resuspended in normal saline

| Detection time | Normal saline | | | |
|---|---|---|---|---|
| | RT | | 4°C | |
| 0h | 4.45 | 95.0 | 4.45 | 95.0 |
| 3h | 4.12 | 88.6 | 3.66 | 85.0 |

(continued)

| Detection time | Normal saline | | | |
|---|---|---|---|---|
| | RT | | 4°C | |
| 5h | 4.08 | 87.3 | 3.33 | 82.3 |
| 24h | 3.26 | 80.0 | 1.86 | 53.0 |
| 48h | 0.06 | 4.0 | 0.90 | 29.0 |

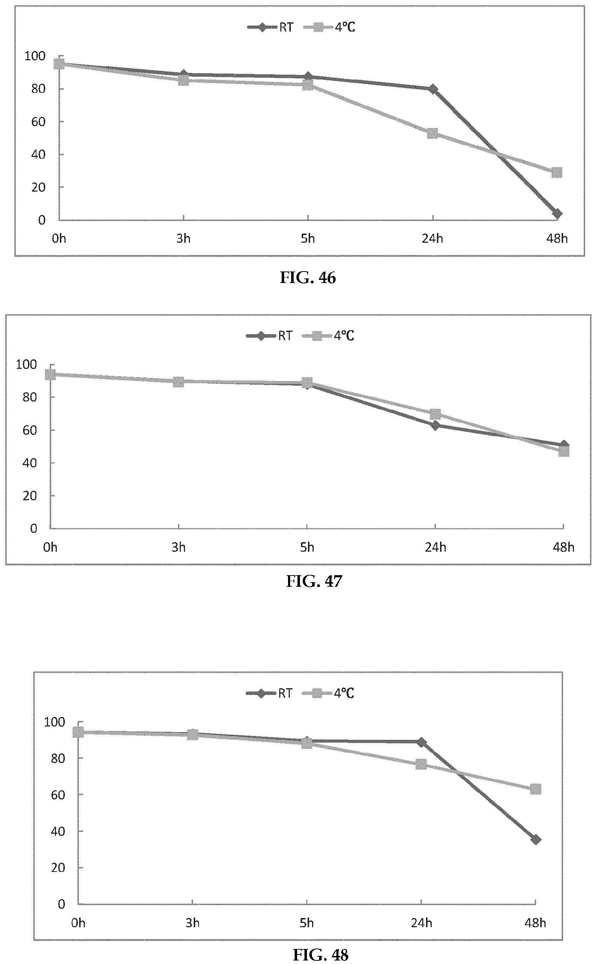

The detection results of cell viability were shown in FIG. 46. After the M cell pellet was resuspended in normal saline, the viability of M cells was maintained above 80% within 24 hours at RT; at 4°C, the viability of M cells was maintained above 80% within 5 hours; Compared with 4°C storage conditions, the cell viability was better under RT storage conditions.

(II) Cells resuspended in sodium lactated Ringer's injection solution:

1. Experimental steps:

(1) The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of sodium lactate Ringer's injection was taken to perform resuspension;

(4) The cell suspension was divided equally into two tubes, about 1.5ml/tube, one of them was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 3h, 5h, 24h, 48h.

2. Experimental results:

Table 5-2: Viability detection results of cells resuspended in sodium lactate Ringer's injection solution

| Detection time | Cells resuspended in sodium lactate Ringer's injection solution | | | |
|---|---|---|---|---|
| | RT | | 4°C | |
| 0h | 5.11 | 94.0 | 5.11 | 94.0 |
| 3h | 4.01 | 89.8 | 3.77 | 89.5 |
| 5h | 3.68 | 88.0 | 4.22 | 89.0 |
| 24h | 2.05 | 63.0 | 3.19 | 70.0 |
| 48h | 1.85 | 51.0 | 1.64 | 47.0 |

The detection results of cell viability were shown in FIG. 47. After the M cell pellet was resuspended in sodium lactate Ringer's injection, the viability of M cells was maintained above 85% within 5 hours at RT and 4°C; that was, there was no obvious difference in advantages or disadvantages between RT and 4°C storage conditions.

(III) Cells resuspended in compound electrolyte injection solution

1. Experimental steps:

(1) The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of compound electrolyte injection solution was taken to perform resuspension;

(4) The cell suspension solution was divided equally into two tubes, about 1.5ml/tube, one of them was kept at room temperature (RT), and the other was stored at 4°C. Samples of 50ul to 100ul of cell suspension were taken before the storage, stained with trypan blue, and subjected to detection of cell density and viability;

(5) The sampling and detection were performed at different time points in sequence: 3h, 5h, 24h, 48h.

2. Experimental results:

Table 5-3: Viability detection results of cells resuspended in compound electrolyte injection solution

| Detection time | Cells resuspended in compound electrolyte injection solution | | | |
|---|---|---|---|---|
| | RT | | 4°C | |
| 0h | 3.70 | 94.3 | 3.70 | 94.3 |
| 3h | 4.37 | 93.3 | 3.93 | 92.8 |
| 5h | 3.80 | 89.5 | 3.78 | 88.0 |
| 24h | 3.93 | 89.0 | 2.71 | 76.7 |
| 48h | 0.84 | 35.7 | 2.61 | 63.0 |

The cell viability detection results were shown in FIG. 48. After the M cell pellet was resuspended in compound electrolyte injection solution, the viability of M cells was maintained above 85% within 5 hours at RT and 4°C; while under RT storage conditions, the cell viability could reach above 85%; as compared with the 4°C condition, the RT condition showed better preservation effect for M cells.

(IV) Cells resuspended in 5% glucose injection solution

1. Experimental steps:

(1) The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of 5% glucose injection solution was taken to perform resuspension;

(4) The cell suspension was divided equally into two tubes, about 1.5ml/tube, one of them was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 3h, 5h, 24h, 48h.

2. Experimental results:

Table 5-4: Viability detection results of cells resuspended in 5% glucose injection solution

| Detection time | Cells resuspended in 5% glucose injection solution | | | |
|---|---|---|---|---|
| | RT | | 4°C | |
| 0h | 1.65 | 44.0 | 1.65 | 44.0 |
| 3h | 1.22 | 59.3 | 1.06 | 32.7 |
| 5h | 1.19 | 70.8 | 0.95 | 32.3 |
| 24h | 2.21 | 19.3 | 5.98 | 46.3 |
| 48h | 0.09 | 12.0 | 0.63 | 63.5 |

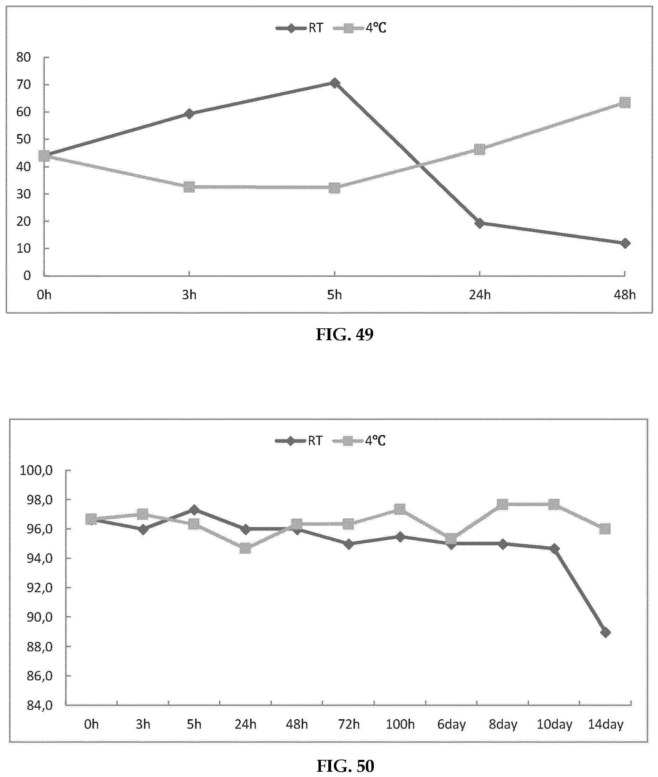

The cell viability detection results were shown in FIG. 49. After the M cells were resuspended with 5% glucose injection, the sampling and cell viability detection were immediately carried out, and it was found that the cell viability dropped sharply. Therefore, it may be necessary to avoid direct use alone in subsequent use.

(V) Cells resuspended in 20% HSA cell injection solution

1. Experimental steps:

(1) The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of 20% HSA injection solution was taken to perform resuspension;

(4) The cell suspension was divided equally into two tubes, about 1.5ml/tube, one of them was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 3h, 5h, 24h, 48h, 72h, 100h, 6day, 8day, 10day, 14day.

2. Experimental results:

Table 5-5: Viability detection results of cells resuspended in 20% HSA injection solution

| Detection time | Cells resuspended in 20%HSA injection solution | | | |
|---|---|---|---|---|
| | RT | | 4°C | |
| 0h | 3.12 | 96.7 | 3.12 | 96.7 |
| 3h | 3.31 | 96.0 | 3.21 | 97.0 |
| 5h | 3.86 | 97.3 | 3.88 | 96.3 |
| 24h | 4.74 | 96.0 | 4.86 | 94.7 |
| 48h | 4.69 | 96.0 | 5.13 | 96.3 |

(continued)

| Detection time | Cells resuspended in 20%HSA injection solution | | | |
|---|---|---|---|---|
| | RT | | 4°C | |
| 72h | 4.82 | 95.0 | 4.65 | 96.3 |
| 100h | 4.79 | 95.5 | 4.98 | 97.3 |
| 6day | 4.89 | 95.0 | 5.29 | 95.3 |
| 8day | 5.00 | 95.0 | 4.86 | 97.7 |
| 10day | 5.29 | 94.7 | 4.78 | 97.7 |
| 14day | 5.05 | 89.0 | 4.74 | 96.0 |

The cell viability detection results were shown in FIG. 50. After the M cell pellet was resuspended with 20% HSA injection solution, and stored at RT and 4°C for 14 days, respectively, the cell viability could reach above 85%; while the preservation effect on the M cells under 4°C conditions was much better, and the viability of M cells was kept above 90% within 14 days.

(VI) Cells resuspended in succinyl gelatin injection solution:

1. Experimental steps:

(1) The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of succinyl gelatin injection solution was taken to perform resuspension;

(4) The cell suspension was divided equally into two tubes, about 1.5ml/tube, one of them was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 3h, 5h, 24h, 48h, 72h, 100h, 6day, 8day, 10day, 14day.

2. Experimental results:

Table 5-6: Viability detection results of cells resuspended in succinyl gelatin injection solution

| Detection time | Cells resuspended in succinyl gelatin injection solution | | | |
|---|---|---|---|---|
| | RT | | 4°C | |
| 0h | 5.07 | 96.8 | 5.07 | 96.8 |
| 3h | 4.78 | 97.3 | 4.98 | 96.5 |
| 5h | 5.55 | 96.5 | 5.18 | 95.7 |
| 24h | 4.90 | 91.0 | 4.37 | 95.3 |
| 48h | 3.91 | 87.0 | 4.38 | 86.0 |
| 72h | 4.14 | 84.3 | 4.07 | 83.5 |
| 100h | 3.20 | 78.0 | 4.25 | 78.3 |

(continued)

| Detection time | Cells resuspended in succinyl gelatin injection solution | | | |
|---|---|---|---|---|
| | RT | | 4°C | |
| 6day | 3.41 | 78.0 | 3.76 | 72.0 |
| 8day | 3.29 | 72.3 | 3.40 | 72.3 |
| 10day | 2.91 | 65.5 | 3.13 | 67.3 |
| 14day | 2.06 | 62.0 | 3.60 | 68.7 |

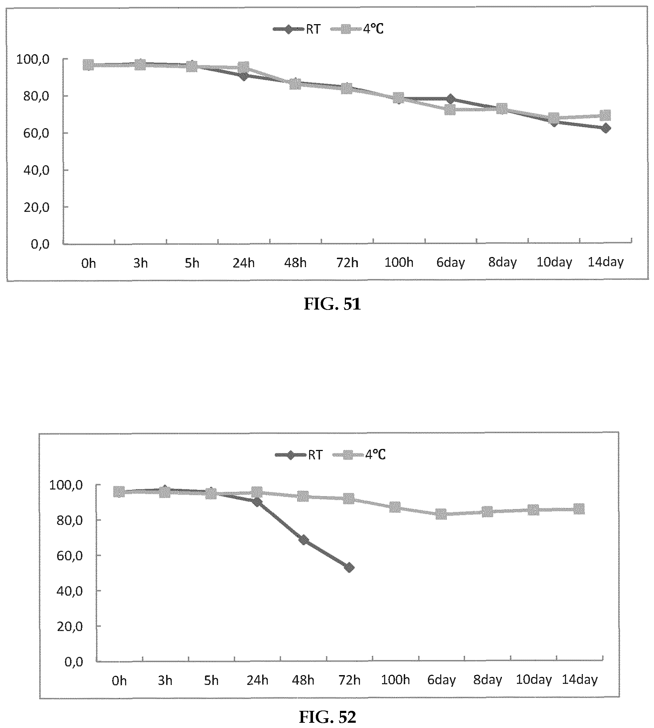

The cell viability detection results were shown in FIG. 51. After the M cell pellet was resuspended with succinyl gelatin injection, the M cell viability was maintained above 90% within 24 hours, and the M cell viability was maintained above 80% within 72 hours, under both RT and 4°C storage conditions, that was, there was no significant difference between RT and 4°C storage conditions.

(VII) MZJ injection solution 1 (non-cryopreserved)

1. Experimental steps:

(1) Preparation of MZJ injection solution 1: 6.5ml of compound electrolyte solution, 2.5ml of 20% HSA, 1ml of DMSO were respectively taken and placed in a 50ml centrifuge tube, mixed well, and stored at 4°C;
The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of MZJ injection solution was taken to perform resuspension;

(4) The cell suspension was divided equally into two tubes, about 1.5ml/tube, one of them was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 3h, 5h, 24h, 48h, 72h, 100h, 6day, 8day, 10day, 14day.

2. Experimental results:

Table 5-7: Viability detection results of cells resuspended in MZJ injection solution 1

| Detection time | Cells resuspended in MZJ injection solution 1 | | | |
|---|---|---|---|---|
| | RT | | 4°C | |
| 0h | 4.04 | 96.0 | 4.04 | 96.0 |
| 3h | 4.59 | 97.0 | 4.79 | 95.5 |
| 5h | 4.91 | 95.8 | 5.45 | 94.7 |
| 24h | 4.16 | 90.3 | 4.73 | 95.3 |
| 48h | 2.24 | 68.7 | 4.65 | 93.0 |
| 72h | 2.30 | 53.0 | 4.48 | 91.7 |
| 100h | 2.22 | 51.7 | 3.64 | 86.7 |
| 6day | 3.30 | 66.0 | 3.55 | 82.7 |

**EP 4 047 083 A1**

(continued)

| Detection time | Cells resuspended in MZJ injection solution 1 | | | |
|---|---|---|---|---|
| | RT | | 4°C | |
| 8day | 2.96 | 65.3 | 3.77 | 84.0 |
| 10day | 2.52 | 56.0 | 3.11 | 85.0 |
| 14day | | | 3.72 | 85.5 |

The cell viability detection results were shown in FIG. 52. After the M cell pellet was resuspended with MZJ injection solution 1, the M cell viability was kept above 90% within 24 hours under both RT and 4°C storage conditions; the effect of preserving M cells under 4°C condition was much better, and the M cell viability was maintained above 80% within 14 days.

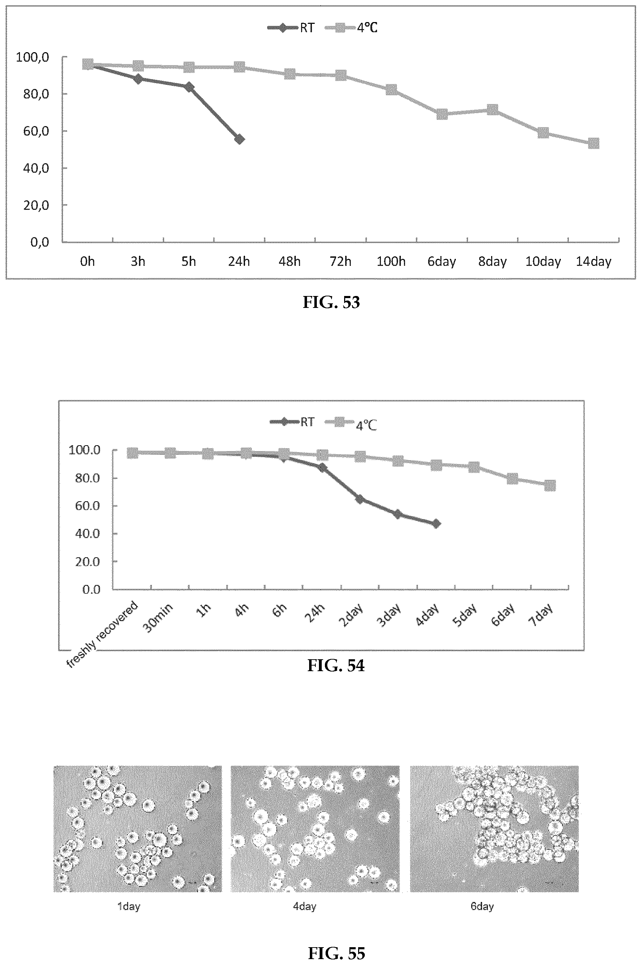

In addition, the MZJ injection was used to cryopreserve M cells at -80°C, and the detection results of M cell viability after the cryopreservation were shown in FIG. 54. After MZJ injection cryopreserved M cells were resuscitated, under RT storage conditions, the M cell viability was maintained above 90% within 5 hours, and the M cell viability was maintained above 80% within 24 hours; the effect of preserving M cells under at 4°C condition was much better, and the M cell viability was maintained above 80% within 5 days.

(VIII) Succinyl gelatin MIX injection

1. Experimental steps:

(1) Preparation of succinyl gelatin MIX injection: 6.5ml of compound electrolyte solution, 2.5ml of succinyl gelatin injection, and 1ml of DMSO were respectively taken and placed in a 50ml centrifuge tube, mixed well, and stored at 4°C;
The cells cultured to the P5 generation were harvested, stained with trypan blue and counted, and the total number of cells was estimated.

(2) 1.5ml of the cell suspension was taken and placed in a 15ml centrifuge tube, and centrifuged at 1500rpm for 5min;

(3) The supernatant was discarded, and 3ml of succinyl gelatin MIX injection was taken to perform resuspension;

(4) The cell suspension was divided equally into two tubes, about 1.5ml/tube, one of them was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 3h, 5h, 24h, 48h, 72h, 100h, 6day, 8day, 10day, 14day.

2. Experimental results:

Table 5-8: Viability detection results of cells resuspended in succinyl gelatin MIX injection solution

| Detection time | Succinyl gelatin MIX injection solution | | | |
|---|---|---|---|---|
| | RT | | 4°C | |
| 0h | 4.99 | 96.0 | 4.99 | 96.0 |
| 3h | 4.40 | 88.3 | 4.70 | 95.0 |
| 5h | 4.07 | 84.0 | 4.64 | 94.3 |
| 24h | 2.82 | 55.7 | 6.04 | 94.5 |

(continued)

| Detection time | Succinyl gelatin MIX injection solution | | | |
|---|---|---|---|---|
| | RT | | 4°C | |
| 48h | 1.06 | 23.3 | 4.32 | 90.7 |
| 72h | 1.38 | 30.3 | 4.51 | 90.0 |
| 100h | 1.29 | 31.3 | 4.35 | 82.3 |
| 6day | 1.09 | 26.5 | 3.54 | 69.0 |
| 8day | 0.39 | 5.7 | 3.48 | 71.3 |
| 10day | 0.06 | 2.0 | 2.47 | 59.0 |
| 14day | | | 2.05 | 53.3 |

The cell viability detection results were shown in FIG. 53. After the M cell pellet was resuspended with succinyl gelatin MIX injection, the M cell viability was maintained above 80% within 5 hours under RT storage conditions; the effect of preserving the M cells under 4°C storage conditions was much better, and the M cell viability was maintained above 90% within 72 hours.

(IX) MZJ Injection 1 (cryopreserved)

1. Experimental steps:

(1) Preparation of MZJ injection solution 1: 6.5ml of compound electrolyte solution, 2.5ml of 20% HSA, 1ml of DMSO were respectively taken and placed in a 50ml centrifuge tube, mixed well, and stored at 4°C;
The cells cultured to the P5 generation were harvested, stained with trypan blue and counted, and the total number of cells was estimated.

(2) A certain volume of the cell suspension was taken and placed in a 50ml centrifuge tube, and centrifuged at 1500rpm for 5min;

(3) The supernatant was discarded, and the MZJ injection solution 1 was taken to perform resuspension;

(4) The cell suspension was divided equally into two tubes, 1.0ml/tube, they were placed in a programmed cryopreservation box and subjected to programmed cryopreservation at -80°C, after 24 hours, they were transferred to liquid nitrogen or stored at -80°C for long-term storage.

(5) After a period of time, 2 tubes of M cells were taken out and resuscitated by a resuscitator, mixed, and divided equally in 2 tubes, among which one was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 30min, 1h, 4h, 6h, 24h, 2day, 3day, 4day, 5day, 6day, 7day.

2. Experimental results:

Table 5-9: Viability detection results of M cells after cryopreserved in MZJ injection solution 1 at -80°C

| Detection time | MZJ injection solution 1 | | | |
|---|---|---|---|---|
| | RT | | 4°C | |
| Just resuscitated | 4.92 | 98.3 | 4.92 | 98.3 |
| 30min | 5.80 | 98.0 | 5.49 | 97.4 |
| 1h | 5.01 | 98.0 | 4.85 | 98.0 |
| 4h | 5.54 | 96.8 | 5.57 | 97.5 |
| 6h | 5.14 | 94.5 | 6.48 | 97.2 |
| 24h | 4.78 | 83.0 | 6.40 | 96.5 |
| 2day | 3.27 | 53.3 | 6.78 | 96.0 |
| 3day | 3.04 | 51.8 | 6.36 | 89.5 |
| 4day | 2.92 | 45.4 | 6.58 | 85.7 |
| 5day | | | 5.20 | 84.5 |
| 6day | | | 4.86 | 75.5 |
| 7day | | | 4.87 | 72.5 |

The cell viability detection results were shown in FIG. 54. After MZJ injection solution 1 cryopreserved M cells were resuscitated, under RT storage conditions, the M cell viability was maintained above 90% within 5 hours, and the M cell viability was maintained above 80% within 24 hours; the effect of preserving M cells was better under 4°C conditions, and the M cell viability was maintained above 80% within 5 days.

(X) MZJ injection solution 2 (cryopreserved)

1. Experimental steps:

(1) Preparation of MZJ injection solution 2: 6.5ml of compound electrolyte solution, 2.5ml of 20% HSA, 300ul of 3% adenosine, and 1ml of DMSO were respectively taken and placed in a 50ml centrifuge tube, mixed well, and stored at 4°C;
The cells cultured to the P5 generation were harvested, stained with trypan blue and counted, and the total number of cells was estimated.

(2) a certain volume of the cell suspension was taken and placed in a 50ml centrifuge tube, and centrifuged at 1500rpm for 5min;

(3) The supernatant was discarded, and the MZJ injection solution 2 was taken to perform resuspension;

(4) The cell suspension was divided equally into two tubes, 1.0ml/tube, they were placed in a programmed cryopreservation box and subjected to programmed cryopreservation at -80°C, after 24 hours, they were transferred to liquid nitrogen or stored at -80°C for long-term storage.

(5) After a period of time, 2 tubes of M cells were taken out and resuscitated by a resuscitator, mixed, and divided equally in 2 tubes, among which one was kept at room temperature (RT), and the other was stored at 4°C. Samples of 50ul to 100ul of the cell suspension was taken before the storage, stained with trypan blue and subjected to detection of cell density and viability;

(5) The sampling and detection were performed at different time points in sequence: 30min, 1h, 4h, 6h, 24h, 2day, 3day, 4day, 5day, 6day, 7day.

2. Experimental results:

Table 5-10: Viability detection results of M cells after cryopreserved in MZJ injection solution 2

| Detection time | MZJ injection solution 2 | | | |
|---|---|---|---|---|
| | RT | | 4°C | |
| Just resuscitated | 5.46 | 98.3 | 5.46 | 98.3 |
| 30min | 5.92 | 98.0 | 6.57 | 98.3 |
| 1h | 6.13 | 98.0 | 5.41 | 97.8 |
| 4h | 6.00 | 97.0 | 6.86 | 98.3 |
| 6h | 5.01 | 95.0 | 6.79 | 97.8 |
| 24h | 5.90 | 87.8 | 6.72 | 96.7 |
| 2day | 4.60 | 65.0 | 7.64 | 95.8 |
| 3day | 3.78 | 54.0 | 7.30 | 92.4 |
| 4day | 3.33 | 47.3 | 5.93 | 89.5 |
| 5day | | | 6.61 | 88.1 |
| 6day | | | 6.50 | 79.7 |
| 7day | | | 5.99 | 75.0 |

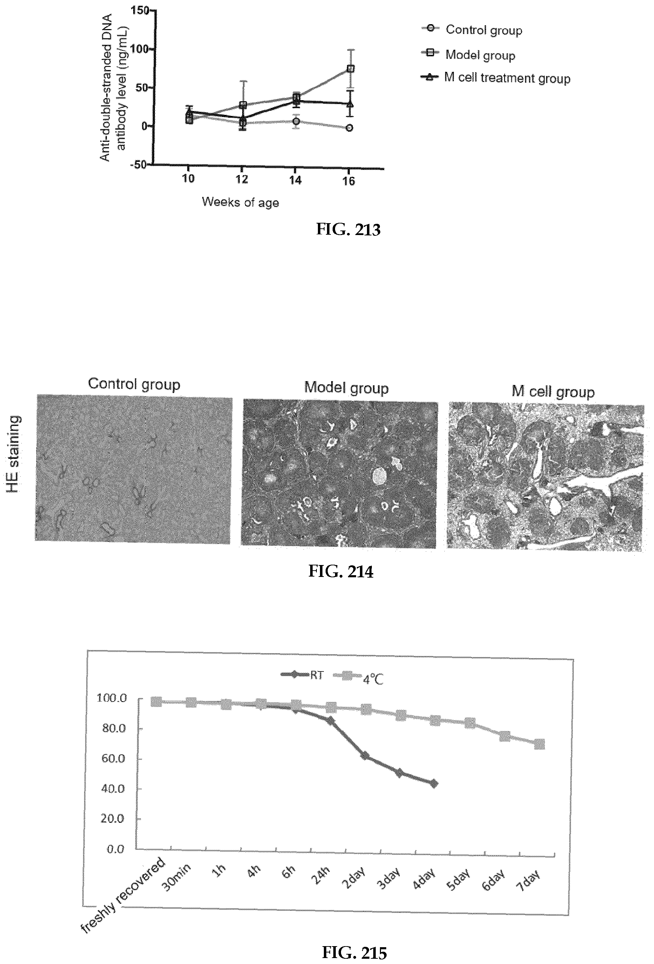

The cell viability detection results were shown in FIG. 215. After the MZJ injection solution 2 cryopreserved M cells were resuscitated, under RT storage conditions, the M cell viability was maintained above 90% within 6 hours, and the M cell viability was maintained above 80% within 24 hours; the effect of preserving M cells under at 4°C conditions was much better, and the M cell viability was maintained above 90% within 3 days, and the M cell viability was maintained above 80% within 5 days.

(XII) MZJ injection 3 (cryopreserved)

1. Experimental procedure

Preparation solution preparation: 2.925ml of compound electrolyte solution was taken and placed in a 15ml centrifuge tube, added with 2.925ml of glucose injection and mixed well, added with 900ul of USP grade DMSO and mixed well, and finally added with 2.25ml of HSA and mixed well, sealed with parafilm, and allowed to stand at 4°C for precooling;

The M cell suspension was mixed evenly, counted, divided equally in two tubes, centrifuged at 1200 rpm for 3 min; the supernatant was discarded, and 4 ml of the preparation solution was respectively taken and the cells were resuspended and counted (not recorded);

Subpackage: 600ul/tube, placed in a programmed cooling box, and stored at -80°C;

After 24 hours, the cells were taken out from the cryopreservation boxes respectively, and placed in liquid nitrogen for cryopreservation;

After cryopreservation for 14 days, 3 tubes of each sample were taken out for resuscitation; after being resuspended and mixed evenly, 550ul of the cell suspension was taken out and added with 550ul of normal saline for dilution, and stored at 4°C, and the cell viability detection was carried out at different time points (0h, 30min, 1h, 2h, 3h, 6h, 9h, 24h, 48h, 72h).

Cell viability detection: In the biological safety cabinet, the cell suspension was gently mixed by pipetting, 10ul of the cell suspension was taken, added with 10ul of trypan blue and mixed well, 10ul of the mixture was taken and added to a chamber at one side of the counting plate, allowed to stand for 10 to 30s, then inserted into a counter for counting; each sample was detected 3 times;

Table 5-11: Viability detection results of M cells after cryopreserved in MZJ injection solution 3

| Dilution factor | Detection time | Number of cells | Cell viability |
|---|---|---|---|
| Undiluted | 0h | 5.32 | 98 |
| Diluted 2 times | 0h | 2.85 | 97 |
| | 30min | 2.66 | 97 |
| | 1h | 2.85 | 97 |
| | 2h | 2.84 | 96 |
| | 3h | 2.80 | 96 |
| | 6h | 2.69 | 96 |
| | 9h | 2.93 | 96 |
| | 24h | 2.92 | 95 |
| | 48h | 2.69 | 93 |
| | 72h | 2.82 | 88 |

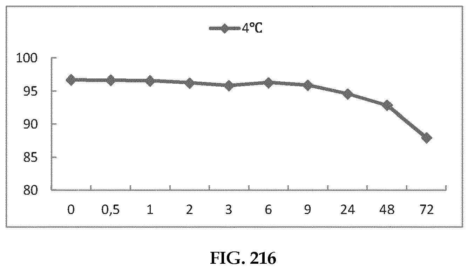

The cell viability detection results were shown in FIG. 216. After MZJ injection solution 3 cryopreserved M cells were resuscitated, under 4°C storage conditions, the M cell viability was maintained above 90% within 48 hours, and the M cell viability was maintained above 85% within 3 days.

Example 6: Method for culturing human embryonic stem cell-derived M cells on microcarrier

Experimental process and methods:

(1) Culturing M cells with different microcarriers:

The M cells were cultured to the P4 generation and harvested, resuspended in the culture medium, inoculated onto commercial microcarriers, placed in a 37°C incubator for static culture, and the culture medium was changed every other day. Digestion was carried out by using lysis solution or Tryple, the cells were harvested and subjected to identification of cell surface markers. During the culturing process, samples were taken for live-dead detection to observe the cell growth state.

(2) Dynamic suspension culture of M cells:

The M cells were cultured to the P2 generation and harvested, resuspended in the culture medium, inoculated onto porous gelatin microcarriers, and placed in a 37°C incubator for rolling culture, in which the interval-type incubation was used within 24 hours, and after 24 hours, the rolling culture at constant speed was carried out, and the culture medium was replaced during the culture. After 6 days of culture, the microcarriers were lysed with microcarrier lysis solution, then passage was carried out until the cells were cultured to P5, and the harvested cells were subjected to identification of surface markers. During the culture, samples were taken for live-dead detection to observe the cell growth state.

Equipment/equipment, reagents and consumables used were as follows:

| Reagents | | | |
|---|---|---|---|
| Name | Manufacturer | Cat. No. | |
| 20% human serum albumin | Shandong Taibang Biological Products Co., Ltd. | SFDA Approval No. S10970005 | |
| Compound electrolyte solution | Shanghai Baxter | A6E2543 | |

(continued)

| Reagents | | | |
|---|---|---|---|
| Name | Manufacturer | Cat. No. | |
| DMSO | OriGen | CP-70 | |
| CS10 | stem cell | 7930 | |
| live-dead | Thermo | L3224 | |
| BSA | SIGMA | A8022 | |
| SSEA-4 | BD Pharmingen | 560128 | |
| CD34 | BD Pharmingen | 555822 | |
| CD90 | eBioscience | 12-0909-42 | |
| CD105 | BioLegend | 800503 | |
| CD45 | eBioscience | 11-9459-42 | |
| CD73 | BD Bioscience | 561014 | |
| CD11b | BioLegend | 301305 | |
| CD19 | BD Pharmingen | 561741 | |
| CD29 | BioLegend | 303004 | |
| HLA-DR | BD Pharmingen | 555558 | |
| PE IgG1 | BD Pharmingen | 551436 | |
| FITC IgG1 | BD Pharmingen | 555748 | |
| FITC IgG2a | BD Pharmingen | 555573 | |
| PE IgG3 | BD Pharmingen | 556659 | |
| Consumables | | | |
| Name | Manufacturer | Cat. No. | |
| TableTrix microslide | Huakan Biological | F01-50 | |
| Cultispher | Sigma | M9418 | |
| Coring microslide | CORING | m-Dev45 | |
| Cytodex3 | GE | 17-0485-01 | |
| Solohill | Sigma | SLBL2958L | |
| 50ml centrifuge tube | CORING | 430829 | |
| 5ml pipette | CORING | 4487 | |
| 10ml pipette | CORING | 4488 | |
| 25ml pipette | CORING | 4489 | |
| 10cm low-attachment dish | CORING | 3236 | |
| 0.2um Supor$^R$ Membrane | PALL life Sciences | 4652 | |
| 10ml syringe | Jiangsu Zhiyu Medical Equipment Co., Ltd. | CFDSM Approval No. 20173150338 | |
| Counting plate | Invitrogen | 100078809 | |
| Blue tip | Axygen | T-1000-B-R-S | |
| White tip | Axygen | T-300-R-S | |
| Yellow tip | Axygen | T-200-Y-R-S | |

(continued)

| Reagents | | | |
|---|---|---|---|
| Name | Manufacturer | Cat. No. | |
| Instrument/equipment | | | |
| Name | Manufacturer | Model | Device |
| Biological safety cabinet | Thermo | 09RS1200 | 1300 series A2 |
| 4°C refrigerator | Haier | RCD-256KDC | 08AS1021 |
| $CO_2$ incubator | Thermo scientific | 3131 | 08RS1202 |
| $CO_2$ incubator | Thermo scientific | 3131 | 07RS 1203 |
| Inverted microscope | Leica | DNil | 15RS1208 |
| Centrifuge | eppendorf | 5804R | 15RS1232 |
| Cell counter | Coutess | Coutess II FL | 15RS0304 |
| Flow cytometer | BECKMAN | CytoFLEX | 19QS1092 |

I. M cells cultured on Cytodex3:

1. Experimental steps:

    (1) Cell inoculation:

        a. The P4 generation M cells cultured with T225 were digested: the supernatant was discarded, 10ml DPBS was added to wash once, 10ml of TrypLE was added, digestion was carried out at 37°C for 3min, 10ml of DPBS was added to stop the reaction, transferred to a 50ml centrifuge tube; centrifugation was carried out at 1500rpm for 5min; the supernatant was discarded, the cell pellet was resuspended with the culture medium, and counted by using trypan blue;

        b. Centrifugation was carried out at 1500rpm for 5min, the supernatant was discarded, and the cell suspension was resuspended to a density of $4 \times 10^6$ cells/ml.

        c. 40mg of microcarriers was weighed, sterilized, placed in a 10cm low-attachment dish, added with 12ml of culture medium; the above cell suspension was inoculated at 200um/each microcarrier, and cultured at 37°C;

        d. Medium was replenished to reach 16ml on the first day, and then the culture medium was changed every other day: 8ml of culture medium was discarded, and 8ml was added.

    (2) Live-dead detection:

        a. The live-dead detection solution was prepared in the dark: 10ml of DPBS was taken and placed in a 15ml centrifuge tube, added with 5ul of Calcein AM and 20ul of ethidium homodimer-1 respectively, mixed well and stored at 4°C;

        b. The microcarriers were gently mixed, 1ml of the suspension was taken out and placed in a centrifuge tube, allowed to settle for 1 to 2 minute, the supernatant was discarded, and DPBS was added to wash twice;

        c. The live-dead detection solution was added, 1ml/tube, and incubated at room temperature for 30min;

        d. The supernatant was discarded, DPBS was added to wash once, and photos were taken with a fluorescence microscope.

(3) Cell digestion:

a. The microcarriers were naturally precipitated for 1 to 2 minutes, the supernatant was discarded, and washing was carried out with DPBS;

b. 5ml of TrypLE/10cm dish was added, digestion was carried out at 37°C for 10min, 5ml of DPBS was added, transferred to a 50ml centrifuge tube, and a 70um filter was used to filter and collect the cells;

c. Centrifugation was carried out at 1200rpm for 3min, and the supernatant was discarded;

d. After resuspending with culture medium, trypan blue staining was performed for counting;

e. An appropriate amount of the cells was taken, and centrifuged at 1200rpm for 3min to collect the cells;

(4) Flow cytometry:

a. After the cell pellet was resuspended in 2% BSA, the cells were blocked for 30min;

b. Centrifugation was carried out at 1200rpm for 3min, and the supernatant was discarded;

c. After resuspending in 1% BSA, the cells were subpackaged, 200ul/tube;

d. After adding antibodies according to the antibody instructions, the incubation was carried out at room temperature for 30 minutes;

e. Washing was performed twice with DPBS;

f. After resuspending in DPBS, the cells were filtered with a 0.22um filter;

g. After loading, the detection was performed.

(5) In-situ cryopreservation:

a. The harvested P4 generation M cell suspension was inoculated at $9.5 \times 10^5$, incubation was carried out at 37°C for 2h, and then medium replenishing was performed;

b. After culturing at 37°C for 24 hours, the medium was replenished and changed: 3ml of culture medium was discarded and 3ml of fresh medium was added; subsequently, the culture medium was changed every other day: 4ml of culture medium was discarded and 4ml of fresh medium was added.

c. Sampling and live-dead detection were performed before cryopreservation to detect cell status, and cryopreservation was carried out after 5 days of culture;

d. Preparation of cryopreservation solution MZJ: 20ml = 13ml of compound electrolyte solution + 5ml of 20%HSA + 2ml of DMSO;

e. The microcarriers were washed twice with DPBS respectively, the Cytodex3 microcarriers were divided into 3 equal parts, the supernatant was discarded, and the residue was cryopreserved with MZJ, CS10 and vitrification cryopreservation solution, respectively; except for the vitrification cryopreservation solution, the others were placed in programmed cryopreservation boxes, and transferred to liquid nitrogen for storage after 24 hours;

f. Vitrification cryopreservation procedure: (1) the exchange with balance solution ES was performed for 12 to 18min, 0.5ml/tube; (2) after the supernatant was discarded, the vitrification cryopreservation solution VS30-50s was added; (3) the supernatant was discarded, and the residue was transferred in cryopreservation tube; (4) the cryopreservation tube was immediately placed into liquid nitrogen to perform instant quick-freezing, and then transferred into a liquid nitrogen tank for storage;

g. After 12 days of cryopreservation in liquid nitrogen, the Cytodex3 after vitrification cryopreservation was taken from liquid nitrogen and resuscitated; in which the resuscitation method for vitrification cryopreservation comprises: (1) after taking from liquid nitrogen, it was immediately placed in 300ul of TS reagent, placed in a metal bath at 37°C for 1min, and allowed to stand for about 1 min, the TS was discarded; (2) 300ul of DS reagent was added, allowed to stand at room temperature for 3min, the supernatant was discarded, 300ul of WS1 was added and allowed to stand at room temperature for 5min, the supernatant was discarded, 300ul of WS2 was added and allowed to stand at room temperature for 1 to 2 minutes, the supernatant was discarded, the culture solution was added to wash once, then it was transferred to 2ml of culture solution/well/6-well plate, and cultured at 37°C;

h. After about 30min to 1h, a part of it was taken and used for the live-dead detection, in which the part was observed under a fluorescence microscope, and pictures thereof were taken;

i. After cryopreservation in liquid nitrogen for 15 days, the microcarriers cryopreserved in MZJ and CS10 were taken from liquid nitrogen, a resuscitator was used for resuscitation, the culture medium was added to wash twice, then they were transferred to 6-well plate with 2ml of culture medium/well, and cultured at 37°C;

j. After about 30min to 1h, a part of it was taken for live-dead detection, in which the part was observed under a fluorescence microscope, and photos thereof were taken.

2. Experimental results:

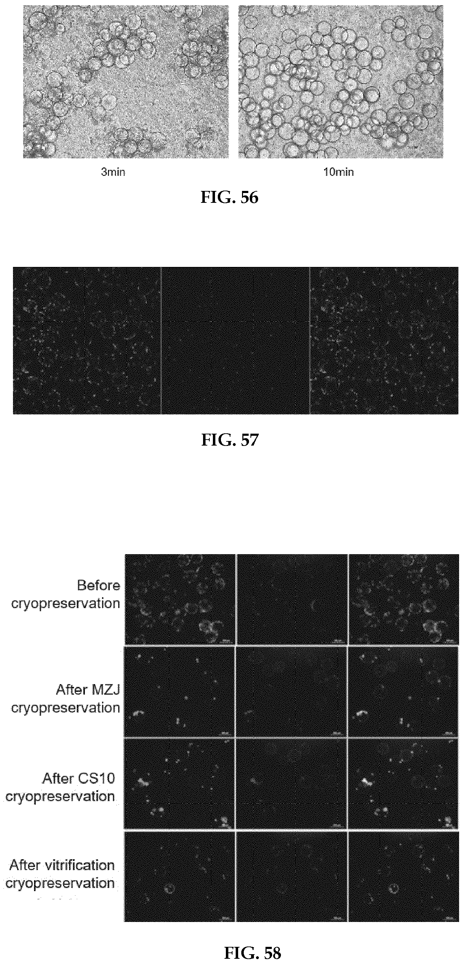

The morphological photos of M cells cultured on Cytodex3 were shown in FIG. 55, and the results showed that the M cells could cultured and proliferated on Cytodex3 microcarriers. The morphological photos of M cells after digestion were shown in FIG. 56, and the results showed that the M cells could be harvested by TrypLE digestion. The results of live-dead detection were shown in FIG. 57, which indicated that the M cells could well attached to the Cytodex3 microcarriers.

The flow cytometry results for surface markers were shown in the table below, which indicated that culturing M cells on Cytodex3 microcarriers did not affect their marker expression.

Table 6-1: Flow cytometry results of M cells cultured on Cytodex3

| Marker | Cytodex3 |
|---|---|
| CD34 | 0.68% |
| CD90 | 99.64% |
| CD105 | 99.09% |
| CD73 | 99.98% |
| CD11b | 0.17% |
| HLA-ABC | 99.62% |
| HLA-DR | 0.08% |
| CD19 | 0.48% |
| CD29 | 99.95% |

The detection results of Cytodex3 in-situ cryopreservation were shown in FIG. 58, and the results showed that detachment could be observed after Cytodex3 in-situ cryopreservation.

II. M cells cultured on Cultispher:

1. Experimental steps:

(1) Cell inoculation:

a. The P4 generation M cells cultured with T225 were digested: the supernatant was discarded, 10ml of DPBS was added to wash once, 10ml of TrypLE was added, digested at 37°C for 3min, added with 10ml of DPBS to stop the reaction, transferred to a 50ml centrifuge tube; centrifuged at 1500rpm for 5min; the supernatant was discarded, the cell pellet was resuspended with culture medium, and the counting was performed with trypan blue;

b. Centrifugation was performed at 1500rpm for 5min, the supernatant was discarded, and the cell suspension was resuspended to a density of $4 \times 10^6$ cells/ml.

c. 40mg of microcarriers was weighed, sterilized, placed in a 10cm low-attachment dish, 12ml of the culture medium was added; the above cell suspension was inoculated at 200ul/each of microcarrier, and cultured at 37°C;

d. On the 1st day, the medium was replenished to reach 16ml, and then the culture medium was changed every other day: 8ml of culture medium was discarded, and 8ml was added.

(2) Live-dead detection:

a. The live-dead detection solution was prepared in the dark: 10ml of DPBS was taken and placed in a 15ml centrifuge tube, added with 5ul of Calcein AM and 20ul of ethidium homodimer-1 respectively, mixed well and stored at 4°C;

b. The microcarriers were gently mixed, 1ml of the suspension was taken out and placed in a centrifuge tube, allowed to settle for 1 to 2 minute, the supernatant was discarded, and DPBS was added to wash twice;

c. The live-dead detection solution was added, 1ml/tube, and incubated at room temperature for 30min;

d. The supernatant was discarded, DPBS was added to wash once, and photos were taken with a fluorescence microscope.

(3) Cell digestion:

a. The microcarriers were naturally precipitated for 1 to 2 minutes, the supernatant was discarded, and washing was carried out with DPBS;

b. 5ml of TrypLE/10cm dish was added, digestion was carried out at 37°C for 10min, 5ml of DPBS was added, transferred to a 50ml centrifuge tube, and a 70um filter was used to filter and collect the cells;

c. Centrifugation was carried out at 1200rpm for 3min, and the supernatant was discarded;

d. After resuspending with culture medium, trypan blue staining was performed for counting;

e. An appropriate amount of the cells was taken, and centrifuged at 1200rpm for 3min to collect the cells;

(4) Flow cytometry:

a. After the cell pellet was resuspended in 2% BSA, the cells were blocked for 30min;

b. Centrifugation was carried out at 1200rpm for 3min, and the supernatant was discarded;

c. After resuspending in 1% BSA, the cells were subpackaged, 200ul/tube;

d. After adding antibodies according to the antibody instructions, the incubation was carried out at room temperature for 30 minutes;

e. Washing was performed twice with DPBS;

f. After resuspending in DPBS, the cells were filtered with a 0.22um filter;

g. After loading, the detection was performed.

(5) In-situ cryopreservation:

a. The harvested P4 generation M cell suspension was inoculated at $1.1 \times 10^6$, incubated at 37°C for 2h, and then medium replenishing was performed;

b. After culturing at 37°C for 24 hours, the medium was replenished and changed: 3ml of culture medium was discarded, and 3ml of fresh medium was added; subsequently, the culture medium was changed every other day: 4ml of culture medium was discarded and 4ml of fresh medium was added.

c. Sampling for live-dead detection were performed before cryopreservation to detect cell status, and cryopreservation was carried out after 5 days of culture;

d. Preparation of cryopreservation solution MZJ: 20ml = 13ml of compound electrolyte solution + 5ml of 20%HSA + 2ml of DMSO;

e. The microcarriers were washed twice with DPBS respectively, the Cultispher microcarriers were divided into 3 equal parts, the supernatant was discarded, and the residue was cryopreserved with MZJ, CS10 and vitrification cryopreservation solution, respectively; except for the vitrification cryopreservation solution, the others were placed in programmed cryopreservation boxes, and transferred to liquid nitrogen for storage after 24 hours;

f. Vitrification cryopreservation procedure: (1) the exchange with balance solution ES was performed for 12 to 18min, 0.5ml/tube; (2) after the supernatant was discarded, the vitrification cryopreservation solution VS30-50s was added; (3) the supernatant was discarded, and the residue was transferred in cryopreservation tube; (4) the cryopreservation tube was immediately placed into liquid nitrogen to perform instant quick-freezing, and then transferred into a liquid nitrogen tank for storage;

g. After 12 days of cryopreservation in liquid nitrogen, the Cultispher after vitrification cryopreservation was taken from liquid nitrogen and resuscitated; in which the resuscitation method for vitrification cryopreservation comprises: (1) after taking from liquid nitrogen, it was immediately placed in 300ul of TS reagent, placed in a metal bath at 37°C for 1min, and allowed to stand for about 1 min, the TS was discarded; (2) 300ul of DS reagent was added, allowed to stand at room temperature for 3min, the supernatant was discarded, 300ul of WS1 was added and allowed to stand at room temperature for 5min, the supernatant was discarded, 300ul of WS2 was added and allowed to stand at room temperature for 1 to 2 minutes, the supernatant was discarded, the culture solution was added to wash once, then it was transferred to 6-well plate with 2ml of culture solution/well, and cultured at 37°C;

h. After about 30min to 1h, a part thereof was taken and used for the live-dead detection, in which the part was observed under a fluorescence microscope, and pictures thereof were taken;

i. After cryopreservation in liquid nitrogen for 15 days, the microcarriers cryopreserved in MZJ and CS10 were taken from liquid nitrogen, a resuscitator was used for resuscitation, the culture medium was added to wash twice, then they were transferred to 6-well plate with 2ml of culture medium/well, and cultured at 37°C;

j. After about 30min to 1h, a part thereof was taken for live-dead detection, in which the part was observed under a fluorescence microscope, and photos thereof were taken.

2. Experimental results:

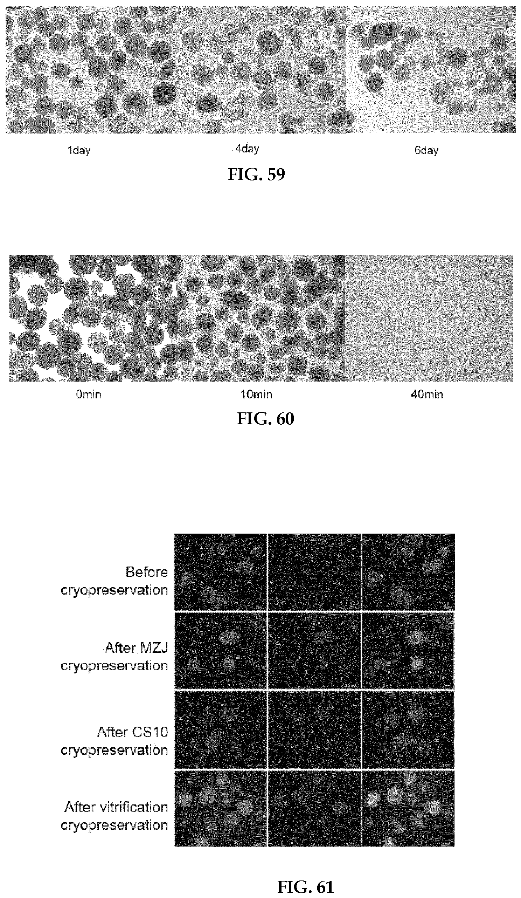

The morphological photos of M cells cultured on Cultispher were shown in FIG. 59, and the results showed that the M cells were cultured on Cultispher microcarriers, and it was difficult to observe cell proliferation under microscope. The morphological photos of M cells cultured with Cultispher after digestion were shown in FIG. 60, and the results showed that the M cells could be harvested by TrypLE digestion.

The results of flow cytometry for surface markers were shown in the table below, which indicated that culturing M cells on Cultispher microcarriers did not affect their marker expression.

Table 6-2: Flow cytometry results of M cells cultured on Cultispher

| Marker | Cultispher |
|---|---|
| CD34 | 0.10% |
| CD90 | 98.92% |
| CD105 | 99.98% |
| CD73 | 99.99% |
| CD11b | 0.08% |
| HLA-ABC | 99.95% |
| HLA-DR | 0.11% |
| CD19 | 0.08% |
| CD29 | 99.92% |
| CD45 | 0.56% |

The results of Cultispher in-situ cryopreservation were shown in FIG. 61, and the results showed that the cells died after Cultispher in-situ cryopreservation.

III. M cells cultured on TableTrix microslides:

1. Experimental method:

(1) Cell inoculation:

a. The P4 generation M cells cultured with T225 were digested: the supernatant was discarded, 10ml of DPBS was added to wash once, 10ml of TrypLE was added, digested at 37°C for 3min, added with 10ml of DPBS to stop the reaction, transferred to a 50ml centrifuge tube; centrifuged at 1500rpm for 5min; the supernatant was discarded, the cell pellet was resuspended with culture medium, and the counting was performed with trypan blue;

b. Centrifugation was performed at 1500rpm for 5min, the supernatant was discarded, and the cell suspension was resuspended to a density of $4\times10^6$ cells/ml.

c. Two TableTrix microslides were taken and placed in a 10cm low-attachment dish, 200ul of the above cell suspension was inoculated on each microcarrier, incubated at 37°C for 2h, and replenished to reach 12ml of culture solution/10cm dish;

d. On the 1st day, the medium was replenished to reach 16ml, and then the culture medium was changed every other day: 8ml of culture medium was discarded, and 8ml was added.

(2) Live-dead detection:

a. The live-dead detection solution was prepared in the dark: 10ml of DPBS was taken and placed in a 15ml centrifuge tube, added with 5ul of Calcein AM and 20ul of ethidium homodimer-1 respectively, mixed well and stored at 4°C;

b. The microcarriers were gently mixed, 1ml of the suspension was taken out and placed in a centrifuge tube, allowed to settle for 1 to 2 minute, the supernatant was discarded, and DPBS was added to wash twice;

c. The live-dead detection solution was added, 1ml/tube, and incubated at room temperature for 30min;

d. The supernatant was discarded, DPBS was added to wash once, and photos were taken with a fluorescence microscope.

(3) Cell digestion:

    a. The microcarriers were naturally precipitated for 1 to 2 minutes, the supernatant was discarded, and washing was carried out with DPBS;

    b. Digest lysis buffer was used to lyse for 40 to 60 minutes, pipetting once in the middle, and transferred to a 50ml centrifuge tube;

    c. Centrifugation was carried out at 1200rpm for 3min, and the supernatant was discarded;

    d. After resuspending with culture medium, trypan blue staining was performed for counting;

    e. An appropriate amount of the cells was taken, and centrifuged at 1200rpm for 3min to collect the cells;

(4) Flow cytometry:

    a. After the cell pellet was resuspended in 2% BSA, the cells were blocked for 30min;

    b. Centrifugation was carried out at 1200rpm for 3min, and the supernatant was discarded;

    c. After resuspending in 1% BSA, the cells were subpackaged, 200ul/tube;

    d. After adding antibodies according to the antibody instructions, the incubation was carried out at room temperature for 30 minutes;

    e. Washing was performed twice with DPBS;

    f. After resuspending in DPBS, the cells were filtered with a 0.22um filter;

    g. After loading, the detection was performed.

(5) In-situ cryopreservation:

    a. The harvested P4 generation M cells were formulated into a cell suspension with a density of $4 \times 10^6$, two TableTrix microslides were inoculated: each microslide was inoculated with the above cell suspension, incubated at 37°C for 2h, and then medium replenishing was performed;

    b. After culturing at 37°C for 24 hours, the medium was replenished and changed: 3ml of culture medium was discarded, and 3ml of fresh medium was added; subsequently, the culture medium was changed every other day: 4ml of culture medium was discarded and 4ml of fresh medium was added.

    c. Sampling for live-dead detection were performed before cryopreservation to detect cell status, and cryopreservation was carried out after 5 days of culture;

    d. Preparation of cryopreservation solution MZJ: 20ml = 13ml of compound electrolyte solution + 5ml of 20%HSA + 2ml of DMSO;

    e. The microcarriers were washed twice with DPBS respectively, the TableTrix microslides were divided into 3 equal parts, the supernatant was discarded, and the residue was cryopreserved with MZJ, CS10 and vitrification cryopreservation solution, respectively; except for the vitrification cryopreservation solution, the others were placed in programmed cryopreservation boxes, and transferred to liquid nitrogen for storage after 24 hours;

    f. Vitrification cryopreservation procedure: (1) the exchange with balance solution ES was performed for 12 to 18min, 0.5ml/tube; (2) after the supernatant was discarded, the vitrification cryopreservation solution VS30-50s was added; (3) the supernatant was discarded, and the residue was transferred in cryopreservation tube; (4) the cryopreservation tube was immediately placed into liquid nitrogen to perform instant quick-freezing, and then transferred into a liquid nitrogen tank for storage;

g. After 12 days of cryopreservation in liquid nitrogen, the TableTrix after vitrification cryopreservation was taken from liquid nitrogen and resuscitated; in which the resuscitation method for vitrification cryopreservation comprises: (1) after taking from liquid nitrogen, it was immediately placed in 300ul of TS reagent, placed in a metal bath at 37°C for 1min, and allowed to stand for about 1 min, the TS was discarded; (2) 300ul of DS reagent was added, allowed to stand at room temperature for 3min, the supernatant was discarded, 300ul of WS1 was added and allowed to stand at room temperature for 5min, the supernatant was discarded, 300ul of WS2 was added and allowed to stand at room temperature for 1 to 2 minutes, the supernatant was discarded, the culture solution was added to wash once, then it was transferred to 6-well plate with 2ml of culture solution/well, and cultured at 37°C;

h. After about 30min to 1h, a part thereof was taken and used for the live-dead detection, in which the part was observed under a fluorescence microscope, and pictures thereof were taken;

i. After cryopreservation in liquid nitrogen for 15 days, the microcarriers cryopreserved in MZJ and CS10 were taken from liquid nitrogen, a resuscitator was used for resuscitation, the culture medium was added to wash twice, then they were transferred to 6-well plate with 2ml of culture medium/well, and cultured at 37°C;

j. After about 30min to 1h, a part thereof was taken for live-dead detection, in which the part was observed under a fluorescence microscope, and photos thereof were taken.

2. Experimental results:

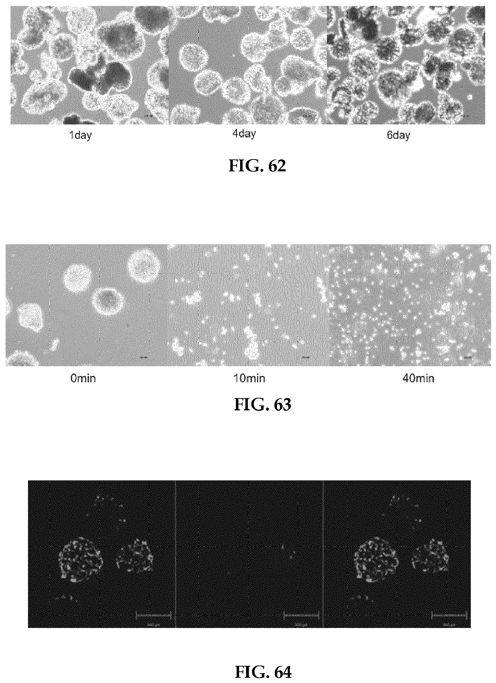

The morphological photos of M cells cultured with TableTrix were shown in FIG. 62, and the results showed that M cells could be cultured on TableTrix microcarriers, and it was difficult to observe cell proliferation phenomenon under the microscope. FIG. 63 showed the morphological photos of M cells cultured on TableTrix after digestion, and the results showed that the M cells could be harvested by digestion with Digest and Tryple. TableTrix microcarriers allowed for sphere-to-sphere passaging.

The results of the live-dead detection were shown in FIG. 64, and the results showed that the M cells could be well attached to the TableTrix microcarriers.

The results of flow cytometry detection for surface markers were shown in the table below, which indicated that culturing M cells on TableTrix microcarriers did not affect their marker expression.

Table 6-3: Flow cytometry detection results of M cells cultured on TableTrix

| Marker | 3D TableTrix™ |
|--------|---------------|
| CD34 | 0.37% |
| CD90 | 98.75% |
| CD105 | 99.59% |
| CD73 | 99.91% |
| HLA-ABC | 96.75% |

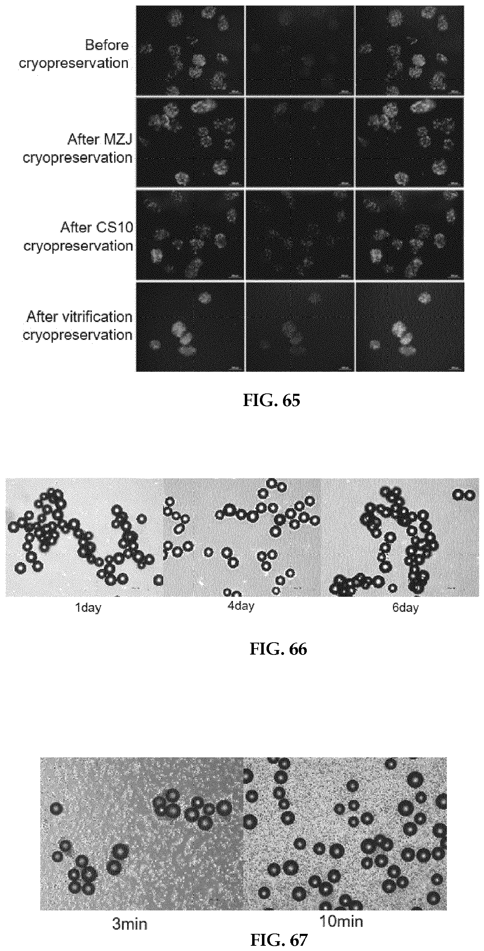

The TableTrix in-situ cryopreservation experiment results were shown in FIG. 65, and MZJ showed better effect on TableTrix in-situ cryopreservation.

IV. M cells cultured on Solohill microcarriers:

1. Experimental steps:

(1) Cell inoculation:

a. The P4 generation M cells cultured with T225 were digested: the supernatant was discarded, 10ml of DPBS was added to wash once, 10ml of TrypLE was added, digested at 37°C for 3min, added with 10ml of DPBS to stop the reaction, transferred to a 50ml centrifuge tube; centrifuged at 1500rpm for 5min; the supernatant was discarded, the cell pellet was resuspended with culture medium, and the counting was performed with trypan blue;

b. Centrifugation was performed at 1500rpm for 5min, the supernatant was discarded, and the cell suspension was resuspended to a density of $4 \times 10^6$ cells/ml.

c. 40mg of microcarriers were weighed, sterilized, placed in a 10cm low-attachment dish, added with 12ml of culture medium; 200ul of the above cell suspension was inoculated on each microcarrier, incubated at 37°C for 2h;

d. On the 1st day, the medium was replenished to reach 16ml, and then the culture medium was changed every other day: 8ml of culture medium was discarded, and 8ml was added.

(2) Live-dead detection:

a. The live-dead detection solution was prepared in the dark: 10ml of DPBS was taken and placed in a 15ml centrifuge tube, added with 5ul of Calcein AM and 20ul of ethidium homodimer-1 respectively, mixed well and stored at 4°C;

b. The microcarriers were gently mixed, 1ml of the suspension was taken out and placed in a centrifuge tube, allowed to settle for 1 to 2 minutes, the supernatant was discarded, and DPBS was added to wash twice;

c. The live-dead detection solution was added to each tube, 1ml/tube, and incubated at room temperature for 30min;

d. The supernatant was discarded, DPBS was added to wash once, and photos were taken with a fluorescence microscope.

(3) Cell digestion:

a. The microcarriers were naturally precipitated for 1 to 2 minutes, the supernatant was discarded, and washing was carried out with DPBS;

b. 5ml TrypLE/10cm dish was added, digested at 37°C for 10min, added with 5ml of DPBS, transferred to a 50ml centrifuge tube, and filtered with a 70um filter to collect cells;

c. Centrifugation was carried out at 1200rpm for 3min, and the supernatant was discarded;

d. After resuspending with culture medium, trypan blue staining was performed for counting;

e. An appropriate amount of the cells was taken, and centrifuged at 1200rpm for 3min to collect the cells;

(4) Flow cytometry:

a. After the cell pellet was resuspended in 2% BSA, the cells were blocked for 30min;

b. Centrifugation was carried out at 1200rpm for 3min, and the supernatant was discarded;

c. After resuspending in 1% BSA, the cells were subpackaged, 200ul/tube;

d. After adding antibodies according to the antibody instructions, the incubation was carried out at room temperature for 30 minutes;

e. Washing was performed twice with DPBS;

f. After resuspending in DPBS, the cells were filtered with a 0.22um filter;

g. After loading, the detection was performed.

(5) In-situ cryopreservation:

a. The harvested P4 generation M cell generation was harvested and inoculated at $8 \times 10^5$, incubated at 37°C for 2h, and replenished with medium;

b. After culturing at 37°C for 24 hours, the medium was replenished and changed: 3ml of culture medium was discarded, and 3ml of fresh medium was added; subsequently, the culture medium was changed every other day: 4ml of culture medium was discarded and 4ml of fresh medium was added.

c. Sampling for live-dead detection were performed before cryopreservation to detect cell status, and cryopreservation was carried out after 5 days of culture;

d. Preparation of cryopreservation solution MZJ: 20ml = 13ml of compound electrolyte solution + 5ml of 20%HSA + 2ml of DMSO;

e. The microcarriers were washed twice with DPBS respectively, the Solohill microslides were divided into 2 equal parts, the supernatant was discarded, and the residue was cryopreserved with MZJ, CS10, respectively; placed in programmed cryopreservation boxes, and transferred to liquid nitrogen for storage after 24 hours;

f. After cryopreservation in liquid nitrogen for 15 days, the Solohill microcarriers were taken out from the liquid nitrogen, a resuscitator was used for resuscitation, the culture solution was added to wash twice, it was then transferred to 6-well plate with 2ml of culture solution/well, and cultured at 37°C;

g. After about 30min to 1h, a part thereof was taken and used for live-dead detection, in which the part was observed under a fluorescence microscope, and photos were taken.

2. Experimental results:

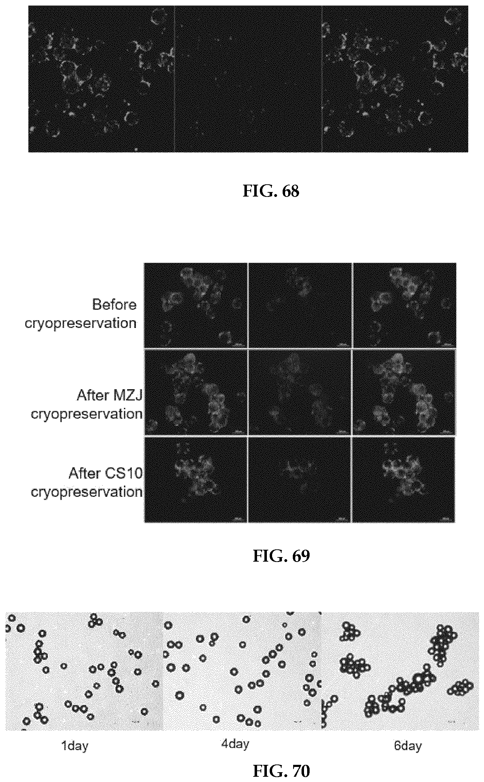

The morphological photos of M cells cultured with Solohill were shown in FIG. 66, and the results showed that M cells could be cultured and proliferated on Solohill microcarriers. FIG. 67 showed the morphological photos of M cells cultured on Solohill after digestion, and the results showed that the M cells could be harvested by digestion with TrypleE.
The results of the live-dead detection were shown in FIG. 68, and the results showed that the M cells could be well attached to the Solohill microcarriers.
The results of flow cytometry detection for surface markers were shown in the table below, which indicated that culturing M cells on Solohill microcarriers did not affect their marker expression.

Table 6-4: Flow cytometry detection results of M cells cultured on Solohill

| Marker | Sigma Solohill |
|---|---|
| CD34 | 0.10% |
| CD90 | 99.63% |
| CD105 | 99.88% |
| CD73 | 99.96% |
| CD11b | 0.12% |
| HLA-ABC | 99.05% |
| HLA-DR | 0.02% |
| CD19 | 0.22% |
| CD29 | 99.24% |
| CD45 | 0.19% |

The results of Solohill in-situ cryopreservation were shown in FIG. 69, and death phenomenon was observed after Solohill in-situ cryopreservation.

V. M cells cultured on Coring polystyrene microcarriers:

1. Experimental steps:

(1) Cell inoculation:

a. The P4 generation M cells cultured with T225 were digested: the supernatant was discarded, 10ml of DPBS was added to wash once, 10ml of TrypLE was added, digested at 37°C for 3min, added with 10ml of DPBS to stop the reaction, transferred to a 50ml centrifuge tube; centrifuged at 1500rpm for 5min; the supernatant was discarded, the cell pellet was resuspended with culture medium, and the counting was performed with trypan blue;

b. Centrifugation was performed at 1500rpm for 5min, the supernatant was discarded, and the cell suspension was resuspended to a density of $4 \times 10^6$ cells/ml.

c. 40mg of microcarriers were weighed, sterilized, placed in a 10cm low-attachment dish, added with 12ml of culture medium; 200ul of the above cell suspension was inoculated on each microcarrier, incubated at 37°C for 2h;

d. On the 1st day, the medium was replenished to reach 16ml, and then the culture medium was changed every other day: 8ml of culture medium was discarded, and 8ml was added.

(2) Live-dead detection:

a. The live-dead detection solution was prepared in the dark: 10ml of DPBS was taken and placed in a 15ml centrifuge tube, added with 5ul of Calcein AM and 20ul of ethidium homodimer-1 respectively, mixed well and stored at 4°C;

b. The microcarriers were gently mixed, 1ml of the suspension was taken out and placed in a centrifuge tube, allowed to settle for 1 to 2 minutes, the supernatant was discarded, and DPBS was added to wash twice;

c. The live-dead detection solution was added to each tube, 1ml/tube, and incubated at room temperature for 30min;

d. The supernatant was discarded, DPBS was added to wash once, and photos were taken with a fluorescence microscope.

(3) Cell digestion:

a. The microcarriers were naturally precipitated for 1 to 2 minutes, the supernatant was discarded, and washing was carried out with DPBS;

b. 5ml TrypLE/10cm dish was added, digested at 37°C for 10min, added with 5ml of DPBS, transferred to a 50ml centrifuge tube, and filtered with a 70um filter to collect cells;

c. Centrifugation was carried out at 1200rpm for 3min, and the supernatant was discarded;

d. After resuspending with culture medium, trypan blue staining was performed for counting;

e. An appropriate amount of the cells was taken, and centrifuged at 1200rpm for 3min to collect the cells;

(4) Flow cytometry:

a. After the cell pellet was resuspended in 2% BSA, the cells were blocked for 30min;

b. Centrifugation was carried out at 1200rpm for 3min, and the supernatant was discarded;

c. After resuspending in 1% BSA, the cells were subpackaged, 200ul/tube;

d. After adding antibodies according to the antibody instructions, the incubation was carried out at room temperature for 30 minutes;

e. Washing was performed twice with DPBS;

f. After resuspending in DPBS, the cells were filtered with a 0.22um filter;

g. After loading, the detection was performed.

(5) In-situ cryopreservation:

a. The harvested P4 generation M cell generation was harvested and inoculated at $8 \times 10^5$, incubated at 37°C for 2h, and replenished with medium;

b. After culturing at 37°C for 24 hours, the medium was replenished and changed: 3ml of culture medium was discarded, and 3ml of fresh medium was added; subsequently, the culture medium was changed every other day: 4ml of culture medium was discarded and 4ml of fresh medium was added.

c. Sampling for live-dead detection were performed before cryopreservation to detect cell status, and cryopreservation was carried out after 5 days of culture;

d. Preparation of cryopreservation solution MZJ: 20ml = 13ml of compound electrolyte solution + 5ml of 20%HSA + 2ml of DMSO;

e. The microcarriers were washed twice with DPBS respectively, the Coring polystyrene microcarriers were divided into 2 equal parts, the supernatant was discarded, and the residue was cryopreserved with MZJ, CS10, respectively; placed in programmed cryopreservation boxes, and transferred to liquid nitrogen for storage after 24 hours;

f. After cryopreservation in liquid nitrogen for 15 days, the Coring polystyrene microcarriers were taken out from the liquid nitrogen, a resuscitator was used for resuscitation, the culture solution was added to wash twice, it was then transferred to 6-well plate with 2ml of culture solution/well, and cultured at 37°C;

g. After about 30min to 1h, a part thereof was taken and used for live-dead detection, in which the part was observed under a fluorescence microscope, and photos were taken;

2. Experimental results:

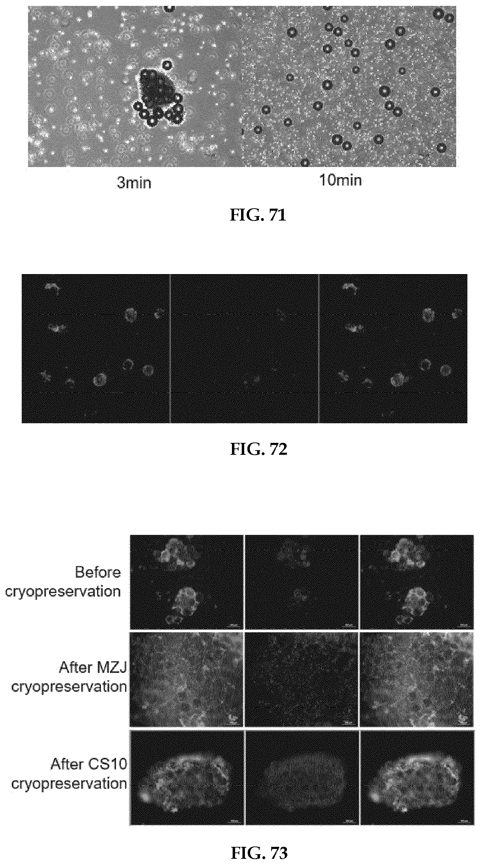

FIG. 70 showed the morphological photos of the M cells cultured in Coring polystyrene, which indicated that the M cells could be cultured and proliferated on Coring polystyrene microcarriers. FIG. 71 showed the morphological photos of the M cells cultured in Coring polystyrene after digestion, which indicated that the M cells could be harvested by TrypLE digestion.

The live-dead detection results were shown in FIG. 72, which indicated that the M cells could be well attached to the Coring polystyrene microcarriers.

The results of flow cytometry detection for surface markers were shown in the table below, which indicated that culturing M cells on Coring polystyrene microcarriers did not affect their marker expression.

Table 6-5: Flow cytometry detection results of M cells cultured on Coring polystyrene microcarriers

| Marker | Coring |
| --- | --- |
| CD34 | 0.05% |
| CD90 | 99.39% |
| CD105 | 99.80% |
| CD73 | 99.93% |

FIG. 73 showed the detection results of Coring polystyrene in-situ cryopreservation, which indicated that there was death phenomenon after Coring polystyrene in-situ cryopreservation, and the effect of MZJ cryopreservation was relatively good.

VI. M cells cultured on microcarriers as prepared by 8GeL-toluene method

The 8GeL-toluene method was a double-emulsion method, in which the main material components were 8% Gelatin, 5% Tween and 5% toluene, gelatin was used as raw material, and macroporous gelatin microcarriers were prepared by suspending into spheres and making pores by toluene. The weak links between toluene droplets were broken up mainly by mechanical stirring and removing the toluene droplets, so as to form connectivity of pores.

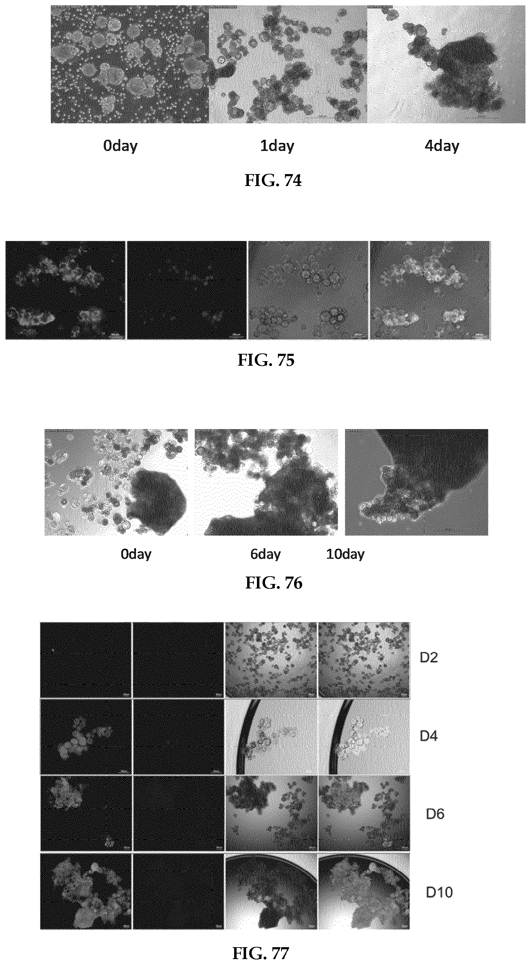

For the M cells cultured on the microcarriers prepared by 8GeL-toluene method, the photos of cell morphology were shown in FIG. 74, which indicated that the M cells could be cultured and proliferated on the microcarriers prepared by 8GeL-toluene method. The live-dead detection results were shown in FIG. 75, which indicated that the M cells could be well attached to the microcarriers prepared by 8GeL-toluene method. FIG. 76 showed the passaging of M cells cultured on the microcarriers prepared by 8GeL-toluene method, and the results showed that the M cells cultured on the microcarriers prepared by 8GeL-toluene method could be passaged and proliferated. FIG. 77 showed the live-dead detection of the M cells cultured with microcarriers prepared by 8GeL-toluene method.

VII. M cells cultured with 25GF-Gel microcarriers

The 25GF-Gel microcarriers were porous microspheres mainly made of gelatin-ferulic acid and Gelatin.

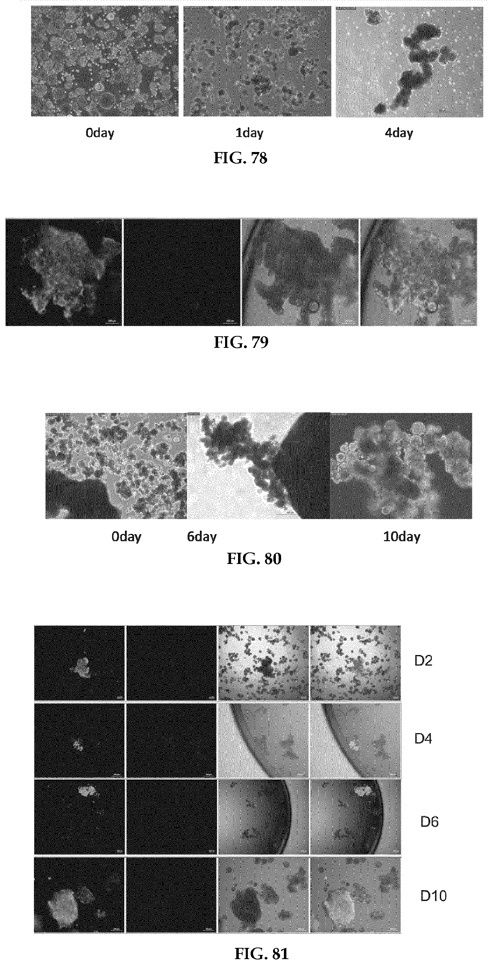

FIG. 78 showed the morphological photos of M cells cultured with 25GF-Gel microcarriers. FIG. 79 showed the results of live-dead detection of the M cells cultured with 25GF-Gel microcarriers. FIG. 80 showed the passage of the M cells cultured with microcarriers prepared from 25GF-Gel microcarriers. FIG. 81 showed the live-dead detection results of the passaging of M cells cultured with 25GF-Gel microcarriers. The M cells could be cultured and proliferated with 25GF-Gel microcarriers.

VIII. M cells cultured with Gel microcarriers

The Gel microcarriers were non-porous microspheres mainly made of gelatin by emulsification method.

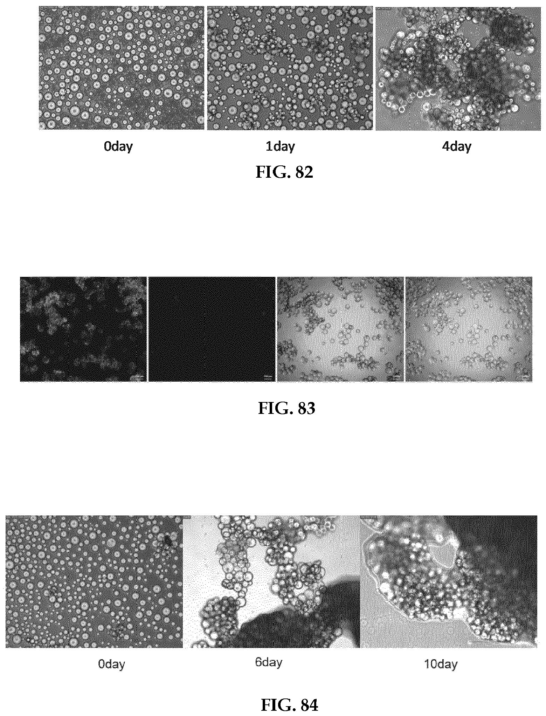

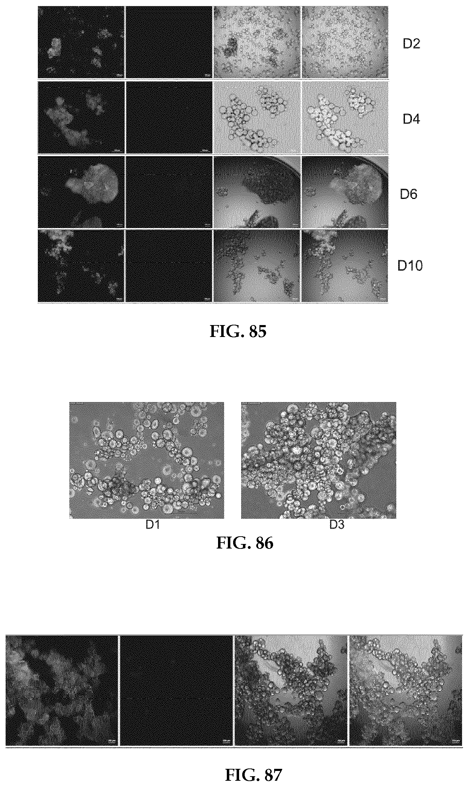

FIG. 82 showed the morphological photos of the M cells cultured with the Gel microcarriers. FIG. 83 showed the results of live-dead detection of the M cells cultured with the microcarriers prepared with Gel. The M cells could be cultured and proliferated with the Gel microcarriers. FIG. 84 showed the passaging of the M cells cultured with the Gel microcarriers. FIG. 85 showed the live-dead detection results of the passaging of the M cells cultured with the Gel microcarriers. The M cells cultured with Gel microcarriers could be passaged sphere-to-sphere and proliferated.

IX. M cells cultured with 25GF-2HA microcarriers

The 25GF-2HA microcarriers were porous microspheres mainly made of gelatin-ferulic acid and hyaluronic acid.

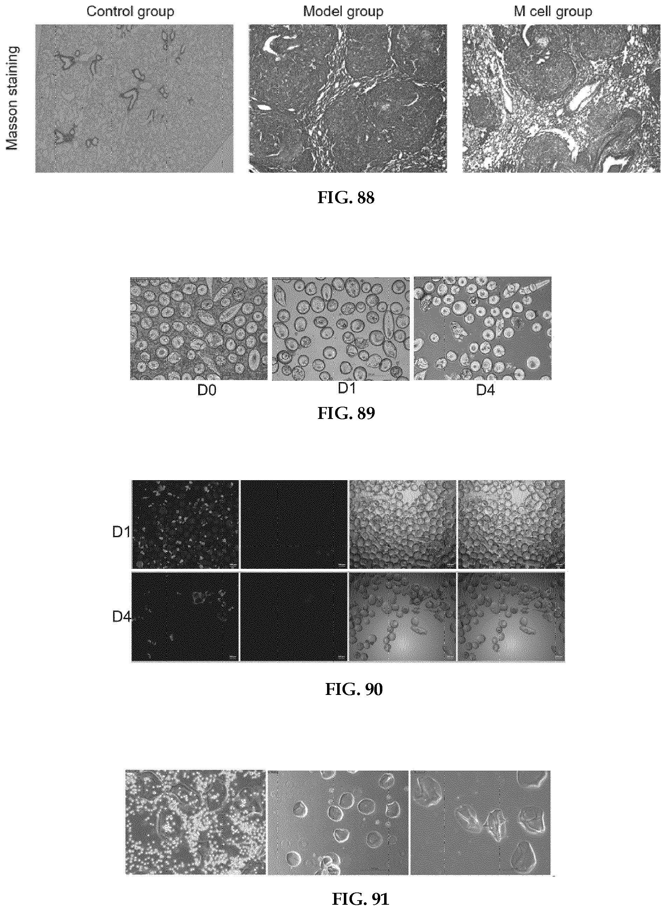

FIGS. 86 and 89 showed the morphological photos of the M cells cultured with 25GF-2HA microcarriers. The live-dead detection results were shown in FIG. 87 and FIG. 90. The M cells could not be attached to the 25GF-2HA microcarriers.

X. M cells cultured with Alg microcarriers

The Alg microcarriers were non-porous microspheres mainly made of sodium alginate.

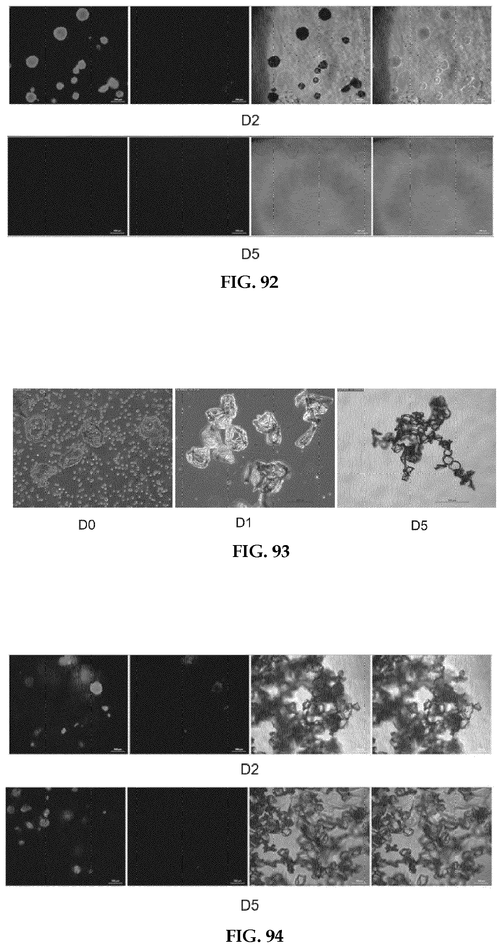

FIG. 91 showed the morphological photos of the M cells cultured with Alg microcarriers, and FIG. 92 showed the results of live-dead detection. The M cells could not be attached to the Alg microcarriers.

XI. M cells cultured with Alg-lysine microcarriers

The Alg-lysine microcarriers were non-porous microspheres mainly made of sodium alginate and lysine.

FIG. 93 showed the morphological photos of the M cells cultured with Alg-lysine microcarriers, and FIG. 94 showed the results of live-dead detection. M cells could not be attached to Alg-lysine microcarriers.

XII. M cells cultured with Gel-lysine microcarriers

The Gel-lysine microcarriers were non-porous microspheres mainly made of gelatin and polylysine.

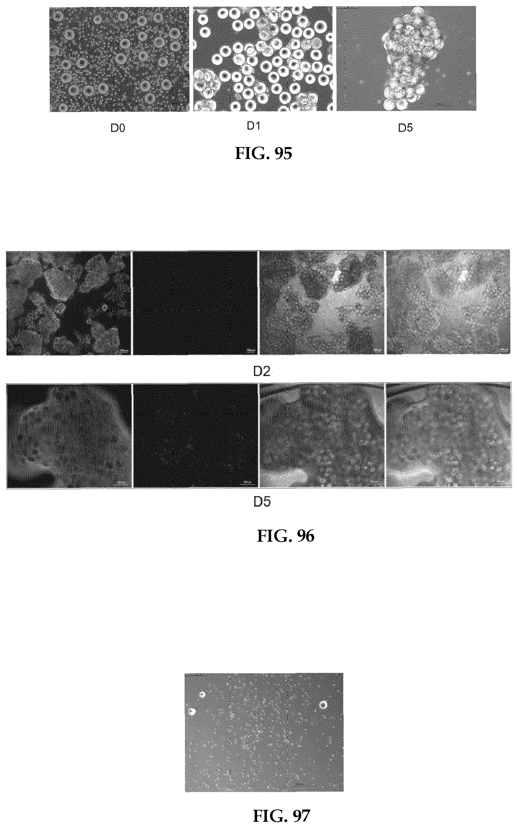

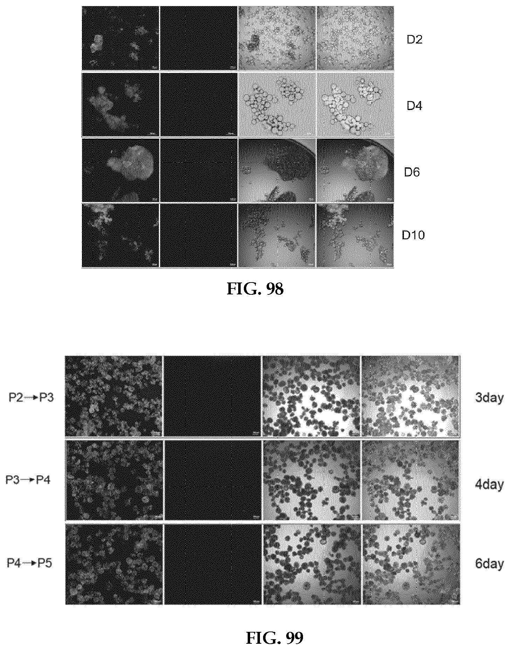

FIG. 95 showed the morphological photos of the M cells cultured with Gel-lysine microcarriers, and FIG. 96 showed the results of live-dead detection. M cells could not attach to the Gel-lysine microcarriers. FIG. 97 showed the results of digestion of M cells cultured with the microcarriers prepared from Gel-lysine microcarriers, which indicated that the M cells cultured with the microcarriers prepared from Gel-lysine microcarriers could be digested with TrypLE. FIG. 98 showed the results of live-dead detection of passaging of the M cells with Gel microcarriers .

XIII. M cells cultured with 16GeL-6HA- bubbles microcarriers

The 16GeL-6HA-bubbles microcarriers were porous microspheres mainly made of gelatin and hyaluronic acid, in which the pores were mainly caused by the gases generated by heating $NH_4HCO_3$.
The morphological photos of the M cells cultured with the 16GeL-6HA-bubbles lysine microcarriers were shown in the figure, and the results of live-dead detection were shown in the figure. The M cells were less attached to the 16GeL-6HA-bubbles microcarriers.

XII. Preparation of M cells by dynamic culture (porous microcarriers, non-porous/microporous microcarriers):

1. Experimental method:

(1) Dynamic culture:

a. The P2 generation M cells cultured with T225 were digested: the supernatant was discarded, 10ml of DPBS was added to wash once, 10ml of TrypLE was added, digestion was carried out at 37°C for 3min, 10ml of DPBS was added to stop the reaction, it was transferred to a 50ml centrifuge tube; centrifugation was performed at 1500rpm for 5min; the supernatant was discarded, the cell pellet was resuspended with culture medium, and counting was carried out with trypan blue;

b. 35ml of culture medium was added to a spinner flask, 2 pieces of TableTrix microcarriers were added, dissolution was performed for 10min, inoculation was performed at an amount of $1.6\times10^6$ cells, and dynamically culture was carried out at 37°C;

c. On the second day of culture, 30ml of supernatant was discarded and 40ml of culture medium was added;

d. On the third day of culture, 35ml of supernatant was discarded and 50ml of culture medium was added;

e. On the 4th day of culture, 45ml of supernatant was discarded and 60ml of culture medium was added;

f. On the 5th day of culture, 30ml of supernatant was discarded, the microcarriers were transferred to a 50ml centrifuge tube, and centrifuged at 1500rpm for 5min;

g. The supernatant was discarded, 10ml of lysis solution was added to lyse for 40 to 50min, pipetting was performed once in the middle, and centrifugation was carried out at 1500rpm for 5min;

h. The supernatant was discarded, 6ml of TrypLE was added to perform digestion at 37°C for 3min, 6ml of DPBS was added to stop the digestion, and centrifugation was carried out at 1500rpm for 5min;

i. The supernatant was discarded, 2ml of culture medium was added to resuspend, and counting was performed;

j. Inoculation and passaging were performed at $8\times10^5$/piece to P4 generation;

k. On the second day of culture, 20ml of culture medium was added;

l. On the third day of culture, 20ml of supernatant was discarded and 30ml of culture medium was added;

m. On the 4th day of culture, 40ml of supernatant was discarded and 50ml of culture medium was added;

n. On the 5th day of culture, 50ml of supernatant was discarded and 100ml of culture medium was added;

o. On the sixth day of culture, the supernatant was discarded, the microcarriers were transferred to a 50ml centrifuge tube, and centrifugation was carried out at 1500rpm for 5min;

p. The supernatant was discarded, 10ml of lysis buffer was added to lyse for 40 to 50min, pipetting was performed once in the middle, and centrifugation was carried out at 1500rpm for 5min;

q. The supernatant was discarded, 6ml of TrypLE was added to perform digestion at 37°C for 3min, 6ml of DPBS was added to stop the digestion, and centrifugation was carried out at 1500rpm for 5min;

r. The supernatant was discarded, the culture medium was added to resuspend, and counting was performed;

s. Inoculation and passaging were performed at $8 \times 10^5$/piece to P5 generation;

t. On the second day of culture, 20ml of culture medium was added; on the third day of culture, 40ml of supernatant was discarded and 50ml of culture medium was added; on the fourth day of culture, 55ml of supernatant was discarded and 65ml of culture medium was added; on the fifth day of culture, 60ml of supernatant was discarded and 150ml of culture medium was added; on the 6th day of culture, the supernatant was discarded, the microcarriers were transferred to a 50ml centrifuge tube, and centrifuged at 1500rpm for 5min;

u. The supernatant was discarded, 16ml of lysis solution was added to lyse for 40 to 50min, pipetting was performed once in the middle, and centrifugation was carried out at 1500rpm for 5min;

v. The supernatant was discarded, 10ml of TrypLE was added to perform digestion at 37°C for 3min, 10ml of DPBS was added to stop the digestion, and centrifugation was carried out at 1500rpm for 5min;

w. The supernatant was discarded, 4ml of culture medium was added to resuspend, and counting was performed.

(2) Flow cytometry:

a. 2%BSA was added for blocking for 30min;

b. Centrifugation was carried out at 1200rpm for 3min, and the supernatant was discarded;

c. After being resuspended with 1%BSA, it was subpackaged, 200ul/tube, a total of 8 tubes;

d. Antibodies were added according to the antibody instructions, and incubated at room temperature for 30 minutes;

e. Washing was performed twice with DPBS;

f. After being resuspended DPBS, filtration was performed with 0.22um filter;

g. After being loaded, the detection was carried out.

(3) Live-dead detection:

a. The live-dead detection solution was prepared in the dark: 10ml of DPBS was taken and placed in a 15ml centrifuge tube, added with 5ul of Calcein AM and 20ul of ethidium homodimer-1 respectively, mixed well and stored at 4°C;

b. About 200ul to 500ul of the microcarrier suspension was taken out from the spinner flask, and placed in a centrifuge tube, allowed to settle for 1 to 2min, the supernatant was discarded, DPBS was added to wash twice; it was then transferred to a 96-well plate, and the supernatant was discarded;

c. The live-dead detection solution was added, 1ml/tube, and incubation was carried out at room temperature for 30min;

d. The supernatant was discarded, DPBS was added to wash once, photos were taken with a fluorescence microscope.

2. Experimental results:

2.1 Preparation of M cells by dynamic culture with porous microcarriers:

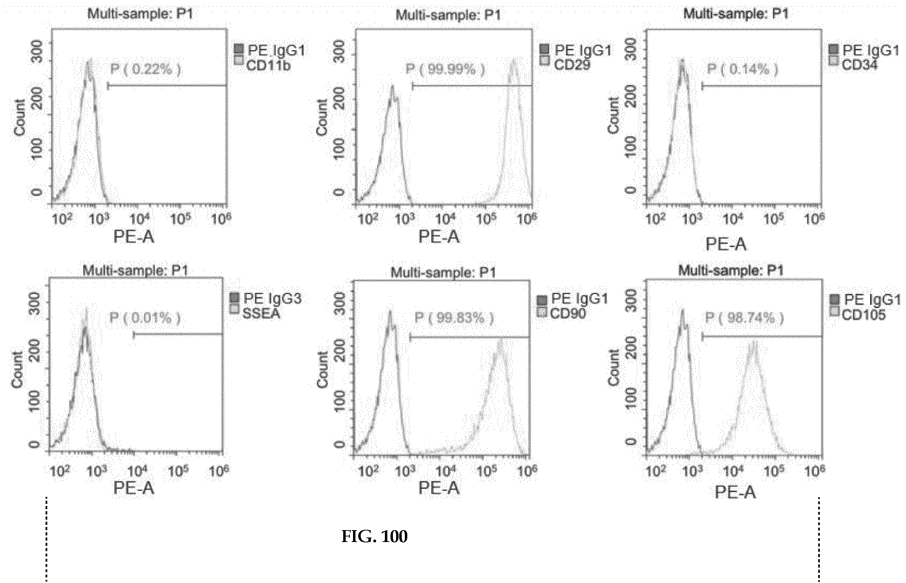

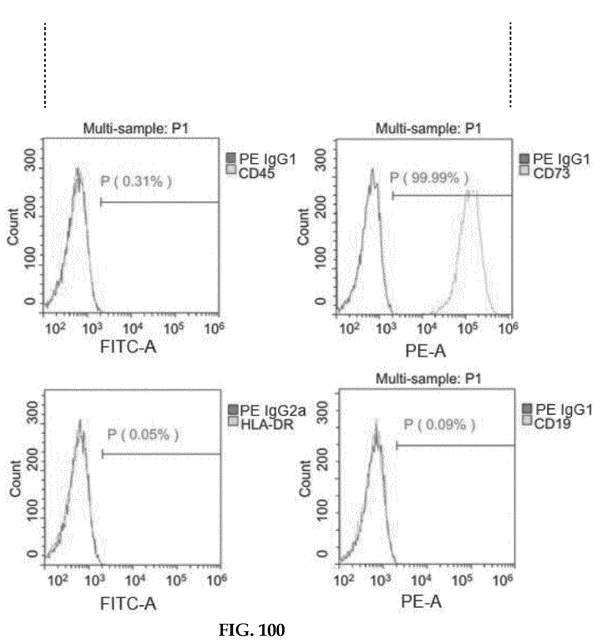

The results of live-dead detection were shown in FIG. 99, which indicated that the M cells could be well attached to the TableTrix microcarriers. The flow cytometry results of surface markers were shown in the following table and FIG. 100, which indicated that culturing the M cells with TableTrix microcarriers did not affect their marker expression. The human embryonic stem cell-derived M cells could be dynamically cultured and prepared on the porous microcarriers, and the expression of markers was normal.

Table 6-6: Flow cytometry results of M cells cultured with TableTrix microcarriers

| Marker | Detection result |
|---|---|
| CD34 | 0.15% |
| SSEA-4 | 0.04% |
| CD90 | 97.78% |
| CD105 | 98.15% |
| CD73 | 99.97% |
| CD11b | 0.30% |
| HLA-DR | 0.05% |
| CD19 | 0.09% |
| CD29 | 99.98% |
| CD45 | 0.23% |

2.2 Preparation of M cells by dynamic culture with non-porous or microporous microcarriers:

The morphology and adhesion of the cells were good when observed under microscope;

The results of live-dead detection showed that the adhesion rate and growth state of the M cells were good;

Scanning electron microscope was used to observe the cell attachment morphology;

Passage method verification: both of enzyme passage and sphere-passage could be achieved, and meet the requirements;

The in-situ cryopreservation of M cells was performed to indicate whether the microcarriers were suitable for in-situ cryopreservation;

Cryopreservation after digestion: it was used to verify the changes in cell viability after the cells cultured with the microcarriers were cryopreserved;

The results of flow cytometry showed that the microcarriers themselves had no effect on the cell characteristics;

Quality inspection: it indicated that the detection of cell viability, mycoplasma, endotoxin, sterility, virus, etc. all met the standard;

The results of RNA-seq or single-cell sequencing showed the differences in pros and cons of the M cells harvested by culture or differentiation with the microcarriers as compared with the cells harvested by 2D carriers.

Human embryonic stem cell-derived M cells could be dynamically cultured and prepared on the non-porous or microporous microcarriers, and the expression of Marker was normal.

Example 7: Evaluation of therapeutic activity of M cells on lung cell fibrosis

The human lung fibroblasts HFL1 (purchased from Beina Bio) were inoculated in a 6-well plate, and when the cell fusion reached 70%, they were treated according to the following groups: (1) adding basal medium (HF12K + 10% FBS); (2) adding basal medium + 10ng/ml TGF-β1 (purchased from Peprotech, Cat. No. 100-21); (3) adding 50% basal medium + 50% MSC culture supernatant of Preparation Example 1 + 10ng/ml TGF-β1, in which the method for obtaining the culture supernatant was as follows: $1\times10^6$ MSCs were inoculated in a 10cm dish, when it reached 50%, the medium was changed to 10mL of fresh medium, and after culturing for 24 hours, the supernatant was collected, and centrifuged at 1200rpm for 3 minutes, and the supernatant was taken.

Subsequently, the expression of α-SMA (anti-α-SMA antibody: Sigma, A5228) and type I collagen (anti-Collagen I antibody: CST, 84338) in the cells treated with the above different groups was analyzed by western blot.

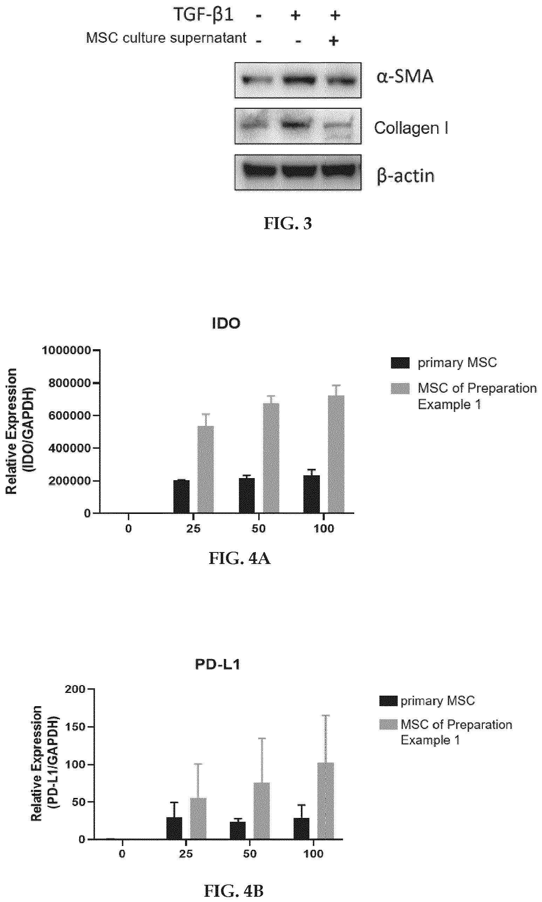

The results were shown in FIG. 3, which indicated that the treatment with TGF-β1 led to the increased expression levels of α-SMA and type I collagen in lung fibroblasts, while the treatment with MSC culture supernatant could significantly reduce the expression levels of α-SMA and Collagen I. The above results suggested that the M cell culture supernatant of the present invention could inhibit pulmonary fibrosis.

Example 8: Evaluation of anti-inflammatory activity of M cells

The primary MSCs and the MSCs obtained in Preparation Example 1 were inoculated in a 6-well plate, and when the cells reached 70% to 80% aggregation, they were treated for 24h by adding IFN-γ at different concentrations (0, 25, 50, 100ng/ml), and then the mRNA expression levels of IDO, PD-L1 and PGE2 were detected by RT-qPCR. The results shown were the average of three replicate experiments.

The detection results of IDO were shown in FIG. 4A and Table 8-1, the detection results of PDL1 were shown in FIG. 4B and Table 8-2, and the detection results of PGE2 were shown in FIG. 4C and Table 8-3. The results showed that after stimulation with IFN-γ, the expression levels of IDO, PD-L1 and PGE2 in the M cells of the present invention were significantly higher than those in the primary MSCs. The above results suggested that the M cells of the present invention had better immune regulation function and anti-inflammatory activity.

Table 8-1: Detection of mRNA expression level of IDO

| IFN-γ (ng/ml) | Primary MSC | M cells of Preparation Example 1 | Fold change compared to primary MSC |
|---|---|---|---|
| 0 | 1.07 | 119.53 | 111.67 |
| 25 | 203001.00 | 535208.47 | 2.64 |
| 50 | 214784.70 | 674937.10 | 3.14 |
| 100 | 232771.60 | 723105.07 | 3.11 |

Table 8-2: Detection of mRNA expression level of PD-L1

| IFN-γ (ng/ml) | Primary MSC | M cells of Preparation Example 1 | Fold change compared to primary MSC |
|---|---|---|---|
| 0 | 1.01 | 0.11 | 0.11 |
| 25 | 29.98 | 55.95 | 1.87 |
| 50 | 23.55 | 76.06 | 3.23 |
| 100 | 29.41 | 102.54 | 3.49 |

Table 8-2: Detection of mRNA expression level of PEG2

| IFN-γ (ng/ml) | Primary MSC | M cells of Preparation Example 1 | Fold change compared to primary MSC |
|---|---|---|---|
| 0 | 0.90 | 74.98 | 83.10 |
| 25 | 1.54 | 50.53 | 32.72 |

(continued)

| IFN-γ (ng/ml) | Primary MSC | M cells of Preparation Example 1 | Fold change compared to primary MSC |
|---|---|---|---|
| 50 | 1.06 | 74.23 | 69.75 |
| 100 | 1.38 | 75.40 | 54.63 |

Example 9: Evaluation of therapeutic activity of M cells against intrauterine adhesions

Endometrial injury could result in endometrial fibrosis, partial or complete obstruction of uterine cavity, which in turn causes oligomenorrhea, amenorrhea, infertility or recurrent miscarriage, which happened in patients with secondary amenorrhea, with female infertility, and with curettage after miscarriage . In recent years, due to the frequent uterine cavity operation and the popularization of hysteroscopic surgery, the incidence and detection rate have gradually increased, and the age of onset has become younger, and it has become the second leading cause of female secondary infertility. Although clinicians continue to seek new treatment options, its cure rate and pregnancy rate are still not significantly improved, and the recurrence rate is relatively high (for patients with mild conditions, the recurrence rate after treatment is ; for patients with severe conditions, the recurrence rate after treatment is up to ), and obstetric complications such as infertility, recurrent miscarriage, premature birth, placenta previa, placenta adhesion or implantation are a serious threat to women's reproductive health. Its high incidence rate and the resulting damage to women's reproductive function have become an urgent clinical problem to be solved. The current clinical treatment aims to restore the shape of uterine cavity, prevent the recurrence of adhesions, promote the repair and regeneration of the damaged endometrium, and restore normal reproductive function. The important steps of treatment are hysteroscopic separation of uterine adhesions, intraoperative placement of intrauterine device, and postoperative application of estrogen and progesterone, but there are problems such as long treatment cycle, low cure rate, easy recurrence of adhesions, low pregnancy rate, high-dose estrogen application-increased risk of breast and endometrial tumors in patients, and due to severe muscular or connective damage in endometrial basal layer, there are poor responses to estrogen and progesterone.

So far, the scholars at home and abroad have carried out a lot of research on the pathogenesis of the disease, and it is agreed that the disorder of endometrial repair may be the main mechanism of its formation. For example, due to curettage after abortion or other uterine cavity operations, some pathological factors hinder the endometrial repair, resulting in scar formation and adhesions.

The present invention overcomes the problems such as the serious endometrium injuries caused by mechanical separation of intrauterine adhesions in hysteroscopic surgery, postoperative placement of intrauterine device or anti-adhesion material, postoperative administration of estrogen to promote endometrium growth, and endometrium scars which cannot be repaired, as well as the defects such as impossible of functional repair of endometrium, and recurrence of adhesions.

Experimental animals: SD rats, female, 8 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: the embryonic stem cells were suspended to form EB spheres, and adherent differentiation was carried out, the M cells of PO generation were obtained, passaged and screened, and cryopreserved at P3 generation for subsequent experiments.

The M cells of P3 generation were resuscitated, digested and passaged, and used at the P5 generation for subsequent experiments.

| Reagent/Equi pment | Manufacturer | Cat. No. |
|---|---|---|
| Electronic scale | domestic | 1000212 |
| Embedding machine | Leica | EG1150H/C |
| Sectioning machine | Leica | RM2235 |
| Section displaying machine | Leica | HI1210 |

(continued)

| Reagent/Equi pment | Manufacturer | Cat. No. |
|---|---|---|
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Ethanol | Aladdin | E111992-500ml |
| DMSO | Sigma | D2650-100ML |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |
| 5-0 surgical suture | Stones | EB01 |
| 3-0 surgical suture | Stones | EB03 |

Animal modeling: After SD rats were anesthetized, a small incision was cut in the abdomen, the uterus left was exposed with forceps, the uterus was clamped with two hemostatic forceps (4 cm apart), and 100μl of 95% ethanol was injected for treatment for 5 minutes, followed by rinsing with saline three times, 3 minutes for each time. After the modeling was completed, random grouping was performed on the 7th day, and perfusion sampling and analysis was performed on the 28th day.

Grouping: normal group, model group, M cell group, 5 mice in each group.

Normal group: not treated.

Model group: subjected to modeling with 95% ethanol on the 0th day, injection with 100 μl of normal saline on the 7th day, and perfusion sampling and analysis on the 28th day.

M cell group: subjected to modeling with 95% ethanol on the 0th day, injection with 100 μl of normal saline containing $3 \times 10^6$ M cells on the 7th day, and perfusion sampling and analysis on the 28th day.

Sample collection:

When collecting the specimens, after the rats were subjected to intraperitoneal anesthesia, the rats were in a supine position, the skin was cut in the middle of abdomen, the chest was opened, the heart was exposed, and the heart was perfused with ice-cold normal saline. Each rat needed about 50 ml of saline. After the saline perfusion was completed, the uterus was fixed with 50 ml of paraformaldehyde. After the perfusion was completed, the uterus was fixed with paraformaldehyde, and the sections were analyzed.

Steps for tissue paraffin sectioning:

(1) Fixation: the tissue was socked in 4% PFA overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

Preclinical pharmacodynamic evaluation of M cells on intrauterine adhesions

SD rats (purchased from Weitong Lihua) were anesthetized, a small incision was cut in the abdomen, and the uterus left was exposed with forceps. The uterus was clamped with two hemostatic forceps (4 cm apart), injected with 100 μL of 95% ethanol (purchased from Aladdin, Cat. No. A298792), and after five minutes, rinsed with 100 μL of normal saline three times, 3 minutes each time, the wound was sutured, and the modeling was completed. On the 7th day after modeling, the model rats were randomly divided into three groups: normal group, model group and M cell group. The normal group was not treated, the model group was injected with 100 μL of normal saline on the 7th day after modeling, and the M cell group was injected with $3 \times 10^6$ M cells/100 μL of normal saline after modeling. Some animals were caged with male rats on the 14th day after modeling, and were continuously observed for 42 days. The number of fetal rats born in each group was counted, and 35-day-old offspring rats were selected for the relevant safety inspection. Some animals were perfused on the 28th day after modeling, and uterus thereof was taken for photographing to observe the morphological changes of uterus. Paraffin tissue sections were stained with HE to observe the structural changes of the endometrium.

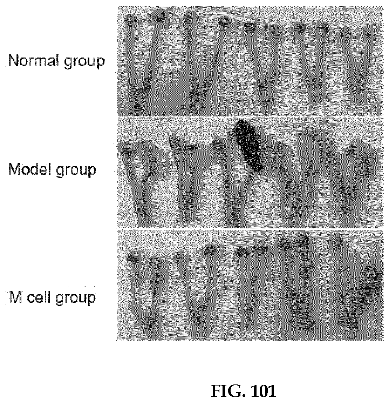

FIG. 101 shows the results of the light microscope photos of uterus samples. After 95% ethanol was injected into the uterine cavity, the rat uterus had severe adhesions, the uterine cavity was blocked, and a large amount of uterine effusion appeared. The intrauterine effusion was retained in uterus and formed transparent uterine effusion bubbles. The results showed that the rat model of intrauterine adhesions was successfully induced. The M cell treatment significantly inhibited intrauterine adhesions, significantly reduced intrauterine effusion, and significantly reduced the volume and number of uterine effusion bubbles, and the shape of uterine cavity basically returned to normal. The above results suggested that the M cells of the present invention could significantly improve the shape of uterus and inhibit intrauterine adhesions.

The results of uterus HE staining were shown in FIG. 102. After modeling with 95% ethanol, the endometrium of rat became thinner, the glands disappeared, and the blood vessels were sparse. The results showed that the intrauterine adhesion model was successfully built. After the M cell treatment, the endometrium and myometrium were significantly thickened, and the number of new blood vessels and glands increased significantly. The above results suggested that the M cells of the present invention could promote the repair and regeneration of damaged endometrium.

The results showed that after the M cell treatment, the number of progenies increased significantly, and the number increased by 1.2 times compared with the model group. The 35-day-old offspring rats were tested, and the results showed that the offspring had normal growth status and no obvious growth defects. The above results suggested that the M cells of the present invention could treat intrauterine adhesions, promote the repair and regeneration of damaged endometrium, enhance the ability to reproduce offspring, and have no effect on the growth and development of offspring.

Clinical pharmacodynamic evaluation of M cells on intrauterine adhesion

The patient was diagnosed with moderate-severe intrauterine adhesions. The patient's endometrial volume, endometrial thickness and shape, and scar area were evaluated preoperatively. Hysteroscopy was performed under general anesthesia and ultrasonography guidance. The shape of uterine cavity was observed by hysteroscopy. Under the guidance of B-ultrasonography and direct vision under hysteroscopy, blunt dissection of adhesions was performed through dilation rods or water sacs, supplemented with sharp dissection by micro-scissors or resectoscope (as far as possible to avoid). Under ultrasonography guidance, $3\times10^6$ M cells in suspension were injected into the junction of endometrium and myometrium with a 21G syringe needle.

The clinical treatment results of the M cells on intrauterine adhesions were shown in FIG. 103. The patient had moderate-severe intrauterine adhesions, with muscle adhesion at the bottom of the uterus and formation of scars. The fallopian tube was obstructed and the opening was not obvious. After the M cell injection treatment to uterine wall, the patient's uterine shape returned to normal, no adhesion was found, the scar was repaired, and the bilateral fallopian tube openings were visible. The results suggested that the M cells could be used for clinical treatment of intrauterine adhesions. The figure showed that (1) the patient had intrauterine adhesions and obstruction at bilateral fallopian tubes at the time of surgery, and the adhesions were moderate-severe (mixed type); the M cells ($3\times10^6$ cells) were injected after separation of adhesion; (2) after 4 weeks of follow-up, it was found that the scars were recovered; (3) during the 4-month follow-up, the condition of the uterine cavity was poor; and (4) during the 8-month follow-up, the uterine cavity basically returned to normal.

The patient's endometrial thickness was significantly increased, and the thickness was $\geq7$ mm. The patient had a successful pregnancy and delivered a healthy offspring. The patient's menstruation returned to normal. The above results suggested that the M cells could restore fertility in patients with intrauterine adhesions without affecting the growth and development of offspring.

Example 10: Evaluation of therapeutic activity of M cells against acute liver injury

Experimental animals: C57 mice, male, 6 to 7 weeks old, purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2$°C, relative humidity was 50% to 60%.

Preparation and culture of M cells: the embryonic stem cells were suspended with EB spheres, and adherent differentiation was carried out, the M cells of PO generation were obtained, passaged and screened, and cryopreserved at P3 generation for subsequent experiments.

The M cells of the P3 generation were resuscitated, digested and passaged, and used at the P5 generation for subsequent experiments.

| Reagent/Equipment | Manufacturer | Cat. No. |
|---|---|---|
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Water bath | Saiou Huachuang | SDY-1 |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solarbio | G8590-100 |
| Corn Oil | Aladdin | C116025 |
| Carbon tetrachloride (CC14) | Aladdin | C112040 |
| Blood chemistry analyzer | Beckman | AU5800 |
| Centrifuge | Xiangyi | TD25-WS |

(continued)

| Reagent/Equi pment | Manufacturer | Cat. No. |
|---|---|---|
| Electronic Scale | Yasuwang | CC-1013-04 |

Preparation of animal model: carbon tetrachloride (CC14) (3ml/kg, 1:1, dissolved in corn oil) was intraperitoneally injected.

Control group: only the same dose of corn oil was injected;

CC14 group: carbon tetrachloride (CC14) (3ml/kg, 1:1, dissolved in corn oil) was intraperitoneally injected, followed by tail vein injection of normal saline immediately after the CC14 injection;

CC14+M cell group: carbon tetrachloride (CC14) (3ml/kg, 1:1 dissolved in corn oil) was injected intraperitoneally, and $3\times10^6$ cells/mouse were injected into the tail vein immediately after the CC14 injection.

The statistics of mortality was performed on days 0, 1, 2, 3, 4, 5 and 6, and serum was collected on day 7 for blood biochemical analysis.

Sample collection:

When collecting specimens, the mice were intraperitoneally anesthetized, and in a supine position. The skin was cut in the middle of abdomen, the abdominal cavity was opened, and blood was collected from the central vein. The chest was opened, the heart was exposed, and the heart was perfused with ice-cold normal saline. After the normal saline perfusion was completed, it was fixed with 50 mL of paraformaldehyde. After the perfusion was completed, the lungs were taken for fixed section analysis. The collected blood was centrifuged at 5,000 rpm for 15 min at room temperature, and the supernatant was taken for blood biochemical analysis.

Steps for tissue paraffin sectioning

(1) Fixation: the tissue was socked in 4% PFA and fixed overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene: paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

Statistical analysis

One-way ANOVA and T-TEST in Prism 7.0 statistical analysis software were used for variance analysis and significance test, and the experimental data were expressed as mean $\pm$ standard error (Mean $\pm$ SE). *, $p<0.05$; **, $p<0.01$; ***, $p<0.001$.

Experimental results

(1) After injection of CCl4 and the M cells in each group, the mice were weighed every day. The weight of mice in the CC14 group continued to decrease. The weight loss rate of mice in the CC14+M cell group was significantly lower than that in the CC14 group, indicating that the M cells could reduce the weight loss rate of mice with acute liver injury.

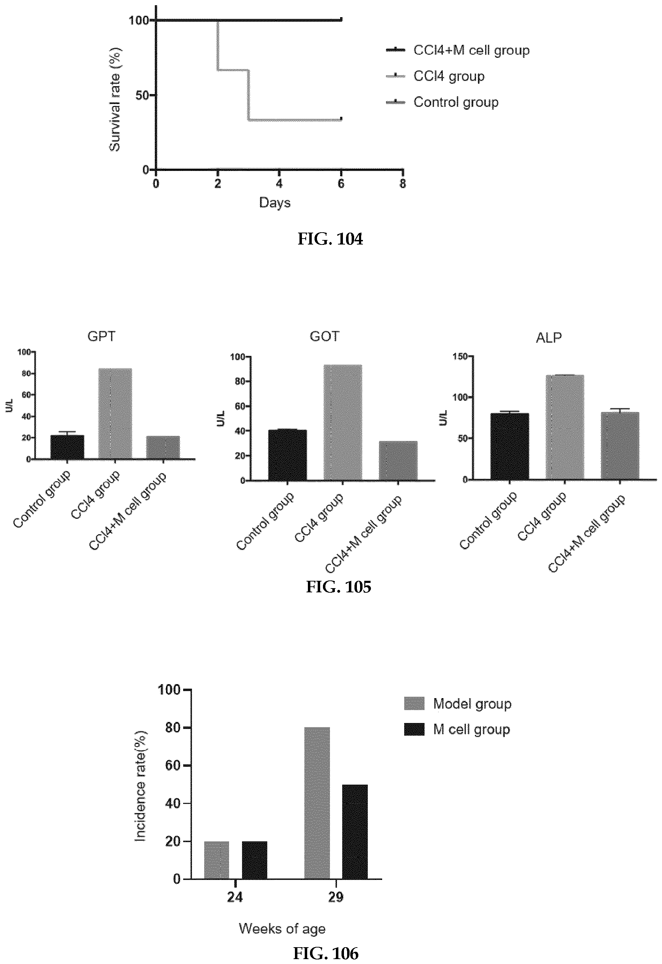

(2) After injection of CC14 and the M cells in each group, the mortality of mice was calculated every day. The results were as follows, within 3 days after the injection of CC14, a large number of deaths were observed in the CC14 group. However, the mice in the CC14+M cell group did not die (the line of the CC14+M cell group was overlapped with that of the control group), indicating that the M cells could reduce the mortality of mice with acute liver injury and could effectively treat acute liver injury (Table 10-1, FIG. 104).

(3) The serums of mice were collected to perform the blood biochemical detection of liver function, and it was found that the levels of alanine aminotransferase, aspartate aminotransferase and alkaline phosphatase in the CC14 group were increased, indicating obvious liver injury. However, the levels in the CC14+M cell group showed no increase, indicating that the M cells could reduce the levels of transaminases and alkaline phosphatase in serum and protect liver function (FIG. 105).

(4) HE staining results showed that a large number of liver cells died and a large number of inflammatory cells infiltrated in the livers of the CC14 group mice. In the CC14+M cell group, there was no large number of liver cell death, nor a large number of inflammatory cells infiltrated in the liver of the mice. It indicated that the M cells could inhibit the injury of CCl4 to hepatocytes and protect the function of hepatocytes.

Table 10-1: Statistics of survival rate of mice in each group

| Survival rate | Control group | CC14 group | CC14+M cell group |
|---|---|---|---|
| Number of mice remaining on day 0 | 6 | 6 | 6 |
| Number of mice remaining on day 1 | 6 | 6 | 6 |
| Number of mice remaining on day 2 | 6 | 4 | 6 |
| Number of mice remaining on day 3 | 6 | 2 | 6 |
| Number of mice remaining on day 4 | 6 | 2 | 6 |
| Number of mice remaining on day 5 | 6 | 2 | 6 |
| Number of mice remaining on day 6 | 6 | 2 | 6 |

Example 11: Evaluation of therapeutic activity of M cells against muscular atrophy

Amyotrophic lateral sclerosis (ALS), commonly known as ALS, is a spontaneous and fatal neurodegenerative disease that affects the upper motor neurons of motor cortex and the lower motor neurons of brainstem and spinal cord. The loss of large numbers of motor neurons results in muscle wasting and spontaneous contraction and spasm. ALS is divided into two categories: familial ALS (FALS) and sporadic ALS (SALS), the former accounts for 10% and the latter accounts for 90%. The onset age of ALS patients is usually after the age of 40, and the high incidence of FALS and SALS occurs at the age of 47-52 and 58-63, respectively, while the incidence decreases after the age of 80, and men are more prone to the disease than women. Patients generally survive 3 to 5 years from the onset of the disease. Various factors are closely related to the incidence of ALS, such as: genetics, occupation, lifestyle, age, etc. On the one hand, the pathogenesis of ALS is that astrocytes fail to restore in time the glutamate accumulated in the synapse, resulting in glutamate excitotoxicity; on the other hand, the mutated genes including SOD1, UBQLN2, OPTN, VCP, TDP43, FUS, C9ORF72 lead to the production of polymers with wrong protein conformation that bring toxicity, and the production of toxic RNA species, which aggravate motor neuron damage, cause synapse retraction and fails to bind to postsynaptic membrane receptor and to complete electrical signal transmission, and finally the clinical manifestations appear. Drug treatments are available for the treatment of ALS. Currently, only two neuroprotective drugs approved by the U.S. Food and Drug Administration (FDA) and the European Medicines Agency (EMA) can extend life for some patients by several months: riluzole, which is able to block excess glutamine neurotransmission; edaravone, which is able to prevent oxidative stress damage; surgical treatment: nasogastric feeding or gastrostomy may be performed if the patient has difficulty in swallowing or masticating. If the respiratory muscles are paralyzed, tracheotomy should be performed as soon as possible, and ventilation should be used to maintain breathing; there is also adjuvant therapy: rehabilitation training. Clinically, there are specific treatment methods corresponding to specific symptoms. The aforementioned treatment methods can only extend the survival time of patients by several months, but do not significantly improve the patient's quality of life. Therefore, there is still an urgent need for more effective treatments. In addition to the above treatment methods, gene editing is currently a hot topic in preclinical research. For example, the direct editing of SOD1 through the CRISPR/Cas9 gene editing system is used to treat amyotrophic lateral sclerosis in vitro and in transgenic mice. However, there are still many uncertainties in gene editing.

Preparation and culture of M cells:

The embryonic stem cells were suspended with EB spheres, and subjected to adherent differentiation, and the M cells of P0 generation were obtained, passaged and screened, and cryopreserved at P3 generation for subsequent experiments.

The M cells at P3 generation were resuscitated, digested and passaged, and P5 was used for subsequent experiments.

Experimental animals:

Male SOD1(G93A) mice (18 weeks old) with C57BL6 background were purchased from Jiangsu Jinzhihe Biotechnology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%. Experiments were started after two weeks of adaptive feeding in mice.

Experimental materials: electronic scale, disposable sterile syringe 1ml

Experimental reagents: saline,

Equipment: mouse rotarod, mouse grip tester

| Consumables/Reagents/Instruments | Manufacturer | Cat. No./Model |
|---|---|---|
| Electronic Scale | Yasuwang | CC-1013-04 |
| Disposable sterile syringe 1ml | Jiangsu Zhiyu Medical Equipment Co., Ltd. | None |
| Normal saline | domestic | |
| Mouse rotarod | Ugo Basile | 47650 |
| Mouse grip tester | Ugo Basile | 47200 |

Experimental groups: normal control group, ALS mice+solvent (solvent group), ALS mice+M cells (M cell group), 3 mice in each group.

Statistics: All data were analyzed by the T-Test in Prism 7.0 statistical analysis software for variance analysis and significance test, and the experimental data were expressed as mean $\pm$ standard deviation (Mean $\pm$ SD). *, $p<0.05$; **, $p<0.01$; ***, $p<0.001$.

Pharmacodynamic evaluation of M cells in amyotrophic lateral sclerosis

The ALS mice were adaptively fed in the animal room for one week after purchase. Behavioral training was performed in the following week. The behavioral training comprised: Rotarod test and Grip strength test. Rotarod experiment: the rotation speed and time of the rotarod: from 5 rpm to 40 rpm, 300 seconds, after which the mice were placed on the rig to adapt for 30 seconds, and then the training was started. This experiment was repeated three times, with an interval of 30 minutes each time. The grip strength experiment was conducted as follows, the mouse was placed on the grid, the mouse tail was caught and dragged back until the mouse escaped from the grid. This experiment was repeated three times with a 30-second interval between each time. After a week of training, the formal experiment began. The body weight of the mice was recorded, and the hindlimb extension reflex was scored at the same time. The mice were grouped according to these two points. After that, drug injection was performed, and the normal control group was left untreated. The ALS mice+solvent group was injected with 200 $\mu$l of normal saline through the tail vein, and the ALS mice+M cell group was injected with 200 $\mu$l of cells through the tail vein: $3\times10^6$/mice. The following monitoring was performed every week: body weight, survival rate, rotarod test, grip strength test, gait analysis. At the end of the experiment, the lumbar spinal cord was harvested by perfusion immediately after the mice were euthanized, frozen, sectioned and embedded, and subjected to immunohistochemical staining.

The statistical results of the incidence rate of ALS mice were shown in FIG. 106. The results showed that the incidence rate of the M cell group was significantly lower than that of the solvent group (50% vs. 80%) in the 27th week, and the M cells significantly delayed the onset in the mice.

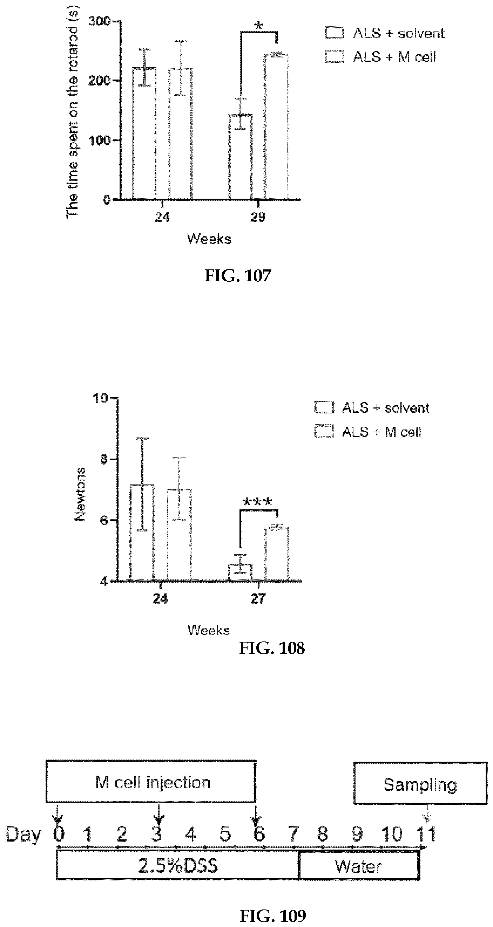

Analysis of the results: The rotarod test could monitor the limb coordination and motor ability of mice, and the grip strength test can monitor muscle strength. In the 24th and 29th weeks, the mice in the solvent group stayed on the rotarod for less time, and their muscle strength was weak, especially in the 29th week. However, the residence time of ALS mice in the M cell group was significantly higher than that in the solvent group, especially in the 29th week (144 vs. 244), with a significant difference, **$p<0.01$ (Table 11-1, FIG. 107); which indicated that the M cells could significantly improve the exercise ability of mice. Moreover, the grip strength of mice in the M cell group was higher than that in the solvent group, and there was a significant difference in the 29th week, ** $p<0.01$ (Table 11-2, FIG. 108); which indicated that the M cells significantly improved the muscle strength of mice, and which indirectly indicated that the M cells could reduce motor neuron damage.

Table 11-1: Statistics of mouse rotarod test

|         | ALS+solvent |        |        | ALS+M cells |        |        |
|---------|-------------|--------|--------|-------------|--------|--------|
| Week 24 | 209.33      | 197.33 | 155.33 | 217.00      | 223.00 | 203.00 |
| Week 29 | 173.00      | 135.00 | 124.67 | 247.67      | 242.67 | 241.67 |

Table 11-2: Statistics of mouse grip strength test

|         | ALS+solvent |      |      | ALS+M cells |      |      |
|---------|-------------|------|------|-------------|------|------|
| Week 24 | 5.53        | 6.29 | 8.65 | 7           | 7.67 | 6.91 |
| Week 29 | 4.76        | 4.71 | 4.84 | 5.94        | 5.46 | 5.64 |

The results of body weight and survival rate of the mice showed that the M cells could promote the weight gain of mice and also improved the survival rate of mice. Immunohistochemical results showed that the lumbar spinal cord motor neurons were less, and the microglia and astrocytes were significantly increased in the solvent group, while the lumbar spinal cord motor neurons increased, and the microglia and astrocytes decreased in the M cell group. It indicated that the M cells could protect motor neurons and alleviate disease progression.

Example 12: Evaluation of therapeutic activity of M cells against inflammatory bowel disease

Inflammatory bowel disease (IBD) is a typical chronic relapsing disease associated with dysregulation of the mucosal immune system and commensal ecosystem, embodying the interaction between host genetics, host immunology, microbiome and environmental exposures. effect. IBD manifests as two major clinical entities: Crohn's disease (CD) and Ulcerative colitis (UC). UC affects the colon, CD may affect any area of the gastrointestinal tract, but mainly occurs in the ileum at the terminal end of the small intestine.

The current clinical treatment of IBD is conservative, mainly relying on alleviating symptoms and inhibiting its excessive deterioration to avoid intestinal obstruction and physical resection of colon cancer. The current treatment methods mainly include aminosalicylic acid drugs, corticosteroids, immunosuppressive agent, biological agents, etc. These drugs reduce disease-related complications and improve the quality of life of patients. 5-Aminosalicylic acid (5-ASA) is commonly used in the anti-inflammatory treatment of UC patients, which can effectively relieve tissue inflammation and may reduce the risk of colitis-related tumors in these patients, and its action mechanism is that through inhibiting the activity of cyclooxygenase, the synthesis of prostaglandins is reduced, the production of proinflammatory cytokines and oxygen free radicals is inhibited, and the neutrophil chemotaxis and mast cell activation are inhibited. However, 5-ASA cannot relieve tissue inflammation in CD patients. Treatment with corticosteroids can relieve symptoms of ulcerative colitis, but they do not maintain long-term effects. After binding to specific cytoplasmic receptors, glucocorticoids are transported into the nucleus, thereby activating or inhibiting the expression of related genes. It can also inactivate proinflammatory transcription factors and prevent the activation of inflammatory mediators through protein-protein interactions, such as NF-κB and activator protein-1 (AP1). Immunosuppressive drugs include Azathioprine, 6-mercaptopurine, Methotrexate, Cyclosporin A, Tacrolimus, which come into play by inducing apoptosis of cells and influencing the survival of immune cells, thereby inhibiting the expression of proinflammatory genes. Tumor necrosis factor (TNF) antagonists are the main progress in this field. It has achieved good treatment of CD and UC in clinic, illustrating the key pathogenic role of TNF in IBD. However, the lack or secondary loss of response to anti-TNF therapy in many patients is an important clinical issue. The aforementioned therapeutic approaches do not treat all patients with IBD, and there are some IBD patients are resistant to the aforementioned drug treatments, and their symptoms cannot be relieved. Finally, surgical resection has to be used for treatment. In recent years, mesenchymal stem cells (MSCs) have been increasingly used in the treatment of IBD and achieved good therapeutic effects. MSCs have the ability of tissue repair and immune regulation, which makes them have broad application prospects in the treatment of autoimmune diseases and IBD.

Experimental animals: 7 to 8 weeks old C57BL/6 female mice (SPF grade), weighing between 18 to 19.5 g, the C57BL/6 female mice were purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended with EB spheres and subjected to adherent differentiation, the M cells of P0 generation were obtained, passaged and screened, and cryopreserved at P3 generation for subsequent experiments.

M cells at P3 generation were resuscitated, digested and passaged, and used at the P5 generation for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Mouse Chemokine Panel, 31-plex | Bio-Rad | 12009159 |
| Embedding machine | Leica | EG1150H/C |
| Sectioning machine | Leica | RM2235 |
| Section displaying machine | Leica | HI1210 |
| Isoflurane | Ruiwode | 970-00026-00 |
| Disposable sterile syringe 1ml | Jiangsu Zhiyu Medical Equipment Co., Ltd. | None |
| Normal saline | Shijiazhuang No. 4 Pharmaceutical Co., Ltd. | None |
| Dextran Sulfate (DSS) | MP | 216011080 |
| Paraformaldehyde | LEAGENE | DF0135 |
| Xylene | Beijing Reagent Co., Ltd. | None |
| Paraffin | Leica | 39601006 |

(continued)

| Reagent/Equi pment | Manufacturer | Cat. No. |
|---|---|---|
| Hematoxylin staining solution | Zhongshan Jinqiao | ZLI-9610 |
| Eosin staining solution | Zhongshan Jinqiao | ZLI-9644 |
| Neutral resin | Solarbio | G8590-100 |

The C57BL/6 female mice were randomly divided into normal group, model group and treatment group according to their body weight.

By using dextran sulphate sodium (DSS) (molecular weight 36,000 to 50,000, MP), the experimental colitis model was established by drinking water.

The flow chart of 2.5% DSS-induced inflammatory bowel disease was shown in FIG. 109.

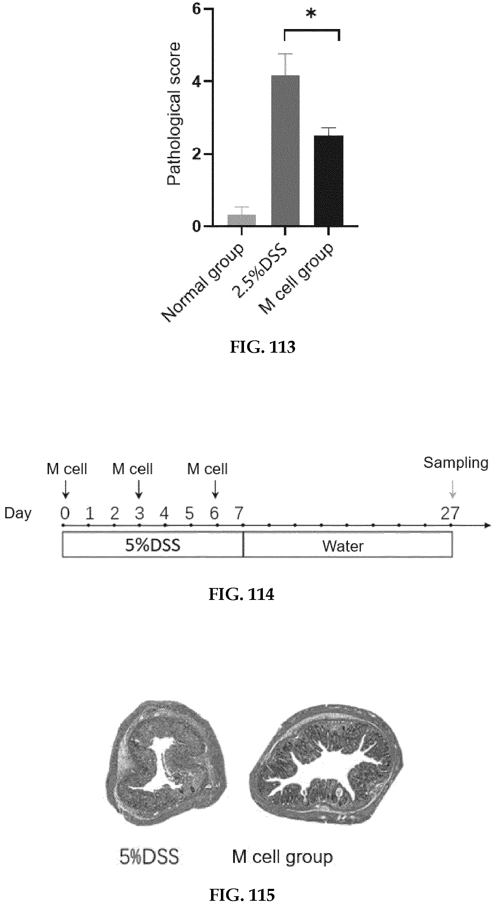

The flow chart of 5% DSS-induced inflammatory bowel disease was shown in FIG. 114.

Normal control group: those drunk with distilled water were used as the control.

Model group: configuration of 2.5% DSS (12.5 g of DSS powder was added to distilled water, mixed and dissolved, and the final constant volume was 500 ml);

configuration of 5% DSS (25 g of DSS powder was added to distilled water, mixed and dissolved, and the final constant volume was 500 ml).

Treatment group: On days 0, 3 and 6, 300μl of cell suspension was intraperitoneally injected, and the cell amount was $3 \times 10^6$.

Observation of vital signs of mice:

In every morning, the mice were subjected to weighing, observation of stool hardness and blood in stool. The DAI score was composed of the cumulative sum of the three scores: body weight change, stool hardness and blood in stool.

DAI scoring criteria

| Body weight loss (%) | Stool hardness | Blood in stool | Score |
|---|---|---|---|
| 0 | Normal | Normal | 0 |
| 1-5 | | Occult blood | 1 |
| 5-10 | Loose | Occult blood, blood in stool visible to the naked eye | 2 |
| 10-20 | | | 3 |
| >20 | loose stool | blood in stool, blood around anus | 4 |

Sample collection

When collecting specimens, after the mice were intraperitoneally anesthetized, the mice were placed in a supine position, the skin of the mice was cut in the middle of the abdomen, the chest was opened, the heart was exposed, and the heart was perfused with ice-cold normal saline. Each mouse needed about 20 ml of normal saline. The peritoneal tissues were exposed and the entire colon from the ileum to the colon was carefully isolated. The colonic tissue was laid flat on the gauze and photoed to record its length.

Extraction of total proteins from animal tissue

1. The centrifuge tube column and receiver tube cannula were pre-cooled on ice.

2. 15 to 20 mg of tissue was placed on a centrifuge tube column, twisted and ground 50 to 60 times with a plastic stick, added with 200 μl of cell lysis solution, and continued to grind 30 to 60 times.

3. It was covered with a lid, and incubation was carried out at room temperature for 1 to 2 minutes, then centrifugation was carried out at 14000 to 16000rpm for 2 minutes.

4. The collection tube was immediately placed on ice and the centrifuge tube column was discarded. After the protein extraction was completed, it could be used in downstream experiments and was cryopreserved in a -80°C refrigerator.

Detection of factor secretion by suspension chip system

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C.

(2) The cryopreserved sample was taken from the -80°C refrigerator. After thawing, 0.5 % BSA (w/v) was added to the sample for dilution.

(3) The Bio-Plex system was calibrated with Bio-Plex ManagerTM.

(4) To the standard bottle, 250 $\mu$L of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1× with Bio-Plex detection buffer, and stored in the dark.

(7) The diluted magnetic beads were vortexed, and 50 $\mu$L of the magnetic beads was added to each well.

(8) The plate was washed twice with 100 $\mu$L of washing solution.

(9) The sample, standard, blank control and the control with known concentration were vortexed, and added in an amount of 50 $\mu$L to each well.

(10) The plate was sealed with a sealing film, and shaken on a high-frequency shaker at 850±50 rpm at room temperature for 30 min.

(11) In step (10), when 10 min of shaking time was left, the detection antibody was vortexed for 5 s and diluted to 1×.

(12) The plate was washed twice with 100 $\mu$L of washing solution.

(13) The diluted antibody was vortexed, and added in an amount of 250 $\mu$L to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when 10 min of shaking time was left, SA-PE 5 was vortexed and diluted to 1×.

(17) The plate was washed twice with 100 $\mu$L of washing solution.

(18) The diluted SA-PE was vortexed, and added in an amount of 50 $\mu$L to each well.

(19) The plate was sealed with a sealing film, and shaken on a high-frequency shaker at 850±50 rpm at room temperature for 30 min.

(20) The plate was washed three times with 100 $\mu$L of washing solution.

(21) The magnetic beads were resuspended with 125 $\mu$L of detection solution, the plate was sealed with a sealing film, and shaken on a high-frequency shaker at room temperature at 850±50 rpm for 30 s.

(22) After the sealing film was discarded, loading to machine was started.

Steps for tissue paraffin sectioning:

(1) Fixation: the tissue was socked in 4% PFA and fixed overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene: paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Scoring standards for colon histomorphology

Histological scoring was performed in a blinded manner: crypt structure (normal, 0; severe crypt aberration, loss of entire crypt, 3), degree of inflammatory cell infiltration (normal, 0; dense inflammatory infiltration, 3), muscle thickening (crypt located on mucosa, 0; obvious muscle thickening, 3), crypt abscess (absent, 0; present, 1) and goblet cell depletion (absent, 0; present, 1). The total histological score was the sum of each sub-scoring item.

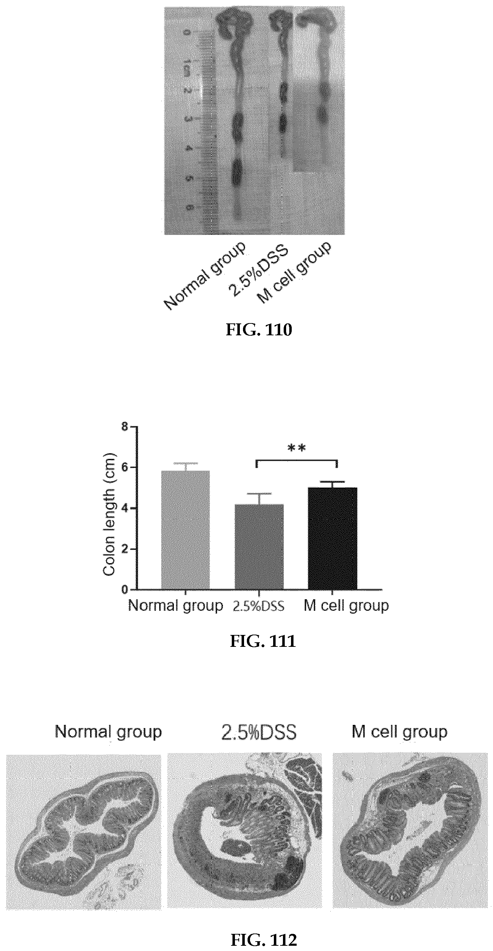

FIG. 110 showed the light microscope photos of the mouse colon samples.
The mouse colon lengths were shown in FIG. 111 and Table 12-1.

Table 12-1: Statistical values of mouse colon length, the statistics of mouse colon length on the 11th day.

|  | Colon length (cm) | | | | | |
| --- | --- | --- | --- | --- | --- | --- |
| Normal group | 6.32 | 5.95 | 6.17 | 5.45 | 5.67 | 5.47 |
| 2.5%DSS | 4.00 | 4.10 | 4.63 | 4.58 | 3.29 | 4.54 |
| M cell group | 4.93 | 4.78 | 4.74 | 5.54 | 4.93 | 5.07 |

Table 12-2: Statistics of mouse histological score

| | Histological score | | | | | |
|---|---|---|---|---|---|---|
| Normal group | 0 | 1 | 0 | 1 | 0 | 0 |
| 2.5%DSS | 7 | 3 | 4 | 4 | 3 | 4 |
| M cell group | 2 | 3 | 2 | 3 | 2 | 3 |

Table 12-2 illustrated: the pathological score statistics of mouse colon HE sections in FIG. 112.

The sample proteins were detected by multi-factor kits and ELISA kits (refer to Xin Zhou, et.al, 2019, Cell reports, Emanuela Sala, et, al, Gastroenterology, 2015): as compared with the 2.5% DSS group, the M cell group had significantly decreased contents of proinflammatory factors, such as IFN-$\gamma$, IL-6, TFN-$\alpha$, iNOS, etc., and the contents of anti-inflammatory factors such as IL-10 were significantly increased, and the contents of nutritional factors such as VEGF, HGF, SDF-1a, etc. were also significantly increased, indicating that the M cells could inhibit the occurrence of inflammation in the treatment of inflammatory bowel disease, and increase the secretion of nutritional factors to promote the repair of colon tissue, and had function of inhibiting inflammation and promoting tissue repair in the treatment of inflammatory bowel disease.

The pathological score statistics of mouse colon HE staining were shown in FIG. 113.

Table 12-3: Histological score statistics of mice, the pathological score statistics of mouse colon HE sections in FIG. 115.

| | Histological score | | | | | |
|---|---|---|---|---|---|---|
| 5%DSS | 10 | 11 | 12 | 9 | 10 | 10 |
| M cell group | 1 | 0 | 0 | 0 | 1 | 1 |

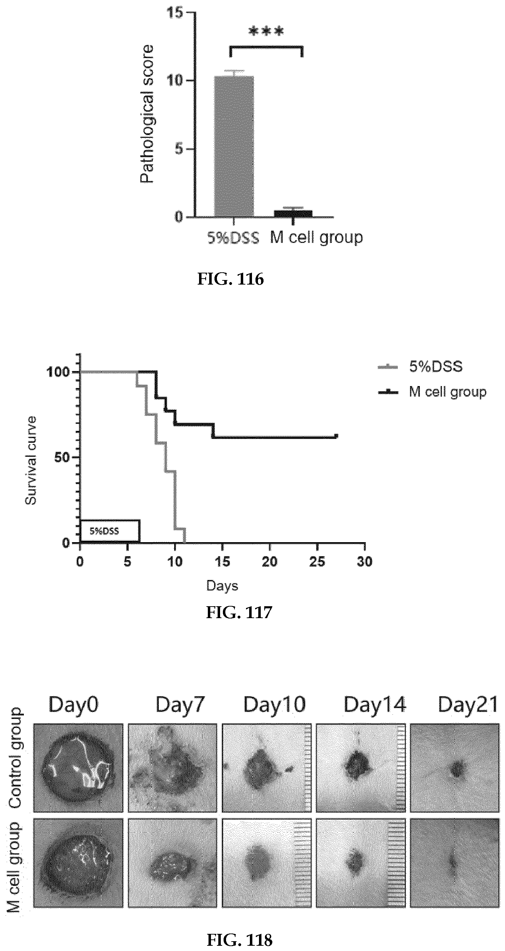

The pathological score statistics of mouse colon HE staining were shown in FIG. 116.

The statistics of mouse survival were shown in FIG. 117 and Table 12-4.

Table 12-4: Statistics of surviving mice

| | | Surviving mice (number) |
|---|---|---|
| Days | 5%DSS | M cell group |
| 0 | 12 | 12 |
| 6 | 11 | 12 |
| 7 | 9 | 12 |
| 8 | 7 | 10 |
| 9 | 5 | 9 |
| 10 | 1 | 8 |
| 11 | 0 | 8 |
| 14 | 0 | 7 |
| 27 | 0 | 7 |

The sample proteins were detected by multi-factor kit and ELISA kit. As compared with the 5% DSS group, the M cell group had significantly decreased contents of proinflammatory factors, such as IFN-$\gamma$, IL-6, TFN-$\alpha$, iNOS, etc., and the contents of anti-inflammatory factors such as IL-10 were significantly increased, and the contents of nutritional factors such as VEGF, HGF, SDF-1a, etc. were also significantly increased, indicating that the M cells could inhibit the occurrence of inflammation in the treatment of inflammatory bowel disease, and increase the secretion of nutritional factors to promote the repair of colon tissue, and had function of inhibiting inflammation and promoting tissue repair in the treatment of inflammatory bowel disease.

It was found in the immunofluorescence staining that after the M cell treatment, the proportion of M2-type macrophages in colon tissue was significantly up-regulated, while the proportion of M1-type macrophages was down-regulated, which would help relieve inflammation and promote tissue repair.

The above results showed that the M cell treatment could reduce inflammation in IBD mice and protect colon tissue, and in the case of high-concentration DSS induction, the M cells could greatly improve the survival rate of mice. In conclusion, the M cells could reduce colon inflammation, promote colon tissue repair, improve the survival rate of mice, and had effective therapeutic effect on inflammatory bowel diseases.

Example 13: Evaluation of therapeutic activity of M cells against burns

Burns often cause extensive skin damage, resulting in loss of skin barrier function and disturbance of internal environment balance, and wound healing takes a long time. Clinical treatment often requires large-area skin transplantation, but burn patients have limited skin, and there is secondary damage to skin extraction. Wound infection leads to various complications such as difficult wound healing and septic shock, progressive deepening of infected and necrotic wounds, and scar healing after wound healing leads to contracture deformities, resulting in unsightly appearance and functional obstacles. The prognosis of patients is poor, the function recovery is poor, and the later rehabilitation treatment increases the psychological and economic burden of the patients. Therefore, finding a method that can promote quick wound healing and restore better appearance and function of skin has become the need in the field of burns.

So far, although skin injury has been treated by autologous skin grafting or artificial skin grafting, it is still insufficient in treatment of large-area burns and scalds. The emergence of mesenchymal stem cells, and combined treatment with materials have brought some hope for treatment of skin injury.

The present invention overcomes the problems such as limited number of skins for autologous graft, skin damages cannot be treated timely and effectively, the skin in the damaged area cannot be functionally treated. The present inventions overcomes the problems of insufficient number and limited sources of skins for autologous graft, the functional cannot be restored after skin damage. The present invention solves the problems such as limited sources of skin for graft, and limited skin for autologous graft, builds appendages after skin damage, achieves the accelerated wound healing after skin damage, reduces the occurrence of fibrosis, and achieves the functional recovery of skin.

Achieved effects: After transplantation of M cells, the healing speed of the scalded area of the rats was significantly accelerated, and the wound area became smaller. The results of tissue sections showed that the M cells combined with Matrigel treatment could reduce the occurrence of fibrosis.

(1) Preparation of M cells

(2) Method and dosage of M cell transplantation

(3) Rat scald model

Experimental animals: SD rats, male, 7 to 8 weeks old, purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended with EBs and subjected to adherent differentiation, and the M cells at the P0 generation were obtained, passaged and screened, and cryopreserved at the P3 generation for subsequent experiments.

The M cells at P3 generation were resuscitated, digested and passaged, and P5 generation was used for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Embedding machine | Leica | EG1150H/C |
| Sectioning machine | Leica | RM2235 |

(continued)

| Reagent/Equi pment | Manufacturer | Cat. No. |
|---|---|---|
| Section displaying machine | Leica | HI1210 |
| Isoflurane | Ruiwode | 970-00026-00 |
| Matrigel | Corning | 354248 |
| 150mm petri dish | Corning | 430599 |
| Water bath | Saiou Huachuang | SDY-1 |
| Metal rod | Self-made (diameter 1.3cm, height 3cm) | None |
| 3M Tegaderm-Film | 3M | 1626W |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Masson staining solution | Nanjing Jiancheng | D026-1-2 |
| Neutral resin | Solarbio | G8590-100 |

Animal model preparation: SD rats were anesthetized with 5% chloral hydrate, and the back hair was shaved after anesthesia. The metal bar was heated in boiling water in a water bath for 5 min, taken out, the aluminum bar was clamped with tweezers, placed on the left and right sides 1cm away from of the midline of rat back, and burns were caused by the aluminum bar for the action of gravity for 20 s, thereby making the model. Two days later, the skin of the injured area was cut off, and the rats were grouped and treated. The skin of the scalded area was cut off, photos were taken, the height for photoing was fixed, and a ruler with scale was placed next to the wound. In order to measure the modeling area, ImageJ software was used for the measurement and determination.

Grouping: control group, M cell group.

Control group: only 3M bandages were applied.

M cell group: $2\times10^6$ M cells (p5 generation) were mixed in 200ul of Matrigel, and 200ul of the mixture was added to each wound for treatment, and 3M bandages were applied.

The photos were taken on days 0, 7, 10, 14 and 21, the perfusion and sampling were carried out on day 21, the samples were soaked in paraformaldehyde overnight, then paraffin section was carried out, and HE staining and Masson staining were performed.

Sample collection:

When collecting the specimens, the rat was intraperitoneally anesthetized, then the rats were in a supine position, the skin was cut in the middle of the abdomen of the rat, the chest was opened, the heart was exposed, and the heart was perfused with ice-cold normal saline. Each rat needed about 50 ml of normal saline. After the perfusion of normal saline was completed, 50ml of paraformaldehyde was used for fixation. After the perfusion was completed, the skin of the damaged area was cut off, fixed, sectioned and analyzed.

Steps for tissue paraffin sectioning

(1) Fixation: the tissue was socked in 4% PFA and fixed overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene: paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided; after the slides were air-dried, they were observed under a microscope.

Masson staining

(1) Dewaxing paraffin sections to water: The sections were placed in xylene I for 20 minutes, xylene II for 20 minutes, anhydrous ethanol I for 10min, anhydrous ethanol II for 10min, 95% alcohol for 5min, 90% alcohol for 5min, 80% alcohol for 5min, 70% alcohol for 5min in sequence, and washed with distilled water.

(2) Hematoxylin staining of nuclei: staining was performed for 5 min with Weigert's iron hematoxylin in the Masson staining kit; after being washed with tap water, differentiation was performed with 1% hydrochloric acid-alcohol for several seconds, rinsing was performed with tap water, and returning to blue was achieved by rinsing with running water for several minutes.

(3) Ponceau red staining: staining was performed for 5 to 10 min with Ponceau red acid fuchsin solution in the Masson staining kit, and rinsing was quickly performed with distilled water.

(4) Phosphomolybdic acid treatment: the treatment with phosphomolybdic acid aqueous solution in the Masson staining kit was performed for about 3 to 5 min.

(5) Aniline blue staining: instead of washing with water, counterstaining was performed for 5 min with aniline blue solution in the Masson staining kit.

(6) Differentiation: the treatment with 1% glacial acetic acid was performed for 1 min.

(7) Dehydration and mounting: the sections were placed in 95% alcohol I for 5min, 95% alcohol II for 5min, absolute ethanol I for 5min, absolute ethanol II for 5min, xylene I for 5min, xylene II for 5min in sequence to perform dehydration and transparentizing, then the sections were taken out from xylene and slightly air-dried, and mounted with neutral resin.

(8) Microscopic examination was performed with a microscope, and images were acquired and analyzed.

Immunohistochemical staining:

Immunohistochemical staining was performed on the paraffin sections using an immunohistochemical kit (Fuzhou Maixin, KIT-9710). The specific steps were as follows:

1. Dewaxing: (1) xylene I, II, 10 min each; (2) gradient alcohol: 100% absolute ethanol, 2 min; 95% absolute ethanol, 2 min; 80% absolute ethanol, 2 min; 70% absolute ethanol, 2 min;

2. Hydration: washing was performed twice with distilled water, 5min each time (placed on a shaker);

3. After deparaffinization and hydration of paraffin sections, rinsing was performed 3 times with PBS, 3 minutes each time;

4. Preparation of antigen retrieval solution (10 mM pH 6.0 sodium citrate buffer):

(1) Preparation of stock solution: Solution A: 29.41 g of trisodium citrate dihydrate + 1,000 mL of distilled water; Solution B: 21 g of citric acid + 1,000 mL of distilled water;

(2) Preparation of working solution: 82 mL of Solution A + 18 mL of solution B + 900 mL of distilled water;

5. Antigen retrieval: the sections were placed in a plastic or heat-resistant glass container filled with sodium citrate buffer, the sections were immersed, treated with a microwave oven at mid-range or high-range power for 5 minutes; sodium citrate buffer was replenished, and treatment was performed again at mid-range or high-range power for 5 minutes;

6. Reagent A (peroxidase blocking solution) was added, and incubated at room temperature for 10 min to block the activity of endogenous peroxidase; rinsing was performed with PBS 3 times, 3 min each time;

7. PBS was discarded, 1 drop or 50 μL of Reagent B (normal non-immune animal serum) was added, and incubated at room temperature for 10 min;

8. The serum was discarded, 1 drop or 50 μL of primary antibody was added, and incubated at 4°C overnight or at room temperature for 60 min; rinsing was performed with PBS 3 times, 3 min each time;

9. The PBS was discarded, 1 drop or 50 μL of biotin-labeled secondary antibody (Reagent C) was added, and incubated at room temperature for 10 min; rinsing was performed with PBS 3 times, 3 min each time;

10. The PBS was discarded, 1 drop or 50 μL of streptavidin-peroxidase solution (reagent D) was added, incubated at room temperature for 10 min; rinsing was performed with PBS 3 times, 3 min each time;

11. The PBS was discarded, 2 drops or 100 μL of freshly prepared DAB solution was added, and observation was performed under microscope for 3 to 10 min;

12. Rinsing was performed with tap water, counterstaining was carried out with hematoxylin, and rinsing was performed with PBS or tap water so as to return to blue;

13. When using DAB for color development, the sections should be dehydrated with gradient alcohol and dried, transparentizing was performed with xylene, and mounting was performed with neutral resin;

14. Photos were taken with a microscope.

Immunofluorescence staining:

(1) The tissue sections were dewaxed and transferred into water;

(2) Antigen microwave retrieval was performed at a temperature of 92°C to 96°C for 10 to 15min, naturally cooled to room temperature;

(3) Normal goat serum blocking was performed at 37°C for 60 min;

(4) The excess serum was poured off, primary antibody was added dropwise, allowed to stand at 37°C for 2 hours or 4°C overnight, rinsing was performed with PBS, 5min×3 times;

(5) The fluorescein-labeled secondary antibody was added dropwise, allowed to stand in the dark at 37°C for 60 min, rinsing was performed with 0.01M PBS, 5 min×3 times;

(6) The sections were mounted with anti-quenching mounting medium, and stored at 4°C in the dark.

(7) Observation and photoing were performed with a fluorescence microscope.

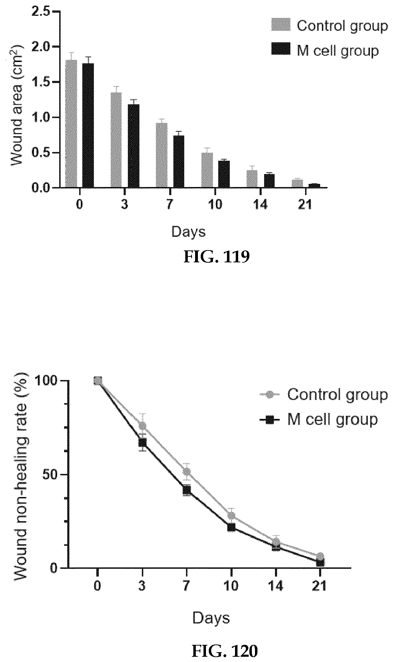

The photos of wounds on different days were shown in FIG. 118.
The wound area size statistics were shown in FIG. 119.
The wound non-healing rate statistics were shown in FIG. 120.
The wound area size statistics on day 21 were shown in FIG. 121.
Table 13-1 showed the statistical values of the injured skin area of the rats on the Day 0, 3, 7, 10, 14 and 21.

Table 13-1: Statistics of wound area size

| Time (days) | Wound area (cm$^2$) | | | | | | | | | | | |
| | Control group | | | | | | M cell group | | | | | |
| 0 | 1.754 | 1.7315 | 1.5005 | 1.6765 | 1.9445 | 2.278 | 1.9135 | 1.6665 | 1.6155 | 1.648 | 1.6335 | 2.1355 |
| 3 | 1.569 | 1.644 | 1.288 | 1.104 | 1.216 | 1.29 | 1.282 | 1.154 | 1.251 | 1.31 | 0.837 | 1.257 |
| 7 | 0.763 | 1.029 | 0.9365 | 0.8495 | 1.146 | 0.7925 | 0.7105 | 0.597 | 0.6895 | 0.8825 | 0.5835 | 0.978 |
| 10 | 0.456 | 0.8105 | 0.392 | 0.3905 | 0.5515 | 0.418 | 0.3375 | 0.3195 | 0.3685 | 0.492 | 0.3935 | 0.38 |
| 14 | 0.199 | 0.533 | 0.1325 | 0.185 | 0.274 | 0.211 | 0.186 | 0.1545 | 0.222 | 0.29 | 0.1835 | 0.156 |
| 21 | 0.073 | 0.1515 | 0.1375 | 0.0425 | 0.1085 | 0.1895 | 0.0605 | 0.035 | 0.077 | 0.0435 | 0.064 | 0.0665 |

Table 13-2: Statistics of non-healing rate of wounds

| Time (days) | Wound non-healing rate statistics | | | | | | | | | | | |
|---|---|---|---|---|---|---|---|---|---|---|---|---|
| | Control group | | | | | | M cell group | | | | | |
| 0 | 100 | 100 | 100 | 100 | 100 | 100 | 100 | 100 | 100 | 100 | 100 | 100 |
| 3 | 89.452 68 | 94.946 58 | 85.838 05 | 65.851 48 | 62.535 36 | 56.628 62 | 66.997 65 | 69.246 92 | 77.437 33 | 79.490 29 | 51.239 67 | 58.862 09 |
| 7 | 43.500 57 | 59.428 24 | 62.412 53 | 50.671 04 | 58.935 46 | 34.789 29 | 37.130 91 | 35.823 58 | 42.680 28 | 53.549 76 | 35.720 84 | 45.797 24 |
| 10 | 25.997 72 | 46.809 13 | 26.124 63 | 23.292 57 | 28.362 05 | 18.349 43 | 17.637 84 | 19.171 92 | 22.810 28 | 29.854 37 | 24.089 38 | 17.794 43 |
| 14 | 11.345 5 | 30.782 56 | 8.8303 9 | 11.034 89 | 14.091 03 | 9.2625 11 | 9.7204 08 | 9.2709 27 | 13.741 88 | 17.597 09 | 11.233 55 | 7.3050 81 |
| 21 | 4.1619 16 | 8.7496 39 | 9.1636 12 | 2.5350 43 | 5.5798 41 | 8.3187 01 | 3.1617 45 | 2.1002 1 | 4.7663 26 | 2.6395 63 | 3.9179 68 | 3.1140 25 |

Table 13-3 showed the statistics of the wound area of the control group and the M cell group on the Day 21.

Table 13-3: Statistics of wound area size on Day 21

| Time (days) | Wound area (cm$^2$) | | | | | | | | | | | |
|---|---|---|---|---|---|---|---|---|---|---|---|---|
| | Control group | | | | | | M cell group | | | | | |
| 21 | 4.1619 16 | 8.7496 39 | 9.1636 12 | 2.5350 43 | 5.5798 41 | 8.3187 01 | 3.1617 45 | 2.1002 1 | 4.7663 26 | 2.6395 63 | 3.9179 68 | 3.1140 25 |

The results of HE staining and Masson staining were shown in FIG. 122. The results of HE staining showed that the wound healing of the M cell group was better than that of the control group. The would in M cell group had completely healed and the epidermis had healed completely, while the middle part of the epidermis of wound in the control group had not yet healed completely. The Masson staining results showed that the coloration was darker and the collagen was precipitated in the control group, indicating severe fibrosis. However, the M cell treatment group had less collagen deposition before staining, and the degree of fibrosis was lower than that in the control group. After the transplantation of M cells and Matrigel, the wound recovery speed of the rat skin injury was accelerated; and the tissue section showed less fibrosis.

Immunohistochemical staining was performed on the skin of rats at the modeling site on the 5th and 7th days. The expressions of CD3, F4/80 and MPO in the M cell group were significantly lower than those in the model group, indicating that the M cells could reduce inflammation at the wound site after burning, and suppress inflammation. The expression of K14 in the M cells was significantly higher than that in the model group, indicating that the M cells could accelerate the epidermalization of wounds after skin injury, accelerate the wound healing, and play an effective therapeutic role in the skin injury.

Immunofluorescence identification of rat skin sections on the 14th day showed that the expression of CD31 marker in the M cell group was significantly higher than that in the control group, indicating that the M cell treatment could promote the angiogenesis of skin wounds and had important effect to the regeneration of skin after injury.

Immunofluorescence staining of skin wound sections showed that the expressions of β-Catenin, CD133, and Ki67 in the M cell group were significantly higher than those in the model group, indicating that in the M cell group, there were more hair follicle organs, and the M cell treatment could promote the regeneration of hair follicles after skin injury.

In conclusion, the M cell treatment could accelerate the healing of skin wounds, reduce the inflammation of skin wounds, promote the regeneration of blood vessels and hair follicles, reduce the deposition of collagen, and inhibit the occurrence of fibrosis. Therefore, the M cells could effectively treat skin injury.

Example 14: Evaluation of therapeutic activity of M cells against diseases of male reproductive system

The organs of the reproductive system are composed of gonads, reproductive ducts, and appendages. The reproductive organs function to reproduce offspring through their various activities, fertilization, pregnancy and other physiological processes. Reproductive system diseases mainly include, reproductive system tumors, such as testicular cancer, prostate cancer, ovarian cancer, uterine cervical cancer, etc.; reproductive system infections, such as specific infections and non-specific infections, such as tuberculosis infection, chronic prostatitis, epididymitis etc.; reproductive system malformations, such as concealed penis, webbed penis, cryptorchidism, etc.; sexual dysfunction-related diseases, such as male erectile dysfunction, varicocele, female polycystic ovary, etc.

Diseases of the male reproductive system include abnormal urination, pyuria, abnormal urethral discharge, pain, mass, sexual dysfunction and male infertility related to urological diseases. They mainly include urinary system inflammation, such as cystitis, urethritis, urinary incontinence, urinary retention, etc.; reproductive system inflammation, such as testicular epididymitis, seminal vesiculitis, prostatitis, etc.; reproductive tract tuberculosis, such as testicular epididymal tuberculosis, seminal vesicle tuberculosis, etc.; reproductive system tract injury, such as testicular contusion, penis fracture, urethral rupture, etc.; male infertility diseases, such as varicocele, asthenozoospermia, congenital vas deferens obstruction, absence of vas deferens, etc.; male sexual dysfunction diseases, such as male erectile dysfunction, premature ejaculation, loss of libido, non-ejaculation, delayed ejaculation, etc. With the pollution of living environment, the increase of work pressure and the change of diet structure, the incidence of modern male reproductive system diseases is increasing year by year.

Male infertility refers to infertility caused by male factors, generally speaking, when living together for more than 2 years after marriage without taking any contraceptive measures, the woman is not pregnant. Male infertility includes testicular atrophy, testicular hypoplasia, oligospermia, spermatogenesis disorder, azoospermia, obstructive azoospermia, asthenozoospermia, Klinefelter's syndrome, XYY syndrome, Kallmann's syndrome, selective LH deficiency and FSH deficiency, adrenal hyperplasia, hyperprolactinemia, varicocele, teratospermia, etc.

Oligospermia is defined as the number of sperm in semen is lower than that of normal healthy fertile men, including endocrine dysfunction, reproductive system infection, varicocele, anti-sperm antibody, cryptorchidism, hydrocele, malnutrition, and oligospermia caused by chemotherapy, radiation therapy, obesity, etc.

Experimental animals: SD rats, male, 6 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended to form EB spheres and subjected to adherent differentiation, the M cells of P0 generation were obtained, passaged and screened, and cryopreserved at P3 generation for subsequent experiments.

The M cells at P3 generation were resuscitated, digested and passaged, and used at the P5 generation for subsequent experiments.

Preparation of busulfan: 0.4g of busulfan powder was weighed and dissolved in 10ml of dimethyl sulfoxide to obtain a 40mg/ml solution. After complete dissolution, the busulfan with the concentration was subpackaged in centrifuge tubes and stored at 4°C in the dark for later use.

| Reagent/Equi pment | Manufacturer | Cat. No. |
|---|---|---|
| Electronic scale | domestic | 1000212 |
| Embedding machine | Leica | EG1150H/C |
| Sectioning machine | Leica | RM2235 |
| Section displaying machine | Leica | HI1210 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Busulfan | Sigma | B2635-10G |
| DMSO | Sigma | D2650-100ML |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |

Animal modeling: After the SD rats were anesthetized, a small incision was cut in the abdomen, the left testis was exposed with tweezers, and 50 $\mu$l of the prepared busulfan was injected for modeling, and then the rats were divided into groups, 4 mice in each group.

Model group: 100ul of normal saline was injected.

M cell group: 100ul of normal saline containing $3\times10^6$ M cells (P5 generation) was injected.

The second treatment was performed on the 10th day, the perfusion and sampling were performed on the 20th day, and the left and right testes were weighed.

Sample collection:

When collecting the specimens, the rat was anesthetized, and then placed in a supine position, the skin was cut in the middle of the abdomen of the rat, the chest was opened, the heart was exposed, and the heart was perfused with ice-cold normal saline. Each rat needed about 50 ml of saline. After the saline perfusion was completed, the fixation was performed with 50 ml of paraformaldehyde. After the perfusion was completed, the testicles were fixed with paraformaldehyde, sectioned and analyzed.

Steps for tissue paraffin sectioning

(1) Fixation: The tissue was socked in 4% PFA overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Detection of sperm concentration, motility and deformity rate in rat epididymis

Rat epididymal sperm density and survival rate were determined by counting with a sperm count plate. It was generally deemed that all sperms in motion were viable sperms, including linear motion, irregular motion and in-situ motion.
Counting with sperm counting plate (this method was used for counting when the sperm density was low): the milky white sperm mass was squeezed out from the tail of the epididymis, a glass tube with fine-drawn round head was used to transfer the sperm into 1ml of fertilization solution, incubation was performed for 30min in an incubator, and the sperms were counted when they were all dispersed. 10 μl of sperm was added to the sperm counting plate each time, the total number of sperm was counted in 10 fields of view under the microscope, the counting was repeated 3 times for each sample, and the average value was calculated. The calculation of sperm density was:

$$\text{Sperm count/field of view} = \text{total sperm count in 10 fields of view}/10$$

Detection of sperm deformity rate: the diluted sperm was dropped on a clean glass slide, pushed with a new cover glass, dried, and fixed with fixative solution (methanol: glacial acetic acid = 3:1) for 15 minutes, the back of the slide was rinsed slowly with running water, after rinsing completely to clean, it was air-dried. Giemsa staining was performed for 1.5 hours, the back was rinsed with running water until the front was not blue, air-dried, and subjected to microscopy. Five fields of view were randomly selected with a 10× objective, 200 spermatozoa were counted each time, the total number of spermatozoa and the number of deformed spermatozoa were counted, and the averages were taken.

Observation of ultrastructure by transmission electron microscopy

Rat testis tissue was fixed in 2.5% glutaraldehyde at 4°C for 12 h; dehydrated with gradient ethanol, infiltrated and embedded in epoxy resin; stained with lead citrate and uranyl acetate in the dark, after semi-thin sectioning, the observation was performed under ordinary light microscope. After positioning, ultrathin sections were subjected to double-lead staining for electron microscopy, and then dried in an oven for 20 min, and the ultrastructure of testis was observed under transmission electron microscope.
The light microscope photos of the testis were shown in FIG. 123.
The weight ratio of left testis to right testis was shown in FIG. 124.

The HE staining picture of testis were shown in FIG. 125.

Table 14-1 showed the summary of rat testis weight when the samples were taken on the 20th day.

Table 14-1: summary of rat testis weight

|  | Left testis/right testis (g) | | | |
|---|---|---|---|---|
| Model group | 0.7/1.9 | 0.7/1.8 | 1.4/1.1 | 1.3/1.7 |
| M cell group | 1.4/1.6 | 1.9/1.6 | 1.4/1.6 | 1.4/1.7 |

Table 14-2: Weight ratio of left testis to right testis

|  | Left testis/right testis (%) | | | |
|---|---|---|---|---|
| Model group | 36.84 | 38.88 | 78.57 | 76.47 |
| M cell group | 87.50 | 105.55 | 87.50 | 82.35 |

Detection of sperm density and viability by hemocytometer:

The detection of sperm count, motility and abnormality rate in epididymis of rats showed that the sperm density in the M cell treatment group was significantly higher than that in the model group; the sperm motility in the M cell treatment group was significantly higher than that in the model group, and the sperm abnormality rate was significantly lower than that in the model group, which indicated that the M cell treatment could promote sperm regeneration and had important effects on maintaining sperm motility and normal morphology.

Transmission electron microscope ultrastructure of testicular tissue: It was found that in the M cell treatment group, the cellular vacuoles in the testicular seminiferous tubules were reduced, the cells were tightly arranged, the nuclei were full, the organelles were clearly visible, and normal and complete sperm could be seen; while in the model group, the vacuoles in cell center were obvious, the cell structure was severely damaged, the nucleus was shrunken, and the organelle structure was unclear, indicating that the M cell treatment played an important role in the recovery of normal sperm morphology.

The above results indicated that the M cell injection therapy could promote sperm regeneration, played an important role in maintaining sperm motility and normal morphology, and played an important role in the recovery of normal sperm morphology and function. Therefore, the M cell injection could effectively treat oligospermia and azoospermia. and other symptoms.

Example 15: Evaluation of therapeutic activity of M cells against epilepsy

Epilepsy is a chronic disease in which the brain's neurons suddenly discharge abnormally, resulting in transient brain dysfunction. It is the second most common neurological disease after stroke. The "abnormal firing" of neurons in the brain causes epileptic seizure, and is characterized as repetitive and transient. Epilepsy affects more than 70 million people worldwide, and the incidence rate in the Chinese population is between 5 to 7 ‰, with 6.5 to 9.1 million patients nationwide. Some cerebrovascular complications, head trauma, central nervous system infections, etc. may lead to secondary epilepsy; sleep, age, and genetics are closely related to idiopathic epilepsy. For the treatment of epilepsy, the most widely used therapies are antiepileptic drugs. However, despite the existence of 30 antiepileptic drugs (AEDs) with different molecular targets, there are still many challenges in the drug treatment of epilepsy, such as drug resistance, side effects, toxicity associated with frequent dependence and memory deficits, etc. In addition, brain surgery is the most important alternative treatment; however, eligibility for enrolment as well as risks and costs must be considered. At present, clinical trials are mainly drug treatments, and more than 200 are in phase III. There is one MSC clinical trial, which is in phase II.

Preparation and culture of M cells:

The embryonic stem cells were suspended with EB spheres and subjected to adherent differentiation, and the M cells of the P0 generation were obtained, passaged and screened, and cryopreserved at the P3 generation for subsequent experiments.

The P3 generation M cells were digested and passaged, and used at the P5 generation for subsequent experiments.

Experimental animals:

Male Sprague-Dawley rats, 5 to 7 weeks old, were purchased from Beijing Sibeifu Biotechnology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences. After 1 week of adaptive feeding of rats, the experiment was started.

Feeding conditions: Free food and water; 12/12h day and night alternation, 7:00 to 19:00 was daytime, room temperature was $22\pm2°C$, relative humidity was 50% to 60%. The experiment was started after one week of adaptive feeding of rats.

Experimental group:

Normal control group, PTZ+solvent (solvent group), PTZ+M cells (M cell group), 6 rats in each group.

Experimental material: 1ml syringe

Experimental reagents: pentylenetetrazole (PTZ), normal saline

| Consumables/Reagents/Instruments | Manufacturer | Cat. No. |
|---|---|---|
| Disposable sterile syringe 1ml | Jiangsu Zhiyu Medical Equipment Co., Ltd. | None |
| Normal saline | domestic | |
| Pentylenetetrazole (PTZ) | SIGMA | P6500-10 |

After intraperitoneal injection of PTZ, 50 mg/kg, the test substance was injected into the tail vein immediately: 1 ml of normal saline was injected into the tail vein of the PTZ+solvent group, and 1 ml of cells was injected into the tail vein of the PTZ+M cell group: $5\times10^6$/rat. Afterwards, the rats were placed in a clear glass cage, and the time spent on the initial seizure, the grade of the initial seizure, the seizure grade, the latent period of the initial grand mal, and the duration and proportion of grand mal were recorded.

Statistics: All data were analyzed by One-way ANOVA in Prism 7.0 statistical analysis software for variance analysis and significance test, and the experimental data were expressed as mean $\pm$ standard error (Mean $\pm$ SD). *, $p<0.05$; **, $p<0.01$; ***, $p<0.001$.

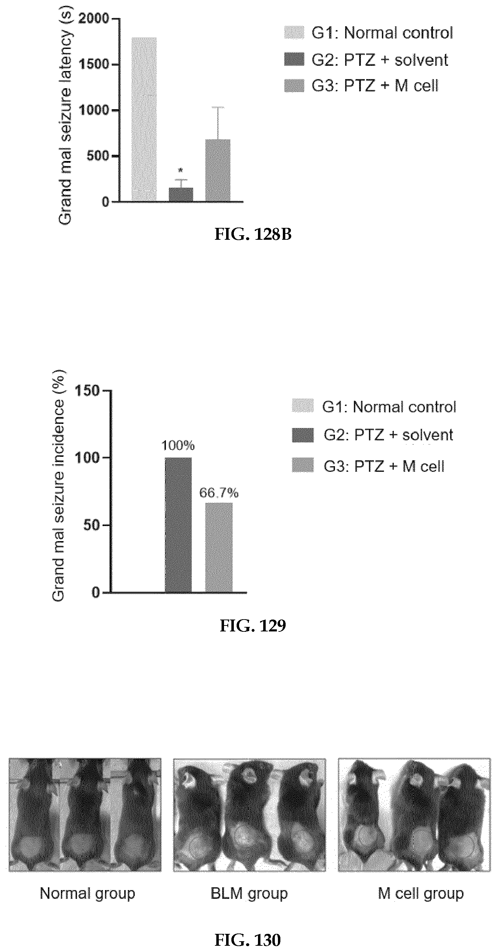

FIG. 126 showed the statistics of the initial seizure latent period in rats. FIG. 127 showed the statistics of the level of initial seizures in rats. The grading statistics of initial seizures in rats were shown in FIG. 128A. The grading statistics of the initial seizure in rats were shown in FIG. 128B. FIG. 129 showed the statistics of the proportion of grand mal in rats.

In the M cell group of tail vein injection, the initial seizure latent period of rats was prolonged by more than 300 seconds, the epileptic seizure grade was reduced from grade 3 to grade 2, the seizure grade was reduced from grade 4 to grade 3, the latent period of grand mal seizure was prolonged by more than 500 seconds, and the proportion of epileptic seizures was reduced to 66.7% as compared to the solvent group. The above data suggested that the M cells could delay, reduce and alleviate epileptic seizures and had good therapeutic effect.

Table 15-1 showed the statistics of the initial seizure latency in rats.

Table 15-1: Statistics of the initial seizure latency in rats

| Group | Normal control | PTZ + solvent | PTZ + M cells |
|---|---|---|---|
| Initial seizure latency(s) | 1800 | 67 | 1091 |
| | 1800 | 229 | 1209 |
| | 1800 | 77 | 74 |
| | 1800 | 40 | 61 |
| | 1800 | 70 | 81 |
| | 1800 | 70 | 61 |

Table 15-2 and FIG. 127 showed the statistics of the initial seizure grades in rats. In the M cell group, the seizure grade was down-regulated, from grade 3 to grade 2, suggesting that the M cells could alleviate seizures.

Table 15-2: Statistics of the initial seizure grades in rats

| Group | Normal group | | | | | | PTZ + solvent | | | | | | PTZ + M cells | | | | | |
|---|---|---|---|---|---|---|---|---|---|---|---|---|---|---|---|---|---|---|
| Initial seizure grade | 0 | 0 | 0 | 0 | 0 | 0 | 5 | 2 | 3 | 2 | 2 | 5 | 1 | 2 | 2 | 2 | 2 | 3 |

Table 15-3 and FIG. 128A showed the grading statistics of the initial seizures in rats. The tail vein injection of M cells significantly reduced seizure grades, indicating that the M cells could alleviate seizures.

Table 15-3: Grading statistics of initial seizures in rats

| Group | Normal group | | | | | | PTZ + solvent | | | | | | PTZ + M cells | | | | | |
|---|---|---|---|---|---|---|---|---|---|---|---|---|---|---|---|---|---|---|
| Epileptic seizure grade | 0 | 0 | 0 | 0 | 0 | 0 | 5 | 5 | 4 | 4 | 5 | 5 | 1 | 2 | 5 | 4 | 5 | 4 |

Table 15-4 and FIG. 128B showed the statistics of the latency of grand mal seizures in rats. The tail vein injection of M cells significantly prolonged the latency of grand mal seizures, from about 156.3 seconds to 681.2 seconds, indicating that the M cells could delay the grand mal seizures.

Table 15-4: Statistics of latency of grand mal seizures in rats

| Group | Normal control | PTZ + solvent | PTZ + M cells |
|---|---|---|---|
| Latency of grand mal seizure (s) | 1800 | 67 | 1800 |
| | 1800 | 585 | 1800 |
| | 1800 | 90 | 108 |
| | 1800 | 52 | 66 |
| | 1800 | 74 | 136 |
| | 1800 | 70 | 177 |

Table 15-5 and FIG. 129 showed the statistics of proportion of grand mal seizures in rats. The tail vein injection of M cells significantly reduced the proportion of grand mal seizures, as compared with the solvent group, the proportion decreased by 33.3%, indicating that the M cells could significantly reduce the number of grand mal seizures.

Table 15-5: Statistics of proportion of grand mal seizures in rats

| Group | Normal control | PTZ + solvent | PTZ + M cells |
|---|---|---|---|
| Proportion of grand mal seizures | 0.0 | 100.0 | 66.7 |

Identification of neuronal cell type and number by staining of brain sections:

Methods: After perfusion of rat hearts, the brains were taken out and fixed in 4% PFA at 4°C overnight, dehydrated gradiently with 15% and 30% sucrose solutions prepared with PBS, embedded in OCT, and frozen in -80°C refrigerator. The section had a thickness of 12 to 15 $\mu$m, patched on polylysine-coated glass slides. Blocking+permeabilization were performed at room temperature for 1h by using a blocking solution prepared with 2%BSA+0.2%TritonX100. The incubation with the primary antibody was performed overnight at 4°C. The incubation with the secondary antibody was performed at room temperature for 2 h. The incubation with Hoechst 33342 was performed at room temperature for 10 min to stain nuclei, and finally the mounting on slides was performed for observation.

It was found from staining sections that as compared with the control, the number of GABAergic neurons in the brains of the animals receiving cell transplantation increased, and the number of microglia decreased, proving that the GABAergic neurons were activated, and the differentiation ability of endogenous stem cells to the GABAergic lineage was enhanced, the inflammatory responses were suppressed, and the cells could remodel and repair neural circuits deficient in GABAergic neurons in the models (subjects).

Behavioral tests:

For the method, refer to Li et al., 2016, Frontiers in Aging Neuroscience.

The results of behavioral tests showed that the animals in the experimental group spent less time searching for specific targets, proving that the cell transplantation could improve the memory and learning abilities of model animals (subjects).

Mass spectrometric detection:

The brain tissue was ground and homogenized, and the supernatant was collected by centrifugation and detected by mass spectrometer.

The mass spectrometric results of brain proteins showed that the secretion levels of GDNF and other nutritional factors in the brains of the animals in the experimental group were increased, which proved that the cell transplantation had the function of promoting the secretion of neurotrophic factors and neuroprotection.

The solvent group had a significantly shortened seizure latency, had higher seizure grades and grading, shortened grand mal latency and decreased proportion of grand mal seizures. These data indicated that the solvent group had severe seizures and multiple seizures as compared with the normal control group and the cell group.

Example 16: Evaluation of therapeutic activity of M cells against scleroderma

Scleroderma is an autoimmune disease characterized by skin thickening and regional or diffuse fibrosis, which can affect the lungs, kidneys, liver, heart and other organs, and its pathogenesis is unknown. Current studies have found that the disease mainly involves three aspects: small vessel disease, fibrosis caused by excessive accumulation of extracellular matrix, and immune abnormality. Inflammatory cell infiltration is the main feature in the early stage of scleroderma, and mainly includes T lymphocyte infiltration. Studies have shown that T lymphocytes may release a variety of cytokines, causing inflammation and vascular lesions, activating fibroblasts and promoting the synthesis of collagen fibers. At present, immunosuppressive agents and symptomatic treatments are mainly used therapies for scleroderma, but their treatment effects are not ideal, there are many adverse reactions, and thus more effective treatment methods need to be found.

Experimental animals: C57BL/6, female mice, 7 to 8 weeks old, purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended to form EB spheres and subjected to adherent differentiation, and the M cells ate the P0 generation were obtained, passaged and screened, and cryopreserved at the P3 generation for subsequent experiments.

The M cells at P3 generation were resuscitated, digested and passaged, and used at the P5 generation for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |

(continued)

| Reagent/Equi pment | Manufacturer | Cat. No. |
|---|---|---|
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Masson staining solution | Nanjing Jiancheng | D026-1-2 |
| Neutral resin | Solebol | G8590-100 |
| Bleomycin | MP | 190306 |
| Multifactors Suspension Chip System | Bio-Rad | Bio-Plex® 200 |
| 23-Factors Kit | Bio-Rad | M60009RDPD |
| MMP1 ELISA kit | | |

Preparation of animal model: The C57BL/6 female mice were randomly divided into groups according to their body weight. From day 0 to day 21, the prepared bleomycin solution was injected subcutaneously every day, i.e., subcutaneously injected at single point on the back, at a dose of 100 $\mu$l (1 mg/ml), to perform the modeling. On the 14th day, the mice were randomly divided into normal group, BLM (bleomycin) group, and M cell group.

Normal group: the mice were only shaved on day 0, and no other treatments were performed.

BLM group: the mice were shaved on day 0, daily subcutaneously injected with bleomycin solution from day 0 to day 21, and subcutaneously injected with 100 $\mu$l of normal saline at single point on the back on day 14 and day 21, then subjected to photoing and sampling on day 28, and the samples were subjected to sectioning and staining.

M cell group: the mice were shaved on day 0, daily subcutaneously injected with bleomycin solution from day 0 to day 21, and subcutaneously injected with 100 $\mu$l of normal saline containing $3\times10^6$ M cells at single point on the back on day 14 and day 21, then subjected to photoing and sampling on day 28, and the samples were subjected to sectioning and staining.

Sample collection:

When collecting the specimens, after the mice were intraperitoneally anesthetized, the mice were in a supine position, the skin was cut in the middle of the abdomen of the mice, the chest was opened, the heart was exposed, and the heart was perfused with ice-cold normal saline. Each mouse needed about 20ml of normal saline, after the normal saline perfusion was completed, 20ml of paraformaldehyde was used for fixation. After the perfusion was completed, the skin in the modeling area was cut off, and subjected to fixation, sectioning and analysis.

Steps for tissue paraffin sectioning

(1) Fixation: the tissue was socked in 4% PFA overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the slides were sealed with neutral resion to avoid air bubbles; after the slides were air-dried, they were observed under a microscope.

Masson staining

(1) Dewaxing paraffin sections to water: The sections were placed in xylene I for 20 minutes, xylene II for 20 minutes, anhydrous ethanol I for 10min, anhydrous ethanol II for 10min, 95% alcohol for 5min, 90% alcohol for 5min, 80% alcohol for 5min, 70% alcohol for 5min in sequence, and washed with distilled water.

(2) Hematoxylin staining of nuclei: staining was performed for 5 min with Weigert's iron hematoxylin in the Masson staining kit; after being washed with tap water, differentiation was performed with 1% hydrochloric acid-alcohol for several seconds, rinsing was performed with tap water, and returning to blue was achieved by rinsing with running water for several minutes.

(3) Ponceau red staining: staining was performed for 5 to 10 min with Ponceau red acid fuchsin solution in the Masson staining kit, and rinsing was quickly performed with distilled water.

(4) Phosphomolybdic acid treatment: the treatment with phosphomolybdic acid aqueous solution in the Masson staining kit was performed for about 3 to 5 min.

(5) Aniline blue staining: instead of washing with water, counterstaining was performed for 5 min with aniline blue solution in the Masson staining kit.

(6) Differentiation: the treatment with 1% glacial acetic acid was performed for 1 min.

(7) Dehydration and mounting: the sections were placed in 95% alcohol I for 5min, 95% alcohol II for 5min, absolute ethanol I for 5min, absolute ethanol II for 5min, xylene I for 5min, xylene II for 5min in sequence to perform dehydration and transparentizing, then the sections were taken out from xylene and slightly air-dried, and mounted with neutral resin.

(8) Microscopic examination was performed with a microscope, and images were acquired and analyzed.

Extraction of total proteins from animal tissue

Sample collection:
When collecting specimens, after the mice were intraperitoneally anesthetized, the mice were placed in a supine position, the skin was cut in the middle of the abdomen, the chest was opened, the heart was exposed, and the heart was perfused with ice-cold normal saline. Each mouse needed about 20 ml of normal saline. After the normal saline perfusion was completed, the skin of the modeling area was cut off.

(1) The centrifuge column and receiver tube were pre-cooled on ice.

(2) 15 to 20 mg of tissue was placed on a centrifuge column, twisted and ground 50 to 60 times with a plastic stick, added with 200 µl of cell lysis solution, and continued to grind 30 to 60 times.

(3) It was covered with a lid, and incubation was carried out at room temperature for 1 to 2 minutes, then centrifugation was carried out at 14000 to 16000rpm for 2 minutes.

(4) The receiver tube was immediately placed on ice and the centrifuge column was discarded. After the protein extraction was completed, it was cryopreserved in a -80°C refrigerator and could be used in downstream experiments.

Detection of inflammatory factor by suspension chip system

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C.

(2) The cryopreserved sample was taken from the -80°C refrigerator. After thawing, 0.5 % BSA (w/v) was added to the sample for dilution.

(3) The Bio-Plex system was calibrated with Bio-Plex ManagerTM.

(4) To the standard bottle, 250 µL of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1× with Bio-Plex detection buffer, and stored in the dark.

(7) The diluted magnetic beads were vortexed, and 50 µL of the magnetic beads was added to each well.

(8) The plate was washed twice with 100 µL of washing solution.

(9) The sample, standard, blank control and the control with known concentration were vortexed, and added in an amount of 50 µL to each well.

(10) The plate was sealed with a sealing film, and shaken on a high-frequency shaker at 850±50 rpm at room temperature for 30 min.

(11) In step (10), when the remaining 10 min of shaking time was left, the detection antibody was vortexed for 5 s and diluted to 1×.

(12) The plate was washed twice with 100 µL of washing solution.

(13) The diluted antibody was vortexed, and added in an amount of 250 µL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1×.

(17) The plate was washed twice with 100 µL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 μL of detection solution, the plate was sealed with a sealing film, and shaken on a high-frequency shaker at room temperature at 850±50 rpm for 30 s.

(22) After the sealing film was discarded, loading to machine was started.

Immunofluorescence detection of a-SMA

The method referred to: Jong-Sung Park, Yumin Oh, Yong Joo Park, et al., Targeting of dermal myofibroblasts through death receptor 5 arrests fibrosis in mouse models of scleroderma. Nat Commun. 2019 Mar 8;10(1):1128.

Results:

For the extracted total protein of subcutaneous tissue of mice, it was fount through the multi-factor detection system that after the M cell transplantation, the levels of inflammatory cytokines such as IL-17, IL-6 and TNF were significantly decreased, and the content of IL-10 was significantly increased.
The total protein of the subcutaneous tissue of mice was extracted and detected by ELISA kit. It was found that the content of MMP1 in the M cell transplantation group was significantly increased.
The expression level of smooth muscle actin (a-SMA) was detected by immunofluorescence detection, and it was found that the expression level of a-SMA was significantly decreased after the M cell transplantation.
The photos of mouse back on day 28 were shown in FIG. 130.
The HE staining photos of mouse skin were shown in FIG. 131.
The Masson staining photos of mouse skin were shown in FIG. 132.

Example 17: Evaluation of therapeutic activity of M cells against refractory skin lesions

Refractory skin damage is not a disease, but a phenomenon of skin damage caused by a variety of diseases or injuries, and manifested by repeated skin ulceration, loss of partial skin function, and easy generation of scars and other skin hyperplasia tissues. Common factors that lead to refractory skin damage include burns, diabetes, lupus erythematosus, and psoriasis. At present, there is no one-size-fits-all solution to these problems, because such damage is often accompanied by complex immune disorders and tissue regeneration disorders, and a single treatment plan cannot solve all problems.
Experimental animals: ZDF rats, male, 7 to 8 weeks old, purchased from Beijing Weitong Lihua Company.
All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.
Feeding conditions: Free food and water; 12/12h day and night alternation, 7:00 to 19:00 was daytime, room temperature was 22±2°C, relative humidity was 50% to 60%.
Preparation and culture of M cells: The embryonic stem cells were suspended with EB spheres and subjected to adherent differentiation, the M cells at P0 generation were obtained, passaged and screened, and cryopreserved at P3 generation for subsequent experiments.
The P3 generation M cells were resuscitated, digested and passaged, and used for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Roche Excellence Glucose Meter | Roche | ACCU-CHEK |

(continued)

| Reagent/Equi pment | Manufacturer | Cat. No. |
|---|---|---|
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Paraformaldehyde | LEAGENE | DF0135 |
| High-fat feed | Purina | 5008C |
| Blood glucose test strips | Johnson & Johnson | 1297006 |

Animal modeling: The ZDF rats were fed with normal diet for one week and then induced with high-fat diet. The changes of random blood glucose level of rats were detected every week, and when the random blood glucose level was $\geq$ 11.1 mmol/L, the induction of model was deemed to be successful.

After the model was successfully induced, the modeling of skin damage was performed. The ZDF rats were anesthetized with a gas anesthesia machine. After anesthesia, the hair on the back was removed, and the skin was wiped with gauze sprayed with alcohol. The skin on the right side of the back was cut off with scissors, the size was $2\times2$ cm, resulting in a full-thickness skin defect. The rats were then grouped. The photos were taken on the 7th, 14th, 21st, and 28th days, and the perfusion and sampling were performed on the 28th day. The skin samples were sectioned and identified by HE staining.

Control group: normal saline was injected at four points around the wound, i.e., up, down, left and right points, 50 $\mu$l of normal saline per point, and then 3M bandage was applied.

M cell group: normal saline was injected at four points around the wound, i.e., up, down, left and right points, 50 $\mu$l of normal saline per point, in which the 50 $\mu$l of normal saline contained $7.5\times10^5$ M cells (at P5 generation), and then 3M bandage was applied.

Sample collection:

When collecting the specimens, after the rats were intraperitoneally anesthetized, the rats were in a supine position, the skin was cut in the middle of the abdomen of the rat, the chest was opened, the heart was exposed, and the heart was perfused with ice-cold normal saline. Each rat needed about 50ml of normal saline, after the normal saline perfusion was completed, 50ml of paraformaldehyde was used for fixation. After the perfusion was completed, the skin at the damaged area was cut off, fixed, sectioned and analyzed.

Steps for tissue paraffin sectioning

(1) Fixation: The tissue was socked in 4% PFA and fixed overnight.

(2) Cleaning: The fixed tissue was washed 3 times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.

Masson staining

(1) Dewaxing paraffin sections to water: The sections were placed in xylene I for 20 minutes, xylene II for 20 minutes, anhydrous ethanol I for 10min, anhydrous ethanol II for 10min, 95% alcohol for 5min, 90% alcohol for 5min, 80% alcohol for 5min, 70% alcohol for 5min in sequence, and washed with distilled water.

(2) Hematoxylin staining of nuclei: staining was performed for 5 min with Weigert's iron hematoxylin in the Masson staining kit; after being washed with tap water, differentiation was performed with 1% hydrochloric acid-alcohol for several seconds, rinsing was performed with tap water, and returning to blue was achieved by rinsing with running water for several minutes.

(3) Ponceau red staining: staining was performed for 5 to 10 min with Ponceau red acid fuchsin solution in the Masson staining kit, and rinsing was quickly performed with distilled water.

(4) Phosphomolybdic acid treatment: the treatment with phosphomolybdic acid aqueous solution in the Masson staining kit was performed for about 3 to 5 min.

(5) Aniline blue staining: instead of washing with water, counterstaining was performed for 5 min with aniline blue solution in the Masson staining kit.

(6) Differentiation: the treatment with 1% glacial acetic acid was performed for 1 min.

(7) Dehydration and mounting: the sections were placed in 95% alcohol I for 5min, 95% alcohol II for 5min, absolute ethanol I for 5min, absolute ethanol II for 5min, xylene I for 5min, xylene II for 5min in sequence to perform dehydration and transparentizing, then the sections were taken out from xylene and slightly air-dried, and mounted with neutral resin.

(8) Microscopic examination was performed with a microscope, and images were acquired and analyzed.

Immunohistochemical staining:

Immunohistochemical staining was performed on the paraffin sections using an immunohistochemical kit (Fuzhou Maixin, KIT-9710). The specific steps were as follows:

1. Dewaxing: (1) xylene I, II, 10 min each; (2) gradient alcohol: 100% absolute ethanol, 2 min; 95% absolute ethanol, 2 min; 80% absolute ethanol, 2 min; 70% absolute ethanol, 2 min;

2. Hydration: washing was performed twice with distilled water, 5min each time (placed on a shaker);

3. After deparaffinization and hydration of the paraffin sections, rinsing was performed 3 times with PBS, 3 minutes each time;

4. Preparation of antigen retrieval solution (10 mM pH 6.0 sodium citrate buffer):

(1) Preparation of stock solution: Solution A: 29.41 g of trisodium citrate dihydrate + 1,000 mL of distilled water; Solution B: 21 g of citric acid + 1,000 mL of distilled water;

(2) Preparation of working solution: 82 mL of Solution A + 18 mL of solution B + 900 mL of distilled water;

5. Antigen retrieval: the sections were placed in a plastic or heat-resistant glass container filled with sodium citrate buffer, the sections were immersed, treated with a microwave oven at mid-range or high-range power for 5 minutes; sodium citrate buffer was replenished, and treatment was performed again at mid-range or high-range power for 5 minutes;

6. Reagent A (peroxidase blocking solution) was added, and incubated at room temperature for 10 min to block the activity of endogenous peroxidase; rinsing was performed with PBS 3 times, 3 min each time;

7. PBS was discarded, 1 drop or 50 μL of Reagent B (normal non-immune animal serum) was added, and incubated at room temperature for 10 min;

8. The serum was discarded, 1 drop or 50 μL of primary antibody was added, and incubated at 4°C overnight or at room temperature for 60 min; rinsing was performed with PBS 3 times, 3 min each time;

9. The PBS was discarded, 1 drop or 50 μL of biotin-labeled secondary antibody (Reagent C) was added, and incubated at room temperature for 10 min; rinsing was performed with PBS 3 times, 3 min each time;

10. The PBS was discarded, 1 drop or 50 μL of streptavidin-peroxidase solution (reagent D) was added, incubated at room temperature for 10 min; rinsing was performed with PBS 3 times, 3 min each time;

11. The PBS was discarded, 2 drops or 100 μL of freshly prepared DAB solution was added, and observation was performed under microscope for 3 to 10 min;

12. Rinsing was performed with tap water, counterstaining was carried out with hematoxylin, and rinsing was performed with PBS or tap water so as to return to blue;

13. When using DAB for color development, the sections should be dehydrated with gradient alcohol and dried, transparentizing was performed with xylene, and mounting was performed with neutral resin;

14. Photos were taken with a microscope.

Immunofluorescence staining:

(1) The tissue sections were dewaxed and transferred into water;

(2) Antigen microwave retrieval was performed at a temperature of 92°C to 96°C for 10 to 15min, naturally cooled to room temperature;

(3) Normal goat serum blocking was performed at 37°C for 60 min;

(4) The excess serum was poured off, primary antibody was added dropwise, allowed to stand at 37°C for 2 hours or 4°C overnight, rinsing was performed with PBS, 5min×3 times;

(5) The fluorescein-labeled secondary antibody was added dropwise, allowed to stand in the dark at 37°C for 60 min, rinsing was performed with 0.01M PBS, 5 min×3 times;

(6) The sections were mounted with anti-quenching mounting medium, and stored at 4°C in the dark.

(7) Observation and photoing were performed with a fluorescence microscope.

Conclusions:

The light microscope photos of skin wounds at different time points after modeling were shown in FIG. 133.

The statistics of the wound area size of FIG. 133 were shown in FIG. 134.

The unhealed rates of wounds were shown in FIG. 135.

Table 17-1 showed the statistical values of the skin wound areas on the 7th, 14th, 21st, and 28th days after modeling. It could be seen from the table that on the 7th day, the areas of the two groups tended to be the same, but on the 28th day, the area of the M cell group was lower than that of the model group. It showed that the M cells had therapeutic effect on skin injury and could accelerate wound healing.

Table 17-1: Statistics of wound area values

|  | Wound area ($cm^2$) | | | |
|---|---|---|---|---|
| Time (days) | Model group | | M cell group | |
| 7 | 6.52 | 6.20 | 6.15 | 6.30 |
| 14 | 1.79 | 1.82 | 1.34 | 1.58 |
| 21 | 1.42 | 1.63 | 0.99 | 0.90 |
| 28 | 1.37 | 1.41 | 0.43 | 0.74 |

Table 17-2 showed the statistics of the unhealed proportion of skin wounds at different days.

Table 17-2: Statistics on proportion of unhealed wounds

|  | Statistics on the proportion of unhealed wounds (%) | | | |
|---|---|---|---|---|
| Time (days) | Model group | | M cell group | |
| 7 | 0.00 | 0.00 | 0.00 | 0.00 |
| 14 | 27.39 | 29.35 | 21.77 | 25.13 |
| 21 | 21.76 | 26.32 | 16.07 | 14.27 |
| 28 | 21.03 | 22.74 | 7.02 | 11.75 |

4. On the skin at the modeling site of the rats, the immunohistochemical staining was performed on the 5th and 7th days. The expressions of CD3, F4/80, and MPO in the M cell group were significantly lower than those in the model group, indicating that the M cells could relieve the skin inflammation at the site of injury, and suppresses inflammation. The expression of K14 in the M cell group was significantly higher than that in the model group, indicating that the M cells could accelerate the epidermalization of wounds after skin injury, accelerate wound healing, and play an effective therapeutic role in skin injury.

5. The immunofluorescence identification was performed on the rat skin sections on the 14th day, which showed that the expression of CD31 marker in the M cell group was significantly higher than that in the control group, indicating that the M cell treatment could promote the vascular regeneration of skin wounds and have an important effect on the regeneration of skin after injury.

6. The immunofluorescence staining of skin wound sections showed that the expressions of $\beta$-Catenin, CD133 and Ki67 in the M cell group were significantly higher than those in the model group, indicating that in the M cell group, there were more hair follicles, and the M cell treatment could promote the regeneration of hair follicles after skin injury.

In the treatment of diabetic with M cells in mice, we found that the M cell transplantation treatment could have the following effects on the complications of diabetes:

Diabetic nephropathy: The M cell transplantation could reduce the expression of proinflammatory factors IL-1$\beta$, IL-6 and TNF$\alpha$, could reduce mesangial thickening and macrophage infiltration, reduce diabetes-induced glomerulopathy, increase rat kidney weight, kidney and body mass index, so that the M cells could have a good therapeutic effect on diabetic nephropathy.

Diabetic foot: The M cell treatment could accelerate the healing of diabetic foot, reduce the inflammation of skin wounds, promote the regeneration of blood vessels and hair follicles, reduce the deposition of collagen, and inhibit the occurrence of fibrosis. Therefore, the M cells could effectively treat skin damage.

Diabetic eye complications: The M cell treatment could lower blood sugar and regulate inflammatory response, significantly reduce fasting blood glucose and HbAlc levels, and improve visual function and macular edema, so that the M cell transplantation could well treat diabetic eye complications.

Vascular calcification complicated by diabetes: The M to cell transplantation could inhibit vascular calcification, so that it has a good therapeutic effect on the vascular calcification in the complications.

Diabetic neuropathy: The intravenous injection of M cells could enhance the ability of astrocytes to resist oxidative stress, enhance their ability to clear glutamate in the brain, and maintain K+ balance in the brain, thereby promoting neuronal function, brain homeostasis and synaptogenesis, and improving cognitive impairment caused by diabetes.

In conclusion, the M cell treatment can accelerate the healing of skin wounds, reduce the inflammation of skin wounds, promote the regeneration of blood vessels and hair follicles, reduce the deposition of collagen, and inhibit the occurrence of fibrosis. Therefore, the M cells could effectively treat skin damage. In addition, the M cell transplantation treatment could also have a good therapeutic effect on diabetic complications.

Example 18: Evaluation of therapeutic activity of M cells against anemia

Anemia is not an independent disease, but refers to a state in which the oxygen-carrying capacity of the blood is reduced, resulting in insufficient oxygen supply and tissue hypoxia. The etiology and pathogenesis of anemia itself are complex and diverse, and may involve a variety of factors and systemic diseases. The basic etiology can be summarized into three aspects, including reduced or insufficient erythropoiesis, excessive destruction of red blood cells, and blood loss. Cancers are a common cause of anemia, and 50% of cancer patients have anemia, which not only brings a variety of clinical symptoms, but also reduces the life quality of patients, which is also one of the factors affecting the prognosis. At the same time, it may also cause many adverse consequences due to the increase of blood transfusion. Tumor-associated anemia is a result of multiple factors, most of which are caused by the tumor itself, which belongs to chronic anemia; in addition, the use of cytotoxic drugs or nephrotoxic drugs for chemotherapy may also cause anemia. Cisplatin is a widely used chemotherapeutic drug in patients, and has renal toxicity. When the cumulative dose of cisplatin increased, anemia would be exacerbated. Although erythropoietin may relieve chronic cancer anemia, it has been reported in the literature that its effective rate for correcting anemia is about 60%. Hence, it is still an important topic how to further improve the anemia of tumor patients and improve the prognosis and lift quality of the patients.

Experimental animals:

Male Sprague-Dawley rats, 6 to 8 weeks old, purchased from Weitong Lihua (Beijing) Biotechnology Co., Ltd.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells

The embryonic stem cells were suspended with EB spheres and subjected to adherent differentiation, and the M cells at the P0 generation were obtained, passaged and screened, and cryopreserved at the P3 generation for subsequent experiments.

The P3 generation M cells were resuscitated, digested and passaged, and used for subsequent experiments.

Experimental reagents and equipment

| Reagent/Equi pment | Manufacturer | Cat. No. |
|---|---|---|
| R540 Enhanced small animal anesthesia machine | Ruiwode | R540 |
| Isoflurane | Ruiwode | 970-00026-00 |
| Disposable sterile syringe 1ml | Jiangsu Zhiyu Medical Equipment Co., Ltd. | None |

(continued)

| Reagent/Equi pment | Manufacturer | Cat. No. |
|---|---|---|
| Disposable sterile syringe 5ml | Jiangsu Zhiyu Medical Equipment Co., Ltd. | None |
| Normal saline | SSY Group Limited | None |
| Paraformaldehyde | LEAGENE | DF0135 |
| Cisplatin | Sigma-Aldrich | 15663-27-1 |
| Busulfan | Sigma | B2635-10G |
| Animal blood routine analyzer | Hailifu | HF-3800 |

Animal modeling:

Female rats were divided into three groups: control group, cisplatin-induced anemia group, and cisplatin-induced anemia + M cell treatment group, 4 rats in each group. The anemia model group was given intraperitoneal injection of cisplatin (150 mg/kg) and intragastric administration of busulfan in distilled water suspension (15 mg/kg) on the 5th day, once a week; the control group was given the same as the anemia model group, but the intragastric administration used normal saline instead of busulfan in distilled water suspension. Three days after the first induction of cisplatin, the cell therapy group began to receive the intravenous infusion of cell drug, $5 \times 10^6$ cells/rat, for a total of two treatments, with a one-week interval for each treatment. After the start of the experiment, the body weight was weighed twice a week, and 18 days after the first cell infusion, the whole blood of the rat was collected for routine blood test.

Statistics: All data were analyzed by T.test for variance analysis and significance test, and experimental data were expressed as mean $\pm$ standard deviation (Mean $\pm$ SD). *, $p<0.05$; **, $p<0.01$; ***, $p<0.001$.

1. Body weight loss in cisplatin-induced anemia model rats

Experimental method: At different time points of the model induction, the rats were taken for weight measurement on an electronic scale.

Experimental results:

FIG. 136 showed the trend of body weight change of anemia model rats.

Table 18-1 showed that the body weight was detected at different time points for the cisplatin-induced rat anemia model, and the data were subjected to statistical analysis.

Table 18-1: Analysis of body weight data of cisplatin-induced anemia model rats

| Group | | Normal control group+solvent | | | | Cisplatin+solvent | | | | Cisplatin+M cells | | | |
|---|---|---|---|---|---|---|---|---|---|---|---|---|---|
| Body weight (g) | Day 1 | 244 | 246 | 250 | 250 | 243 | 241 | 249 | 250 | 240 | 248 | 252 | 252 |
| | Day 4 | 271 | 282 | 277 | 282 | 203 | 207 | 210 | 222 | 225 | 228 | 219 | 204 |
| | Day 7 | 306 | 304 | 307 | 311 | 200 | 199 | 188 | 196 | 225 | 218 | 205 | 196 |
| | Day 11 | 356 | 348 | 360 | 361 | 230 | 230 | 215 | 207 | 248 | 243 | 228 | 233 |
| | Day 14 | 396 | 368 | 385 | 391 | 239 | 239 | 232 | 216 | 267 | 260 | 238 | 239 |
| | Day 18 | 407 | 403 | 419 | 420 | 248 | 250 | 255 | 228 | 292 | 292 | 253 | 254 |
| | Day 21 | 437 | 425 | 427 | 443 | 244 | 273 | 270 | 228 | 312 | 324 | 269 | 267 |

2. Blood biochemical testing

Experimental method: On the 21st day of model induction, the blood of rats was collected, and various indexes of blood routine were detected on the animal blood routine analyzer.

Experimental results:

FIG. 137 showed the total number analysis of the leukocytes in peripheral blood of cisplatin-induced anemia model rats. FIG. 138 showed the total number analysis of the red blood cells in peripheral blood of cisplatin-induced anemia model rats. FIG. 139 showed the analysis of peripheral hemoglobin content in cisplatin-induced anemia model rats. FIG. 140 showed the peripheral blood hematocrit analysis of cisplatin-induced anemia model rats. FIG. 141 showed the analysis of the average hemoglobin concentration of peripheral blood of cisplatin-induced anemia model rats. FIG. 142 showed the analysis of the volume distribution width of peripheral blood red blood cells in cisplatin-induced anemia model rats. FIG. 143 showed the analysis of the distribution width of peripheral blood hemoglobin content in cisplatin-induced anemia model rats.

Table 18-2 showed that the blood collection was performed on the 21st day of the cisplatin-induced rat anemia model, followed by the routine blood biochemical analysis. The statistics were shown in the table below.

Table 18-2: Analysis of routine blood biochemical data of cisplatin-induced anemia model

| Group | WBC (*10^9 cells/L) | RBC (*10^6 cells/μL) | HGB (g/L) | HCT (%) | MCHC (g/L) | RDW (%) | HDW (g/L) |
|---|---|---|---|---|---|---|---|
| Normal control | 23.46 | 7.80 | 162 | 51.6 | 320 | 11.4 | 22.0 |
| group + solvent | 13.42 | 7.80 | 155 | 49.8 | 318 | 11.4 | 22.4 |
| | 15.62 | 7.64 | 160 | 50.0 | 320 | 11.5 | 23.1 |
| | 14.49 | 7.82 | 162 | 51.4 | 315 | 11.5 | 23.8 |
| Cisplatin +solvent | 11.14 | 2.03 | 40 | 15.0 | 267 | 31.0 | 54.7 |
| | 11.53 | 3.75 | 73 | 24.2 | 300 | 18.3 | 36.4 |
| | 10.52 | 3.29 | 71 | 25.4 | 280 | 28.5 | 36.7 |
| | 12.66 | 2.49 | 56 | 21.8 | 258 | 36.4 | 44.0 |
| Cisplatin +M cells | 17.36 | 5.75 | 112 | 36.7 | 306 | 14.8 | 27.0 |
| | 14.18 | 4.28 | 87 | 29.8 | 311 | 13.5 | 25.1 |
| | 14.80 | 5.47 | 109 | 35.1 | 312 | 16.7 | 30.6 |
| | 22.47 | 4.68 | 98 | 31.4 | 312 | 14.6 | 30.4 |

3. Bone marrow cytology

Experimental method: After the myelogram examination and the abdominal aorta blood sampling were completed, the muscles of the thighs were cut off with surgical scissors, the femur was fully exposed, the femur was cut with large scissors, and the bone marrow was squeezed out with medium-sized forceps. If it was difficult to take out bone marrow, a straight end ophthalmic tweezers could be inserted into the bone marrow cavity to pick out the bone marrow, and the bone marrow was placed on a clean glass slide.

Experimental results: Compared with the results of the bone marrow smear of the control group, the bone marrow smear in the cisplatin+solvent group was significantly fatty, the number of cells decreased, the number of non-hematopoietic cells increased, the proliferation of bone marrow nucleated cells was extremely low, the number of cells was sparse, the number of hematopoietic cells was extremely low, the oil droplets on the bone marrow smear increased significantly, that was, the number of vacuoles under the microscope increased significantly, and many large or extra-large vacuoles appeared, showing symptoms of anemia. In the cisplatin+M cell treatment group, the bone marrow proliferation was active and adipocytes were less. The above results showed that the M cell treatment had a significant promoting effect on the myelogram recovery in anemia treatment.

Example 19: Evaluation of therapeutic activity of M cells against pulmonary hypertension

Pulmonary hypertension (PH) is a hemodynamic abnormality in which the pulmonary arterial pressure exceeds a certain threshold. The patients are accompanied by major symptoms such as weakness and dyspnea, the course of the disease progresses rapidly without treatment, and often develops to right heart failure, which leads to death. It is characterized by pulmonary vascular remodeling, vascular occlusion-induced pulmonary vascular resistance (PVR), increased pulmonary artery pressure and right ventricular hypertrophy. At present, the most effective treatment method is drug therapy, including three categories of drugs: prostacyclin, endothelin-1 receptor antagonists, and phosphodiesterase type 5 inhibitors. Although these drugs can improve the condition, they do not fundamentally improve the pulmonary vascular remodeling, and the overall costs is high, which cannot meet the needs of long-term treatment.

In the past 10 years, stem cell treatment has shown great potential. Cell therapies such as endothelial progenitor cells (EPCs) and mesenchymal stem cells (MSCs) have achieved breakthrough research results in animal experiments. More and more studies have proved that stem cell therapies have a certain effect on the treatment of pulmonary hypertension. Among various types of stem cells, MSCs are the most widely studied and most valuable for regenerative medicine research. When monocrotaline (MCT)-induced PH rats were treated with MSCs, hemodynamics and pulmonary vascular remodeling could be improved. Some researchers found that the genetically modified MSCs could alleviate MCT-induced PH endothelial dysfunction by secreting calcitonin gene-related peptides. After transplantation of HGF gene-modified MSCs into MCT-induced PH rats, the cardiopulmonary dynamic indexes were also significantly improved, which were better than those of the monotherapy with MSCs. At present, the research on MSCs has achieved phased results, but in the treatment of PH, its unknown intervention mechanism as compared with EPCs has become the main factor limiting the clinical application of MSCs. However, the clinical application of adult tissue-derived MSCs mainly has the following disadvantages: (1) a therapeutically effective amount of adult tissue-derived MSCs cannot be obtained from a single individual tissue; (2) the adult tissue-derived MSCs are derived from different individual tissues, which cannot achieve teh required consistency of product quality; (3) even MSCs derived from the same individual tissue are still highly heterogeneous; (4) the donor tissue sources of adult tissue-derived MSCs are complex and have potential infectious pathogen infection risks; (5) the adult tissue-derived MSCs rapidly senesce with in vitro expansion. Therefore, new cell sources for MSCs are needed for the treatment of pulmonary hypertension.

Experimental animals:

SD rats, male, 6 to 8 weeks old. The animals were purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells:

The embryonic stem cells were suspended with EB spheres and subjected to adherent differentiation, and the M cells at P0 generation were obtained, passaged and screened, and cryopreserved at the P3 generation for subsequent experiments.

The P3 generation M cells were resuscitated, digested and passaged, and used for subsequent animal experiments.

| Reagent/Equi pment | Manufacturer | Cat. No. |
|---|---|---|
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Water bath | Saiou Huachuang | SDY-1 |
| Normal saline | Shijiazhuang No. 4 Pharmaceutical Co., Ltd. | None |

(continued)

| Reagent/Equi pment | Manufacturer | Cat. No. |
|---|---|---|
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |
| Monocrotaline (MCT) | Selleck | S3812 |
| B-Ultrasonic scanner for small animals | VisualSonics | Vevo LAZR |
| Multifactor Suspension Chip System | Bio-Rad | Bio-Plex® 200 |
| 24-Factors Kit | Bio-Rad | 171-K1001M |

Animal grouping:

| Group | Modeling | Animal number | Test drug | Administration volume | Administration times | Administration route |
|---|---|---|---|---|---|---|
| Control group | Normal saline | 5 | - | 200 μL | Once | Tail vein injection |
| MCT group | One-time intraperitoneal injection of 60mg/kg monocrotaline | 5 | Normal saline | 200 μL | Once | Tail vein injection |
| MCT + M cell group | One-time intraperitoneal injection of 60mg/kg monocrotaline | 5 | $5×10^6$ cells/200 μL/rat | 200 μL | Once | Tail vein injection |

Pulmonary arterial hypertension modeling: one-time intraperitoneal injection of 60 mg/kg monocrotaline.

Cell injection:

One week after the MCT injection, rat tail vein injection of $5×10^6$ cells/rat was performed, and ultrasonographic evaluation and sampling were performed two weeks later.

Ultrasonographic evaluation:

The short-axis section of parasternal aorta was taken, the pulse doppler sampling volume was placed on the pulmonary valve, and the pulmonary valve systolic blood flow spectrum and electrocardiogram were recorded simultaneously, and time from the starting point to the highest point of pulmonary artery systolic blood flow spectrum was the pulmonary artery blood flow acceleration time (PAT).

The short-axis section of parasternal cardiac base aorta was taken, the pulmonary artery regurgitation spectrum was obtained with continuous doppler sampling lines, the spectrum was divided into three equal parts by time, namely early, middle and late stages of diastole, and the maximum pulmonary valve regurgitation pressure difference in the early stage of diastole was measured, that was the mean pulmonary artery pressure.

The parasternal short-axis section was taken, and the inner diameter of the pulmonary artery was measured.

The four-chamber view in two-dimensions was taken, the basal part inner diameters of the right ventricle and the left ventricle were measured, and the basal part inner diameter ratio of the right ventricle to the left ventricle (RV/LV) was calculated.

Measurement of right ventricular systolic blood pressure:

A blood pressure monitor was used for hemodynamic testing. After tracheal intubation, the chest was opened, and a 0.7mm × 19mm closed indwelling needle was inserted 5 mm into the apex of heart to measure the right ventricular pressure.

Sample collection:

When collecting the specimens, the rats were intraperitoneally anesthetized and placed in a supine position, the skin was cut in the middle of the abdomen of the rats, the abdominal cavity was opened, and blood was collected from the central vein. The chest was opened, the heart was exposed, and the heart was perfused with ice-cold normal saline. After the normal saline perfusion was completed, the fixation was performed with 50 ml of paraformaldehyde. After the perfusion was completed, the lungs were taken, fixed, sectioned and analyzed.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 48-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex ManagerTM.

(4) To the standard bottle, 250 μL of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1 time with Bio-Plex detection buffer, and stored in the dark.

(7) The diluted magnetic beads were vortexed, and 50 μL of the magnetic beads was added to each well.

(8) The plate was washed twice with 100 μL of washing solution.

(9) The sample, standard, blank, and control of known concentration were vortexed, and added in an amount of 50 μL to each well.

(10) The plate was sealed with a sealing film, and shaken on a high-frequency shaker at 850±50 rpm at room temperature for 30 min.

(11) In step (10), when the remaining 10 min of shaking time was left, the detection antibody was vortexed for 5 s and diluted to 1 time.

(12) The plate was washed twice with 100 μL of washing solution.

(13) The diluted antibody was vortexed, and added in an amount of 250 μL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1 time.

(17) The plate was washed twice with 100 μL of washing solution.

(18) The diluted SA-PE was vortexed, and added in an amount of 50 $\mu$L to each well.

(19) The plate was sealed with a sealing film, and shaken on a high-frequency shaker at 850$\pm$50 rpm at room temperature for 30 min.

(20) The plate was washed three times with 100 $\mu$L of washing solution.

(21) The magnetic beads were resuspended with 125 $\mu$L of detection solution, the plate was sealed with a sealing film, and shaken on a high-frequency shaker at room temperature at 850$\pm$50 rpm for 30 s.

(22) After the sealing film was discarded, loading to machine was started.

Steps for tissue paraffin sectioning:

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Statistical analysis:

One-way ANOVA and T-TEST in Prism 7.0 statistical analysis software were used for variance analysis and significance test, and the experimental data were expressed as mean $\pm$ standard error (Mean $\pm$ SE). *, $p<0.05$; **, $p<0.01$; ***, $p<0.001$.

2. Experimental results

(1) The measurement results of right ventricular blood pressure showed that the right ventricular systolic blood pressure in the MCT group was significantly higher than that in the control group. Compared with the MCT group, the right ventricular systolic blood pressure in the MCT+M cell group was significantly lower. The results showed that the M cells could reduce right ventricular systolic blood pressure in the rats with pulmonary hypertension.

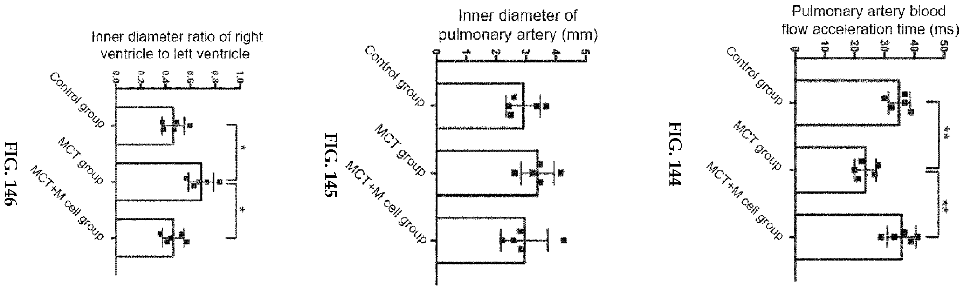

(2) The ultrasonography results showed that after injection of MCT, compared with the control group, the pulmonary artery blood flow acceleration time in the MCT group became shorter, the diameter ratio of the right ventricle to the left ventricle became larger, and the mean pulmonary artery pressure increased, indicating the occurrence of pulmonary hypertension in the MCT group (Table 19-1 to Table 19-4, FIG. 144 to FIG. 147). After infusion of the M cells, compared with the MCT group, the pulmonary artery blood flow acceleration time in the MCT+M cell group increased, the diameter ratio of the right ventricle to the left ventricle decreased, and the mean pulmonary artery pressure decreased, indicating that the M cells could inhibit the formation of pulmonary hypertension (Table 19-1 to Table 19-4, FIG. 144 to FIG. 147). However, the right ventricular outflow tract diameter did not vary between groups (Table 19-2, FIG. 145). It showed that the M cells could increase the pulmonary artery blood flow acceleration time, reduce the diameter ratio of the right ventricle to the left ventricle, and reduce the mean pulmonary artery pressure.

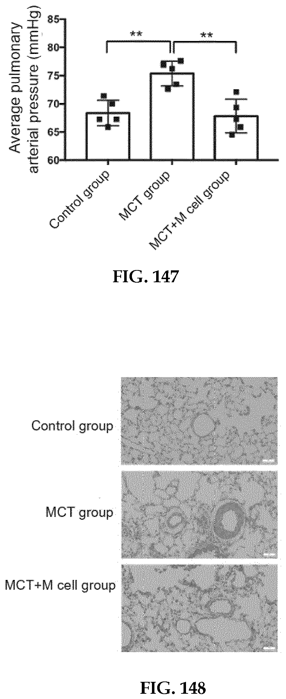

(3) The results of HE staining showed that the fibroblasts proliferated massively in the pulmonary artery wall in the MCT group, but did not proliferate in the MCT+M cell group (FIG. 148). The pulmonary arteriole intima-media thickness in the MCT group was significantly greater than that in the MCT+M cell group. At the same time, the ratio of pulmonary arteriole wall area/total pulmonary artery area in the MCT group was significantly higher than that in the MCT+M cell group. These results showed that the M cells could reduce the pulmonary arteriole intima-media thickness and the pulmonary arteriole wall area in the pulmonary arterial hypertension rats.

(4) The inflammatory factors in the serum of each group were detected. The results showed that compared with the MCT group, the levels of proinflammatory factors in the M cell treatment group were significantly decreased, and the levels of anti-inflammatory factors were significantly increased. It was shown that the M cells had the effect of inhibiting inflammation.

Table 19-1: Ultrasonography measurement of pulmonary artery blood flow acceleration time in each group

| | Control group | | | | | MCT group | | | | | MCT+M cell group | | | | |
|---|---|---|---|---|---|---|---|---|---|---|---|---|---|---|---|
| Pulmonary artery blood flow acceleration time (ms) | 30.00 | 38.89 | 36.67 | 36.67 | 32.22 | 26.67 | 21.11 | 22.22 | 28.00 | 20.00 | 36.67 | 33.33 | 28.89 | 41.11 | 38.89 |

Table 19-2: Ultrasonography measurement of pulmonary artery diameter in each group

| | Control group | | | | | MCT group | | | | | MCT+M cell group | | | | |
|---|---|---|---|---|---|---|---|---|---|---|---|---|---|---|---|
| Pulmonary artery inner diameter (mm) | 2.43 | 3.36 | 3.68 | 2.60 | 2.49 | 3.49 | 4.18 | 2.62 | 3.47 | 3.22 | 2.20 | 4.27 | 2.84 | 2.81 | 2.5833 |

Table 19-3: Ultrasonography measurement of inner diameter ratio of right ventricle to left ventricle in each group

| | Control group | | | | | MCT group | | | | | MCT+M cell group | | | | |
|---|---|---|---|---|---|---|---|---|---|---|---|---|---|---|---|
| Right ventricle diameter/left ventricle diameter | 0.38 | 0.47 | 0.49 | 0.60 | 0.37 | 0.73 | 0.63 | 0.57 | 0.67 | 0.83 | 0.58 | 0.36 | 0.52 | 0.43 | 0.41 |

Table 19-4: Ultrasonography measurement of mean pulmonary artery pressure in each group

| | Control group | | | | | MCT group | | | | | MCT+M cell group | | | | |
|---|---|---|---|---|---|---|---|---|---|---|---|---|---|---|---|
| Mean pulmonary artery pressure (mmHg) | 71.40 | 65.89 | 67.27 | 67.27 | 70.02 | 73.47 | 76.91 | 76.22 | 72.64 | 77.60 | 67.27 | 69.33 | 72.09 | 64.51 | 65.89 |

Example 20: Evaluation of therapeutic activity of M cells against spinal cord injury

Spinal cord injury (SCI) is a traumatic disease of spinal surgery caused by trauma, which manifests as sensory, motor and autonomic dysfunction below the injured segment. Foreign epidemiological surveys show that there are 130,000 new spinal cord injury patients worldwide each year, and more than 2.5 million patients are suffering from different degrees of spinal cord injury sequelae, and the annual medical expenditure of these patients will exceed 6 billion US dollars, placing a heavy burden on the families and community.

Experimental animals: Wistar rats, male, 7 to 8 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and P5 generation was used for subsequent experiments.

| Reagent/Equi pment | Manufacturer | Cat. No. |
|---|---|---|
| Micro Vascular Clip- Straight/26mm | Ruiwode | R31008-26 |
| Sterilized suture with needle non-absorbable 5-0 | domestic | F504 |
| Sterilized suture with needle non-absorbable 3-0 | domestic | 18-TL-530 3 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | SSY Group Limited | None |
| 15ml centrifuge tube | Corning | 430791-BP |
| Iodophor | Hangzhou Langsuo Medical Disinfectant Co., Ltd. | None |

Animal modeling: The Wistar rats were anesthetized, the back hair was removed, the T9 segment of the rat spinal cord was found, the skin and muscles of the back were cut with scissors, the vertebral plate was exposed, the T9 segment vertebral plate was pried off with needle forceps, and the spinal cord of the T9 segment was clamped with a vascular clamp for 90s. After the clamping, the muscle layer and the skin were sutured and wiped with iodine. The sutured rat was plated on the rat electric blanket, and put into the cage after the rat waked up. One week later, the rats were subjected to BBB scoring and grouped. After that, the BBB scoring was performed weekly.

Grouping: model group, M cell group, 3 rats in each group.

Model group: Intravenous injection with 300ul of normal saline was performed.

M cell group: Intravenous injection with 300ul of normal saline containing $3 \times 10^6$ cells at P5 generation was performed.

BBB (Basso, Beattie & Bresnahan locomotor rating scale, BBB scale) behavioral scoring: The rats were placed in an open behavior box, and forced to crawl by tapping the box wall, and the animal's hip, knee, ankle joints walking, trunk movements and coordination thereof were videotaped for 4 minutes for the subsequent BBB scoring.

BBB scoring (Basso, Beattie & Bresnahan locomotor rating scale, BBB scale) (rat spinal cord injury) criteria:

0 points: no visible hindlimb movement was observed.

1 point: one or two joints, usually hip and/or knee, were slightly movable.

2 points: one joint was greatly movable, or one joint was greatly movable and another joint was slightly movable.

3 points: two joints were greatly movable.

4 points: all three joints of hind limbs were slightly movable.

5 points: two joints were slightly movable, and the third joint was greatly movable.

6 points: two joints were greatly movable, and the third joint was slightly movable.

7 points: all three joints of hind limbs were greatly movable.

8 points: paws could land on the ground under non-load bearing condition.

9 points: soles of feet were only in the weight-bearing position, or weight-bearing walking with dorsum of feet occurred occasionally/frequently/continuously, no weight-bearing walking with soles of feet occurred. Weight-bearing: HL extensors contracted when soles of feet were in weight-bearing position or only rear torso was elevated.

10 points: weight-bearing movement with paw surface occurred occasionally; no coordinated movement of fore and hind limbs was observed.

11 points: more weight-bearing movements with palm surface occurred, but no coordinated movement of fore and hind limbs was observed.

12 points: more weight-bearing movements with palm surface occurred, and coordinated movement of fore and hind limbs was occasionally observed.

13 points: weight-bearing movements with palm surface were frequently observed, and coordinated movements of fore and hind limbs was frequently observed.

14 points: weight-bearing movements with palm surface and coordinated movements of fore and hind limbs occurred continuously; or frequent movements with palm surface, continuous coordinated movement of fore and hind limbs, and occasional movement with dorsal part of claw were observed.

15 points: weight-bearing movements with palm surface and coordinated movements of fore and hind limbs occurred continuously, not or occasionally grasping ground was observed during the forward movement with forelimbs; the position of active claw was parallel to the body at the initial contact.

16 points: continuous movement with palm surface and continuous coordinated movements of fore and hind limbs were observed in the gait, and frequently grasping ground was observed during the forward movement with forelimbs; the position of active claw was parallel to the body at the initial contact, and rotated after weight-bearing transfer.

17 points: continuous movement with palm surface and continuous coordinated movements of fore and hind limbs were observed in the gait, and frequently grasping ground was observed during the forward movement with forelimbs; the position of active claw was parallel to the body at the initial contact and after weight-bearing transfer.

18 points: continuous movement with palm surface and continuous coordinated movements of fore and hind limbs were observed in the gait, and continuously grasping ground was observed during the forward movement with forelimbs; the position of active claw was parallel to the body at the initial contact, and rotated after weight-bearing transfer.

19 points: continuous movement with palm surface and continuous coordinated movements of fore and hind limbs were observed in the gait, and continuously grasping ground was observed during the forward movement with forelimbs; the position of active claw was parallel to the body at the initial contact and after weight-bearing transfer. The tail was sometimes or always drooping.

20 points: continuous movement with palm surface, continuous coordinated gait, and continuously grasping ground with toes were observed, the position of active claw was always parallel to the body at the initial contact and after weight-bearing transfer, the torso was not stable, and the tail was continuously raised.

21 points: continuous movement with palm surface, continuous coordinated gait, and continuously grasping ground with toes were observed, the position of active claw was always parallel to the body during the movement, the torso was continuously stable, and the tail was continuously raised.

The rat BBB scores were shown in FIG. 149.

Table 20-1 showed the behavioral BBB scores of rats from week 1 to week 7 after modeling. It could be concluded from the table that the BBB scores of the M cell treatment group were significantly higher than those of the model group, indicating that the intravenous injection of M cells could effectively treat spinal cord injury.

Table 20-1: Statistics of BBB scores in rats

| | BBB score | | | | | |
|---|---|---|---|---|---|---|
| Time (weeks) | Model group | | | M cell group | | |
| 1 | 0 | 0 | 0 | 0 | 0 | 0 |
| 2 | 2 | 0 | 0 | 0 | 1 | 4 |
| 3 | 2 | 0 | 0 | 5 | 2 | 8 |
| 4 | 2 | 1 | 1 | 2 | 4 | 9 |
| 5 | 2 | 2 | 2 | 8 | 8 | 10 |
| 6 | 2 | 2 | 2 | 9 | 8 | 10 |
| 7 | 2 | 2 | 2 | 10 | 10 | 12 |

Mechanical pain and urinary system function:

The method was referred to Fandel et. al., 2016, cell stem cell.

The results showed that tactile allodynia and hyperalgesia were reduced in spinal cord injury model animals treated with the M cells. Using spontaneous voiding tests and conscious cystometry, the results showed that the M cell transplanted animals had a wider range of urine spot diameters, indicating that the animals regained partial bladder control and improved bladder function. At the same time, the animals transplanted with the M cells showed decreased bladder outlet resistance and detrusor hyperactivity, which corresponded to the improved voiding function.

Section staining:

Methods were referred to Fandel et. al., 2016, cell stem cell.

The results showed that the number of neurons (TUJ1+) near the injury site increased and the length of axons increased after the M cell treatment in spinal cord injury model animals, indicating that the M cells could promote cell survival and enhance axon regeneration. The number of glial cells decreased, and the expression of collagen was significantly reduced, indicating that the M cells had the effect of inhibiting glial cell activation and the function of anti-fibrosis. Moreover, the number of microglia was reduced, proving that the M cells could effectively reduce the inflammatory response at the injury site.

The intravenous injection of M cells could improve the exercise ability of spinal cord injured mice, and the BBB scores were significantly increased. It showed that the M cells could treat spinal cord injury very well.

Example 21: Evaluation of the therapeutic activity of M cells against stroke

Stroke is a type of cerebrovascular disease in which cerebral blood vessels are narrowed, blocked or ruptured, leading to ischemia or hemorrhage of cerebral tissue, resulting in necrosis of brain cells and tissues. It is divided into ischemic stroke (also known as cerebral infarction) and hemorrhagic stroke (including intraparenchymal hemorrhage, intraventricular hemorrhage, and subarachnoid hemorrhage). The incidence rates of ischemic stroke in men and women are 212/100,000 and 170/100,000; hemorrhagic stroke: 12-15/100,000. However, people with lifestyles such as smoking, poor diet, inactivity, etc. and those with complications including hypertension, diabetes, hyperlipidemia, obesity, etc. are often prone to stroke. At present, for the treatment of stroke, the most widely used thrombolytic drug is tissue plasminogen activator (t-PA). The application of t-PA treatment requires patients to meet the eligibility criteria, so that t-PA is for specific stroke patients, and the treatment time window is short, limited to 4.5 hours. In addition, endovascular therapy is also a major treatment strategy. However, there are also drawbacks, and intravascular stents are only suitable for solving the problem of blocked blood flow in large blood vessels. Although the use of preventive measures including medication and healthy lifestyle and aerobic exercise has led to a decrease in the incidence of stroke, the high recurrence rate remains unsolved. Existing clinical trials mainly study some electronic technology products or software systems to help stroke patients recover, behavioral and lifestyle improvements, drug therapy and cell therapy for stroke patients' recovery. In clinical trials, mesenchymal stem cells (MSCs) are basically in phases I and II. For the treatment of stroke, there are limitations in the treatment methods, the scope of application is narrow, and they are all indirect treatments, with a high recurrence rate in the later period.

Preparation and culture of M cells: The embryonic stem cells were suspended with EB spheres and subjected to adherent differentiation, the M cells at P0 generation were obtained, passaged and screened, and cryopreserved at P3 generation for subsequent experiments.

The M cells at P3 generation were resuscitated, digested and passaged, and was used at P5 generation for subsequent experiments.

Experimental animals: male SD rats (7 weeks old), the animals were purchased from Beijing Weitong Lihua Company. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%. The experiment was started after one week of adaptive feeding of rats.

Experimental materials: surgical instruments, suture emboli, 5-0 surgical sutures, rat experimental table, rat body weight scale, 1ml disposable sterile syringe, 5ml disposable sterile syringe.

Experimental reagents: isoflurane, iodophor, normal saline, phosphate buffer solution (PBS), 2,3,5-triphenyltetrazolium chloride (TTC)

Equipment: R540 Enhanced Small Animal Anesthesia Machine

| Consumable/Reagent/Instrument | Manufacturer | Cat. No. |
|---|---|---|
| Suture embolus | Kunming Huangbao Trading Co., Ltd. | 2634 A4 |
| 5-0 Surgical suture | Shanghai Yuyan Scientific Instrument Co., Ltd. | |
| Rat experimental table | Lige | LG41-206-4 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Isoflurane | Ruiwode | 970-00026-0 0 |
| Iodophor | Hangzhou Langso Medical Disinfectant Co., Ltd. | |
| Normal saline | domestic | |
| Phosphate buffered saline | CORNING | 21-040-CVR |
| 5-Triphenyltetrazolium chloride | Sigma | T8877-100G |
| R540 Enhanced Small Animal Anesthesia Machine | Ruiwode | R540 |

Preparation of middle cerebral artery occlusion (MCAO) model: an appropriate amount of isoflurane was poured into the gas anesthesia machine, the rats were placed in the gas anesthesia box, and the scale of the gas anesthesia machine was adjusted to 3.5; then the rats were deeply anesthetized and maintained at anesthesia state, the rat in a supine position was fixed on the rat experimental table, the neck skin was wiped with iodophor, the middle skin was cut longitudinally for 1 to 2 cm, the muscle layer was isolated, the common carotid, internal carotid and external carotid arteries were exposed and isolated, sutures were buried under the common carotid, internal and external carotid vessels, respectively, sutures were buried at the proximal end and distal end of the external carotid vessels, respectively, suture was buried at and ligated the external carotid collateral arterioles, the common carotid and internal carotid were clamped with vascular clamps, the distal end of the external carotid was ligated and cut to form a small opening, a suture was inserted into the common carotid, the proximal end of common carotid was ligated, the vascular clamps at the common carotid and internal carotid were loosen, and the suture embolus was inserted into the internal carotid for 18mm, after 90 minutes, the suture was pulled out, the external carotid was ligated, and the muscle layer and the skin layer were sutured.

Experimental groups: normal control group, MCAO+solvent (solvent group), MCAO+M cells (M cell group), 3 rats in each group.

Cell injection: Three hours after the operation and the mNSS scoring, the rats were grouped, and those with ≤7 points were eliminated; the rats with 8 to 12 points, 13 to 18 points were grouped, the MCAO+solvent group was injected with 500 µl of normal saline through tail vein, and the MCAO+M cell group was injected with $5\times10^6$ M cells/500µl/rat through tail vein.

Behavioral scoring: mNSS scoring was performed 3, 24 and 72 hours after surgery. The mNSS scoring comprised: observing the motion, sensory and reflex function in rats. The motor function included tail-lifting test, floor test and balance beam test. The tail-lifting test comprised: forelimb flexion (1 point), hindlimb flexion (1 point), head rising within 30s (1 point); the floor test comprised: unable to walk a straight line (1 point), hemiplegia (1 point), rotation (1 point); the balance beam test was further divided into: grasping edge of balance beam (1 point); grasping balance beam firmly, one limb falling from balance beam (2 points); grasping balance beam, two limbs falling from balance beam or rotating on balance beam (>60s) (3 points); attempting to balance on beam but falling (>40s) (4 points); attempting to balance on beam but falling (>20s) (5 points); falling directly without attempting to balance on beam (<20s) (6 points); the sensory touch test comprised: visual and tactile placement (1 point), proprioceptive placement (1 point); and the reflex function test comprised: auricular reflex (1 point), corneal reflex (1 point), startle reflex (1 point), myoclonus, dystonia, and seizure (1 point).

Detection of cerebral infarction and cerebral edema: 72 hours after the operation, the rats were euthanized, and the brain tissue was taken for wet weight and TTC staining, and then the brain tissue was dried for dry weight. TTC staining process comprised: 50ml of 2% TTC staining solution (1 g of TTC was weighed, added to 50ml of PBS to dissolve, and protected from light) was prepared, the rat brain tissues of all groups were allowed to stand at -20°C for 30 minutes, taken out one by one, sectioned to form 2mm sections, then the brain sections were placed in a 6-well plate, added with 3 ml of TTC staining solution to each well in the dark, the brain sections were shaken to prevent the brain sections from sticking to the 6-well plate, and then incubated in a 37°C constant temperature incubator; after 15 minutes, the brain sections were flipped and incubated again for 15 minutes; the brain sections were neatly arranged and photoed, and the statistics of infarct size was performed by Image J software; finally, the photoed brain sections were placed in a 65°C oven for 3 days, after which the dry weights of the brain sections were weighed and recorded.

Statistics: All data were analyzed by One-way ANOVA in Prism 7.0 statistical analysis software for analysis of variance and significance test, and experimental data were expressed as mean $\pm$ standard error (Mean $\pm$ SD). *, p<0.05; **, p<0.01; ***, p<0.001.

Analysis of results:

In the mNSS score results at 24 and 72 hours after the operation, the M cell group showed a reduced mNSS score, the solvent group vs the cell group: 12.67 vs 10.33 (24 hours), 6.33 vs 5.33 (72 hours), indicating the improvement of behavior in stroke rats (subjects); cerebral infarction reflected the necrosis of brain tissue, as compared with the solvent control group, the M cell group showed a reduced degree of brain infarction (62.77 vs 56.72), and an infarct size decreased by 6.05%; severe cerebral edema could cause an increased intracranial pressure, brain herniation was formed, and the water content of brain tissue in the solvent group was significantly higher than that in the normal control group (80.85 vs 82.94) and increased by 2%, while the M cell group showed a water content of brain tissue in rats decreased by 2% as compared with the solvent group (82.94 vs 80.49). All the above results showed that the M cells had a good therapeutic effect in stroke rats (subjects).

Table 21-1 and FIG. 150 showed the mNSS behavioral scores at 3, 24 and 72 hours. The intravenous injection of the M cells 3 hours after surgery could reduce the mNSS scores at 24 hours (12.67 vs 10.33) and 72 hours (6.33 vs 5.33) after surgery, indicating that the M cells could improve the behavior of stroke rats (subjects).

Table 21-1: Statistics of mNSS behavioral scores at 3, 24 and 72 hours

| Time (hours) | Normal control group | | | MCAO+solvent group | | | MCAO+M cell | | |
|---|---|---|---|---|---|---|---|---|---|
| 3 | 0 | 0 | 0 | 15 | 11 | 12 | 15 | 11 | 12 |
| 24 | 0 | 0 | 0 | 13 | 12 | 13 | 11 | 12 | 8 |

(continued)

| Time (hours) | Normal control group | | | MCAO+solvent group | | | MCAO+M cell | | |
|---|---|---|---|---|---|---|---|---|---|
| 72 | 0 | 0 | 0 | 7 | 6 | 6 | 6 | 6 | 4 |

Table 21-2 and FIG. 151 showed the statistics of cerebral infarct size in rats after 72 hours. The intravenous injection of the M cells 3 hours after surgery could reduce the infarct size of brain tissue by 6.05% as compared with the solvent group, indicating that the M cells could reduce the degree of brain tissue necrosis.

Table 21-2: Statistics of cerebral infarct size in rats after 72 hours.

| Group | Normal control group | MCAO+solvent group | MCAO+M cell |
|---|---|---|---|
| Infarct size (%) | 0.00 | 61.97 | 73.34 |
| | 0.00 | 71.30 | 46.04 |
| | 0.00 | 55.03 | 50.80 |

Table 21-3 and FIG. 152 showed the statistics of brain tissue water content in rats after 72 hours. The intravenous injection of the M cells 3 hours after surgery could reduce the water content of brain tissue by 2% as compared with the solvent group (82.94 vs 80.49), indicating that the M cells could reduce the degree of brain tissue edema.

Table 21-3: Statistics of brain tissue water content in rats after 72 hours.

| Group | Normal control group | MCAO+solvent group | MCAO+M cell |
|---|---|---|---|
| Brain tissue water content (%) | 80.90 | 84.70 | 78.54 |
| | 80.79 | 81.69 | 81.42 |
| | 80.85 | 82.44 | 81.49 |

Rat forelimb placement test:

The method was referred to the published article: Matsuda F., et al., Acta Physiol Neurosci, 2011.

After 48 hours, the forelimb use ability of the rats was detected by the forelimb placement test, and it was found that compared with the solvent control group, the use rate of the contralateral forelimb of the rats in the transplanted M cell group was significantly increased.

Rat rotarod test:

The method was referred to the published article: Shen H., et al., J Neurosci Methods, 2010.

After 72 hours, the motor coordination ability of the rats was detected by the rotarod test, and it was found that compared with the solvent control group, the rotarod exercise time of the rats in the M cell transplanted group was significantly increased. It showed that the M cells could enhance the exercise ability of the stroke animals (subjects).

Detection of brain damage by MRI

Methods: After the animals were anesthetized, the small animal nuclear magnetic resonance imaging was performed, and MRI T2 sequence was selected for plain scan.

After 72 hours, the rat brain injury was detected by MRI, and it was found that the intravenous injection of the M cells could reduce the cerebral infarction volume 3 hours after the surgery, indicating that the M cells could attenuate the degree of nerve cell damage caused by stroke.

Statistical results of staining rat brain frozen sections

The method was referred to the published article: Kriks et al., Nature, 2011.

After 72 hours, the frozen sections were stained to detect the regeneration of nerve cells in rats. It was found that compared with the solvent control group, the rats in the M cell transplanted group had significantly increased new neurons (Tuj1+) at the injury site, significantly decreased numbers of reactive astrocytes (GFAP+) and microglia (IBA1+ CD11B+) in the edge of ischemic injury area, and significantly increased number of neurons (NeuN+) per unit area in the injury area. It showed that the M cell transplantation could promote neuron regeneration, reduce neuron damage and death, and could provide nutrients to neurons and promote synapse regeneration.

ELISA and WB detection results of brain tissue inflammatory factors in rats

The method was referred to the published article, Bétemps et al., 2015, J Vis Exp.

After 72 hours, the rat brain tissue was taken to detect the levels of inflammatory factors. Compared with the solvent control group, it was found that the levels of TFN-$\alpha$, IL1-$\beta$, IL-6 and other proinflammatory factors in the brain tissue of the rats in the transplanted M cell group were significantly decreased, while the levels of anti-inflammatory factors such as IL-10 and IL-3 were significantly decreased. It showed that the M cells could attenuate the inflammatory response in the brain after stroke and improve the microenvironment in the brain.

Example 22: Evaluation of therapeutic activity of M cells against ocular surface injury

Ocular surface injury is one of the main causes of blindness in the world, among which the most common causes are ocular chemical burns (e.g., alkali and acid burns) and thermal burns, which seriously damage the ocular surface and are difficult to treat, the prognosis is poor, often leading to blindness and even loss of the eyeball. Corneal alkali burns are the most serious chemical burns. Alkaline substances can cause corneal tissue liquefaction and necrosis, resulting in serious damage to limbal stem cells. Severe depletion of limbal stem cells results in persistent inflammation, corneal and conjunctival metaplasia, ingrowth of new blood vessels, and scarring of corneal stroma. The subsequently induced immune inflammatory response is more likely to develop deep, and cause corneal ulcers and perforations, secondary glaucoma and concurrent cataracts, and severely damage the anatomical structure and visual function of the eye.

At present, there is no effective treatment for severe alkali burns, and corneal transplantation is still the main treatment method. However, corneal transplantation has many difficulties, such as difficulty in obtaining materials, low long-term graft survival rate, and post-transplant rejection, which limit its application and efficacy. In addition to the corneal transplantation, other surgical treatments, including (1) autologous limbal stem cell transplantation, (2) allogeneic limbal stem cell transplantation, and (3) amniotic membrane transplantation, also have some problems. The present invention overcame the problems such as difficulty in obtaining materials for corneal transplantation, transplant rejection and the like, and could be used in the treatment by transplantation of M cells and material scaffolds, which could promote the healing of corneal epithelium, reduce the degree of corneal turbidity, reduce the generation of corneal blood vessels, could more effectively repair corneal epithelial tissue, and could be used for the treatment of corneal alkali burns;

The rats with corneal alkali damage were treated by transplantation of M cells and collagen scaffolds, in which $1 \times 10^5$ M cells were inoculated on $5 \times 5$ mm collagen scaffold, the culture medium was changed every day, culture was carried out for 7 days, and the transplantation treatment was performed on the 7$^{th}$ day.

The present invention overcame the problems such as difficulty in obtaining materials, transplant rejection, and used in the treatment by transplantation of the M cells and material scaffolds, which could promote the healing of corneal epithelium, reduce the degree of corneal turbidity, reduce the generation of corneal blood vessels, can more effectively repair corneal epithelial tissue, and could be used for the treatment of corneal alkali burns;

Experimental animals: SD rats, male, 7 to 8 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended with EB spheres and subjected to adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equipment | Manufacturer | Cat. No. |
|---|---|---|
| R540 Enhanced Small Animal Anesthesia Machine | Ruiwode | R540 |

(continued)

| Reagent/Equipment | Manufacturer | Cat. No. |
|---|---|---|
| Stereomicroscope | Nikon | SMZ745 |
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Paraformaldehyde | LEAGENE | DF0135 |
| NaOH | Sigma | S5881-1KG |
| High-strength cross-linked collagen scaffold material | Xiamen Ningfu Biotechnology Co., Ltd. | None |

Culturing M cells on collagen scaffolds: $1 \times 10^5$ M cells (at p4 generation) were inoculated on $5 \times 5$ mm collagen scaffolds, and the culture medium was changed every day for 7 days. On the 7th day, the transplantation was performed.

Animal modeling: SD rats were anesthetized with 5% chloral hydrate, and modeling was performed after anesthesia. A filter paper sheet with a diameter of 7 mm was soaked in 1 mol/L NaOH for 30 s, the excess liquid was absorbed by a dry filter paper, then it was placed in the center of the right cornea of the rat for 30 s, and rinsing with normal saline was performed for 1 min. After rinsing, the rats were grouped and their eyelids were sutured. On day 3, the sutures were removed. The optical photos of the eyes were taken on days 3, 5, 7, 10, 14, and 21, and sampling was performed on day 21.

Grouping:

Control group:

NaOH group, only eyelid was sutured.

Collagen group, $5 \times 5$mm collagen scaffold was placed, and eyelid was sutured.

Treatment group: M cell group, collagen scaffold carrying M cells was placed in the center of rat cornea, and eyelid was sutured. After 3 days, the sutures were removed, and photos were taken at different time points for scoring.

Photoing rat eyeballs: After SD rats were anesthetized, photos were taken under the stereomicroscope, and the magnification was adjusted to 1.25 times. When taking photos, proportional scale should be added, and long-term stimulation of strong light to rat eyes should be avoided.

Rat corneal opacity score: 0 points, completely transparent cornea; 1 point, less corneal opacity, but clearly visible iris; 2 points, mild corneal opacity, iris blood vessels still visible; 3 points, moderate corneal opacity, blood vessels in margins of pupil, but no blood vessels in iris; 4 points, completely opaque cornea, and pupil invisible.

Sample collection:

When collecting the specimens, after the rats were anesthetized, the rats were placed in a supine position, the skin was cut in the middle of the abdomen of the rat, the chest was opened, the heart was exposed, and the heart was perfused with ice-cold normal saline. Each rat needed about 50 ml of normal saline. After the normal saline perfusion was completed, the fixation was performed with 50 ml of paraformaldehyde. After the perfusion was completed, the eyeballs of the rats were removed, fixed with paraformaldehyde, and stored at 4°C for subsequent sectioning.

Quantification of number of new blood vessels:

See Joo Youn Oh et, al, anti-inflammatory protein TSG-6 reduces inflammatory damage to the cornea following chemical and mechanical injury. 2010, PNAS, 107(39). 16875-16880

H&E staining and immunohistochemistry (IHC)

See Joo Youn Oh et, al, anti-inflammatory protein TSG-6 reduces inflammatory damage to the cornea following chemical and mechanical injury. 2010, PNAS, 107(39). 16875-16880

Detection of concentrations of related factors by ELISA method

See Joo Youn Oh et, al, anti-inflammatory protein TSG-6 reduces inflammatory damage to the cornea following chemical and mechanical injury. 2010, PNAS, 107(39). 16875-16880

Conclusion:

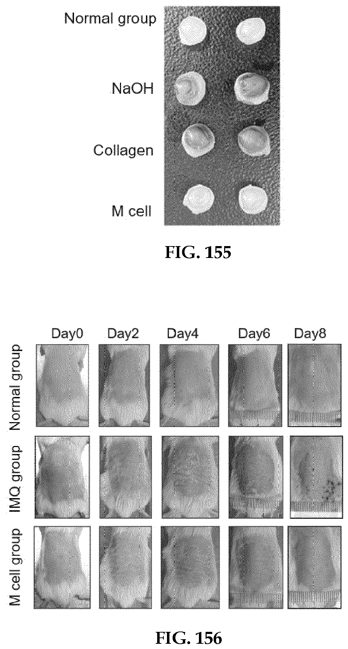

Rat eye optical photos were shown in FIG. 153.
Rat corneal opacity scores were shown in FIG. 154.
Rat eyeball sample photos were shown in FIG. 155.
Table 22-1 showed the statistics of corneal opacity scores in rats of different groups on days 3, 5, 7, 10, 14 and 21.

Table 22-1: Rat corneal opacity scores

| | Corneal score | | | | | | | | | | | | | | |
|---|---|---|---|---|---|---|---|---|---|---|---|---|---|---|---|
| Time (days) | M cell group | | | | | Collagen | | | | | NaOH | | | | |
| 3 | 2 | 3 | 1 | 3 | 2 | 2 | 3 | 3 | 3 | 3 | 3 | 3 | 3 | 3 | 2 |
| 5 | 1 | 2 | 1 | 2 | 2 | 3 | 2 | 3 | 2 | 3 | 2 | 3 | 2 | 3 | 2 |
| 7 | 1 | 2 | 1 | 1 | 2 | 3 | 3 | 3 | 3 | 3 | 3 | 3 | 2 | 3 | 2 |
| 10 | 1 | 2 | 1 | 2 | 1 | 2 | 3 | 3 | 4 | 3 | 3 | 3 | 3 | 3 | 2 |
| 14 | 1 | 1 | 1 | 2 | 1 | 4 | 4 | 4 | 4 | 4 | 4 | 4 | 4 | 4 | 4 |
| 21 | 0 | 1 | 0 | 1 | 0 | 4 | 4 | 4 | 4 | 4 | 3 | 4 | 3 | 4 | 4 |

On the 21st day, the cornea was clear, the pupil was visible, and lower score was obtained, and there was a statistical difference, indicating that the effect of M cell treatment was obvious. One-way ANOVA in Prism 7.0 statistical analysis software was used for analysis of variance and significance test, and the experimental data were expressed as mean $\pm$ standard error (Mean $\pm$ SE). *, $p<0.05$; **, $p<0.01$; ***, $p<0.001$. It was found that the eyeballs of the M cell treatment group were more similar to those of the normal group, and there was no accumulation of fluid and congestion. It showed that the M cells could reduce the inflammation of corneal alkali burn animal model and promote the recovery of corneal alkali injury.

It was found from the H&E staining and immunohistochemistry (IHC) detection that neutrophil elastase showed severe neutrophil infiltration on day 3 after the corneal injury in the control group. In addition, fibrovascular corneal stroma thickening was observed on day 21. In contrast, neutrophil infiltration was significantly reduced in the M cell treatment group on day 3 after the injury, and the epithelium and stroma returned to normal on day 21.

In order to quantitatively measure neutrophil infiltration, corneal myeloperoxidase (MPO) concentrations were measured by ELISA, and it was found that MPO concentrations were significantly reduced after the treatment with M cells.

The number of corneal new blood vessels was quantified by calculating the number of blood vessels grew in wedge-shaped area. The results showed that the number of new blood vessels was significantly reduced in the M cell injection group.

The level of MMP-9 in the whole cornea was detected by ELISA, and it was found that the level of MMP-9 was significantly reduced in the M cell treatment group.

It was found by ELISA that the levels of proinflammatory cytokines (IL-6 and IL-1 inhibitory factors) and chemokines (CXCL1/cinc1 and CCL2/MCP-1) in the control group were significantly increased on the 3rd day after the corneal injury. In contrast, the corresponding factors in the M cell treatment group were significantly lower than those in the control group.

Example 23: Evaluation of therapeutic activity of M cells against psoriasis

Psoriasis (commonly known as serpedo) is a well-known skin disease. Once it occurs, red papules or plaques may appear on the skin, and are covered with multiple layers of silvery white scales. It tends to occur on the limbs, head and back, and even the whole body. In some cases, it lasts almost a lifetime. There is currently no effective treatment. The disease mainly affects young and middle-aged people, which has a great impact on the physical health and mental status of patients, and has caused a huge burden on the society and economy. Epidemiological surveys show that there are currently about 6.5 million psoriasis patients in China, with an incidence rate of 0.47%.

At present, psoriasis is considered to be an autoimmune skin disease caused by the domination of dendritic cells (DC) and T lymphocytes, the participation of innate and adaptive immunity, and the interaction of genetic background and environmental factors. The characteristic lesions of psoriasis include excessive proliferation of keratinocytes caused by inflammatory conditions, and so on. Antagonistic biological agents targeting key cytokines (TFN-$\alpha$, IL-12, IL-23, IL-17) in the pathogenesis of psoriasis are extremely effective in clinical treatment, but the high costs for maintaining long-term treatment and the potential serious adverse reactions limit the wide application of such biological agents.

Objective: To achieve the treatment of psoriasis by subcutaneous point injection of M cells.

Achieved results: (1) relieved rash and erythema; (2) relieved scale; (3) relieved infiltration degree; (4) relieved psoriatic dermatitis phenotype; (5) relieved psoriatic skin lesions; (6) reduced epidermal spinous layer; (7) reduced thickness of stratum corneum;

Experimental animals: BALB/c mice, female, male, 7 to 8 weeks old, purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22$\pm$2°C, relative humidity was 50% to 60%.

4-2. Preparation and culture of M cells: The embryonic stem cells were suspended with EB spheres and subjected to adherent differentiation, and the M cells at the P0 generation were obtained, passaged and screened, and cryopreserved at the P3 generation for subsequent experiments.

The M cells at P3 generation were resuscitated, digested and passaged, and used at P5 generation for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No.4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solarbio | G8590-100 |
| Imiquimod cream (IMQ) | Aldara | None |

4-3. Experimental methods: comprising sample processing, experimental steps, specific conditions/parameters, sequence, etc.;

4-3-1. Animal model: BALB/c mice were weighed and randomly divided into groups according to their body weight. After the BALB/c mice were anesthetized with a gas anesthesia machine, the back hair was shaved, and the mice were grouped, 6 mice in each group.

4-3-2. Grouping:

Normal group: only shaved, not subjected to other treatment.

Imiquimod (IMQ) group: injected at 3 points on the back, 50 $\mu$l of normal saline per point.

M cell group: injected at 3 points on the back, 50 $\mu$l of normal saline containing $1\times10^6$ M cells (P5 generation) per point.

The day of the above treatment was recorded as day -1. From day 0 to day 6, 62.5 mg of IMQ was applied topically every day, and photos were taken. On day 6, the second treatment was performed by the method same as day -1. On the day 8, photoing, perfusion and sampling were performed.

4-3-3. Sample collection:

When collecting specimens, after the mice were intraperitoneally anesthetized, the mice were in supine position, the skin was cut in the middle of the abdomen of the mice, the chest was opened, the heart was exposed, and the heart was perfused with ice-cold normal saline. About 20 ml of normal saline was needed for each mouse. After the normal saline perfusion was completed, the fixation was performed with 20 ml of paraformaldehyde. After the perfusion was completed, the skin in the modeling area was cut off, and the spleen and lymph nodes were taken, fixed, sectioned and analyzed.

4-3-4. Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

4-3-5. Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) HE Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

Clinical examination:

Methods: The spleen size and the number of lymph node masses in each group were compared.

Experimental results: The M cells could effectively reduce spleen enlargement and lymph node (axillary, lateral axillary, inguinal) masses were significantly reduced. Detection of cell compositions in skin, spleen, and lymph nodes.

1) Detection by flow cytometry

(1) The mouse tissue was taken out, ground with a grinder, the grinding fluid was transferred into an EP tube, centrifuged with centrifuge at 500G for 5min, the supernatant was discarded, then 5ml of red blood cell lysate was added, incubated at 37°C for 15min, centrifuged again, the supernatant was discarded, the cell concentration was adjusted to $1 \times 10^6$, the cells were transferred into a centrifuge tube, centrifuged at 400G for 5min, the supernatant was discarded, CD4 antibody was added to each tube, vortexed, and incubated in the dark for 30 min.

(2) A part of the cell suspension obtained from each tissue was loaded on H2DCFH-DA (5 $\mu$M) to detect the total ROS content.

(3) Another part was washed twice with 1ml of staining buffer, the first tube was added with Gr1 and CD11 isophil antibodies, and the other tubes were added with 2ul of Grl and CD11 antibodies; after vortexing, incubation was carried out at 4°C for 30min.

(4) The cells were resuspended by adding 500ul of PBS, loaded to perform detection and analysis, the CD4$^+$ T cell gate was determined based on CD4 fluorescence, 10,000 CD4$^+$ T cells were counted in each sample, and the total and absolute numbers of T cells, and the content of neutrophils and dendritic cells were calculated.

2) Immunohistochemical (IHC) staining

Immunohistochemical staining was performed on the paraffin sections using an immunohistochemical kit (Fuzhou Maixin, KIT-9710). The specific steps were as follows:

1. Dewaxing: (1) xylene I, II, 10 min each; (2) gradient alcohol: 100% absolute ethanol, 2 min; 95% absolute ethanol, 2 min; 80% absolute ethanol, 2 min; 70% absolute ethanol, 2 min;

2. Hydration: washing was performed twice with distilled water, 5min each time (placed on a shaker);

3. After deparaffinization and hydration of paraffin sections, rinsing was performed 3 times with PBS, 3 minutes each time;

4. Preparation of antigen retrieval solution (10 mM pH 6.0 sodium citrate buffer):

(1) Preparation of stock solution: Solution A: 29.41 g of trisodium citrate dihydrate + 1,000 mL of distilled water; Solution B: 21 g of citric acid + 1,000 mL of distilled water;
(2) Preparation of working solution: 82 mL of Solution A + 18 mL of solution B + 900 mL of distilled water;

5. Antigen retrieval: the sections were placed in a plastic or heat-resistant glass container filled with sodium citrate buffer, the sections were immersed, treated with a microwave oven at mid-range or high-range power for 5 minutes; sodium citrate buffer was replenished, and treatment was performed again at mid-range or high-range power for 5 minutes;

6. Reagent A (peroxidase blocking solution) was added, and incubated at room temperature for 10 min to block the activity of endogenous peroxidase; rinsing was performed with PBS 3 times, 3 min each time;

7. PBS was discarded, 1 drop or 50 $\mu$L of Reagent B (normal non-immune animal serum) was added, and incubated at room temperature for 10 min;

8. The serum was discarded, 1 drop or 50 $\mu$L of primary antibody was added, and incubated at 4°C overnight or at room temperature for 60 min; rinsing was performed with PBS 3 times, 3 min each time;

9. The PBS was discarded, 1 drop or 50 $\mu$L of biotin-labeled secondary antibody (Reagent C) was added, and incubated at room temperature for 10 min; rinsing was performed with PBS 3 times, 3 min each time;

10. The PBS was discarded, 1 drop or 50 $\mu$L of streptavidin-peroxidase solution (reagent D) was added, incubated at room temperature for 10 min; rinsing was performed with PBS 3 times, 3 min each time;

11. The PBS was discarded, 2 drops or 100 $\mu$L of freshly prepared DAB solution was added, and observation was performed under microscope for 3 to 10 min;

12. Rinsing was performed with tap water, counterstaining was carried out with hematoxylin, and rinsing was performed with PBS or tap water so as to return to blue;

13. When using DAB for color development, the sections should be dehydrated with gradient alcohol and dried, transparentizing was performed with xylene, and mounting was performed with neutral resin;

14. Photos were taken with a microscope.

Experimental results: In the M cell treatment group, the level of ROS was reduced, the recruitment of neutrophils and dendritic cells in the spleen were effectively reduced, and the inflammatory infiltrating cells was decreased, indicating that the M cells had a strong immune regulation effect.

3) Detection of expression of specific cytokines and transcription factors

1. RNA extraction and RT-PCR identification

RNA extraction was performed using Invitrogen's TRIZOL in a fume hood.

The sample tissue was ground with an electric grinding rod, then transferred into a 1.5 ml RNA-free tube, added with 1 ml of TRIZOL to lyse the cells, and the lysate was collected and added into a 1.5 ml RNA-free EP tube. Incubation was carried out at 4°C for 15 min, 500 $\mu$l of chloroform was added to each tube, mixed by vortexing and shaking, and allowed to stand on ice for 10 min; centrifugation was carried out at 4°C, 12,000 rpm for 15 min; the upper layer of the separated liquid layers was collected with a 1 ml pipette, transferred to a new 1.5 ml RNA-free EP tube, added with isopropanol in an equal volume of the transferred upper layer, mixed by vortexing and shaking, and allowed to stand on ice for 10 min; centrifugation was carried out at 4°C, 12,000 rpm/10 min; the supernatant was discarded, the pellet was washed twice with 75% ethanol, centrifuged at 4°C, 12,000 rpm/10 min; the supernatant was discarded, the RNA was dried in a fume hood for 5 to 10 min, in which the drying time should not be too long, otherwise the solubility of RNA would be reduced, and the quality of RNA would be decreased. RNA-free water was added, and heated on a metal bath at 55°C for 10 min. The RNA concentration and OD value were measured by Nanodrop.

2. Reverse transcription of mRNA

(1) 2 $\mu$g of RNA extracted by reverse transcription, 1 $\mu$l of Oligo(dT) Primer, 1 $\mu$l of dNTP Mixture were added with RNA-free water to reach 10 $\mu$l. Denaturation was performed at 65°C for 5 min, and incubation was carried out at 4°C for 3 min.
(2) The following reagents were further added to the above 10 $\mu$l system for reaction, and the total system was 20 $\mu$l.
(3) After mixing gently, reaction was carried out at 42°C for 60 min, and then reaction was carried out at 70°C for 15 min.

| 10 $\mu$l reaction system | |
| --- | --- |
| Reagent | Volume ($\mu$l) |
| 5X PrimeScript Buffer | 4 |
| RNase Inhibitor | 0.5 |
| PrimeScript RT | 0.7 |
| RNase free H2O | 4.8 |

3. Real-time PCR

The reverse transcribed cDNA was diluted 5 times, and RT-PCR was performed.

| 10 $\mu$l reaction system | |
| --- | --- |
| Reagent | Volume ($\mu$l) |
| cDNA | 1 |
| SYBP | 5 |
| $H_2O$ | 3.4 |
| Primer | 0.6 |

Experimental results: The injection of M cells could inhibit the IL-23-induced expression of skin proinflammatory genes, and inhibited the expression of proinflammatory factors IL-6, IL-17 and TFN-α.

The photos of the backs of mice on different days were shown in FIG. 156.

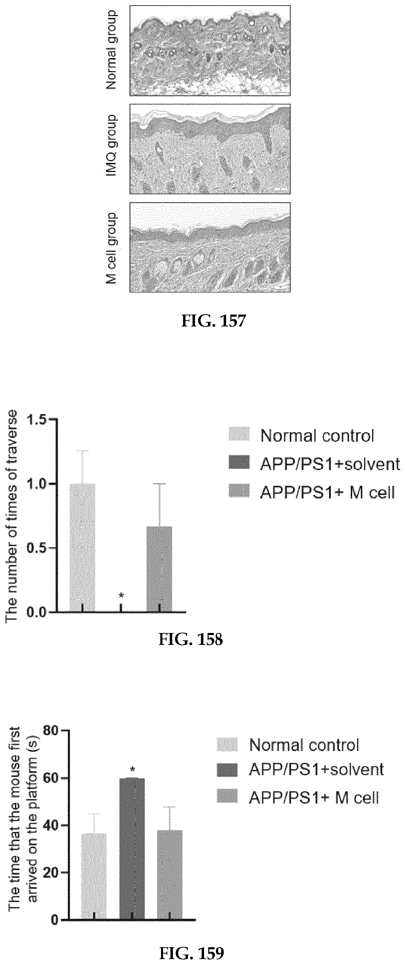

The photos of HE staining were shown in FIG. 157.

Example 24: Evaluation of therapeutic activity of M cells against Alzheimer's disease

Alzheimer's disease (AD) is one of the most common chronic diseases in old age. It affects more than 35 million people worldwide. The clinical manifestations of AD are progressive memory loss and cognitive impairment. Alzheimer's disease is associated with two pathogenic features, i.e., extracellular amyloid beta (Aβ) deposition and intracellular neurofibrillary tangles (NTFs), with neuroinflammation and extensive neuronal and synaptic loss, leading to progressive memory loss and cognitive impairment. At present, there is no specific drug that can cure Alzheimer's disease or effectively reverse the disease process. The combination of drug therapy, non-drug therapy and careful nursing can reduce and delay the onset of the disease. Therefore, it is important to develop effective therapeutic strategies that can cure AD or delay AD. At present, clinical trials have been carried out mainly on drug researches, and there are more than 200 clinical trials. There are 10 clinical trials of mesenchymal stem cells (MSCs), which are in clinical phases I and II.

Preparation and culture of M cells: The embryonic stem cells were suspended with EB spheres and subjected to adherent differentiation, the M cells at P0 generation were obtained, passaged and screened, and cryopreserved at P3 generation for subsequent experiments.

The M cells at P3 generation were resuscitated, digested and passaged, and P5 generation was used for subsequent experiments.

Experimental animals: APP/PS1 mice and male C57b1/6 mice, 7 months old, purchased from Shanghai Southern Model Organism Research Center and Beijing Sibeifu Biotechnology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%. Experiments were started after one week of adaptive feeding of mice.

Experimental groups: normal control group, APP/PS1+solvent, APP/PS1+M cells, 6 cells in each group.

Experimental materials: surgical instruments, 5-0 surgical suture, mouse weight scale

Experimental reagents: normal saline, iodophor, isoflurane

Equipment: R540 enhanced small animal anesthesia machine, brain stereotaxic instrument, microinjection pump, water maze

| Consumable/Reagent/Instrument | Manufacturer | Cat. No./Model |
|---|---|---|
| 5-0 Surgical suture | Shanghai Yuyan Scientific Instrument Co., Ltd. | |
| Isoflurane | Ruiwode | 970-00026-00 |
| Iodophor | Hangzhou Langso Medical Disinfectant Co., Ltd. | |
| Normal saline | domestic | |
| R540 Enhanced small animal anesthesia machine | Ruiwode | R540 |
| Brain stereotaxic instrument | Ruiwode | 69100 |
| Microinjection pump | Ruiwode | 788130 |
| Water maze | Noldus | |

Experimental method, comprising: sample treatment, experimental steps, specific conditions/parameters, sequence, etc.;

Experimental steps: the scale line of the anesthesia machine was adjusted to 2.5, the mice were placed in the box; after deep anesthesia, the mice were fixed in the prone position with the brain stereotaxic instrument, the mouse brain skin was wiped with iodophor, cut to form a 1cm opening, and the positioning was performed according to the below position: AP: -2.06, ML ±1.75, DV: -1.75, relative to bregma, the injection was performed with the microinjection pump, for the APP/PS1+solvent group, bilateral hippocampus each was injected with 1 μl of normal saline; for the APP/PS1+M cells group, bilateral hippocampus each was injected with 1 μl of M cells: $5 \times 10^5/1\mu L$; the injection was performed for 10 minutes; after the injection, the needle was kept for 5 minutes, then the needle was pulled out, and the skin was sutured.

Water maze behavior: Water maze training, including acquisition training and exploratory training, was performed 24 to 27 days after surgery. The acquired training comprised: (1) the mouse was put in water with its head facing the pool wall, and the placement position is randomly selected from one of the four starting positions of east, west, south and north. The time (in seconds) that the animal found the underwater platform was recorded. In the first few training sessions, if this time exceeded 60 seconds, the animal was guided to the platform. The animal was allowed to stay on the platform for 10 seconds. (2) The animal was taken out, dried, and put back into the cage. Each animal was trained 4 times a day, with an interval of 15 to 20 min between the two training sessions, for 5 consecutive days. For the exploratory training, on the day after the last acquired training, the platform was removed and a 60-second exploratory training started. The animal was placed in the water from the opposite side of the original platform quadrant. The time spent in the target quadrant (the quadrant where the platform was originally placed) and the number of times the animal entered the quadrant were recorded as indicators of spatial memory. After training, the water maze test was performed on day 28.

Statistics: All data were analyzed by One-way ANOVA in Prism 7.0 statistical analysis software for variance analysis and significance test, and experimental data were expressed as mean ± standard error (Mean ± SD). *, $p < 0.05$; **, $p < 0.01$; ***, $p < 0.001$.

Results: The water maze could objectively measure the changes of spatial memory, working memory and spatial discrimination ability of animals (subjects). The statistics of mice crossing platform and the statistics of time spent to reach platform for the first time showed that the M cells could effectively improve the spatial learning and memory ability of the mice (subjects).

Table 24-1 and FIG. 158 showed the statistics of the total number of times mice crossing the platform. The total crossing times of the normal control group was 1 time. Compared with the normal control group, the total crossing times of the mice in the solvent group was 0 times, with a significant difference, * $p < 0.05$; the total crossing times of the mice in the M cell group was 0.66 times, with a significant difference, which was more than that of the solvent group, indicating that the M cells could effectively improve the spatial learning and memory ability of the mice.

Table 24-1: Statistics of the total number of times mice crossing platform.

| Total crossing times | Group 1, normal control | | | | | | Group 2, APP/PS1+solvent | | | | | | Group 3, APP/PS1+Mcells | | | | | |
|---|---|---|---|---|---|---|---|---|---|---|---|---|---|---|---|---|---|---|
| | 1 | 1 | 1 | 2 | 0 | 1 | 0 | 0 | 0 | 0 | 0 | 0 | 0 | 0 | 0 | 1 | 2 | 1 |

Table 24-2 and FIG. 159 showed the statistics of time spent by mice to arrive at platform for the first time. Compared with the solvent group, the time spent in the M cell group was significantly reduced (38.0 vs 59.8 seconds), indicating that the M cells could effectively improve the spatial learning and memory ability of the mice.

Table 24-2: Statistics of time spent for mice to reach platform for the first time

| Time of first arrival (seconds) | Group 1, normal control | | | | | | Group 2, APP/PS1+solvent | | | | | | Group 3, APP/PS1+Mcells | | | | | |
|---|---|---|---|---|---|---|---|---|---|---|---|---|---|---|---|---|---|---|
| | 55 | 4 | 33 | 30 | 60 | 38 | 60 | 60 | 60 | 59 | 60 | 60 | 60 | 60 | 60 | 12 | 17 | 19 |

Pathological detection of amyloid deposits (Aβ)

The method was referred to the published article, Paolicelli et al., 2017.

According to the statistics of fluorescent staining results of the sections of the mice, the model mice injected with the M cells had significantly reduced number and proportion of plaques per unit area than those of the control group. It indicated that the M cells could reduce the accumulation of amyloid deposits, thereby reducing their adverse effects on nerves.

Inflammation detection in brain

The method was referred to the published literature Paolicelli et al., 2017

ELLSA and WB were used to detect inflammatory factors such as IL-6, TFN-α, iNOS, etc., and it was found that the indexes of inflammatory factors in the model mice injected with the M cells were significantly reduced as compared with the control group. It indicated that the M cells could reduce the transformation of microglia to proinflammatory form, and prevent the excessive activation and dysfunction of microglia.

Immunofluorescence staining detection:

The method was referred to the published literature Pan et al., 2019

The immunofluorescence staining was used to detect the markers of microglia, and it was found that the phagocytic ability of microglia in the model mice injected with M cells was significantly higher than that in the control group. It showed that the M cells could improve the phagocytic ability of microglia and remove amyloid deposits and apoptotic cell debris.

It was found that the number of A1 astrocytes per unit area in the model mice injected with M cells was smaller than that in the control group. It indicated that the M cells could inhibit the generation of A1 astrocytes in the inflammatory environment of AD brain and inhibit the transient activation of immunity.

At the same time, it was also found that the number of neurons (TUJ1+) increased, indicating that the injection of M cells could improve the survival of nerves and improve cognition and memory.

Barnes maze test:

The method was referred to the published literature Zhang et al., 2019.

It was found from the test that the mouse model injected with the M cells performed was better than the control group in terms of the training to find flat hole, or the time to reach the hole, and the crossing times. This indicated that the injection of M cells could improve the memory and cognitive deficits in the AD mice (subjects).

Example 25: Evaluation of therapeutic activity of M cells against arthritis

Osteoarthritis (OA) has become an increasingly common joint disease. It is known that OA can be treated using some anti-inflammatory drugs, analgesics, or lubricating supplements, and alternatively, a surgery involving drilling, microfractures, and autologous osteochondral mosaic grafting (mosaicplasty) can be performed to repair or reconstruct the defect site so as to treat OA, but this approach only temporarily improves symptoms and does not permanently cure or regenerate degenerated tissue.

This example evaluates the therapeutic activity of M cells for osteoarthritis.

Experimental animals: SD rats, male, 7 to 8 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended to form EB spheres and subjected to adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equi pment | Manufacturer | Cat. No. |
|---|---|---|
| R540 Enhanced small animal anesthesia machine | Ruiwode | R540 |
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No.4 Pharmaceutical Co., Ltd. | None |
| Paraformaldehyde | LEAGENE | DF0135 |
| Sodium iodoacetate | Sigma | S104897-5g |

Animal modeling: SD rats were anesthetized with a gas anesthesia machine, shaved off the hair of the left joint, wiped with gauze sprayed with alcohol, and then injected with 50 $\mu$l of MIA solution into the joint cavity with 1 ml syringe for modeling. The mice were grouped, 6 mice in each group, and analyzed on the day 21.

Grouping: normal group, model group, M cell group.

Normal group: not treated.

Model group: 50 $\mu$l of sodium iodoacetate (MIA) was injected into the joint cavity on the day 0, and 100 $\mu$l of normal saline was injected into the joint cavity on day 7 and day 14.

M cell group: 50 $\mu$l of sodium iodoacetate (MIA) was injected into the joint cavity on day 0, and 100 $\mu$l of normal saline containing $3 \times 10^6$ M cells was injected into the joint cavity on day 7 and day 14.

Preparation of MIA: sodium iodoacetate (MIA) was dissolved in normal saline, 1 mg of MIA was dissolved in 1 ml of normal saline, and the concentration was adjusted to 100 $\mu$g/$\mu$l MIA solvent.

Forced walking assessment (rotarod test)

Animals were randomly placed on a rotating cylinder (Roto-rod) with increasing speed, forced to walk continuously to avoid drop, and the performance indexes mainly included motor learning and use of the affected limb. Firstly, the test rats were placed on the roto-rod for 5 minutes to acclimate to the device. Five minutes after the acclimation period, the rats were placed on the roto-rod again and the rotational speed was increased from 5 rpm to 35 rpm over a 5-minute range. The waiting time for a drop is automatically measured by a mechanical sensor on the bottom of the device. The results of animal motor activity were assessed on the day 1 in all animals, and on the days 7, 14, 21 and 28 after induction.

Experimental results

The experimental results showed that the score of the M cell group was higher than that of the model group, indicating that the motor activity of rats with osteoarthritis could be relatively improved after the M cell treatment.

Tactile allodynia assessment (Von Frey test)

The test mice were placed in a single acrylic transparent box with 5 mm$^2$ grid on the bottom, in the acclimatization environment, non-abrasive metal wire with 1 mm thickness was placed for 15 min before the experiment. A mirror was placed 25 cm below the experimental box to facilitate viewing in the plantar area of hindlimb. Through the holes of the grid, the tester applied a linearly increasing pressure to the central region of the hindlimb until the hindlimb was stimulated and withdrawal response occurred. The stimulation was repeated up to six times to the ipsilateral and contralateral hindlimbs until the animals exhibited three similar hindlimb withdrawal responses.

Experimental results

Compared with the model group, the test mice in the M cell group had a significantly delayed withdrawal response time, indicating that the stimulatory pressure the mice could withstand was significantly higher than that in the model group, and the M cells had effect on inhibiting nerves to produce pain.

X-ray exposure assessment

Osteoarthritis rats underwent CT imaging on the day of administration of M cells, before the second administration of cells, and at the time of sampling. CT imaging: The test mice were anesthetized and placed on the CT table for fixation, so that the X-rays could be irradiated on the injured leg during the whole process for imaging. The images were analyzed after imaging.

Experimental results

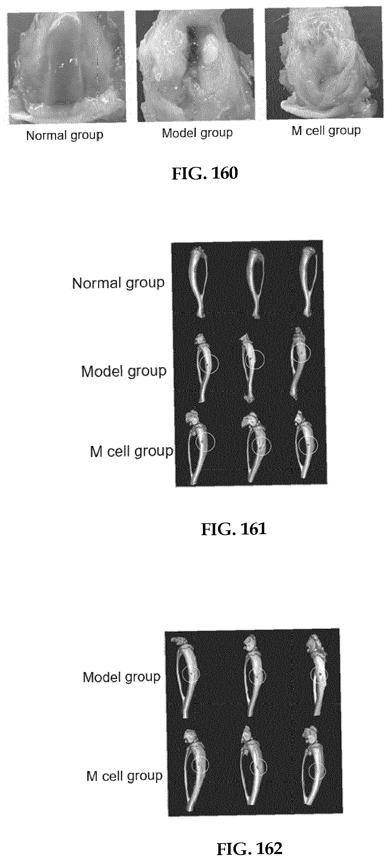

The photos of rat arthroscopic were shown in FIG. 160. In the model group, obvious joint space narrowing, irregular joint surfaces, erosion of articular cartilage, and osteophytes were observed. In the M cell group, the articular surface irregularity was lower, the articular cartilage was slightly eroded, and there was a slight osteophytes. This indicated that the M cells had effect on osteoarthritis partially in terms of tissue damage.

Specimen collection

When collecting specimens, the rats were placed in a supine position after intraperitoneal anesthesia, the skin was cut in the middle of the abdomen of the rat, the chest was opened, the heart was exposed, and the heart was perfused with ice-cold normal saline. Each rat needed about 50 ml of normal saline. After the perfusion of normal saline was completed, the joints at the modeling site were cut off, fixed, sectioned and analyzed.

Safranin-O/Fast green staining and OARSI scoring

Working solution: 0.1% safranine staining solution: 0.1 g+100 ml ddwater

0.15% fast green staining solution: 0.15 g+100 ml ddwater

1% glacial acetic acid: 2 ml glacial acetic acid + 198 ml ddwater

Steps: To prepare paraffin sections, the sections were baked at 60°C overnight, then immersed in xylene, alcohol, and ddwater in sequence, and then stained with Safranin O solution for 4 min, pulled 3 times in tap water, dipped and stained in fast green staining solution for 4 min, washed in tap water for 1 min, then the sections were washed with glacial acetic acid solution for 1 to 2 min, washed in tap water for 1 min, dehydrated with 95% ethanol and anhydrous ethanol, respectively; after 10 to 15 s, mounting with resin was performed.
The cartilage degeneration was assessed using the method recommended by the Osteoarthritis Research Society International (OARSI) (total score: 0 to 24). In simple terms, the depth and extent of cartilage damage on the tibial medial plateau were divided into 6 and 4 grades, respectively, and the grades were multiplied to obtain the score. Each sample was scored independently by 3 observers and the mean value was taken.

Experimental results

In the model group, small-scale defects with depths to the middle cartilage were observed, which reached the calcified cartilage in the later stage, while in the M cell treatment group, the cartilage defects had smaller depths and reached the deep cartilage in the later stage, and had smaller area than that of the model group, and the OARSI score was lower than that of the model group.

Type II collagen immunohistochemistry and semi-quantitative analysis

After deparaffinization, rehydration, and antigen retrieval, the sections were applied with rat type II collagen monoclonal antibody (Santa Cruz) at 4°C for 14 to 18 h. On the next day, the sections were rewarmed, and the DAB immunohistochemistry kit (R & D system) was operated according to the instructions. After the end, the sections were dehydrated and mounted. Image Pro Plus 6.0 software was used to calculate the cumulative positive integral IOD of the cartilage layer of the tibial medial plateau, and the detection area, Area, in which IOD/Area represented the positive degree of type II collagen.

Experimental results

Compared with the M cell treatment group, the loss of type II collagen in the cartilage layer of the tibial plateau was more obvious in the model group.

Example 26: Evaluation of therapeutic effect of M cells against fracture

Fracture refers to the complete breakage of a continuous portion of the bone structure. It is a common clinical bone injury, more common in children and the elderly, and also occurs in young and middle-aged people. It is often a single fracture, and a few are multiple fractures. By timely and appropriate treatment, original functions can be restored in most cases, and a few patients may leave sequelae of different degrees. The main reason for fractures may be that violence directly or indirectly (through longitudinal conduction, leverage or torsion, etc., causing simultaneous fractures of bones far away from the point of violence) acts on a certain part of the bone, which is often accompanied by soft tissue damage of different degrees; long-term, repeated, minor direct or indirect injuries may cause fractures in a specific part of the limb, also known as fatigue fractures, which are also common in a variety of occupational diseases; in addition, some bone-related genetic diseases or connective tissue diseases may also be accompanied by clinical symptoms of multiple fractures. The typical clinical manifestations of fracture patients are local swelling, deformation, pain, congestion, etc., as well as abnormal movements or movement disorders of limbs.

In the traditional treatment of fractures, reduction, fixation and functional exercise are the three basic principles. However, for severe bone injury, traditional fracture treatment cannot cure well, or cause deformity after treatment. Therefore, for bone injury caused by injury or disease, autologous and allogeneic bone grafts are often relied on to perform bone repair. Autologous bone transplantation has a good repair effect, but there are limitations such as the limited amount of autologous bone grafts, the need for secondary surgery and postoperative complications as high as 8%; while allogeneic bone transplantation is difficult to match, and there is serious immune rejection phenomenon, and thus it is not the most ideal choice for bone repair.

References:

Mesenchymal stem cell sheet transplantation combined with locally released simvastatin enhances bone formation in a rat tibia osteotomy model;

Mesenchymal stem cell-conditioned culture medium facilitates angiogenesis and fracture healing in diabetic rats;

SYSTEMIC MESENCHYMAL stem cell ADMINISTRATION ENHANCES BONE FORMATION IN FRACTURE REPAIR BUT NOT LOAD-INDUCED BONE FORMATION;

Adipose derived pericytes rescue fractures from a failure of healing - non-union.

Achieved effect: Through M cell transplantation, the healing of bone injury site was accelerated, and the therapeutic effect on bone injury was achieved.

Experimental animals: SD rats, male, 12 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended to form EB spheres and subjected to adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equi pment | Manufacturer | Cat. No. |
|---|---|---|
| R540 Enhanced small animal anesthesia machine | Ruiwode | R540 |
| Miniature handheld cranial drill | Ruiwode | 78001 |

(continued)

| Reagent/Equi pment | Manufacturer | Cat. No. |
|---|---|---|
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No.4 Pharmaceutical Co., Ltd. | None |
| Paraformaldehyde | LEAGENE | DF0135 |
| Live animal in vivo imaging system (small animal CT) | PE | PE Quantum FX |

Sample collection:

When collecting the specimens, the rats were placed in a supine position after intraperitoneal anesthesia, the skin was cut in the middle of the abdomen of the rat, the chest was opened, the heart was exposed, and the heart was perfused with ice-cold normal saline. Each rat needed about 50 ml of normal saline. After the normal saline perfusion was completed, the fixation was performed with 50 ml of paraformaldehyde. After the perfusion was completed, the bones at the injured site were cut, fixed with paraformaldehyde, sectioned and analyzed.

Modeling: The 12-week-old SD rat was anesthetized, then the left hind limb of the rat was drilled with the miniature handheld cranial drill to form a hole with a diameter of 3 mm. After the modeling, the rats were grouped, 6 rats per group.

Model group: 100ul of normal saline was injected around the muscle.

M cell group: 100ul of normal saline containing $3 \times 10^6$ M cells at P5 generation was injected around the muscle; after that, the rats were photoed and observed with the small animal CT on Day 10, 22 and 50, respectively. The results were shown in FIGS. 161 to 163.

Use of small animal CT: After the rat was anesthetized, it was fixed to the scanning position of the PE Quantum FX instrument. Micro-CT scans were performed near the site of bone injury in rats. The scanning conditions were: source voltage of 90kV, depth of 14bit, resolution FOV of 60mm, and scanning time of Fine 2 min. The scanning was performed with 0° rotation.

Experimental results: It could be seen in the CT images of the day 10 that the healing of the bone injury in the M cell treatment group had a better trend than that in the model group; it could be seen in the CT images of the day 22 that as compared with the model group, the healing of bone injury in the M cell treatment group had obvious advantages, and the bone injury area was smaller, indicating that the M cell transplantation could accelerate the healing of bone injury, and the M cells had a good therapeutic effect on bone injury; it could be seen from the CT images of the day 50 that the bone injury site in the M cell transplantation treatment group had been completely healed, while that in the model group had not yet healed completely. It indicated that the M cells could treat bone injury very well.

X-ray examination: After the rats were anesthetized, they were placed in a high-resolution digital radiography system (Faxitron MX-20) using a voltage of 32 kV for 10 s. The callus width of femoral fractures was determined by X-ray photoing and analyzed by Image-Pro Plus software.

From the above, it could be seen that the healing time of bone injury in the M cell treatment group was significantly shorter than that in the model group, the bone injury in the M cell treatment group healed earlier than in the model group, the size of callus was significantly larger than that in the model group, and the hole gap was smaller, indicating that the M cells could significantly accelerate the healing speed of bone injury.

Determination of bone mineral density after bone injury healing: The principle of bone mineral density measurement was that two kinds of energies, namely low-energy and high-energy photon peaks were obtained through X-ray tube via a certain device, after the photon peaks penetrated the body, the scanned signals were sent to the computer for data processing to obtain the bone mineral content.

By Micro-CT analysis: the bone mineral density of the M cell group was better than that of the model group.

Four-point bending mechanical test: After the experimental tissue was taken, the excised tissue was tested at room temperature within 24 h; through a four-point bending device (H25KS), a constant displacement rate of 5 mm/min was used to test whether the tissue sample was broken or not. The tibia was placed in an anteroposterior direction within blades with inner and outer spans of 8 mm and 20 mm, respectively. During testing, the long axis of the tibia was oriented perpendicular to the blade. After the test was completed, the built-in software (QMAT Professional Material test software) was used to record and analyze the ultimate load to failure, the energy absorbed by failure (the area under the load-displacement curve, referred to as toughness), and the elastic modulus (E-modulus, the slope of the stress-strain curve, referred to as tissue stiffness). Biomechanical properties of healed fractures were expressed as a percentage relative to the properties of contralateral intact bone.

The results of the four-point bending mechanical test clearly showed that the toughness, ultimate failure load (F) and E-modulus (G) of the M cell treatment group were higher than those of the model group. This indicated that the recovery of mechanical properties would be enhanced after the M cell treatment.

Histological analysis (HE staining): The tibia after sampling was fixed in 4% buffered formalin solution for 1 day, and then decalcified with 9% formic acid for 5 to 7 days. It was tried to cut the sample in half by using a slicer (longitudinal direction was in the sagittal plane), so that the section at the midsagittal plane for each sample was normalized. The samples were subjected to tissue treatment and then embedded in paraffin. Thin sections (7 $\mu$) were made along the long axis of each tibia in the sagittal plane on a rotary microtome (HM 355S). The sections were mounted on coated glass slides. Paraffin was removed by immersing the slides in xylene (twice at room temperature with changes every 5 min). The slides were then immersed in graded ethanol and distilled water, then stained with hematoxylin and eosin (H&E), and finally dehydrated and fixed.

HE analysis results: In the M cell group, although there were still mature osteocytes, cartilage tissue, fibrous tissue and undifferentiated tissue in the callus of the unhealed part, some connections had appeared in the holes, and its relative amount was higher than that of the model group, indicating that the M cells could promote the formation of various osteocytes.

Immunohistochemistry: The tissue sections were washed three times with PBS, then soaked in TBS blocking solution ((0.3%) Triton + (5%) BSA + PBS) for 30 min, and then soaked in antibody diluent ((0.3%) Triton + (1 %) BSA + PBS) with primary antibody (rabbit anti-GFP; 1:300) overnight, washed twice with PBS for 5 min each, and then socked in antibody diluent and secondary antibody (Cy3 goat anti-rabbit IgG; 1:1000) for 2 h, subjected to nuclear staining with DAPI for 10 min, washed twice with PBS for 5 min each time, and then observed and photoed, and cells were quantified with ImageJ.

Results of immunohistochemical analysis: The content of the observed osteoblast transcription factors in the M cell treatment group was higher than that in the model group, indicating the promotion of healing of bone injury.

The following were the preferred cell dosage regimens after screening:

100ul of saline suspension with $1 \times 10^6$ M cells;

50ul of saline suspension with $1 \times 10^6$ M cells;

100ul of saline suspension with $3 \times 10^6$ M cells;

50ul of saline suspension with $3 \times 10^6$ M cells;

100ul of saline suspension with $5 \times 10^6$ M cells;

50ul of saline suspension with $5 \times 10^6$ M cells.

Example 27: Evaluation of therapeutic activity of M cells against rhinitis

Allergic rhinitis (AR), also known as nasal allergy, is a common otolaryngology disease and a common respiratory allergic disease. The disease is an allergic disease that occurs in the nasal mucosa and is characterized by itching, sneezing, rhinorrhea and nasal discharge, and swelling of nasal mucosa. The prevalence of allergic rhinitis is 10% to 40%, among which pollen allergy is more common in Europe and North America, and perennial allergic rhinitis is more common in Asia. Although allergic rhinitis is not fatal, it affects the patient's study and work because of the obvious discomfort in the nose and the whole body. If not properly treated, about 30% of patients will develop bronchial asthma, and even pulmonary heart disease and other diseases that seriously affect the health and life quality of patients. Corticosteroids and antihistamines are currently the first-line treatments for allergic rhinitis. Allergic rhinitis is an allergic inflammatory reaction mediated by IgE under the action of environmental factors in vitro, and is dominated by the immune response of nasal mucosa.

References:

Adipose Tissue-Derived Mesenchymal stem cell Modulates the Immune Response of Allergic Rhinitis in a Rat Model.

This example evaluated the therapeutic effect of M cells on rhinitis. The experimental results showed that after the M cells were transplanted, the number of times of sneezing and scratching nose in the mice was significantly reduced; the symptoms of rhinitis were significantly improved.

Experimental animals: BALB/c mice, female, male, 7 to 8 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments. The M cells at P3 generation were resuscitated, digested and passaged, and used at P5 generation for subsequent experiments.

| Reagent/Equi pment | Manufacurer | Cat. No. |
|---|---|---|
| Ovalbumin | Sigma | S25067-25g |
| Aluminum hydroxide | Sigma | 239186-500G |
| 10µl pipette tip | Axygen | YC-HC01019 |
| 1ml pipette tip | Axygen | TF-1000-R-S |
| 0.5-10µL | eppendorf | 1449888 |
| 1000µL pipette | eppendorf | J46096F |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No.4 Pharmaceutical Co., Ltd. | None |
| Paraformaldehyde | LEAGENE | DF0135 |

Animal modeling and treatment: BALB/c mice were used for rhinitis modeling, which were divided into normal group, model group and M cell group, with 6 mice in each group.

Normal group: no treatment was carried out.

Model group: intraperitoneal injection of 200 µl of OVA-containing emulsion was performed on days 0, 3 and 7, intranasal instillation of 10 µl of solution containing 100 µg of OVA was applied to each nostril from day 7 to day 14, intravenous injection of 100 µl of normal saline was performed on days 14 and 17, and sampling was performed for analysis on day 21.

M cell group: intraperitoneal injection of 200 µl of OVA-containing emulsion was performed on days 0, 3 and 7, intranasal instillation of 10 µl of solution containing 100 µg of OVA was applied to each nostril from day 7 to day 14, intravenous injection of 100 µl of normal saline containing $3 \times 10^6$ M cells was performed on days 14 and 17, and sampling was performed for analysis on day 21.

Detection methods and results:

1) Evaluation of sneezing and scratching nose:

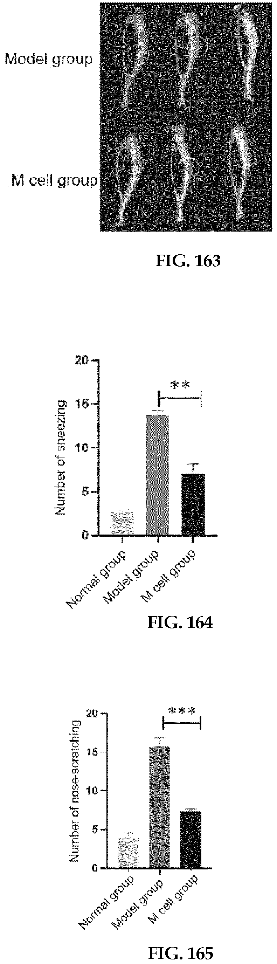

Mice were dripped with 10 µl of solution containing 100 µg of OVA in each nostril, and after acclimating for 5 minutes, the statistics of numbers of sneezing and scratching nose in an empty cage within 5 minutes were performed, and detailed in Table 27-1, Table 27-2, FIG. 164 and FIG. 165.

Table 27-1: Statistical values of sneezing within 5 minutes in mice on day 21 after stimulation

|  | Number of sneezing in mice (times) | | |
| --- | --- | --- | --- |
| Normal group | 3 | 2 | 3 |
| Model group | 15 | 13 | 13 |
| M cell group | 7 | 5 | 9 |

Table 27-2: Statistical values of scratching nose within 5 minutes in mice on day 21 after stimulation

|  | Number of scratching nose in mice (times) | | |
| --- | --- | --- | --- |
| Normal group | 3 | 4 | 5 |
| Model group | 18 | 15 | 14 |
| M cell group | 7 | 8 | 7 |

2) Enzyme-linked immunosorbent assay (ELISA)

(1) Coating antigen: the antigen was diluted with coating buffer to the optimal concentration (5 to 20 ug/ml) and added at 0.3 ml to each well of the micro-reaction plate, allowed to stand overnight at 4°C or in a water bath at 37°C for 2 to 3 hours, and stored in a refrigerator.

(2) Washing: the coating solution was removed, and the wells were washed three times with washing buffer (containing 0.05% Tween-20) for 5 minutes each time.

(3) Adding sample to be tested: to each well, 0.2 ml of the serum to be tested was added, which was diluted with the dilution buffer containing 0.05% Tween-20, and allowed to stand at 37°C for 1 to 2 hours.

(4) Washing: the coating solution was removed, and the wells were washed three times with washing buffer (containing 0.05% Tween-20) for 5 minutes each time.

(5) Adding enzyme conjugate: 0.2 ml of the enzyme conjugate diluted with dilution buffer was added to each well, and acted at 37°C for 1 to 2 hours.

(6) Washing: the coating solution was removed, and the wells were washed three times with washing buffer (containing 0.05% Tween-20) for 5 minutes each time.

(7) To each well (OPD or OT), 0.2 ml of substrate solution was added and acted for 30 minutes at room temperature (another blank control was set, in which 0.4 ml of substrate + 0.1 ml of terminating agent was added).

(8) Adding terminating agent: 0.05 ml of 2 M $H_2SO_4$ or 2 M citric acid was added to each well.

(9) Observation and recording results: OD values were measured visually or with an enzyme-labeled colorimeter (OPD was 492 nm).

Experimental results: Compared with the AR model group, the specific IgE, IgG1 and IgG2a of the M cell treatment group were significantly lower, the level of PGE2 was significantly higher than that of the AR model group, and the level of histamine was significantly lower than that of the AR model group. The results showed that the injection of M cells could reduce the level of serum antigen-specific antibody response, and reduce the expression of inflammatory mediators.

3) Histopathological examination

3-1. Specimen collection:

When collecting specimens, the mice were in supine position after intraperitoneal anesthesia, the skin was cut in the middle of the abdomen of the mice, the chest was opened, the heart was exposed, and the heart was perfused with ice-cold normal saline. About 20 ml of normal saline was needed for each mouse. After the normal saline perfusion was completed, the fixation was carried out with 20 ml of paraformaldehyde. After the perfusion was completed, the nasal cavity of the mice was taken, fixed, sectioned and analyzed.

3-2. Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

3-3. Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.
Experimental results: After administration of OVA allergen, the nasal mucosa structure changed significantly, epithelial cells were lost, the mucosa was exfoliated, inflammatory cells were infiltrated, and the blood vessels were reduced. After the M cell treatment, epithelial cells increased, a few cells infiltrated, and the number of blood vessels increased. The results showed that the M cells could promote angiogenesis in inflammatory sites, promote epithelial cell generation, and reduce inflammatory cell infiltration.

3-4. Masson's staining

Operation steps:

(1) Paraffin sections were provided, dewaxed to water;

(2) 1% potassium permanganate oxidized the sections for 5 minutes;

(3) Washed with water, and bleached with oxalic acid for 1 min;

(4) Washed with water, washed with distilled water;

(5) Stained with celestine blue for 5min;

(6) Washed with water, and shaken off the remaining liquid;

(7) Stained by dripping Mayer hematoxylin for 3 to 5min;

(8) Rinsed with running water for 5 to 10min;

(9) Stained with ponceau red-picric acid saturated solution for 5min;

(10) Washed with 1% acetic acid aqueous solution;

(11) Differentiated the sections with 1% phosphomolybdic acid for about 5 minutes;

(12) Washed with distilled water;

(13) Stained by dripping with 1% toluidine blue for 30s;

(14) Washed with 1% acetic acid aqueous solution;

(15) Differentiated with 95% ethanol;

(16) Dehydrated with absolute ethanol;

(17) Transparentized with xylene;

(19) Mounted with neutral resin.

Experimental results: The basal plate and lamina propria of the nasal mucosa in the rhinitis model group had obvious collagen fiber aggregation and collagen fiber deposition. In the M cell treatment group, there were fewer collagen fibers in the nasal mucosa lamina propria, and the deposition of collagen fibers was significantly reduced, indicating that the M cells could improve rhinitis epithelial fibrosis.

3-5. Detection by transmission electron microscopy

Methods: The samples were fixed with 1% osmic acid for 30 min and washed three times with PBS (10 min each time). The samples were dehydrated with ethanol (30%, 50%, 70%, 90% and absolute ethanol) for 30 min at each concentration. The samples were embedded in 502 resin after soaking in acetone for 1 h. Plastic molds were cut with a microtome and stained with 1% toluidine blue. After semi-thin section examination, ultrathin sections (thickness of 50 to 60 nm) were cut, stained with uranyl acetate, and then stained with lead citrate, and detected and photoed with a transmission electron microscope.

Experimental results: In the model group, the surface of epithelial cells was severely damaged, the nasal cilia were reduced, the cytoplasmic vacuolated nuclei were broken, mast cells increased, and granulocytes were infiltrated. In the M cell treatment group, the nasal mucosa epithelial surface was intact, the cilia were intact, the organelle morphology was normal, the fibroblasts were normal, and the cytoplasm was intact.

Example 28: Evaluation of therapeutic activity of M cells against graft-versus-host disease

Graft-versus-host disease (GVHD) is mainly due to the fact that after transplantation, T lymphocytes in the allogeneic donor transplant enhances the immune response to the recipient antigen through the influence of related cytokines in the recipient, so that a cytotoxic attack is launched with the target cells of the patient as target, of which the skin, liver and intestinal tract are the main targets, and the occurrence of GVHD mainly has the following three points: (1) the graft contains immunocompetent cells; (2) the donor is different from the recipient in histocompatibility antigen; (3) the immunocompetent cells of the donor survive because they are not rejected, and divide and proliferate when recognizing different histocompatibility antigens. It is generally believed that the immunocompetent cells involved in GVHD are the contaminated mature T cells, and the higher the contamination rate, the greater the probability of GVHD

At present, steroids, immunosuppressive factors and monoclonal antibodies are used as first- and second-line drugs for the treatment of GVHD. The action mechanism of glucocorticoid therapy is to inhibit the immune attack response to receptors mediated by T lymphocytes, but hormone therapy is not very ideal, high-dose hormone therapy will increase the body's infection and tumor recurrence rate, so that new, safer and more effective treatments are still needed for the treatment of GVHD. In recent years, the study of mesenchymal stem cells (MSCs) has become a hot spot in the field of modern biology. As a class of stem cells with self-renewal and multi-directional differentiation potential, they can differentiate into a variety of functional cells and organs under certain induction conditions, and their ability of proliferation and multi-directional differentiation of stem cells can be utilized to bring new hope for clinically intractable diseases, and they have gradually become a new treatment method in modern clinical medicine. Some studies have found that MSCs can inhibit the inflammatory response by inhibiting the proliferation of T cells, which have immune and inflammatory regulatory effects, which provides a new research direction for the treatment of GVHD

A large number of animal experiments have shown that MSCs transplantation in the treatment of GVHD shows good efficacy and safety. However, the clinical application of adult tissue-derived MSCs mainly has the following shortcomings: (1) the therapeutic amount of adult tissue-derived MSCs can hardly be obtained from a single individual tissue; (2) the adult tissue-derived MSCs are derived from different individual tissues, so that high consistency of product quality can hardly be achieved; (3) even MSCs derived from the same individual tissue are highly heterogeneous; (4) the donor tissue sources of adult tissue-derived MSCs are complex and have potential infectious pathogen infection risks; (5) the rapid senescence of adult tissue-derived MSCs occurs with in vitro expansion. Therefore, new sources of MSC cells are needed for the treatment of GVHD

References:

(1) Functional dosing of mesenchymal stromal cell-derived extracellular vesicles for the prevention of acute graft-versus-host to disease.

(2) Optimization of the Therapeutic Efficacy of Human Umbilical Cord Blood to Mesenchymal Stromal Cells in an NSG Mouse Xenograft Model of Graft-versus-Host Disease.

(3) An experimental model of idiopathic pneumonia syndrome after bone marrow transplantation: I. The roles of minor H antigens and endotoxin.

(4) Highly Sensitive Model for Xenogenic GVHD Using Severe Immunodeficient NOG Mice.

This example evaluated the therapeutic activity of M cells against GVHD, and the experimental protocol of this example was formulated with reference to the aforementioned documents.

Experimental animals: NCG mice, male, 6 weeks old. The animals were purchased from Beijing Weitongda Biotechnology Co., Ltd.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended to form EB spheres for adherent differentiation, the M cells at P0 generation were obtained, passaged and screened, and cryopreserved at P3 generation for subsequent experiments.

The P3 generation M cells were resuscitated, digested and passaged, and used for subsequent experiments.

| Reagent/Equi pment | Manufacturer | Cat. No. |
|---|---|---|
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Water bath | Sail Huachuang | SDY-1 |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |
| Electronic scale | Yasuwang | CC-1013-04 |
| Flow cytometer | Beckman | Cyto FLEX |
| hPBMC (human peripheral blood cells) | Cell Applications | 690PBK |
| hCD45 | Biolegend | 368511 |
| mCD45 | Biolegend | 103107 |
| Human lymphocyte separation solution | MP Biomedicals | 0850494X |
| Multifactor suspension chip system | Bio-Rad | Bio-Plex® 200 |
| 23-Factors kit | Bio-Rad | M60009RDPD |

Preparation of animal model:

(1) After mice were irradiated with 1.75G $\gamma$-ray for 6 hours, $5 \times 10^6$ hPBMCs were transplanted into the tail vein of mice.

(2) Experimental grouping:

Control group: not irradiated;

GVHD group: only normal saline was injected on days 2, 5 and 8 after irradiation and transplantation of hPBMC;

GVHD+low-dose M cell group: $1.5 \times 10^6$M cells were injected into the tail vein of mice on days 2, 5 and 8 after irradiation and transplantation of hPBMC;

GVHD+high-dose M cell group: $5 \times 10^6$M cells were injected into the tail vein of mice on days 2, 5 and 8 after irradiation and transplantation of hPBMC;

(3) The body weight was measured until the day 14; the survival rate was counted until the day 19. On the day 19, bone marrow was taken, perfusion was performed, and kidneys, colons, lungs and livers were collected, the collected samples were soaked in paraformaldehyde overnight, and followed by paraffin section and HE staining.

Sample collection:

When collecting the specimens, the mice were placed in a prone position after intraperitoneal anesthesia, the skin was cut in the middle of the abdomen of the mice, the abdominal cavity was opened, and blood was collected from the central vein. The chest was opened, the heart was exposed, and the heart was perfused with ice-cold normal saline.

After the normal saline perfusion was completed, the fixation was performed with 50 ml of paraformaldehyde. After the perfusion, kidneys, colons, lungs and livers were taken, fixed, sectioned and analyzed. The collected blood was centrifuged at 5,000 rpm for 15 min at room temperature, and the supernatant was collected for multifactor ELISA analysis.

Detection of human CD45-positive cell infiltration in bone marrow by flow cytometry

(1) 0.5 ml of bone marrow fluid was aseptically extracted.

(2) The bone marrow samples were dropped into 1 mL of PBS containing 1,000 U/ml heparin anticoagulant.

(3) it was then diluted to 10mL with PBS.

(4) 5 mL of the diluted bone marrow fluid was pipetted and slowly added to the surface of 4 mL of human lymphocyte separation solution.

(5) Under the above conditions, the bone marrow nucleated cells were layered on the interface formed between the PBS human lymphocyte separation solutions.

(6) The layer of nucleated cells was aspirated, added to 10 mL of PBS, and mixed well.

(7) Centrifugation was carried out at 1,000 r/min for 5 min.

(8) The supernatant was discarded, the pellet was resuspended in PBS, filtered with a cell sieve to remove cell clusters, the cells were counting, and subpackaged, $2 \times 10^6$ per tube.

(9) Centrifugation was carried out at 1200 rpm for 3 min.

(10) After blocking with 2% BSA blocking solution for 20 min, centrifugation was carried out at 1200 rpm for 3 min.

(11) The supernatant was discarded, the cells were resuspended with 100 μL of 1% BSA antibody dilution solution, added with direct-labeled antibody, and incubated at room temperature for 30 to 45 min.

(12) Washing was performed three times with 1 mL of PBS, centrifugation was carried out at 1200 rpm for 3 min, and the supernatant was discarded.

(13) After being resuspended in 300 μL of PBS, the cells were filtered with a 40 μm cell sieve, and loaded on the machine for detection.

Steps for tissue paraffin sectioning

(1) Fixation: the tissue was socked in 4% PFA overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 23-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex ManagerTM.

(4) 250 $\mu$L of standard dilution HB was added to the standard bottle, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1$\times$ with Bio-Plex detection buffer, and stored in the dark.

(7) The diluted magnetic beads were vortexed, and 50 $\mu$L of the magnetic beads was added to each well.

(8) The plate was washed twice with 100 $\mu$L of washing solution.

(9) The sample, standard, blank, and control of known concentration were vortexed, and added in an amount of 50 $\mu$L to each well.

(10) The plate was sealed with a sealing film, and shaken on a high-frequency shaker at 850$\pm$50 rpm at room temperature for 30 min.

(11) In step (10), when the remaining 10 min of shaking time was left, the detection antibody was vortexed for 5 s and diluted to 1$\times$.

(12) The plate was washed twice with 100 $\mu$L of washing solution.

(13) The diluted antibody was vortexed, and added in an amount of 250 $\mu$L to each well.

(14) The plate was sealed with a sealing film, and shaken on a high-frequency shaker at 850$\pm$50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1$\times$.

(17) The plate was washed twice with 100 μL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 μL of detection solution, the plate was sealed with a sealing film, and shaken on a high-frequency shaker at room temperature at 850±50 rpm for 30 s.

(22) After the sealing film was discarded, loading to machine was started.

Statistical analysis

One-way ANOVA and T-TEST in Prism 7.0 statistical analysis software were used for variance analysis and significance test, and the experimental data were expressed as mean ± standard error (Mean ± SE). *, $p<0.05$; **, $p<0.01$; ***, $p<0.001$.

2. Experimental results

Table 28-1: Statistical table of body weight change rate of each group.

| Body weight change rate (%) | Day 1 | Day 2 | Day 5 | Day 8 | Day 10 | Day 11 | Day 12 | Day 13 | Day 14 |
|---|---|---|---|---|---|---|---|---|---|
| Control group | 100.00 | 98.80 | 103.21 | 106.43 | 107.43 | 106.43 | 104.42 | 106.43 | 112.05 |
| | 100.00 | 99.62 | 103.08 | 102.69 | 103.46 | 103.46 | 100.77 | | |
| | 100.00 | 98.83 | 104.67 | 103.89 | 105.84 | 105.45 | 103.89 | 103.11 | 107.78 |
| | 100.00 | 97.22 | 100.00 | 103.57 | 105.16 | 104.37 | 101.98 | 103.97 | 108.73 |
| GVHD group | 100.00 | 97.20 | 98.40 | 86.40 | 83.20 | 82.00 | 73.20 | | |
| | 100.00 | 95.65 | 99.13 | 96.26 | 93.3 | 91.87 | 83.26 | 75.00 | |
| | 100.00 | 96.57 | 101.29 | | | | | | |
| | 100.00 | 96.54 | 96.10 | 91.77 | 89.88 | 87.98 | 79.06 | | |
| | 100.00 | 94.67 | 95.90 | 94.31 | 93.54 | 91.18 | 85.98 | 81.97 | 74.23 |
| GVHD+low-dose M cells | 100.00 | 100.46 | 99.54 | 88.53 | | | | | |
| | 100.00 | 100.00 | 100.41 | 93.88 | 97.14 | 96.18 | 90.90 | 86.00 | |
| | 100.00 | 100.93 | 104.63 | 101.85 | 97.87 | 94.26 | 92.39 | 88.46 | 86.90 |
| | 100.00 | 99.15 | 98.72 | 94.47 | 94.47 | 88.53 | 86.30 | 83.30 | |
| GVHD+high-dose M cells | 100.00 | 97.08 | 97.50 | 100.42 | 98.33 | 98.33 | 94.17 | 93.75 | 93.67 |
| | 100.00 | 100.88 | 100.88 | 99.56 | 99.56 | 102.64 | 98.68 | 93.39 | 92.99 |
| | 100.00 | 101.40 | 102.80 | 102.8 | 105.14 | 99.07 | 93.93 | 95.33 | 97.20 |

Table 28-2: Statistical table of number of survival mice in each group.

| Remaining number of mice | Control group | GVHD group | GVHD+low-dose M cells | GVHD+high-dose M cells |
|---|---|---|---|---|
| Day 0 | 4 | 5 | 4 | 3 |
| Day 8 | 4 | 4 | 4 | 3 |

(continued)

| Remaining number of mice | Control group | GVHD group | GVHD+low-dose M cells | GVHD+high-dose M cells |
|---|---|---|---|---|
| Day 10 | 4 | 4 | 3 | 3 |
| Day 12 | 4 | 2 | 3 | 3 |
| Day 13 | 4 | 1 | 1 | 3 |
| Day 14 | 4 | 0 | 1 | 3 |
| Day 16 | 4 | 0 | 1 | 2 |
| Day 19 | 4 | 0 | 1 | 2 |

Table 28-3: Statistical table of bone marrow chimeric rate of mice in each group.

| | Control group | GVHD | GVHD+low-dose M cells | GVHD+high-dose M cells |
|---|---|---|---|---|
| Flow cytometry detection of bone marrow chimeric rate (%) | 0.00 | 54.62 | 34.22 | 0.00 |
| | 0.60 | 59.06 | 19.06 | 9.52 |
| | 0.00 | 43.11 | | |
| | | 63.35 | | |

(1) The results of monitoring body weight showed that on the day 14, the body weight of the GVHD group was significantly lower than that of the control group (***p<0.001); there was no significant difference between the GVHD group and the low-dose M cell group, but was significantly lower than the high-dose M cell group (**p<0.01); there was no significant difference in body weight between the low-dose and high-dose M cell groups (Table 28-1, FIG. 170). The results showed that the M cells could increase the body weight of GVHD mice.

(2) Survival rate statistics showed that there was a significant difference in the survival rate between the control group and the GVHD group (*** p<0.001); there was a significant difference in the survival rate between the GVHD group and the GVHD+high-dose M cell treatment group (** p<0.01) (Table 28-2, FIG. 171). The results showed that M cells could improve the survival rate of GVHD mice.

(3) On the day 14, the bone marrow was taken, and the human and mouse CD45 positive cells were detected by flow cytometry, and the bone marrow chimeric rate of each group was compared. The results of the bone marrow chimeric rate of mice showed that the bone marrow chimeric rate of the GVHD+high-dose M cell group was significantly lower than that of the GVHD group (*p<0.05), indicating that M cells alleviated GVHD by reducing the infiltration of hPBMCs in the bone marrow (Table 28-3, FIG. 172). The results showed that the M cells could reduce the bone marrow chimeric rate of human CD45-positive cells in GVHD mice.

(4) On day 14, the intestine, kidneys, livers and lungs were taken, paraffin sectioned and HE stained. The results showed that the intestinal crypt structure of the GVHD+low/high-dose M cell groups was significantly better than that of the GVHD group, and there were more complete intestinal crypt structure. The infiltration of inflammatory cells in various organs in the GVHD+ low/high-dose M cell groups was significantly lower than that in the GVHD group, indicating that the M cells had the function of inhibiting inflammation and maintaining tissue integrity (FIG. 173). The results showed that the M cells could reduce inflammation and tissue damage in GVHD mice.

(5) The detection results of serum inflammatory factors in mice showed that as compared with the GVHD group, the levels of proinflammatory factors in the M cell treatment group were significantly decreased, and the levels of anti-inflammatory factors were significantly increased. It showed that the M cells had the effect of suppressing inflammation.

Non-patent documents:

1. Functional dosing of mesenchymal stromal cell-derived extracellular vesicles for the prevention of acute graft-versus-host-disease.

2. Optimization of the Therapeutic Efficacy of Human Umbilical Cord Blood to Mesenchymal Stromal Cells in an NSG Mouse Xenograft Model of Graft-versus-Host Disease.

3. An experimental model of idiopathic pneumonia syndrome after bone marrow transplantation: I. The roles of minor H antigens and endotoxin.

4. Highly Sensitive Model for Xenogenic GVHD Using Severe Immunodeficient NOG Mice.

Patent documents:

1. Mesenchymal lineage precursor or stem cells with enhanced immunosuppression (CN201880036997.2)

2. Method for selecting high-efficiency stem cells for the treatment of immune disorders (CN201780077281.2)

3. Use of hAMSCs in the manufacture of medicament for the treatment of acute graft-versus-host disease (CN201811145836.5)

4. Method for regulating immunomodulatory effect of stem cells (CN201811227664.6)

5. Use of mesenchymal stem cells in the manufacture of a drug for the treatment of M5 leukemia (CN201610208206.2)

6. Method for regulating immunomodulatory effect of stem cells (CN201380072996.0)

7. Use of recombinant mesenchymal stem cells in the manufacture of immunosuppressive agent (CN201410188453.1)

8. Preparation for suppressing immunity and treating graft-versus-host disease (GVHD) and preparation method thereof (CN201110041925.7)

Example 29: Evaluation of therapeutic activity of M cells against primary ovarian insufficiency

Primary ovarian insufficiency (POI) refers to the loss of ovarian function in women before the age of 40. In the 2015 ESHER guidelines, it is defined as: (1) amenorrhea/oligomenorrhea for at least 4 months; (2) 2 blood FSH>25U/L (interval of monitoring time is at least 4 weeks). It is characterized by menstrual disorders (amenorrhea or oligomenorrhea), elevated gonadotropins, and low estrogen levels (hot flashes, sweating, facial flushing, low libido, etc.). The incidence of POI is about 1%, and the incidence varies slightly among different ethnic groups. The incidence of POI in patients with primary amenorrhea is 10% to 28%, and the incidence of POI in patients with secondary amenorrhea is 4% to 18%.

The causes of POI include genetic, immune, iatrogenic (radiotherapy, chemotherapy, immunosuppressive therapy, and surgical treatment, etc.) and the like, but most POI causes are unknown. POI may be associated with a variety of endocrine disorders, including hypoparathyroidism and hypoadrenalism. Pelvic surgery may also lead to impaired ovarian function. Adrenal or ovarian antibodies are present in approximately 4% of patients with POI, suggesting that the disease is autoimmune. In many cases, the mechanism is unclear [1]. POI may cause loss of female fertility and increase the risk of osteoporosis, lipid metabolism disorders, and cardiovascular disease. Early amenorrhea and loss of fertility during the reproductive period will increase the psychological burden of women and reduce the quality of married life, resulting in a series of serious psychological and social problems.

Once a patient is diagnosed with POI, treatment options are very limited. At present, the main treatment measures mainly include hormone replacement therapy, immunosuppressive therapy, integrated traditional Chinese and Western medicine therapy, psychological therapy, receiving donated eggs, ovarian tissue and ovarian transplantation. Although these methods have certain effects, they cannot fundamentally repair the damaged ovarian function and restore the patient's fertility. Hormone replacement therapy may relieve the clinical symptoms of hormone deficiency, but the side effects of long-term use of estrogen and progesterone make it difficult for patients to use it for a long time. There have been reported that pregnancy was achieved by treating the immune factors-induced POI with immunosuppressive therapy, but immunosuppressive therapy may cause serious side effects, and the blind application of immunosuppressive therapy to POI is not recommended in clinical practice. Chinese medicine adjuvant therapy may improve some clinical symptoms. Egg donation-assisted reproductive technology may realize fertility wishes, but the current shortage of egg sources limits its application in solving the fertility problems of POI patients. None of the above methods can fundamentally treat primary ovarian insufficiency and restore fertility in POI patients.

With the continuous promotion of stem cell treatment, several research groups have tried the safety and efficacy of stem cell treatment for POI through animal experiments this year. Johnson et al. found that intraperitoneal transplantation of bone marrow mesenchymal stem cells can directly reach the injured ovary, reduce the apoptosis of granulosa cells, repair ovarian damage caused by chemotherapy drugs, and improve ovarian function. Professor Yao Yuanqing's research team transplanted umbilical cord mesenchymal stem cells (UCMSCs) into POF mice, and found that ovarian granulosa cell apoptosis decreased, the number of follicles increased, ovarian function recovered, and sex hormone levels increased, but the umbilical cord mesenchymal stem cells cannot differentiate to form follicles. The above studies suggest that stem cells may repair damaged ovarian tissue and improve ovarian function.

However, various sources of adult tissue-derived MSCs also have many problems in practical clinical applications, such as the limited number of MSCs derived from a single tissue; the high heterogeneity of MSCs from different tissue sources; individual donor tissue sources with potential pathogen infection risk; rapid senescence when expanded in vitro. The above shortcomings make it impossible to standardize the preparation of tissue-derived MSCs, and the cell quality cannot be guaranteed. With the gradual maturation of embryonic stem cell differentiation induction systems and culture methods, embryonic stem cells can stably differentiate in vitro to form mesenchymal cells, thereby overcoming the shortcomings of direct application of embryonic stem cells and adult tissue-derived MSCs, and meeting the standards for standardized preparation and cell medicine.

The present invention overcomes the limitation of MSCs in clinical application, uses the M cells with a higher standardization degree to treat POI mouse model induced by chemical drugs, and provides a safer and more effective basis for clinical treatment of POI.

References:

[1] Committee opinion no. 605: primary ovarian insufficiency in adolescents and young women [J]. Obstet Gynecol, 2014, 124(1):193 to 197.

[2] Tavassoli M, Crosby WH. Transplantation of marrow to extramedullary sites [J]. Science, 1968, 161(3836):54 to 56.

[3] Johnson J, Bagley J, Skaznik-Wikiel M, et al. Oocyte generation in adult mammalian ovaries by putative germ cells in bone marrow and peripheral blood [J]. Cell, 2005, 122(2):303 to 315.

[4] Wang S, Yu L, Sun M, et al. The therapeutic potential of umbilical cord mesenchymal stem cells in mice premature ovarian failure [J]. Biomed Res Int, 2013, 2013:690491.

[5] Gibson, J.D., et al., Regeneration of Articular Cartilage by Human ESC-Derived Mesenchymal Progenitors Treated Sequentially with BMP to 2 and Wnt5a. stem cellS Translational Medicine, 2017. 6(1): p. 40 to 50.

[6] Gonzalo to Gil, E., et al., Human embryonic stem cell-derived mesenchymal stromal cells ameliorate collagen-induced arthritis by inducing host-derived indoleamine 2,3 dioxygenase. Arthritis Res Ther, 2016. 18: p. 77.

[7] Ninagawa, N.T., et al., Transplantated mesenchymal stem cells derived from embryonic stem cells promote muscle regeneration and accelerate functional recovery of injured skeletal muscle. Biores Open Access, 2013. 2(4): p. 295 to 306.

[8] Zhang, Y., et al., Improved cell survival and paracrine capacity of human embryonic stem cell-derived mesenchymal stem cells promote therapeutic potential for pulmonary arterial hypertension. Cell Transplant, 2012. 21(10): p. 2225 to 39.

[9] Wang, X., et al., Immune modulatory mesenchymal stem cells derived from human embryonic stem cells through a trophoblast to like stage. stem cells, 2016. 34(2): p. 380 to 385

Experimental animals: ICR mice, female, 7 to 8 weeks old, purchased from Beijing Weitong Lihua Company.

All animals were kept at SPF grade in experimental animals of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals the were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended to form EB spheres for adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Masson staining solution | Nanjing Jiancheng | D026-1-2 |
| Neutral resin | Solebol | G8590-100 |

Preparation of animal model: SPF grade female ICR mice, 6 weeks old, 100 mice, purchased from Sibeifu (Beijing) Biotechnology Co., Ltd. The feeding and handling of the experimental animals were performed strictly in accordance with the relevant regulations promulgated by the Laboratory Animal Ethics Committee of the Institute of Zoology, Chinese Academy of Sciences. The mice with a normal estrous cycle of 4 to 5 days determined by vaginal smear method were recruited in the experiment. In this experiment, busulfan (BUS) and cyclophosphamide (CTX) were selected and combined to perform chemotherapy. The mice were administered by intraperitoneal injection of chemotherapy, and the dose was 120 mg/kg CTX+30 mg/kg BUS according to the body weight of the mice. The experiment was divided into three groups: (1) Normal group: mice were intraperitoneally injected with solvent DMSO, $N$=35 mice; (2) Model group: after chemotherapy drug treatment, 0.1 M DPBS was infused into tail vein, $N$=35 mice; (3) M cell group: after chemotherapy drug treatment, each mouse was infused via tail vein with 100 $\mu$L of 0.1 M DPBS cell suspension containing $1\times10^6$ M cells, $N$=35 mice.

Body weight and ovarian weight measurements:

Body weight and ovarian weight were determined using the analytical balance method.

Follicle counting

Ovaries were collected on the day 10 after the M cell treatment, and the number of follicles was counted. Fresh ovarian specimens were fixed with 4% paraformaldehyde (Sigma, P6148) for at least 12 hours. After dehydration and paraffin embedding, serial sections were made at 5 $\mu$m thickness, and adherence was performed once every 5 sections. Routine hematoxylin (Solarbio, G1080-100) and eosin (ZSGB-BIO, ZLI-9613) (H&E) staining was performed for further histological examination. Primitive follicles, primary follicles, secondary follicles, and sinus follicles were classified and counted. To avoid double counting any follicles, only those with oocytes were included for further analysis.

Cell tracing

In cell tracking studies, flow cytometry, animal imaging, and GFP signal detection methods were employed. Flow cytometry was used to collect venous blood from endothelial cells of each mouse at 1, 4, 24 and 48 hours after humsc transplantation. After incubation with whole blood erythrocyte lysate for 30 min at room temperature, the cell suspension was washed with PBS and analyzed by flow cytometry. To detect GFP signal, the mice were sacrificed on the day 7 after cell transplantation. Ovarian specimens were paraffin embedded and sectioned as above described. The GFP signals were observed with a fluorescence microscope after sectioning.

E2 and FSH detection

Venous blood was collected from endothelial cells during estrus in mice. Blood samples were left at room temperature for 60 minutes. After coagulation, centrifugation was carried out at 4,000 rpm/min for 15 minutes at 4°C. The supernatant was collected and sent to Beijing Northern Institute of Biotechnology (Beijing, China) to determine serum FSH and E2.

RNA extraction and RT-PCR identification

RNA extraction was performed using Invitrogen's TRIZOL in a fume hood.

The sample tissue was ground with an electric grinding rod, then transferred into a 1.5 ml RNA-free tube, added with 1 ml of TRIZOL to lyse the cells, and the lysate was collected and added into a 1.5 ml RNA-free EP tube. Incubation was carried out at 4°C for 15 min, 500 μl of chloroform was added to each tube, mixed by vortexing and shaking, and allowed to stand on ice for 10 min; centrifugation was carried out at 4°C, 12,000 rpm for 15 min; the upper layer of the separated liquid layers was collected with a 1 ml pipette, transferred to a new 1.5 ml RNA-free EP tube, added with isopropanol in an equal volume of the transferred upper layer, mixed by vortexing and shaking, and allowed to stand on ice for 10 min; centrifugation was carried out at 4°C, 12,000 rpm/10 min; the supernatant was discarded, the pellet was washed twice with 75% ethanol, centrifuged at 4°C, 12,000 rpm/10 min; the supernatant was discarded, the RNA was dried in a fume hood for 5 to 10 min, in which the drying time should not be too long, otherwise the solubility of RNA would be reduced, and the quality of RNA would be decreased. RNA-free water was added, and heated on a metal bath at 55°C for 10 min. The RNA concentration and OD value were measured by Nanodrop.

Reverse transcription of mRNA

(1) 2 μg of RNA extracted by reverse transcription, 1 μl of Oligo(dT) Primer, 1 μl of dNTP Mixture were added with RNA-free water to reach 10 μl. Denaturation was performed at 65°C for 5 min, and incubation was carried out at 4°C for 3 min.
(2) The following reagents were further added to the above 10 μl system for reaction, and the total system was 20 μl.
(3) After mixing gently, reaction was carried out at 42°C for 60 min, and then reaction was carried out at 70°C for 15 min.

| 10 μl reaction system | |
| --- | --- |
| Reagent | Volume (μl) |
| 5X PrimeScript Buffer | 4 |
| RNase Inhibitor | 0.5 |
| PrimeScript RT | 0.7 |
| RNase free H2O | 4.8 |

Real-time PCR

The reverse transcribed cDNA was diluted 5 times, and RT-PCR was performed.

| 10 μl reaction system | |
| --- | --- |
| Reagent | Volume (μl) |
| cDNA | 1 |
| SYBP | 5 |
| $H_2O$ | 3.4 |

(continued)

| Reagent | Volume (μl) |
|---------|-------------|
| Primer | 0.6 |

Results:

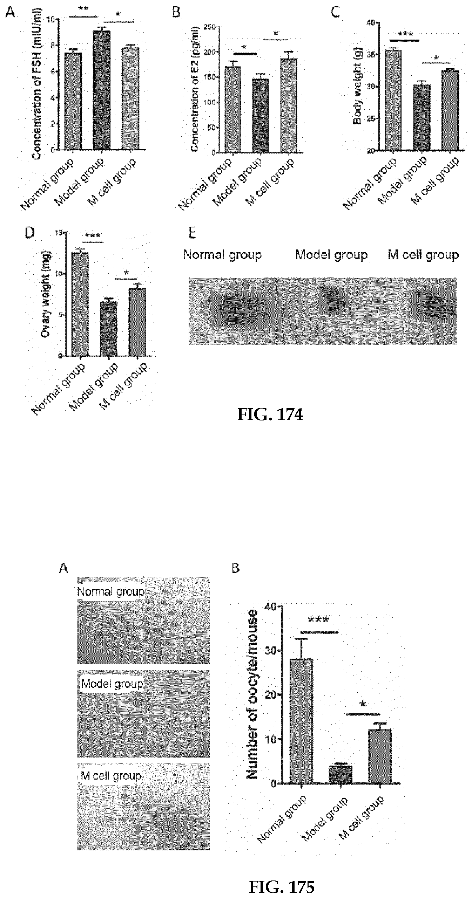

1. After modeling, the hormone levels in the mice changed significantly, the FSH level increased, the E2 level decreased, and the body weight and ovarian weight decreased significantly, showing the pathological characteristics of premature ovarian failure. The results were shown in FIG. 174. With the M cell treatment, the hormone levels in the mice with premature ovarian failure were significantly improved, and their body weight and ovarian weight were also significantly increased. In addition, the ovulation levels of mice with premature ovarian failure were also significantly restored after the M cell injection treatment.

2. Fluorescently labeled M cells were injected into mouse ovaries, and the cells could still be detected after 3 weeks, indicating that the M cells could survive in mice and were ideal seed cells for the treatment of POF.

3. The apoptosis of granulosa cells was determined by detecting the changes of bcL-2 gene mRNA expression in follicular granulosa cells. The results showed that the expression level of bcL-2 gene mRNA in granulosa cells increased and apoptosis decreased in the M cell treatment group. It showed that the M cells could rebuild ovarian function and reduce the apoptosis of granulosa cells.

4. By mating with normal male mice, the ability to produce offspring between the two groups was compared; it was found that the total number of offspring produced in the M cell treatment group was significantly higher than that in the control group.

5. By sectioning and staining of the ovaries, it was found that the ovary structure of the mice in the M cell treatment group was closer to that of the mice in the normal group.

6. By counting of the follicles, it was found that the number of follicles in the mice treated with the M cells was significantly higher than that in the control group. The results were shown in FIG. 175.

In conclusion, the M cell transplantation treatment could improve the symptoms of premature ovarian failure, and the level of ovulation could also be significantly restored; it could rebuild ovarian function and reduce the apoptosis of granulosa cells. It showed that the M cell treatment could treat premature ovarian failure symptoms very well.

Example 30: Evaluation of therapeutic activity of M cells against renal fibrosis

Renal fibrosis is a pathophysiological change, which is a gradual process that kidney changes in function from healthy to injured, then damaged, and finally lost its function. Due to the stimulation of various pathogenic factors such as trauma, infection, inflammation, blood circulation disorder, and immune response, the intrinsic cells of the kidney are damaged, and a large amount of collagen deposition and accumulation occurs in the later stage of development, causing the renal parenchyma to gradually harden and form scars until the kidney completely loses its organ function. In this example, the treatment of renal fibrosis was achieved by the transplantation of M cells.

Non-patent documents:

1. Serum-free medium Enhances the Immunosuppressive and Antifibrotic Abilities of Mesenchymal stem cells Utilized in Experimental Renal Fibrosis

2. Mesenchymal stem cells Deliver Exogenous MicroRNA-let7c via Exosomes to Attenuate Renal Fibrosis

3. Rat Mesenchymal Stromal Cell Sheets Suppress Renal Fibrosis via Microvascular Protection

4. Mesenchymal stem cells attenuate renal fibrosis through immune modulation and remodeling properties in a rat remnant kidney model

Patent documents:

1. Novel anti-renal fibrosis biological preparation of human umbilical cord MSC exosomes and preparation method thereof (CN201910389341.5)

2. Gene enhancing anti-inflammatory ability of human adipose tissue-derived mesenchymal stem cells and uses thereof (CN201810277760.5)

3. Patent title: Use of human adipose tissue-derived mesenchymal stem cells in kidney and fundus diseases (CN200910209321.1)

Experimental animals: C57BL/6J mice, male, 7 to 8 weeks old, purchased from Beijing Weitong Lihua Company.

All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equipment | Manufacturer | Cat. No. |
|---|---|---|
| Embedding machine | Leica | EG1150H/C |
| Sectioning machine | Leica | RM2235 |
| Section displaying machine | Leica | HI1210 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solarbio | G8590-100 |
| Masson staining solution | Nanjing Jiancheng | D026-1-2 |
| Chemray 240 Automatic Biochemical Analyzer | Rayto | Chemray 240 |
| Chloral hydrate | 3A | A46191-500g |
| α-SMA | SAB | 41550 |
| CD31 antibody | Biorbyt | orb302533-100ul |
| TGF-β1 antibody | Biovision | 5559-30T |
| E-cadherin antibody | Santa-Cruz | sc-71009 |
| Vimentin antibody | Proteintech | 60330-1-Ig-100ul |

Animal modeling: The mice were anesthetized by intraperitoneal injection of 10% chloral hydrate solution, and then fixed, and a 1.5 cm incision was made in the middle of the lower abdomen. The left ureter was dissociated, ligated and cut off to make the left kidney completely obstructed. After the operation, each mouse was treated with penicillin injection for 3 days. They were divided into sham operation group, surgery+solvent group, and surgery+test substance group, with 4 mice in each group.

Sham operation group: only the left ureter was dissociated, without ligation and cutting.

Solvent group: 100ul of normal saline was injected.

M cell group: 100ul of normal saline containing $3 \times 10^6$ M cells (P5 generation) was injected.

Treatment was performed on the day of surgery, the mice were placed in a metabolic cage on the 13th day, urine, blood and samples were collected on the 14th day.

Sample collection:

On the day 13 after transplantation, the mice in each group were put into a metabolic cage, 24 h urine was collected, and blood was collected from the tail vein to separate serum.

On the day 14 after transplantation, the mice in each group were sacrificed, and the kidneys were quickly removed. Half of the tissues were fixed and dehydrated, and then made into 5 $\mu$m paraffin sections for HE staining and Masson staining; the other half of the tissues were sharply frozen with liquid nitrogen, subjected to immunohistochemical staining of $\alpha$-SMA and CD31, and observed to identify the pathological changes of kidney structure and fibrosis.

Mouse body weight measurement:

The body weight of the mice was measured on the day of transplantation (Day 1) and on the Day 5, Day 8 and Day 14 after transplantation, respectively. The results were shown in FIG. 176.

Steps for tissue paraffin sectioning:

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:

Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:

After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

Masson staining:

(1) Dewaxing paraffin sections to water: The sections were placed in xylene I for 20 minutes, xylene II for 20 minutes, anhydrous ethanol I for 10min, anhydrous ethanol II for 10min, 95% alcohol for 5min, 90% alcohol for 5min, 80% alcohol for 5min, 70% alcohol for 5min in sequence, and washed with distilled water.

(2) Hematoxylin staining of nuclei: staining was performed for 5 min with Weigert's iron hematoxylin in the Masson staining kit; after being washed with tap water, differentiation was performed with 1% hydrochloric acid-alcohol for several seconds, rinsing was performed with tap water, and returning to blue was achieved by rinsing with running water for several minutes.

(3) Ponceau red staining: staining was performed for 5 to 10 min with Ponceau red acid fuchsin solution in the Masson staining kit, and rinsing was quickly performed with distilled water.

(4) Phosphomolybdic acid treatment: the treatment with phosphomolybdic acid aqueous solution in the Masson staining kit was performed for about 3 to 5 min.

(5) Aniline blue staining: instead of washing with water, counterstaining was performed for 5 min with aniline blue solution in the Masson staining kit.

(6) Differentiation: the treatment with 1% glacial acetic acid was performed for 1 min.

(7) Dehydration and mounting: the sections were placed in 95% alcohol I for 5min, 95% alcohol II for 5min, absolute ethanol I for 5min, absolute ethanol II for 5min, xylene I for 5min, xylene II for 5min in sequence to perform dehydration and transparentizing, then the sections were taken out from xylene and slightly air-dried, and mounted with neutral resin.

(8) Microscopic examination was performed with a microscope, and images were acquired and analyzed.

Biochemical testing:

The 24h urine protein, serum creatinine and blood urea nitrogen levels were detected by a Chemray 240 automatic biochemical analyzer. The results were shown in FIGS. 177 to 183.

Table 30-1: Statistics of body weight values of mice in each group of renal fibrosis model on days 1, 5, 8, 12 and 14

| Group | Body weight (g) | | | | |
| --- | --- | --- | --- | --- | --- |
| | Day 1 | Day 5 | Day 8 | Day 12 | Day 14 |
| Sham operation | 22.5 | 22.3 | 23.8 | 24.5 | 22.7 |
| | 21.3 | 21.3 | 21.9 | 22.3 | 20.7 |
| | 23.1 | 23.0 | 24.0 | 24.3 | 23.2 |
| | 22.3 | 22.2 | 23.1 | 24.4 | 22.4 |
| Surgery+solvent | 21.5 | 20.2 | 21.3 | 22.3 | 21.2 |
| | 21.7 | 19.8 | 19.8 | 22.4 | 20.9 |
| | 22.1 | 19.7 | 21.0 | 22.3 | 20.9 |
| | 22.6 | 21.1 | 21.9 | 22.5 | 21.0 |
| Surgery+test substance | 21.7 | 20.2 | 22.0 | 22.9 | 21.9 |
| | 22.9 | 22.1 | 23.4 | 24.4 | 23.5 |
| | 21.7 | 21.1 | 21.0 | 22.8 | 21.2 |
| | 22.9 | 20.8 | 23.0 | 23.7 | 22.3 |

Table 30-2: Statistical results of urinary microalbumin values of mice in each group of renal fibrosis model on day 14

|  | Sham operation | | | | Surgery+solvent | | | | Surgery+test substance | | | |
|---|---|---|---|---|---|---|---|---|---|---|---|---|
| Urinary microalbumin (pg) | 6.3 | 2.0 | 2.0 | 7.1 | 7.7 | 14.5 | 7.3 | 8.3 | 0.5 | 7.3 | 2.4 | 6.0 |

Table 30-3: Detection results of urine creatinine (CREA), urea (UREA), and uric acid (UA) of mice in each group of renal fibrosis model on day 14

| Group | Sham operation | | | | Surgery+solvent | | | | Surgery+test substance | | | |
|---|---|---|---|---|---|---|---|---|---|---|---|---|
| CREA ($\mu$mol/L ) | 44.8 | 42.1 | 46.2 | 41.7 | 53.5 | 48.9 | 45.1 | 47.7 | 38.6 | 42.6 | 43.7 | 43.2 |
| UREA (mmol/L) | 8.0 | 8.2 | 8.0 | 7.6 | 13.6 | 12.3 | 11.1 | 10.9 | 10.2 | 10.6 | 9.1 | 10.0 |
| UA ($\mu$mol/L ) | 123.2 | 111.9 | 119.5 | 113.3 | 156.6 | 177.3 | 273.3 | 197.4 | 75.5 | 72.5 | 128.9 | 141.6 |

Table 30-1 and FIG. 176 showed the statistical results of body weight values of mice in each group of the renal fibrosis model on days 1, 5, 8, 12 and 14, in which the body weight of the sham operation group at each time point was significantly higher than that of the surgery+solvent group (P<0.05); the body weight values of the surgery+M cell treatment group on days 12 and 14 were significantly higher than those of the surgery+solvent group (P<0.05); however, there was no significant difference in body weight between the sham operation group and the surgery+M cell treatment group on the days 12 and 14. The above results indicated that the M cell treatment had a significant promoting effect on the body weight of renal fibrosis mice.

Table 30-2 and FIG. 177 showed the statistical results of urinary microalbumin of mice in each group of the renal fibrosis model, in which the urinary microalbumin value of the surgery+solvent group was significantly higher than that of the sham operation group (*, P<0.05), indicating that the model was successfully constructed. Compared with the sham operation group, the urinary microalbumin content in the M cell treatment group was not significantly different, but was significantly lower than that in the solvent group (#, P<0.05), indicating that M cells had a certain therapeutic effect on renal fibrosis.

Table 30-3 and FIG. 178 showed the statistical results of urine creatinine content of mice in each group of the renal fibrosis model on the 14th day, in which the urine creatinine value of the surgery+solvent group was significantly higher than that of the sham operation group (*, P<0.05), indicating that the model was successfully constructed. Compared with the sham operation group, the urinary creatinine content of the M cell treatment group was not significantly different, but was significantly lower than that of the solvent group (#, P<0.05), indicating that the M cells had a certain therapeutic effect on renal fibrosis.

Table 30-3 and FIG. 179 showed the statistical results of urea content of mice in each group of the renal fibrosis model, in which the urea value of the surgery+solvent group was significantly higher than that of the sham operation group (**, P<0.01), indicating that the model was successfully constructed. Compared with the sham operation group, the urea content in the M cell treatment group was significantly higher (*, P<0.05), and was lower than that in the solvent group, but there was no significant difference, indicating that the M cells had a certain treatment trend for renal fibrosis.

Table 30-3 and FIG. 180 showed the statistical results of uric acid content of mice in each group of the renal fibrosis model, in which the uric acid value in the surgery+solvent group was significantly higher than that in the sham operation group (*, P<0.05), indicating that the model was successfully constructed. The content of uric acid in the M cell treatment group was significantly lower than that in the solvent group (#, P<0.05), indicating that the M cells had a certain therapeutic effect on renal fibrosis.

FIG. 181 showed the results that the mice in each group of the renal fibrosis model were sampled on the day 14, and the kidneys were embedded and sectioned, followed by HE staining, in which G1 was the sham operation group, G2 was the surgery+solvent group, and G3 was the surgery+M cell group. The left kidney was operated kidney, and the right kidney was not operated and used as a control. It could be seen from the figure that the basic structure of kidney of the mice in G2 group disappeared, and a large number of fibroblasts proliferated; the kidney structure of the mice in G3 group was improved, the tubular atrophy was alleviated, the infiltration of inflammatory factors and the proliferation of fibroblasts were reduced, and the necrosis area was reduced in some extent.

FIG. 182 showed the results that the mice in each group of the renal fibrosis model were sampled on day 14, and the kidneys were embedded and sectioned, followed by Masson staining, in which G1 was the sham operation group, G2 was the surgery+solvent group, and G3 was the operation+M cell group. The left kidney was operated kidney, and the right kidney was not operated and used as a control. It could be seen from the figure that there was no obvious collagen deposition in the kidney tissue of G1 group; there were sheet-like positive areas stained in blue in G2 group, which were mostly distributed around the renal tubules, indicating that a large number of collagen fibers were deposited in the renal interstitium; the blue area of the kidney tissue of the mice in G3 group was significantly reduced, and the color was lightened. The above results indicated that the M cells had played an inhibitory role in the mouse renal fibrosis model, improved renal structure, reduced collagen deposition, and delayed the progression of renal fibrosis.

FIG. 183 showed the results that the mice in each group of the renal fibrosis model were sampled on day 14, and the kidneys were subjected to immunohistochemical staining for $\alpha$-SMA and CD31, in which G1 was the sham operation group, G2 was the surgery+solvent group, and G3 was the surgery+M cell group. The left kidney was operated kidney, and the right kidney was not operated and used as a control. Endothelial-mesenchymal transition (EndoMT) is an important mechanism for the generation of myofibroblasts in injured kidneys. EndoMT refers to a process in which endothelial cells lose their anchoring connections and polar functions, and then transform into highly invasive and migratory slender spindle-shaped mesenchymal cells; endothelial cells change in morphology and polarity, as well as in biochemical properties, lose their characteristic marker CD31, etc., while regain the mesenchymal cell marker $\alpha$-smooth muscle actin (a-SMA), and convert into viable mesenchymal cells. It could be seen from the figure that compared with the left kidney of G1 group, the expression of $\alpha$-SMA in the kidney tissue of G2 group increased on the day 14, and the expression of CD31 did not change significantly, indicating that the model was constructed successfully, and the mice showed renal fibrosis phenotype. On the day 14, the expression of $\alpha$-SMA in the left kidney of G3 group was lower than that of G2 group, and the expression of CD31 was higher. The above results showed that the M cell treatment had a tendency of alleviating renal fibrosis, which could inhibit the process of renal fibrosis by reducing EndoMT.

TGF-β1 and mesenchymal transition indicators in kidney tissue of mice in each group were detected by western blotting (HU Yu to yan, et.al., 2020, Journal of Jiangsu University (Medicine Edition)).

Experimental results: The Western blotting results showed that the expressions of TGF-β1 and vimentin in the surgery+solvent group were higher than those in the sham operation group, while the expression of E-cadherin was down-regulated; the expressions of TGF-β1 and vimentin in the surgery+M cell group decreased, E-cadherin content was up-regulated. The results showed that the M cells could inhibit the expression of pro-fibrotic factors such as TGF-β1 in the renal interstitium, and could reverse the interstitial transition, thereby protecting the kidneys. The M cells could have therapeutic effect on renal fibrosis and related diseases such as glomerular disease, ureteral obstruction and renal failure.

Example 31: Evaluation of therapeutic activity of M cells against Parkinson's disease

Parkinson's disease (PD), also known as "shaking palsy", is a common neurodegenerative disease in the elderly and the most common extrapyramidal disease in the middle-aged and elderly. The disease has characteristic motor symptoms, including resting tremor, bradykinesia, myotonia, and postural balance disturbances, as well as non-motor symptoms, including constipation, olfactory disturbance, sleep disturbance, autonomic dysfunction, and mental, cognitive and cognitive disorders. Among Chinese people over 65 years old, there are 1700 PD patients per 100,000 people. Genetic factors, environmental factors (long-term exposure to industrial or agricultural toxins), and age are closely related to the development of PD. The treatment for PD is mainly drug therapy, which has developed to the third generation so far. The first generation of anticholinergic drugs include: anticholinergic drugs (trihexyphenidyl, benzatropine, procyclidine, biperiden, scopolamine); the second generation is levodopa; the third generation is dopamine receptor agonist and enhancer (benserazide). Drug therapy can effectively improve symptoms and improve quality of life within five years, but there is currently no solution to the side effects and related complications of drugs. Surgical treatment can significantly improve motor symptoms, especially limb tremors and muscle rigidity, but has no significant effect on non-motor symptoms. Surgery cannot cure the disease, and medical treatment is still necessary after surgery. In addition, there are some treatment methods including rehabilitation training, nutritional support and psychological support.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were recovered, digested and passaged, and P5 generation was used for subsequent experiments.

Experimental animals: Sprague-Dawley male rats, 6 to 8 weeks old, purchased from Beijing Sibeifu Biotechnology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%. The experiment was started after one week of adaptive feeding of rats.

Experimental groups: normal control group, PD+solvent (solvent group), PD+M cells (M cell group).

Experimental materials: surgical instruments, 5-0 surgical sutures, rat body weight scale.

Experimental reagents: 6-hydroxydopamine, normal saline, L-ascorbic acid, apomorphine hydrochloride, isoflurane, iodophor.

Equipment: R540 enhanced small animal anesthesia machine, brain stereotaxic instrument, microinjection pump

| Consumable/Reagent/Instrument | Manufacturer | Cat. No./Model |
|---|---|---|
| 5-0 surgical suture | Stones | EB01 |
| Isoflurane | Ruiwode | 970-00026-00 |
| Iodophor | Hangzhou Langso Medical Disinfectant Co., Ltd. | |
| Normal saline | domestic | |
| 6-Hydroxydopamine | Sigma | 162957-1 |

(continued)

| Consumable/Reagent/Instrument | Manufacturer | Cat. No./Model |
|---|---|---|
| Ascorbic acid | Sigma | A8960 |
| Apomorphine hydrochloride | Sigma | A4393-250MG |
| R540 Enhanced small animal anesthesia machine | Ruiwode | R540 |
| Brain stereotaxic instrument | Ruiwode | 69100 |
| Microinjection pump | Ruiwode | 788130 |

Experimental steps: the scale of the anesthesia machine was adjusted to 3.5, the rats were anesthetized and maintained at the anesthetized state, the rats were in a prone position, the skin of rat head was wiped with cotton swabs dipped with iodophor, a 1-1.5 cm incision was made, after the meninges was removed by cotton swabs, the rat head was fixed with a brain stereotaxic instrument, the striatum was injected with 2.5 mg/ml 6-hydroxydopamine, positioned as follows: +2 mm left of midline; -2.5 mm behind bregma; -8.5 mm under skull, 4 $\mu$l of injection volume, 1 $\mu$l per minute, after the injection, the needle was kept for 5 minutes, then pulled out, and the skin was sutured.

Cell injection: the injection at 6 points was performed in the striatum, the positioning points were as follows: injection coordinate 1 (+3 mm left of midline; +1 mm before anterior bregma; -5.0 and -4.5 mm under skull); injection coordinate 2 (+3.7 mm left of midline; +0.1 mm before anterior bregma; -5.0 and -4.5 mm under skull); injection coordinates 3 (+4.5 mm left of midline; +1.2 mm before anterior bregma; -5.0 and -4.5 mm under skull). The PD+solvent group was injected with normal saline, and the PD+M cell group was injected with M cells: $1 \times 10^5$/1ul per site, and the total cells at 6 sites were $6 \times 10^5$.

Rotation experiment: At 3 and 7 weeks after operation, apomorphine (0.5 mg/kg, 0.1% ascorbic acid) was injected intraperitoneally, and 10 minutes later, the number of rotations of the rats was recorded, and the recording time was 35 minutes.

Analysis of results: Induction of rotation in Parkinson's rats using apomorphine is a classic method to test for unilateral nigrostriatal lesions. The severity of dopamine neuron damage was measured by recording the number of rotations in the rats over a 35-minute period.

In FIG. 184, the number of rotations of rats in the M cell group was significantly lower than that in the solvent group (240.5 vs 360.5), indicating that in Parkinson's rats with nigrostriatal lesions, the M cells could reduce dopaminergic denervation and improve Parkinson's disease symptoms.

The cylinder test and the staining methods of brain nerve cell sections were referred to the published literature Kriks et al., Nature, 2011.

The results of the cylinder test showed that the wall contact frequencies of the bilateral forelimbs tended to be the same after the animals received transplantation, which was close to 50%, indicating that the cell transplantation could improve the stiffness of the limbs and enhance the motor ability in the Parkinson's animals (subjects).

The staining results of brain sections showed that compared with the control group, the number of dopaminergic neurons in striatum of the experimental groups increased, the length and complexity of neurons increased, and the number of gliacytes and microglia decreased, indicating that the M cell transplantation could protect neurons, reduce neuronal damage and death, and had effects of nourishing neurons and promoting synaptic regeneration, reducing inflammation in the brain, and improving the microenvironment.

Example 32: Evaluation of therapeutic activity of M cells against depression

Depression has now become a disease that poses a huge threat to people's health. The main clinical manifestations of depression are: (1) depressed mood, which mainly refers to persistent low mood, depression and pessimism; (2) slow thinking and slow reaction; (3) decreased volitional activity and slow behavior; (4) occurrence of cognitive impairment; (5) sleep disturbance and decreased appetite. The current mainstay of treatment for depression is a combination of medication and cognitive-behavioral therapy, but this treatment is not a good treatment for depression, and there are still big problems in terms of drug resistance and medication.

Scientists have also conducted a lot of researches on the causes of depression. At present, the most important is the inflammatory immune hypothesis, the main content is that the body's immune system can play a related role in depression. And according to a large number of studies, central inflammatory immunity is a key factor in depression.

Stem cells have immunomodulatory effects. So scientists hope to use stem cells to treat depression. Now, with the advent of stem cell treatment, scientists hope to develop new stem cell therapies for depression.

However, the clinical application of adult tissue-derived MSCs mainly has the following shortcomings: (1) the therapeutic amount of adult tissue-derived MSCs can hardly be obtained from a single individual tissue; (2) the adult tissue-derived MSCs are derived from different individual tissues, so that high consistency of product quality can hardly be achieved; (3) even the MSCs derived from the same individual tissue are highly heterogeneous; (4) the donor tissue sources of adult tissue-derived MSCs are complex and have potential infectious pathogen infection risks; (5) the rapid senescence of adult tissue-derived MSCs occurs with in vitro expansion. Therefore, new sources of MSC cells are needed for the treatment of depression.

Experimental animals:

CD-1 mice, male, 6 to 8 weeks old. The animals were purchased from Beijing Weitong Lihua Company.

All animals were kept at SPF grade in the Experimental Animal Center of the Institute of Zoology, Chinese Academy of Sciences, and were reared adaptively for one week. Care and Use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells:

The embryonic stem cells were suspended with EB spheres and subjected to adherent differentiation, and the M cells at P0 generation were obtained, passaged and screened, and cryopreserved at P3 generation for subsequent experiments.

The M cells at P3 generation were resuscitated, digested and passaged, and P5 generation was used for subsequent animal experiments.

| Reagent/Equi pment | Manufacturer | Cat. No. |
|---|---|---|
| Plexiglass tank | Agilent | 5982-9113 |
| Normal saline | SSY Group Limited | None |
| Brain solid positioner | Brain solid positioner | 51970 |
| Electronic scale | Yasuwang | CC-1013-04 |

Animal grouping:

| Group | Test drug | Animal number | Dosage | Administration volume | Administration times | Administration route |
|---|---|---|---|---|---|---|
| Forced Swimming | Normal saline | 3 | - | $5\mu L$ | once | Lateral intracerebroventricular injection |
| Forced swimming + M cells | M cells | 3 | $5\times10^5$ cells/ 5 $\mu$L/mouse | $5\mu L$ | Once | Lateral intracerebroventricular injection |

Lateral intracerebroventricular administration:

Cell transplantation was performed in the lateral ventricle using a brain solid positioner, with coordinates of: AP, -0.6 mm; ML, 1.2 mm; DV, -1.8 mm. Each animal in the test drug group was injected with 5 $\mu$L of $1\times10^5/\mu L$ M cell suspension, a total of $5\times10^5$ cells. The injection speed was 1 $\mu$L/min, the needle was kept in place for 8 min after injection, and the needle was slowly withdrawn for 2 min, and the wound was sutured. Before transplantation, the cells were kept in the syringe for a time as consistent as possible to avoid interindividual differences due to cell sedimentation.

The control group was given normal saline to the ventricle, and the administration process was the same as that of the test drug group.

Forced swimming test of mice:

One week after the lateral intracerebroventricular administration, the mice were subjected to a forced swimming test. The mice were placed in cylindrical transparent plexiglass tanks with a height of 28.5 cm and a diameter of 11 cm, one mouse per tank. The water depth in the tank was 15 cm, and the water temperature was (24 $\pm$1)°C. During the test, the mice swam in the tank for 5 minutes, and the accumulated immobility time of the mice within 4 minutes was recorded. The immobility time meant that the mice stopped struggling and appeared floating in the water.

Statistical analysis:

One-way ANOVA and T-TEST in Prism 7.0 statistical analysis software were used for variance analysis and significance test, and the experimental data were expressed as mean $\pm$ standard error (Mean $\pm$ SE). *, $p<0.05$; **, $p<0.01$; ***, $p<0.001$.

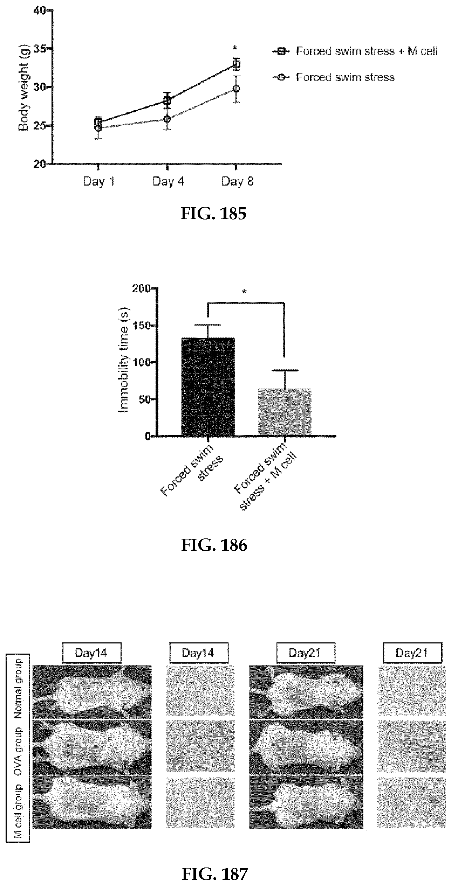

The purpose of this test was to evaluate the effect of the M cells on the immobility time or behavioral despair of male CD-1 mice in the forced swimming test. In this test, forced swimming was used to induce the mouse depression model, and normal saline or the M cells were administered in advance, and the immobility time of each group of mice in the forced swimming test was observed and recorded 7 days after a single administration. The experimental results showed that compared with the normal saline group, the weight of the mice in the M cell injection group increased faster (Table 32-1, FIG. 185), and the immobility time in the forced swimming test was significantly reduced (Table 32-2, FIG. 186). In conclusion, the M cells had a therapeutic effect on the depressive behavior of mice in the forced swimming test.

Table 32-1: Body weights of mice in each group.

|  |  | Forced swimming | | | Forced swimming+M cells | | |
|---|---|---|---|---|---|---|---|
| Body weight (g) | Day 1 | 23.1 | 25.7 | 25.3 | 25.8 | 24.9 | 25.5 |
|  | Day 4 | 25.4 | 27.4 | 24.8 | 27.3 | 29.3 | 28.2 |
|  | Day 8 | 28.1 | 31.6 | 29.6 | 32.8 | 33.8 | 32.3 |

Table 32-2: Immobility time of mice of each group in forced swimming test.

|  | Forced swimming | Forced swimming+M cells |
|---|---|---|
| Immobility time (seconds) | 145.79 | 88.52 |
|  | 139.63 | 64.21 |
|  | 110.69 | 36.24 |

Assessment of neurogenic potential of M cells

The mRNA expression of BDNF, FGF-2 and IGF-1 in the M cells was assessed using Real-time PCR so as to analyze the potential of these cells to support neurogenesis. A bioassay was constructed to assess the potential of M cell-secreted factors to support neurosphere development in rat neocortical cell cultures. The M cells were incubated with conditioned culture medium (serum-free high-glucose DMEM) for 24 h. The harvested conditioned culture medium was filtered with a 0.2 $\mu$m sterile filter supplemented with 1% B27 supplement, and used to culture rat neocortical cells ($10^4$/well) in 24-well plates. The incubation was performed at 37°C for 10 min in the presence of cortical cells obtained from freshly sacrificed naive Sprague-Dawley rats and 0.25% trypsin (Biological Industries) to obtain a suspension of rat neocortical cells. After 5 days of incubation, the number of neurospheres formed in the culture of rat neocortical cells grown in M cell-conditioned culture medium was determined, and counted under a microscope. The immunostaining using Nestin, neuroglial fibrillary acidic protein (GFAP) and doublecortin was performed to further analyze whether the neurosphere cells had differentiated neurons and glial cells.

Experimental results

The experimental results showed that the expanded undifferentiated M cells expressed different mRNA levels of a variety of neurotrophic factors (including IGF-1, BDNF and FGF-2), and the factors secreted by the M cells had a promoting effect on the paracrine of neurogenesis, the M cell-conditioned culture medium composed of DMEM could support the growth and development of neurospheres derived from neural progenitor cells in neonatal rat cortical cell cultures.

Dominant-submissive relationship (DSR) paradigm

The DSR paradigm used a single device, the device had two chambers connected by a tunnel, and the tunnel had a lactose feeder at its midpoint. Thirty FSL rats were randomly paired. In each pair, the animals were placed in opposing chambers of the DSR device and allowed to compete for milk for 5 minutes after 30 seconds of acclimatization. The test was repeated daily for 10 days prior to the M cell transplantation. The milking time of each animal was measured in each test. In each pair, the M cells were injected into the lateral ventricle of animals with a shorter milking time, while the animals on the other side were injected with vehicle. On day 10 after surgery, the same animal pair was tested again in the DSR paradigm and the test was continued for 7 days.

Experimental results

In the DSR paradigm, the FSL rat pair failed to show a significant dominant-submission relationship. In each pair, the animals with lower scores were injected with $10^5$ M cells, while their paired animals were injected with vehicle. On day 17 after the injection, a significant relationship was established, favoring M cell-injected animals.

Tissue sampling and immunofluorescence test

The FSL rats were anesthetized and perfused intracardially with 10U/mL heparin, followed by the addition of PBS (pH 7.4), and finally added with 4% paraformaldehyde (Sigma) in 0.1 M phosphate buffer (pH 7.4). The brains were taken out, fixed overnight, and balanced in phosphate-buffered 30% sucrose. The free-floating coronal hippocampal sections with a thickness of 20 to 40 $\mu$m were prepared in a cryostat and stored in 0.1% sodium azide (Sigma) at 4°C prior to immunofluorescence test. The frozen tissue sections and cultured cells were washed with PBS, incubated in 0.1% Triton X-100 (Sigma) for 5 min, and then blocked with blocking solution (0.1% Triton X-100 and 5% bovine serum albumin in PBS) for 45 minutes. The samples were then incubated with the following primary antibodies for 1 h at room temperature: rabbit polyclonal anti-BDNF (6.6 ng/mL), mouse monoclonal anti-PCNA (1.4 $\mu$g/mL), mouse monoclonal anti-Nestin (56 $\mu$g/mL), rabbit polyclonal anti-DCX (1 $\mu$g/mL) and rabbit polyclonal anti-GFAP (1:100 dilution), followed by incubation with the appropriate secondary antibodies (fluorescein isothiocyanate goat anti-rabbit and goat anti-mouse) at a 1:100 dilution for 1 h at room temperature. Between incubations, the samples were washed three times with PBS. The assay results of the samples were visualized using a fluorescence microscope (TE2000-U, Nikon, Tokyo, Japan).

Experimental results

The immunostaining results showed that there were more PCNA-positive nuclei in the ipsilateral hippocampus (radial layer) as compared with the contralateral hippocampus or animals injected with controls. Although no PCNA-positive nuclei were observed in the dentate gyrus 2 weeks after the treatment, an increase in DCX-expressing cells was observed in the granulosa cell layer of the ipsilateral dentate gyrus as compared with the contralateral dentate gyrus and the control animals. Similarly, an increase in BDNF-expressing cells was observed in the subgranular region of the dentate gyrus, and an increase in GFAP-expressing cells was observed in the dentate gyrus. It was important to note that while some engrafted M cells were also found to express the neural markers DCX and GFAP, the majority of engrafted Dil-labeled cells had not such expression.

Related documents:

1. Adipose-derived mesenchymal stem cells protect against CMS-induced depression-like behaviors in mice via regulating the Nrf2/HO-1 and TLR4/NF-κB signaling pathways.

Example 33: Evaluation of therapeutic activity of M cells against atopic dermatitis

Atopic dermatitis (AD) is a chronic, recurrent, pruritic and inflammatory skin disease. AD has become an important public health problem, with a prevalence of up to 20% in children and 3 to 10% in adults. The pathogenesis of AD is complex, involving many factors such as genetics, immunity and environment factors, among which the abnormal immune function, especially the immune response effect of immune cells, plays an important role in the pathogenesis of AD.

At present, the treatment of AD usually involves the local and/or systemic use of glucocorticoids and immunosuppressive agent, but the topical use of glucocorticoids is limited in moderate-severe AD patients, and the systemic use of immune preparations has myelosuppression and increased infection opportunity and other risks; new biologics such as anti-interleukin (IL)-4R monoclonal antibody Dupilum-ab and anti-immunoglobulin IgE monoclonal antibody Omalizumab are limited in research results, and there are significant differences. So, it is necessary to develop new and safe and effective methods for the treatment of AD. The present invention treats dermatitis by subcutaneously transplanting M cells.

Documents:
Human adipose tissue-derived mesenchymal stem cells alleviate atopic dermatitis via regulation of B lymphocyte maturation.

Enhanced therapeutic effects of human mesenchymal stem cells transduced with superoxide dismutase 3 in a murine atopic dermatitis to like skin inflammation model.

Priming with Toll-like receptor 3 agonist or interferon to gamma enhances the therapeutic effects of human mesenchymal stem cells in a murine model of atopic dermatitis.

Experimental animals: BALB/c mice, female, male, 7 to 8 weeks old, purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equi pment | Manufacturer | Cat. No. |
|---|---|---|
| R540 Enhanced small animal anesthesia machine | Ruiwode | R540 |
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Isoflurane | Ruiwode | 970-00026-00 |
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solarbio | G8590-100 |
| Ovalbumin (OVA) | Sigma | S25067-25g |
| Aluminum hydroxide | Sigma | 239186-500G |

Animal model: BALB/c mice were weighed and randomly grouped according to their body weight. After BALB/c mice were anesthetized with a gas anesthesia machine, the back hair was shaved, and the mice were grouped, with 6 mice in each group.

Grouping:

Normal group: only shaved, no other treatment was carried out.

OVA group: OVA+aluminum hydroxide was injected at 3 points on the back, 50 μl of normal saline per point.

M cell group: OVA+aluminum hydroxide was injected at 3 points on the back, 50 μl of normal saline per point, containing $1 \times 10^6$ M cells (P5 generation).

The above treatment was recorded as day 0, OVA+aluminum hydroxide was injected on the day 3 and day 7 respectively, and only 100 μg of OVA was injected every day from day 7 to day 14. On the days 14 and 17, normal saline or the M cells were injected, respectively.

Clinical manifestations and severity scores: On the day 14 and day 17, photos were taken to record the skin lesion severity scores and clinical manifestations. The results were shown in FIG. 187.

It was found that the subcutaneous injection of M cells could reduce the degree of skin epidermal hyperplasia, reduce allergic inflammatory response, promote hair follicle regeneration, relieve the symptoms of dermatitis mice, and play an effective therapeutic role in dermatitis.

(2) Behavior study:

The frequency of touching and scratching the affected area of dermatitis was observed in the mice.

Experimental results: It was found that the frequency of scratching in the M cell group was significantly lower than that in the model group, indicating that the degree of itching in mice was reduced in the treatment group.

(3) Histopathological analysis:

1) Specimen collection:

When collecting specimens, the mice were in supine position after intraperitoneal anesthesia, the skin was cut in the middle of the abdomen of the mice, the chest was opened, the heart was exposed, and the heart was perfused with ice-cold normal saline. About 20 ml of normal saline was needed for each mouse. After the normal saline perfusion was completed, the fixation was performed with 20 ml of paraformaldehyde. After the perfusion was completed, the skin in the modeling area was cut off, fixed, sectioned and analyzed.

2) Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

3) Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

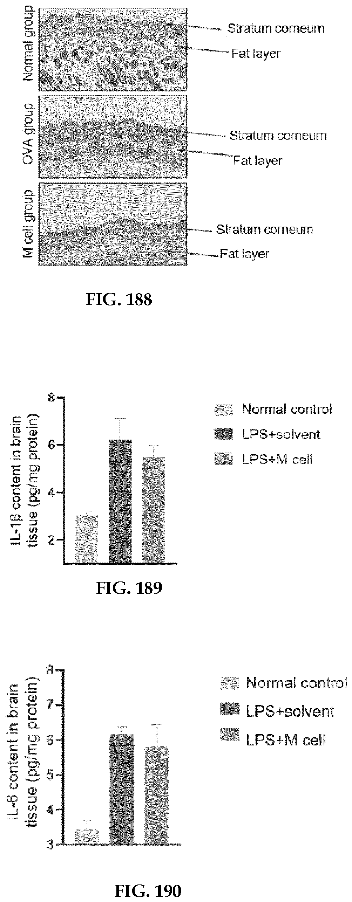

FIG. 188 showed the HE staining pictures of dermatitis model mice. Compared with the OVA group, the stratum corneum of the mice after the M cell treatment was thinner and the fat layer was thickened, indicating that the M cell treatment could improve the microenvironment of the mouse skin and inhibit inflammation; the number of skin appendages was higher than that of the OVA group, indicating that the M cells could protect the skin appendages and had a good therapeutic effect on atopic dermatitis.

4) Toluidine blue (TB) staining

1. The tissue sections were subjected to routine dewaxing and dehydration.

2. Added to toluidine blue solution for 30min.

3. Slightly rinsed with water.

4. Added to 0.5% glacial acetic acid solution for differentiation, until the nuclei and particles were clear.

5. Slightly rinsed with water and dried with cold air.

6. Transparentized with xylene, and mounted with neutral resin.

Experimental results: The M cells could reduce mast cell proliferation.

5) Detection of Th1 and Th2 cells in spleen by flow cytometry

(5) The mouse spleen was taken out, the tissue was ground with a grinder, and the grinding fluid was transferred into an EP tube, centrifuged with a centrifuge at 500G for 5min, the supernatant was discarded, then 5ml of red blood cell lysis solution was added, incubated at 37°C for 15min, centrifuged again, the supernatant was discarded, the cell concentration was adjusted to $1 \times 10^6$, the cells was pipetted into a centrifuge tube, centrifuged at 400G for 5min, the supernatant was discarded, CD4 antibody was added to each tube, vortexed, and incubated in the dark for 30 min.

(6) Washing was carried out twice with 1ml staining buffer, IL-4 and IFN-γ isophil antibodies were added to the first tube, and 2ul of IL-4 and IFN-γ antibodies were added to each of the other tubes; after vortexing, incubation was performed at 4°C for 30min.

(7) After washing twice with fixation and permeabilization solution, the cells were resuspended by adding 500ul of PBS, loaded to machine for analysis. The CD4$^+$ T cell gate was determined according to CD4 fluorescence, 10,000 CD4$^+$ T cells in each specimen was counted, and the percentages of Th1 (CD4$^+$IFN-γ$^+$) and Th2 (CD4$^+$IL-4$^+$) cells were calculated.

The experimental results showed that the Th1/Th2 cells in the dermatitis model group were significantly reduced, and the M cell treatment could mediate the imbalance of Th1/Th2 cells and inhibit the inflammatory response.

5) Flow cytometry of B cells

(1) Blood was collected from the mouse heart, anticoagulated with heparin sodium, and the blood was added to a tube for flow cytometry, 100ul per tube, the BD red blood cell lysis solution was added to each tube, incubated for 15 minutes, and washed twice with PBS.

(2) PerCP-CD19, PE-CD27 and FITC-38 antibodies were added to each tube and incubated for 30 min.

(3) In the intracellular marker staining, the BD intracellular staining buffer was used for fixation and permeation.

(4) FCR blocking was performed.

(5) FITC-IgE antibody incubation was performed.

(6) Detection was carried out by load to a flow cytometer.

Experimental results: It was shown that the M cells could reduce the IgE expression intensity of CD19-positive cells and improve allergic diseases.

In conclusion, the M cell treatment could reduce the degree of pruritus in mice, reduce mast cell proliferation, mediate Th1/Th2 cell imbalance, inhibit inflammatory response, reduce the intensity of IgE expression in CD19-positive cells, and improve allergic diseases. Therefore, the M cells could well treat dermatitis.

Example 34: Evaluation of therapeutic activity of M cells against neuroinflammation

Neuroinflammation is involved in traumatic brain injury, stroke, cerebral hemorrhage and various neurodegenerative diseases. Under normal conditions, neuroinflammation maintains homeostasis and promotes tissue repair. However, uncontrolled neuroinflammation can be harmful to the brain. Therefore, controlling deleterious inflammatory responses is a promising therapeutic approach for nervous system diseases.

Documents:

Mesenchymal stem cells enhance microglia M2 polarization and attenuate neuroinflammation through TSG-6.

Experimental animals: C57bl/6 mice, 5 to 7 weeks old, purchased from Weitong Lihua Laboratory Animal Technology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%. Experiments were started after one week of adaptive feeding of mice.

Experimental groups: normal control group, LPS+solvent (solvent group), LPS+M cells (M cell group), with 3 mice in each group.

Experimental materials: surgical instruments, 20ml syringe, 5ml disposable sterile syringe, 20ml disposable sterile syringe, electronic scale.

Experimental reagents: normal saline, lipopolysaccharides (LPS), RIPA lysis solution, mouse interleukin 1β ELISA Kit (Mouse Interleukin 1β, IL-1β ELISA Kit), mouse interleukin 6 ELISA Kit (Mouse Interleukin 6, IL-6 ELISA KIT), mouse interleukin 10 ELISA kit (Mouse interleukin 10, IL-10 ELISA KIT).

Equipment: tissue homogenizer

| Consumable/Reagent/Instrument | Manufacturer | Cat. No./Model |
|---|---|---|
| Electronic scale | Yasuwang | CC-1013-04 |

(continued)

| Consumable/Reagent/Instrument | Manufacturer | Cat. No./Model |
|---|---|---|
| Disposable sterile syringe 20ml | Jiangsu Zhiyu Medical Equipment Co., Ltd. | |
| Disposable sterile syringe 5ml | Jiangsu Zhiyu Medical Equipment Co., Ltd. | |
| Normal saline | domestic | |
| Lipopolysaccharides | Kangwei | CW2333S |
| RIPA lysis solution | Sigma | A8960 |
| Tissue homogenizer | IKA | 201110158 |
| Mouse Interleukin 1β ELISA Kit | CUSABIO | CSB-E08054m |
| Mouse Interleukin 6 ELISA Kit | CUSABIO | CSB-E04639m |
| Mouse Interleukin 10 ELISA Kit | CUSABIO | CSB-E04594m |

Experimental procedure: The mice were fasted for 16 hours before LPS injection, and then intraperitoneally injected with LPS (0.05 mg/kg). The brain tissue was harvested 24 hours later. Immediately after the mice were euthanized, the cardiac perfusion was performed: the thoracic cavity was opened to expose the heart, a 20ml syringe was used to draw 20ml of normal saline, the syringe was changed with a 5ml syringe, inserted from the apex of heart, the right auricula dextra was cut, and 20ml of normal saline was quickly injected, and then the mouse brain tissue was taken out, added with RIPA lysis solution at a ratio of 1:3, then homogenized with a tissue homogenizer, placed on ice for 5 minutes, centrifuged at 5000g, 4°C for 10 minutes, then the supernatant was taken for ELISA detection. The ELISA was carried out according to the instructions.

Cell injection: It was performed simultaneous with the injection of LPS. The LPS+solvent group was subjected to the injection of 100 µl of normal saline through the tail vein, and the LPS+M cell group was subjected to the injection of 100 µl of M cells through the tail vein, $3 \times 10^6$/cell.

Statistics: All data were analyzed by One-way ANOVA in Prism 7.0 statistical analysis software for analysis of variance and significance test, and experimental data were expressed as mean $\pm$ standard deviation (Mean $\pm$ SD). *, $p < 0.05$; **, $p < 0.01$; ***, $p < 0.001$.

Analysis of results: In the brain tissue of mice with LPS-induced neuroinflammation, compared with the solvent group, the M cell group had a tendency to reduce the content of proinflammatory factors IL-1β and IL-6. Moreover, the M cells significantly increased the expression of the anti-inflammatory factor IL-10. Therefore, the M cells could reduce inflammation.

Table 34-1: Content of IL-1β (pg/ml) in brain tissue of mice with LPS-induced neuroinflammation

| Group | Normal control | | | LPS+solvent | | | LPS+M cells | | |
|---|---|---|---|---|---|---|---|---|---|
| IL-1β (pg/mg) | 3.3 | 2.7 | 3.2 | 5.1 | 8.0 | 5.5 | 5.7 | 6.3 | 4.5 |

Table 34-1 and FIG. 189 showed the statistical results of IL-1β content (pg/ml) in the brain tissue of mice with LPS-induced neuroinflammation. IL-1β promoted inflammation and was a proinflammatory factor. The content of IL-1β in the brain tissue of the solvent group was significantly higher than that of the normal group. Compared with the solvent group, the M cell group had a tendency to decrease the content of IL-1β.

Table 34-2: Content of IL-6 (pg/mg) in brain tissue of mice with LPS-induced neuroinflammation

| Group | Normal control | | | LPS+solvent | | | LPS+M cells | | |
|---|---|---|---|---|---|---|---|---|---|
| IL-6 (pg/mg) | 4.0 | 3.3 | 3.1 | 6.5 | 5.7 | 6.3 | 6.7 | 6.2 | 4.6 |

Table 34-2 and FIG. 190 showed the statistical results of IL-6 content in the brain tissue of mice with LPS-induced neuroinflammation. Compared with the normal group, the content of IL-6 in the brain tissue of the solvent group was significantly increased. IL-6 promoted inflammation and was a proinflammatory factor. Compared with the solvent group, the M cell group had a tendency to reduce the content of IL-6.

Table 34-3: Content of IL-10 (pg/mg) in brain tissue of mice with LPS-induced neuroinflammation

| Group | Normal control | | | LPS+solvent | | | LPS+M cells | | |
|---|---|---|---|---|---|---|---|---|---|
| IL-6 (pg/mg) | 2.3 | 2.0 | 1.8 | 2.1 | 2.1 | 1.8 | 2.7 | 2.7 | 2.8 |

Table 34-3 and FIG. 191 showed the statistical results of IL-10 (pg/mg) in the brain tissue of mice with LPS-induced neuroinflammation. IL-10 inhibited the occurrence of inflammation and was an anti-inflammatory factor. The concentration of IL-10 in the brain tissue of the M cell group was significantly higher than that of the solvent group (2.0 vs 2.7), indicating that the M cells had an anti-inflammatory effect.

Staining of frozen mouse brain sections and statistical results

The method was referred to the published literature Kriks et al., Nature, 2011.
The staining of frozen brain sections was used to detect the regeneration of nerve cells in mice. It was found that, compared with the control group, the number of reactive astrocytes (GFAP+) and microglia (IBA1+ CD11B+) in the brain and peripheral tissues of the animals in the transplanted M cell group was significantly reduced. It showed that the M cell transplantation could promote neuron regeneration, reduce neuron damage and death, reduce neuroinflammation, provide nutrition to neurons and promote synapse regeneration.

ELISA and WB detection results of inflammatory factors in brain tissue

The methods were referred to the published literature Bétemps et al., 2015, J Vis Exp.
The mouse brain tissues were taken to detect the levels of inflammatory factors. Compared with the control group, it was found that the levels of TFN-$\alpha$, IL1-$\beta$, IL-6 and other proinflammatory factors in the brain tissue of the transplanted M cell group were significantly decreased, while the levels of anti-inflammatory factors such as IL-10 and IL-3 were significantly increased. It showed that the M cells could attenuate the inflammatory response and improve the microenvironment of the nervous system.

Memory testing:

The method was referred to the published literature Lykhmus et al., 2019, Frontiers in Pharmacology.
The results showed that the M cell transplantation significantly improved the scene memory ability in the model animals (subjects).
Compared with the normal control group, the concentrations of proinflammatory factors IL-1$\beta$ and IL-6 in the solvent group were significantly increased, and the anti-inflammatory factor IL-10 was significantly decreased, indicating that the mice in the solvent group had more severe inflammation.

Example 35: Evaluation of therapeutic activity of M cells against meniscal injury

The meniscuses are two half-moon-shaped fibrocartilages located on the medial and lateral articular surfaces of the tibial plateau, have the functions of stabilizing the knee joint, transmitting and dispersing the load force of knee joint, and promoting intra-articular nutrition, and are important organs to maintain stability and motor function of knee joint. Meniscal injuries are mostly caused by torsional external forces, resulting in joint pain, limited joint movement, and leading to muscle atrophy and inconvenience in walking, which seriously affects the lives of patients. Meniscus injuries are one of the most common sports injuries to the knee joint.
From outside to inside, the meniscus is divided into: the outer 10 to 20% is the red area, which is supplied with blood by the medial and lateral knee arteries, forming the arterial plexus around the meniscus, the inner 30% or so is the white area without blood supply, and the middle 50 to 60% or so is the transitional red-white area. At present, the clinical repair methods for meniscal injury mainly include: suture, excision and meniscal prosthesis transplantation. The suture is limited to the red area with blood supply and some simple injuries in the red-white area, and these areas can heal on their own after suturing, but such cases are rare in clinical practice, accounting for about 20 to 30% of the total meniscus cases. However, due to the structural characteristics and stress characteristics of the meniscus, most injuries occur in the white area, and such injuries cannot heal on their own due to the lack of blood supply, so that meniscectomy is required, in which the principle of operation is to preserve as many meniscus as possible, so partial resection is common, and subtotal resection is performed in some more severe cases, that is, the outermost layer of the red area of the meniscus is retained, and total meniscectomy is performed only in rare cases. Partial or total meniscectomy can relieve symptoms and pain obviously, but due to the importance of meniscal function, long-term follow-up results after meniscectomy show that articular cartilage degenerates, and even severe osteoarthritis occurs. Therefore, some patients after meniscectomy require meniscal prosthesis transplantation to protect articular cartilage, maintain joint stability, and restore motor function.

Non-patent documents:

1. Meniscus repair using mesenchymal stem cells-a comprehensive review.

2. Mesenchymal stem cells in human meniscal regeneration: A systematic review

3. Role of mesenchymal stem cells in meniscal repair

Patent documents:

CN103920188B: Tissue engineering meniscus repair sheet and preparation method thereof

CN104398698A: Traditional Chinese medicine composition for treating meniscus injury

Objective: To achieve the treatment of meniscus injury by transplantation of M cells.

Achieved effect: After transplantation of M cells, the meniscus injury was completely recovered.

Preparation and culture of M cells: The embryonic stem cells were suspended to form EB spheres and subjected to adherent differentiation, and the M cells at P0 generation were obtained, passaged and screened, and cryopreserved at P3 generation for subsequent experiments.

The M cells at P3 generation were resuscitated, digested and passaged, and used at P5 generation for subsequent experiments.

The M cells at P5 generation were cryopreserved with the clinical preparation prepared by the national stem cell resource bank, and stored in a liquid nitrogen tank, ready for later clinical use.

| Reagent/Equipment | Manufacturer | Cat. No. |
|---|---|---|
| Liquid nitrogen cabinet | Thermo | 7403TF |
| Biological safety cabinet | Thermo | 1389 A2 |

Treatment method: Patients with meniscus injury were treated with intra-articular injection of the M cell preparation, and were divided into a low-dose group ($1\times10^7$/knee joint) and a middle-dose group ($5\times10^7$/knee joint). After injection of the M cell preparation, safety and efficacy (knee joint pain, local edema) were evaluated, and imaging observations were performed.

Pain visual analog scale (VAS): scaled from 0 to 10.

0 points: no pain at all;

3 points or lower: slight pain, tolerable;

4 to 6 points: pain affecting sleep of patients, but still tolerable.

7 to 10 points: severe pain, which is unbearable, affects appetite and sleep of patients.

Lysholm score: It was proposed by Lysholm and Gillqui in 1982, and widely used in various knee joint diseases, such as meniscus injury, cartilage degeneration or softening. The total Lysholm score is 100 points. If the self-assessment score is lower than 70 points, it indicates that the functional state of the knee joint is already very poor. The content of scores include limp, locking, pain, support, instability, swelling, difficulty in climbing stairs, and restricted squatting. The Lysholm score can not only evaluate the functional perception of the most important daily activities of the subject, but also make a preliminary assessment of the motor function level of the subject at different intensities.

Table 35-1: Clinical observation of low-dose group and middle-dose group after receiving M cell preparation

| Group | Subject | Safety | | Efficacy | |
|---|---|---|---|---|---|
| | | Adverse reaction | Serious adverse reaction | Knee pain | Local edema/effusion |
| Low-dose group | 1 | None | None | Disappeared (2-0) | Mitigated |
| | 2 | None | None | Mitigated (3-1) | Mitigated |
| | 3 | None | None | No change (3-3) | Not changed |
| | 4 | None | None | Mitigated (6-4) | Mitigated |
| | 5 | None | None | Mitigated (3-1) | Mitigated |
| | 6 | None | None | Mitigated (8-2) | Mitigated |
| Middle-dose group | 7 | None | None | Slightly mitigated (2-2) | Mitigated |
| | 8 | None | None | Mitigated (2-1) | Mitigated |
| | 9 | None | None | Not obviously changed for the time being | Not changed for the time being |
| | 10 | None | None | Mitigated (3-1) | Not changed for the time being |

Description of Table 35-1: For the 6 subjects in the low-dose group, 3 months after stem cell transplantation, 4 of which had repaired meniscus to different degrees; for the middle-dose subjects, 1 month after stem cell transplantation, they were still in follow-up.

Table 35-2: VAS scores of subjects

| Item | Index | Total | Low-dose group | Middle-dose group |
|---|---|---|---|---|
| VAS pain score - before injection | N(Missing) | 10(0) | 6(0) | 4(0) |
| | Mean(SD) | 3.70(2.00) | 4.17(2.32) | 3.00(1.41) |
| | Median | 3.00 | 3.00 | 2.50 |
| | Q1,Q3 | 2.00,5.00 | 3.00,6.00 | 2.00,4.00 |
| | Min,Max | 2.00,8.00 | 2.00,8.00 | 2.00,5.00 |
| VAS pain score - 1 week after treatment | N(Missing) | 10(0) | 6(0) | 4(0) |
| | Mean(SD) | 2.60(1.43) | 3.00(1.79) | 2.00(0.00) |
| | Median | 2.00 | 2.50 | 2.00 |
| | Q1,Q3 | 2.00,3.00 | 2.00,4.00 | 2.00,2.00 |
| | Min,Max | 1.00,6.00 | 1.00,6.00 | 2.00,2.00 |
| VAS pain score - 1 month after treatment | N(Missing) | 8(2) | 6(0) | 2(2) |
| | Mean(SD) | 2.69(1.62) | 3.25(1.33) | 1.00(1.41) |
| | Median | 2.50 | 3.50 | 1.00 |
| | Q1,Q3 | 1.75,4.00 | 2.00,4.00 | 0.00,2.00 |
| | Min,Max | 0.00,5.00 | 1.50,5.00 | 0.00,2.00 |
| VAS pain score - 2 months | N(Missing) | 7(3) | 6(0) | 1(3) |

(continued)

| Item | Index | Total | Low-dose group | Middle-dose group |
|------|-------|-------|----------------|-------------------|
| after treatment | Mean(SD) | 1.93(1.54) | 2.25(1.41) | 0.00(.) |
| | Median | 2.00 | 2.50 | 0.00 |
| | Q1,Q3 | 0.00,3.00 | 1.50,3.00 | 0.00,0.00 |
| | Min,Max | 0.00,4.00 | 0.00,4.00 | 0.00,0.00 |
| VAS pain score - 3 months after treatment | N(Missing) | 4(6) | 4(2) | 0(4) |
| | Mean(SD) | 2.63(2.56) | 2.63(2.56) | .(.) |
| | Median | 2.25 | 2.25 | |
| | Q1,Q3 | 0.75,4.50 | 0.75,4.50 | .,. |
| | Min,Max | 0.00,6.00 | 0.00,6.00 | .,. |

Combining Table 35-2 and FIG. 192, it could be seen that although the VAS scores of the two dose groups fluctuated up and down, they showed a downward trend as a whole, that was, the subjects' knee pain was relieved to a certain extent after receiving the injection of test drug.

Table 35-3: Lysholm scores (total) of subjects

| Item | Index | Total | Low-dose group | Middle-dose group |
|------|-------|-------|----------------|-------------------|
| Lysholm total score - before injection | N (Missing) | 10(0) | 6(0) | 4(0) |
| | Mean (SD) | 61.00 (16.33) | 52.50(11.54) | 73.75(14.73) |
| | Median | 58.00 | 56.00 | 77.00 |
| | Q1,Q3 | 55.00,69.00 | 49.00,60.00 | 62.00,85.50 |
| | Min,Max | 31.00,86.00 | 31.00,63.00 | 55.00,86.00 |
| Lysholm total score - 1 week after treatment | N (Missing) | 10(0) | 6(0) | 4(0) |
| | Mean (SD) | 61.10 (21.48) | 51.00(18.18) | 76.25(17.99) |
| | Median | 58.00 | 50.00 | 77.00 |
| | Q1,Q3 | 44.00,80.00 | 35.00,60.00 | 62.00,90.50 |
| | Min,Max | 31.00,96.00 | 31.00,80.00 | 55.00,96.00 |
| Lysholm total score - 1 month after treatment | N (Missing) | 8(2) | 6(0) | 2(2) |
| | Mean (SD) | 59.38 (21.73) | 51.83(17.90) | 82.00(18.38) |
| | Median | 58.00 | 52.50 | 82.00 |
| | Q1,Q3 | 42.00,74.50 | 35.00,60.00 | 69.00,95.00 |
| | Min,Max | 31.00,95.00 | 31.00,80.00 | 69.00,95.00 |
| Lysholm total score - 2 months after treatment | N (Missing) | 6(4) | 6(0) | 0(4) |
| | Mean (SD) | 56.33 (19.46) | 56.33(19.46) | .(.) |

(continued)

| Item | Index | Total | Low-dose group | Middle-dose group |
|---|---|---|---|---|
| | Median | 52.50 | 52.50 | |
| | Q1,Q3 | 40.00,79.00 | 40.00,79.00 | .,. |
| | Min,Max | 34.00,80.00 | 34.00,80.00 | .,. |
| Lysholm total score - 3 months after treatment | N (Missing) | 4(6) | 4(2) | 0(4) |
| | Mean (SD) | 62.00 (20.54) | 62.00(20.54) | .(.) |
| | Median | 64.00 | 64.00 | |
| | Q1,Q3 | 44.50,79.50 | 44.50,79.50 | .,. |
| | Min,Max | 40.00,80.00 | 40.00,80.00 | .,. |

Note: It could be seen from Table 35-3 and FIG. 193 that after the subjects in the two dose groups received the test drug injection, the Lysholm total score showed an upward trend. According to the results of single assessments, the increase in the score values after injection of the test drug was mainly reflected in terms of limp, locking and pain, while there was no significant change in the scores for other single assessments. Therefore, after the subjects received the injection of the test drug, there was some improvements in the Lysholm scores, mainly in relief of limp, locking and pain.

Before the M cell treatment, there was meniscus injury and severe knee joint pain. After 3 months of the intra-articular transplantation of the M cell preparation, the meniscus injury completely recovered, and the knee joint pain score changed from 8 points to 2 points, the local edema was alleviated, indicating that the intra-articular transplantation of the M cells could effectively treat the meniscus injury. After the transplantation of M cells, the meniscus injury had a good repair effect, the knee joint pain score was reduced, and the local edema was reduced.

Example 36: Evaluation of therapeutic activity of M cells against non-alcoholic steatohepatitis

Non-alcoholic steatohepatitis (NASH), also known as metabolic steatohepatitis, is a progressive form of non-alcoholic fatty liver disease, defined as the presence of 5% or more hepatocytes with hepatic steatosis-accompanied inflammation and hepatocyte damage (e.g., ballooning change), with or without fibrosis. NASH easily develops into liver cirrhosis, liver cancer and other diseases. There are 3% to 5% of NASH patients worldwide. There are about 1.09 million patients with liver cirrhosis in China, and in 2030, it will increase to 2.32 million. The development of NASH is closely related to heredity (polymorphism of PNPLA3), living habits (eating habits, number of meals, sleep-wake cycle, etc. of hosts), obesity, metabolic syndrome, etc. Common symptoms of NASH include anorexia, fatigue, abdominal distension, nausea and vomiting, dull pain in liver area, and hepatomegaly. Environmental, metabolic, and genetic factors lead to the accumulation of free fatty acids in the liver, which in turn causes a series of cell damage. Currently, there are non-clinical and clinical treatments for NASH treatment. The former includes lifestyle changes to improve the course of the disease, while the latter includes liver transplant, surgery and drugs under investigation to treat the disease. A therapeutic strategy for developing a healthy lifestyle is more suitable for adjuvant therapy. Liver transplantation is expensive and donors are scarce; surgical treatment requires patients to meet eligibility criteria and has limitations; there are currently no FDA-approved drugs for NASH. Therefore, the treatment of NASH is still in a state of urgent shortage.

The problem: NASH lacks effective treatments.

Experimental animals: C57bl/6 mice, 7 to 9 weeks old, purchased from Sibeifu (Beijing) Biotechnology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for the animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%. Experiments were started after one week of adaptive feeding of mice.

Reagents and equipment:

| Reagent/Equipment | Manufacturer | Cat. No. |
|---|---|---|
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Water bath | Sail Huachuang | SDY-1 |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solarbio | G8590-100 |
| Corn Oil | Aladdin | C116025 |
| Blood chemistry analyzer | Beckman | AU5800 |
| Centrifuge | Xiangyi | TD25-WS |
| Electronic Scale | Yasuwang | CC-1013-04 |
| Methionine choline deficiency feed (MCD feed) | Guangzhou Yike Biotechnology Co., Ltd. | RBG-9N |
| Heparin sodium | Maikelin | H810907-1g |
| Multifactor suspension chip system | Bio-Rad | Bio-Plex® 200 |
| 23-Factors Kit | Bio-Rad | M60009RDPD |
| Collagen I antibody | Miltenyi Biotec | GB13091 |
| $\alpha$-SMA antibody | Servicebio | GB13044 |
| Immunohistochemistry kit | Fuzhou Maixin | KIT-9710 |

Experimental groups:

Normal feed+solvent group: normal feed was fed, at the 2nd and 4th week of feeding, 100 $\mu$L of normal saline was injected into the tail vein;

MCD feed+solvent group: MCD feed was fed, at the 2nd and 4th week of feeding, 100 $\mu$L of normal saline was injected into the tail vein;

MCD feed+M cell group: MCD feed was fed, at the 2nd and 4th week of feeding, $3\times10^6$ M cells per mouse were injected into the tail vein;

Experimental materials:

After 6 weeks of feeding with MCD, sampling was performed. The mice were placed in a supine position after intraperitoneal anesthesia, the skin was cut in the middle of the abdomen of the mice, the abdominal cavity was opened, and blood was collected from the central vein. The chest was opened, the heart was exposed, and the heart was perfused with ice-cold normal saline. After the completion of the normal saline perfusion, the fixation was performed with 50 mL of paraformaldehyde. After the perfusion was completed, the liver was taken, fixed, sectioned and analyzed. The collected blood was centrifuged at 5,000 rpm for 15 min at room temperature, and the supernatant was taken for blood biochemical analysis.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 23-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex ManagerTM.

(4) To the standard bottle, 250 $\mu$L of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1$\times$ with Bio-Plex detection buffer, and stored in the dark.

(7) The diluted magnetic beads were vortexed, and 50 $\mu$L of the magnetic beads was added to each well.

(8) The plate was washed twice with 100 $\mu$L of washing solution.

(9) The sample, standard, blank, and control of known concentration were vortexed, and added in an amount of 50 $\mu$L to each well.

(10) The plate was sealed with a sealing film, and shaken on a high-frequency shaker at 850$\pm$50 rpm at room temperature for 30 min.

(11) In step (10), when 10 min of shaking time was left, the detection antibody was vortexed for 5 s and diluted to 1$\times$.

(12) The plate was washed twice with 100 $\mu$L of washing solution.

(13) The diluted antibody was vortexed, and added in an amount of 250 $\mu$L to each well.

(14) The plate was sealed with a sealing film, and shaken on a high-frequency shaker at 850$\pm$50 rpm at room temperature for 30 min.

(15) The arrangement information of the standard (provided in the kit), plate and sample were input.

(16) In step (14), when 10 min of shaking time was left, SA-PE 5 was vortexed and diluted to 1$\times$.

(17) The plate was washed twice with 100 $\mu$L of washing solution.

(18) The diluted SA-PE was vortexed, and added in an amount of 50 $\mu$L to each well.

(19) The plate was sealed with a sealing film, and shaken on a high-frequency shaker at 850$\pm$50 rpm at room temperature for 30 min.

(20) The plate was washed three times with 100 $\mu$L of washing solution.

(21) The magnetic beads were resuspended with 125 $\mu$L of detection solution, the plate was sealed with a sealing film, and shaken on a high-frequency shaker at room temperature at 850$\pm$50 rpm for 30 s.

(22) After the sealing film was discarded, loading to machine was started.

Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Immunohistochemical staining:

Immunohistochemical staining was performed on the paraffin sections using an immunohistochemical kit (Fuzhou Maixin, KIT-9710). The specific steps were as follows:

1. Dewaxing: (1) xylene I, II, 10 min each; (2) gradient alcohol: 100% absolute ethanol, 2 min; 95% absolute ethanol, 2 min; 80% absolute ethanol, 2 min; 70% absolute ethanol, 2 min;

2. Hydration: washing was performed twice with distilled water, 5min each time (placed on a shaker);

3. After deparaffinization and hydration of paraffin sections, rinsing was performed 3 times with PBS, 3 minutes each time;

4. Preparation of antigen retrieval solution (10 mM pH 6.0 sodium citrate buffer):

(1) Preparation of stock solution: Solution A: 29.41 g of trisodium citrate dihydrate + 1,000 mL of distilled water; Solution B: 21 g of citric acid + 1,000 mL of distilled water;

(2) Preparation of working solution: 82 mL of Solution A + 18 mL of solution B + 900 mL of distilled water;

5. Antigen retrieval: the sections were placed in a plastic or heat-resistant glass container filled with sodium citrate buffer, the sections were immersed, treated with a microwave oven at mid-range or high-range power for 5 minutes; sodium citrate buffer was replenished, and treatment was performed again at mid-range or high-range power for 5 minutes;

6. Reagent A (peroxidase blocking solution) was added, and incubated at room temperature for 10 min to block the activity of endogenous peroxidase; rinsing was performed with PBS 3 times, 3 min each time;

7. PBS was discarded, 1 drop or 50 μL of Reagent B (normal non-immune animal serum) was added, and incubated at room temperature for 10 min;

8. The serum was discarded, 1 drop or 50 μL of primary antibody was added, and incubated at 4°C overnight or at room temperature for 60 min; rinsing was performed with PBS 3 times, 3 min each time;

9. The PBS was discarded, 1 drop or 50 μL of biotin-labeled secondary antibody (Reagent C) was added, and incubated at room temperature for 10 min; rinsing was performed with PBS 3 times, 3 min each time;

10. The PBS was discarded, 1 drop or 50 μL of streptavidin-peroxidase solution (reagent D) was added, incubated at room temperature for 10 min; rinsing was performed with PBS 3 times, 3 min each time;

11. The PBS was discarded, 2 drops or 100 μL of freshly prepared DAB solution was added, and observation was performed under microscope for 3 to 10 min;

12. Rinsing was performed with tap water, counterstaining was carried out with hematoxylin, and rinsing was performed with PBS or tap water so as to return to blue;

13. When using DAB for color development, the sections should be dehydrated with gradient alcohol and dried, transparentizing was performed with xylene, and mounting was performed with neutral resin;

14. Photos were taken with a microscope.

Oil Red O staining:

1. Preparation of Oil Red O: 0.5g of oil red dry powder that had been ground and pulverized in advance was weighed, dissolved in 10 mL of isopropanol, then added with isopropanol to reach 100 mL, sealed and stored at 4°C in a brown bottle (or wrapped with tin foil to protect from light), and it was a storage solution and could be stored for a long time. When using, 6 mL of the oil red solution was taken, added with 4 mL of triple-distilled water and mixed well, filtered with qualitative filter paper, and used up within 3 hours after dilution;

2. The tissue was frozen and sectioned, rinsed with PBS, and fixed with 4% PFA for 20 min at room temperature;

3. 4% PFA was discarded, and rinsing was performed with PBS once;

4. The oil red stock solution was diluted, oil red:deionized water = 3:2, filtered with filter paper, and allowed to stand at room temperature for 10 minutes;

5. The oil red staining solution was discarded, 60% isopropanol was added to rinse once to remove the excessive dye;

6. 60% isopropanol was discarded, PBS was added, and photos were taken under a microscope.

Statistical analysis

One-way ANOVA and T-TEST in Prism 7.0 statistical analysis software were used for variance analysis and significance test, and the experimental data were expressed as mean $\pm$ standard error (Mean $\pm$ SE). *, $p<0.05$; **, $p<0.01$; ***, $p<0.001$.

Experimental results

(1) Anatomical observation showed that the livers of the mice in the MCD feed+solvent group turned white color, with a granular surface. However, the livers of the mice in the M cell group were normal reddish-brown, with a smooth surface, indicating that the M cells could inhibit hepatic steatosis.

(2) The livers were weighed, and the results showed that the M cells could significantly reduce the liver weight and inhibit the accumulation of fat in the liver.

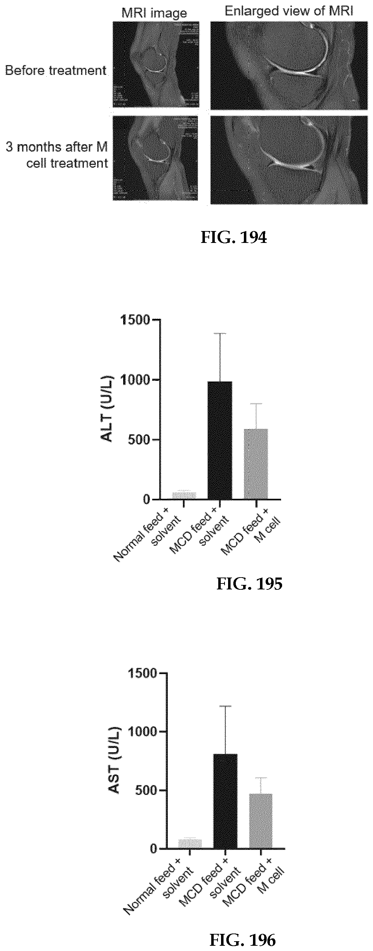

(3) The results of blood biochemical analysis showed that the M cells could significantly reduce the content of alanine aminotransferase (ALT) and aspartate aminotransferase (AST) in blood (Table 36-1, Table 36-2, FIG. 195 and FIG. 196), indicating that the M cells could reduce the levels of aminotransferase (ALT) and aspartate aminotransferase (AST), and improve liver function.

Table 36-1: ALT levels in blood of NASH model mice.

| Group | Normal control group | | | MCD feed+solvent | | | MCD feed+M cells | | |
|---|---|---|---|---|---|---|---|---|---|
| ALT(U/L) | 80 | 48 | 47 | 607 | 947 | 1406 | 786 | 625 | 369 |

Table 36-2: AST levels in blood of NASH model mice.

| Group | Normal control group | | | MCD feed+solvent | | | MCD feed+M cells | | |
|---|---|---|---|---|---|---|---|---|---|
| AST(U/L) | 103 | 69 | 72 | 499 | 664 | 1274 | 605 | 482 | 333 |

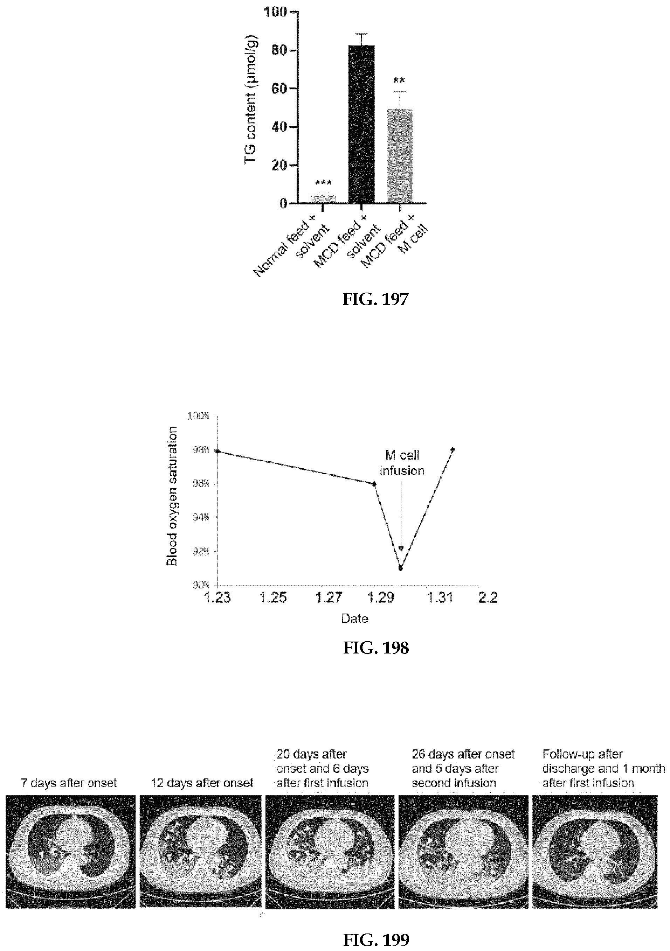

(4) The results of blood biochemical analysis showed that the M cells could significantly reduce the levels of blood triglyceride (TG) (Table 36-3, FIG. 197) and total cholesterol (CHO), indicating that the M cells could reduce the indicators of fatty liver.

Table 36-3: TG levels in liver of NASH model mice.

| Group | Normal control group | | | MCD feed+solvent | | | MCD feed+M cells | | |
|---|---|---|---|---|---|---|---|---|---|
| TG (umol/g) | 4.9 | 5.9 | 3.5 | 88.0 | 84.0 | 76.0 | 50.8 | 57.8 | 40.0 |

(5) HE staining results showed that there were a large number of fat droplets and bubble-like hepatocytes in the hepatocytes of the mice in the MCD feed+solvent group, and the hepatocytes in the MCD feed+M cell group were normal in shape, indicating that the M cells could reduce liver steatosis.

(6) Oil red O staining showed that there were a large number of lipid droplets in the liver cells of the mice in the MCD feed+solvent group, but only a small amount of lipid droplets existed in the liver of the mice in the MCD feed+M cell group, indicating that the M cells could reduce liver steatosis.

(7) Immunohistochemical results showed that the livers of the mice in the MCD feed+solvent group had expressed a large amount of Collagen I and $\alpha$-SMA protein, showing symptoms of fibrosis. The livers of the mice in the MCD feed+M cell group had expressed only a small amount, indicating that the M cells could inhibit the formation of fibrosis.

(8) HE staining results showed that a large number of inflammatory cells were infiltrated in the livers of the mice in the MCD feed+solvent group, while the liver morphology of the mice in the MCD feed+M cell group was normal, indicating that the M cells could inhibit the infiltration of inflammatory cells.

(9) The detection results of inflammatory factors in the serum of mice showed that compared with the MCD feed+solvent group, the levels of proinflammatory factors in the MCD feed+M cell group were significantly decreased, and the levels of anti-inflammatory factors were significantly increased. It indicated that the M cells had the effect of suppressing inflammation.

The levels of ALT and AST in the blood of the solvent group were significantly increased, and the concentration of TG in the liver was also significantly increased as compared with the normal group.

Example 37: Evaluation of therapeutic activity of M cells against acute respiratory distress syndrome (ARDS)

The pneumonia "COVID-19" caused by "SARS-CoV-2" infection has a long latent period, strong infectivity and great harm. Up to now, there is no effective treatment for COVID-19, but the prognosis of severe and critical patients with COVID-19 is poor, the mortality is high, and its clinical treatment needs are particularly urgent.

According to the latest epidemiological data, about 15.7% (173/1099 cases) of patients with COVID-19 are seriously ill, and some patients develop acute respiratory distress syndrome (ARDS), which leads to respiratory failure, which in turn affects the function of other organs, and even lead to death. Current data show that the fatality rate of severe cases is as high as 15%. ARDS presents as a clinical syndrome of rapidly progressive dyspnea, hypoxemia, diffuse pulmonary infiltrates, and respiratory failure. The current treatment options for ARDS are limited to symptomatic treatments such as basic medical care and supportive ventilation strategies, and are still unable to reverse the disease process, to improve the quality of life of patients, and to reduce the mortality rate. Mechanical ventilation is the mainstay of treatment for patients with acute respiratory distress syndrome. In the process of mechanical ventilation, complications such as ventilator-associated pneumonia, ventilator-associated lung injury, deep vein thrombosis, difficulty in weaning from mechanical ventilation, and pulmonary fibrosis often occur. Drug treatment methods include: corticosteroids, statins, aspirin, $\beta$-2 receptor agonists, surfactants, and inhaled NO, all of which have not shown significant efficacy. The above two treatment methods, together with auxiliary methods such as blood purification treatment, nutritional intervention, and fluid control, are far from satisfying the treatment of ARDS caused by COVID-19.

Mesenchymal stem cells (MSCs) are pluripotent cells with certain self-renewal and differentiation capabilities. Under specific culture conditions in vitro, MSCs can be directed to differentiate into adipocytes, chondroblasts, and osteoblasts. Adult MSCs come from a wide range of sources and can be isolated from bone marrow, umbilical cord or adipose tissue. MSCs are characterized by low immunogenicity and secrete a variety of factors, including endothelial and epithelial growth factors, anti-inflammatory cytokines and antimicrobial peptides. Preclinical studies have shown that MSCs have good safety and efficacy in the treatment of ARDS models caused by various reasons, including ARDS caused by septic shock, acute lung injury model caused by pathogens (e.g., *E. coli*), ventilator-associated lung injury model, thoracic trauma-induced lung injury animal model and ischemia-reperfusion lung injury model, etc. MSCs can be used for the clinical treatment of COVID-19 by regulating the immune response of the body, reducing the immune damage of lung tissue, secreting related proteins to promote the recovery of lung injury, and promoting the clearance of pathogenic bacteria.

In recent years, a large number of preclinical and clinical research results have shown that stem cell technology is expected to treat refractory lung diseases such as ARDS and PF. However, the clinical applications of adult tissue-derived MSCs mainly have the following disadvantages: (1) the therapeutic amount of adult tissue-derived MSCs can hardly be obtained from a single individual tissue; (2) the adult tissue-derived MSCs are derived from different individual tissues, and the consistency of product quality can hardly be achieved; (3) even the tissue-derived MSCs from the same individual are also highly heterogeneous; (4) the donor tissue sources of adult tissue-derived MSCs are complex and have potential infectious pathogen infection risks; (5) adult tissue-derived MSCs rapidly senesce with in vitro expansion.

Related documents:

(1) Transplantation of ACE2 to Mesenchymal stem cells Improves the Outcome of Patients with COVID-19 Pneumonia.

(2) Mesenchymal stem cell treatment in severe COVID-19: A retrospective study of short-term treatment efficacy and side effects

(3) Repair of Acute Respiratory Distress Syndrome by Stromal Cell Administration in COVID-19 (REALIST-COVID-19): A structured summary of a study protocol for a randomised, controlled trial

(4) Safety and efficacy assessment of allogeneic human dental pulp stem cells to treat patients with severe COVID-19: structured summary of a study protocol for a randomized controlled trial (Phase I/II)

(5) Adipose to derived mesenchymal stromal cells for the treatment of patients with severe SARS-CoV-2 pneumonia requiring mechanical ventilation. A proof of concept study

Through the study of the above documents, the possibility of M cell treatment for ARDS was explored, and it also provided a reference for the formulation of the experimental protocol.

Achieved results: After transplantation of the M cells, there was no stem cell drug-associated adverse reactions and serious adverse reactions occurred during the treatment period. One month after the infusion, CT showed that it returned to normal and no fibrosis was formed.

Preparation and culture of M cells:

The embryonic stem cells were suspended with EB spheres for adherent differentiation, and the M cells at P0 generation were obtained, passaged and screened, and cryopreserved at P3 generation for subsequent experiments.

The P3 generation M cells were resuscitated, digested and passaged, and used for subsequent clinical trials.

| Reagent/Equipment | Manufacturer | Cat. No. |
|---|---|---|
| Multifactor suspension chip system | Bio-Rad | Bio-Plex® 200 |
| 48-Factors kit | Bio-Rad | 12007283 |
| Computerized tomography (CT) | Philips | Ingenuity CT |
| Blood gas analyzer | Instrumentation Laboratory | GEM3500 |
| Blood biochemistry analyzer | Beckman | AU5800 |
| Hematology analyzer | Beijing Baolingman Sunshine Technology Co., Ltd. | BM830 |

Patient profile:

Male, 44 years old. He was admitted to Beijing You'an Hospital because of fever and cough for 6 days. Outside the hospital, he was treated for influenza A for 6 days, but his condition did not improve. The patient lived in Wuhan for a long time and came to Beijing to accompany his family for medical treatment. He is generally healthy, with no past medical or family history. Physical examination on admission: body temperature 37.9 °C, blood pressure 120/60 mmHg, heart rate 80 beats/min, respiration 21 breaths/min. Pulmonary auscultation revealed coarse breath sounds. The SARS-Cov-2 nucleic acid detection was positive, diagnosed with COVID-19, and was admitted to the hospital. Plain CT scan of the chest showed multiple ground-glass opacities in both lungs, especially in the right lower lung. After admission, his vital signs were stable, with intermittent fever and cough, and the highest body temperature was 39°C. Symptomatic support was given, and lopinavir/ritonavir plus Chinese patent medicine were used for combined antiviral therapy. Five days after admission, the patient developed breathlessness. Repeated chest CT showed that the lung lesions were significantly aggravated, with multiple patchy, flaky ground-glass density and high-density shadows in both lungs, and the scope expanded; multiple cystic translucent shadows appeared in both lungs. Six days after admission, his condition worsened, with suffocation, chest tightness, decreased blood oxygen saturation, hypokalemia. Seven days after admission, the patient's condition further deteriorated, and the finger oxygen saturation was 91% in the resting state without oxygen inhalation.

stem cell therapeutic regimen:

Seven days after admission, after the patient signed the informed consent, the M cells were infused intravenously, $3 \times 10^6$ cells/kg body weight. The cells were infused for 7 days, and then a second cell infusion treatment was performed. During stem cell treatment, the patient had been receiving antiviral basic therapy.

Experiment results: It could be given in the form of text description, tables or drawings, preferably with analysis and evaluation of the results.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 48-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex Manager TM.

(4) To the standard bottle, 250 μL of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1 time with Bio-Plex detection buffer, and stored in the dark.

(7) The diluted magnetic beads were vortexed, and 50 $\mu$L of the magnetic beads was added to each well.

(8) The plate was washed twice with 100 $\mu$L of washing solution.

(9) The sample, standard, blank, and control of known concentration were vortexed, and added in an amount of 50 $\mu$L to each well.

(10) The plate was sealed with a sealing film, and shaken on a high-frequency shaker at 850$\pm$50 rpm at room temperature for 30 min.

(11) In step (10), when the remaining 10 min of shaking time was left, the detection antibody was vortexed for 5 s and diluted to 1 time.

(12) The plate was washed twice with 100 $\mu$L of washing solution.

(13) The diluted antibody was vortexed, and added in an amount of 250 $\mu$L to each well.

(14) The plate was sealed with a sealing film, and shaken on a high-frequency shaker at 850$\pm$50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1 time.

(17) The plate was washed twice with 100 $\mu$L of washing solution.

(18) The diluted SA-PE was vortexed, and added in an amount of 50 $\mu$L to each well.

(19) The plate was sealed with a sealing film, and shaken on a high-frequency shaker at 850$\pm$50 rpm at room temperature for 30 min.

(20) The plate was washed three times with 100 $\mu$L of washing solution.

(21) The magnetic beads were resuspended with 125 $\mu$L of detection solution, the plate was sealed with a sealing film, and shaken on a high-frequency shaker at room temperature at 850$\pm$50 rpm for 30 s.

(22) After the sealing film was discarded, loading to machine was started.

Effectiveness evaluation

(1) Clinical symptoms: After the first cell infusion, the blood oxygen saturation increased (Table 37-1, FIG. 198). From the 2nd day, the patient reported that the shortness of breath was relieved; on the 4th day, the clinical symptoms disappeared and the conditions were improved significantly.

Table 37-1: Changes in patient blood oxygen saturation

|  | January 23 | January 29 | January 30 | February 1 |
|---|---|---|---|---|
| Blood oxygen saturation | 98% | 96% | 91% | 98% |

(2) Chest CT: Before the first cell infusion, the patient's chest CT showed multiple patches in both lungs, sheet-like ground-glass density and high density shadows (yellow arrows). On the second day after the patient received the second M cell infusion (at an interval of 6 days), CT showed improved absorption at the lesion site, and 1 month after the first infusion, CT showed it return to normal without fibrosis (FIG. 199).
(3) Blood biochemistry and virus detection: On the second day after the first infusion, the absolute value of lymphocytes

was $0.79 \times 10^9$/L, and rose to $1.02 \times 10^9$/L after the second infusion (Table 37-1). On the 2nd and 3rd days after the second cell infusion, the nucleic acid test for the novel coronavirus was negative for two consecutive days. Two days after the virus turned negative, the discharge criteria were met. Two weeks after discharge from the hospital, he returned to the hospital and found that the nucleic acid of the novel coronavirus was negative, and the blood glucose, liver function, renal function, troponin, and complete blood cell analysis were all normal (Table 37-2).

Table 37-2: Blood biochemical test and virus test before and after M cell infusion

| Item | Unit | Ref. Range | Day 1 after onset/Day 1 in hospital | Day 12 after onset/Day 6 in hospital | Day 15 after onset/Day 9 in hospital/Day 1 after 1st infusion of CA Stem cells | Day 18 after onset/Day 12 in hospital/Day 4 after 1st infusion of CA Stem cells | Day 21 after onset/Day 15 in hospital/The day of 2nd infusion of CA Stem cells | Day 23 after onset/Day 17 in hospital/Day 2 after 2nd infusion of CA Stem cells | Follow-up Discharged 1 Month after 1st infusion of CA Stem cells |
|---|---|---|---|---|---|---|---|---|---|
| White blood cell count | *10^9/L | 3.5-9.5 | 3.83 | 5.69 | 6.33 | 5.20 | 6.75 | 6.69 | 7.11 |
| Absolute neutrophil count | *10^9/L | 1.8-6.3 | 2.90 | 4.44 | 4.75 | 3.83 | 5.21 | 5.00 | 5.1 |
| Absolute value of lymphocytes | *10^9/L | 1.1-3.2 | 0.65 ↓CS* | 0.78 ↓CS | 0.79 ↓CS | 0.77 ↓CS | 0.79 ↓CS | 1.02 ↓CS | 1.42 |
| Monocyte absolute value | *10^9/L | 0.1-0.6 | 0.21 | 0.23 | 0.38 | 0.31 | 0.40 | 0.38 | 0.48 |
| Absolute eosinophil count | *10^9/L | 0.02-0.52 | 0.01 ↓NCS# | 0.01 ↓NCS | 0.05 | 0.09 | 0.10 | 0.09 | 0.05 |
| Absolute basophil count | *10^9/L | 0-0.06 | 0.010 | 0.060 | 0.100 ↑NCS | 0.040 | 0.030 | 0.040 | 0.06 |
| Neutrophil percentage | % | 40-75 | 75.7 ↑CS | 78.0 ↑CS | 75.1 ↑NCS | 73.7 | 77.2 ↑CS | 74.7 | 71.7 |
| Lymphocyte percentage | % | 20-50 | 16.8 ↓CS | 13.6 ↓CS | 12.5 ↓CS | 14.8 ↓CS | 11.7 ↓CS | 15.2 ↓CS | 20 |
| Monocyte percentage | % | 3-10 | 5.5 | 4.0 | 5.9 | 6.0 | 6.0 | 5.7 | 6.8 |
| Hemoglobin concentration distribution width | g/L | 22-32 | 29.5 | 25.8 | 25.1 | 24.3 | 25.0 | 23.8 | 0.7 |
| Eosinophil percentage | % | 0.4-8.0 | 0.2 ↓NCS | 0.2 ↓CS | 0.8 | 1.6 | 1.5 | 1.3 | 0.8 |
| Basophil percentage | % | 0-1 | 0.20 | 1.00 | 1.50 ↑NCS | 0.80 | 0.50 | 0.50 | 4.66 |

| Item | Unit | Ref. Range | Day 1 after onset/Day 1 in hospital | Day 12 after onset/Day 6 in hospital | Day 15 after onset/Day 9 in hospital/Day 1 after 1st infusion of CA Stem cells | Day 18 after onset/Day 12 in hospital/Day 4 after 1st infusion of CA Stem cells | Day 21 after onset/Day 15 in hospital/The day of 2nd infusion of CA Stem cells | Day 23 after onset/Day 17 in hospital/Day 2 after 2nd infusion of CA Stem cells | Follow-up Discharged 1 Month after 1st infusion of CA Stem cells |
|---|---|---|---|---|---|---|---|---|---|
| Red blood cell count | *10^12/L | 4.3-5.8 | 4.93 | 4.89 | 4.78 | 4.59 | 4.71 | 4.58 | 142 |
| Hemoglobin | g/L | 130-175 | 152.0 | 150.0 | 144.0 | 138.0 | 139.0 | 136.0 | 41 |
| Hematocrit | % | 40-50 | 43.9 | 45.7 | 44.0 | 42.4 | 43.6 | 43.0 | 88 |
| Mean red blood cell volume | fL | **82-100** | 89.2 | 93.5 | 92.1 | 92.4 | 92.5 | 93.8 | 30.5 |
| Mean erythrocyte hemoglobin content | pg | 27-34 | 30.8 | 30.7 | 30.2 | 30.1 | 29.6 | 29.7 | 346 |
| Mean erythrocyte hemoglobin concentration | g/L | 316-354 | 345 | 328 | 328 | 326 | 319 | 317 | |
| Red blood cell volume distribution width | % | 10-20 | 14.1 | 13.1 | 12.8 | 12.8 | 13.0 | 13.1 | 13 |
| Large unstained cell count | *10^9/L | 0-0.4 | 0.06 | 0.18 | 0.27 | 0.16 | 0.22 | 0.17 | - |
| Platelet count | *10^9/L | 125-350 | 122 ↓CS | 186 | 257 | 332 | 292 | 278 | - |
| Mean platelet volume | fL | 8-12 | 8.2 | 10.1 | 9.7 | 9.8 | 9.6 | 9.8 | 189 |
| Large unstained cells | % | 0-4 | 1.50 | 3.10 | 4.20 ↑NCS | 3.10 | 3.20 | 2.60 | 10.9 |
| Platelet crit | % | 0.2-0.5 | 0.10 ↓CS | 0.19 ↓CS | 0.25 | 0.33 | 0.28 | 0.27 | 0.21 |
| Platelet distribution width | fL | 9.8-17.0 | 88.70 ↑CS | 57.4 ↑NCS | 54.4 ↑NCS | 48.1 ↑NCS | 52.90 ↑NCS | 50.7 ↑NCS | 12 |

(continued)

| Item | Unit | Ref. Range | Day 1 after onset/Day 1 in hospital | Day 12 after onset/Day 6 in hospital | Day 15 after onset/Day 9 in hospital/Day 1 after 1st infusion of CA Stem cells | Day 18 after onset/Day 12 in hospital/Day 4 after 1st infusion of CA Stem cells | Day 21 after onset/Day 15 in hospital/The day of 2nd infusion of CA Stem cells | Day 23 after onset/Day 17 in hospital/Day 2 after 2nd infusion of CA Stem cells | Follow-up Discharged 1 Month after 1st infusion of CA Stem cells |
|---|---|---|---|---|---|---|---|---|---|
| Absolute value of nucleated cells | *10^3/UL | - | 0.00 | 0.00 | 0.00 | 0.00 | 0.00 | 0.00 | - |
| Percentage of nucleated red blood cells | /100WBC | - | 0.00 | 0.00 | 0.00 | 0.00 | 0.00 | 0.00 | - |
| Alanine aminotransferase | U/L | 9-50 | 80↑CS | 48 | 75↑CS | 67↑CS | 43 | 42 | 47 |
| Aspartate aminotransferase | U/L | 15-40 | 78↑CS | 50↑CS | 68↑CS | 47↑CS | 31 | 36 | 37 |
| GOT/GPT | - | - | 0.97 | 1.04 | 0.91 | 0.70 | 0.72 | 0.86 | 0.79 |
| Total bilirubin | Umol/L | 5-21 | 8.2 | 37.5↑CS | 20.1 | 20.2 | 15.0 | 13.5 | 7 |
| Direct bilirubin | Umol/L | <7 | 1.8 | 26.7↑CS | 10.0↑CS | 9.6↑CS | 6.6 | 7.1↑CS | 2.1 |
| Direct bilirubin/ total bilirubin | - | - | 0.22 | 0.71 | 0.50 | 0.48 | 0.44 | 0.53 | 0.3 |
| Total protein | g/L | 65-85 | 73.0 | 72.5 | 73.1 | 78.3 | 84.0 | 85.5↑CS | 84.6 |
| Albumin | g/L | 40-55 | 33.6↓CS | 26.9↓CS | 28.0↓CS | 29.6↓CS | 34.6↓CS | 35.6↓CS | 42.6 |
| Globulin | g/L | 20-40 | 39.4 | 45.6↑CS | 45.1↑CS | 48.7↑CS | 49.4↑CS | 49.9↑CS | 42 |
| Albumin/globulin | - | 1.2-2.4 | 0.85↓CS | 0.59↓CS | 0.62↓CS | 0.61↓CS | 0.70↓CS | 0.71↓CS | 1.01 |
| Creatinine (enzymatic method) | Umol/L | 57-97 | 78 | 65 | 61 | 64 | 71 | 68 | 60 |
| Glomerular filtration rate | mL/mln/L | >90 | 104.4 | 112.5 | 115.5 | 113.2 | 108.5 | 110.4 | 116.2 |
| Carbon dioxide binding force | mmol/L | 22-29 | 21.7↓CS | 30.2↑NCS | 31.5↑CS | 29.5↑NCS | 27.4 | 25.4 | 28.8 |

| Item | Unit | Ref. Range | Day 1 after onset/Day 1 in hospital | Day 12 after onset/Day 6 in hospital | Day 15 after onset/Day 9 in hospital/Day 1 after 1st infusion of CA Stem cells | Day 18 after onset/Day 12 in hospital/Day 4 after 1st infusion of CA Stem cells | Day 21 after onset/Day 15 in hospital/The day of 2nd infusion of CA Stem cells | Day 23 after onset/Day 17 in hospital/Day 2 after 2nd infusion of CA Stem cells | Follow-up Discharged 1 Month after 1st infusion of CA Stem cells |
|---|---|---|---|---|---|---|---|---|---|
| Potassium | mmol/L | 3.5-5.3 | 3.61 | 3.32 ↓CS | 3.34 ↓CS | 4.04 | 4.76 | 4.39 | 3.78 |
| Sodium | mmol/L | 137-147 | 134.5 ↓NCS | 133.7 ↓CS | 135.8 ↓CS | 136.2 ↓CS | 135.5 ↓CS | 134.7 ↓CS | 139.6 |
| Chloride | mmol/L | 99-110 | 99.0 | 97.2 ↓CS | 98.1 ↓CS | 99.2 | 101.1 | 103.0 | 102.2 |
| Anion gap | mmol/L | 10-14 | 13.8 | 6.3 ↓CS | 6.2 ↓CS | 7.5 ↓NCS | 7.0 ↓CS | 6.3 ↓CS | 8.6 |
| **Creatine kinase** | U/L | 50-310 | 80 | 105 | 34 ↓NCS | - | - | 25 ↓NCS | 67 |
| Creatine kinase isoenzyme | ng/mL | <3.6 | 0.09 | 0.33 | 0.00 | - | - | 0.28 | 0.17 |
| Myoglobin | ng/mL | 16-96 | 52 | 53 | 31 | - | - | 36 | 44 |
| Troponin I | ng/mL | <0.056 | 0.010 | 0.010 | 0.010 | - | - | 0.020 | 0.02 |
| C-reactive protein | mg/mL | <3 | 11.8 ↑CS | 80.8 ↑CS | 38.9 ↑CS | 50.2 ↑CS | 26.4 ↑CS | 19.4 ↑CS | - |
| Prothrombin time HS | S | 9.9-12.8 | 12.0 | 12.8 | 12.7 | 12.3 | - | 12.5 | - |
| Prothrombin activity HSPT% | % | 80-120 | 81.0 | 74.0 ↓CS | 74.0 ↓CS | 78.0 ↓CS | - | 76.0 ↓CS | - |
| Prothrombin ratio HS | R | 0.8-1.3 | 1.07 | 1.14 | 1.13 | 1.10 | - | 1.12 | - |
| International normalized ratio of prothrombin | INR | 0.8-1.2 | 1.07 | 1.14 | 1.13 | 1.10 | - | 1.11 | - |
| Activated partial thromboplastin time | S | 25-36.5 | 32.0 | 32.8 | 31.7 | 33.6 | - | 37.4 ↑CS | - |

| Item | Unit | Ref. Range | Day 1 after onset/Day 1 in hospital | Day 12 after onset/Day 6 in hospital | Day 15 after onset/Day 9 in hospital/Day 1 after 1st infusion of CA Stem cells | Day 18 after onset/Day 12 inhospital/Day 4 after 1st infusion of CA Stem cells | Day 21 after onset/Day 15 in hospital/The day of 2nd infusion of CA Stem cells | Day 23 after onset/Day 17 in hospital/Day 2 after 2nd infusion of CA Stem cells | Follow-up Discharged 1 Month after 1st infusion of CA Stem cells |
|---|---|---|---|---|---|---|---|---|---|
| Activated partial thromboplastin time ratio | R | 0.9-1.3 | 1.05 | 1.08 | 1.04 | 1.10 | - | 1.23 | - |
| Fibrinogen content | g/L | 2-4 | 3.58 | 4.21 ↑NCS | 5.22 ↑NCS | 5.04 ↑NCS | - | 5.87 ↑NCS | - |
| Thrombin time | S | 11-18 | 14.70 | 16.00 | 14.90 | 14.80 | - | 14.00 | - |
| Procalcitonin | ng/mL | <0.1 | 0.11 ↑NCS | 0.11 ↑NCS | | 0.14 ↑NCS | 0.14 ↑NCS | 0.16 ↑NCS | |
| Lactic acid | Mmol/L | 0.4-2.0 | 1.75 | 1.58 | 1.32 | 1.32 | | 1.84 | |
| Influenza A virus generic | Negative/ negative | Negative | Negative | - | - | - | | - | |
| Influenza a virus H1N1 | Negative/ negative | Negative | Negative | - | - | - | | - | |
| Avian influenza virus H7N9 | Negative/ negative | Negative | Negative | - | - | - | - | - | - |
| **Novel coronavirus nucleic acid detection** | Negative/ negative | Negative | Negative | - | - | - | Negative | Negative | Negative |
| *: CS, Clinically Significant <br> #: NSC, Not Clinically Significant | | | | | | | | | |

274

EP 4 047 083 A1

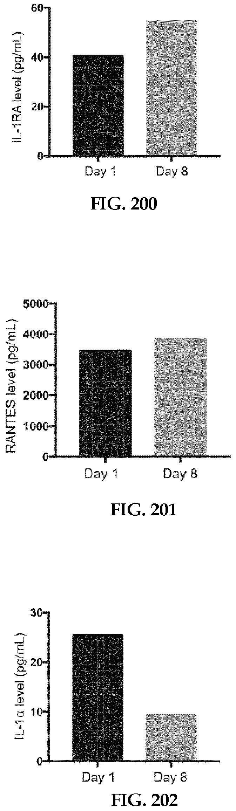

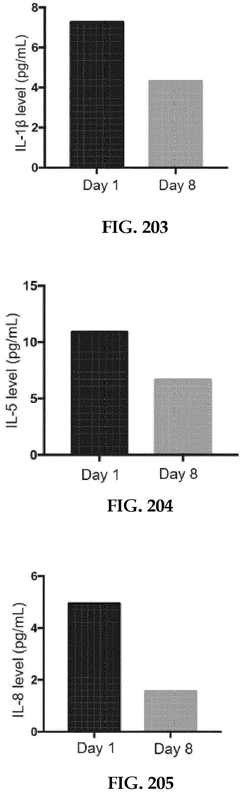

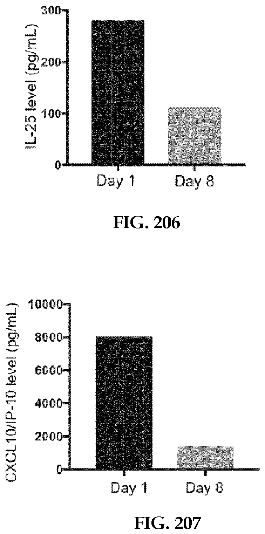

(4) Detection of cytokines: On the 8th day after the first infusion, the levels of anti-inflammatory cytokines such as IL-1RA and RANTES increased (Table 37-3 and Table 37-4, FIGS. 200 and 201), and proinflammatory cytokines such as IL-1$\alpha$, IL-1$\beta$, IL-5, IL-8, IL-25 and CXCL10/IP-10 were significantly reduced (Table 37-5 to Table 37-10, Figure 202 to Figure 207). Correspondingly, C-reactive protein levels were significantly reduced (Table 37-2). In conclusion, no adverse events occurred after intravenous infusion of M cells in the ARDS patient. The M cell infusion had the functions of promoting the improvement of absorption at the lesion site, inhibiting inflammation and promoting pulmonary recovery.

Table 37-3: Change in IL-1RA levels of the patient

|  | Day 1 | Day 8 |
|---|---|---|
| IL-1RA (pg/mL) | 40.50 | 54.65 |

Table 37-4: Change in RANTES levels of the patient

|  | Day 1 | Day 8 |
|---|---|---|
| RANTES (pg/mL) | 3457.00 | 3843.00 |

Table 37-5: Change in IL-1$\alpha$ levels of the patient

|  | Day 1 | Day 8 |
|---|---|---|
| IL-1$\alpha$ (pg/mL) | 25.48 | 9.35 |

Table 37-6: Change in IL-1$\beta$ levels of the patient

|  | Day 1 | Day 8 |
|---|---|---|
| IL-1$\beta$ (pg/mL) | 7.27 | 4.33 |

Table 37-7: Change in IL-5 levels of the patient

|  | Day 1 | Day 8 |
|---|---|---|
| IL-5 (pg/mL) | 10.91 | 6.69 |

Table 37-8: Change in IL-8 levels of the patient

|  | Day 1 | Day 8 |
|---|---|---|
| IL-8 (pg/mL) | 4.95 | 1.57 |

Table 37-9: Change in IL-25 levels of the patient

|  | Day 1 | Day 8 |
|---|---|---|
| IL-25 (pg/mL) | 279.22 | 109.78 |

Table 37-10: Change in CXCL10/IP-10 levels of the patient

|  | Day 1 | Day 8 |
|---|---|---|
| CXCL10/IP-10 (pg/mL) | 7993.00 | 1343.00 |

Example 38: Evaluation of therapeutic effect of M cells against idiopathic pulmonary fibrosis

Idiopathic pulmonary fibrosis (IPF) is a chronic progressive lung disease characterized by pulmonary interstitial fibrosis. Its etiology is still unclear, and it is more common in the elderly with an upward trend of incidence in recent years. However, the diagnosis of IPF is still a clinical problem. The onset of IPF is insidious, there are often no obvious clinical manifestations in the early stage, and the imaging and pulmonary function manifestations are not typical. Therefore, patients with IPF are often diagnosed after the development of the disease to the occurrence of various complications. However, there is currently no effective treatment for IPF, and the lung function of patients continues to deteriorate with the progression of the disease, and the median survival time is only 2 to 3 years.

Mesenchymal stem cells (MSCs) are a kind of adult stem cells, which have the characteristics of self-renewal, low immunogenicity, multi-directional differentiation, immune regulation and tissue repair ability. In recent years, studies have found that in injured lung tissue, mesenchymal stem cells can be directed to differentiate into type II alveolar epithelial cells and fibroblasts under the mediation of inflammatory factors or receptor pathways such as CXCL8, SDF-1, and CXCR4, suggesting that pluripotent interstitial stromal cells are extensively involved in the repair and fibrosis of lung injury. A number of animal experimental studies have found that intravenous injection of pluripotent stromal cells can reduce lung inflammation and fibrosis in bleomycin-induced pulmonary fibrosis model mice, suggesting that pluripotent mesenchymal stromal cell therapy may become a novel approach for the treatment of pulmonary fibrosis in the future. In recent years, Greece, Australia and the United States have successively carried out phase I clinical trials of pluripotent mesenchymal stromal cell therapy in IPF patients. During the follow-up period of 6 to 15 months, no significant adverse events occurred, suggesting that pluripotent mesenchymal stromal cells are safe and reliable in the treatment of IPF.

However, the clinical application of adult tissue-derived MSCs mainly has the following disadvantages: (1) the therapeutic amount of adult tissue-derived MSCs can hardly be obtained from a single individual tissue; (2) the adult tissue-derived MSCs are derived from different individual tissues, which can hardly achieve a high consistency of product quality; (3) even MSCs derived from the same individual tissue are highly heterogeneous; (4) the donor tissue sources of adult tissue-derived MSCs are complex and have potential infectious pathogen infection risks; (5) the adult tissue-derived MSCs rapid senesce with in vitro expansion. Therefore, new sources of MSCs are needed for pulmonary fibrosis.

Related documents:

(1) Cell Therapy in Idiopathic Pulmonary Fibrosis.

(2) Idiopathic pulmonary fibrosis

(3) Mesenchymal stem cells in idiopathic pulmonary fibrosis

(4) Mesenchymal stem cells and Idiopathic Pulmonary Fibrosis

Achieved results: After transplantation of the M cells, there was no stem cell drug-associated adverse reactions and serious adverse reactions occurred during the treatment period. After infusion of the M cells, CT showed that the absorption of the subject's lung lesions was significantly improved.

Preparation and culture of M cells:

The embryonic stem cells were suspended with EB spheres for adherent differentiation, and the M cells at P0 generation were obtained, passaged and screened, and cryopreserved at P3 generation for subsequent experiments.

The P3 generation M cells were resuscitated, digested and passaged, and used for the following clinical trials.

| Reagent/Equi pment | Manufacturer | Cat. No. |
|---|---|---|
| Computerized tomography (CT) | Philips | Ingenuity CT |

Patient profile:

After screening, a total of four patients with pulmonary fibrosis caused by COVID-19 were enrolled.

Stem cell therapeutic regimen:

After the patients signed the informed consent, the M cells were infused intravenously, $3 \times 10^6$ cells/kg body weight. After the cells were infused, CT scans of the lungs were performed to observe pulmonary fibrosis.

Effectiveness evaluation

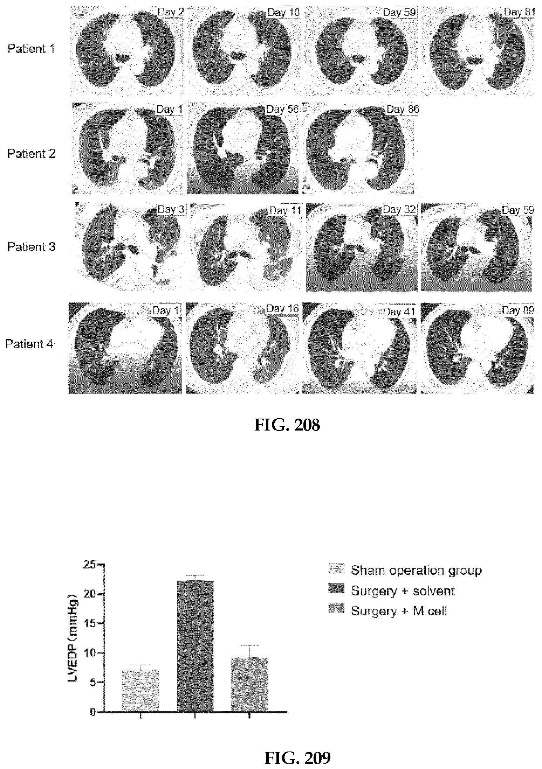

(1) Chest CT: Before the M cell infusion, the chest CT of the patients showed bilateral lung multiple patches, sheet-like ground-glass density and high-density shadows (indicated by arrows). After the patients received the M cell infusion, CT showed that the absorption of all the patient's lesions was improved, and 1 month after the infusion, CT showed the lungs basically returned to normal. After 50 days, pulmonary fibrosis disappeared in all patients (FIG. 208).

Example 39: Evaluation of therapeutic activity of M cells against myocardial vascular reperfusion

Ischemic heart disease is the leading cause of death in humans, and early and successful recovery of myocardial reperfusion is the most effective way to improve clinical outcomes. However, the process of restoring blood flow to the ischemic myocardium may cause damage, a phenomenon called myocardial ischemia/reperfusion injury (MI/RI). I/R will bring some adverse effects, such as oxidative stress, intracellular calcium overload, etc., which may lead to cardiomyocyte apoptosis. Apoptosis is an important reason for the loss of tissue function in ischemia-reperfusion injury. It is a very complex process, and its detailed triggering mechanism is not completely clear.

1. Experimental method:

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and P5 generation was used for subsequent experiments.

Experimental animals: Sprague-Dawley rats, 6 to 8 weeks old, purchased from Weitong Lihua Laboratory Animal Technology Co., Ltd. All animals were kept at the SPF grade of the Laboratory Animal Center of the Institute of Zoology, Chinese Academy of Sciences. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22 \pm 2°C$, relative humidity was 50% to 60%. The experiment was started after one week of adaptive feeding of rats.

Experimental groups: normal control group, surgery+solvent (solvent group), surgery+M cells (M cell group), with 3 rats in each group.

Experimental materials: surgical instruments, 1ml disposable sterile syringe, 3-0 surgical suture, weight scale.

Experimental reagents: isoflurane, iodophor, 2,3,5-triphenyl tetrazolium chloride (TTC)

Equipment:

| Consumable/Reagent/Instrument | Manufacturer | Cat. No./Model |
|---|---|---|
| 3-0 Surgical suture | Stones | EB03 |

(continued)

| Consumable/Reagent/Instrument | Manufacturer | Cat. No./Model |
|---|---|---|
| Disposable sterile syringe 1ml | Jiangsu Zhiyu Medical Equipment Co., Ltd. | None |
| 2,3,5-Triphenyl tetrazolium chloride | Sigma | T8877-100G |
| Masson trichrome staining solution | Solarbio | G1343 |
| Isoflurane | Ruiwode | 970-00026-00 |
| Iodophor | Hangzhou Langso Medical Disinfectant Co., Ltd. | |
| Blood chemistry analyzer | Beckman | AU5800 |
| R540 Enhanced small animal anesthesia machine | Ruiwode | R540 |
| Small animal ventilator | Ruiwode | R419 |
| Small animal B-ultrasonography | VisualSonics | Vevo LAZR |

All data were analyzed by One-way ANOVA in Prism 7.0 statistical analysis software for analysis of variance and significance test, and the experimental data were expressed as mean $\pm$ standard deviation (Mean $\pm$ SD). *, p<0.05; **, p<0.01; ***, p<0.001.

SD male rats were purchased from Weitong Lihua, and after one week of adaptive feeding, a rat model of myocardial infarction was established by ligating the left anterior descending coronary artery. The rats were placed in an anesthesia box, anesthetized and maintained the anesthesia state, the chest was depilated, wiped with iodophor, a longitudinal incision of about 2 cm was made on the left side of the sternum and parallel to the sternum, the skin was cut, and the pectoralis major muscle was bluntly separated, the thoracic cavity was expanded with an ophthalmic eyelid opener on the intercostal muscles between the 3rd and 4th ribs, the heart was exposed, and the pericardium was separated. The coronary arteries were carefully identified, and non-invasive sutures were used to ligate the distal 1/3 of the left anterior descending coronary artery (LAD) together with the myocardium, tied with a slip knot, and reperfusion was performed after 45 minutes of ischemia. Immediately after that, the drug injection was performed. The normal control group was left untreated, the surgery+solvent (solvent group) group was injected with 1ml of normal saline, and the surgery+M cells (M cell group) was injected with $2.5 \times 10^6$/1ml/ratl via the tail vein. On the 3rd day after the operation, a small animal B-ultrasonography was used to detect changes in cardiac function indicators, including: left ventricular end diastolic pressure (LVEDP), left ventricular ejection fraction (EF), left ventricular shortening fraction (FS), left ventricular end diastolic diameter (LVEDD), left ventricular end systolic diameter (LVESD), left ventricular end diastolic volume (LVEDV), left ventricular end systolic volume (LVESV), and left ventricular free wall. The rats were deeply anesthetized after B-ultrasonography, and then the abdominal aorta was taken for perfusion, and the heart was taken. Some rat heart tissues were stained with TTC, and some rat heart tissues were stained with Masson's trichrome. Blood samples were tested for contents of lactate dehydrogenase (LDH) and creatine kinase (Creatine Kinase, CK) by a blood biochemical analyzer.

2. Experimental results:

Result analysis:

(1) LVEDP could reflect the resistance or load before myocardial contraction, EF and FS reflected left ventricular systolic function, and LVEDD, LVESD, LVEDV and LVESV reflected cardiac systolic and diastolic function. In the solvent group, LVEDP was significantly increased, EF and FS were significantly decreased, and LVEDD, LVESD, LVEDV and LVESV were significantly increased. LVEDP in the M cell group decreased by 13 mmHg as compared with the solvent group (Table 39-1, FIG. 209). It indicated that the M cells could reduce the resistance or load before myocardial contraction. In the M cell group, EF and FS were significantly increased, while LVEDD, LVESD, LVEDV and LVESV were decreased. Left ventricular contraction was significantly improved in the M cell group. All the above results indicated that the M cells could improve the cardiac function of rats with myocardial ischemia-reperfusion.

Table 39-1: Cardiac LVEDP (mmHg)

| Group | Sham group | | | Surgery+solvent | | | Surgery+M cells | | |
|---|---|---|---|---|---|---|---|---|---|
| LVEDP (mmHg) | 6.1 | 7.9 | 7.5 | 21.5 | 21.9 | 23.3 | 10.8 | 6.9 | 10.0 |

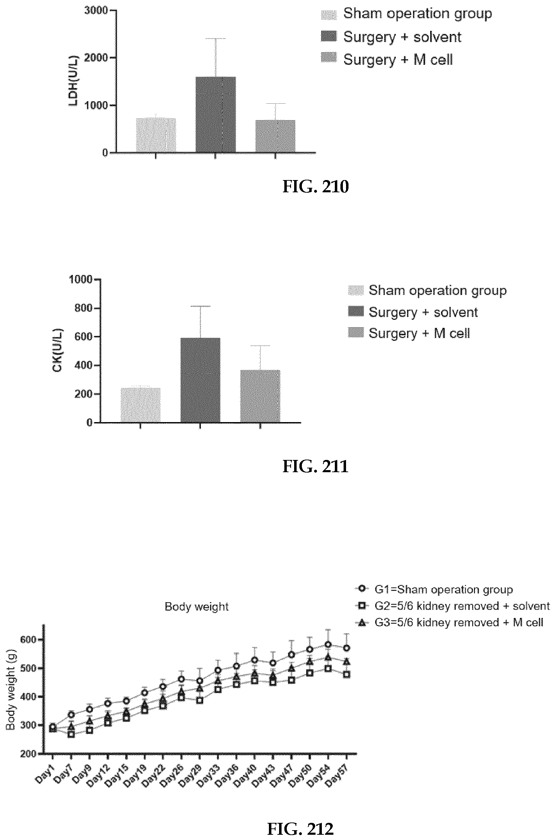

(2) LDH and CK could reflect the degree of cardiac infarction. The results showed that the contents of both enzymes were significantly increased in the solvent group. The M cell group had significantly less LDH and CK, indicating that the M cells could reduce the degree of cardiac infarction (Table 39-2/FIG. 210, Table 39-3/FIG. 211). The TTC results more directly and intuitively confirm the above results.

Table 39-2: LDH (U/L) content in blood

| Group | Sham group | | | Surgery+solvent | | | Surgery+M cells | | |
|---|---|---|---|---|---|---|---|---|---|
| LDH (U/L) | 738 | 642 | 830 | 1001 | 1291 | 2516 | 721 | 339 | 1030 |

Table 39-3: CK (U/L) content in blood

| Group | Sham group | | | Surgery+solvent | | | Surgery+M cells | | |
|---|---|---|---|---|---|---|---|---|---|
| CK (U/L) | 228 | 250 | 260 | 382 | 575 | 822 | 403 | 179 | 518 |

(3) Masson's trichrome staining showed that the collagen-enriched scar tissue was stained in blue, and living myocardial tissue was stained in red. In the M cell group, the scar size was smaller and the left ventricular wall thickness was larger.

The above results indicated that the M cells could improve cardiac function indicators and reduce cardiac infarct size.

Example 40: Evaluation of therapeutic activity of M cells for nephrectomy

Nephrectomy is a surgical procedure to treat kidney disease. Its indications include renal malignancy, renal tuberculosis, severe hydronephrosis or kidney stones, severe renal injury and unilateral pyonephrosis. In this example, the purpose of treating nephrectomy or kidney disease is achieved by transplanting the M cells.

1. Experimental method:

Experimental animals: SD rats, male, 6 weeks old, purchased from Beijing Weitong Lihua Company. All animals were kept at SPF grade in the Laboratory Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and used at P5 generation for subsequent experiments.

| Reagent/Equi pment | Manufacturer | Cat. No. |
|---|---|---|
| Disposable sterile syringe 1ml | Jiangsu Zhiyu Medical Equipment Co., Ltd. | None |
| Disposable sterile syringe 5ml | Jiangsu Zhiyu Medical Equipment Co., Ltd. | None |
| Normal saline | Shijiazhuang No. 4 Pharmaceutical Co., Ltd. | None |
| Chemray 240 Automatic biochemical analyzer | Rayto | Chemray 240 |
| Electronic scale | Yasuwang | CC-1013-04 |

Animal modeling: Rats were anesthetized by intraperitoneal injection of 100 ml/L chloral hydrate, 300 mg/kg, laparotomy was performed, the left kidney and renal pedicle were exposed and bluntly separated, the left kidney was exposed, the fat sac around the kidney was separated, and the upper and lower kidneys were excised, a total of 2/3 of the left kidney was excised, the bleeding was stopped by compression with gelatin sponge for 1 min, the kidney was reset, and the abdomen was closed layer by layer. The second-stage operation was performed 4 days after the first-stage operation. The same method was used for anesthesia, laparotomy, exposure of the right kidney, ligation of the renal pedicle, and removal of the right kidney; a total of 5/6 of the kidneys were removed in two operations.

The animals were divided into sham group, surgery+solvent group, and surgery+test substance group, with 4 rats per group.

Sham group: Only the tissues around the kidney were freed and the abdomen was closed after removing the renal capsule.

Solvent group: 1 mL of normal saline was injected.

M cell group: 1 mL of normal saline containing $5 \times 10^6$ M cells (P5 generation) was injected.

The day of the operation was recorded as the day 1, and the treatment was performed two weeks after the operation, and the body weight was weighed on the days 7, 15, 19, 22, 26, 29, 33, 36, 40, 43, 47, 50, 54, 57. Injections of MSCs were performed on days 15, 29 and 43. Blood and urine sampling were performed on day 57.

Sample collection:

The rats were placed in metabolic cages, and 24 h urine was collected every other day. Urine samples on days 14, 29, 43 and 57 were collected for testing. After 57 days of modeling, the rats were sacrificed and blood samples were obtained.

The 24 h urine protein, serum creatinine and blood urea nitrogen levels were detected by a Chemray 240 automatic biochemical analyzer.

2. Experimental results

(1) Statistics of body weight:

The body weight test results of different groups of rats were shown in the following table and FIG. 212. The body weight of the sham group at each time point was significantly higher than that of the surgery+solvent group (P<0.05); the body weight of the surgery+M cell treatment group was higher than that of the surgery+solvent group at the days 1 to 50, but there was no significant difference. At the days 54 and 57, the body weight of the surgery+M treatment group was significantly higher than that of the surgery+solvent group (P<0.05); the above results showed that the M cell treatment group had a significant promoting effect on the body weight of nephrectomized rats.

Table 40-1: Test results of rat body weight

| Group | | G1= Sham group | | | | G2 = 5/6 kidney removed+solvent | | | | G3 = 5/6 kidney removed+M cells | | | |
|---|---|---|---|---|---|---|---|---|---|---|---|---|---|
| Body weight (g) | Day 1 | 313 | 287 | 287 | 291 | 296 | 276 | 299 | 278 | 273 | 288 | 292 | 294 |
| | Day 7 | 354 | 321 | 332 | 340 | 300 | 227 | 317 | 227 | 291 | 299 | 296 | 271 |
| | Day 9 | 381 | 336 | 350 | 356 | 308 | 241 | 341 | 238 | 313 | 320 | 314 | 286 |
| | Day 12 | 401 | 362 | 363 | 381 | 335 | 269 | 355 | 273 | 340 | 338 | 332 | 311 |
| | Day 15 | 405 | 374 | 380 | 382 | 344 | 293 | 362 | 300 | 350 | 349 | 348 | 330 |
| | Day 19 | 442 | 399 | 411 | 405 | 361 | 311 | 405 | 329 | 375 | 384 | 372 | 353 |
| | Day 22 | 472 | 412 | 435 | 426 | 375 | 331 | 423 | 345 | 407 | 403 | 397 | 347 |
| | Day 26 | 504 | 440 | 455 | 451 | 403 | 363 | 455 | 367 | 437 | 434 | 432 | 383 |
| | Day 29 | 521 | 435 | 426 | 445 | 397 | 365 | 453 | 335 | 441 | 455 | 443 | 387 |
| | Day 33 | 547 | 472 | 471 | 485 | 436 | 393 | 482 | 393 | 476 | 472 | 465 | 420 |
| | Day 36 | 574 | 485 | 480 | 495 | 448 | 410 | 496 | 421 | 485 | 492 | 484 | 429 |
| | Day 40 | 594 | 502 | 514 | 510 | 464 | 433 | 508 | 422 | 501 | 499 | 504 | 441 |
| | Day 43 | 573 | 492 | 498 | 516 | 457 | 408 | 515 | 426 | 472 | 503 | 478 | 457 |
| | Day 47 | 622 | 522 | 524 | 525 | 476 | 442 | 513 | 404 | 508 | 510 | 521 | 466 |
| | Day 50 | 630 | 546 | 544 | 545 | 504 | 460 | 545 | 427 | 547 | 534 | 537 | 494 |
| | Day 54 | 661 | 554 | 551 | 567 | 510 | 487 | 551 | 450 | 568 | 560 | 548 | 516 |
| | Day 57 | 646 | 548 | 540 | 550 | 509 | 447 | 515 | 445 | 540 | 520 | 516 | 522 |

(2) Biochemical tests of urine and blood

Experimental method: The rats were placed in metabolic cages, and 24 h urine was collected every other day. Urine was collected on days 14, 29, 43 and 57 for testing. After 57 days of the modeling, the rats were sacrificed and blood samples were obtained.

The 24 h urine protein, serum creatinine and blood urea nitrogen levels were detected by a Chemray 240 automatic biochemical analyzer.

Experimental results: On the 57th day, the rats of different groups were subjected to biochemical tests of urine and blood, and the statistics of values including contents of uric acid (UA), urea (UREA) and urine creatinine (CREA) were carried out. The results showed that on the 57th day, the contents of uric acid, urea and urine creatinine of rats in the surgery+solvent group were significantly higher than those in the sham group. It showed that the nephrectomy model was successfully constructed, and the kidneys of the rats were severely damaged; the body weight of the M cell treatment group increased on the 57th day, which was significantly higher than that of the surgery+solvent group. The biochemical tests of urine and blood showed that the contents of uric acid, urea and urine creatinine in the M cell treatment group were significantly lower than those in the surgery+solvent group. It showed that the M cells had a protective effect on renal injury in rats and improve renal function. After the transplantation of M cells, the urine volume and uric acid of the nephrectomy rat model recovered to a certain extent in the early stage. It indicated that the M cells had a certain therapeutic effect in the early recovery of nephrectomy.

(3) Distribution of BMSCs

Experimental method: Presence of BMSCs in kidney tissue: Fresh lung and kidney tissues of each group were frozen and serially sectioned (8 μm thick), and the Hoechst33342-labeled BMSCs were observed by fluorescence microscope. If they exist, they should show blue fluorescence.

Experimental results: On the 57th day after the modeling, a large amount of blue fluorescence was observed in the lungs of the rats in the M cell group, and a small amount of blue fluorescence was uniformly dispersed in the renal cortex and renal medulla, indicating that the M cells could be localized to the kidney after transplantation, and had a repairing function for kidney damage. It could be expected that the M cells also had repair and therapeutic activities for kidney injury-related diseases (e.g., renal fibrosis, renal failure, etc.).

(4) Histomorphological observation

Experimental methods: After routine HE and Masson staining, 30 glomeruli and 20 cortical fields of 100 magnifications were observed in each section, which was completed by another pathologist according to the principle of blinding. Semi-quantitative methods were used to calculate the glomerular sclerosis index and tubulointerstitial injury index; the glomerular sclerosis was defined as obliteration or hyalinization of focal or glomerular capillary loops, tubular interstitial injury was defined as inflammatory cell infiltration, tubular atrophy/compensatory dilation, and interstitial fibrosis.

Experimental results: The HE and Masson staining results showed that the residual kidney tissue in the surgery+solvent group showed extensive glomerular hypertrophy, segmental sclerosis or global sclerosis in some glomeruli, turbidity and vacuolar degeneration of renal tubular epithelial cells, and some renal tubules showed obvious expansion or atrophy; a large number of inflammatory cells infiltrated, focal interstitial edema or fibrosis in renal interstitium, while the pathological changes in the M cell treatment group were significantly improved. Further calculations found that the M cells could effectively reduce the glomerular sclerosis index and the tubulointerstitial injury index.

Example 41: Evaluation of therapeutic activity of M cells against neuropathic pain CCI

Pain is an unpleasant feeling that people experience frequently throughout their lives, and its occurrence provides the body with an alert signal that it is threatened. On the other hand, it is the most common symptom of various diseases, and neuropathic pain is an intractable pain state caused by damage or abnormality of the nervous system. Neuropathic pain such as neuropathy is a pain that occurs in nerve tissue. This kind of pain is mainly based on the pathological changes of the nerves, and the characteristics of neuropathic pain are paroxysmal. Sometimes local pain is felt, but there is no pain when pressing. This is the characteristics of neuropathic pain. The general treatment of neuropathic pain is mainly the use of drugs for nourishing nerves and drugs for pain relief, preferably under the guidance of a doctor. In this example, the purpose of treating neuropathic pain is achieved by transplanting the M cells.

1. Experimental method:

Experimental animals: SD rats, male, 6 weeks old, purchased from Beijing Weitong Lihua Company. All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and P5 generation was used for subsequent experiments.

| Reagent/Instrument | Manufacturer | Cat. No./Model |
|---|---|---|
| Disposable sterile syringe 20ml | Jiangsu Zhiyu Medical Equipment Co., Ltd. | None |
| Disposable sterile syringe 5ml | Jiangsu Zhiyu Medical Equipment Co., Ltd. | None |
| DMSO | Sigma | D2650-100ML |
| Normal saline | Shijiazhuang No.4 Pharmaceutical Co., Ltd. | None |
| Rotarod Rotarometer | Panlab, Spain | Panlab, Spain |
| Von frey hair pain tester | Youcheng Biotechnology Co., Ltd. | 37450 |

Animal modeling:

1. Anesthetization was performed by intraperitoneal injection of sodium pentobarbital, 40 mg/kg.

2. The right hind limb was fixed horizontally, the femur was taken as the reference, and an incision in the middle of the thigh was made.

3. The sciatic nerve was exposed to the trifurcation at the distal end of the mid-thigh, and the connective tissue was separated. The 3 peripheral branches of the sciatic nerve (sural, common peroneal, and tibial nerves) were exposed without distraction to the neural structures.

4. The minimally invasive forceps was used to gently place no. 5 surgical thread under the common peroneal and tibial nerves.

5. The common peroneal and tibial nerves were ligated (avoid pulling the nerves or touching the sural nerve with surgical equipment).

6. Muscle and skin were sutured separately; ligation was not performed in the sham group, and other operation steps were the same as above.

7. The animals were divided into sham group, surgery+solvent group, surgery+M cell group.

8. The injection was given on the day of the surgery, which was recorded as day 1. The M cells were injected on the days 1, 5, 8, 12, 14, 19, 21 respectively, and the administration method was as follows: $2 \times 10^6$ M cells were intramuscularly injected at the modeling site (injected at four points, $5 \times 10^5$ M cells at each point). Von Frey hair test and foot-weighing test were performed on day 14, and Von Frey hair test was performed on day 21. On the day 21, sampling was performed and blood was collected.

Test I:

Experimental method: Mechanically induced pain test: the 50% withdrawal reflex threshold was calculated by the up-down method. The mid-plantar part of the left hind limb of the rat was vertically stimulated with von frey hair for a duration of $\leq 4$ s, and it was deemed as a positive response when the rat lifted foot or licked foot, otherwise it was a negative response. The measurement started from the minimum stimulus intensity. When the stimulus of this intensity could not cause a positive response, the stimulus of the adjacent higher intensity was given; when there was a positive response, the stimulus of the adjacent lower intensity was given; such proceeding was continued until the first overturn of positive response and negative response occurred; and then the measurement was continuously carried out for 4 times, and the average value thereof was taken as the threshold. The test was performed before surgery, and on the days 5, 8, 14 and 21 after molding.

Experimental results: The results showed that the tolerance of the rats in the surgery+solvent group to the mechanically induced pain was significantly lower than that of the sham group, indicating that the model was successfully constructed, and the model rats were intolerant to the mechanically induced pain. The tolerance degree of the rats in the M cell treatment group was significantly increased, indicating that the M cell treatment had a significant promoting effect on the tolerance degree to the mechanically induced pain in neuropathic pain model rats.

Test II:

Experimental method: Cold allodynia test: Cold allodynia test was performed using cold stimulation caused by the volatility of acetone. By using a syringe, a drop of approximately 0.5 mL of acetone was placed on the lateral sole of the model where the operation was performed. The responses of the test animals were observed and scored according to the responses of the animals. The scoring rules were as follows: (1) no obvious reaction, 0 points; (2) frightened or shocked, but no paw withdrawal, 1 point; (3) significant paw withdrawal, 2 points; (4) paw withdrawal lasting for 5 to 30 seconds, and paw licking phenomenon, 3 points; (5) paw withdrawal lasting for more than 30s, or squawking, 4 points. The test was performed before surgery and on the day 20 after molding.

Experimental results: The results showed that the tolerance of the rats in the surgery+ solvent group to the cold allodynia was significantly lower than that in the sham group, indicating that the model was successfully constructed, and the model rats were intolerant to the mechanically induced pain. The tolerance of the rats in the M cell treatment group to the cold allodynia was significantly increased, indicating that the M cell treatment had a significant promoting effect on the tolerance to the cold allodynia in the neuropathic pain model rats.

Test III:

Experimental method: Motor coordination performance test: The neurological function and motor coordination performance of the animals were evaluated by the rotarod motor test. The test animals were placed on a cylindrical rotarod with a uniformly increased rotation speed. After 30s of adaptation, the rotation speed of the rotarod was slowly increased from 5 r/min to 40 r/min. There were partitions between the test animals to separate each animal, and the testing instrument could test 5 animals at the same time without affecting each other. If the subject animal fell on the metal plate of the instrument, the instrument could automatically stop the time recording. The maximum recording time was 5 minutes, and if it exceeded 5 minutes, it was recorded as 5 minutes. All test animals were tested 3 times, with an interval of 1h for each time. The average value of the 3 tests was taken as the holding time on the rotarod. The test was performed before surgery and on the day 20 after molding.

Experimental results: The results showed that the time for the rats in the surgery+solvent group to maintain balance on the rotarod was significantly shortened, indicating that the model was successfully constructed, and the motor coordination ability of the rats was significantly affected. Compared with the solvent group, the time of maintaining balance on the rotarod in the M cell treatment group was significantly longer, indicating that the M cell treatment had a significant promoting effect on maintaining the motor coordination ability of the rats in the model group.

Test IV:

Experimental method: ELISA test: The rats in all experimental groups were killed immediately after the last behavioral test, and the muscles at the surgical site were removed (length: width: thickness = 2cm: 1em: 0.5em), and an appropriate amount of normal saline was added, homogenized, and centrifuged at 3000rmp/min for 15min. After the treatment, the inflammatory factors IL-1B, IL-10 and IL-17 were determined, and the experimental steps were performed according to the instructions of the kit (Beijing Yaanda Biotechnology Co., Ltd.).

Experimental results: The content of inflammatory factors of the mice in the surgery+solvent group was significantly higher than that in the sham group, indicating that the model was successfully constructed and there was an inflammatory response in the rats. The content of inflammatory factors in the M cell treatment group was significantly lower than that in the solvent group, indicating that the M cell treatment had a significant inhibitory effect on the inflammatory response of rats with neuropathic pain. The M cells played a significant role in improving the symptoms of neuropathic pain and inhibiting the inflammatory response.

The above results showed that after transplantation of the M cells, the coordination and movement ability of rats with neuropathic pain were significantly improved, and the pain tolerance ability to the mechanical force and abnormal cold stimuli were improved. The M cells could suppress the inflammatory response in rats with neuropathic pain. In conclusion, the M cells had a certain therapeutic effect on neuropathic pain.

Example 42: Evaluation of therapeutic activity of M cells against multiple sclerosis

Experimental animals: 7 to 8 week old C57BL/6 female mice (SPF grade), with body weight between 18 to 19.5 g, the C57BL/6 female mice were purchased from Beijing Weitong Lihua Company.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The M cells at P3 generation were resuscitated, digested and passaged, and P5 generation was used for subsequent experiments.

| Reagent/Equipment | Manufacturer | Cat. No. |
|---|---|---|
| Upright phase contrast microscope | Carl Zeiss | Axioscope5 |
| MOG35-55 | GL Biochem | 51716 |
| Mycobacterium tuberculosis H37Ra | Difco Laboratories | 231141 |
| PTX | List Biological Laboratories | 180) |
| CFA | Sigma-Aldrich | F5881 |
| Embedding machine | Leica | EG1150H/C |
| Sectioning machine | Leica | RM2235 |
| Section displaying machine | Leica | HI1210 |
| Disposable sterile syringe 1ml | Jiangsu Zhiyu Medical Equipment Co., Ltd. | None |
| Normal saline | Shijiazhuang No.4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |

Induction of EAE model:

To 200 μl of CFA, 200 μg of MOG35-55 and 200 μg of *Mycobacterium tuberculosis* H37Ra were added, mixed well, and injected subcutaneously on the right and left posterior sides of C57BL/6 mice. On the day of immunization and 2 days after immunization, each mouse received 200 ng of PTX (dissolved in normal saline) by intraperitoneal injection. The mice were divided into 3 groups: normal group, model group, M cell group, with 15 mice in each group, and samples were collected on the 30th day.

The normal group did not receive any treatment, and the model group and the M cell group received different treatments after modeling. The M cell group was injected with 200 μl of normal saline containing $3\times10^6$ M cells on the days 9, 11 and 13 via the tail vein; the model group was injected with 200 μl of normal saline through the tail vein.

EAE scoring criteria:

From day 1, the mice were scored daily for clinical signs of EAE. As follows: 0, no clinically showed disease; 1, flaccid tail without hindlimb weakness; 2, hindlimb weakness; 3, complete hindlimb paralysis and floppy tail; 4, hindlimb paralysis with soft tail and urinary or fecal incontinence; 5, moribund.

Sample collection

When collecting specimens, the mice were placed in supine position after intraperitoneal anesthesia, the skin was cut in the middle of the abdomen of the mice, the chest was opened, the heart was exposed, and the heart was perfused with ice-cold normal saline. Each mouse needed about 20ml of normal saline and 20ml of paraformaldehyde. After the perfusion was completed, the spinal cords of the mice were taken and fixed in paraformaldehyde for subsequent section and identification.

Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Fast Blue staining

1. Paraffin sections of 5 to 8 $\mu$m were dewaxed to water.

2. The sections entered into and were slightly washed with 95% ethanol.

3. Stained in Luxol Fast Blue staining solution at room temperature.

4. Rinsed with 95% ethanol to remove excess staining solution, and rinsed with distilled water.

5. Subjected to color separation in differentiation solution, entered in 70% ethanol to perform color separation until the cinereum matter was clear.

6. Rinsed with distilled water (if the color separation is insufficient, repeat steps 4 to 5).

7. Counterstained and washed.

8. Subjected to conventional dehydration, transparentized with xylene, and mounted with neutral resin.

Immunofluorescence staining:

(1) The tissue sections were dewaxed to water;

(2) Subjected to antigen microwave retrieval at a temperature of 92°C to 96°C, for 10 to 15min, and naturally cooled to room temperature;

(3) Blocked with normal goat serum, 37°C, 60 min;

(4) The excess serum was poured off, the primary antibody was added dropwise, allowed to stand at 37°C for 2 hours or at 4°C overnight, rinsed with PBS, 5min×3 times;

(5) The fluorescein-labeled secondary antibody was added dropwise, protected from light, allowed to stand at 37°C, 60 min, rinsed with 0.01M PBS, 5 min×3 times;

(6) The sections were mounted with anti-quenching mounting medium at 4°C, and stored in the dark.

(7) Fluorescence microscope was used for observation and photoing.

Extraction of total protein from animal tissue

1. The centrifuge tube column and receiver tube cannula were pre-cooled on ice.

2. 15 to 20 mg of tissue was placed on a centrifuge tube column, twisted and ground 50 to 60 times with a plastic stick, added with 200 $\mu$l of cell lysis solution, and continued to grind 30 to 60 times.

3. It was covered with a lid, and incubation was carried out at room temperature for 1 to 2 minutes, then centrifugation was carried out at 14000 to 16000rpm for 2 minutes.

4. The collection tube was immediately placed on ice and the centrifuge tube column was discarded. After the protein extraction was completed, it was cryopreserved in a -80°C refrigerator and could be used in downstream experiments.

Detection of factor secretion by suspension chip system

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C.

(2) The cryopreserved sample was taken from the -80°C refrigerator. After thawing, 0.5 % BSA (w/v) was added to the sample for dilution.

(3) The Bio-Plex system was calibrated with Bio-Plex Manager TM.

(4) To the standard bottle, 250 $\mu$L of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1 time with Bio-Plex detection buffer, and stored in the dark.

(7) The diluted magnetic beads were vortexed, and 50 $\mu$L of the magnetic beads was added to each well.

(8) The plate was washed twice with 100 μL of washing solution.

(9) The sample, standard, blank control and the control with known concentration were vortexed, and added in an amount of 50 μL to each well.

(10) The plate was sealed with a sealing film, and shaken on a high-frequency shaker at 850±50 rpm at room temperature for 30 min.

(11) In step (10), when the remaining 10 min of shaking time was left, the detection antibody was vortexed for 5 s and diluted to 1 time.

(12) The plate was washed twice with 100 μL of washing solution.

(13) The diluted antibody was vortexed, and added in an amount of 250 μL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1 time.

(17) The plate was washed twice with 100 μL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 μL of detection solution, the plate was sealed with a sealing film, and shaken on a high-frequency shaker at room temperature at 850±50 rpm for 30 s.

(22) After the sealing film was discarded, loading to machine was started.

Conclusions:

It could be seen from the score results of EAE that the clinical score of the mice in the model group reached 4 points on the 15th day, and then stabilized, while the score of the M cell treatment group was only 2 points on the 15th day, and it had been recovering since then, when the sampling was collected on the 30th day at the end of the experiment, the score was only 0 to 1. It indicated that the tail vein injection of the M cells had obvious therapeutic effect on EAE mice, so the M cells had a good therapeutic effect on multiple sclerosis.
(Liming Du, et. al, 2019, Cell Metabolism; Lianhua Bai, et. al, 2012, Nature neuroscience)

It could be seen from the HE staining and Fast Blue staining of spinal cord sections (referred to Du, et.al, 2019, Cell Metabolism; Dang, et. al, 2014, Autophagy; Bai, et. al, 2012, Nature neuroscience) that after the M cell treatment, the spinal cord demyelination was significantly reduced, and there was a significant difference compared with the model group. It indicated that the M cells could reduce spinal cord demyelination very well and had a good application in multiple sclerosis.

It could be seen from the immunofluorescence images of spinal cord sections that after the M cell treatment, the proportion of CD4+ T cells was significantly decreased compared with the model group, indicating that the M cells could inhibit inflammation in the spinal cord and had a good therapeutic effect on relieving the spinal cord segmental inflammation of the EAE mice.

Compared with the model group, the ratio of GFAP decreased by 15%, A2B5 increased by 10%, O4 increased by 30%, and β-tubulin increased by 30% in the M cell group, indicating that the M cell treatment could reduce the number of astrocytes, increase the proportion of oligodendrocytes, and suggesting that the M cells could effectively treat mice with EAE disease and reduce demyelination in mice.

The multi-factor kit was used to detect the proteins in the spinal cord segment of rats, and it was found that after the M cell treatment, the content of proinflammatory factors was significantly decreased, the content of anti-inflammatory factors was significantly increased, and the content of nutritional factors was significantly increased. Among them, the decrease of IFN-$\gamma$, IL-17, TFN-$\alpha$, and IL-2 was particularly significant. The content of anti-inflammatory factor IL-10 was significantly increased. It showed that the M cells could inhibit inflammation, regulate the microenvironment of the spinal cord, and provide evidence for the M cells in the treatment of multiple sclerosis.

Conclusion: The M cell treatment could reduce the clinical score of EAE mice, reduce the demyelination of the spinal cord, inhibit the proliferation of astrocytes, protect oligodendrocytes, and reduce the content of proinflammatory factors in spinal cord segments. The above results indicated that the M cells could have an effective therapeutic effect on EAE disease mice.

Example 43: Evaluation of therapeutic activity of M cells against pneumoconiosis

Pneumoconiosis is a systemic disease mainly manifested by diffuse fibrosis (scar) of lung tissue caused by long-term inhalation of productive dust (ash) during occupational activities and retention thereof in the lungs. Pneumoconiosis can be divided into inorganic pneumoconiosis and organic pneumoconiosis according to the type of inhaled dust. Pneumoconiosis caused by inhalation of inorganic dust in production labor is called inorganic pneumoconiosis. Most of the pneumoconiosis is inorganic pneumoconiosis. Pneumoconiosis caused by inhalation of organic dust is called organic pneumoconiosis, such as cotton pneumoconiosis and farmers' lung.

Pneumoconiosis is a progressive chronic disease. Unlike acute infectious diseases or other chronic diseases (e.g., tuberculosis, hypertension, diabetes, etc.), in which obvious therapeutic effects can be seen in a short period of time, it generally requires long-term treatment for several years to obtain more obvious curative effect.

A large number of animal experiments have shown that MSCs transplantation in the treatment of pneumoconiosis shows good efficacy and safety. However, the clinical application of adult tissue-derived MSCs mainly has the following shortcomings: (1) the therapeutic amount of adult tissue-derived MSCs can hardly be obtained from a single individual tissue; (2) the adult tissue-derived MSCs from different individual tissues can hardly achieve high consistency of product quality; (3) even MSCs derived from the same individual tissue are highly heterogeneous; (4t The donor tissue sources of adult tissue-derived MSCs are complex and have potential infectious pathogen infection risks; (5) the adult tissue-derived MSCs rapidly senesce with in vitro expansion. Therefore, new sources of MSC cells are needed for the treatment of pneumoconiosis.

Related documents:

(1) CT/NIRF duaL-modal imaging tracking and therapeutic efficacy of transplanted mesenchymal stem cells labeled with Au nanoparticles in silica-induced pulmonary fibrosis.

(2) Therapeutic effects of adipose-tissue-derived mesenchymal stromal cells and their extracellular vesicles in experimental silicosis.

(3) Transplantation of adipose-derived mesenchymal stem cells attenuates pulmonary fibrosis of silicosis via anti-inflammatory and anti-apoptosis effects in rats.

Object: To overcome the lack of cell sources for stem cell treatment of systemic lupus erythematosus.

Achieved effect: After transplantation of the M cells, the increase rate of anti-double-stranded DNA antibodies in serum was slowed down, and the disease process was slowed down.

Experimental animals: C57 mice, male, 6 to 8 weeks old. The animals were purchased from Beijing Weitong Lihua.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was 22±2°C, relative humidity was 50% to 60%.

Preparation and culture of M cells: The embryonic stem cells were suspended with EB spheres for adherent differentiation, the M cells at P0 generation were obtained, passaged and screened, and cryopreserved at P3 generation for subsequent experiments.

The P3 generation M cells were resuscitated, digested and passaged, and used for subsequent animal experiments.

| Reagent/Equipment | Manufacturer | Cat. No. |
|---|---|---|
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Water bath | Saiou Huachuang | SDY-1 |
| Normal saline | SSY Group Limited | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |
| Multifactor suspension chip system | Bio-Rad | Bio-Plex® 200 |
| 23-Factors kit | Bio-Rad | M60009RDPD |
| Silica (1 to 5μm) | Sigma | S5631 |
| Collagen I antibody | Miltenyi Biotec | GB13091 |
| α-SMA antibody | Servicebio | GB13044 |
| Immunohistochemistry kit | KIT-9710 | Fuzhou Maixin |
| R540 Enhanced small animal anesthesia machine | Ruiwode | R540 |
| Pulmonary function meter | Beijing Guangyuanda Technology Development Co., Ltd. | SCIREQ-FV-FXM2-FEV1 |
| Small animal CT | GE | PE Quantum FX |
| Masson staining solution | Nanjing Jiancheng | D026-1-2 |

Construction of animal model:

Control group: sham operation was performed, and 1 mL of normal saline was instilled through the neck trachea;

Model group: 1 mL of 80% silica suspension was instilled through the neck trachea, and 100 μL of normal saline was injected into the tail vein on days 7 and 21 after modeling;

M cell group: 1 mL of 80% silica suspension was instilled through the neck trachea, and $3 \times 10^6$ cells/100 μL/mouse were injected into the tail vein on the days 7 and 21 after modeling.

Sample collection:

On the day 28 after modeling, the experiment was over, and samples were collected. After intraperitoneal anesthesia, the mice were placed in a supine position, the skin was cut in the middle of the abdomen of the mice, the abdominal cavity was opened, and blood was collected from the central vein. The chest was opened, the heart was exposed, and the heart was perfused with ice-cold normal saline. After the normal saline perfusion was completed, the fixation was performed with 50 mL of paraformaldehyde. After the perfusion was completed, the lungs were taken, fixed, sectioned and analyzed. The collected blood was centrifuged at 5,000 rpm for 15 min at room temperature, and the supernatant was collected for ELISA analysis.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 23-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex Manager TM.

(4) To the standard bottle, 250 μL of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1 time with Bio-Plex detection buffer, and stored in the dark.

(7) The diluted magnetic beads were vortexed, and 50 μL of the magnetic beads was added to each well.

(8) The plate was washed twice with 100 μL of washing solution.

(9) The sample, standard, blank, and control of known concentration were vortexed, and added in an amount of 50 μL to each well.

(10) The plate was sealed with a sealing film, and shaken on a high-frequency shaker at 850±50 rpm at room temperature for 30 min.

(11) In step (10), when the remaining 10 min of shaking time was left, the detection antibody was vortexed for 5 s and diluted to 1 time.

(12) The plate was washed twice with 100 μL of washing solution.

(13) The diluted antibody was vortexed, and added in an amount of 250 μL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1 time.

(17) The plate was washed twice with 100 μL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 μL of detection solution, the plate was sealed with a sealing film, and shaken on a high-frequency shaker at room temperature at 850±50 rpm for 30 s.

(22) After the sealing film was discarded, loading to machine was started.

Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 μm of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Immunohistochemical staining:

Immunohistochemical staining was performed on the paraffin sections using an immunohistochemical kit (Fuzhou Maixin, KIT-9710). The specific steps were as follows:

1. Dewaxing: (1) xylene I, II, 10 min each; (2) gradient alcohol: 100% absolute ethanol, 2 min; 95% absolute ethanol, 2 min; 80% absolute ethanol, 2 min; 70% absolute ethanol, 2 min;

2. Hydration: washing was performed twice with distilled water, 5min each time (placed on a shaker);

3. After deparaffinization and hydration of paraffin sections, rinsing was performed 3 times with PBS, 3 minutes each time;

4. Preparation of antigen retrieval solution (10 mM pH 6.0 sodium citrate buffer):

(1) Preparation of stock solution: Solution A: 29.41 g of trisodium citrate dihydrate + 1,000 mL of distilled water; Solution B: 21 g of citric acid + 1,000 mL of distilled water;
(2) Preparation of working solution: 82 mL of Solution A + 18 mL of solution B + 900 mL of distilled water;

5. Antigen retrieval: the sections were placed in a plastic or heat-resistant glass container filled with sodium citrate buffer, the sections were immersed, treated with a microwave oven at mid-range or high-range power for 5 minutes; sodium citrate buffer was replenished, and treatment was performed again at mid-range or high-range power for 5 minutes;

6. Reagent A (peroxidase blocking solution) was added, and incubated at room temperature for 10 min to block the activity of endogenous peroxidase; rinsing was performed with PBS 3 times, 3 min each time;

7. PBS was discarded, 1 drop or 50 $\mu$L of Reagent B (normal non-immune animal serum) was added, and incubated at room temperature for 10 min;

8. The serum was discarded, 1 drop or 50 $\mu$L of primary antibody was added, and incubated at 4°C overnight or at room temperature for 60 min; rinsing was performed with PBS 3 times, 3 min each time;

9. The PBS was discarded, 1 drop or 50 $\mu$L of biotin-labeled secondary antibody (Reagent C) was added, and incubated at room temperature for 10 min; rinsing was performed with PBS 3 times, 3 min each time;

10. The PBS was discarded, 1 drop or 50 $\mu$L of streptavidin-peroxidase solution (reagent D) was added, incubated at room temperature for 10 min; rinsing was performed with PBS 3 times, 3 min each time;

11. The PBS was discarded, 2 drops or 100 $\mu$L of freshly prepared DAB solution was added, and observation was performed under microscope for 3 to 10 min;

12. Rinsing was performed with tap water, counterstaining was carried out with hematoxylin, and rinsing was performed with PBS or tap water so as to return to blue;

13. When using DAB for color development, the sections should be dehydrated with gradient alcohol and dried, transparentizing was performed with xylene, and mounting was performed with neutral resin;

14. Photos were taken with a microscope.

Masson staining

(1) Dewaxing paraffin sections to water: The sections were placed in xylene I for 20 minutes, xylene II for 20 minutes, anhydrous ethanol I for 10min, anhydrous ethanol II for 10min, 95% alcohol for 5min, 90% alcohol for 5min, 80% alcohol for 5min, 70% alcohol for 5min in sequence, and washed with distilled water.

(2) Hematoxylin staining of nuclei: staining was performed for 5 min with Weigert's iron hematoxylin in the Masson staining kit; after being washed with tap water, differentiation was performed with 1% hydrochloric acid-alcohol for several seconds, rinsing was performed with tap water, and returning to blue was achieved by rinsing with running water for several minutes.

(3) Ponceau red staining: staining was performed for 5 to 10 min with Ponceau red acid fuchsin solution in the Masson staining kit, and rinsing was quickly performed with distilled water.

(4) Phosphomolybdic acid treatment: the treatment with phosphomolybdic acid aqueous solution in the Masson staining kit was performed for about 3 to 5 min.

(5) Aniline blue staining: instead of washing with water, counterstaining was performed for 5 min with aniline blue solution in the Masson staining kit.

(6) Differentiation: the treatment with 1% glacial acetic acid was performed for 1 min.

(7) Dehydration and mounting: the sections were placed in 95% alcohol I for 5min, 95% alcohol II for 5min, absolute ethanol I for 5min, absolute ethanol II for 5min, xylene I for 5min, xylene II for 5min in sequence to perform dehydration and transparentizing, then the sections were taken out from xylene and slightly air-dried, and mounted with neutral resin.

(8) Microscopic examination was performed with a microscope, and images were acquired and analyzed.

Experimental results

(1) The results of body weight monitoring showed that the body weight of the model group was significantly lower than those of the control group and the M cell group; the results showed that M cells could increase the body weight of pneumoconiosis mice.

(2) The statistical results of survival rate showed that the body weight of the pneumoconiosis group was significantly lower than those of the control group and the M cell group; the results showed that M cells could improve the survival rate of pneumoconiosis mice.

(3) The CT results showed that compared with the model group, the M cells could reduce the area of lung compact parts.

(4) The pulmonary function measurement results showed that compared with the model group, the M cells could improve lung functions, including improved vital capacity and maximal ventilation, and reduced airway resistance.

(5) The results of HE staining showed that the lung tissue of the mice in the model group showed typical pneumoconiosis-like changes, various types of inflammatory cell infiltration, thicken alveolar walls, pulmonary interstitial congestion, cellular nodules (granulomas), fibrosis in center of some nodules, or coexistence of cellular nodules of different sizes and fibrous nodules, gradual expansion and fusion of visible nodules that even form into sheets, and significant decrease in alveolar structure. Compared with the model group, the M cells could reduce inflammatory cell infiltration, reduce the appearance of cellular nodules, and maintain the integrity of alveolar structure (FIG. 214).

(6) The results of Masson staining showed that the M cells could reduce the content of pulmonary fibers and inhibit the occurrence of fibrosis (FIG. 88).

(7) The immunohistochemical results showed that the M cells could reduce the expressions of Collagen I and $\alpha$-SMA proteins in the lungs and inhibit the occurrence of fibrosis.

(8) The inflammatory factors in the serum of mice were detected, and the results showed that compared with the pneumoconiosis group, the levels of proinflammatory factors in the M cell treatment group were significantly decreased, and the levels of anti-inflammatory factors were significantly increased. It was shown that the M cells had the effect of suppressing inflammation.

Conclusion: After the injection of M cells in the pneumoconiosis mice, the level of inflammatory factors in serum was reduced, the lung function of mice was improved, the area of lung compact parts was reduced, and there was less fibrosis, showing a good effect in the treatment of pneumoconiosis. At the same time, it had good therapeutic potential for other respiratory diseases (e.g., pulmonary fibrosis, chronic pulmonary obstruction, etc.).

Example 44: Evaluation of therapeutic activity of M cells against chronic pulmonary obstruction

Experimental animals: C57 mice, male, 6 to 8 weeks old. The animals were purchased from Beijing Weitong Lihua.
All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.
Feeding conditions: Free food and water; 12/12h day and night alternation, 7:00 to 19:00 was daytime, room temperature was 22±2°C, relative humidity was 50% to 60%.
Preparation and culture of M cells: The embryonic stem cells were suspended with EB spheres for adherent differentiation, the M cells at P0 generation were obtained, passaged and screened, and cryopreserved at P3 generation for subsequent experiments.
The P3 generation M cells were resuscitated, digested and passaged, and used for subsequent animal experiments.

| Reagent/Equipment | Manufacturer | Cat. No. |
|---|---|---|
| Upright phase contrast microscope | Carl Zeiss | Axioscope5 |

(continued)

| Reagent/Equipment | Manufacturer | Cat. No. |
|---|---|---|
| Embedding machine | Leica | EG1150H/C |
| Sectioning machine | Leica | RM2235 |
| Section displaying machine | Leica | HI1210 |
| Water bath | Saiou Huachuang | SDY-1 |
| Normal saline | SSY Group Limited | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |
| Multifactor suspension chip system | Bio-Rad | Bio-Plex® 200 |
| 23-Factors Kit | Bio-Rad | M60009RDPD |
| Pancreatin | Sigma | E1250 |
| Collagen I antibody | Miltenyi Biotec | GB13091 |
| $\alpha$-SMA antibody | Servicebio | GB13044 |
| Immunohistochemistry kit | Fuzhou Maixin | KIT-9710 |
| R540 Enhanced small animal anesthesia machine | Ruiwode | R540 |
| Pulmonary function meter | Beijing Guangyuanda Technology Development Co., Ltd. | SCIREQ-FV-FXM2-FEV1 |
| Small Animal CT | GE | PE Quantum FX |
| Blood Gas Analyzer | Instrumentation Laboratory | GEM3500 |

Construction of animal model:

Control group: subjected to sham operation, instilled with normal saline through the neck trachea;

Chronic obstructive pulmonary disease (COPD) group: subjected to operation, instilled with 0.05 U/g body weight of pancreatic enzyme through the neck trachea, and injected via tail vein with 100 $\mu$L of normal saline on the day 1 and 7 after modeling;

COPD+M cell group: subjected to operation, instilled with 0.05 U/g body weight of trypsin through the neck trachea, and injected via tail vein with $3\times10^6$ cells/100 $\mu$L/mouse on the day 1 and 7 after modeling.

Sample collection:

On the day 21 after modeling, the experiment was over, and samples were collected. After intraperitoneal anesthesia, the mice were placed in a supine position, the skin was cut in the middle of the abdomen of the mice, the abdominal cavity was opened, and blood was collected from the central vein. The chest was opened, the heart was exposed, and the heart was perfused with ice-cold normal saline. After the normal saline perfusion was completed, the fixation was performed with 50 mL of paraformaldehyde. After the perfusion was completed, the lungs were taken, fixed, sectioned and analyzed. The collected blood was centrifuged at 5,000 rpm for 15 min at room temperature, and the supernatant was collected for ELISA analysis.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 23-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex Manager TM.

(4) To the standard bottle, 250 $\mu$L of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1 time with Bio-Plex detection buffer, and stored in the dark.

(7) The diluted magnetic beads were vortexed, and 50 $\mu$L of the magnetic beads was added to each well.

(8) The plate was washed twice with 100 $\mu$L of washing solution.

(9) The sample, standard, blank, and control of known concentration were vortexed, and added in an amount of 50 $\mu$L to each well.

(10) The plate was sealed with a sealing film, and shaken on a high-frequency shaker at 850$\pm$50 rpm at room temperature for 30 min.

(11) In step (10), when the remaining 10 min of shaking time was left, the detection antibody was vortexed for 5 s and diluted to 1 time.

(12) The plate was washed twice with 100 $\mu$L of washing solution.

(13) The diluted antibody was vortexed, and added in an amount of 250 $\mu$L to each well.

(14) The plate was sealed with a sealing film, and shaken on a high-frequency shaker at 850$\pm$50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1 time.

(17) The plate was washed twice with 100 $\mu$L of washing solution.

(18) The diluted SA-PE was vortexed, and added in an amount of 50 $\mu$L to each well.

(19) The plate was sealed with a sealing film, and shaken on a high-frequency shaker at 850$\pm$50 rpm at room temperature for 30 min.

(20) The plate was washed three times with 100 $\mu$L of washing solution.

(21) The magnetic beads were resuspended with 125 $\mu$L of detection solution, the plate was sealed with a sealing film, and shaken on a high-frequency shaker at room temperature at 850$\pm$50 rpm for 30 s.

(22) After the sealing film was discarded, loading to machine was started.

Steps for tissue paraffin sectioning

(1) Fixation: the tissue was socked in 4% PFA and fix it overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Immunohistochemical staining:

Immunohistochemical staining was performed on the paraffin sections using an immunohistochemical kit (Fuzhou Maixin, KIT-9710). The specific steps were as follows:

1. Dewaxing: (1) xylene I, II, 10 min each; (2) gradient alcohol: 100% absolute ethanol, 2 min; 95% absolute ethanol, 2 min; 80% absolute ethanol, 2 min; 70% absolute ethanol, 2 min;

2. Hydration: washing was performed twice with distilled water, 5min each time (placed on a shaker);

3. After deparaffinization and hydration of paraffin sections, rinsing was performed 3 times with PBS, 3 minutes each time;

4. Preparation of antigen retrieval solution (10 mM pH 6.0 sodium citrate buffer):

(1) Preparation of stock solution: Solution A: 29.41 g of trisodium citrate dihydrate + 1,000 mL of distilled water; Solution B: 21 g of citric acid + 1,000 mL of distilled water;

(2) Preparation of working solution: 82 mL of Solution A + 18 mL of solution B + 900 mL of distilled water;

5. Antigen retrieval: the sections were placed in a plastic or heat-resistant glass container filled with sodium citrate buffer, the sections were immersed, treated with a microwave oven at mid-range or high-range power for 5 minutes;

sodium citrate buffer was replenished, and treatment was performed again at mid-range or high-range power for 5 minutes;

6. Reagent A (peroxidase blocking solution) was added, and incubated at room temperature for 10 min to block the activity of endogenous peroxidase; rinsing was performed with PBS 3 times, 3 min each time;

7. PBS was discarded, 1 drop or 50 µL of Reagent B (normal non-immune animal serum) was added, and incubated at room temperature for 10 min;

8. The serum was discarded, 1 drop or 50 µL of primary antibody was added, and incubated at 4°C overnight or at room temperature for 60 min; rinsing was performed with PBS 3 times, 3 min each time;

9. The PBS was discarded, 1 drop or 50 µL of biotin-labeled secondary antibody (Reagent C) was added, and incubated at room temperature for 10 min; rinsing was performed with PBS 3 times, 3 min each time;

10. The PBS was discarded, 1 drop or 50 µL of streptavidin-peroxidase solution (reagent D) was added, incubated at room temperature for 10 min; rinsing was performed with PBS 3 times, 3 min each time;

11. The PBS was discarded, 2 drops or 100 µL of freshly prepared DAB solution was added, and observation was performed under microscope for 3 to 10 min;

12. Rinsing was performed with tap water, counterstaining was carried out with hematoxylin, and rinsing was performed with PBS or tap water so as to return to blue;

13. When using DAB for color development, the sections should be dehydrated with gradient alcohol and dried, transparentizing was performed with xylene, and mounting was performed with neutral resin;

14. Photos were taken with a microscope.

Experimental results

(1) The CT results showed that compared with the model group, the M cells could reduce the area of lung parts.

(2) The pulmonary function measurement results showed that compared with the model group, the M cells could improve lung function, including improved vital capacity and maximum ventilation, and reduced airway resistance.

(3) The HE staining results showed that compared with the model group, the M cells could reduce the mean intercept of alveoli and better maintain the structural integrity of the lungs.

(4) The results of blood gas analysis showed that compared with the model group, the M cells could increase the partial pressure of oxygen in arterial blood.

(5) The inflammatory factors in the serum of mice were detected. The results showed that compared with the pneumoconiosis group, the M cells could reduce the level of proinflammatory factors and increase the level of anti-inflammatory factors. It was shown that the M cells had the effect of suppressing inflammation.

(6) The immunohistochemical results showed that the M cells could reduce the expression of Collagen I and $\alpha$-SMA proteins in the lungs and inhibit the occurrence of fibrosis.

Conclusion: After the injection of M cells in chronic pulmonary obstructive mice, the mean alveolar intercept was reduced, the level of inflammatory factors in serum was reduced, the partial pressure of oxygen in arterial blood and pulmonary function of mice were increased, the area of lung compact parts was reduced, and fewer fibrosis was formed, indicating that it had a good effect in the treatment of chronic pulmonary obstruction. At the same time, it had good therapeutic potential for other respiratory diseases (e.g., pulmonary fibrosis, pneumoconiosis, etc.).

Related documents:

(1) CT/NIRF dual-modal imaging tracking and therapeutic efficacy of transplanted mesenchymal stem cells labeled with Au nanoparticles in silica-induced pulmonary fibrosis.

(2) Therapeutic effects of adipose to tissue-derived mesenchymal stromal cells and their extracellular vesicles in experimental silicosis.

(3) Transplantation of adipose-derived mesenchymal stem cells attenuates pulmonary fibrosis of silicosis via anti-inflammatory and anti-apoptosis effects in rats.

Example 45: Evaluation of therapeutic activity of M cells against crescentic nephritis

Kidney diseases are kidney-related diseases, mainly including: primary glomerular disease, secondary glomerulonephritis, hereditary kidney disease, urinary tract infection kidney disease, renal tubular disease, interstitial nephritis, kidney stones and obstructive nephropathy, cystic kidney disease and kidney tumors, renal vascular disease, kidney-related hypertension, pregnancy-related kidney disease, elderly kidney disease, drug (food)-induced kidney damage, renal failure. Relevant epidemiological data show that the incidence of chronic kidney diseases (CKD) in the Chinese population is about 11 to 13%. Accordingly, there are more than 100 million CKD patients in China. The complex physiological functions of the kidneys and their unique organizational characteristics make them susceptible to damage in many cases.

Nephritis refers to the non-suppurative inflammatory lesions of both kidneys, with nephritis phenomena such as edema, hypertension, proteinuria due to damaged renal corpuscles, and is the most common type of kidney disease. According to etiology, nephritis can be divided into secondary and primary glomerulonephritis. Primary glomerulonephritis accounts for 0.67 to 0.8% of all hospitalized patients. Secondary glomerulonephritis is caused by other diseases (e.g., diabetes, hypertension, systemic lupus erythematosus, allergic purpura, vasculitis, etc.), and is a systemic disease involving the kidneys. According to clinical classification, nephritis can be divided into acute, chronic and rapidly progressive nephritic syndromes, latent nephritis (asymptomatic hematuria and/or proteinuria). Chronic nephritis includes mesangial proliferative glomerulonephritis, focal segmental glomerulosclerosis, membranous nephropathy, mesangial capillary glomerulonephritis, and sclerosing nephritis. The pathological changes of rapidly progressive nephritis are characterized by the formation of crescents in the glomeruli, also known as crescentic nephritis.

Crescentic nephritis is also known as rapidly progressive nephritis. Crescentic nephritis is a general term for a group of glomerulonephritis that develops rapidly, with hematuria, proteinuria, edema, and hypertension as the main clinical manifestations, and rapidly develops into oliguria, anuria and renal failure with poor prognosis. According to the etiology, it can be divided into two categories: primary and secondary. Among them, primary crescentic nephritis can be divided into anti-glomerular basement membrane antibody type, immune complex type, and type with unknown pathogenesis. Secondary crescentic nephritis may be caused by primary glomerular diseases, such as membranous proliferative nephritis, membranous nephropathy, IgA nephropathy (less common), etc.; secondary to infectious diseases: such as infective endocarditis, nephritis after streptococcal infection, occult organ bacterial lesions, hepatitis B and influenza, etc.; secondary to other systemic diseases: such as systemic lupus erythematosus, systemic vasculitis, pulmonary hemorrhage-nephritic syndrome, allergic purpura, spontaneous cryoglobulinemia, malignant tumor and relapsing polychondritis.

Crescentic nephritis is the most serious glomerular disease in nephrology. The disease progresses rapidly, with rapid progression and poor prognosis. The clinical manifestations are rapidly progressive glomerulonephritis, which rapidly progresses to uremia, and the pathological manifestation is massive crescent formation. More than half of the patients are complicated with diffuse alveolar hemorrhage, which can lead to suffocation and death due to massive hemoptysis. At present, the most effective treatment method is the combined treatment of plasma exchange, glucocorticoid and cyclophosphamide, which is the strongest treatment for kidney disease, and requires a lot of precious blood resources and great expenses. Even so, the one-year survival rate of patients is only 70 to 80%, and the one-year survival rate of kidney is only 20 to 30% [Cui, Z. and M.H. Zhao, Nat Rev Nephrol, 2011. 7(12): 697]. Most patients depend on lifetime dialysis or receive kidney transplantation. The low quality of life of patients and heavy medical burden are important reasons for the "impoverishment due to illness, and return to poverty due to illness" in patients with kidney diseases. Therefore, new breakthroughs in the treatment of this disease are urgently needed to reduce renal tissue damage and promote cellular repair and structural reconstruction.

Crescent nephritis is a typical autoimmune kidney disease and an ideal disease model to study the pathogenesis of immune inflammation in glomerular diseases. Its characteristic feature is that the anti-basement membrane (GBM) autoantibodies in the patient's peripheral blood is detected, and the antibody is seen as a line-like deposition on the glomerular basement membrane of the kidney tissue. At the same time, a variety of inflammatory cells and complement systems are also involved in the occurrence and development of the disease, and various cytokines secreted by these cells are involved in the regulation of the disease.

Mesenchymal stem cells (MSCs) have not only the potential of multi-directional differentiation, but also the function of secreting bioactive factors and immune regulation, and thus have great potential to reduce renal tissue damage, promote cell repair and structural reconstruction. Furuhashi et al. found that the adipose-derived MSCs cultured with less serum could effectively alleviate the progression of rat crescentic nephritis [Furuhashi, K., et al, J Am Soc Nephrol, 2013. 24(4): 587], Suzuki et al found that the bone marrow-derived MSCs can effectively alleviate the condition of anti-GBM disease in rats [Suzuki, T., et al., PLoS One, 2013. 8(6): e67475].

Animal model:

Experimental animals: WKY rats, male, 4 to 5 weeks old, purchased from Beijing Weitong Lihua Company.
All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.
Feeding conditions: Free food and water; 12/12h day and night alternation, 7:00 to 19:00 was daytime, room temperature was $22\pm2°C$, relative humidity was 50% to 60%.
Preparation and culture of M cells: The embryonic stem cells were suspended in EBs for adherent differentiation, and the M cells at P0 generation were obtained, passaged and screened, and cryopreserved at P3 generation for subsequent experiments.
The M cells at P3 generation were resuscitated, digested and passaged, and used at P5 generation for subsequent experiments.
Animal modeling: Each WKY rat was injected with 20ug of anti-GBM pathogenic epitope P14 through the footpad so as to induce the occurrence of the disease. Grouping: normal group, model group, M cell treatment group.
Normal group: subjected to no treatment.
Model group: injected with 300 $\mu$l of normal saline into the tail vein, three times per week.
M cell treatment group: injected with 300 $\mu$l of normal saline containing $3\times10^6$ M cells into the tail vein, three times per week.

Sample collection

When collecting the specimen, the rats were placed in a supine position after intraperitoneal anesthesia, the skin was cut in the middle of the abdomen of the rat, the chest was opened, the heart was exposed, and the heart was perfused with ice-cold normal saline. About 50 ml of normal saline was needed for each rat. Kidney tissue was taken and subjected to subsequent analysis.

Extraction of total protein from animal tissue

1. The centrifuge tube column and receiver tube cannula were pre-cooled on ice.

2. 15 to 20 mg of tissue was placed on a centrifuge tube column, twisted and ground 50 to 60 times with a plastic stick, added with 200 $\mu$l of cell lysis solution, and continued to grind 30 to 60 times.

3. It was covered with a lid, and incubation was carried out at room temperature for 1 to 2 minutes, then centrifugation was carried out at 14000 to 16000rpm for 2 minutes.

4. The collection tube was immediately placed on ice and the centrifuge tube column was discarded. After the protein extraction was completed, it was cryopreserved in a -80°C refrigerator.

Detection of factor secretion by suspension chip system

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C.

(2) The cryopreserved sample was taken from the -80°C refrigerator. After thawing, 0.5 % BSA (w/v) was added to the sample for dilution.

(3) The Bio-Plex system was calibrated with Bio-Plex Manager™.

(4) To the standard bottle, 250 μL of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1× with Bio-Plex detection buffer, and stored in the dark.

(7) The diluted magnetic beads were vortexed, and 50 μL of the magnetic beads was added to each well.

(8) The plate was washed twice with 100 μL of washing solution.

(9) The sample, standard, blank control and the control with known concentration were vortexed, and added in an amount of 50 μL to each well.

(10) The plate was sealed with a sealing film, and shaken on a high-frequency shaker at 850±50 rpm at room temperature for 30 min.

(11) In step (10), when 10 min of shaking time was left, the detection antibody was vortexed for 5 s and diluted to 1×.

(12) The plate was washed twice with 100 μL of washing solution.

(13) The diluted antibody was vortexed, and added in an amount of 250 μL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when 10 min of shaking time was left, SA-PE 5 was vortexed and diluted to 1×.

(17) The plate was washed twice with 100 μL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 μL of detection solution, the plate was sealed with a sealing film, and shaken on a high-frequency shaker at room temperature at 850±50 rpm for 30 s.

(22) After the sealing film was discarded, loading to machine was started.

Steps for tissue paraffin sectioning:

(1) Fixation: the tissue was socked in 4% PFA and fixed overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.
Returning to blue: Returning to blue was performed with tap water for 15 minutes.
Eosin staining: staining was performed for 3 min.
Dehydration and transparentizing: alcohol was used for gradient dehydration, and xylene was used for transparentizing.
Mounting on slides: the sections were mounted with the neutral resin, and air bubbles should be avoided. After the slides were air-dried, they were observed under a microscope.

Results:

Compared with the model group, the weight of kidney of the rats in the M cell treatment group decreased significantly; and it was found from the detection of urine protein and serum creatinine of the rats that after the M cell injection treatment, the urine protein and serum creatinine of the rats decreased significantly as compared with the model group, indicating that the M cells could effectively restore the function of the kidney.
The flow cytometry was used to detect Th1, Th2, Th17 and Treg cells in the spleen and kidney of the rats, and the results showed that after the M cell treatment, Th1, Th2, Th17 were significantly down-regulated, and the proportion of Treg cells was significantly up-regulated, indicating that the M cells could inhibit the inflammation of kidneys, thereby promoting the recovery of the rats.
The results of kidney HE staining of the rats showed that the model group showed severe fibrinoid necrosis of glomeruli, a large number of cell crescents, and partial segmental glomerulosclerosis; while the M cell treatment group did not develop glomerular necrosis, and there was less crescents, indicating that the M cell treatment could inhibit the formation of crescents, had protective effect on the kidneys, and good therapeutic effect against anti-GBM disease.
The immunohistochemistry on kidney tissue sections showed that after the M cell treatment, the proportions of IL-1$\beta$, CD8 and ED1 cells in the kidney tissue were significantly down-regulated, indicating that the M cells could inhibit the inflammation of kidney, thereby promoting the recovery of the rats.
The multi-factor detection results showed that in the M cell treatment group, the proinflammatory factors, such as IFN-$\gamma$, IL-6, TFN-$\alpha$, iNOS, etc., were significantly down-regulated, and the anti-inflammatory factor IL-10 was significantly up-regulated. It was proved that the M cells could inhibit the inflammation in rats with crescentic nephritis, and were beneficial to the recovery of rat kidneys.
In conclusion, the M cell treatment could inhibit inflammation, reduce kidney inflammation in model rats, and promote the recovery of kidney function in rats, indicating that the M cells could effectively treat crescentic nephritis.

Example 46: Evaluation of therapeutic activity of M cells against systemic lupus erythematosus

Systemic lupus erythematosus (SLE) is an autoimmune disease, and its etiology has not yet been determined. A large number of studies have shown that it is related to immune abnormalities. SLE is an autoimmune disease involving multiple organs and systems. It is common in women aged 15 to 40. In addition to skin manifestations, there are also organ involvement, mainly in kidneys, and the clinical manifestations of renal injury are accounted for 45% to 85%. Although traditional treatment methods, such as glucocorticoid combined with immunosuppressive therapy, can effectively improve the long-term survival rate of SLE patients, some patients still have treatment resistance, some patients are ineffective, and there is still a potential risk of death. At present, it is believed that SLE is caused by a variety of factors, leading to the disorder of immune regulation and the occurrence of autoimmune reactions. Studies have shown that the main pathogenesis of SLE is related to the abnormal activation of T and B lymphocytes. In recent years, a large number of studies have shown that MSCs have immunomodulatory effects on T cells, B cells, natural killer cells (NK) and dendritic cells (DC). Therefore, some scholars believe that SLE is a stem cell disease. Therefore, MSCs provide a new perspective for SLE therapy.

A large number of animal experiments have shown that MSCs transplantation shows good efficacy and safety in the treatment of SLE. However, the clinical application of adult tissue-derived MSCs mainly has the following shortcomings: (1) the therapeutic amount of adult tissue-derived MSCs can hardly be obtained from a single individual tissue; (2) the adult tissue-derived MSCs from different individual tissues can hardly achieve high consistency of product quality; (3) even the MSCs derived from the same individual tissue are highly heterogeneous; (4) the donor tissue sources of adult tissue-derived MSCs are complex and have potential infectious pathogen infection risks; (5) the rapid senescence of adult tissue-derived MSCs occurs with in vitro expansion. Therefore, new sources of MSC cells are needed for the treatment of SLE.

Objective: To overcome the lack of cell sources for the stem cell treatment of systemic lupus erythematosus.

Achieved effect: After transplantation of the M cells, the increase rate of anti-double-stranded DNA antibodies in serum was slowed down, and the disease process was slowed down. The present invention has no toxic and side effects, with low mortality rate of mice after treatment, and has obvious therapeutic effect.

Experimental animals: MRL/lpr mice (spontaneously developed lupus erythematosus symptoms, ICR mice were used as background control), female, 8 weeks old. The animals were purchased from Nanjing Junke Biological Engineering Co., Ltd.

All animals were kept at SPF grade in the Experimental Animal Center of the Institute of Zoology, Chinese Academy of Sciences, and were reared adaptively for one week. The care and use of the animals were approved by the Laboratory Animal Center, Institute of Zoology, Chinese Academy of Sciences. All experimental procedures for animals were performed in accordance with the regulations of the Laboratory Animal Welfare and Ethics Committee of the Institute of Zoology, Chinese Academy of Sciences.

Feeding conditions: Free food and water; 12/12h day and night alternation, 7:00 to 19:00 was daytime, room temperature was $22\pm2°C$, relative humidity was 50% to 60%.

Preparation and culture of M cells: The embryonic stem cells were suspended to form EB spheres for adherent differentiation, the M cells at P0 generation were obtained, passaged and screened, and cryopreserved at P3 generation for subsequent experiments.

The P3 generation M cells were resuscitated, digested and passaged, and used for subsequent experiments.

| Reagent/Equipment | Manufacturer | Cat. No. |
|---|---|---|
| Upright phase contrast microscope | Carl Zeiss | Axioscope5 |
| Embedding machine | Leica | EG1150H/C |
| Sectioning machine | Leica | RM2235 |
| Section displaying machine | Leica | HI1210 |
| Water bath | Saiou Huachuang | SDY-1 |
| Normal saline | Shijiazhuang No. 4 Pharmaceutical Co., Ltd. | None |
| Paraformaldehyde | LEAGENE | DF0135 |
| Xylene | Beijing Reagent Co., Ltd. | None |
| Paraffin | Leica | 39601006 |
| Hematoxylin staining solution | Zhongshan Jinqiao | ZLI-9610 |
| Eosin staining solution | Zhongshan Jinqiao | ZLI-9644 |
| Neutral resin | Solebol | G8590-100 |
| Multifactor suspension chip system | Bio-Rad | Bio-Plex® 200 |
| Anti-double-stranded DNA antibody ELISA kit | CUSABIO | CSB-E11194M |

(continued)

| Reagent/Equipment | Manufacturer | Cat. No. |
|---|---|---|
| 23-Factors Kit | Bio-Rad | M60009RDPD |
| PE-IgG 1 Isotype Control | BD | 555749 |
| FITC-IgG 1 Isotype Control | BD | 555748 |
| mCD3 antibody | Miltenyi Biotec | 130-121-133 |
| mCD4 antibody | Miltenyi Biotec | 130-121-131 |
| mCD8 antibody | Miltenyi Biotec | 130-118-329 |

Preparation of animal model: MRL/lpr mice (which could spontaneously develop lupus erythematosus symptoms),

(1) Control group: ICR mice; (2) model group: MRL/lpr+normal saline; (3) treatment group: MRL/lpr+M cells. The mice in the treatment group were injected with $3\times10^6$ cells/mouse via tail vein every two weeks, for a total of 3 injections.

At the 10, 12, 14 and 16 weeks of age, blood was collected from the tail vein of the mice, and ELISA was performed to detect the level of anti-double-stranded DNA antibodies. Serum was collected at 18 weeks of age, and kidneys were collected by perfusion. The samples were soaked in paraformaldehyde overnight, and then paraffin sectioned and stained with HE.

Sample collection:

When collecting specimens, the mice were in a supine position after intraperitoneal anesthesia, the skin was cut in the middle of the abdomen of the mice, the abdominal cavity was opened, and blood was collected from the central vein. The chest was opened, the heart was exposed, and the heart was perfused with ice-cold normal saline. After the perfusion of normal saline was completed, the fixation was performed with 50 ml of paraformaldehyde. After the perfusion was completed, the kidneys were taken, fixed, sectioned and analyzed. The collected blood was centrifuged at 5,000 rpm for 15 min at room temperature, and the supernatant was collected for ELISA analysis.

Detection of anti-double-stranded DNA antibodies in serum by ELISA

(1) Blood was drawn from the tail vein of the mice, centrifuged at 5,000 rpm for 15 min, and the supernatant was taken, which could be stored in a -80°C refrigerator or directly tested;

(2) The kit was balanced at room temperature for 30 min;

(3) Preparation of standards: The standards with gradient concentrations of 20 ng/ml, 10 ng/ml, 5 ng/ml, 2.5 ng/ml, 1.25 ng/ml, 0.625 ng/ml, 0.312 ng/ml, 0 ng/ml were prepared with sample diluent;

(4) The coated ELISA plate was taken, added with 100 μL of sample to be tested and standards respectively, and incubated at 37°C for 2h;

(5) The liquid was discarded and dried by spinning without washing.

(6) 100 μL of biotin-labeled antibody was added to each well and incubated at 37°C for 1 h;

(7) 200 μL of Wash Buffer was added to each well to wash the plate for 5 times;

(8) 100 μL of horseradish peroxidase-labeled antibody was added to each well, and incubated at 37°C for 1 h;

(9) 200 μL of Wash Buffer was added to each well to wash the plate for 5 times;

(10) 90 μL of substrate was added to each well, and incubated at 37°C for 15 to 30 min in the dark;

(11) After the color became blue and stable, 50 μL of stop solution was added to each well to stop the reaction;

(12) The OD value at 450 nm was optically measured with a microplate reader, standard curves were drawn and the content of albumin in the sample was calculated.

Detection of inflammatory factors by suspension chip system:

(1) Bio-Plex 200 was turned on and preheated for 30 minutes. The kit was allowed to stand at room temperature, the diluent, washing solution, detection solution, standard HB, detection antibody diluent HB, sample diluent HB were allowed to stand at room temperature, and other reagents were allowed to stand at 4°C. The 48-factors kit was used for the detection of inflammatory factors.

(2) The cryopreserved cell supernatant was taken from the -80°C refrigerator and placed on ice. After thawing, 0.5 % BSA (w/v) was added to the cell culture supernatant for dilution.

(3) The Bio-Plex system was calibrated with Bio-Plex ManagerTM.

(4) To the standard bottle, 250 μL of standard dilution HB was added, vortexed for 5 s, and immediately incubated on ice for 30 minutes (the time must be precise).

(5) The standard was diluted from S1 to S9, with 4-fold serial dilution; and blank wells were prepared.

(6) The magnetic beads were mixed by vortexing for 30 s, diluted to 1× with Bio-Plex detection buffer, and stored in the dark.

(7) The diluted magnetic beads were vortexed, and 50 μL of the magnetic beads was added to each well.

(8) The plate was washed twice with 100 μL of washing solution.

(9) The sample, standard, blank, and control of known concentration were vortexed, and added in an amount of 50 μL to each well.

(10) The plate was sealed with a sealing film, and shaken on a high-frequency shaker at 850±50 rpm at room temperature for 30 min.

(11) In step (10), when the remaining 10 min of shaking time was left, the detection antibody was vortexed for 5 s and diluted to 1×.

(12) The plate was washed twice with 100 μL of washing solution.

(13) The diluted antibody was vortexed, and added in an amount of 250 μL to each well.

(14) The plate was sealed with a sealing film, and shaken on a high-frequency shaker at 850±50 rpm at room temperature for 30 min.

(15) The arrangement information of the standard (provided in the kit), plate and sample were input.

(16) In step (14), when the remaining 10 min of shaking time was left, SA-PE 5 was vortexed and diluted to 1×.

(17) The plate was washed twice with 100 μL of washing solution.

(18) The diluted SA-PE was vortexed, and added in an amount of 50 μL to each well.

(19) The plate was sealed with a sealing film, and shaken on a high-frequency shaker at 850±50 rpm at room temperature for 30 min.

(20) The plate was washed three times with 100 μL of washing solution.

(21) The magnetic beads were resuspended with 125 $\mu$L of detection solution, the plate was sealed with a sealing film, and shaken on a high-frequency shaker at room temperature at 850$\pm$50 rpm for 30 s.

(22) After the sealing film was discarded, loading to machine was started.

Detection of T cell proliferation in spleen by flow cytometry

(1) The spleen was taken and digested to form single cells.

(2) Centrifuged at 1,000 r/min for 5 min.

(3) The supernatant was discarded, the pellet was resuspended in PBS, filtered through a cell sieve to remove cell clusters, counted, and subpackaged, $2\times10^6$ per tube.

(4) Centrifuged at 1200 rpm for 3 min.

(5) After blocking with 2% BSA blocking solution for 20 min, centrifugation was performed at 1200 rpm for 3 min.

(6) The supernatant was discarded, the cells were resuspended with 100 $\mu$L of 1% BSA antibody diluent, added with direct-labeled antibody, and incubated at room temperature for 30 to 45 min.

(7) Washing was performed three times with 1 mL of PBS, centrifugation was carried out at 1200 rpm for 3 min, and the supernatant was discarded.

(8) After the cells were resuspended in 300 $\mu$L of PBS, they were filtered with a 40 $\mu$m cell sieve, and loaded on the machine for detection.

Steps for tissue paraffin sectioning

(1) Fixation: the tissue was socked in 4% PFA overnight.

(2) Washing: The fixed tissue was washed three times with PBS.

(3) Sample trimming: The sample was trimmed to an appropriate size and placed in a fixation box.

(4) Alcohol gradient dehydration: 70% alcohol for 1 hour, 80% alcohol for 1 hour, 95% alcohol for 1 hour, 100% alcohol for 40 minutes, and 100% alcohol for 40 minutes.

(5) Transparentizing: xylene I for 20 min, xylene II for 20 min.

(6) Dipping wax: xylene:paraffin (1:1) for 1 h, paraffin I for 1 h, and paraffin II for 1 h.

(7) Embedding.

Hematoxylin-eosin (HE) staining

(1) The tissues embedded in paraffin were sectioned, 5 $\mu$m of thickness. The obtained sections were displayed and mounted in water in a 42°C section-displaying machine, and dried overnight in a 37°C oven.

(2) Dewaxing and rehydration of paraffin sections:
Xylene I for 10 min, xylene II for 10 min, 100% alcohol I for 5 min, 100% alcohol II for 5 min, 95% alcohol for 5 min, 80% alcohol for 5 min, and 75% alcohol for 5 min. Rinsing with PBS for 3 times, 5 min each time.

(3) Staining:
After hematoxylin staining for 3 min, dark blue-purple nuclei could be observed under microscope, and the staining was terminated with tap water.

Differentiation: the stained paraffin sections were differentiated in 1% hydrochloric acid-alcohol for 3 to 5 s.

Returning to blue: Returning to blue was performed with tap water for 15 minutes.

Eosin staining: staining was performed for 3 min.

Dehydration and transparentizing: alcohol was used for gradient dehydration, and xylene was used for transparentizing.

Mounting on slides: the sections were mounted with the neutral resin, and air bubbles should be avoided. After the slides were air-dried, they were observed under a microscope.

3. Experimental results

(1) The level of anti-double-stranded DNA antibodies in serum was detected every two weeks. The results showed that the anti-double-stranded DNA antibody levels of the model group and the treatment group increased gradually with the increase of the weeks of age, but the increase of the anti-double-stranded DNA antibody level of the treatment group was lower than that of the model group, indicating that the injection of M cells could slow down the development of systemic lupus erythematosus.

The detection of serum anti-double-stranded DNA antibody levels in each group was shown in FIG. 213.

Table 46-1: Detection of serum anti-double-stranded DNA antibody levels in each group

| Anti-double-stranded DNA antibody level | Week 10 | Week 12 | Week 14 | Week 16 |
|---|---|---|---|---|
| Control group | 29.27 | 5.99 | 7.02 | 1.64 |
| | 7.98 | 3.39 | 1.80 | 0.00 |
| | 11.46 | 3.39 | 22.70 | 6.90 |
| | 10.22 | 13.50 | | 2.16 |
| | 17.44 | 4.43 | 6.76 | 1.30 |
| Model group | 7.31 | 45.16 | 45.16 | 62.48 |
| | 6.19 | 14.48 | 43.97 | 122.50 |
| | 11.35 | 76.98 | 34.12 | 70. 70 |
| | 9.21 | 6.25 | 34.75 | 72.64 |
| | | 5.21 | 46.93 | 67.28 |
| M cell treatment group | 28.47 | 4.95 | 26.08 | 39.20 |
| | 16.88 | 36.01 | 44.37 | 46.28 |
| | 11.69 | 6.50 | 28.29 | 16.75 |
| | 22.77 | 3.39 | 39.33 | 51.30 |
| | | | 41.77 | 15.89 |

(2) The anatomical observation showed that the spleen and cervical lymph nodes of the mice in the model group were significantly enlarged, indicating systemic inflammation. However, the spleen and cervical lymph nodes of the mice in the M cell transplantation group were only slightly enlarged.

(3) The HE staining results showed that the formation rate of glomerular crescents in the M cell group was higher than that in the model group.

(4) The inflammatory factors in the serum of the mice were detected. The results showed that compared with the model group, the levels of proinflammatory factors in the M cell group were significantly decreased, and the levels of anti-inflammatory factors were significantly increased. It was shown that the M cells had the effect of suppressing inflammation.

(5) In spleen cells, the results of flow cytometry of T cell population showed that the $CD3^+T$ cells, $CD4^+T$ cells and $CD4^+T$ cells in the M cell group were all significantly lower than those in the model group.

Conclusion: The M cell injection in the systemic lupus erythematosus mice could reduce serum levels of anti-double-stranded DNA antibodies and inflammatory factors, increase the formation rate of glomerular crescents, and decrease the number of T-cell populations in the spleen, suggesting that better effect in the treatment of systemic lupus erythematosus. At the same time, it also had good therapeutic potential for other autoimmune diseases (e.g., rheumatoid arthritis, systemic vasculitis, dermatomyositis, etc.).

Although specific embodiments of the present invention have been described in detail, those skilled in the art will appreciate that various modifications and changes can be made to the details in light of all the teachings that have been published, and that these changes are all within the scope of the present invention. The whole scope of the present invention is given by the appended claims and any equivalents thereof.

## Claims

1. A mesenchymal stem cell population, wherein the mesenchymal stem cell population has an average MMP1 expression level of at least about 10 times higher than that of a primary mesenchymal stem cell; and/or, the mesenchymal stem cell population has an average PGE2 expression level of at least about 10 times higher than that of a primary mesenchymal stem cell.

2. The mesenchymal stem cell population according to claim 1, which has the following characteristics: the mesenchymal stem cell population has an average PD-L1 expression level higher than that of a primary mesenchymal stem cell after being stimulated by IFN-γ;
   preferably, the mesenchymal stem cell population has an average PD-L1 expression level of at least 2 times higher than that of a primary mesenchymal stem cell after being stimulated by IFN-γ.

3. The mesenchymal stem cell population according to claim 1 or 2, wherein the mesenchymal stem cell population has a cell expressing CD24;
   preferably, the proportion of CD24+ cells is not less than 50%.

4. The mesenchymal stem cell population according to any one of claims 1 to 3, wherein the mesenchymal stem cell population further has the following characteristics:

   (1) comprising ≥80% (e.g., ≥85%, ≥90%, ≥95%, ≥96%, ≥97%, ≥98%, ≥99%, or 100%) of cells expressing one or more selected from the group consisting of CD105, CD73, CD90, CD13, CD29, CD44, CD166 and HLA-ABC;
   (2) comprising ≤2% (e.g., ≤1%, ≤0.5%, ≤0.2%, ≤0.1%, or ≤0.01%) of cells expressing one or more selected from the group consisting of CXCL1, CD34, CD45, CD133, FGFR2, CD271, Stro-1 and CXCR4.

5. The mesenchymal stem cell population according to any one of claims 1 to 4, which further has one or more of the following characteristics:

   (1) having a cell expressing CD274; for example, the proportion of CD274+ cells is not less than 80%;
   (2) having a cell expressing CD31; for example, the proportion of CD31+ cells is not less than 5%;
   (3) the mesenchymal stem cell population has an average IDO expression level higher than that of a primary mesenchymal stem cell; for example, the mesenchymal stem cell population has an average IDO expression level of at least about 10 times higher than that of a primary mesenchymal stem cell.

6. The mesenchymal stem cell population according to any one of claims 1 to 5, wherein the expression level is an mRNA level or a protein level;
   preferably, the expression level is an mRNA level.

7. The mesenchymal stem cell population according to any one of claims 1 to 6, wherein the mesenchymal stem cell population is derived from a stem cell;
   preferably, the stem cell is a totipotent stem cell or pluripotent stem cell; preferably, the pluripotent stem cell is selected from the group consisting of embryonic stem cell, haploid stem cell, induced pluripotent stem cell, or adult stem cell.

8. A method for producing a mesenchymal stem cell population, comprising the steps of:

(1) culturing a stem cell to form an embryoid body by using a first culture medium; wherein the first culture medium is a basal medium supplemented with the following substances: one or more serum replacements, one or more non-essential amino acids, glutamine or stabilized dipeptide of L-alanyl-L-glutamine, and bFGF;

(2) culturing the embryoid body by using a second culture medium to induce its differentiation into mesenchymal stem cells; wherein the second culture medium is a basal medium supplemented with the following substances: one or more serum replacements, one or more non-essential amino acids, glutamine or stabilized dipeptide of L-alanyl-L-glutamine, and one or more growth factors;

preferably, the method is used to generate the mesenchymal stem cell population according to any one of claims 1 to 7.

9. The method according to claim 8, wherein the stem cell is a totipotent stem cell or pluripotent stem cell; preferably, the pluripotent stem cell is selected from the group consisting of embryonic stem cell, haploid stem cell, induced pluripotent stem cell, or adult stem cell.

10. The method according to claim 8 or 9, wherein the first culture medium possesses one or more of the following characteristics:

(i) the one or more serum replacements has a total content of 3 to 30% (v/v);
(ii) the one or more non-essential amino acids each has a content of 0.1 to 0.5 mM;
(iii) the glutamine or stabilized dipeptide of L-alanyl-L-glutamine has a content of 1 to 5 mM;
(iv) the bFGF has a content of 1 to 100 ng/ml.

11. The method according to any one of claims 8 to 10, wherein the first culture medium possesses one or more of the following characteristics:

(a) the serum replacement is selected from the group consisting of KOSR, MSC serum-free Supplement, Ultroser™ G and any combination thereof;
(b) the non-essential amino acid is selected from the group consisting of glycine, L-alanine, L-asparagine, L-aspartic acid, L-glutamic acid, L-proline, L-serine and combination thereof;
(c) the basal medium is selected from the group consisting of KO-DMEM, KO-DMEM/F12, DMEM, α-MEM, F-12, MEM, BME, RPMI 1640, G-MEM and any combination thereof; preferably, the basal medium is selected from the group consisting of KO-DMEM, KO-DMEM/F12, DMEM, DMEM/F12.

12. The method according to any one of claims 8 to 11, wherein the first substratum comprises: KO-DMEM, KOSR, glycine, L-alanine, L-asparagine, L-aspartate, L-glutamic acid, L-proline, L-serine, stabilized dipeptide of L-alanyl-L-glutamine, and bFGF;

preferably, the first culture medium comprises: 3 to 30% (v/v) of KOSR, 1 to 5 mM of stabilized dipeptide of L-alanyl-L-glutamine, 1 to 100 ng/ml of bFGF, and the following amino acids each at a concentration of 0.1 to 0.5 mM: glycine, L-alanine, L-asparagine, L-aspartic acid, L-glutamic acid, L-proline, L-serine.

13. The method according to any one of claims 8 to 12, wherein the first culture medium further comprises β-mercaptoethanol;

preferably, the β-mercaptoethanol has a content of 0.1 to 0.5% (v/v).

14. The method according to any one of claims 8 to 13, wherein the second substratum possesses one or more of the following characteristics:

(i) the one or more serum replacements has a total content of 1 to 40% (v/v);
(ii) the one or more non-essential amino acids each has a content of 0.1 to 0.5 mM;
(iii) the glutamine or stabilized dipeptide of L-alanyl-L-glutamine has a content of 1 to 5 mM;
(iv) the one or more growth factors each has a content of 1 to 100 ng/ml.

15. The method according to any one of claims 8 to 14, wherein the second culture medium possesses one or more of the following characteristics:

(a) the serum replacement is selected from the group consisting of KOSR, MSC serum-free Supplement, Ultroser™ G and any combination thereof;
(b) the non-essential amino acid is selected from the group consisting of glycine, L-alanine, L-asparagine, L-aspartic acid, L-glutamic acid, L-proline, L-serine and combinations thereof;

(c) the basal medium is selected from the group consisting of KO-DMEM, KO-DMEM/F12, α-MEM, DMEM, F12, MEM, BME, RPMI 1640, G-MEM and any combination thereof; preferably, the basal medium is selected from the group consisting of KO-DMEM, KO-DMEM/F12, α-MEM, DMEM, DMEM/F12;

(d) the one or more growth factors is selected from the group consisting of VEGF, bFGF, EGF, TGFβ or PDGF.

16. The method according to any one of claims 8 to 15, wherein the second culture medium comprises: KO-DMEM/F12, α-MEM, MSC serum-free Supplement or Ultraser G, KOSR, glycine, L-alanine, L-asparagine, L-aspartic acid, L-glutamic acid, L-proline, L-serine, stabilized dipeptide of L-alanyl-L-glutamine, one or more growth factors (e.g., one or more selected from the group consisting of VEGF, bFGF, EGF, TGFβ, PDGF);

preferably, the second culture medium comprises: 1 to 10% (v/v) of Ultroser G, 1 to 20% (v/v) of KOSR, 1 to 5 mM of stabilized dipeptide of L-alanyl-L-glutamine, one or more growth factors (e.g., one or more selected from the group consisting of VEGF, bFGF, EGF, TGFβ, PDGF) each at a concentration of 1 to 100 ng/ml, and the following amino acids each at a concentration 0.1 to 0.5 mM: glycine, L-alanine, L-asparagine, L-aspartic acid, L-glutamic acid, L-proline, L-serine.

17. The method according to any one of claims 8 to 16, wherein the second culture medium further comprises ascorbic acid;

preferably, the ascorbic acid has a content of 1 to 100 μg/ml.

18. The method according to any one of claims 8 to 17, wherein the step (1) comprises culturing the stem cell in a low-attachment cell culture vessel.

19. The method according to in any one of claim 8 to 18, wherein, the step (2) comprises culturing the embryoid body in a culture dish coated with gelatin, collagen type I, collagen type IV, vitronectin, fibronectin or polylysine.

20. The method according to any one of claims 8 to 19, wherein the method further comprises: (3) separating the cells attached to culture container in step (2), thereby obtaining mesenchymal stem cells.

21. The method according to claim 20, wherein the method further comprises passaging the mesenchymal stem cells of step (3);

preferably, the cells are passaged when the cells have a confluence of greater than or equal to about 80% (e.g., greater than or equal to about 85%, greater than or equal to about 90%, or greater than or equal to about 95%); preferably, the mesenchymal stem cells are passaged for 1, 2, 3, 4 or 5 passages; preferably, the passaging comprises inoculating cells in the second culture medium for culturing.

22. A culture, which comprises the mesenchymal stem cell population according to any one of claims 1 to 7, and a culture medium.

23. A culture supernatant, which is a culture supernatant produced by culturing the mesenchymal stem cell population according to any one of claims 1 to 7 in a culture medium.

24. A composition, which comprises the mesenchymal stem cell population according to any one of claim 1 to 7, the culture according to claim 22 or the culture supernatant according to claim 23, and a carrier or excipient;

preferably, the composition is an injection, microinjection, mucosal patch, enema, suppository, gel, oral preparation, aerosol, drop, ointment, implant, capsule or aerosol; preferably, the composition is an injection; preferably, the composition comprises a pharmaceutically acceptable sterile isotonic aqueous or non-aqueous solution, dispersion, suspension or emulsion; preferably, the composition is a pharmaceutical composition.

25. A kit, which comprises a first culture medium and a second culture medium, wherein,

the first culture medium is a basal medium supplemented with the following substances: one or more serum replacements, one or more non-essential amino acids, glutamine or stabilized dipeptide of L-alanyl-L-glutamine, and bFGF;

the second culture medium is a basal medium supplemented with the following substances: one or more serum replacements, one or more non-essential amino acids, glutamine or stabilized dipeptide of L-alanyl-L-glutamine, and one or more growth factors;

preferably, the first culture medium is as defined in any one of claims 10 to 13;

preferably, the second culture medium is as defined in any one of claims 14 to 17;

preferably, the first culture medium and the second culture medium are provided separately.

26. Use of the mesenchymal stem cell population according to any one of claim 1 to 7, the culture according to claim 22, the culture supernatant according to claim 23 or the composition according to claim 24, in the manufacture of a medicament for the prevention and/or treatment of a disease in a subject, the disease being selected from the group consisting of osteoarthropathy (e.g., meniscus injury, osteoarthritis, or bone injury), reproductive system disease (e.g., ovarian aging, ovarian insufficiency, endometrial damage, uterine trauma, intrauterine adhesions, or thin uterus), cardiac disease (e.g., myocardial infarction), lung disease (e.g., idiopathic pulmonary fibrosis, acute respiratory distress disorder, pneumoconiosis, or pneumonia), skin disease (e.g., psoriasis, skin injury, bedsore, pressure ulcer, or burn), eye disease (e.g., corneal injury), nervous system disease (e.g., spinal cord injury, cerebral palsy, cerebral apoplexy, Alzheimer's disease, dementia, or neuropathic pain), digestive system disease (e.g., inflammatory bowel disease, colitis, Crohn's disease, or irritable bowel syndrome), kidney disease (e.g., anti-glomerular basement membrane disease, diabetic nephropathy, lupus nephritis, or acute nephritis), liver disease (liver injury, liver fibrosis, hepatitis, cirrhosis, or liver failure), autoimmune disease (e.g., scleroderma, lupus erythematosus, or multiple sclerosis), transplant rejection (e.g., graft-versus-host disease), metabolic disease (e.g., diabetes).

FIG. 1

FIG. 2A

FIG. 2B

FIG. 2C

FIG. 2D

TGF-β1    -   +   +
MSC culture supernatant   -   -   +

α-SMA

Collagen I

β-actin

**FIG. 3**

IDO

■ primary MSC
▨ MSC of Preparation Example 1

**FIG. 4A**

PD-L1

■ primary MSC
▨ MSC of Preparation Example 1

**FIG. 4B**

FIG. 4C

FIG. 5

FIG. 6

Continued

FIG. 6

Formulation 2

FIG. 7

Continued

FIG. 7

FIG. 8

Formulation 3

322

Continued

FIG. 8

**FIG. 9**

FIG. 9

FIG. 10

Formulation 5

Continued

FIG. 10

FIG. 11

FIG. 12

FIG. 13

328

# Formulation 1

**FIG. 14**

EP 4 047 083 A1

Formulation 2

FIG. 15

330

Formulation 3

FIG. 16

Formulation 4

FIG. 17

## Formulation 5

FIG. 18

EP 4 047 083 A1

**FIG. 19**

**FIG. 20**

FIG. 21

Formulation 1

FIG. 22

FIG. 23

FIG. 24

FIG. 24

FIG. 24

FIG. 25

EP 4 047 083 A1

FIG. 25

342

FIG. 25

## MMP1

**FIG. 26**

## PGE2

**FIG. 27**

Formulation 3-1  Formulation 3-2  Formulation 3-3  Formulation 3-4  Formulation 3-5

200 μm

**FIG. 28**

FIG. 29

FIG. 29

FIG. 30

FIG. 30

FIG. 31

FIG. 31

EP 4 047 083 A1

MMP1

**FIG. 32**

PGE2

**FIG. 33**

Formulation 3-2-1    Formulation 3-2-2    Formulation 3-2-3

Formulation 3-5-1    Formulation 3-5-2    Formulation 3-5-3    Formulation 3-5-4

**FIG. 34**

## Formulation 3-2-3

**FIG. 35**

Formulation 3-5-4

FIG. 36

FIG. 37

FIG. 38

FIG. 39

FIG. 40

FIG. 41

**Day 1**

**Day 3**

magnification

High concentration cell suspension seeded in electrospun gelatin fiber sheet

**FIG. 42**

Xiamen collagen scaffold  Day5-blank material  Day5  Day7  Day9

**FIG. 43**

Shandong Zhenghai skin repair membrane  Day5-blank material  Day5  Day7  Day9

**FIG. 44**

FIG. 45

**FIG. 46**

**FIG. 47**

**FIG. 48**

FIG. 49

FIG. 50

**FIG. 51**

**FIG. 52**

**FIG. 53**

**FIG. 54**

1day          4day          6day

**FIG. 55**

3min | 10min

**FIG. 56**

**FIG. 57**

Before cryopreservation

After MZJ cryopreservation

After CS10 cryopreservation

After vitrification cryopreservation

**FIG. 58**

1day        4day        6day

**FIG. 59**

0min        10min        40min

**FIG. 60**

Before cryopreservation

After MZJ cryopreservation

After CS10 cryopreservation

After vitrification cryopreservation

**FIG. 61**

1day 4day 6day

**FIG. 62**

0min 10min 40min

**FIG. 63**

**FIG. 64**

**FIG. 65**

1day                4day                6day

**FIG. 66**

3min                          10min

**FIG. 67**

FIG. 68

Before
cryopreservation

After MZJ
cryopreservation

After CS10
cryopreservation

FIG. 69

1day　　　　　　　　4day　　　　　　　　6day

FIG. 70

3min        10min

**FIG. 71**

**FIG. 72**

Before
cryopreservation

After MZJ
cryopreservation

After CS10
cryopreservation

**FIG. 73**

0day      1day      4day

FIG. 74

FIG. 75

0day      6day    10day

FIG. 76

D2

D4

D6

D10

FIG. 77

0day    1day    4day

FIG. 78

FIG. 79

0day    6day    10day

FIG. 80

D2

D4

D6

D10

FIG. 81

0day          1day          4day

**FIG. 82**

**FIG. 83**

0day          6day          10day

**FIG. 84**

D2

D4

D6

D10

**FIG. 85**

D1                     D3

**FIG. 86**

**FIG. 87**

FIG. 88

FIG. 89

FIG. 90

FIG. 91

D2

D5

**FIG. 92**

D0　　　　　　　D1　　　　　　　D5

**FIG. 93**

D2

D5

**FIG. 94**

D0          D1          D5

FIG. 95

D2

D5

FIG. 96

FIG. 97

**FIG. 98**

**FIG. 99**

FIG. 100

FIG. 100

Normal group

Model group

M cell group

FIG. 101

Normal group | Model group | Model + M cell group

**FIG. 102**

Day of surgery

Muscle adhesions at the bottom of uterus, without separation | Separation of adhesions and scar at left uterine horn

4 weeks after surgery

Adhesions at the bottom of uterus and surrounding glands | Repair of scar at left uterine horn

4 months after surgery

The opening of right fallopian tube was not observed | View of uterine cavity and scar at left uterine horn

8 months after surgery

The opening of right fallopian tube was observed | View of uterine cavity and recover of left uterine horn

**FIG. 103**

FIG. 104

FIG. 105

FIG. 106

**FIG. 107**

**FIG. 108**

**FIG. 109**

FIG. 110

FIG. 111

FIG. 112

**FIG. 113**

**FIG. 114**

**FIG. 115**

FIG. 116

FIG. 117

FIG. 118

FIG. 119

FIG. 120

**FIG. 121**

**FIG. 122**

Model group    M cell group

Day 10

normal saline        M cell

Day 20

normal saline        M cell

**FIG. 123**

**FIG. 124**

Normal group        Model group        M cell group

**FIG. 125**

FIG. 126

FIG. 127

FIG. 128A

FIG. 128B

FIG. 129

Normal group          BLM group          M cell group

FIG. 130

**FIG. 131**

**FIG. 132**

**FIG. 133**

**FIG. 134**

| Time (day) | non-healing rate of wounds (%) | | | |
|---|---|---|---|---|
| | Model group | | M cell group | |
| 7 | 100 | 100 | 100 | 100 |
| 14 | 27.39 | 29.35 | 21.77 | 25.13 |
| 21 | 21.76 | 26.32 | 16.06 | 14.27 |
| 28 | 21.03 | 22.73 | 7.02 | 11.74 |

**FIG. 135**

**FIG. 136**

**FIG. 137**

Total number of RBC

Normal control + solvent
Cisplatin + solvent
Cisplatin + M cell

**FIG. 138**

Hemoglobin

Normal control + solvent
Cisplatin + solvent
Cisplatin + M cell

**FIG. 139**

FIG. 140

FIG. 141

**FIG. 142**

**FIG. 143**

**FIG. 144**

**FIG. 145**

**FIG. 146**

**FIG. 147**

**FIG. 148**

**FIG. 149**

**FIG. 150**

**FIG. 151**

**FIG. 152**

**FIG. 153**

**FIG. 154**

**FIG. 155**

**FIG. 156**

FIG. 157

FIG. 158

FIG. 159

Normal group     Model group     M cell group

FIG. 160

Normal group

Model group

M cell group

FIG. 161

Model group

M cell group

FIG. 162

Model group

M cell group

FIG. 163

FIG. 164

FIG. 165

EB          EB-P0          IPS-M-P5

**FIG. 166**

**FIG. 167**

FIG. 168

FIG. 169

FIG. 170

FIG. 171

FIG. 172

FIG. 173

FIG. 174

FIG. 175

**FIG. 176**

**FIG. 177**

**FIG. 178**

FIG. 179

FIG. 180

HE

**FIG. 181**

Masson

**FIG. 182**

**α-Smooth Muscle Actin**

| G1 left kidney | G2 left kidney | G3 left kidney |
| G1 right kidney | G2 right kidney | G3 right kidney |

**CD31**

| G1 left kidney | G2 left kidney | G3 left kidney |
| G1 right kidney | G2 right kidney | G3 right kidney |

**FIG. 183**

**FIG. 184**

**FIG. 185**

**FIG. 186**

**FIG. 187**

**FIG. 188**

**FIG. 189**

**FIG. 190**

EP 4 047 083 A1

FIG. 191

FIG. 192

FIG. 193

414

MRI image       Enlarged view of MRI

Before treatment

3 months after M
cell treatment

FIG. 194

FIG. 195

FIG. 196

**FIG. 197**

**FIG. 198**

**FIG. 199**

FIG. 200

FIG. 201

FIG. 202

**FIG. 203**

**FIG. 204**

**FIG. 205**

FIG. 206

FIG. 207

**FIG. 208**

**FIG. 209**

FIG. 210

FIG. 211

FIG. 212

**FIG. 213**

**FIG. 214**

**FIG. 215**

**FIG. 216**

## INTERNATIONAL SEARCH REPORT

| International application No. |
|---|
| **PCT/CN2020/116626** |

**A. CLASSIFICATION OF SUBJECT MATTER**

C12N 5/0775(2010.01)i; A61K 35/545(2015.01)i

According to International Patent Classification (IPC) or to both national classification and IPC

**B. FIELDS SEARCHED**

Minimum documentation searched (classification system followed by classification symbols)

C12N; A61K

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, USTXT, EPTXT, CNKI, 万方数据资源系统, Wanfang Data Resource System, 百度学术, BAIDU XUESHU, PubMed, ISI Web of Knowledge: 申请人/发明人, applicant/inventor, 间充质干细胞, MSC, 基质金属蛋白酶1, MMP1, 前列腺素E2, PGE2, 拟胚体, embryoid, 胚状体, IFN-gamma, PD-L1, bFGF, TGF-beta

**C. DOCUMENTS CONSIDERED TO BE RELEVANT**

| Category* | Citation of document, with indication, where appropriate, of the relevant passages | Relevant to claim No. |
|---|---|---|
| X | CN 105473709 A (STEMPEUTICS RESEARCH PVT. LTD.) 06 April 2016 (2016-04-06) | 1, 3-7, 22-26 |
| Y | CN 105473709 A (STEMPEUTICS RESEARCH PVT. LTD.) 06 April 2016 (2016-04-06) | 2, 8-21 |
| Y | GUAN, Q.D. et al. "Inducible Indoleamine 2, 3-Dioxygenase 1 and Programmed Death Ligand 1 Expression as the Potency Marker for Mesenchymal Stromal Cells" *Cytotherapy*, Vol. 20, 31 December 2018 (2018-12-31), pp. 639-649 | 2 |
| Y | CN 109735488 A (HANGZHOU YUANSHENG BIOTECHNOLOGY CO., LTD.) 10 May 2019 (2019-05-10) | 8-21 |
| X | DE MAYO, T. et al. "The Role of Bone Marrow Mesenchymal Stromal Cell Derivatives in Skin Wound Healing in Diabetic Mice" *PLoS ONE*, Vol. 12, No. 6, 08 June 2017 (2017-06-08), article number e0177533, pp. 1-17 | 1, 3-7, 22-26 |
| Y | WO 2018225705 A1 (TERUMO KABUSHIKI KAISHA et al.) 13 December 2018 (2018-12-13) the abstract, and claims 1-15 | 8-21 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 December 2020** | **21 December 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

EP 4 047 083 A1

**INTERNATIONAL SEARCH REPORT**

International application No.

**PCT/CN2020/116626**

### C. DOCUMENTS CONSIDERED TO BE RELEVANT

| Category* | Citation of document, with indication, where appropriate, of the relevant passages | Relevant to claim No. |
|---|---|---|
| Y | KR 20180078160 A (SUNGKWANG MEDICAL FOUND et al.) 09 July 2018 (2018-07-09)<br>the abstract, and the figure of the abstract | 8-21 |
| A | WO 2011101834 A1 (ADVANCED NEURO-SCIENCE ALLIES PRIVATE LIMITED) 25 August 2011 (2011-08-25)<br>entire document | 1-26 |

Form PCT/ISA/210 (second sheet) (January 2015)

425

**INTERNATIONAL SEARCH REPORT**

| International application No. |
| --- |
| **PCT/CN2020/116626** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

 a.  ☑ forming part of the international application as filed:

  ☑ in the form of an Annex C/ST.25 text file.

  ☐ on paper or in the form of an image file.

 b.  ☐ furnished together with the international application under PCT Rule 13*ter*.1(a) for the purposes of international search only in the form of an Annex C/ST.25 text file.

 c.  ☐ furnished subsequent to the international filing date for the purposes of international search only:

  ☐ in the form of an Annex C/ST.25 text file (Rule 13*ter*.1(a)).

  ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
**Information on patent family members**

International application No.

**PCT/CN2020/116626**

| Patent document cited in search report | | | Publication date (day/month/year) | Patent family member(s) | | | Publication date (day/month/year) |
|---|---|---|---|---|---|---|---|
| CN | 105473709 | A | 06 April 2016 | IL | 243530 | D0 | 31 March 2016 |
| | | | | HK | 1217514 | A1 | 13 January 2017 |
| | | | | JP | 2016528911 | A | 23 September 2016 |
| | | | | PH | 12016500252 | A1 | 16 May 2016 |
| | | | | SG | 11201600219 T | A | 26 February 2016 |
| | | | | AU | 2014313874 | A1 | 25 February 2016 |
| | | | | US | 2016206550 | A1 | 21 July 2016 |
| | | | | BR | 112016002040 | A2 | 01 August 2017 |
| | | | | MX | 2016002084 | A | 23 June 2016 |
| | | | | WO | 2015028900 | A1 | 05 March 2015 |
| | | | | EP | 3039124 | A1 | 06 July 2016 |
| CN | 109735488 | A | 10 May 2019 | None | | | |
| WO | 2018225705 | A1 | 13 December 2018 | JP | WO2018225705 | A1 | 02 April 2020 |
| | | | | EP | 3569693 | A1 | 20 November 2019 |
| | | | | CN | 110506109 | A | 26 November 2019 |
| | | | | US | 2020102541 | A1 | 02 April 2020 |
| KR | 20180078160 | A | 09 July 2018 | None | | | |
| WO | 2011101834 | A1 | 25 August 2011 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7410798 B
- CN 201880036997
- CN 201780077281
- CN 201811145836
- CN 201811227664
- CN 201610208206
- CN 201380072996
- CN 201410188453
- CN 201110041925
- CN 201910389341
- CN 201810277760
- CN 200910209321
- CN 103920188 B
- CN 104398698 A

**Non-patent literature cited in the description**

- Cell Therapy: stem cell Transplantation, Gene Therapy, and Cellular Immunotherapy. Cambridge University Press, 1996
- **E.D. BALL ; J. LISTER ; P. LAW.** Hematopoietic stem cell treatment. Churchill Livingstone, 2000
- **KLIMANSKAYA et al.** *Lancet,* 07 May 2005, vol. 365 (9471), 1636-41
- **RICHARDS et al.** *stem cell Cells,* 2003, vol. 21 (5), 546-56
- **ILIC et al.** *stem cells Dev.,* November 2009, vol. 18 (9), 1343-5
- **XU et al.** *Nat Biotechnol.,* October 2001, vol. 19 (10), 971-4
- Cell Therapy: stem cell Transplantation, Gene Therapy and Cellular Immunotherapy. Cambridge University Press, 1996
- **E.D. BALL ; J. LISTER ; P.LAW.** Hematopoietic stem cell treatment. Churchill Livingstone, 2000
- Cell Therapy: Stem Cell Transplantation, Gene Therapy, and Cellular Immunotherapy. Cambridge University Press, 1996
- **E.D. BALL ; J. LISTER ; P. LAW.** Hematopoietic Stem Cell Therapy. Churchill Livingstone, 2000
- Remington's Pharmaceutical Sciences. Mack Publishing Company, 1995
- **HWANG NS et al.** *Proc Natl Acad Sci US A.,* 30 December 2008, vol. 105 (52), 20641-6
- **JONG-SUNG PARK ; YUMIN OH ; YONG JOO PARK et al.** Targeting of dermal myofibroblasts through death receptor 5 arrests fibrosis in mouse models of scleroderma. *Nat Commun.,* 08 March 2019, vol. 10 (1), 1128
- **MATSUDA F. et al.** *Acta Physiol Neurosci,* 2011
- **SHEN H. et al.** *J Neurosci Methods,* 2010
- **KRIKS et al.** *Nature,* 2011
- **BÉTEMPS et al.** *J Vis Exp.,* 2015
- **SEE JOO YOUN OH.** anti-inflammatory protein TSG-6 reduces inflammatory damage to the cornea following chemical and mechanical injury. *PNAS,* 2010, vol. 107 (39), 16875-16880
- Committee opinion no. 605: primary ovarian insufficiency in adolescents and young women. *Obstet Gynecol,* 2014, vol. 124 (1), 193-197
- **TAVASSOLI M ; CROSBY WH.** Transplantation of marrow to extramedullary sites. *Science,* 1968, vol. 161 (3836), 54-56
- **JOHNSON J ; BAGLEY J ; SKAZNIK-WIKIEL M et al.** Oocyte generation in adult mammalian ovaries by putative germ cells in bone marrow and peripheral blood. *Cell,* 2005, vol. 122 (2
- **WANG S ; YU L ; SUN M et al.** The therapeutic potential of umbilical cord mesenchymal stem cells in mice premature ovarian failure. *Biomed Res Int,* 2013, vol. 2013, 690491
- **GIBSON, J.D. et al.** Regeneration of Articular Cartilage by Human ESC-Derived Mesenchymal Progenitors Treated Sequentially with BMP to 2 and Wnt5a. *stem cellS Translational Medicine,* 2017, vol. 6 (1), 40-50

- **GONZALO TO GIL, E. et al.** Human embryonic stem cell-derived mesenchymal stromal cells ameliorate collagen-induced arthritis by inducing host-derived indoleamine 2,3 dioxygenase. *Arthritis Res Ther,* 2016, vol. 18, 77
- **NINAGAWA, N.T. et al.** Transplantated mesenchymal stem cells derived from embryonic stem cells promote muscle regeneration and accelerate functional recovery of injured skeletal muscle. *Biores Open Access,* 2013, vol. 2 (4), 295-306
- **ZHANG, Y. et al.** Improved cell survival and paracrine capacity of human embryonic stem cell-derived mesenchymal stem cells promote therapeutic potential for pulmonary arterial hypertension. *Cell Transplant,* 2012, vol. 21 (10), 2225-39
- **WANG, X. et al.** Immune modulatory mesenchymal stem cells derived from human embryonic stem cells through a trophoblast to like stage. *stem cells,* 2016, vol. 34 (2), 380-385
- **HU YU.** *Journal of Jiangsu University,* 2020
- **BÉTEMPS et al.** *J Vis Exp,* 2015
- **LYKHMUS et al.** *Frontiers in Pharmacology,* 2019
- **LIMING DU.** *Cell Metabolism,* 2019
- **LIANHUA BAI.** *Nature neuroscience,* 2012
- **DU.** *Cell Metabolism,* 2019
- **DANG.** *Autophagy,* 2014
- **BAI.** *Nature neuroscience,* 2012
- **CUI, Z. ; M.H. ZHAO.** *Nat Rev Nephrol,* 2011, vol. 7 (12), 697
- **FURUHASHI, K. et al.** *J Am Soc Nephrol,* 2013, vol. 24 (4), 587
- **SUZUKI, T. et al.** *PLoS One,* 2013, vol. 8 (6), e67475

SEQUENCE LISTING

<110>  BEIJING INSTITUTE FOR STEM CELL AND REGENERATIVE MEDICINE
       INSTITUTE OF ZOOLOGY, CHINESE ACADEMY OF SCIENCES

<120>  PLURIPOTENT STEM CELL, PHARMACEUTICAL COMPOSITION, AND PREPARATION
METHOD THEREFOR AND APPLICATION THEREOF

<130>  IEC200342PCT

<150>  201910893475.0
<151>  2019-09-20

<150>  202010587096.1
<151>  2020-06-24

<160>  10

<170>  PatentIn version 3.5

<210>  1
<211>  21
<212>  DNA
<213>  Artificial sequence

<220>
<223>  IDO-F

<400>  1
gccagcttcg agaaagagtt g                                                    21


<210>  2
<211>  21
<212>  DNA
<213>  Artificial sequence

<220>
<223>  IDO-R

<400>  2
atcccagaac tagacgtgca a                                                    21


<210>  3
<211>  22
<212>  DNA
<213>  Artificial sequence

<220>
<223>  MMP1-F

<400>  3
aaaattacac gccagatttg cc                                                   22


<210>  4
<211>  23
<212>  DNA
<213>  Artificial sequence

<220>
<223>  MMP1-R

<400> 4
ggtgtgacat tactccagag ttg                                                    23

<210> 5
<211> 21
<212> DNA
<213> Artificial sequence

<220>
<223> PDL1-R

<400> 5
ggacaagcag tgaccatcaa g                                                      21

<210> 6
<211> 23
<212> DNA
<213> Artificial sequence

<220>
<223> PDL1-F

<400> 6
cccagaatta ccaagtgagt cct                                                    23

<210> 7
<211> 18
<212> DNA
<213> Artificial sequence

<220>
<223> PGE2-R

<400> 7
ggcgggcgtt tcgaactt                                                          18

<210> 8
<211> 18
<212> DNA
<213> Artificial sequence

<220>
<223> PGE2-F

<400> 8
cgggtccatg ttcgctcc                                                          18

<210> 9
<211> 20
<212> DNA
<213> Artificial sequence

<220>
<223> GAPDH-F

<400> 9
ctctgctcct cctgttcgac                                                        20

```
<210>   10
<211>   20
<212>   DNA
<213>   Artificial sequence

<220>
<223>   GAPDH-R

<400>   10
cgaccaaatc cgttgactcc                                                        20
```